# EUROPEAN PATENT APPLICATION

(11) **EP 3 112 382 A1**
(43) Date of publication of application: **04.01.2017**
(21) Application number: 16169947.5
(22) Date of filing: 29.12.2010
(51) Int. Cl.: C07K 16/28, C07K 16/46, A61K 39/395, A61P 35/00, A61P 37/00

(54) **HETERODIMER BINDING PROTEINS AND USES THEREOF**

(30) Priority: 29.12.2009 US 290840 P; 16.07.2010 US 365266 P; 22.07.2010 US 366743 P
(62) Divisional of application: 10803713.6
(71) Applicant: Emergent Product Development Seattle, LLC, Seattle, Washington 98121 (US)
(72) Inventor: BLANKENSHIP, John W., Seattle, WA 98117 (US); TAN, Philip, Edmonds, WA 98026 (US)
(74) Representative: Walker, Ross Thomson

(57) **Abstract**

The present disclosure provides polypeptide heterodimers formed between two different single chain fusion polypeptides via natural heterodimerization of an immunoglobulin CH1 region and an immunoglobulin light chain constant region (CL). The polypeptide heterodimer comprises two or more binding domains that specifically bind one or more targets (e.g., a receptor). In addition, both chains of the heterodimer further comprise an Fc region portion. The present disclosure also provides nucleic acids, vectors, host cells and methods for making polypeptide heterodimers as well as methods for using such polypeptide heterodimers, such as in directing T cell activation, inhibiting solid malignancy growth, and treating autoimmune or inflammatory conditions.

SEQUENCE LISTING

<110> Emergent Product Development Seattle, LLC
      Blankenship, John W.
      Tan, Philip

<120> HETERODIMER BINDING PROTEINS AND USES
  THEREOF

<130> 910180.422PC

<140> PCT
<141> 2010-12-29

<150> US 61/290,840
<151> 2009-12-29

<150> US 61/365,266
<151> 2010-07-16

<160> 882

<170> FastSEQ for Windows Version 4.0

<210> 1
<211> 2088
<212> DNA
<213> Artificial Sequence

<220>
<223> nucleotide sequence of 2E12CH1CH2CH3Ck (X0130)

<400> 1
caggtgcagc tgaaggagtc aggacctggc agcgtggcgc cctcacagag cctgtccatc 60
acatgcaccg tctcagggtt ctcattaacc ggctatggtg taaactgggt tcgccagcct 120
ccaggaaagg gtctggagtg gctgggaatg atatggggtg atggaagcac agactataat 180
tcagctctca aatccagact atcgatcacc aaggacaact ccaagagcca agttttctta 240
aaaatgaaca gtctgcaaac tgatgacaca gccagatact actgtgctcg ggatggttat 300
agtaactttc attactatgt tatggactac tggggtcaag gaacctcagt caccgtctcc 360
tctgggggtg gaggctctgg tggcggtgga tccggcggag gtgggtcggg tggcggcgga 420
tctgacattg tgctcaccca atctccagct tctttggctg tgtctctagg tcagagagcc 480
accatctcct gcagagccag tgaaagtgtt gaatattatg tcacaagttt aatgcagtgg 540
taccaacaga aaccaggaca gccacccaaa ctcctcatct ctgctgctag caacgtagaa 600
tctggggtcc ctgccaggtt tagtggcagt gggtctggga cagactttag cctcaacatc 660
catcctgtgg aggaggatga tattgcaatg tatttctgtc agcaaagtag gaaggttcca 720
tggacgttcg gtggaggcac caagctggaa atcaagtcct cgagcgcctc caccaagggc 780
ccatcggtct tccccctggc accctcctcc aagagcacct ctgggggcac agcggccctg 840
ggctgcctgg tcaaggacta cttccccgag ccggtgacgg tgtcgtggaa ctcaggcgcc 900
ctgaccagcg gcgtgcacac cttcccggct gtcctacagt cctcaggact ctactccctc 960
agcagcgtgg tgaccgtgcc ctccagcagc ttgggcaccc agacctacat ctgcaacgtg 1020
aatcacaagc ccagcaacac caaggtggac aagaaagttc gtacggagcc caaatcttct 1080
gacaaaactc acacatgccc accgtgccca tccggagcac ctgaactcct gggtggaccg 1140
tcagtcttcc tcttcccccc aaaacccaag gacaccctca tgatctcccg gacccctgag 1200
gtcacatgcg tggtggtgga cgtgagccac gaagaccctg aggtcaagtt caactggtac 1260
gtggacggcg tggaggtgca taatgccaag acaaagccgc gggaggagca gtacaacagc 1320
acgtaccgtg tggtcagcgt cctcaccgtc ctgcaccagg actggctgaa tggcaaggag 1380
tacaagtgca aggtctccaa caaagccctc ccagccccca tcgagaaaac catctccaaa 1440
gccaaagggc agccccgaga accacaggtg tacaccctgc ccccatcccg ggatgagctg 1500
accaagaacc aggtcagcct gacctgcctg gtcaaaggct tctatccaag cgacatcgcc 1560
gtggagtggg agagcaatgg gcagccggag aacaactaca agaccacgcc tcccgtgctg 1620
gactccgacg gctccttctt cctctacagc aagctcaccg tggacaagag caggtggcag 1680
caggggaacg tcttctcatg ctccgtgatg catgaggctc tgcacaacca ctacacgcag 1740

aagagcctct ccctgtctcc gggtaaatct agaactgtgg ctgcaccatc tgtcttcatc 1800
ttcccgccat ctgatgagca gttgaaatct ggaactgcct ctgttgtgtg cctgctgaat 1860
aacttctatc ccagagaggc caaagtacag tggaaggtgg ataacgccct ccaatcgggt 1920
aactcccagg agagtgtcac agagcaggac agcaaggaca gcacctacag cctcagcagc 1980
accctgacgc tgagcaaagc agactacgag aaacacaaag tctacgcctg cgaagtcacc 2040
catcagggcc tgagctcgcc cgtcacaaag agcttcaaca ggggagag 2088

<210> 2
<211> 696
<212> PRT
<213> Artificial Sequence

<220>
<223> amino acid sequence of 2E12CH1CH2CH3Ck (X0130)

<400> 2
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala
                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser Gly Gly Gly Gly Ser Gly Gly
        115                 120                 125
Gly Gly Ser Gly Gly Gly Gly Ser Gly Gly Gly Gly Ser Asp Ile Val
    130                 135                 140
Leu Thr Gln Ser Pro Ala Ser Leu Ala Val Ser Leu Gly Gln Arg Ala
145                 150                 155                 160
Thr Ile Ser Cys Arg Ala Ser Glu Ser Val Glu Tyr Tyr Val Thr Ser
                165                 170                 175
Leu Met Gln Trp Tyr Gln Gln Lys Pro Gly Gln Pro Pro Lys Leu Leu
            180                 185                 190
Ile Ser Ala Ala Ser Asn Val Glu Ser Gly Val Pro Ala Arg Phe Ser
        195                 200                 205
Gly Ser Gly Ser Gly Thr Asp Phe Ser Leu Asn Ile His Pro Val Glu
    210                 215                 220
Glu Asp Asp Ile Ala Met Tyr Phe Cys Gln Gln Ser Arg Lys Val Pro
225                 230                 235                 240
Trp Thr Phe Gly Gly Gly Thr Lys Leu Glu Ile Lys Ser Ser Ser Ala
                245                 250                 255
Ser Thr Lys Gly Pro Ser Val Phe Pro Leu Ala Pro Ser Ser Lys Ser
            260                 265                 270
Thr Ser Gly Gly Thr Ala Ala Leu Gly Cys Leu Val Lys Asp Tyr Phe
        275                 280                 285
Pro Glu Pro Val Thr Val Ser Trp Asn Ser Gly Ala Leu Thr Ser Gly
    290                 295                 300
Val His Thr Phe Pro Ala Val Leu Gln Ser Ser Gly Leu Tyr Ser Leu
305                 310                 315                 320
Ser Ser Val Val Thr Val Pro Ser Ser Ser Leu Gly Thr Gln Thr Tyr
                325                 330                 335
Ile Cys Asn Val Asn His Lys Pro Ser Asn Thr Lys Val Asp Lys Lys
            340                 345                 350
Val Arg Thr Glu Pro Lys Ser Ser Asp Lys Thr His Thr Cys Pro Pro
        355                 360                 365
Cys Pro Ser Gly Ala Pro Glu Leu Leu Gly Gly Pro Ser Val Phe Leu

370                 375                 380
Phe Pro Pro Lys Pro Lys Asp Thr Leu Met Ile Ser Arg Thr Pro Glu
385                 390                 395                 400
Val Thr Cys Val Val Val Asp Val Ser His Glu Asp Pro Glu Val Lys
                405                 410                 415
Phe Asn Trp Tyr Val Asp Gly Val Glu Val His Asn Ala Lys Thr Lys
            420                 425                 430
Pro Arg Glu Glu Gln Tyr Asn Ser Thr Tyr Arg Val Val Ser Val Leu
        435                 440                 445
Thr Val Leu His Gln Asp Trp Leu Asn Gly Lys Glu Tyr Lys Cys Lys
    450                 455                 460
Val Ser Asn Lys Ala Leu Pro Ala Pro Ile Glu Lys Thr Ile Ser Lys
465                 470                 475                 480
Ala Lys Gly Gln Pro Arg Glu Pro Gln Val Tyr Thr Leu Pro Pro Ser
                485                 490                 495
Arg Asp Glu Leu Thr Lys Asn Gln Val Ser Leu Thr Cys Leu Val Lys
            500                 505                 510
Gly Phe Tyr Pro Ser Asp Ile Ala Val Glu Trp Glu Ser Asn Gly Gln
        515                 520                 525
Pro Glu Asn Asn Tyr Lys Thr Thr Pro Pro Val Leu Asp Ser Asp Gly
    530                 535                 540
Ser Phe Phe Leu Tyr Ser Lys Leu Thr Val Asp Lys Ser Arg Trp Gln
545                 550                 555                 560
Gln Gly Asn Val Phe Ser Cys Ser Val Met His Glu Ala Leu His Asn
                565                 570                 575
His Tyr Thr Gln Lys Ser Leu Ser Leu Ser Pro Gly Lys Ser Arg Thr
            580                 585                 590
Val Ala Ala Pro Ser Val Phe Ile Phe Pro Pro Ser Asp Glu Gln Leu
        595                 600                 605
Lys Ser Gly Thr Ala Ser Val Val Cys Leu Leu Asn Asn Phe Tyr Pro
    610                 615                 620
Arg Glu Ala Lys Val Gln Trp Lys Val Asp Asn Ala Leu Gln Ser Gly
625                 630                 635                 640
Asn Ser Gln Glu Ser Val Thr Glu Gln Asp Ser Lys Asp Ser Thr Tyr
                645                 650                 655
Ser Leu Ser Ser Thr Leu Thr Leu Ser Lys Ala Asp Tyr Glu Lys His
            660                 665                 670
Lys Val Tyr Ala Cys Glu Val Thr His Gln Gly Leu Ser Ser Pro Val
        675                 680                 685
Thr Lys Ser Phe Asn Arg Gly Glu
    690                 695


<210> 3
<211> 2142
<212> DNA
<213> Artificial Sequence

<220>
<223> Nucleotide sequence of  CKCH2CH3CH1-H68-2E12
      (X0168)

<400> 3
actgtggctg caccatctgt cttcatcttc ccgccatctg atgagcagtt gaaatctgga 60
actgcctctg ttgtgtgcct gctgaataac ttctatccca gagaggccaa agtacagtgg 120
aaggtggata acgccctcca atcgggtaac tcccaggaga gtgtcacaga gcaggacagc 180
aaggacagca cctacagcct cagcagcacc ctgacgctga gcaaagcaga ctacgagaaa 240
cacaaagtct acgcctgcga agtcacccat cagggcctga gctcgcccgt cacaaagagc 300
ttcaacaggg gagagcgtac ggagcccaaa tcttctgaca aaactcacac atgcccaccg 360
tgcccatccg gagcacctga actcctgggt ggaccgtcag tcttcctctt ccccccaaaa 420
cccaaggaca ccctcatgat ctcccggacc cctgaggtca catgcgtggt ggtggacgtg 480
agccacgaag accctgaggt caagttcaac tggtacgtgg acggcgtgga ggtgcataat 540
gccaagacaa agccgcggga ggagcagtac aacagcacgt accgtgtggt cagcgtcctc 600

accgtcctgc accaggactg gctgaatggc aaggagtaca agtgcaaggt ctccaacaaa 660
gccctcccag cccccatcga gaaaaccatc tccaaagcca aagggcagcc ccgagaacca 720
caggtgtaca ccctgccccc atcccgggat gagctgacca agaaccaggt cagcctgacc 780
tgcctggtca aaggcttcta tcccagcgac atcgccgtgg agtgggagag caatgggcag 840
ccggagaaca actacaagac cacgcctccc gtgctggact ccgacggctc cttcttcctc 900
tacagcaagc tcaccgtgga caagagcagg tggcagcagg ggaacgtctt ctcatgctcc 960
gtgatgcatg aggctctgca caaccactac acgcagaaga gcctctccct gtctccgggt 1020
aaatctagag cctccaccaa gggcccatcg gtcttccccc tggcaccctc ctccaagagc 1080
acctctgggg gcacagcggc cctgggctgc ctggtcaagg actacttccc cgagccggtg 1140
acggtgtcgt ggaactcagg cgccctgacc agcggcgtgc acaccttccc ggctgtccta 1200
cagtcctcag gactctactc cctcagcagc gtggtgaccg tgccctccag cagcttgggc 1260
acccagacct acatctgcaa cgtgaatcac aagcccagca acaccaaggt ggacaagaaa 1320
gttaactcat tattcaacca agaagttcaa attcccttga ccgaaagtta cagcccgaat 1380
tctcaggtgc agctgaagga gtcaggacct ggcagcgtgg cgccctcaca gagcctgtcc 1440
atcacatgca ccgtctcagg gttctcatta accggctatg gtgtaaactg ggttcgccag 1500
cctccaggaa agggtctgga gtggctggga atgatatggg gtgatggaag cacagactat 1560
aattcagctc tcaaatccag actatcgatc accaaggaca actccaagag ccaagttttc 1620
ttaaaaatga acagtctgca aactgatgac acagccagat actactgtgc tcgggatggt 1680
tatagtaact ttcattacta tgttatggac tactggggtc aaggaacctc agtcaccgtc 1740
tcctctgggg gtggaggctc tggtggcggt ggatccggcg gaggtgggtc gggtggcggc 1800
ggatctgaca ttgtgctcac ccaatctcca gcttctttgg ctgtgtctct aggtcagaga 1860
gccaccatct cctgcagagc cagtgaaagt gttgaatatt atgtcacaag tttaatgcag 1920
tggtaccaac agaaaccagg acagccaccc aaactcctca tctctgctgc tagcaacgta 1980
gaatctgggg tccctgccag gtttagtggc agtgggtctg ggacagactt tagcctcaac 2040
atccatcctg tggaggagga tgatattgca atgtatttct gtcagcaaag taggaaggtt 2100
ccatggacgt tcggtggagg caccaagctg gaaatcaagt ga 2142

<210> 4
<211> 713
<212> PRT
<213> Artificial Sequence

<220>
<223> Amino acid sequence of CKCH2CH3CH1-H68-2E12
(X0168)

<400> 4
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Arg Thr Glu Pro Lys Ser Ser
            100                 105                 110
Asp Lys Thr His Thr Cys Pro Pro Cys Pro Ser Gly Ala Pro Glu Leu
        115                 120                 125
Leu Gly Gly Pro Ser Val Phe Leu Phe Pro Pro Lys Pro Lys Asp Thr
    130                 135                 140
Leu Met Ile Ser Arg Thr Pro Glu Val Thr Cys Val Val Val Asp Val
145                 150                 155                 160
Ser His Glu Asp Pro Glu Val Lys Phe Asn Trp Tyr Val Asp Gly Val
                165                 170                 175
Glu Val His Asn Ala Lys Thr Lys Pro Arg Glu Glu Gln Tyr Asn Ser
            180                 185                 190
Thr Tyr Arg Val Val Ser Val Leu Thr Val Leu His Gln Asp Trp Leu
        195                 200                 205

Asn Gly Lys Glu Tyr Lys Cys Lys Val Ser Asn Lys Ala Leu Pro Ala
    210                 215                 220
Pro Ile Glu Lys Thr Ile Ser Lys Ala Lys Gly Gln Pro Arg Glu Pro
225                 230                 235                 240
Gln Val Tyr Thr Leu Pro Pro Ser Arg Asp Glu Leu Thr Lys Asn Gln
                245                 250                 255
Val Ser Leu Thr Cys Leu Val Lys Gly Phe Tyr Pro Ser Asp Ile Ala
            260                 265                 270
Val Glu Trp Glu Ser Asn Gly Gln Pro Glu Asn Asn Tyr Lys Thr Thr
        275                 280                 285
Pro Pro Val Leu Asp Ser Asp Gly Ser Phe Phe Leu Tyr Ser Lys Leu
    290                 295                 300
Thr Val Asp Lys Ser Arg Trp Gln Gln Gly Asn Val Phe Ser Cys Ser
305                 310                 315                 320
Val Met His Glu Ala Leu His Asn His Tyr Thr Gln Lys Ser Leu Ser
                325                 330                 335
Leu Ser Pro Gly Lys Ser Arg Ala Ser Thr Lys Gly Pro Ser Val Phe
            340                 345                 350
Pro Leu Ala Pro Ser Ser Lys Ser Thr Ser Gly Gly Thr Ala Ala Leu
        355                 360                 365
Gly Cys Leu Val Lys Asp Tyr Phe Pro Glu Pro Val Thr Val Ser Trp
    370                 375                 380
Asn Ser Gly Ala Leu Thr Ser Gly Val His Thr Phe Pro Ala Val Leu
385                 390                 395                 400
Gln Ser Ser Gly Leu Tyr Ser Leu Ser Ser Val Val Thr Val Pro Ser
                405                 410                 415
Ser Ser Leu Gly Thr Gln Thr Tyr Ile Cys Asn Val Asn His Lys Pro
            420                 425                 430
Ser Asn Thr Lys Val Asp Lys Lys Val Asn Ser Leu Phe Asn Gln Glu
        435                 440                 445
Val Gln Ile Pro Leu Thr Glu Ser Tyr Ser Pro Asn Ser Gln Val Gln
    450                 455                 460
Leu Lys Glu Ser Gly Pro Gly Ser Val Ala Pro Ser Gln Ser Leu Ser
465                 470                 475                 480
Ile Thr Cys Thr Val Ser Gly Phe Ser Leu Thr Gly Tyr Gly Val Asn
                485                 490                 495
Trp Val Arg Gln Pro Pro Gly Lys Gly Leu Glu Trp Leu Gly Met Ile
            500                 505                 510
Trp Gly Asp Gly Ser Thr Asp Tyr Asn Ser Ala Leu Lys Ser Arg Leu
        515                 520                 525
Ser Ile Thr Lys Asp Asn Ser Lys Ser Gln Val Phe Leu Lys Met Asn
    530                 535                 540
Ser Leu Gln Thr Asp Asp Thr Ala Arg Tyr Tyr Cys Ala Arg Asp Gly
545                 550                 555                 560
Tyr Ser Asn Phe His Tyr Tyr Val Met Asp Tyr Trp Gly Gln Gly Thr
                565                 570                 575
Ser Val Thr Val Ser Ser Gly Gly Gly Gly Ser Gly Gly Gly Gly Ser
            580                 585                 590
Gly Gly Gly Gly Ser Gly Gly Gly Gly Ser Asp Ile Val Leu Thr Gln
        595                 600                 605
Ser Pro Ala Ser Leu Ala Val Ser Leu Gly Gln Arg Ala Thr Ile Ser
    610                 615                 620
Cys Arg Ala Ser Glu Ser Val Glu Tyr Tyr Val Thr Ser Leu Met Gln
625                 630                 635                 640
Trp Tyr Gln Gln Lys Pro Gly Gln Pro Pro Lys Leu Leu Ile Ser Ala
                645                 650                 655
Ala Ser Asn Val Glu Ser Gly Val Pro Ala Arg Phe Ser Gly Ser Gly
            660                 665                 670
Ser Gly Thr Asp Phe Ser Leu Asn Ile His Pro Val Glu Glu Asp Asp
        675                 680                 685
Ile Ala Met Tyr Phe Cys Gln Gln Ser Arg Lys Val Pro Trp Thr Phe
    690                 695                 700
Gly Gly Gly Thr Lys Leu Glu Ile Lys

705                 710

<210> 5
<211> 1770
<212> DNA
<213> Artificial Sequence

<220>
<223> Nucleotide sequence of 2E12CH1CH2CH3 (X0112)

<400> 5
caggtgcagc tgaaggagtc aggacctggc agcgtggcgc cctcacagag cctgtccatc 60
acatgcaccg tctcagggtt ctcattaacc ggctatggtg taaactgggt tcgccagcct 120
ccaggaaagg gtctggagtg gctgggaatg atatggggtg atggaagcac agactataat 180
tcagctctca aatccagact atcgatcacc aaggacaact ccaagagcca agttttctta 240
aaaatgaaca gtctgcaaac tgatgacaca gccagatact actgtgctcg ggatggttat 300
agtaactttc attactatgt tatggactac tggggtcaag gaacctcagt caccgtctcc 360
tctgggggtg gaggctctgg tggcggtgga tccggcggag gtgggtcggg tggcggcgga 420
tctgacattg tgctcaccca atctccagct tctttggctg tgtctctagg tcagagagcc 480
accatctcct gcagagccag tgaaagtgtt gaatattatg tcacaagttt aatgcagtgg 540
taccaacaga aaccaggaca gccacccaaa ctcctcatct ctgctgctag caacgtagaa 600
tctggggtcc ctgccaggtt tagtggcagt gggtctggga cagactttag cctcaacatc 660
catcctgtgg aggaggatga tattgcaatg tatttctgtc agcaaagtag gaaggttcca 720
tggacgttcg gtggaggcac caagctggaa atcaagtcct cgagcgcctc caccaagggc 780
ccatcggtct tccccctggc accctcctcc aagagcacct ctgggggcac agcggccctg 840
ggctgcctgg tcaaggacta cttccccgag ccggtgacgg tgtcgtggaa ctcaggcgcc 900
ctgaccagcg gcgtgcacac cttcccggct gtcctacagt cctcaggact ctactccctc 960
agcagcgtgg tgaccgtgcc ctccagcagc ttgggcaccc agacctacat ctgcaacgtg 1020
aatcacaagc ccagcaacac caaggtggac aagaaagttc gtacggagcc caaatcttct 1080
gacaaaactc acacatgccc accgtgccca tccggagcac ctgaactcct gggtggaccg 1140
tcagtcttcc tcttcccccc aaaacccaag gacaccctca tgatctcccg gacccctgag 1200
gtcacatgcg tggtggtgga cgtgagccac gaagaccctg aggtcaagtt caactggtac 1260
gtggacggcg tggaggtgca taatgccaag acaaagccgc gggaggagca gtacaacagc 1320
acgtaccgtg tggtcagcgt cctcaccgtc ctgcaccagg actggctgaa tggcaaggag 1380
tacaagtgca aggtctccaa caaagccctc ccagccccca tcgagaaaac catctccaaa 1440
gccaaagggc agccccgaga accacaggtg tacaccctgc ccccatcccg ggatgagctg 1500
accaagaacc aggtcagcct gacctgcctg gtcaaaggct tctatccaag cgacatcgcc 1560
gtggagtggg agagcaatgg gcagccggag aacaactaca agaccacgcc tcccgtgctg 1620
gactccgacg gctccttctt cctctacagc aagctcaccg tggacaagag caggtggcag 1680
caggggaacg tcttctcatg ctccgtgatg catgaggctc tgcacaacca ctacacgcag 1740
aagagcctct ccctgtctcc gggtaaatga                                  1770

<210> 6
<211> 589
<212> PRT
<213> Artificial Sequence

<220>
<223> Amino acid sequence of 2E12CH1CH2CH3 (X0112)

<400> 6
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala

                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser Gly Gly Gly Gly Ser Gly Gly
        115                 120                 125
Gly Gly Ser Gly Gly Gly Gly Ser Gly Gly Gly Gly Ser Asp Ile Val
    130                 135                 140
Leu Thr Gln Ser Pro Ala Ser Leu Ala Val Ser Leu Gly Gln Arg Ala
145                 150                 155                 160
Thr Ile Ser Cys Arg Ala Ser Glu Ser Val Glu Tyr Tyr Val Thr Ser
                165                 170                 175
Leu Met Gln Trp Tyr Gln Gln Lys Pro Gly Gln Pro Pro Lys Leu Leu
            180                 185                 190
Ile Ser Ala Ala Ser Asn Val Glu Ser Gly Val Pro Ala Arg Phe Ser
        195                 200                 205
Gly Ser Gly Ser Gly Thr Asp Phe Ser Leu Asn Ile His Pro Val Glu
    210                 215                 220
Glu Asp Asp Ile Ala Met Tyr Phe Cys Gln Gln Ser Arg Lys Val Pro
225                 230                 235                 240
Trp Thr Phe Gly Gly Gly Thr Lys Leu Glu Ile Lys Ser Ser Ser Ala
                245                 250                 255
Ser Thr Lys Gly Pro Ser Val Phe Pro Leu Ala Pro Ser Ser Lys Ser
            260                 265                 270
Thr Ser Gly Gly Thr Ala Ala Leu Gly Cys Leu Val Lys Asp Tyr Phe
        275                 280                 285
Pro Glu Pro Val Thr Val Ser Trp Asn Ser Gly Ala Leu Thr Ser Gly
    290                 295                 300
Val His Thr Phe Pro Ala Val Leu Gln Ser Ser Gly Leu Tyr Ser Leu
305                 310                 315                 320
Ser Ser Val Val Thr Val Pro Ser Ser Ser Leu Gly Thr Gln Thr Tyr
                325                 330                 335
Ile Cys Asn Val Asn His Lys Pro Ser Asn Thr Lys Val Asp Lys Lys
            340                 345                 350
Val Arg Thr Glu Pro Lys Ser Ser Asp Lys Thr His Thr Cys Pro Pro
        355                 360                 365
Cys Pro Ser Gly Ala Pro Glu Leu Leu Gly Gly Pro Ser Val Phe Leu
    370                 375                 380
Phe Pro Pro Lys Pro Lys Asp Thr Leu Met Ile Ser Arg Thr Pro Glu
385                 390                 395                 400
Val Thr Cys Val Val Val Asp Val Ser His Glu Asp Pro Glu Val Lys
                405                 410                 415
Phe Asn Trp Tyr Val Asp Gly Val Glu Val His Asn Ala Lys Thr Lys
            420                 425                 430
Pro Arg Glu Glu Gln Tyr Asn Ser Thr Tyr Arg Val Val Ser Val Leu
        435                 440                 445
Thr Val Leu His Gln Asp Trp Leu Asn Gly Lys Glu Tyr Lys Cys Lys
    450                 455                 460
Val Ser Asn Lys Ala Leu Pro Ala Pro Ile Glu Lys Thr Ile Ser Lys
465                 470                 475                 480
Ala Lys Gly Gln Pro Arg Glu Pro Gln Val Tyr Thr Leu Pro Pro Ser
                485                 490                 495
Arg Asp Glu Leu Thr Lys Asn Gln Val Ser Leu Thr Cys Leu Val Lys
            500                 505                 510
Gly Phe Tyr Pro Ser Asp Ile Ala Val Glu Trp Glu Ser Asn Gly Gln
        515                 520                 525
Pro Glu Asn Asn Tyr Lys Thr Thr Pro Pro Val Leu Asp Ser Asp Gly
    530                 535                 540
Ser Phe Phe Leu Tyr Ser Lys Leu Thr Val Asp Lys Ser Arg Trp Gln
545                 550                 555                 560
Gln Gly Asn Val Phe Ser Cys Ser Val Met His Glu Ala Leu His Asn
                565                 570                 575
His Tyr Thr Gln Lys Ser Leu Ser Leu Ser Pro Gly Lys
            580                 585

<210> 7
<211> 1026
<212> DNA
<213> Artificial Sequence

<220>
<223> Nucleotide sequence of CkCH2CH3 (X0113)

<400> 7
actgtggctg caccatctgt cttcatcttc ccgccatctg atgagcagtt gaaatctgga 60
actgcctctg ttgtgtgcct gctgaataac ttctatccca gagaggccaa agtacagtgg 120
aaggtggata acgccctcca atcgggtaac tcccaggaga gtgtcacaga gcaggacagc 180
aaggacagca cctacagcct cagcagcacc ctgacgctga gcaaagcaga ctacgagaaa 240
cacaaagtct acgcctgcga agtcacccat cagggcctga gctcgcccgt cacaaagagc 300
ttcaacaggg gagagcgtac ggagcccaaa tcttctgaca aaactcacac atgcccaccg 360
tgcccatccg gagcacctga actcctgggt ggaccgtcag tcttcctctt ccccccaaaa 420
cccaaggaca ccctcatgat ctcccggacc cctgaggtca catgcgtggt ggtggacgtg 480
agccacgaag accctgaggt caagttcaac tggtacgtgg acggcgtgga ggtgcataat 540
gccaagacaa agccgcggga ggagcagtac aacagcacgt accgtgtggt cagcgtcctc 600
accgtcctgc accaggactg gctgaatggc aaggagtaca agtgcaaggt ctccaacaaa 660
gccctcccag cccccatcga gaaaaccatc tccaaagcca aagggcagcc ccgagaacca 720
caggtgtaca ccctgccccc atcccgggat gagctgacca agaaccaggt cagcctgacc 780
tgcctggtca aaggcttcta tccaagcgac atcgccgtgg agtgggagag caatgggcag 840
ccggagaaca actacaagac cacgcctccc gtgctggact ccgacggctc cttcttcctc 900
tacagcaagc tcaccgtgga caagagcagg tggcagcagg ggaacgtctt ctcatgctcc 960
gtgatgcatg aggctctgca caaccactac acgcagaaga gcctctccct gtctccgggt 1020
aaatga 1026

<210> 8
<211> 341
<212> PRT
<213> Artificial Sequence

<220>
<223> Amino acid sequence of CkCH2CH3 (X0113)

<400> 8
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Arg Thr Glu Pro Lys Ser Ser
            100                 105                 110
Asp Lys Thr His Thr Cys Pro Pro Cys Pro Ser Gly Ala Pro Glu Leu
        115                 120                 125
Leu Gly Gly Pro Ser Val Phe Leu Phe Pro Pro Lys Pro Lys Asp Thr
    130                 135                 140
Leu Met Ile Ser Arg Thr Pro Glu Val Thr Cys Val Val Val Asp Val
145                 150                 155                 160
Ser His Glu Asp Pro Glu Val Lys Phe Asn Trp Tyr Val Asp Gly Val
                165                 170                 175
Glu Val His Asn Ala Lys Thr Lys Pro Arg Glu Glu Gln Tyr Asn Ser
            180                 185                 190

Thr Tyr Arg Val Val Ser Val Leu Thr Val Leu His Gln Asp Trp Leu
        195                 200                 205
Asn Gly Lys Glu Tyr Lys Cys Lys Val Ser Asn Lys Ala Leu Pro Ala
    210                 215                 220
Pro Ile Glu Lys Thr Ile Ser Lys Ala Lys Gly Gln Pro Arg Glu Pro
225                 230                 235                 240
Gln Val Tyr Thr Leu Pro Pro Ser Arg Asp Glu Leu Thr Lys Asn Gln
                245                 250                 255
Val Ser Leu Thr Cys Leu Val Lys Gly Phe Tyr Pro Ser Asp Ile Ala
            260                 265                 270
Val Glu Trp Glu Ser Asn Gly Gln Pro Glu Asn Asn Tyr Lys Thr Thr
        275                 280                 285
Pro Pro Val Leu Asp Ser Asp Gly Ser Phe Phe Leu Tyr Ser Lys Leu
    290                 295                 300
Thr Val Asp Lys Ser Arg Trp Gln Gln Gly Asn Val Phe Ser Cys Ser
305                 310                 315                 320
Val Met His Glu Ala Leu His Asn His Tyr Thr Gln Lys Ser Leu Ser
                325                 330                 335
Leu Ser Pro Gly Lys
            340

<210> 9
<211> 1839
<212> DNA
<213> Artificial Sequence

<220>
<223> Nucleotide sequence of Ck(YAE) Fc (N297A) 2E12
      (X0244)

<400> 9
actgtggctg caccatctgt cttcatcttc ccgccatctg atgagcagtt gaaatctgga 60
actgcctctg ttgtgtgcct gctgaattac ttctatccca gagaggccaa agtacagtgg 120
aaggtggata acgccctcca atcgggtaac tcccaggaga gtgccacaga gcaggacagc 180
aaggacagca cctacagcct cagcagcgag ctgacgctga gcaaagcaga ctacgagaaa 240
cacaaagtct acgcctgcga agtcacccat cagggcctga gctcgcccgt cacaaagagc 300
ttcaacaggg gagagcgtac ggagcccaaa tcttctgaca aaactcacac atgcccaccg 360
tgcccatccg gagcacctga actcctgggt ggaccgtcag tcttcctctt ccccccaaaa 420
cccaaggaca ccctcatgat ctcccggacc cctgaggtca catgcgtggt ggtggacgtg 480
agccacgaag accctgaggt caagttcaac tggtacgtgg acggcgtgga ggtgcataat 540
gccaagacaa agccgcggga ggagcagtac gccagcacgt accgtgtggt cagcgtcctc 600
accgtcctgc accaggactg gctgaatggc aaggagtaca agtgcaaggt ctccaacaaa 660
gccctcccag cccccatcga gaaaaccatc tccaaagcca aagggcagcc ccgagaacca 720
caggtgtaca ccctgccccc atcccgggat gagctgacca agaaccaggt cagcctgacc 780
tgcctggtca aaggcttcta tccaagcgac atcgccgtgg agtgggagag caatgggcag 840
ccggagaaca actacaagac cacgcctccc gtgctggact ccgacggctc cttcttcctc 900
tacagcaagc tcaccgtgga caagagcagg tggcagcagg ggaacgtctt ctcatgctcc 960
gtgatgcatg aggctctgca caaccactac acgcagaaga gcctctccct gtctccgggt 1020
aactcattat tcaaccaaga agttcaaatt cccttgaccg aaagttacag cccgaattct 1080
caggtgcagc tgaaggagtc aggacctggc agcgtggcgc cctcacagag cctgtccatc 1140
acatgcaccg tctcagggtt ctcattaacc ggctatggtg taaactgggt tcgccagcct 1200
ccaggaaagg gtctggagtg gctgggaatg atatggggtg atggaagcac agactataat 1260
tcagctctca aatccagact atcgatcacc aaggacaact ccaagagcca agttttctta 1320
aaaatgaaca gtctgcaaac tgatgacaca gccagatact actgtgctcg ggatggttat 1380
agtaactttc attactatgt tatggactac tggggtcaag gaacctcagt caccgtctcc 1440
tctgggggtg gaggctctgg tggcggtgga tccggcggag gtgggtcggg tggcggcgga 1500
tctgacattg tgctcaccca atctccagct tctttggctg tgtctctagg tcagagagcc 1560
accatctcct gcagagccag tgaaagtgtt gaatattatg tcacaagttt aatgcagtgg 1620
taccaacaga aaccaggaca gccacccaaa ctcctcatct ctgctgctag caacgtagaa 1680
tctggggtcc ctgccaggtt tagtggcagt gggtctggga cagactttag cctcaacatc 1740
catcctgtgg aggaggatga tattgcaatg tatttctgtc agcaaagtag gaaggttcca 1800
tggacgttcg gtggaggcac caagctggaa atcaagtga                        1839

<210> 10
<211> 612
<212> PRT
<213> Artificial Sequence

<220>
<223> Amino acid sequence of Ck(YAE) Fc (N297A) 2E12
      (X0244)

<400> 10
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Tyr Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Arg Thr Glu Pro Lys Ser Ser
            100                 105                 110
Asp Lys Thr His Thr Cys Pro Pro Cys Pro Ser Gly Ala Pro Glu Leu
        115                 120                 125
Leu Gly Gly Pro Ser Val Phe Leu Phe Pro Pro Lys Pro Lys Asp Thr
    130                 135                 140
Leu Met Ile Ser Arg Thr Pro Glu Val Thr Cys Val Val Val Asp Val
145                 150                 155                 160
Ser His Glu Asp Pro Glu Val Lys Phe Asn Trp Tyr Val Asp Gly Val
                165                 170                 175
Glu Val His Asn Ala Lys Thr Lys Pro Arg Glu Glu Gln Tyr Ala Ser
            180                 185                 190
Thr Tyr Arg Val Val Ser Val Leu Thr Val Leu His Gln Asp Trp Leu
        195                 200                 205
Asn Gly Lys Glu Tyr Lys Cys Lys Val Ser Asn Lys Ala Leu Pro Ala
    210                 215                 220
Pro Ile Glu Lys Thr Ile Ser Lys Ala Lys Gly Gln Pro Arg Glu Pro
225                 230                 235                 240
Gln Val Tyr Thr Leu Pro Pro Ser Arg Asp Glu Leu Thr Lys Asn Gln
                245                 250                 255
Val Ser Leu Thr Cys Leu Val Lys Gly Phe Tyr Pro Ser Asp Ile Ala
            260                 265                 270
Val Glu Trp Glu Ser Asn Gly Gln Pro Glu Asn Asn Tyr Lys Thr Thr
        275                 280                 285
Pro Pro Val Leu Asp Ser Asp Gly Ser Phe Phe Leu Tyr Ser Lys Leu
    290                 295                 300
Thr Val Asp Lys Ser Arg Trp Gln Gln Gly Asn Val Phe Ser Cys Ser
305                 310                 315                 320
Val Met His Glu Ala Leu His Asn His Tyr Thr Gln Lys Ser Leu Ser
                325                 330                 335
Leu Ser Pro Gly Asn Ser Leu Phe Asn Gln Glu Val Gln Ile Pro Leu
            340                 345                 350
Thr Glu Ser Tyr Ser Pro Asn Ser Gln Val Gln Leu Lys Glu Ser Gly
        355                 360                 365
Pro Gly Ser Val Ala Pro Ser Gln Ser Leu Ser Ile Thr Cys Thr Val
    370                 375                 380
Ser Gly Phe Ser Leu Thr Gly Tyr Gly Val Asn Trp Val Arg Gln Pro
385                 390                 395                 400
Pro Gly Lys Gly Leu Glu Trp Leu Gly Met Ile Trp Gly Asp Gly Ser
                405                 410                 415

Thr Asp Tyr Asn Ser Ala Leu Lys Ser Arg Leu Ser Ile Thr Lys Asp
            420                 425                 430
Asn Ser Lys Ser Gln Val Phe Leu Lys Met Asn Ser Leu Gln Thr Asp
        435                 440                 445
Asp Thr Ala Arg Tyr Tyr Cys Ala Arg Asp Gly Tyr Ser Asn Phe His
    450                 455                 460
Tyr Tyr Val Met Asp Tyr Trp Gly Gln Gly Thr Ser Val Thr Val Ser
465                 470                 475                 480
Ser Gly Gly Gly Gly Ser Gly Gly Gly Gly Ser Gly Gly Gly Gly Ser
                485                 490                 495
Gly Gly Gly Gly Ser Asp Ile Val Leu Thr Gln Ser Pro Ala Ser Leu
            500                 505                 510
Ala Val Ser Leu Gly Gln Arg Ala Thr Ile Ser Cys Arg Ala Ser Glu
        515                 520                 525
Ser Val Glu Tyr Tyr Val Thr Ser Leu Met Gln Trp Tyr Gln Gln Lys
    530                 535                 540
Pro Gly Gln Pro Pro Lys Leu Leu Ile Ser Ala Ala Ser Asn Val Glu
545                 550                 555                 560
Ser Gly Val Pro Ala Arg Phe Ser Gly Ser Gly Ser Gly Thr Asp Phe
                565                 570                 575
Ser Leu Asn Ile His Pro Val Glu Glu Asp Asp Ile Ala Met Tyr Phe
            580                 585                 590
Cys Gln Gln Ser Arg Lys Val Pro Trp Thr Phe Gly Gly Gly Thr Lys
        595                 600                 605
Leu Glu Ile Lys
    610


<210> 11
<211> 1770
<212> DNA
<213> Artificial Sequence

<220>
<223> Nucleotide sequence of 2E12 CH1 Fc (N297A)
      (X0245)

<400> 11
caggtgcagc tgaaggagtc aggacctggc agcgtggcgc cctcacagag cctgtccatc 60
acatgcaccg tctcagggtt ctcattaacc ggctatggtg taaactgggt tcgccagcct 120
ccaggaaagg gtctggagtg gctgggaatg atatggggtg atggaagcac agactataat 180
tcagctctca aatccagact atcgatcacc aaggacaact ccaagagcca agttttctta 240
aaaatgaaca gtctgcaaac tgatgacaca gccagatact actgtgctcg ggatggttat 300
agtaactttc attactatgt tatggactac tggggtcaag gaacctcagt caccgtctcc 360
tctgggggtg gaggctctgg tggcggtgga tccggcggag gtgggtcggg tggcggcgga 420
tctgacattg tgctcaccca atctccagct tctttggctg tgtctctagg tcagagagcc 480
accatctcct gcagagccag tgaaagtgtt gaatattatg tcacaagttt aatgcagtgg 540
taccaacaga aaccaggaca gccacccaaa ctcctcatct ctgctgctag caacgtagaa 600
tctggggtcc ctgccaggtt tagtggcagt gggtctggga cagactttag cctcaacatc 660
catcctgtgg aggaggatga tattgcaatg tatttctgtc agcaaagtag gaaggttcca 720
tggacgttcg gtggaggcac caagctggaa atcaagtcct cgagcgcctc caccaagggc 780
ccatcggtct tccccctggc accctcctcc aagagcacct ctgggggcac agcggccctg 840
ggctgcctgg tcaaggacta cttccccgag ccggtgacgg tgtcgtggaa ctcaggcgcc 900
ctgaccagcg gcgtgcacac cttcccggct gtcctacagt cctcaggact ctactccctc 960
agcagcgtgg tgaccgtgcc ctccagcagc ttgggcaccc agacctacat ctgcaacgtg 1020
aatcacaagc ccagcaacac caaggtggac aagaaagttc gtacggagcc caaatcttct 1080
gacaaaactc acacatgccc accgtgccca tccggagcac ctgaactcct gggtggaccg 1140
tcagtcttcc tcttcccccc aaaacccaag gacaccctca tgatctcccg gacccctgag 1200
gtcacatgcg tggtggtgga cgtgagccac gaagaccctg aggtcaagtt caactggtac 1260
gtggacggcg tggaggtgca taatgccaag acaaagccgc gggaggagca gtacgccagc 1320
acgtaccgtg tggtcagcgt cctcaccgtc ctgcaccagg actggctgaa tggcaaggag 1380
tacaagtgca aggtctccaa caaagccctc ccagccccca tcgagaaaac catctccaaa 1440
gccaaagggc agccccgaga accacaggtg tacaccctgc ccccatcccg ggatgagctg 1500

accaagaacc aggtcagcct gacctgcctg gtcaaaggct tctatccaag cgacatcgcc 1560
gtggagtggg agagcaatgg gcagccggag aacaactaca agaccacgcc tcccgtgctg 1620
gactccgacg gctccttctt cctctacagc aagctcaccg tggacaagag caggtggcag 1680
caggggaacg tcttctcatg ctccgtgatg catgaggctc tgcacaacca ctacacgcag 1740
aagagcctct ccctgtctcc gggtaaatga 1770

<210> 12
<211> 589
<212> PRT
<213> Artificial Sequence

<220>
<223> Amino acid sequence of 2E12 CH1 Fc (N297A)
      (X0245)

<400> 12
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala
                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser Gly Gly Gly Gly Ser Gly Gly
        115                 120                 125
Gly Gly Ser Gly Gly Gly Gly Ser Gly Gly Gly Gly Ser Asp Ile Val
    130                 135                 140
Leu Thr Gln Ser Pro Ala Ser Leu Ala Val Ser Leu Gly Gln Arg Ala
145                 150                 155                 160
Thr Ile Ser Cys Arg Ala Ser Glu Ser Val Glu Tyr Tyr Val Thr Ser
                165                 170                 175
Leu Met Gln Trp Tyr Gln Gln Lys Pro Gly Gln Pro Pro Lys Leu Leu
            180                 185                 190
Ile Ser Ala Ala Ser Asn Val Glu Ser Gly Val Pro Ala Arg Phe Ser
        195                 200                 205
Gly Ser Gly Ser Gly Thr Asp Phe Ser Leu Asn Ile His Pro Val Glu
    210                 215                 220
Glu Asp Asp Ile Ala Met Tyr Phe Cys Gln Gln Ser Arg Lys Val Pro
225                 230                 235                 240
Trp Thr Phe Gly Gly Gly Thr Lys Leu Glu Ile Lys Ser Ser Ser Ala
                245                 250                 255
Ser Thr Lys Gly Pro Ser Val Phe Pro Leu Ala Pro Ser Ser Lys Ser
            260                 265                 270
Thr Ser Gly Gly Thr Ala Ala Leu Gly Cys Leu Val Lys Asp Tyr Phe
        275                 280                 285
Pro Glu Pro Val Thr Val Ser Trp Asn Ser Gly Ala Leu Thr Ser Gly
    290                 295                 300
Val His Thr Phe Pro Ala Val Leu Gln Ser Ser Gly Leu Tyr Ser Leu
305                 310                 315                 320
Ser Ser Val Val Thr Val Pro Ser Ser Ser Leu Gly Thr Gln Thr Tyr
                325                 330                 335
Ile Cys Asn Val Asn His Lys Pro Ser Asn Thr Lys Val Asp Lys Lys
            340                 345                 350
Val Arg Thr Glu Pro Lys Ser Ser Asp Lys Thr His Thr Cys Pro Pro
        355                 360                 365
Cys Pro Ser Gly Ala Pro Glu Leu Leu Gly Gly Pro Ser Val Phe Leu

370                 375                 380
Phe Pro Pro Lys Pro Lys Asp Thr Leu Met Ile Ser Arg Thr Pro Glu
385                 390                 395                 400
Val Thr Cys Val Val Val Asp Val Ser His Glu Asp Pro Glu Val Lys
                405                 410                 415
Phe Asn Trp Tyr Val Asp Gly Val Glu Val His Asn Ala Lys Thr Lys
            420                 425                 430
Pro Arg Glu Glu Gln Tyr Ala Ser Thr Tyr Arg Val Val Ser Val Leu
        435                 440                 445
Thr Val Leu His Gln Asp Trp Leu Asn Gly Lys Glu Tyr Lys Cys Lys
    450                 455                 460
Val Ser Asn Lys Ala Leu Pro Ala Pro Ile Glu Lys Thr Ile Ser Lys
465                 470                 475                 480
Ala Lys Gly Gln Pro Arg Glu Pro Gln Val Tyr Thr Leu Pro Pro Ser
                485                 490                 495
Arg Asp Glu Leu Thr Lys Asn Gln Val Ser Leu Thr Cys Leu Val Lys
            500                 505                 510
Gly Phe Tyr Pro Ser Asp Ile Ala Val Glu Trp Glu Ser Asn Gly Gln
        515                 520                 525
Pro Glu Asn Asn Tyr Lys Thr Thr Pro Pro Val Leu Asp Ser Asp Gly
    530                 535                 540
Ser Phe Phe Leu Tyr Ser Lys Leu Thr Val Asp Lys Ser Arg Trp Gln
545                 550                 555                 560
Gln Gly Asn Val Phe Ser Cys Ser Val Met His Glu Ala Leu His Asn
                565                 570                 575
His Tyr Thr Gln Lys Ser Leu Ser Leu Ser Pro Gly Lys
            580                 585

<210> 13
<211> 1749
<212> DNA
<213> Artificial Sequence

<220>
<223> Nucleotide sequence of P2C2 Ck(YAE) Fc (N297A)
      (X0246)

<400> 13
gatattgtga tgatccagga tgaactctcc aatcctgtca gttctggaga atcagtttcc 60
atctcctgta ggtctagtaa gagtctccta gataaggaag ggaagacata cttgaattgg 120
tttctgcaga gaccaggaca atctcctcag ctcctgatct atctgatgtc catgcgtgaa 180
tcaggagtct cagaccggtt tagtggcagt gggtcaggaa cagatttcac cctggaaatc 240
agtagagtga aggctgagga tgtgggtgtg tattactgtc aacaacttgt agagtatccg 300
tggacgttcg gtggaggcac caagctggaa atcaaaggtg gcggtggctc tggcggaggt 360
ggatccggtg gcggcggctc tcagatccag ttggtgcagt ctggacctga gctgaagaag 420
cctggagaga cagtcaagat ctcctgcaag gcttctggtt ataccttcac agacttttca 480
atgcactggg tgaggcaggc tccaggaaag ggtttaaagt ggatgggctg gataaacact 540
gagactggtg agccaacata tgcagatgac ttcaggggac ggtttgcctt ctctttggaa 600
acttctgcca gcactgccta tttgcagatc aacaacctca aaaatgagga cacggctaca 660
tatttttgta cctggtctgc ttactggggc caagggactc tggtcactgt ctctgcctcg 720
agcactgtgg ctgcaccatc tgtcttcatc ttcccgccat ctgatgagca gttgaaatct 780
ggaactgcct ctgttgtgtg cctgctgaat tacttctatc ccagagaggc caaagtacag 840
tggaaggtgg ataacgccct ccaatcgggt aactcccagg agagtgccac agagcaggac 900
agcaaggaca gcacctacag cctcagcagc gagctgacgc tgagcaaagc agactacgag 960
aaacacaaag tctacgcctg cgaagtcacc catcagggcc tgagctcgcc cgtcacaaag 1020
agcttcaaca ggggagagcg tacggagccc aaatcttctg acaaaactca cacatgccca 1080
ccgtgcccat ccggagcacc tgaactcctg ggtggaccgt cagtcttcct cttcccccca 1140
aaacccaagg acaccctcat gatctcccgg acccctgagg tcacatgcgt ggtggtggac 1200
gtgagccacg aagaccctga ggtcaagttc aactggtacg tggacggcgt ggaggtgcat 1260
aatgccaaga caaagccgcg ggaggagcag tacgccagca cgtaccgtgt ggtcagcgtc 1320
ctcaccgtcc tgcaccagga ctggctgaat ggcaaggagt acaagtgcaa ggtctccaac 1380
aaagccctcc cagcccccat cgagaaaacc atctccaaag ccaaagggca gccccgagaa 1440

ccacaggtgt acaccctgcc cccatcccgg gatgagctga ccaagaacca ggtcagcctg 1500
acctgcctgg tcaaaggctt ctatccaagc gacatcgccg tggagtggga gagcaatggg 1560
cagccggaga acaactacaa gaccacgcct cccgtgctgg actccgacgg ctccttcttc 1620
ctctacagca agctcaccgt ggacaagagc aggtggcagc aggggaacgt cttctcatgc 1680
tccgtgatgc atgaggctct gcacaaccac tacacgcaga agagcctctc cctgtctccg 1740
ggtaaatga 1749

<210> 14
<211> 582
<212> PRT
<213> Artificial Sequence

<220>
<223> amino acid sequence of P2C2 Ck(YAE) Fc (N297A)
(X0246)

<400> 14
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser
    50                  55                  60
Asp Arg Phe Ser Gly Ser Gly Ser Gly Thr Asp Phe Thr Leu Glu Ile
65                  70                  75                  80
Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110
Gly Gly Gly Gly Ser Gly Gly Gly Gly Ser Gly Gly Gly Gly Ser Gln
        115                 120                 125
Ile Gln Leu Val Gln Ser Gly Pro Glu Leu Lys Lys Pro Gly Glu Thr
    130                 135                 140
Val Lys Ile Ser Cys Lys Ala Ser Gly Tyr Thr Phe Thr Asp Phe Ser
145                 150                 155                 160
Met His Trp Val Arg Gln Ala Pro Gly Lys Gly Leu Lys Trp Met Gly
                165                 170                 175
Trp Ile Asn Thr Glu Thr Gly Glu Pro Thr Tyr Ala Asp Asp Phe Arg
            180                 185                 190
Gly Arg Phe Ala Phe Ser Leu Glu Thr Ser Ala Ser Thr Ala Tyr Leu
        195                 200                 205
Gln Ile Asn Asn Leu Lys Asn Glu Asp Thr Ala Thr Tyr Phe Cys Thr
    210                 215                 220
Trp Ser Ala Tyr Trp Gly Gln Gly Thr Leu Val Thr Val Ser Ala Ser
225                 230                 235                 240
Ser Thr Val Ala Ala Pro Ser Val Phe Ile Phe Pro Pro Ser Asp Glu
                245                 250                 255
Gln Leu Lys Ser Gly Thr Ala Ser Val Val Cys Leu Leu Asn Tyr Phe
            260                 265                 270
Tyr Pro Arg Glu Ala Lys Val Gln Trp Lys Val Asp Asn Ala Leu Gln
        275                 280                 285
Ser Gly Asn Ser Gln Glu Ser Ala Thr Glu Gln Asp Ser Lys Asp Ser
    290                 295                 300
Thr Tyr Ser Leu Ser Ser Glu Leu Thr Leu Ser Lys Ala Asp Tyr Glu
305                 310                 315                 320
Lys His Lys Val Tyr Ala Cys Glu Val Thr His Gln Gly Leu Ser Ser
                325                 330                 335
Pro Val Thr Lys Ser Phe Asn Arg Gly Glu Arg Thr Glu Pro Lys Ser
            340                 345                 350
Ser Asp Lys Thr His Thr Cys Pro Pro Cys Pro Ser Gly Ala Pro Glu
        355                 360                 365

Leu Leu Gly Gly Pro Ser Val Phe Leu Phe Pro Pro Lys Pro Lys Asp
    370                 375                 380
Thr Leu Met Ile Ser Arg Thr Pro Glu Val Thr Cys Val Val Val Asp
385                 390                 395                 400
Val Ser His Glu Asp Pro Glu Val Lys Phe Asn Trp Tyr Val Asp Gly
                405                 410                 415
Val Glu Val His Asn Ala Lys Thr Lys Pro Arg Glu Glu Gln Tyr Ala
            420                 425                 430
Ser Thr Tyr Arg Val Val Ser Val Leu Thr Val Leu His Gln Asp Trp
        435                 440                 445
Leu Asn Gly Lys Glu Tyr Lys Cys Lys Val Ser Asn Lys Ala Leu Pro
    450                 455                 460
Ala Pro Ile Glu Lys Thr Ile Ser Lys Ala Lys Gly Gln Pro Arg Glu
465                 470                 475                 480
Pro Gln Val Tyr Thr Leu Pro Pro Ser Arg Asp Glu Leu Thr Lys Asn
                485                 490                 495
Gln Val Ser Leu Thr Cys Leu Val Lys Gly Phe Tyr Pro Ser Asp Ile
            500                 505                 510
Ala Val Glu Trp Glu Ser Asn Gly Gln Pro Glu Asn Asn Tyr Lys Thr
        515                 520                 525
Thr Pro Pro Val Leu Asp Ser Asp Gly Ser Phe Phe Leu Tyr Ser Lys
    530                 535                 540
Leu Thr Val Asp Lys Ser Arg Trp Gln Gln Gly Asn Val Phe Ser Cys
545                 550                 555                 560
Ser Val Met His Glu Ala Leu His Asn His Tyr Thr Gln Lys Ser Leu
                565                 570                 575
Ser Leu Ser Pro Gly Lys
            580


<210> 15
<211> 2583
<212> DNA
<213> Artificial Sequence

<220>
<223> Nucleotide sequence of 2E12 CH1 Fc (N297A) H68
      2E12 (X0247)

<400> 15
caggtgcagc tgaaggagtc aggacctggc agcgtggcgc cctcacagag cctgtccatc 60
acatgcaccg tctcagggtt ctcattaacc ggctatggtg taaactgggt tcgccagcct 120
ccaggaaagg gtctggagtg gctgggaatg atatggggtg atggaagcac agactataat 180
tcagctctca aatccagact atcgatcacc aaggacaact ccaagagcca agttttctta 240
aaaatgaaca gtctgcaaac tgatgacaca gccagatact actgtgctcg ggatggttat 300
agtaactttc attactatgt tatggactac tggggtcaag gaacctcagt caccgtctcc 360
tctgggggtg gaggctctgg tggcggtgga tccggcggag gtgggtcggg tggcggcgga 420
tctgacattg tgctcaccca atctccagct tctttggctg tgtctctagg tcagagagcc 480
accatctcct gcagagccag tgaaagtgtt gaatattatg tcacaagttt aatgcagtgg 540
taccaacaga aaccaggaca gccacccaaa ctcctcatct ctgctgctag caacgtagaa 600
tctggggtcc ctgccaggtt tagtggcagt gggtctggga cagactttag cctcaacatc 660
catcctgtgg aggaggatga tattgcaatg tatttctgtc agcaaagtag gaaggttcca 720
tggacgttcg gtggaggcac caagctggaa atcaagtcct cgagcgcctc caccaagggc 780
ccatcggtct tccccctggc accctcctcc aagagcacct ctgggggcac agcggccctg 840
ggctgcctgg tcaaggacta cttccccgag ccggtgacgg tgtcgtggaa ctcaggcgcc 900
ctgaccagcg gcgtgcacac cttcccggct gtcctacagt cctcaggact ctactccctc 960
agcagcgtgg tgaccgtgcc ctccagcagc ttgggcaccc agacctacat ctgcaacgtg 1020
aatcacaagc ccagcaacac caaggtggac aagaaagttc gtacggagcc caaatcttct 1080
gacaaaactc acacatgccc accgtgccca tccggagcac ctgaactcct gggtggaccg 1140
tcagtcttcc tcttcccccc aaaacccaag gacaccctca tgatctcccg gacccctgag 1200
gtcacatgcg tggtggtgga cgtgagccac gaagaccctg aggtcaagtt caactggtac 1260
gtggacggcg tggaggtgca taatgccaag acaaagccgc gggaggagca gtacgccagc 1320
acgtaccgtg tggtcagcgt cctcaccgtc ctgcaccagg actggctgaa tggcaaggag 1380

tacaagtgca aggtctccaa caaagccctc ccagccccca tcgagaaaac catctccaaa 1440
gccaaagggc agccccgaga accacaggtg tacaccctgc ccccatcccg ggatgagctg 1500
accaagaacc aggtcagcct gacctgcctg gtcaaaggct tctatccaag cgacatcgcc 1560
gtggagtggg agagcaatgg gcagccggag aacaactaca agaccacgcc tcccgtgctg 1620
gactccgacg gctccttctt cctctacagc aagctcaccg tggacaagag caggtggcag 1680
caggggaacg tcttctcatg ctccgtgatg catgaggctc tgcacaacca ctacacgcag 1740
aagagcctct ccctgtctcc gggtaactca ttattcaacc aagaagttca aattcccttg 1800
accgaaagtt acagcccgaa ttctcaggtg cagctgaagg agtcaggacc tggcagcgtg 1860
gcgccctcac agagcctgtc catcacatgc accgtctcag ggttctcatt aaccggctat 1920
ggtgtaaact gggttcgcca gcctccagga aagggtctgg agtggctggg aatgatatgg 1980
ggtgatggaa gcacagacta taattcagct ctcaaatcca gactatcgat caccaaggac 2040
aactccaaga gccaagtttt cttaaaaatg aacagtctgc aaactgatga cacagccaga 2100
tactactgtg ctcgggatgg ttatagtaac tttcattact atgttatgga ctactggggt 2160
caaggaacct cagtcaccgt ctcctctggg ggtggaggct ctggtggcgg tggatccggc 2220
ggaggtgggt cgggtggcgg cggatctgac attgtgctca cccaatctcc agcttctttg 2280
gctgtgtctc taggtcagag agccaccatc tcctgcagag ccagtgaaag tgttgaatat 2340
tatgtcacaa gtttaatgca gtggtaccaa cagaaaccag gacagccacc caaactcctc 2400
atctctgctg ctagcaacgt agaatctggg gtccctgcca ggtttagtgg cagtgggtct 2460
gggacagact ttagcctcaa catccatcct gtggaggagg atgatattgc aatgtatttc 2520
tgtcagcaaa gtaggaaggt tccatggacg ttcggtggag gcaccaagct ggaaatcaag 2580
tga 2583

<210> 16
<211> 860
<212> PRT
<213> Artificial Sequence

<220>
<223> Amino acid sequence of 2E12 CH1 Fc (N297A) H68
      2E12 (X0247)

<400> 16
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala
                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser Gly Gly Gly Gly Ser Gly Gly
        115                 120                 125
Gly Gly Ser Gly Gly Gly Gly Ser Gly Gly Gly Gly Ser Asp Ile Val
    130                 135                 140
Leu Thr Gln Ser Pro Ala Ser Leu Ala Val Ser Leu Gly Gln Arg Ala
145                 150                 155                 160
Thr Ile Ser Cys Arg Ala Ser Glu Ser Val Glu Tyr Tyr Val Thr Ser
                165                 170                 175
Leu Met Gln Trp Tyr Gln Gln Lys Pro Gly Gln Pro Pro Lys Leu Leu
            180                 185                 190
Ile Ser Ala Ala Ser Asn Val Glu Ser Gly Val Pro Ala Arg Phe Ser
        195                 200                 205
Gly Ser Gly Ser Gly Thr Asp Phe Ser Leu Asn Ile His Pro Val Glu
    210                 215                 220
Glu Asp Asp Ile Ala Met Tyr Phe Cys Gln Gln Ser Arg Lys Val Pro
225                 230                 235                 240
Trp Thr Phe Gly Gly Gly Thr Lys Leu Glu Ile Lys Ser Ser Ser Ala

                245                 250                 255
Ser Thr Lys Gly Pro Ser Val Phe Pro Leu Ala Pro Ser Ser Lys Ser
            260                 265                 270
Thr Ser Gly Gly Thr Ala Ala Leu Gly Cys Leu Val Lys Asp Tyr Phe
        275                 280                 285
Pro Glu Pro Val Thr Val Ser Trp Asn Ser Gly Ala Leu Thr Ser Gly
    290                 295                 300
Val His Thr Phe Pro Ala Val Leu Gln Ser Ser Gly Leu Tyr Ser Leu
305                 310                 315                 320
Ser Ser Val Val Thr Val Pro Ser Ser Ser Leu Gly Thr Gln Thr Tyr
                325                 330                 335
Ile Cys Asn Val Asn His Lys Pro Ser Asn Thr Lys Val Asp Lys Lys
            340                 345                 350
Val Arg Thr Glu Pro Lys Ser Ser Asp Lys Thr His Thr Cys Pro Pro
        355                 360                 365
Cys Pro Ser Gly Ala Pro Glu Leu Leu Gly Gly Pro Ser Val Phe Leu
    370                 375                 380
Phe Pro Pro Lys Pro Lys Asp Thr Leu Met Ile Ser Arg Thr Pro Glu
385                 390                 395                 400
Val Thr Cys Val Val Val Asp Val Ser His Glu Asp Pro Glu Val Lys
                405                 410                 415
Phe Asn Trp Tyr Val Asp Gly Val Glu Val His Asn Ala Lys Thr Lys
            420                 425                 430
Pro Arg Glu Glu Gln Tyr Ala Ser Thr Tyr Arg Val Val Ser Val Leu
        435                 440                 445
Thr Val Leu His Gln Asp Trp Leu Asn Gly Lys Glu Tyr Lys Cys Lys
    450                 455                 460
Val Ser Asn Lys Ala Leu Pro Ala Pro Ile Glu Lys Thr Ile Ser Lys
465                 470                 475                 480
Ala Lys Gly Gln Pro Arg Glu Pro Gln Val Tyr Thr Leu Pro Pro Ser
                485                 490                 495
Arg Asp Glu Leu Thr Lys Asn Gln Val Ser Leu Thr Cys Leu Val Lys
            500                 505                 510
Gly Phe Tyr Pro Ser Asp Ile Ala Val Glu Trp Glu Ser Asn Gly Gln
        515                 520                 525
Pro Glu Asn Asn Tyr Lys Thr Thr Pro Pro Val Leu Asp Ser Asp Gly
    530                 535                 540
Ser Phe Phe Leu Tyr Ser Lys Leu Thr Val Asp Lys Ser Arg Trp Gln
545                 550                 555                 560
Gln Gly Asn Val Phe Ser Cys Ser Val Met His Glu Ala Leu His Asn
                565                 570                 575
His Tyr Thr Gln Lys Ser Leu Ser Leu Ser Pro Gly Asn Ser Leu Phe
            580                 585                 590
Asn Gln Glu Val Gln Ile Pro Leu Thr Glu Ser Tyr Ser Pro Asn Ser
        595                 600                 605
Gln Val Gln Leu Lys Glu Ser Gly Pro Gly Ser Val Ala Pro Ser Gln
    610                 615                 620
Ser Leu Ser Ile Thr Cys Thr Val Ser Gly Phe Ser Leu Thr Gly Tyr
625                 630                 635                 640
Gly Val Asn Trp Val Arg Gln Pro Pro Gly Lys Gly Leu Glu Trp Leu
                645                 650                 655
Gly Met Ile Trp Gly Asp Gly Ser Thr Asp Tyr Asn Ser Ala Leu Lys
            660                 665                 670
Ser Arg Leu Ser Ile Thr Lys Asp Asn Ser Lys Ser Gln Val Phe Leu
        675                 680                 685
Lys Met Asn Ser Leu Gln Thr Asp Asp Thr Ala Arg Tyr Tyr Cys Ala
    690                 695                 700
Arg Asp Gly Tyr Ser Asn Phe His Tyr Tyr Val Met Asp Tyr Trp Gly
705                 710                 715                 720
Gln Gly Thr Ser Val Thr Val Ser Ser Gly Gly Gly Gly Ser Gly Gly
                725                 730                 735
Gly Gly Ser Gly Gly Gly Gly Ser Gly Gly Gly Gly Ser Asp Ile Val
            740                 745                 750

Leu Thr Gln Ser Pro Ala Ser Leu Ala Val Ser Leu Gly Gln Arg Ala
        755                 760                 765
Thr Ile Ser Cys Arg Ala Ser Glu Ser Val Glu Tyr Tyr Val Thr Ser
    770                 775                 780
Leu Met Gln Trp Tyr Gln Gln Lys Pro Gly Gln Pro Pro Lys Leu Leu
785                 790                 795                 800
Ile Ser Ala Ala Ser Asn Val Glu Ser Gly Val Pro Ala Arg Phe Ser
                805                 810                 815
Gly Ser Gly Ser Gly Thr Asp Phe Ser Leu Asn Ile His Pro Val Glu
            820                 825                 830
Glu Asp Asp Ile Ala Met Tyr Phe Cys Gln Gln Ser Arg Lys Val Pro
        835                 840                 845
Trp Thr Phe Gly Gly Gly Thr Lys Leu Glu Ile Lys
    850                 855                 860

<210> 17
<211> 2547
<212> DNA
<213> Artificial Sequence

<220>
<223> Nucleotide sequence of P2C2 CK(YAE) Fc (N297A) H68
      A2 (X0248)

<400> 17
gatattgtga tgatccagga tgaactctcc aatcctgtca gttctggaga atcagtttcc 60
atctcctgta ggtctagtaa gagtctccta gataaggaag ggaagacata cttgaattgg 120
tttctgcaga gaccaggaca atctcctcag ctcctgatct atctgatgtc catgcgtgaa 180
tcaggagtct cagaccggtt tagtggcagt gggtcaggaa cagatttcac cctggaaatc 240
agtagagtga aggctgagga tgtgggtgtg tattactgtc aacaacttgt agagtatccg 300
tggacgttcg gtggaggcac caagctggaa atcaaaggtg gcggtggctc tggcggaggt 360
ggatccggtg gcggcggctc tcagatccag ttggtgcagt ctggacctga gctgaagaag 420
cctggagaga cagtcaagat ctcctgcaag gcttctggtt ataccttcac agacttttca 480
atgcactggg tgaggcaggc tccaggaaag ggtttaaagt ggatgggctg gataaacact 540
gagactggtg agccaacata tgcagatgac ttcaggggac ggtttgcctt ctctttggaa 600
acttctgcca gcactgccta tttgcagatc aacaacctca aaaatgagga cacggctaca 660
tatttttgta cctggtctgc ttactggggc caagggactc tggtcactgt ctctgcctcg 720
agcactgtgg ctgcaccatc tgtcttcatc ttcccgccat ctgatgagca gttgaaatct 780
ggaactgcct ctgttgtgtg cctgctgaat tacttctatc ccagagaggc caaagtacag 840
tggaaggtgg ataacgccct ccaatcgggt aactcccagg agagtgccac agagcaggac 900
agcaaggaca gcacctacag cctcagcagc gagctgacgc tgagcaaagc agactacgag 960
aaacacaaag tctacgcctg cgaagtcacc catcagggcc tgagctcgcc cgtcacaaag 1020
agcttcaaca ggggagagcg tacggagccc aaatcttctg acaaaactca cacatgccca 1080
ccgtgcccat ccggagcacc tgaactcctg ggtggaccgt cagtcttcct cttcccccca 1140
aaacccaagg acaccctcat gatctcccgg acccctgagg tcacatgcgt ggtggtggac 1200
gtgagccacg aagaccctga ggtcaagttc aactggtacg tggacggcgt ggaggtgcat 1260
aatgccaaga caaagccgcg ggaggagcag tacgccagca cgtaccgtgt ggtcagcgtc 1320
ctcaccgtcc tgcaccagga ctggctgaat ggcaaggagt acaagtgcaa ggtctccaac 1380
aaagccctcc cagcccccat cgagaaaacc atctccaaag ccaaagggca gccccgagaa 1440
ccacaggtgt acaccctgcc cccatcccgg gatgagctga ccaagaacca ggtcagcctg 1500
acctgcctgg tcaaaggctt ctatccaagc gacatcgccg tggagtggga gagcaatggg 1560
cagccggaga acaactacaa gaccacgcct cccgtgctgg actccgacgg ctccttcttc 1620
ctctacagca agctcaccgt ggacaagagc aggtggcagc aggggaacgt cttctcatgc 1680
tccgtgatgc atgaggctct gcacaaccac tacacgcaga agagcctctc cctgtctccg 1740
ggtaactcat tattcaacca agaagttcaa attcccttga ccgaaagtta cagcccgaat 1800
tctgaagttc aattgttaga gtctggtggc ggtcttgttc agcctggtgg ttctttacgt 1860
ctttcttgcg ctgcttccgg attcactttc tctttttacc agatgcattg ggttcgccaa 1920
gctcctggta aaggtttgga gtgggtttct ggtatctatc cttctggtgg ctatactaag 1980
tatgctgact ccgttaaagg tcgcttcact atctctagag acaactctaa gaatactctc 2040
tacttgcaga tgaacagctt aagggctgag gacactgcag tctactattg tgcgagagat 2100
ctagggtacg gcagtagctg gtactacttt gactactggg gccagggaac cctggtcacc 2160
gtctcaagcg gtggcggcgg ttcggggggt ggcggaagtg gaggtggagg gagtcagagc 2220

gctttgactc agcctgcctc cgtgtctggg tctcctggac agtcgatcac catctcctgc 2280
agtggaaccg acagtgacgt tggtggttat aaccatgtct cctggtacca acaacaccca 2340
ggcaaggccc ccaaactcat aatttatgat gtcgatcatc ggccctcagg gatctctaac 2400
cgtttctctg gctccaagtc tggcaacacg gcctccctga ccatctctgg gctccaggct 2460
gaggacgagg ctgattatta ctgcagctca tatagaagcg gtagcactta tgtcttcgga 2520
actgggacca aggtcaccgt cctatga 2547

<210> 18
<211> 848
<212> PRT
<213> Artificial Sequence

<220>
<223> Amino acid sequence of P2C2 CK(YAE) Fc (N297A) H68
      A2 (X0248)

<400> 18
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser
    50                  55                  60
Asp Arg Phe Ser Gly Ser Gly Ser Gly Thr Asp Phe Thr Leu Glu Ile
65                  70                  75                  80
Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110
Gly Gly Gly Gly Ser Gly Gly Gly Gly Ser Gly Gly Gly Gly Ser Gln
        115                 120                 125
Ile Gln Leu Val Gln Ser Gly Pro Glu Leu Lys Lys Pro Gly Glu Thr
    130                 135                 140
Val Lys Ile Ser Cys Lys Ala Ser Gly Tyr Thr Phe Thr Asp Phe Ser
145                 150                 155                 160
Met His Trp Val Arg Gln Ala Pro Gly Lys Gly Leu Lys Trp Met Gly
                165                 170                 175
Trp Ile Asn Thr Glu Thr Gly Glu Pro Thr Tyr Ala Asp Asp Phe Arg
            180                 185                 190
Gly Arg Phe Ala Phe Ser Leu Glu Thr Ser Ala Ser Thr Ala Tyr Leu
        195                 200                 205
Gln Ile Asn Asn Leu Lys Asn Glu Asp Thr Ala Thr Tyr Phe Cys Thr
    210                 215                 220
Trp Ser Ala Tyr Trp Gly Gln Gly Thr Leu Val Thr Val Ser Ala Ser
225                 230                 235                 240
Ser Thr Val Ala Ala Pro Ser Val Phe Ile Phe Pro Pro Ser Asp Glu
                245                 250                 255
Gln Leu Lys Ser Gly Thr Ala Ser Val Val Cys Leu Leu Asn Tyr Phe
            260                 265                 270
Tyr Pro Arg Glu Ala Lys Val Gln Trp Lys Val Asp Asn Ala Leu Gln
        275                 280                 285
Ser Gly Asn Ser Gln Glu Ser Ala Thr Glu Gln Asp Ser Lys Asp Ser
    290                 295                 300
Thr Tyr Ser Leu Ser Ser Glu Leu Thr Leu Ser Lys Ala Asp Tyr Glu
305                 310                 315                 320
Lys His Lys Val Tyr Ala Cys Glu Val Thr His Gln Gly Leu Ser Ser
                325                 330                 335
Pro Val Thr Lys Ser Phe Asn Arg Gly Glu Arg Thr Glu Pro Lys Ser
            340                 345                 350
Ser Asp Lys Thr His Thr Cys Pro Pro Cys Pro Ser Gly Ala Pro Glu
        355                 360                 365

Leu Leu Gly Gly Pro Ser Val Phe Leu Phe Pro Pro Lys Pro Lys Asp
    370                 375                 380
Thr Leu Met Ile Ser Arg Thr Pro Glu Val Thr Cys Val Val Val Asp
385                 390                 395                 400
Val Ser His Glu Asp Pro Glu Val Lys Phe Asn Trp Tyr Val Asp Gly
                405                 410                 415
Val Glu Val His Asn Ala Lys Thr Lys Pro Arg Glu Glu Gln Tyr Ala
            420                 425                 430
Ser Thr Tyr Arg Val Val Ser Val Leu Thr Val Leu His Gln Asp Trp
        435                 440                 445
Leu Asn Gly Lys Glu Tyr Lys Cys Lys Val Ser Asn Lys Ala Leu Pro
    450                 455                 460
Ala Pro Ile Glu Lys Thr Ile Ser Lys Ala Lys Gly Gln Pro Arg Glu
465                 470                 475                 480
Pro Gln Val Tyr Thr Leu Pro Pro Ser Arg Asp Glu Leu Thr Lys Asn
                485                 490                 495
Gln Val Ser Leu Thr Cys Leu Val Lys Gly Phe Tyr Pro Ser Asp Ile
            500                 505                 510
Ala Val Glu Trp Glu Ser Asn Gly Gln Pro Glu Asn Asn Tyr Lys Thr
        515                 520                 525
Thr Pro Pro Val Leu Asp Ser Asp Gly Ser Phe Phe Leu Tyr Ser Lys
    530                 535                 540
Leu Thr Val Asp Lys Ser Arg Trp Gln Gln Gly Asn Val Phe Ser Cys
545                 550                 555                 560
Ser Val Met His Glu Ala Leu His Asn His Tyr Thr Gln Lys Ser Leu
                565                 570                 575
Ser Leu Ser Pro Gly Asn Ser Leu Phe Asn Gln Glu Val Gln Ile Pro
            580                 585                 590
Leu Thr Glu Ser Tyr Ser Pro Asn Ser Glu Val Gln Leu Leu Glu Ser
        595                 600                 605
Gly Gly Gly Leu Val Gln Pro Gly Gly Ser Leu Arg Leu Ser Cys Ala
    610                 615                 620
Ala Ser Gly Phe Thr Phe Ser Phe Tyr Gln Met His Trp Val Arg Gln
625                 630                 635                 640
Ala Pro Gly Lys Gly Leu Glu Trp Val Ser Gly Ile Tyr Pro Ser Gly
                645                 650                 655
Gly Tyr Thr Lys Tyr Ala Asp Ser Val Lys Gly Arg Phe Thr Ile Ser
            660                 665                 670
Arg Asp Asn Ser Lys Asn Thr Leu Tyr Leu Gln Met Asn Ser Leu Arg
        675                 680                 685
Ala Glu Asp Thr Ala Val Tyr Tyr Cys Ala Arg Asp Leu Gly Tyr Gly
    690                 695                 700
Ser Ser Trp Tyr Tyr Phe Asp Tyr Trp Gly Gln Gly Thr Leu Val Thr
705                 710                 715                 720
Val Ser Ser Gly Gly Gly Gly Ser Gly Gly Gly Gly Ser Gly Gly Gly
                725                 730                 735
Gly Ser Gln Ser Ala Leu Thr Gln Pro Ala Ser Val Ser Gly Ser Pro
            740                 745                 750
Gly Gln Ser Ile Thr Ile Ser Cys Ser Gly Thr Asp Ser Asp Val Gly
        755                 760                 765
Gly Tyr Asn His Val Ser Trp Tyr Gln Gln His Pro Gly Lys Ala Pro
    770                 775                 780
Lys Leu Ile Ile Tyr Asp Val Asp His Arg Pro Ser Gly Ile Ser Asn
785                 790                 795                 800
Arg Phe Ser Gly Ser Lys Ser Gly Asn Thr Ala Ser Leu Thr Ile Ser
                805                 810                 815
Gly Leu Gln Ala Glu Asp Glu Ala Asp Tyr Tyr Cys Ser Ser Tyr Arg
            820                 825                 830
Ser Gly Ser Thr Tyr Val Phe Gly Thr Gly Thr Lys Val Thr Val Leu
        835                 840                 845

<210> 19

<211> 2427
<212> DNA
<213> Artificial Sequence

<220>
<223> Nucleotide sequence of PDL2-CH1 Fc(N297A) H68 2E12
      (X0249)

<400> 19
ttattcacag tgacagtccc taaggaactg tacataatag agcatggcag caatgtgacc 60
ctggaatgca actttgacac tggaagtcat gtgaaccttg gagcaataac agccagtttg 120
caaaaggtgg aaaatgatac atccccacac cgtgaaagag ccactttgct ggaggagcag 180
ctgcccttag ggaaggcctc gttccacata cctcaagtcc aagtgaggga cgaaggacag 240
taccaatgca taatcatcta tggggtcgcc tgggactaca agtacctgac tctgaaagtc 300
aaagcttcct acaggaaaat aaacactcac atcctaaagg ttccagaaac agatgaggta 360
gagctcacct gccaggctac aggttatcct ctggcagaag tatcctggcc aaacgtcagc 420
gttcctgcca acaccagcca ctccaggacc cctgaaggcc tctaccaggt caccagtgtt 480
ctgcgcctaa agccaccccc tggcagaaac ttcagctgtg tgttctggaa tactcacgtg 540
agggaactta ctttggccag cattgacctt caaagtcaga tggaacccag gacccatcca 600
acctcgagcg cctccaccaa gggcccatcg gtcttccccc tggcaccctc ctccaagagc 660
acctctgggg gcacagcggc cctgggctgc ctggtcaagg actacttccc cgagccggtg 720
acggtgtcgt ggaactcagg cgccctgacc agcggcgtgc acaccttccc ggctgtccta 780
cagtcctcag gactctactc cctcagcagc gtggtgaccg tgccctccag cagcttgggc 840
acccagacct acatctgcaa cgtgaatcac aagcccagca acaccaaggt ggacaagaaa 900
gttcgtacgg agcccaaatc ttctgacaaa actcacacat gcccaccgtg cccatccgga 960
gcacctgaac tcctgggtgg accgtcagtc ttcctcttcc ccccaaaacc caaggacacc 1020
ctcatgatct cccggacccc tgaggtcaca tgcgtggtgg tggacgtgag ccacgaagac 1080
cctgaggtca agttcaactg gtacgtggac ggcgtggagg tgcataatgc caagacaaag 1140
ccgcgggagg agcagtacgc cagcacgtac cgtgtggtca gcgtcctcac cgtcctgcac 1200
caggactggc tgaatggcaa ggagtacaag tgcaaggtct ccaacaaagc cctcccagcc 1260
cccatcgaga aaaccatctc caaagccaaa gggcagcccc gagaaccaca ggtgtacacc 1320
ctgcccccat cccgggatga gctgaccaag aaccaggtca gcctgacctg cctggtcaaa 1380
ggcttctatc caagcgacat cgccgtggag tgggagagca atgggcagcc ggagaacaac 1440
tacaagacca cgcctcccgt gctggactcc gacggctcct tcttcctcta cagcaagctc 1500
accgtggaca agagcaggtg gcagcagggg aacgtcttct catgctccgt gatgcatgag 1560
gctctgcaca accactacac gcagaagagc ctctccctgt ctccgggtaa ctcattattc 1620
aaccaagaag ttcaaattcc cttgaccgaa agttacagcc cgaattctca ggtgcagctg 1680
aaggagtcag gacctggcag cgtggcgccc tcacagagcc tgtccatcac atgcaccgtc 1740
tcagggttct cattaaccgg ctatggtgta aactgggttc gccagcctcc aggaaagggt 1800
ctggagtggc tgggaatgat atggggtgat ggaagcacag actataattc agctctcaaa 1860
tccagactat cgatcaccaa ggacaactcc aagagccaag ttttcttaaa aatgaacagt 1920
ctgcaaactg atgacacagc cagatactac tgtgctcggg atggttatag taactttcat 1980
tactatgtta tggactactg gggtcaagga acctcagtca ccgtctcctc tgggggtgga 2040
ggctctggtg gcggtggatc cggcggaggt gggtcgggtg gcggcggatc tgacattgtg 2100
ctcacccaat ctccagcttc tttggctgtg tctctaggtc agagagccac catctcctgc 2160
agagccagtg aaagtgttga atattatgtc acaagtttaa tgcagtggta ccaacagaaa 2220
ccaggacagc cacccaaact cctcatctct gctgctagca acgtagaatc tggggtccct 2280
gccaggttta gtggcagtgg gtctgggaca gactttagcc tcaacatcca tcctgtggag 2340
gaggatgata ttgcaatgta tttctgtcag caaagtagga aggttccatg gacgttcggt 2400
ggaggcacca agctggaaat caagtga 2427

<210> 20
<211> 808
<212> PRT
<213> Artificial Sequence

<220>
<223> Amino acid sequence of PDL2-CH1 Fc(N297A) H68 2E12
      (X0249)

<400> 20

<210> 21
<211> 2331
<212> DNA
<213> Artificial Sequence

<220>
<223> Nucleotide sequence of monoIL10 Ck(YAE) Fc(N297A)
      H68 3D1 (X0281)

<400> 21
   agcccaggcc agggcaccca gtctgagaac agctgcaccc acttcccagg caacctgcct 60
   aacatgcttc gagatctccg agatgccttc agcagagtga agactttctt tcaaatgaag 120
   gatcagctgg acaacttgtt gttaaaggag tccttgctgg aggactttaa gggttacctg 180
   ggttgccaag ccttgtctga gatgatccag ttttacctgg aggaggtgat gccccaagct 240
   gagaaccaag acccagacat caaggcgcat gtgaactccc tgggggagaa cctgaagacc 300
   ctcaggctga ggctacggcg ctgtcatcga tttcttccct gtgaaaacgg tggtggatcc 360
   ggcggtaaga gcaaggccgt ggagcaggtg aagaatgcct ttaataagct ccaagagaaa 420
   ggcatctaca aagccatgag tgagtttgac atcttcatca actacataga agcctacatt 480
   acaatgaaga tacgaaactc gagcactgtg gctgcaccat ctgtcttcat cttcccgcca 540
   tctgatgagc agttgaaatc tggaactgcc tctgttgtgt gcctgctgaa ttacttctat 600
   cccagagagg ccaaagtaca gtggaaggtg gataacgccc tccaatcggg taactcccag 660
   gagagtgcca cagagcagga cagcaaggac agcacctaca gcctcagcag cgagctgacg 720
   ctgagcaaag cagactacga gaaacacaaa gtctacgcct gcgaagtcac ccatcagggc 780
   ctgagctcgc ccgtcacaaa gagcttcaac aggggagagc gtacggagcc caaatcttct 840
   gacaaaactc acacatgccc accgtgccca tccggagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacgccagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaactca ttattcaacc aagaagttca aattcccttg 1560
   accgaaagtt acagcccgaa ttctcaggtc cagctgcagc agtctgggcc tgagctggtg 1620
   aggcctgggg aatcagtgaa gatttcctgc aagggttccg gctacacatt cactgattat 1680
   gctatacagt gggtgaagca gagtcatgca aagagtctag agtggattgg agttattaat 1740
   atttactatg ataatacaaa ctacaaccag aagtttaagg gcaaggccac aatgactgta 1800
   gacaaatcct ccagcacagc ctatatggaa cttgccagat tgacatctga ggattctgcc 1860
   atctattact gtgcaagagc ggcctggtat atggactact ggggtcaagg aacctcagtc 1920
   accgtctcct cagggggtgg aggctctggt ggcggtggct ctggcggagg tggatccggt 1980
   ggcggcggat ctgacattgt gctgtcacag tctccatcct ccctggctgt gtcagcagga 2040
   gagaaggtca ctatgagctg caaatccagt cagagtctgc tcaacagtag aacccgagag 2100
   aactacttgg cttggtacca gcagaaacca gggcagtctc ctaaactgct gatctactgg 2160
   gcatccacta gggaatctgg ggtccctgat cgcttcacag gcagtggatc tgggacagat 2220
   ttcactctca ccatcagcag tgtgcaggct gaagacctgg cagtttatta ctgcacgcaa 2280
   tcttataatc tttacacgtt cggagggggg accaagctgg aaataaaata a 2331

<210> 22
<211> 776
<212> PRT
<213> Artificial Sequence

<220>
<223> Amino acid sequence of monoIL10 Ck(YAE) Fc(N297A)
      H68 3D1 (X0281)

<400> 22
Ser Pro Gly Gln Gly Thr Gln Ser Glu Asn Ser Cys Thr His Phe Pro
1               5                   10                  15
Gly Asn Leu Pro Asn Met Leu Arg Asp Leu Arg Asp Ala Phe Ser Arg
            20                  25                  30
Val Lys Thr Phe Phe Gln Met Lys Asp Gln Leu Asp Asn Leu Leu Leu
        35                  40                  45
Lys Glu Ser Leu Leu Glu Asp Phe Lys Gly Tyr Leu Gly Cys Gln Ala
    50                  55                  60
Leu Ser Glu Met Ile Gln Phe Tyr Leu Glu Glu Val Met Pro Gln Ala
65                  70                  75                  80
Glu Asn Gln Asp Pro Asp Ile Lys Ala His Val Asn Ser Leu Gly Glu
                85                  90                  95
Asn Leu Lys Thr Leu Arg Leu Arg Leu Arg Arg Cys His Arg Phe Leu
            100                 105                 110
Pro Cys Glu Asn Gly Gly Gly Ser Gly Gly Lys Ser Lys Ala Val Glu
        115                 120                 125
Gln Val Lys Asn Ala Phe Asn Lys Leu Gln Glu Lys Gly Ile Tyr Lys
    130                 135                 140
Ala Met Ser Glu Phe Asp Ile Phe Ile Asn Tyr Ile Glu Ala Tyr Ile
145                 150                 155                 160
Thr Met Lys Ile Arg Asn Ser Ser Thr Val Ala Ala Pro Ser Val Phe
                165                 170                 175
Ile Phe Pro Pro Ser Asp Glu Gln Leu Lys Ser Gly Thr Ala Ser Val
            180                 185                 190
Val Cys Leu Leu Asn Tyr Phe Tyr Pro Arg Glu Ala Lys Val Gln Trp
        195                 200                 205
Lys Val Asp Asn Ala Leu Gln Ser Gly Asn Ser Gln Glu Ser Ala Thr

210                 215                 220
Glu Gln Asp Ser Lys Asp Ser Thr Tyr Ser Leu Ser Ser Glu Leu Thr
225                 230                 235                 240
Leu Ser Lys Ala Asp Tyr Glu Lys His Lys Val Tyr Ala Cys Glu Val
                245                 250                 255
Thr His Gln Gly Leu Ser Ser Pro Val Thr Lys Ser Phe Asn Arg Gly
            260                 265                 270
Glu Arg Thr Glu Pro Lys Ser Ser Asp Lys Thr His Thr Cys Pro Pro
        275                 280                 285
Cys Pro Ser Gly Ala Pro Glu Leu Leu Gly Gly Pro Ser Val Phe Leu
    290                 295                 300
Phe Pro Pro Lys Pro Lys Asp Thr Leu Met Ile Ser Arg Thr Pro Glu
305                 310                 315                 320
Val Thr Cys Val Val Val Asp Val Ser His Glu Asp Pro Glu Val Lys
                325                 330                 335
Phe Asn Trp Tyr Val Asp Gly Val Glu Val His Asn Ala Lys Thr Lys
            340                 345                 350
Pro Arg Glu Glu Gln Tyr Ala Ser Thr Tyr Arg Val Val Ser Val Leu
        355                 360                 365
Thr Val Leu His Gln Asp Trp Leu Asn Gly Lys Glu Tyr Lys Cys Lys
    370                 375                 380
Val Ser Asn Lys Ala Leu Pro Ala Pro Ile Glu Lys Thr Ile Ser Lys
385                 390                 395                 400
Ala Lys Gly Gln Pro Arg Glu Pro Gln Val Tyr Thr Leu Pro Pro Ser
                405                 410                 415
Arg Asp Glu Leu Thr Lys Asn Gln Val Ser Leu Thr Cys Leu Val Lys
            420                 425                 430
Gly Phe Tyr Pro Ser Asp Ile Ala Val Glu Trp Glu Ser Asn Gly Gln
        435                 440                 445
Pro Glu Asn Asn Tyr Lys Thr Thr Pro Pro Val Leu Asp Ser Asp Gly
    450                 455                 460
Ser Phe Phe Leu Tyr Ser Lys Leu Thr Val Asp Lys Ser Arg Trp Gln
465                 470                 475                 480
Gln Gly Asn Val Phe Ser Cys Ser Val Met His Glu Ala Leu His Asn
                485                 490                 495
His Tyr Thr Gln Lys Ser Leu Ser Leu Ser Pro Gly Asn Ser Leu Phe
            500                 505                 510
Asn Gln Glu Val Gln Ile Pro Leu Thr Glu Ser Tyr Ser Pro Asn Ser
        515                 520                 525
Gln Val Gln Leu Gln Gln Ser Gly Pro Glu Leu Val Arg Pro Gly Glu
    530                 535                 540
Ser Val Lys Ile Ser Cys Lys Gly Ser Gly Tyr Thr Phe Thr Asp Tyr
545                 550                 555                 560
Ala Ile Gln Trp Val Lys Gln Ser His Ala Lys Ser Leu Glu Trp Ile
                565                 570                 575
Gly Val Ile Asn Ile Tyr Tyr Asp Asn Thr Asn Tyr Asn Gln Lys Phe
            580                 585                 590
Lys Gly Lys Ala Thr Met Thr Val Asp Lys Ser Ser Ser Thr Ala Tyr
        595                 600                 605
Met Glu Leu Ala Arg Leu Thr Ser Glu Asp Ser Ala Ile Tyr Tyr Cys
    610                 615                 620
Ala Arg Ala Ala Trp Tyr Met Asp Tyr Trp Gly Gln Gly Thr Ser Val
625                 630                 635                 640
Thr Val Ser Ser Gly Gly Gly Gly Ser Gly Gly Gly Gly Ser Gly Gly
                645                 650                 655
Gly Gly Ser Gly Gly Gly Gly Ser Asp Ile Val Leu Ser Gln Ser Pro
            660                 665                 670
Ser Ser Leu Ala Val Ser Ala Gly Glu Lys Val Thr Met Ser Cys Lys
        675                 680                 685
Ser Ser Gln Ser Leu Leu Asn Ser Arg Thr Arg Glu Asn Tyr Leu Ala
    690                 695                 700
Trp Tyr Gln Gln Lys Pro Gly Gln Ser Pro Lys Leu Leu Ile Tyr Trp
705                 710                 715                 720

Ala Ser Thr Arg Glu Ser Gly Val Pro Asp Arg Phe Thr Gly Ser Gly
                725                 730                 735
Ser Gly Thr Asp Phe Thr Leu Thr Ile Ser Ser Val Gln Ala Glu Asp
            740                 745                 750
Leu Ala Val Tyr Tyr Cys Thr Gln Ser Tyr Asn Leu Tyr Thr Phe Gly
        755                 760                 765
Gly Gly Thr Lys Leu Glu Ile Lys
    770                 775

<210> 23
<211> 2330
<212> DNA
<213> Artificial Sequence

<220>
<223> Nucleotide sequence of monoIl10CkwtFc(N297A)H68
      3D1 (X0282)

<400> 23
agcccaggcc agggcaccca gtctgagaac agctgcaccc acttcccagg caacctgcct 60
aacatgcttc gagatctccg agatgccttc agcagagtga agactttctt tcaaatgaag 120
gatcagctgg acaacttgtt gttaaaggag tccttgctgg aggactttaa gggttacctg 180
ggttgccaag ccttgtctga gatgatccag ttttacctgg aggaggtgat gccccaagct 240
gagaaccaag acccagacat caaggcgcat gtgaactccc tgggggagaa cctgaagacc 300
ctcaggctga ggctacggcg ctgtcatcga tttcttccct gtgaaaacgg tggtggatcc 360
ggcggtaaga gcaaggccgt ggagcaggtg aagaatgcct ttaataagct ccaagagaaa 420
ggcatctaca aagccatgag tgagtttgac atcttcatca actacataga agcctacatt 480
acaatgaaga tacgaaactc gagcactgtg gctgcaccat ctgtcttcat cttcccgcca 540
tctgatgagc agttgaaatc tggaactgcc tctgttgtgt gcctgctgaa taacttctat 600
cccagagagg ccaaagtaca gtggaaggtg gataacgccc tccaatcggg taactcccag 660
gagagtgtca cagagcagga cagcaaggac agcacctaca gcctcagcag caccctgacg 720
ctgagcaaag cagactacga gaaacacaaa gtctacgcct gcgaagtcac ccatcagggc 780
ctgagctcgc ccgtcacaaa gagcttcaac aggggagagc gtacggagcc caaatcttct 840
gacaaaactc acacatgccc accgtgccca tccggagcac ctgaactcct gggtggaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacgccagc 1080
acgtaccgtg tggtcagcgt cctcaccgtc ctgcaccagg actggctgaa tggcaaggag 1140
tacaagtgca aggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaactca ttattcaacc aagaagttca aattcccttg 1560
accgaaagtt acagcccgaa ttctcaggtc cagctgcagc agtctgggcc tgagctggtg 1620
aggcctgggg aatcagtgaa gatttcctgc aagggttccg gctacacatt cactgattat 1680
gctatacagt gggtgaagca gagtcatgca aagagtctag agtggattgg agttattaat 1740
atttactatg ataatacaaa ctacaaccag aagtttaagg gcaaggccac aatgactgta 1800
gacaaatcct ccagcacagc ctatatggaa cttgccagat tgacatctga ggattctgcc 1860
atctattact gtgcaagagc ggcctggtat atggactact ggggtcaagg aacctcagtc 1920
accgtctcct cagggggtgg aggctctggt ggcggtggct ctggcggagg tggatccggt 1980
ggcggcggat ctgacattgt gctgtcacag tctccatcct ccctggctgt gtcagcagga 2040
gagaaggtca ctatgagctg caaatccagt cagagtctgc tcaacagtag aacccgagag 2100
aactacttgg cttggtacca gcagaaacca gggcagtctc ctaaactgct gatctactgg 2160
gcatccacta gggaatctgg ggtccctgat cgcttcacag gcagtggatc tgggacagat 2220
ttcactctca ccatcagcag tgtgcaggct gaagacctgg cagtttatta ctgcacgcaa 2280
tcttataatc tttacacgtt cggagggggg accaagctgg aaataaaata 2330

<210> 24
<211> 776
<212> PRT

<213> Artificial Sequence

<220>
<223> Amino acid sequence of monoIl10CkwtFc(N297A)H68
      3D1 (X0282)

<400> 24

<210> 25
<211> 1806
<212> DNA
<213> Artificial Sequence

<220>
<223> Nucleotide sequence for ORN116 comprising MET021
      (anti-c-Met) scFv, human IgG1 SCC-P hinge, human
      IgG1 CH2, human IgG1 CH3 and human CH1

<400> 25
atggaagcac cagcgcagct tctcttcctc ctgctactct ggctcccaga taccaccggt 60
gacatccaga tgacccagtc tccatcctcc ctgtctgcat ctgtaggaga cagagtcacc 120
atcacttgtc gggcaagtca gagcattagg aactatttaa attggtatca gcaggaacca 180
gggaaagccc ctaagctcct gatctatgct gcatccagtt tgcaaagtgg ggtcccatca 240
aggttcagtg gcagtggatc tgggacagac ttcattctca ccatcagcag tctgcagcct 300
gaagattttg caacttacta ctgtcaacag agttacgtta ccccgctcac tttcggcgga 360
gggaccaagg tggagatcaa agggggtgga ggctctggtg gcggtggctc tggcggaggt 420
ggatccggtg gcggcggctc tgaagttcaa ttgttagagt ctggtggcgg tcttgttcag 480

cctggtggtt ctttacgtct ttcttgcgct gcttccggat tcactttctc taagtacgat 540
atgctttggg ttcgccaagc tcctggtaaa ggtttggagt gggtttctta tatctatcct 600
tctggtggcc ttactgagta tgctgactcc gttaaaggtc gcttcactat ctctagagac 660
aactctaaga atactctcta cttgcagatg aacagcttaa gggctgagga cacggccgtg 720
tattactgtg cgagacgggc tccccggtcc ctttcttttg atatctgggg ccaagggaca 780
atggtcaccg tctcctcgag cgagcccaaa tcttctgaca aaactcacac atgcccaccg 840
tgcccatccg gagcacctga actcctgggt ggaccgtcag tcttcctctt ccccccaaaa 900
cccaaggaca ccctcatgat ctcccggacc cctgaggtca catgcgtggt ggtggacgtg 960
agccacgaag accctgaggt caagttcaac tggtacgtgg acggcgtgga ggtgcataat 1020
gccaagacaa agccgcggga ggagcagtac aacagcacgt accgtgtggt cagcgtcctc 1080
accgtcctgc accaggactg gctgaatggc aaggagtaca agtgcaaggt ctccaacaaa 1140
gccctcccag cccccatcga gaaaaccatc tccaaagcca aagggcagcc ccgagaacca 1200
caggtgtaca ccctgccccc atcccgggat gagctgacca agaaccaggt cagcctgacc 1260
tgcctggtca aaggcttcta tccaagcgac atcgccgtgg agtgggagag caatgggcag 1320
ccggagaaca actacaagac cacgcctccc gtgctggact ccgacggctc cttcttcctc 1380
tacagcaagc tcaccgtgga caagagcagg tggcagcagg ggaacgtctt ctcatgctcc 1440
gtgatgcatg aggctctgca caaccactac acgcagaaga gcctctccct gtctccgggt 1500
aaatctagag cctccaccaa gggcccatcg gtcttccccc tggcaccctc ctccaagagc 1560
acctctgggg gcacagcggc cctgggctgc ctggtcaagg actacttccc cgagccggtg 1620
acggtgtcgt ggaactcagg cgccctgacc agcggcgtgc acaccttccc ggctgtccta 1680
cagtcctcag gactctactc cctcagcagc gtggtgaccg tgccctccag cagcttgggc 1740
acccagacct acatctgcaa cgtgaatcac aagcccagca acaccaaggt ggacaagaaa 1800
gtttga 1806

<210> 26
<211> 1797
<212> DNA
<213> Artificial Sequence

<220>
<223> Nucleotide sequence for ORN145 comprising : 4C04
      (anti-RON) scFv, human IgG1 SCC-P hinge, human
      IgG1 CH2, human IgG1 CH3 and human CH1

<400> 26
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt gggaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gcgagcccaa atcttctgac aaaactcaca catgcccacc gtgcccatcc 840
ggagcacctg aactcctggg tggaccgtca gtcttcctct tccccccaaa acccaaggac 900
accctcatga tctcccggac ccctgaggtc acatgcgtgg tggtggacgt gagccacgaa 960
gaccctgagg tcaagttcaa ctggtacgtg gacggcgtgg aggtgcataa tgccaagaca 1020
aagccgcggg aggagcagta caacagcacg taccgtgtgg tcagcgtcct caccgtcctg 1080
caccaggact ggctgaatgg caaggagtac aagtgcaagg tctccaacaa agccctccca 1140
gcccccatcg agaaaaccat ctccaaagcc aaagggcagc cccgagaacc acaggtgtac 1200
accctgcccc catcccggga tgagctgacc aagaaccagg tcagcctgac ctgcctggtc 1260
aaaggcttct atccaagcga catcgccgtg gagtgggaga gcaatgggca gccggagaac 1320
aactacaaga ccacgcctcc cgtgctggac tccgacggct ccttcttcct ctacagcaag 1380
ctcaccgtgg acaagagcag gtggcagcag gggaacgtct tctcatgctc cgtgatgcat 1440
gaggctctgc acaaccacta cacgcagaag agcctctccc tgtctccggg taaatctaga 1500
gcctccacca agggcccatc ggtcttcccc ctggcaccct cctccaagag cacctctggg 1560
ggcacagcgg ccctgggctg cctggtcaag gactacttcc ccgagccggt gacggtgtcg 1620
tggaactcag gcgccctgac cagcggcgtg cacaccttcc cggctgtcct acagtcctca 1680

ggactctact ccctcagcag cgtggtgacc gtgccctcca gcagcttggg cacccagacc 1740
tacatctgca acgtgaatca caagcccagc aacaccaagg tggacaagaa agtttga    1797

<210> 27
<211> 1818
<212> DNA
<213> Artificial Sequence

<220>
<223> Nucleotide sequence for ORN146 comprising : 4C04
      (anti-RON) scFv, human IgG1 SCC-P hinge, human
      CH2, human CH3, and human Ck(YAE)

<400> 27
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt gggaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gcgagcccaa atcttctgac aaaactcaca catgcccacc gtgcccatcc 840
ggagcacctg aactcctggg tggaccgtca gtcttcctct tccccccaaa acccaaggac 900
accctcatga tctcccggac ccctgaggtc acatgcgtgg tggtggacgt gagccacgaa 960
gaccctgagg tcaagttcaa ctggtacgtg gacggcgtgg aggtgcataa tgccaagaca 1020
aagccgcggg aggagcagta caacagcacg taccgtgtgg tcagcgtcct caccgtcctg 1080
caccaggact ggctgaatgg caaggagtac aagtgcaagg tctccaacaa agccctccca 1140
gcccccatcg agaaaaccat ctccaaagcc aaagggcagc cccgagaacc acaggtgtac 1200
accctgcccc catcccggga tgagctgacc aagaaccagg tcagcctgac ctgcctggtc 1260
aaaggcttct atccaagcga catcgccgtg gagtgggaga gcaatgggca gccggagaac 1320
aactacaaga ccacgcctcc cgtgctggac tccgacggct ccttcttcct ctacagcaag 1380
ctcaccgtgg acaagagcag gtggcagcag gggaacgtct tctcatgctc cgtgatgcat 1440
gaggctctgc acaaccacta cacgcagaag agcctctccc tgtctccggg taaatctaga 1500
actgtggctg caccatctgt cttcatcttc ccgccatctg atgagcagtt gaaatctgga 1560
actgcctctg ttgtgtgcct gctgaattac ttctatccca gagaggccaa agtacagtgg 1620
aaggtggata acgccctcca atcgggtaac tcccaggaga gtgccacaga gcaggacagc 1680
aaggacagca cctacagcct cagcagcgag ctgacgctga gcaaagcaga ctacgagaaa 1740
cacaaagtct acgcctgcga agtcacccat cagggcctga gctcgcccgt cacaaagagc 1800
ttcaacaggg gagagtga                                                1818

<210> 28
<211> 1806
<212> DNA
<213> Artificial Sequence

<220>
<223> Nucleotide sequence for ORN148 comprising : 11H09
      (anti-RON) scFv, human IgG1 SCC-P hinge, human
      CH2, human CH3, and human Ck(YAE)

<400> 28
atggaagcac cagcgcagct tctcttcctc ctgctactct ggctcccaga taccaccggt 60
gacatccaga tgacccagtc tccatcctcc ttatctgcct ctctgggaga aagagtcagt 120
ctcacttgtc gggcaagtca ggacattggt agtagcttaa gctggcttca gcaggaacca 180
gatggtacta ttaaacgcct gatctactcc acatccagtt tagattctgg tgtccccaaa 240
aggttcagtg gcagtaggtc ggggtcagat tattctctca ccatcagcag ccttgagtct 300
gaagattttg tagactatta ctgtctacaa tatgctagtt ctccgtggac gttcggtgga 360

ggcaccaagc tggaaatcaa aggtggcggt ggctctggcg gaggtggatc cggtggcggc 420
ggctctcagg tccagcttca gcagtctggg gctgaactgg caaaacctgg ggcctcagtg 480
aagatgtcct gcaagtcttc tggctacacc tttactagct actggataca ctgggtaaaa 540
gagaggcctg gacagggtct ggaatggatt ggatacattt atcctaccac tggttatact 600
gagtccaatc agaagttcaa ggacaaggcc acattgactg cagacaaatc ctccagcaca 660
gcctacatgc aactgagcag cctgacatct gaggactctg cagtctatta ctgtttccta 720
ctacggccct ggtacttcga tgtctggggc gcagggacca cggtcaccgt ctcctcgagc 780
gagcccaaat cttctgacaa aactcacaca tgcccaccgt gcccatccgg agcacctgaa 840
ctcctgggtg gaccgtcagt cttcctcttc cccccaaaac ccaaggacac cctcatgatc 900
tcccggaccc ctgaggtcac atgcgtggtg gtggacgtga gccacgaaga ccctgaggtc 960
aagttcaact ggtacgtgga cggcgtggag gtgcataatg ccaagacaaa gccgcgggag 1020
gagcagtaca acagcacgta ccgtgtggtc agcgtcctca ccgtcctgca ccaggactgg 1080
ctgaatggca aggagtacaa gtgcaaggtc tccaacaaag ccctcccagc ccccatcgag 1140
aaaaccatct ccaaagccaa agggcagccc cgagaaccac aggtgtacac cctgccccca 1200
tcccgggatg agctgaccaa gaaccaggtc agcctgacct gcctggtcaa aggcttctat 1260
cccagcgaca tcgccgtgga gtgggagagc aatgggcagc cggagaacaa ctacaagacc 1320
acgcctcccg tgctggactc cgacggctcc ttcttcctct acagcaagct caccgtggac 1380
aagagcaggt ggcagcaggg gaacgtcttc tcatgctccg tgatgcatga ggctctgcac 1440
aaccactaca cgcagaagag cctctccctg tctccgggta aatctagaac tgtggctgca 1500
ccatctgtct tcatcttccc gccatctgat gagcagttga aatctggaac tgcctctgtt 1560
gtgtgcctgc tgaattactt ctatcccaga gaggccaaag tacagtggaa ggtggataac 1620
gccctccaat cgggtaactc ccaggagagt gccacagagc aggacagcaa ggacagcacc 1680
tacagcctca gcagcgagct gacgctgagc aaagcagact acgagaaaca caaagtctac 1740
gcctgcgaag tcacccatca gggcctgagc tcgcccgtca caaagagctt caacagggga 1800
gagtga 1806

<210> 29
<211> 581
<212> PRT
<213> Artificial Sequence

<220>
<223> ORN116 mature protein comprising MET021
      (anti-c-Met) scFv, human IgG1 SCC-P hinge, human
      IgG1 CH2, human IgG1 CH3 and human CH1

<400> 29
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Arg Asn Tyr
            20                  25                  30
Leu Asn Trp Tyr Gln Gln Glu Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Ile Leu Thr Ile Ser Ser Leu Gln Pro
65                  70                  75                  80
Glu Asp Phe Ala Thr Tyr Tyr Cys Gln Gln Ser Tyr Val Thr Pro Leu
                85                  90                  95
Thr Phe Gly Gly Gly Thr Lys Val Glu Ile Lys Gly Gly Gly Gly Ser
            100                 105                 110
Gly Gly Gly Gly Ser Gly Gly Gly Gly Ser Gly Gly Gly Gly Ser Glu
        115                 120                 125
Val Gln Leu Leu Glu Ser Gly Gly Gly Leu Val Gln Pro Gly Gly Ser
    130                 135                 140
Leu Arg Leu Ser Cys Ala Ala Ser Gly Phe Thr Phe Ser Lys Tyr Asp
145                 150                 155                 160
Met Leu Trp Val Arg Gln Ala Pro Gly Lys Gly Leu Glu Trp Val Ser
                165                 170                 175
Tyr Ile Tyr Pro Ser Gly Gly Leu Thr Glu Tyr Ala Asp Ser Val Lys
            180                 185                 190
Gly Arg Phe Thr Ile Ser Arg Asp Asn Ser Lys Asn Thr Leu Tyr Leu
        195                 200                 205

Gln Met Asn Ser Leu Arg Ala Glu Asp Thr Ala Val Tyr Tyr Cys Ala
    210                 215                 220
Arg Arg Ala Pro Arg Ser Leu Ser Phe Asp Ile Trp Gly Gln Gly Thr
225                 230                 235                 240
Met Val Thr Val Ser Ser Ser Glu Pro Lys Ser Ser Asp Lys Thr His
                245                 250                 255
Thr Cys Pro Pro Cys Pro Ser Gly Ala Pro Glu Leu Leu Gly Gly Pro
            260                 265                 270
Ser Val Phe Leu Phe Pro Pro Lys Pro Lys Asp Thr Leu Met Ile Ser
        275                 280                 285
Arg Thr Pro Glu Val Thr Cys Val Val Val Asp Val Ser His Glu Asp
    290                 295                 300
Pro Glu Val Lys Phe Asn Trp Tyr Val Asp Gly Val Glu Val His Asn
305                 310                 315                 320
Ala Lys Thr Lys Pro Arg Glu Glu Gln Tyr Asn Ser Thr Tyr Arg Val
                325                 330                 335
Val Ser Val Leu Thr Val Leu His Gln Asp Trp Leu Asn Gly Lys Glu
            340                 345                 350
Tyr Lys Cys Lys Val Ser Asn Lys Ala Leu Pro Ala Pro Ile Glu Lys
        355                 360                 365
Thr Ile Ser Lys Ala Lys Gly Gln Pro Arg Glu Pro Gln Val Tyr Thr
    370                 375                 380
Leu Pro Pro Ser Arg Asp Glu Leu Thr Lys Asn Gln Val Ser Leu Thr
385                 390                 395                 400
Cys Leu Val Lys Gly Phe Tyr Pro Ser Asp Ile Ala Val Glu Trp Glu
                405                 410                 415
Ser Asn Gly Gln Pro Glu Asn Asn Tyr Lys Thr Thr Pro Pro Val Leu
            420                 425                 430
Asp Ser Asp Gly Ser Phe Phe Leu Tyr Ser Lys Leu Thr Val Asp Lys
        435                 440                 445
Ser Arg Trp Gln Gln Gly Asn Val Phe Ser Cys Ser Val Met His Glu
    450                 455                 460
Ala Leu His Asn His Tyr Thr Gln Lys Ser Leu Ser Leu Ser Pro Gly
465                 470                 475                 480
Lys Ser Arg Ala Ser Thr Lys Gly Pro Ser Val Phe Pro Leu Ala Pro
                485                 490                 495
Ser Ser Lys Ser Thr Ser Gly Gly Thr Ala Ala Leu Gly Cys Leu Val
            500                 505                 510
Lys Asp Tyr Phe Pro Glu Pro Val Thr Val Ser Trp Asn Ser Gly Ala
        515                 520                 525
Leu Thr Ser Gly Val His Thr Phe Pro Ala Val Leu Gln Ser Ser Gly
    530                 535                 540
Leu Tyr Ser Leu Ser Ser Val Val Thr Val Pro Ser Ser Ser Leu Gly
545                 550                 555                 560
Thr Gln Thr Tyr Ile Cys Asn Val Asn His Lys Pro Ser Asn Thr Lys
                565                 570                 575
Val Asp Lys Lys Val
            580

<210> 30
<211> 578
<212> PRT
<213> Artificial Sequence

<220>
<223> ORN145 mature protein comprising : 4C04 (anti-RON)
      scFv, human IgG1 SCC-P hinge, human IgG1 CH2,
      human IgG1 CH3 and human CH1

<400> 30
Asp Ile Gln Met Thr Gln Ser Pro Ala Ser Gln Ser Ala Ser Leu Gly
1               5                   10                  15

Glu Ser Val Thr Ile Thr Cys Leu Ala Ser Gln Thr Ile Gly Thr Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Ala
    50                  55                  60
Ser Gly Ser Gly Thr Lys Phe Ser Phe Lys Ile Asp Ser Leu Gln Ser
65                  70                  75                  80
Glu Asp Phe Val Ser Tyr Tyr Cys Gln Gln Leu Tyr Asn Thr Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Ile Gln Leu Gln Gln
        115                 120                 125
Ser Gly Pro Glu Leu Val Lys Pro Gly Thr Ser Val Lys Ile Ser Cys
    130                 135                 140
Lys Ala Ser Asp Tyr Thr Leu Thr Asp Phe Tyr Met Asn Trp Val Arg
145                 150                 155                 160
Gln Lys Pro Gly Gln Gly Leu Glu Trp Ile Gly Arg Ile Tyr Pro Gly
                165                 170                 175
Thr Asp Lys Thr Arg Tyr Asn Glu Lys Phe Arg Asp Lys Ala Ile Leu
            180                 185                 190
Thr Val Asp Thr Ser Ser Ser Thr Ala Tyr Met Gln Leu Ser Ser Leu
        195                 200                 205
Thr Ser Glu Asp Thr Ala Val Tyr Phe Cys Ala Arg Ser Ala Tyr Tyr
    210                 215                 220
Gly Asn Tyr Val Ala Met Asp Tyr Trp Gly Gln Gly Thr Ser Val Thr
225                 230                 235                 240
Val Ser Ser Ser Glu Pro Lys Ser Ser Asp Lys Thr His Thr Cys Pro
                245                 250                 255
Pro Cys Pro Ser Gly Ala Pro Glu Leu Leu Gly Gly Pro Ser Val Phe
            260                 265                 270
Leu Phe Pro Pro Lys Pro Lys Asp Thr Leu Met Ile Ser Arg Thr Pro
        275                 280                 285
Glu Val Thr Cys Val Val Val Asp Val Ser His Glu Asp Pro Glu Val
    290                 295                 300
Lys Phe Asn Trp Tyr Val Asp Gly Val Glu Val His Asn Ala Lys Thr
305                 310                 315                 320
Lys Pro Arg Glu Glu Gln Tyr Asn Ser Thr Tyr Arg Val Val Ser Val
                325                 330                 335
Leu Thr Val Leu His Gln Asp Trp Leu Asn Gly Lys Glu Tyr Lys Cys
            340                 345                 350
Lys Val Ser Asn Lys Ala Leu Pro Ala Pro Ile Glu Lys Thr Ile Ser
        355                 360                 365
Lys Ala Lys Gly Gln Pro Arg Glu Pro Gln Val Tyr Thr Leu Pro Pro
    370                 375                 380
Ser Arg Asp Glu Leu Thr Lys Asn Gln Val Ser Leu Thr Cys Leu Val
385                 390                 395                 400
Lys Gly Phe Tyr Pro Ser Asp Ile Ala Val Glu Trp Glu Ser Asn Gly
                405                 410                 415
Gln Pro Glu Asn Asn Tyr Lys Thr Thr Pro Pro Val Leu Asp Ser Asp
            420                 425                 430
Gly Ser Phe Phe Leu Tyr Ser Lys Leu Thr Val Asp Lys Ser Arg Trp
        435                 440                 445
Gln Gln Gly Asn Val Phe Ser Cys Ser Val Met His Glu Ala Leu His
    450                 455                 460
Asn His Tyr Thr Gln Lys Ser Leu Ser Leu Ser Pro Gly Lys Ser Arg
465                 470                 475                 480
Ala Ser Thr Lys Gly Pro Ser Val Phe Pro Leu Ala Pro Ser Ser Lys
                485                 490                 495
Ser Thr Ser Gly Gly Thr Ala Ala Leu Gly Cys Leu Val Lys Asp Tyr
            500                 505                 510
Phe Pro Glu Pro Val Thr Val Ser Trp Asn Ser Gly Ala Leu Thr Ser

515                 520                 525
Gly Val His Thr Phe Pro Ala Val Leu Gln Ser Ser Gly Leu Tyr Ser
    530                 535                 540
Leu Ser Ser Val Val Thr Val Pro Ser Ser Ser Leu Gly Thr Gln Thr
545                 550                 555                 560
Tyr Ile Cys Asn Val Asn His Lys Pro Ser Asn Thr Lys Val Asp Lys
                565                 570                 575
Lys Val

<210> 31
<211> 585
<212> PRT
<213> Artificial Sequence

<220>
<223> ORN146 mature protein comprising : 4C04 (anti-RON)
      scFv, human IgG1 SCC-P hinge, human CH2, human
      CH3, and human Ck(YAE)

<400> 31
Asp Ile Gln Met Thr Gln Ser Pro Ala Ser Gln Ser Ala Ser Leu Gly
1               5                   10                  15
Glu Ser Val Thr Ile Thr Cys Leu Ala Ser Gln Thr Ile Gly Thr Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Ala
    50                  55                  60
Ser Gly Ser Gly Thr Lys Phe Ser Phe Lys Ile Asp Ser Leu Gln Ser
65                  70                  75                  80
Glu Asp Phe Val Ser Tyr Tyr Cys Gln Gln Leu Tyr Asn Thr Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Ile Gln Leu Gln Gln
        115                 120                 125
Ser Gly Pro Glu Leu Val Lys Pro Gly Thr Ser Val Lys Ile Ser Cys
    130                 135                 140
Lys Ala Ser Asp Tyr Thr Leu Thr Asp Phe Tyr Met Asn Trp Val Arg
145                 150                 155                 160
Gln Lys Pro Gly Gln Gly Leu Glu Trp Ile Gly Arg Ile Tyr Pro Gly
                165                 170                 175
Thr Asp Lys Thr Arg Tyr Asn Glu Lys Phe Arg Asp Lys Ala Ile Leu
            180                 185                 190
Thr Val Asp Thr Ser Ser Ser Thr Ala Tyr Met Gln Leu Ser Ser Leu
        195                 200                 205
Thr Ser Glu Asp Thr Ala Val Tyr Phe Cys Ala Arg Ser Ala Tyr Tyr
    210                 215                 220
Gly Asn Tyr Val Ala Met Asp Tyr Trp Gly Gln Gly Thr Ser Val Thr
225                 230                 235                 240
Val Ser Ser Ser Glu Pro Lys Ser Ser Asp Lys Thr His Thr Cys Pro
                245                 250                 255
Pro Cys Pro Ser Gly Ala Pro Glu Leu Leu Gly Gly Pro Ser Val Phe
            260                 265                 270
Leu Phe Pro Pro Lys Pro Lys Asp Thr Leu Met Ile Ser Arg Thr Pro
        275                 280                 285
Glu Val Thr Cys Val Val Val Asp Val Ser His Glu Asp Pro Glu Val
    290                 295                 300
Lys Phe Asn Trp Tyr Val Asp Gly Val Glu Val His Asn Ala Lys Thr
305                 310                 315                 320
Lys Pro Arg Glu Glu Gln Tyr Asn Ser Thr Tyr Arg Val Val Ser Val

325                 330                 335
Leu Thr Val Leu His Gln Asp Trp Leu Asn Gly Lys Glu Tyr Lys Cys
            340                 345                 350
Lys Val Ser Asn Lys Ala Leu Pro Ala Pro Ile Glu Lys Thr Ile Ser
        355                 360                 365
Lys Ala Lys Gly Gln Pro Arg Glu Pro Gln Val Tyr Thr Leu Pro Pro
    370                 375                 380
Ser Arg Asp Glu Leu Thr Lys Asn Gln Val Ser Leu Thr Cys Leu Val
385                 390                 395                 400
Lys Gly Phe Tyr Pro Ser Asp Ile Ala Val Glu Trp Glu Ser Asn Gly
                405                 410                 415
Gln Pro Glu Asn Asn Tyr Lys Thr Thr Pro Pro Val Leu Asp Ser Asp
            420                 425                 430
Gly Ser Phe Phe Leu Tyr Ser Lys Leu Thr Val Asp Lys Ser Arg Trp
        435                 440                 445
Gln Gln Gly Asn Val Phe Ser Cys Ser Val Met His Glu Ala Leu His
    450                 455                 460
Asn His Tyr Thr Gln Lys Ser Leu Ser Leu Ser Pro Gly Lys Ser Arg
465                 470                 475                 480
Thr Val Ala Ala Pro Ser Val Phe Ile Phe Pro Pro Ser Asp Glu Gln
                485                 490                 495
Leu Lys Ser Gly Thr Ala Ser Val Val Cys Leu Leu Asn Tyr Phe Tyr
            500                 505                 510
Pro Arg Glu Ala Lys Val Gln Trp Lys Val Asp Asn Ala Leu Gln Ser
        515                 520                 525
Gly Asn Ser Gln Glu Ser Ala Thr Glu Gln Asp Ser Lys Asp Ser Thr
    530                 535                 540
Tyr Ser Leu Ser Ser Glu Leu Thr Leu Ser Lys Ala Asp Tyr Glu Lys
545                 550                 555                 560
His Lys Val Tyr Ala Cys Glu Val Thr His Gln Gly Leu Ser Ser Pro
                565                 570                 575
Val Thr Lys Ser Phe Asn Arg Gly Glu
            580                 585


<210> 32
<211> 581
<212> PRT
<213> Artificial Sequence

<220>
<223> ORN148 mature protein comprising : 11H09
      (anti-RON) scFv, human IgG1 SCC-P hinge, human
      CH2, human CH3, and human Ck(YAE)

<400> 32
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Ser Ser
            20                  25                  30
Leu Ser Trp Leu Gln Gln Glu Pro Asp Gly Thr Ile Lys Arg Leu Ile
        35                  40                  45
Tyr Ser Thr Ser Ser Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Ser Ser Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Val Gln Leu Gln Gln
        115                 120                 125
Ser Gly Ala Glu Leu Ala Lys Pro Gly Ala Ser Val Lys Met Ser Cys

130                 135                 140
Lys Ser Ser Gly Tyr Thr Phe Thr Ser Tyr Trp Ile His Trp Val Lys
145                 150                 155                 160
Glu Arg Pro Gly Gln Gly Leu Glu Trp Ile Gly Tyr Ile Tyr Pro Thr
                165                 170                 175
Thr Gly Tyr Thr Glu Ser Asn Gln Lys Phe Lys Asp Lys Ala Thr Leu
            180                 185                 190
Thr Ala Asp Lys Ser Ser Ser Thr Ala Tyr Met Gln Leu Ser Ser Leu
        195                 200                 205
Thr Ser Glu Asp Ser Ala Val Tyr Tyr Cys Phe Leu Leu Arg Pro Trp
    210                 215                 220
Tyr Phe Asp Val Trp Gly Ala Gly Thr Thr Val Thr Val Ser Ser Ser
225                 230                 235                 240
Glu Pro Lys Ser Ser Asp Lys Thr His Thr Cys Pro Pro Cys Pro Ser
                245                 250                 255
Gly Ala Pro Glu Leu Leu Gly Gly Pro Ser Val Phe Leu Phe Pro Pro
            260                 265                 270
Lys Pro Lys Asp Thr Leu Met Ile Ser Arg Thr Pro Glu Val Thr Cys
        275                 280                 285
Val Val Val Asp Val Ser His Glu Asp Pro Glu Val Lys Phe Asn Trp
    290                 295                 300
Tyr Val Asp Gly Val Glu Val His Asn Ala Lys Thr Lys Pro Arg Glu
305                 310                 315                 320
Glu Gln Tyr Asn Ser Thr Tyr Arg Val Val Ser Val Leu Thr Val Leu
                325                 330                 335
His Gln Asp Trp Leu Asn Gly Lys Glu Tyr Lys Cys Lys Val Ser Asn
            340                 345                 350
Lys Ala Leu Pro Ala Pro Ile Glu Lys Thr Ile Ser Lys Ala Lys Gly
        355                 360                 365
Gln Pro Arg Glu Pro Gln Val Tyr Thr Leu Pro Pro Ser Arg Asp Glu
    370                 375                 380
Leu Thr Lys Asn Gln Val Ser Leu Thr Cys Leu Val Lys Gly Phe Tyr
385                 390                 395                 400
Pro Ser Asp Ile Ala Val Glu Trp Glu Ser Asn Gly Gln Pro Glu Asn
                405                 410                 415
Asn Tyr Lys Thr Thr Pro Pro Val Leu Asp Ser Asp Gly Ser Phe Phe
            420                 425                 430
Leu Tyr Ser Lys Leu Thr Val Asp Lys Ser Arg Trp Gln Gln Gly Asn
        435                 440                 445
Val Phe Ser Cys Ser Val Met His Glu Ala Leu His Asn His Tyr Thr
    450                 455                 460
Gln Lys Ser Leu Ser Leu Ser Pro Gly Lys Ser Arg Thr Val Ala Ala
465                 470                 475                 480
Pro Ser Val Phe Ile Phe Pro Pro Ser Asp Glu Gln Leu Lys Ser Gly
                485                 490                 495
Thr Ala Ser Val Val Cys Leu Leu Asn Tyr Phe Tyr Pro Arg Glu Ala
            500                 505                 510
Lys Val Gln Trp Lys Val Asp Asn Ala Leu Gln Ser Gly Asn Ser Gln
        515                 520                 525
Glu Ser Ala Thr Glu Gln Asp Ser Lys Asp Ser Thr Tyr Ser Leu Ser
    530                 535                 540
Ser Glu Leu Thr Leu Ser Lys Ala Asp Tyr Glu Lys His Lys Val Tyr
545                 550                 555                 560
Ala Cys Glu Val Thr His Gln Gly Leu Ser Ser Pro Val Thr Lys Ser
                565                 570                 575
Phe Asn Arg Gly Glu
            580


<210> 33
<211> 1809
<212> DNA
<213> Artificial Sequence

<220>
<223> Nucleotide sequence for ORN160 comprising : 5D5
      (anti-c-Met) scFv, human IgG1 SCC-P hinge, human
      IgG1 CH2, human IgG1 CH3, and human CH1

<400> 33
atggaagcac cagcgcagct tctcttcctc ctgctactct ggctcccaga taccaccggt 60
gactttatga tgtcacagtc tccatcctcc ctaactgtgt cagttggaga gaaggttact 120
gtgagctgca agtccagtca gtccctttta tatactagca gtcagaagaa ctacttggcc 180
tggtaccagc agaaaccagg tcagtctcct aaactgctga tttactgggc atccactagg 240
gaatctgggg tccctgatcg cttcacaggc agtggatctg ggacagattt cactctcacc 300
atcaccagtg tgaaggctga cgacctggca gtttattact gtcagcaata ttatgcctat 360
ccgtggacgt tcggtggagg caccaagttg gagctcaaag gtggcggtgg ctctggcgga 420
ggtggatccg gtggcggcgg ctctcaggtc caactgcagc agtctgggcc tgagctggtg 480
aggcctgggg cttcagtgaa gatgtcctgc agggcttcgg gctatacctt caccagctac 540
tggttgcact gggttaaaca gaggcctgga caaggccttg agtggattgg catgattgat 600
ccttccaata gtgacactag gtttaatccg aacttcaagg acaaggccac attgaatgta 660
gacagatctt ccaacacagc ctacatgctg ctcagcagcc tgacatctgc tgactctgca 720
gtctattact gtgccacata tggtagctac gtttcccctc tggactactg gggtcaagga 780
acctcagtca ccgtctcctc gagcgagccc aaatcttctg acaaaactca cacatgccca 840
ccgtgcccat ccggagcacc tgaactcctg ggtggaccgt cagtcttcct cttcccccca 900
aaacccaagg acaccctcat gatctcccgg acccctgagg tcacatgcgt ggtggtggac 960
gtgagccacg aagaccctga ggtcaagttc aactggtacg tggacggcgt ggaggtgcat 1020
aatgccaaga caaagccgcg ggaggagcag tacaacagca cgtaccgtgt ggtcagcgtc 1080
ctcaccgtcc tgcaccagga ctggctgaat ggcaaggagt acaagtgcaa ggtctccaac 1140
aaagccctcc cagcccccat cgagaaaacc atctccaaag ccaaagggca gccccgagaa 1200
ccacaggtgt acaccctgcc cccatcccgg gatgagctga ccaagaacca ggtcagcctg 1260
acctgcctgg tcaaaggctt ctatccaagc gacatcgccg tggagtggga gagcaatggg 1320
cagccggaga acaactacaa gaccacgcct cccgtgctgg actccgacgg ctccttcttc 1380
ctctacagca agctcaccgt ggacaagagc aggtggcagc aggggaacgt cttctcatgc 1440
tccgtgatgc atgaggctct gcacaaccac tacacgcaga agagcctctc cctgtctccg 1500
ggtaaatcta gagcctccac caagggccca tcggtcttcc ccctggcacc ctcctccaag 1560
agcacctctg ggggcacagc ggccctgggc tgcctggtca aggactactt ccccgagccg 1620
gtgacggtgt cgtggaactc aggcgccctg accagcggcg tgcacacctt cccggctgtc 1680
ctacagtcct caggactcta ctccctcagc agcgtggtga ccgtgccctc cagcagcttg 1740
ggcacccaga cctacatctg caacgtgaat cacaagccca gcaacaccaa ggtggacaag 1800
aaagtttga                                                         1809

<210> 34
<211> 1821
<212> DNA
<213> Artificial Sequence

<220>
<223> Nucleotide sequence for TSC001 comprising : HD37
      (anti-CD39), human IgG1 SCC-P hinge, human IgG1
      CH2, human IgG1 CH3 and human CH1

<400> 34
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780

tggggtcaag gaacctcagt caccgtctcc tcgagcgagc ccaaatcttc tgacaaaact 840
cacacatgcc caccgtgccc atccggagca cctgaactcc tgggtggacc gtcagtcttc 900
ctcttccccc caaaacccaa ggacaccctc atgatctccc ggacccctga ggtcacatgc 960
gtggtggtgg acgtgagcca cgaagaccct gaggtcaagt tcaactggta cgtggacggc 1020
gtggaggtgc ataatgccaa gacaaagccg cgggaggagc agtacaacag cacgtaccgt 1080
gtggtcagcg tcctcaccgt cctgcaccag gactggctga atggcaagga gtacaagtgc 1140
aaggtctcca acaaagccct cccagccccc atcgagaaaa ccatctccaa agccaaaggg 1200
cagccccgag aaccacaggt gtacaccctg cccccatccc gggatgagct gaccaagaac 1260
caggtcagcc tgacctgcct ggtcaaaggc ttctatccaa gcgacatcgc cgtggagtgg 1320
gagagcaatg ggcagccgga gaacaactac aagaccacgc ctcccgtgct ggactccgac 1380
ggctccttct tcctctacag caagctcacc gtggacaaga gcaggtggca gcaggggaac 1440
gtcttctcat gctccgtgat gcatgaggct ctgcacaacc actacacgca gaagagcctc 1500
tccctgtctc cgggtaaatc tagagcctcc accaagggcc catcggtctt ccccctggca 1560
ccctcctcca agagcacctc tgggggcaca gcggccctgg gctgcctggt caaggactac 1620
ttccccgagc cggtgacggt gtcgtggaac tcaggcgccc tgaccagcgg cgtgcacacc 1680
ttcccggctg tcctacagtc ctcaggactc tactccctca gcagcgtggt gaccgtgccc 1740
tccagcagct tgggcaccca gacctacatc tgcaacgtga atcacaagcc cagcaacacc 1800
aaggtggaca agaaagtttg a 1821

<210> 35
<211> 1800
<212> DNA
<213> Artificial Sequence

<220>
<223> Nucleotide sequence for TSC002 comprising : 2H7
      (anti-CD20) scFv, human IgG1 SCC-P hinge, human
      IgG1 CH2, human IgG1 CH3 and human CH1

<400> 35
atggaagcac cagcgcagct tctcttcctc ctgctactct ggctcccaga taccaccggt 60
caaattgttc tctcccagtc tccagcaatc ctgtctgcat ctccagggga gaaggtcaca 120
atgacttgca gggccagctc aagtgtaagt tacatgcact ggtaccagca gaagccagga 180
tcctccccca aaccctggat ttatgcccca tccaacctgg cttctggagt ccctgctcgc 240
ttcagtggca gtgggtctgg gacctcttac tctctcacaa tcagcagagt ggaggctgaa 300
gatgctgcca cttattactg ccagcagtgg agttttaacc cacccacgtt cggtgctggg 360
accaagctgg agctgaaaga tggcggtggc tcgggcggtg gtggatctgg aggaggtggg 420
gctagccagg cttatctaca gcagtctggg gctgagtcgg tgaggcctgg ggcctcagtg 480
aagatgtcct gcaaggcttc tggctacaca tttaccagtt acaatatgca ctgggtaaag 540
cagacaccta gacagggcct ggaatggatt ggagctattt atccaggaaa tggtgatact 600
tcctacaatc agaagttcaa gggcaaggcc acactgactg tagacaaatc ctccagcaca 660
gcctacatgc agctcagcag cctgacatct gaagactctg cggtctattt ctgtgcaaga 720
gtggtgtact atagtaactc ttactggtac ttcgatgtct ggggcacagg gaccacggtc 780
accgtctcct cgagcgagcc caaatcttct gacaaaactc acacatgccc accgtgccca 840
tccggagcac ctgaactcct gggtggaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggag tacaagtgca aggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agagcctcca ccaagggccc atcggtcttc cccctggcac cctcctccaa gagcacctct 1560
gggggcacag cggccctggg ctgcctggtc aaggactact tccccgagcc ggtgacggtg 1620
tcgtggaact caggcgccct gaccagcggc gtgcacacct tcccggctgt cctacagtcc 1680
tcaggactct actccctcag cagcgtggtg accgtgccct ccagcagctt gggcacccag 1740
acctacatct gcaacgtgaa tcacaagccc agcaacacca aggtggacaa gaaagtttga 1800

<210> 36

<211> 1851
<212> DNA
<213> Artificial Sequence

<220>
<223> Nucleotide sequence for TSC003 comprising : G19-4
      (anti-CD3) scFv, altered human IgA1 hinge,  human
      IgG1 CH2, human IgG1 CH3, and human Ck(YAE)

<400> 36
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcccc tcaactccac ctaccccatc tccctcaact 840
ccacctaccc catctccctc atgcccaccg tgcccagcac ctgaactcct gggtggaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacaacagc 1080
acgtaccgtg tggtcagcgt cctcaccgtc ctgcaccagg actggctgaa tggcaaggag 1140
tacaagtgca aggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaaatct agaactgtgg ctgcaccatc tgtcttcatc 1560
ttcccgccat ctgatgagca gttgaaatct ggaactgcct ctgttgtgtg cctgctgaat 1620
tacttctatc ccagagaggc caaagtacag tggaaggtgg ataacgccct ccaatcgggt 1680
aactcccagg agagtgccac agagcaggac agcaaggaca gcacctacag cctcagcagc 1740
gagctgacgc tgagcaaagc agactacgag aaacacaaag tctacgcctg cgaagtcacc 1800
catcagggcc tgagctcgcc cgtcacaaag agcttcaaca ggggagagtg a         1851

<210> 37
<211> 1812
<212> DNA
<213> Artificial Sequence

<220>
<223> Nucleotide sequence for TSC004 comprising : G19-4
      (anti-CD3) scFv, altered human IgA2 hinge, human
      IgG1 CH2, human IgG1 CH3, and human Ck(YAE)

<400> 37
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600

ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcccc ccacctcccc catgcccacc gtgcccagca 840
cctgaactcc tgggtggacc gtcagtcttc ctcttccccc caaaacccaa ggacaccctc 900
atgatctccc ggacccctga ggtcacatgc gtggtggtgg acgtgagcca cgaagaccct 960
gaggtcaagt tcaactggta cgtggacggc gtggaggtgc ataatgccaa gacaaagccg 1020
cgggaggagc agtacaacag cacgtaccgt gtggtcagcg tcctcaccgt cctgcaccag 1080
gactggctga atggcaagga gtacaagtgc aaggtctcca acaaagccct cccagccccc 1140
atcgagaaaa ccatctccaa agccaaaggg cagccccgag aaccacaggt gtacaccctg 1200
cccccatccc gggatgagct gaccaagaac caggtcagcc tgacctgcct ggtcaaaggc 1260
ttctatccaa gcgacatcgc cgtggagtgg gagagcaatg ggcagccgga gaacaactac 1320
aagaccacgc ctcccgtgct ggactccgac ggctccttct tcctctacag caagctcacc 1380
gtggacaaga gcaggtggca gcaggggaac gtcttctcat gctccgtgat gcatgaggct 1440
ctgcacaacc actacacgca gaagagcctc tccctgtctc cgggtaaatc tagaactgtg 1500
gctgcaccat ctgtcttcat cttcccgcca tctgatgagc agttgaaatc tggaactgcc 1560
tctgttgtgt gcctgctgaa ttacttctat cccagagagg ccaaagtaca gtggaaggtg 1620
gataacgccc tccaatcggg taactcccag gagagtgcca cagagcagga cagcaaggac 1680
agcacctaca gcctcagcag cgagctgacg ctgagcaaag cagactacga gaaacacaaa 1740
gtctacgcct gcgaagtcac ccatcagggc ctgagctcgc ccgtcacaaa gagcttcaac 1800
aggggagagt ga 1812

<210> 38
<211> 1842
<212> DNA
<213> Artificial Sequence

<220>
<223> Nucleotide sequence for TSC005 comprising : G19-4
      (anti-CD3) scFv, altered human IgG3 hinge, human
      IgG1 CH2, human IgG1 CH3, and human Ck(YAE)

<400> 38
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcgag cccaaatcta gcgacacacc tcccccaagc 840
ccacggtccc catgcccacc gtgcccagca cctgaactcc tgggtggacc gtcagtcttc 900
ctcttccccc caaaacccaa ggacaccctc atgatctccc ggacccctga ggtcacatgc 960
gtggtggtgg acgtgagcca cgaagaccct gaggtcaagt tcaactggta cgtggacggc 1020
gtggaggtgc ataatgccaa gacaaagccg cgggaggagc agtacaacag cacgtaccgt 1080
gtggtcagcg tcctcaccgt cctgcaccag gactggctga atggcaagga gtacaagtgc 1140
aaggtctcca acaaagccct cccagccccc atcgagaaaa ccatctccaa agccaaaggg 1200
cagccccgag aaccacaggt gtacaccctg cccccatccc gggatgagct gaccaagaac 1260
caggtcagcc tgacctgcct ggtcaaaggc ttctatccaa gcgacatcgc cgtggagtgg 1320
gagagcaatg ggcagccgga gaacaactac aagaccacgc ctcccgtgct ggactccgac 1380
ggctccttct tcctctacag caagctcacc gtggacaaga gcaggtggca gcaggggaac 1440
gtcttctcat gctccgtgat gcatgaggct ctgcacaacc actacacgca gaagagcctc 1500
tccctgtctc cgggtaaatc tagaactgtg gctgcaccat ctgtcttcat cttcccgcca 1560
tctgatgagc agttgaaatc tggaactgcc tctgttgtgt gcctgctgaa ttacttctat 1620
cccagagagg ccaaagtaca gtggaaggtg gataacgccc tccaatcggg taactcccag 1680
gagagtgcca cagagcagga cagcaaggac agcacctaca gcctcagcag cgagctgacg 1740
ctgagcaaag cagactacga gaaacacaaa gtctacgcct gcgaagtcac ccatcagggc 1800

ctgagctcgc ccgtcacaaa gagcttcaac aggggagagt ga 1842

<210> 39
<211> 1899
<212> DNA
<213> Artificial Sequence

<220>
<223> Nucleotide sequence for TSC006 comprising : G19-4
      (anti-CD3) scFv, altered human IgD hinge, human
      IgG1 CH2, human IgG1 CH3, and human Ck(YAE)

<400> 39
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcgag tctccaaagg cacaggcctc ctccgtgccc 840
actgcacaac cccaagcaga gggcagcctc gccaaggcaa ccacagcccc agccaccacc 900
cgtaacacat gcccaccgtg cccagcacct gaactcctgg gtggaccgtc agtcttcctc 960
ttccccccaa aacccaagga caccctcatg atctcccgga cccctgaggt cacatgcgtg 1020
gtggtggacg tgagccacga agaccctgag gtcaagttca actggtacgt ggacggcgtg 1080
gaggtgcata atgccaagac aaagccgcgg gaggagcagt acaacagcac gtaccgtgtg 1140
gtcagcgtcc tcaccgtcct gcaccaggac tggctgaatg gcaaggagta caagtgcaag 1200
gtctccaaca aagccctccc agcccccatc gagaaaacca tctccaaagc caaagggcag 1260
ccccgagaac cacaggtgta caccctgccc ccatcccggg atgagctgac caagaaccag 1320
gtcagcctga cctgcctggt caaaggcttc tatccaagcg acatcgccgt ggagtgggag 1380
agcaatgggc agccggagaa caactacaag accacgcctc ccgtgctgga ctccgacggc 1440
tccttcttcc tctacagcaa gctcaccgtg gacaagagca ggtggcagca ggggaacgtc 1500
ttctcatgct ccgtgatgca tgaggctctg cacaaccact acacgcagaa gagcctctcc 1560
ctgtctccgg gtaaatctag aactgtggct gcaccatctg tcttcatctt cccgccatct 1620
gatgagcagt tgaaatctgg aactgcctct gttgtgtgcc tgctgaatta cttctatccc 1680
agagaggcca aagtacagtg gaaggtggat aacgccctcc aatcgggtaa ctcccaggag 1740
agtgccacag agcaggacag caaggacagc acctacagcc tcagcagcga gctgacgctg 1800
agcaaagcag actacgagaa acacaaagtc tacgcctgcg aagtcaccca tcagggcctg 1860
agctcgcccg tcacaaagag cttcaacagg ggagagtga 1899

<210> 40
<211> 1842
<212> DNA
<213> Artificial Sequence

<220>
<223> Nucleotide sequence for TSC007 comprising : G19-4
      (anti-CD3) scFv, altered human IgG1 hinge, human
      IgG1 CH2, human IgG1 CH3, and human Ck(YAE)

<400> 40
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360

tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcgag cccaaatctt ctgacaaaac tcacacaagc 840
ccaccgagcc catgcccacc gtgcccagca cctgaactcc tgggtggacc gtcagtcttc 900
ctcttccccc caaaacccaa ggacaccctc atgatctccc ggacccctga ggtcacatgc 960
gtggtggtgg acgtgagcca cgaagaccct gaggtcaagt tcaactggta cgtggacggc 1020
gtggaggtgc ataatgccaa gacaaagccg cgggaggagc agtacaacag cacgtaccgt 1080
gtggtcagcg tcctcaccgt cctgcaccag gactggctga atggcaagga gtacaagtgc 1140
aaggtctcca acaaagccct cccagccccc atcgagaaaa ccatctccaa agccaaaggg 1200
cagccccgag aaccacaggt gtacaccctg cccccatccc gggatgagct gaccaagaac 1260
caggtcagcc tgacctgcct ggtcaaaggc ttctatccaa gcgacatcgc cgtggagtgg 1320
gagagcaatg ggcagccgga gaacaactac aagaccacgc ctcccgtgct ggactccgac 1380
ggctccttct tcctctacag caagctcacc gtggacaaga gcaggtggca gcaggggaac 1440
gtcttctcat gctccgtgat gcatgaggct ctgcacaacc actacacgca gaagagcctc 1500
tccctgtctc cgggtaaatc tagaactgtg gctgcaccat ctgtcttcat cttcccgcca 1560
tctgatgagc agttgaaatc tggaactgcc tctgttgtgt gcctgctgaa ttacttctat 1620
cccagagagg ccaaagtaca gtggaaggtg gataacgccc tccaatcggg taactcccag 1680
gagagtgcca cagagcagga cagcaaggac agcacctaca gcctcagcag cgagctgacg 1740
ctgagcaaag cagactacga gaaacacaaa gtctacgcct gcgaagtcac ccatcagggc 1800
ctgagctcgc ccgtcacaaa gagcttcaac aggggagagt ga 1842

<210> 41
<211> 2115
<212> DNA
<213> Artificial Sequence

<220>
<223> Nucleotide sequence for TSC008 comprising : G19-4
      (anti-CD3) scFv, altered human IgE CH2 hinge,
      human IgG1 CH2, human IgG1 CH3, and human Ck(YAE)

<400> 41
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagctcc agggacttca ccccgcccac cgtgaagatc 840
ttacagtcgt ccagcgacgg cggcgggcac ttccccccga ccatccagct cctgtgcctc 900
gtctctgggt acaccccagg gactatcaac atcacctggc tggaggacgg gcaggtcatg 960
gacgtggact tgtccaccgc ctctaccacg caggagggtg agctggcctc cacacaaagc 1020
gagctcaccc tcagccagaa gcactggctg tcagaccgca cctacacctg ccaggtcacc 1080
tatcaaggtc acacctttga ggacagcacc aagaagtctg catgcccacc gtgctccgga 1140
gcacctgaac tcctgggtgg accgtcagtc ttcctcttcc ccccaaaacc caaggacacc 1200
ctcatgatct cccggacccc tgaggtcaca tgcgtggtgg tggacgtgag ccacgaagac 1260
cctgaggtca agttcaactg gtacgtggac ggcgtggagg tgcataatgc caagacaaag 1320
ccgcgggagg agcagtacaa cagcacgtac cgtgtggtca gcgtcctcac cgtcctgcac 1380
caggactggc tgaatggcaa ggagtacaag tgcaaggtct ccaacaaagc cctcccagcc 1440
cccatcgaga aaaccatctc caaagccaaa gggcagcccc gagaaccaca ggtgtacacc 1500
ctgcccccat cccgggatga gctgaccaag aaccaggtca gcctgacctg cctggtcaaa 1560

ggcttctatc caagcgacat cgccgtggag tgggagagca atgggcagcc ggagaacaac 1620
tacaagacca cgcctcccgt gctggactcc gacggctcct tcttcctcta cagcaagctc 1680
accgtggaca agagcaggtg gcagcagggg aacgtcttct catgctccgt gatgcatgag 1740
gctctgcaca accactacac gcagaagagc ctctccctgt ctccgggtaa atctagaact 1800
gtggctgcac catctgtctt catcttcccg ccatctgatg agcagttgaa atctggaact 1860
gcctctgttg tgtgcctgct gaattacttc tatcccagag aggccaaagt acagtggaag 1920
gtggataacg ccctccaatc gggtaactcc caggagagtg ccacagagca ggacagcaag 1980
gacagcacct acagcctcag cagcgagctg acgctgagca aagcagacta cgagaaacac 2040
aaagtctacg cctgcgaagt cacccatcag ggcctgagct cgcccgtcac aaagagcttc 2100
aacaggggag agtga 2115

<210> 42
<211> 2136
<212> DNA
<213> Artificial Sequence

<220>
<223> Nucleotide sequence for TSC009 comprising : G19-4
      (anti-CD3) scFv, altered human IgM CH2 hinge,
      human IgG1 CH2, human IgG1 CH3, and human Ck(YAE)

<400> 42
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcatt gccgagctgc ctcccaaagt gagcgtcttc 840
gtcccacccc gcgacggctt cttcggcaac ccccgcaagt ccaagctcat ctgccaggcc 900
acgggtttca gtccccggca gattcaggtg tcctggctgc gcgaggggaa gcaggtgggg 960
tctggcgtca ccacggacca ggtgcaggct gaggccaaag agtctgggcc cacgacctac 1020
aaggtgacca gcacactgac catcaaagag agcgactggc tcagccagag catgttcacc 1080
tgccgcgtgg atcacagggg cctgaccttc cagcagaatg cgtcctccat gagtgtcccc 1140
tgcccaccgt gcccatccgg agcacctgaa ctcctgggtg gaccgtcagt cttcctcttc 1200
cccccaaaac ccaaggacac cctcatgatc tcccggaccc ctgaggtcac atgcgtggtg 1260
gtggacgtga gccacgaaga ccctgaggtc aagttcaact ggtacgtgga cggcgtggag 1320
gtgcataatg ccaagacaaa gccgcgggag gagcagtaca acagcacgta ccgtgtggtc 1380
agcgtcctca ccgtcctgca ccaggactgg ctgaatggca aggagtacaa gtgcaaggtc 1440
tccaacaaag ccctcccagc ccccatcgag aaaaccatct ccaaagccaa agggcagccc 1500
cgagaaccac aggtgtacac cctgccccca tcccgggatg agctgaccaa gaaccaggtc 1560
agcctgacct gcctggtcaa aggcttctat ccaagcgaca tcgccgtgga gtgggagagc 1620
aatgggcagc cggagaacaa ctacaagacc acgcctcccg tgctggactc cgacggctcc 1680
ttcttcctct acagcaagct caccgtggac aagagcaggt ggcagcaggg gaacgtcttc 1740
tcatgctccg tgatgcatga ggctctgcac aaccactaca cgcagaagag cctctccctg 1800
tctccgggta aatctagaac tgtggctgca ccatctgtct tcatcttccc gccatctgat 1860
gagcagttga aatctggaac tgcctctgtt gtgtgcctgc tgaattactt ctatcccaga 1920
gaggccaaag tacagtggaa ggtggataac gccctccaat cgggtaactc ccaggagagt 1980
gccacagagc aggacagcaa ggacagcacc tacagcctca gcagcgagct gacgctgagc 2040
aaagcagact acgagaaaca caaagtctac gcctgcgaag tcacccatca gggcctgagc 2100
tcgcccgtca caaagagctt caacagggga gagtga 2136

<210> 43
<211> 1839
<212> DNA
<213> Artificial Sequence

<220>
<223> Nucleotide sequence for TSC010 comprising : HD37
      (anti-CD19) scFv, altered human IgA1 hinge, human
      IgG1 CH2, human IgG1 CH3, and human CH1

<400> 43
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcccct caactccacc taccccatct 840
ccctcaactc cacctacccc atctccctca tgcccaccgt gcccagcacc tgaactcctg 900
ggtggaccgt cagtcttcct cttcccccca aaacccaagg acaccctcat gatctcccgg 960
acccctgagg tcacatgcgt ggtggtggac gtgagccacg aagaccctga ggtcaagttc 1020
aactggtacg tggacggcgt ggaggtgcat aatgccaaga caaagccgcg ggaggagcag 1080
tacaacagca cgtaccgtgt ggtcagcgtc ctcaccgtcc tgcaccagga ctggctgaat 1140
ggcaaggagt acaagtgcaa ggtctccaac aaagccctcc cagcccccat cgagaaaacc 1200
atctccaaag ccaaagggca gccccgagaa ccacaggtgt acaccctgcc cccatcccgg 1260
gatgagctga ccaagaacca ggtcagcctg acctgcctgg tcaaaggctt ctatccaagc 1320
gacatcgccg tggagtggga gagcaatggg cagccggaga acaactacaa gaccacgcct 1380
cccgtgctgg actccgacgg ctccttcttc ctctacagca agctcaccgt ggacaagagc 1440
aggtggcagc aggggaacgt cttctcatgc tccgtgatgc atgaggctct gcacaaccac 1500
tacacgcaga agagcctctc cctgtctccg ggtaaatcta gagcctccac caagggccca 1560
tcggtcttcc ccctggcacc ctcctccaag agcacctctg ggggcacagc ggccctgggc 1620
tgcctggtca aggactactt ccccgagccg gtgacggtgt cgtggaactc aggcgccctg 1680
accagcggcg tgcacacctt cccggctgtc ctacagtcct caggactcta ctccctcagc 1740
agcgtggtga ccgtgccctc cagcagcttg ggcacccaga cctacatctg caacgtgaat 1800
cacaagccca gcaacaccaa ggtggacaag aaagtttga 1839

<210> 44
<211> 1800
<212> DNA
<213> Artificial Sequence

<220>
<223> Nucleotide sequence for TSC011 comprising : HD37
      (anti-CD19) scFv, altered human IgA2 hinge, human
      IgG1 CH2, human IgG1 CH3, and human CH1

<400> 44
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780

tggggtcaag gaacctcagt caccgtctcc tcgagccccc cacctccccc atgcccaccg 840
tgcccagcac ctgaactcct gggtggaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggag tacaagtgca aggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agagcctcca ccaagggccc atcggtcttc cccctggcac cctcctccaa gagcacctct 1560
gggggcacag cggccctggg ctgcctggtc aaggactact tccccgagcc ggtgacggtg 1620
tcgtggaact caggcgccct gaccagcggc gtgcacacct tcccggctgt cctacagtcc 1680
tcaggactct actccctcag cagcgtggtg accgtgccct ccagcagctt gggcacccag 1740
acctacatct gcaacgtgaa tcacaagccc agcaacacca aggtggacaa gaaagtttga 1800

<210> 45
<211> 1830
<212> DNA
<213> Artificial Sequence

<220>
<223> Nucleotide sequence for TSC012 comprising : HD37
      (anti-CD19) scFv, altered human IgG1 hinge, human
      IgG1 CH2, human IgG1 CH3, and human CH1

<400> 45
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcgagc ccaaatcttc tgacaaaact 840
cacacaagcc caccgagccc atgcccaccg tgcccagcac ctgaactcct gggtggaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacaacagc 1080
acgtaccgtg tggtcagcgt cctcaccgtc ctgcaccagg actggctgaa tggcaaggag 1140
tacaagtgca aggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaaatct agagcctcca ccaagggccc atcggtcttc 1560
cccctggcac cctcctccaa gagcacctct gggggcacag cggccctggg ctgcctggtc 1620
aaggactact tccccgagcc ggtgacggtg tcgtggaact caggcgccct gaccagcggc 1680
gtgcacacct tcccggctgt cctacagtcc tcaggactct actccctcag cagcgtggtg 1740
accgtgccct ccagcagctt gggcacccag acctacatct gcaacgtgaa tcacaagccc 1800
agcaacacca aggtggacaa gaaagtttga 1830

<210> 46

<211> 1830
<212> DNA
<213> Artificial Sequence

<220>
<223> Nucleotide sequence for TSC013 comprising : HD37
      (anti-CD19) scFv, altered human IgG3 hinge, human
      IgG1 CH2, human IgG1 CH3, and human CH1

<400> 46
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcgagc ccaaatctag cgacacacct 840
cccccaagcc cacggtcccc atgcccaccg tgcccagcac ctgaactcct gggtggaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacaacagc 1080
acgtaccgtg tggtcagcgt cctcaccgtc ctgcaccagg actggctgaa tggcaaggag 1140
tacaagtgca aggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaaatct agagcctcca ccaagggccc atcggtcttc 1560
cccctggcac cctcctccaa gagcacctct gggggcacag cggccctggg ctgcctggtc 1620
aaggactact tccccgagcc ggtgacggtg tcgtggaact caggcgccct gaccagcggc 1680
gtgcacacct tcccggctgt cctacagtcc tcaggactct actccctcag cagcgtggtg 1740
accgtgccct ccagcagctt gggcacccag acctacatct gcaacgtgaa tcacaagccc 1800
agcaacacca aggtggacaa gaaagtttga 1830

<210> 47
<211> 1887
<212> DNA
<213> Artificial Sequence

<220>
<223> Nucleotide sequence for TSC014 comprising : HD37
      (anti-CD19) scFv, altered human IgD hinge, human
      IgG1 CH2, human IgG1 CH3, and human CH1

<400> 47
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600

ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcgagt ctccaaaggc acaggcctcc 840
tccgtgccca ctgcacaacc ccaagcagag ggcagcctcg ccaaggcaac cacagcccca 900
gccaccaccc gtaacacatg cccaccgtgc ccagcacctg aactcctggg tggaccgtca 960
gtcttcctct tccccccaaa acccaaggac accctcatga tctcccggac ccctgaggtc 1020
acatgcgtgg tggtggacgt gagccacgaa gaccctgagg tcaagttcaa ctggtacgtg 1080
gacggcgtgg aggtgcataa tgccaagaca aagccgcggg aggagcagta caacagcacg 1140
taccgtgtgg tcagcgtcct caccgtcctg caccaggact ggctgaatgg caaggagtac 1200
aagtgcaagg tctccaacaa agccctccca gcccccatcg agaaaaccat ctccaaagcc 1260
aaagggcagc cccgagaacc acaggtgtac accctgcccc catcccggga tgagctgacc 1320
aagaaccagg tcagcctgac ctgcctggtc aaaggcttct atccaagcga catcgccgtg 1380
gagtgggaga gcaatgggca gccggagaac aactacaaga ccacgcctcc cgtgctggac 1440
tccgacggct ccttcttcct ctacagcaag ctcaccgtgg acaagagcag gtggcagcag 1500
gggaacgtct tctcatgctc cgtgatgcat gaggctctgc acaaccacta cacgcagaag 1560
agcctctccc tgtctccggg taaatctaga gcctccacca agggcccatc ggtcttcccc 1620
ctggcaccct cctccaagag cacctctggg ggcacagcgg ccctgggctg cctggtcaag 1680
gactacttcc ccgagccggt gacggtgtcg tggaactcag gcgccctgac cagcggcgtg 1740
cacaccttcc cggctgtcct acagtcctca ggactctact ccctcagcag cgtggtgacc 1800
gtgccctcca gcagcttggg cacccagacc tacatctgca acgtgaatca caagcccagc 1860
aacaccaagg tggacaagaa agtttga 1887

<210> 48
<211> 2103
<212> DNA
<213> Artificial Sequence

<220>
<223> Nucleotide sequence for TSC015 comprising : HD37
      (anti-CD19) scFv, altered human IgE CH2 hinge,
      human IgG1 CH2, human IgG1 CH3, and human CH1

<400> 48
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagctcca gggacttcac cccgcccacc 840
gtgaagatct tacagtcgtc cagcgacggc ggcgggcact tccccccgac catccagctc 900
ctgtgcctcg tctctgggta caccccaggg actatcaaca tcacctggct ggaggacggg 960
caggtcatgg acgtggactt gtccaccgcc tctaccacgc aggagggtga gctggcctcc 1020
acacaaagcg agctcaccct cagccagaag cactggctgt cagaccgcac ctacacctgc 1080
caggtcacct atcaaggtca cacctttgag gacagcacca agaagtctgc atgcccaccg 1140
tgctccggag cacctgaact cctgggtgga ccgtcagtct tcctcttccc cccaaaaccc 1200
aaggacaccc tcatgatctc ccggacccct gaggtcacat gcgtggtggt ggacgtgagc 1260
cacgaagacc ctgaggtcaa gttcaactgg tacgtggacg gcgtggaggt gcataatgcc 1320
aagacaaagc cgcgggagga gcagtacaac agcacgtacc gtgtggtcag cgtcctcacc 1380
gtcctgcacc aggactggct gaatggcaag gagtacaagt gcaaggtctc caacaaagcc 1440
ctcccagccc ccatcgagaa aaccatctcc aaagccaaag ggcagccccg agaaccacag 1500
gtgtacaccc tgcccccatc ccgggatgag ctgaccaaga accaggtcag cctgacctgc 1560
ctggtcaaag gcttctatcc aagcgacatc gccgtggagt gggagagcaa tgggcagccg 1620
gagaacaact acaagaccac gcctcccgtg ctggactccg acggctcctt cttcctctac 1680
agcaagctca ccgtggacaa gagcaggtgg cagcagggga acgtcttctc atgctccgtg 1740

atgcatgagg ctctgcacaa ccactacacg cagaagagcc tctccctgtc tccgggtaaa 1800
tctagagcct ccaccaaggg cccatcggtc ttccccctgg caccctcctc caagagcacc 1860
tctgggggca cagcggccct gggctgcctg gtcaaggact acttccccga accggtgacg 1920
gtgtcgtgga actcaggcgc cctgaccagc ggcgtgcaca ccttcccggc tgtcctacag 1980
tcctcaggac tctactccct cagcagcgtg gtgaccgtgc cctccagcag cttgggcacc 2040
cagacctaca tctgcaacgt gaatcacaag cccagcaaca ccaaggtgga caagaaagtt 2100
tga 2103

<210> 49
<211> 2124
<212> DNA
<213> Artificial Sequence

<220>
<223> Nucleotide sequence for TSC016 comprising : HD37
      (anti-CD19) scFv, altered human IgM CH2 hinge,
      human IgG1 CH2, human IgG1 CH3, and human CH1

<400> 49
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcattg ccgagctgcc tcccaaagtg 840
agcgtcttcg tcccaccccg cgacggcttc ttcggcaacc cccgcaagtc caagctcatc 900
tgccaggcca cgggtttcag tccccggcag attcaggtgt cctggctgcg cgaggggaag 960
caggtggggt ctggcgtcac cacggaccag gtgcaggctg aggccaaaga gtctgggccc 1020
acgacctaca aggtgaccag cacactgacc atcaaagaga gcgactggct cagccagagc 1080
atgttcacct gccgcgtgga tcacaggggc ctgaccttcc agcagaatgc gtcctccatg 1140
agtgtcccct gcccaccgtg cccatccgga gcacctgaac tcctgggtgg accgtcagtc 1200
ttcctcttcc ccccaaaacc caaggacacc ctcatgatct cccggacccc tgaggtcaca 1260
tgcgtggtgg tggacgtgag ccacgaagac cctgaggtca agttcaactg gtacgtggac 1320
ggcgtggagg tgcataatgc caagacaaag ccgcgggagg agcagtacaa cagcacgtac 1380
cgtgtggtca gcgtcctcac cgtcctgcac caggactggc tgaatggcaa ggagtacaag 1440
tgcaaggtct ccaacaaagc cctcccagcc cccatcgaga aaaccatctc caaagccaaa 1500
gggcagcccc gagaaccaca ggtgtacacc ctgcccccat cccgggatga gctgaccaag 1560
aaccaggtca gcctgacctg cctggtcaaa ggcttctatc caagcgacat cgccgtggag 1620
tgggagagca atgggcagcc ggagaacaac tacaagacca cgcctcccgt gctggactcc 1680
gacggctcct tcttcctcta cagcaagctc accgtggaca agagcaggtg gcagcagggg 1740
aacgtcttct catgctccgt gatgcatgag gctctgcaca accactacac gcagaagagc 1800
ctctccctgt ctccgggtaa atctagagcc tccaccaagg gcccatcggt cttccccctg 1860
gcaccctcct ccaagagcac ctctgggggc acagcggccc tgggctgcct ggtcaaggac 1920
tacttccccg agccggtgac ggtgtcgtgg aactcaggcg ccctgaccag cggcgtgcac 1980
accttcccgg ctgtcctaca gtcctcagga ctctactccc tcagcagcgt ggtgaccgtg 2040
ccctccagca gcttgggcac ccagacctac atctgcaacg tgaatcacaa gcccagcaac 2100
accaaggtgg acaagaaagt ttga 2124

<210> 50
<211> 1788
<212> DNA
<213> Artificial Sequence

<220>
<223> Nucleotide sequence for TSC017 comprising : P2C2

(anti-CD79b) scFv, human IgG1 SCC-P hinge, human
      IgG1 CH2, human IgG1 CH3 and human CH1

<400> 50
atggaagcac cagcgcagct tctcttcctc ctgctactct ggctcccaga taccaccggt 60
gatattgtga tgatccagga tgaactctcc aatcctgtca gttctggaga atcagtttcc 120
atctcctgta ggtctagtaa gagtctccta gataaggaag ggaagacata cttgaattgg 180
tttctgcaga gaccaggaca atctcctcag ctcctgatct atctgatgtc catgcgtgaa 240
tcaggagtct cagaccggtt tagtggcagt gggtcaggaa cagatttcac cctggaaatc 300
agtagagtga aggctgagga tgtgggtgtg tattactgtc aacaacttgt agagtatccg 360
tggacgttcg gtggaggcac caagctggaa atcaaaggtg gcggtggctc tggcggaggt 420
ggatccggtg gcggcggctc tcagatccag ttggtgcagt ctggacctga gctgaagaag 480
cctggagaga cagtcaagat ctcctgcaag gcttctggtt ataccttcac agacttttca 540
atgcactggg tgaggcaggc tccaggaaag ggtttaaagt ggatgggctg gataaacact 600
gagactggtg agccaacata tgcagatgac ttcaggggac ggtttgcctt ctctttggaa 660
acttctgcca gcactgccta tttgcagatc aacaacctca aaaatgagga cacggctaca 720
tatttttgta cctggtctgc ttactggggc caagggactc tggtcactgt ctctgcctcg 780
agcgagccca aatcttctga caaaactcac acatgcccac cgtgcccatc cggagcacct 840
gaactcctgg gtggaccgtc agtcttcctc ttccccccaa aacccaagga caccctcatg 900
atctcccgga cccctgaggt cacatgcgtg gtggtggacg tgagccacga agaccctgag 960
gtcaagttca actggtacgt ggacggcgtg gaggtgcata atgccaagac aaagccgcgg 1020
gaggagcagt acaacagcac gtaccgtgtg gtcagcgtcc tcaccgtcct gcaccaggac 1080
tggctgaatg gcaaggagta caagtgcaag gtctccaaca aagccctccc agcccccatc 1140
gagaaaacca tctccaaagc caaagggcag ccccgagaac cacaggtgta caccctgccc 1200
ccatcccggg atgagctgac caagaaccag gtcagcctga cctgcctggt caaaggcttc 1260
tatccaagcg acatcgccgt ggagtgggag agcaatgggc agccggagaa caactacaag 1320
accacgcctc ccgtgctgga ctccgacggc tccttcttcc tctacagcaa gctcaccgtg 1380
gacaagagca ggtggcagca ggggaacgtc ttctcatgct ccgtgatgca tgaggctctg 1440
cacaaccact acacgcagaa gagcctctcc ctgtctccgg gtaaatctag agcctccacc 1500
aagggcccat cggtcttccc cctggcaccc tcctccaaga gcacctctgg gggcacagcg 1560
gccctgggct gcctggtcaa ggactacttc cccgagccgg tgacggtgtc gtggaactca 1620
ggcgccctga ccagcggcgt gcacaccttc ccggctgtcc tacagtcctc aggactctac 1680
tccctcagca gcgtggtgac cgtgccctcc agcagcttgg gcacccagac ctacatctgc 1740
aacgtgaatc acaagcccag caacaccaag gtggacaaga aagtttga 1788

<210> 51
<211> 1812
<212> DNA
<213> Artificial Sequence

<220>
<223> Nucleotide sequence for TSC018 comprising : M0042
      (anti-HLA-DR) scFv, human IgG1 SCC-P hinge, human
      IgG1 CH2, human IgG1 CH3, and human CH1

<400> 51
atggaagcac cagcgcagct tctcttcctc ctgctactct ggctcccaga taccaccggt 60
gacatccaga tgacccagtc tccttccacc ctgtctgcat ctgtaggaga cagagtcacc 120
atcacttgcc gggccagtca gagtattagt agctggttgg cctggtatca gcagaaacca 180
gggaaagccc ctaagctcct ggtctattct gtttccagtt tgcaaagtgg ggtcccatca 240
aggttcagtg gcagtggatc cgggacagat ttcactctca ccatcaacag tctgcaacct 300
gaagattttg ccacttacta ctgtcaacag agttacagta ccccattcac tttcggccct 360
gggaccaaag tggatatcaa agggggtgga ggctctggtg gcggtggctc tggcggaggt 420
ggatccggtg gcggcggctc tgaagttcaa ttgttagagt ctggtggcgg tcttgttcag 480
cctggtggtt ctttacgtct ttcttgcgct gcttccggat tcactttctc tcattactat 540
atgtcttggg ttcgccaagc tcctggtaaa ggtttggagt gggtttctta tatcggtcct 600
tctggtggct ttactgctta tgctgactcc gttaaaggtc gcttcactat ctctagagac 660
aactctaaga atactctcta cttgcagatg aacagcttaa gggctgagga cacggccgtg 720
tattactgtg cgagagacgg gtgggttggt agcgagaact actccggtat ggacgtctgg 780
ggccaaggga ccacggtctc ctcgagcgag cccaaatctt ctgacaaaac tcacacatgc 840
ccaccgtgcc catccggagc acctgaactc ctgggtggac cgtcagtctt cctcttcccc 900
ccaaaaccca aggacaccct catgatctcc cggacccctg aggtcacatg cgtggtggtg 960
gacgtgagcc acgaagaccc tgaggtcaag ttcaactggt acgtggacgg cgtggaggtg 1020

cataatgcca agacaaagcc gcgggaggag cagtacaaca gcacgtaccg tgtggtcagc 1080
gtcctcaccg tcctgcacca ggactggctg aatggcaagg agtacaagtg caaggtctcc 1140
aacaaagccc tcccagcccc catcgagaaa accatctcca aagccaaagg gcagccccga 1200
gaaccacagg tgtacaccct gcccccatcc cgggatgagc tgaccaagaa ccaggtcagc 1260
ctgacctgcc tggtcaaagg cttctatcca agcgacatcg ccgtggagtg ggagagcaat 1320
gggcagccgg agaacaacta caagaccacg cctcccgtgc tggactccga cggctccttc 1380
ttcctctaca gcaagctcac cgtggacaag agcaggtggc agcaggggaa cgtcttctca 1440
tgctccgtga tgcatgaggc tctgcacaac cactacacgc agaagagcct ctccctgtct 1500
ccgggtaaat ctagagcctc caccaagggc ccatcggtct tccccctggc accctcctcc 1560
aagagcacct ctgggggcac agcggccctg ggctgcctgg tcaaggacta cttccccgag 1620
ccggtgacgg tgtcgtggaa ctcaggcgcc ctgaccagcg gcgtgcacac cttcccggct 1680
gtcctacagt cctcaggact ctactccctc agcagcgtgg tgaccgtgcc ctccagcagc 1740
ttgggcaccc agacctacat ctgcaacgtg aatcacaagc ccagcaacac caaggtggac 1800
aagaaagttt ga 1812

<210> 52
<211> 1833
<212> DNA
<213> Artificial Sequence

<220>
<223> Nucleotide sequence for TSC019 comprising : G19-4
      (anti-CD3) scFv, human IgG1 SCC-P hinge, human
      CH2, human CH3, and human Ck(YAE)

<400> 52
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180
catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcgag cccaaatctt ctgacaaaac tcacacatgc 840
ccaccgtgcc catccggagc acctgaactc ctgggtggac cgtcagtctt cctcttcccc 900
ccaaaaccca aggacaccct catgatctcc cggacccctg aggtcacatg cgtggtggtg 960
gacgtgagcc acgaagaccc tgaggtcaag ttcaactggt acgtggacgg cgtggaggtg 1020
cataatgcca agacaaagcc gcgggaggag cagtacaaca gcacgtaccg tgtggtcagc 1080
gtcctcaccg tcctgcacca ggactggctg aatggcaagg agtacaagtg caaggtctcc 1140
aacaaagccc tcccagcccc catcgagaaa accatctcca aagccaaagg gcagccccga 1200
gaaccacagg tgtacaccct gcccccatcc cgggatgagc tgaccaagaa ccaggtcagc 1260
ctgacctgcc tggtcaaagg cttctatcca agcgacatcg ccgtggagtg ggagagcaat 1320
gggcagccgg agaacaacta caagaccacg cctcccgtgc tggactccga cggctccttc 1380
ttcctctaca gcaagctcac cgtggacaag agcaggtggc agcaggggaa cgtcttctca 1440
tgctccgtga tgcatgaggc tctgcacaac cactacacgc agaagagcct ctccctgtct 1500
ccgggtaaat ctagaactgt ggctgcacca tctgtcttca tcttcccgcc atctgatgag 1560
cagttgaaat ctggaactgc ctctgttgtg tgcctgctga attacttcta tcccagagag 1620
gccaaagtac agtggaaggt ggataacgcc ctccaatcgg gtaactccca ggagagtgcc 1680
acagagcagg acagcaagga cagcacctac agcctcagca gcgagctgac gctgagcaaa 1740
gcagactacg agaaacacaa agtctacgcc tgcgaagtca cccatcaggg cctgagctcg 1800
cccgtcacaa agagcttcaa caggggagag tga 1833

<210> 53
<211> 582
<212> PRT
<213> Artificial Sequence

<220>
<223> ORN160 mature protein comprising : 5D5
      (anti-c-Met) scFv, human IgG1 SCC-P hinge, human
      IgG1 CH2, human IgG1 CH3, and human CH1

<400> 53
Asp Phe Met Met Ser Gln Ser Pro Ser Ser Leu Thr Val Ser Val Gly
1               5                   10                  15
Glu Lys Val Thr Val Ser Cys Lys Ser Ser Gln Ser Leu Leu Tyr Thr
            20                  25                  30
Ser Ser Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Thr Ser Val Lys Ala Asp Asp Leu Ala Val Tyr Tyr Cys Gln Gln
                85                  90                  95
Tyr Tyr Ala Tyr Pro Trp Thr Phe Gly Gly Gly Thr Lys Leu Glu Leu
            100                 105                 110
Lys Gly Gly Gly Gly Ser Gly Gly Gly Gly Ser Gly Gly Gly Gly Ser
        115                 120                 125
Gln Val Gln Leu Gln Gln Ser Gly Pro Glu Leu Val Arg Pro Gly Ala
    130                 135                 140
Ser Val Lys Met Ser Cys Arg Ala Ser Gly Tyr Thr Phe Thr Ser Tyr
145                 150                 155                 160
Trp Leu His Trp Val Lys Gln Arg Pro Gly Gln Gly Leu Glu Trp Ile
                165                 170                 175
Gly Met Ile Asp Pro Ser Asn Ser Asp Thr Arg Phe Asn Pro Asn Phe
            180                 185                 190
Lys Asp Lys Ala Thr Leu Asn Val Asp Arg Ser Ser Asn Thr Ala Tyr
        195                 200                 205
Met Leu Leu Ser Ser Leu Thr Ser Ala Asp Ser Ala Val Tyr Tyr Cys
    210                 215                 220
Ala Thr Tyr Gly Ser Tyr Val Ser Pro Leu Asp Tyr Trp Gly Gln Gly
225                 230                 235                 240
Thr Ser Val Thr Val Ser Ser Ser Glu Pro Lys Ser Ser Asp Lys Thr
                245                 250                 255
His Thr Cys Pro Pro Cys Pro Ser Gly Ala Pro Glu Leu Leu Gly Gly
            260                 265                 270
Pro Ser Val Phe Leu Phe Pro Pro Lys Pro Lys Asp Thr Leu Met Ile
        275                 280                 285
Ser Arg Thr Pro Glu Val Thr Cys Val Val Val Asp Val Ser His Glu
    290                 295                 300
Asp Pro Glu Val Lys Phe Asn Trp Tyr Val Asp Gly Val Glu Val His
305                 310                 315                 320
Asn Ala Lys Thr Lys Pro Arg Glu Glu Gln Tyr Asn Ser Thr Tyr Arg
                325                 330                 335
Val Val Ser Val Leu Thr Val Leu His Gln Asp Trp Leu Asn Gly Lys
            340                 345                 350
Glu Tyr Lys Cys Lys Val Ser Asn Lys Ala Leu Pro Ala Pro Ile Glu
        355                 360                 365
Lys Thr Ile Ser Lys Ala Lys Gly Gln Pro Arg Glu Pro Gln Val Tyr
    370                 375                 380
Thr Leu Pro Pro Ser Arg Asp Glu Leu Thr Lys Asn Gln Val Ser Leu
385                 390                 395                 400
Thr Cys Leu Val Lys Gly Phe Tyr Pro Ser Asp Ile Ala Val Glu Trp
                405                 410                 415
Glu Ser Asn Gly Gln Pro Glu Asn Asn Tyr Lys Thr Thr Pro Pro Val
            420                 425                 430
Leu Asp Ser Asp Gly Ser Phe Phe Leu Tyr Ser Lys Leu Thr Val Asp
        435                 440                 445
Lys Ser Arg Trp Gln Gln Gly Asn Val Phe Ser Cys Ser Val Met His

450                 455                 460
Glu Ala Leu His Asn His Tyr Thr Gln Lys Ser Leu Ser Leu Ser Pro
465                 470                 475                 480
Gly Lys Ser Arg Ala Ser Thr Lys Gly Pro Ser Val Phe Pro Leu Ala
                485                 490                 495
Pro Ser Ser Lys Ser Thr Ser Gly Gly Thr Ala Ala Leu Gly Cys Leu
            500                 505                 510
Val Lys Asp Tyr Phe Pro Glu Pro Val Thr Val Ser Trp Asn Ser Gly
        515                 520                 525
Ala Leu Thr Ser Gly Val His Thr Phe Pro Ala Val Leu Gln Ser Ser
    530                 535                 540
Gly Leu Tyr Ser Leu Ser Ser Val Val Thr Val Pro Ser Ser Ser Leu
545                 550                 555                 560
Gly Thr Gln Thr Tyr Ile Cys Asn Val Asn His Lys Pro Ser Asn Thr
                565                 570                 575
Lys Val Asp Lys Lys Val
            580

<210> 54
<211> 586
<212> PRT
<213> Artificial Sequence

<220>
<223> TSC001 mature protein comprising : HD37
      (anti-CD39), human IgG1 SCC-P hinge, human IgG1
      CH2, human IgG1 CH3 and human CH1

<400> 54
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ser Gly Ala Pro Glu

                260                 265                 270
Leu Leu Gly Gly Pro Ser Val Phe Leu Phe Pro Pro Lys Pro Lys Asp
        275                 280                 285
Thr Leu Met Ile Ser Arg Thr Pro Glu Val Thr Cys Val Val Val Asp
    290                 295                 300
Val Ser His Glu Asp Pro Glu Val Lys Phe Asn Trp Tyr Val Asp Gly
305                 310                 315                 320
Val Glu Val His Asn Ala Lys Thr Lys Pro Arg Glu Glu Gln Tyr Asn
                325                 330                 335
Ser Thr Tyr Arg Val Val Ser Val Leu Thr Val Leu His Gln Asp Trp
            340                 345                 350
Leu Asn Gly Lys Glu Tyr Lys Cys Lys Val Ser Asn Lys Ala Leu Pro
        355                 360                 365
Ala Pro Ile Glu Lys Thr Ile Ser Lys Ala Lys Gly Gln Pro Arg Glu
    370                 375                 380
Pro Gln Val Tyr Thr Leu Pro Pro Ser Arg Asp Glu Leu Thr Lys Asn
385                 390                 395                 400
Gln Val Ser Leu Thr Cys Leu Val Lys Gly Phe Tyr Pro Ser Asp Ile
                405                 410                 415
Ala Val Glu Trp Glu Ser Asn Gly Gln Pro Glu Asn Asn Tyr Lys Thr
            420                 425                 430
Thr Pro Pro Val Leu Asp Ser Asp Gly Ser Phe Phe Leu Tyr Ser Lys
        435                 440                 445
Leu Thr Val Asp Lys Ser Arg Trp Gln Gln Gly Asn Val Phe Ser Cys
    450                 455                 460
Ser Val Met His Glu Ala Leu His Asn His Tyr Thr Gln Lys Ser Leu
465                 470                 475                 480
Ser Leu Ser Pro Gly Lys Ser Arg Ala Ser Thr Lys Gly Pro Ser Val
                485                 490                 495
Phe Pro Leu Ala Pro Ser Ser Lys Ser Thr Ser Gly Gly Thr Ala Ala
            500                 505                 510
Leu Gly Cys Leu Val Lys Asp Tyr Phe Pro Glu Pro Val Thr Val Ser
        515                 520                 525
Trp Asn Ser Gly Ala Leu Thr Ser Gly Val His Thr Phe Pro Ala Val
    530                 535                 540
Leu Gln Ser Ser Gly Leu Tyr Ser Leu Ser Ser Val Val Thr Val Pro
545                 550                 555                 560
Ser Ser Ser Leu Gly Thr Gln Thr Tyr Ile Cys Asn Val Asn His Lys
                565                 570                 575
Pro Ser Asn Thr Lys Val Asp Lys Lys Val
            580                 585

<210> 55
<211> 579
<212> PRT
<213> Artificial Sequence

<220>
<223> TSC002 mature protein comprising : 2H7 (anti-CD20)
      scFv, human IgG1 SCC-P hinge, human IgG1 CH2,
      human IgG1 CH3 and human CH1

<400> 55
Gln Ile Val Leu Ser Gln Ser Pro Ala Ile Leu Ser Ala Ser Pro Gly
1               5                   10                  15
Glu Lys Val Thr Met Thr Cys Arg Ala Ser Ser Ser Val Ser Tyr Met
            20                  25                  30
His Trp Tyr Gln Gln Lys Pro Gly Ser Ser Pro Lys Pro Trp Ile Tyr
        35                  40                  45
Ala Pro Ser Asn Leu Ala Ser Gly Val Pro Ala Arg Phe Ser Gly Ser
    50                  55                  60
Gly Ser Gly Thr Ser Tyr Ser Leu Thr Ile Ser Arg Val Glu Ala Glu

65                  70                  75                  80
Asp Ala Ala Thr Tyr Tyr Cys Gln Gln Trp Ser Phe Asn Pro Pro Thr
                85                  90                  95
Phe Gly Ala Gly Thr Lys Leu Glu Leu Lys Asp Gly Gly Gly Ser Gly
            100                 105                 110
Gly Gly Gly Ser Gly Gly Gly Gly Ala Ser Gln Ala Tyr Leu Gln Gln
        115                 120                 125
Ser Gly Ala Glu Ser Val Arg Pro Gly Ala Ser Val Lys Met Ser Cys
    130                 135                 140
Lys Ala Ser Gly Tyr Thr Phe Thr Ser Tyr Asn Met His Trp Val Lys
145                 150                 155                 160
Gln Thr Pro Arg Gln Gly Leu Glu Trp Ile Gly Ala Ile Tyr Pro Gly
                165                 170                 175
Asn Gly Asp Thr Ser Tyr Asn Gln Lys Phe Lys Gly Lys Ala Thr Leu
            180                 185                 190
Thr Val Asp Lys Ser Ser Ser Thr Ala Tyr Met Gln Leu Ser Ser Leu
        195                 200                 205
Thr Ser Glu Asp Ser Ala Val Tyr Phe Cys Ala Arg Val Val Tyr Tyr
    210                 215                 220
Ser Asn Ser Tyr Trp Tyr Phe Asp Val Trp Gly Thr Gly Thr Thr Val
225                 230                 235                 240
Thr Val Ser Ser Ser Glu Pro Lys Ser Ser Asp Lys Thr His Thr Cys
                245                 250                 255
Pro Pro Cys Pro Ser Gly Ala Pro Glu Leu Leu Gly Gly Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Glu Tyr Lys
            340                 345                 350
Cys Lys Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Ala Ser Thr Lys Gly Pro Ser Val Phe Pro Leu Ala Pro Ser Ser
                485                 490                 495
Lys Ser Thr Ser Gly Gly Thr Ala Ala Leu Gly Cys Leu Val Lys Asp
            500                 505                 510
Tyr Phe Pro Glu Pro Val Thr Val Ser Trp Asn Ser Gly Ala Leu Thr
        515                 520                 525
Ser Gly Val His Thr Phe Pro Ala Val Leu Gln Ser Ser Gly Leu Tyr
    530                 535                 540
Ser Leu Ser Ser Val Val Thr Val Pro Ser Ser Ser Leu Gly Thr Gln
545                 550                 555                 560
Thr Tyr Ile Cys Asn Val Asn His Lys Pro Ser Asn Thr Lys Val Asp
                565                 570                 575

Lys Lys Val

<210> 56
<211> 596
<212> PRT
<213> Artificial Sequence

<220>
<223> TSC003 mature protein comprising : G19-4
      (anti-CD3) scFv, altered human IgA1 hinge,  human
      IgG1 CH2, human IgG1 CH3, and human Ck(YAE)

<400> 56
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Pro Ser Thr Pro Pro Thr Pro
                245                 250                 255
Ser Pro Ser Thr Pro Pro Thr Pro Ser Pro Ser Cys Pro Pro Cys Pro
            260                 265                 270
Ala Pro Glu Leu Leu Gly Gly Pro Ser Val Phe Leu Phe Pro Pro Lys
        275                 280                 285
Pro Lys Asp Thr Leu Met Ile Ser Arg Thr Pro Glu Val Thr Cys Val
    290                 295                 300
Val Val Asp Val Ser His Glu Asp Pro Glu Val Lys Phe Asn Trp Tyr
305                 310                 315                 320
Val Asp Gly Val Glu Val His Asn Ala Lys Thr Lys Pro Arg Glu Glu
                325                 330                 335
Gln Tyr Asn Ser Thr Tyr Arg Val Val Ser Val Leu Thr Val Leu His
            340                 345                 350
Gln Asp Trp Leu Asn Gly Lys Glu Tyr Lys Cys Lys Val Ser Asn Lys
        355                 360                 365
Ala Leu Pro Ala Pro Ile Glu Lys Thr Ile Ser Lys Ala Lys Gly Gln
    370                 375                 380

Pro Arg Glu Pro Gln Val Tyr Thr Leu Pro Pro Ser Arg Asp Glu Leu
385                 390                 395                 400
Thr Lys Asn Gln Val Ser Leu Thr Cys Leu Val Lys Gly Phe Tyr Pro
                405                 410                 415
Ser Asp Ile Ala Val Glu Trp Glu Ser Asn Gly Gln Pro Glu Asn Asn
            420                 425                 430
Tyr Lys Thr Thr Pro Pro Val Leu Asp Ser Asp Gly Ser Phe Phe Leu
        435                 440                 445
Tyr Ser Lys Leu Thr Val Asp Lys Ser Arg Trp Gln Gln Gly Asn Val
    450                 455                 460
Phe Ser Cys Ser Val Met His Glu Ala Leu His Asn His Tyr Thr Gln
465                 470                 475                 480
Lys Ser Leu Ser Leu Ser Pro Gly Lys Ser Arg Thr Val Ala Ala Pro
                485                 490                 495
Ser Val Phe Ile Phe Pro Pro Ser Asp Glu Gln Leu Lys Ser Gly Thr
            500                 505                 510
Ala Ser Val Val Cys Leu Leu Asn Tyr Phe Tyr Pro Arg Glu Ala Lys
        515                 520                 525
Val Gln Trp Lys Val Asp Asn Ala Leu Gln Ser Gly Asn Ser Gln Glu
    530                 535                 540
Ser Ala Thr Glu Gln Asp Ser Lys Asp Ser Thr Tyr Ser Leu Ser Ser
545                 550                 555                 560
Glu Leu Thr Leu Ser Lys Ala Asp Tyr Glu Lys His Lys Val Tyr Ala
                565                 570                 575
Cys Glu Val Thr His Gln Gly Leu Ser Ser Pro Val Thr Lys Ser Phe
            580                 585                 590
Asn Arg Gly Glu
        595

<210> 57
<211> 583
<212> PRT
<213> Artificial Sequence

<220>
<223> TSC004 mature protein comprising : G19-4
      (anti-CD3) scFv, altered human IgA2 hinge, human
      IgG1 CH2, human IgG1 CH3, and human Ck(YAE)

<400> 57
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175

Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Pro Pro Pro Pro Pro Cys Pro
                245                 250                 255
Pro Cys Pro Ala Pro Glu Leu Leu Gly Gly Pro Ser Val Phe Leu Phe
            260                 265                 270
Pro Pro Lys Pro Lys Asp Thr Leu Met Ile Ser Arg Thr Pro Glu Val
        275                 280                 285
Thr Cys Val Val Val Asp Val Ser His Glu Asp Pro Glu Val Lys Phe
    290                 295                 300
Asn Trp Tyr Val Asp Gly Val Glu Val His Asn Ala Lys Thr Lys Pro
305                 310                 315                 320
Arg Glu Glu Gln Tyr Asn Ser Thr Tyr Arg Val Val Ser Val Leu Thr
                325                 330                 335
Val Leu His Gln Asp Trp Leu Asn Gly Lys Glu Tyr Lys Cys Lys Val
            340                 345                 350
Ser Asn Lys Ala Leu Pro Ala Pro Ile Glu Lys Thr Ile Ser Lys Ala
        355                 360                 365
Lys Gly Gln Pro Arg Glu Pro Gln Val Tyr Thr Leu Pro Pro Ser Arg
    370                 375                 380
Asp Glu Leu Thr Lys Asn Gln Val Ser Leu Thr Cys Leu Val Lys Gly
385                 390                 395                 400
Phe Tyr Pro Ser Asp Ile Ala Val Glu Trp Glu Ser Asn Gly Gln Pro
                405                 410                 415
Glu Asn Asn Tyr Lys Thr Thr Pro Pro Val Leu Asp Ser Asp Gly Ser
            420                 425                 430
Phe Phe Leu Tyr Ser Lys Leu Thr Val Asp Lys Ser Arg Trp Gln Gln
        435                 440                 445
Gly Asn Val Phe Ser Cys Ser Val Met His Glu Ala Leu His Asn His
    450                 455                 460
Tyr Thr Gln Lys Ser Leu Ser Leu Ser Pro Gly Lys Ser Arg Thr Val
465                 470                 475                 480
Ala Ala Pro Ser Val Phe Ile Phe Pro Pro Ser Asp Glu Gln Leu Lys
                485                 490                 495
Ser Gly Thr Ala Ser Val Val Cys Leu Leu Asn Tyr Phe Tyr Pro Arg
            500                 505                 510
Glu Ala Lys Val Gln Trp Lys Val Asp Asn Ala Leu Gln Ser Gly Asn
        515                 520                 525
Ser Gln Glu Ser Ala Thr Glu Gln Asp Ser Lys Asp Ser Thr Tyr Ser
    530                 535                 540
Leu Ser Ser Glu Leu Thr Leu Ser Lys Ala Asp Tyr Glu Lys His Lys
545                 550                 555                 560
Val Tyr Ala Cys Glu Val Thr His Gln Gly Leu Ser Ser Pro Val Thr
                565                 570                 575
Lys Ser Phe Asn Arg Gly Glu
            580

<210> 58
<211> 593
<212> PRT
<213> Artificial Sequence

<220>
<223> TSC005 mature protein comprising : G19-4
      (anti-CD3) scFv, altered human IgG3 hinge, human
      IgG1 CH2, human IgG1 CH3, and human Ck(YAE)

<400> 58
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Glu Pro Lys Ser Ser Asp Thr
                245                 250                 255
Pro Pro Pro Ser Pro Arg Ser Pro Cys Pro Pro Cys Pro Ala Pro Glu
            260                 265                 270
Leu Leu Gly Gly Pro Ser Val Phe Leu Phe Pro Pro Lys Pro Lys Asp
        275                 280                 285
Thr Leu Met Ile Ser Arg Thr Pro Glu Val Thr Cys Val Val Val Asp
    290                 295                 300
Val Ser His Glu Asp Pro Glu Val Lys Phe Asn Trp Tyr Val Asp Gly
305                 310                 315                 320
Val Glu Val His Asn Ala Lys Thr Lys Pro Arg Glu Glu Gln Tyr Asn
                325                 330                 335
Ser Thr Tyr Arg Val Val Ser Val Leu Thr Val Leu His Gln Asp Trp
            340                 345                 350
Leu Asn Gly Lys Glu Tyr Lys Cys Lys Val Ser Asn Lys Ala Leu Pro
        355                 360                 365
Ala Pro Ile Glu Lys Thr Ile Ser Lys Ala Lys Gly Gln Pro Arg Glu
    370                 375                 380
Pro Gln Val Tyr Thr Leu Pro Pro Ser Arg Asp Glu Leu Thr Lys Asn
385                 390                 395                 400
Gln Val Ser Leu Thr Cys Leu Val Lys Gly Phe Tyr Pro Ser Asp Ile
                405                 410                 415
Ala Val Glu Trp Glu Ser Asn Gly Gln Pro Glu Asn Asn Tyr Lys Thr
            420                 425                 430
Thr Pro Pro Val Leu Asp Ser Asp Gly Ser Phe Phe Leu Tyr Ser Lys
        435                 440                 445
Leu Thr Val Asp Lys Ser Arg Trp Gln Gln Gly Asn Val Phe Ser Cys
    450                 455                 460
Ser Val Met His Glu Ala Leu His Asn His Tyr Thr Gln Lys Ser Leu
465                 470                 475                 480
Ser Leu Ser Pro Gly Lys Ser Arg Thr Val Ala Ala Pro Ser Val Phe

485                         490                         495
Ile Phe Pro Pro Ser Asp Glu Gln Leu Lys Ser Gly Thr Ala Ser Val
                500                         505                         510
Val Cys Leu Leu Asn Tyr Phe Tyr Pro Arg Glu Ala Lys Val Gln Trp
        515                         520                         525
Lys Val Asp Asn Ala Leu Gln Ser Gly Asn Ser Gln Glu Ser Ala Thr
    530                         535                         540
Glu Gln Asp Ser Lys Asp Ser Thr Tyr Ser Leu Ser Ser Glu Leu Thr
545                         550                         555                         560
Leu Ser Lys Ala Asp Tyr Glu Lys His Lys Val Tyr Ala Cys Glu Val
                565                         570                         575
Thr His Gln Gly Leu Ser Ser Pro Val Thr Lys Ser Phe Asn Arg Gly
                580                         585                         590
Glu

<210> 59
<211> 612
<212> PRT
<213> Artificial Sequence

<220>
<223> TSC006 mature protein comprising : G19-4
      (anti-CD3) scFv, altered human IgD hinge, human
      IgG1 CH2, human IgG1 CH3, and human Ck(YAE)

<220>
<221> VARIANT
<222> 375
<223> Xaa = Any Amino Acid

<400> 59
Glu Val Gln Leu Gln Gln Ser Gly Pro Glu Leu Val Lys Pro Gly Ala
 1               5                         10                         15
Ser Met Lys Ile Ser Cys Lys Ala Ser Gly Tyr Ser Phe Thr Gly Tyr
                20                         25                         30
Ile Val Asn Trp Leu Lys Gln Ser His Gly Lys Asn Leu Glu Trp Ile
        35                         40                         45
Gly Leu Ile Asn Pro Tyr Lys Gly Leu Thr Thr Tyr Asn Gln Lys Phe
    50                         55                         60
Lys Gly Lys Ala Thr Leu Thr Val Asp Lys Ser Ser Ser Thr Ala Tyr
65                         70                         75                         80
Met Glu Leu Leu Ser Leu Thr Ser Glu Asp Ser Ala Val Tyr Tyr Cys
                85                         90                         95
Ala Arg Ser Gly Tyr Tyr Gly Asp Ser Asp Trp Tyr Phe Asp Val Trp
                100                         105                         110
Gly Ala Gly Thr Thr Val Thr Val Ser Ser Gly Gly Gly Gly Ser Gly
        115                         120                         125
Gly Gly Gly Ser Gly Gly Gly Gly Ser Ala Gln Asp Ile Gln Met Thr
    130                         135                         140
Gln Thr Thr Ser Ser Leu Ser Ala Ser Leu Gly Asp Arg Val Thr Ile
145                         150                         155                         160
Ser Cys Arg Ala Ser Gln Asp Ile Arg Asn Tyr Leu Asn Trp Tyr Gln
                165                         170                         175
Gln Lys Pro Asp Gly Thr Val Lys Leu Leu Ile Tyr Tyr Thr Ser Arg
                180                         185                         190
Leu His Ser Gly Val Pro Ser Arg Phe Ser Gly Ser Gly Ser Gly Thr
        195                         200                         205
Asp Tyr Ser Leu Thr Ile Ala Asn Leu Gln Pro Glu Asp Ile Ala Thr
    210                         215                         220
Tyr Phe Cys Gln Gln Gly Asn Thr Leu Pro Trp Thr Phe Gly Gly Gly
225                         230                         235                         240

Thr Lys Leu Val Thr Lys Arg Ser Ser Glu Ser Pro Lys Ala Gln Ala
                245                 250                 255
Ser Ser Val Pro Thr Ala Gln Pro Gln Ala Glu Gly Ser Leu Ala Lys
            260                 265                 270
Ala Thr Thr Ala Pro Ala Thr Thr Arg Asn Thr Cys Pro Pro Cys Pro
        275                 280                 285
Ala Pro Glu Leu Leu Gly Gly Pro Ser Val Phe Leu Phe Pro Pro Lys
    290                 295                 300
Pro Lys Asp Thr Leu Met Ile Ser Arg Thr Pro Glu Val Thr Cys Val
305                 310                 315                 320
Val Val Asp Val Ser His Glu Asp Pro Glu Val Lys Phe Asn Trp Tyr
                325                 330                 335
Val Asp Gly Val Glu Val His Asn Ala Lys Thr Lys Pro Arg Glu Glu
            340                 345                 350
Gln Tyr Asn Ser Thr Tyr Arg Val Val Ser Val Leu Thr Val Leu His
        355                 360                 365
Gln Asp Trp Leu Asn Gly Xaa Glu Tyr Lys Cys Lys Val Ser Asn Lys
    370                 375                 380
Ala Leu Pro Ala Pro Ile Glu Lys Thr Ile Ser Lys Ala Lys Gly Gln
385                 390                 395                 400
Pro Arg Glu Pro Gln Val Tyr Thr Leu Pro Pro Ser Arg Asp Glu Leu
                405                 410                 415
Thr Lys Asn Gln Val Ser Leu Thr Cys Leu Val Lys Gly Phe Tyr Pro
            420                 425                 430
Ser Asp Ile Ala Val Glu Trp Glu Ser Asn Gly Gln Pro Glu Asn Asn
        435                 440                 445
Tyr Lys Thr Thr Pro Pro Val Leu Asp Ser Asp Gly Ser Phe Phe Leu
    450                 455                 460
Tyr Ser Lys Leu Thr Val Asp Lys Ser Arg Trp Gln Gln Gly Asn Val
465                 470                 475                 480
Phe Ser Cys Ser Val Met His Glu Ala Leu His Asn His Tyr Thr Gln
                485                 490                 495
Lys Ser Leu Ser Leu Ser Pro Gly Lys Ser Arg Thr Val Ala Ala Pro
            500                 505                 510
Ser Val Phe Ile Phe Pro Pro Ser Asp Glu Gln Leu Lys Ser Gly Thr
        515                 520                 525
Ala Ser Val Val Cys Leu Leu Asn Tyr Phe Tyr Pro Arg Glu Ala Lys
    530                 535                 540
Val Gln Trp Lys Val Asp Asn Ala Leu Gln Ser Gly Asn Ser Gln Glu
545                 550                 555                 560
Ser Ala Thr Glu Gln Asp Ser Lys Asp Ser Thr Tyr Ser Leu Ser Ser
                565                 570                 575
Glu Leu Thr Leu Ser Lys Ala Asp Tyr Glu Lys His Lys Val Tyr Ala
            580                 585                 590
Cys Glu Val Thr His Gln Gly Leu Ser Ser Pro Val Thr Lys Ser Phe
        595                 600                 605
Asn Arg Gly Glu
    610

<210> 60
<211> 593
<212> PRT
<213> Artificial Sequence

<220>
<223> TSC007 mature protein comprising : G19-4
      (anti-CD3) scFv, altered human IgG1 hinge, human
      IgG1 CH2, human IgG1 CH3, and human Ck(YAE)

<400> 60
Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Glu Pro Lys Ser Ser Asp Lys
                245                 250                 255
Thr His Thr Ser Pro Pro Ser Pro Cys Pro Pro Cys Pro Ala Pro Glu
            260                 265                 270
Leu Leu Gly Gly Pro Ser Val Phe Leu Phe Pro Pro Lys Pro Lys Asp
        275                 280                 285
Thr Leu Met Ile Ser Arg Thr Pro Glu Val Thr Cys Val Val Val Asp
    290                 295                 300
Val Ser His Glu Asp Pro Glu Val Lys Phe Asn Trp Tyr Val Asp Gly
305                 310                 315                 320
Val Glu Val His Asn Ala Lys Thr Lys Pro Arg Glu Glu Gln Tyr Asn
                325                 330                 335
Ser Thr Tyr Arg Val Val Ser Val Leu Thr Val Leu His Gln Asp Trp
            340                 345                 350
Leu Asn Gly Lys Glu Tyr Lys Cys Lys Val Ser Asn Lys Ala Leu Pro
        355                 360                 365
Ala Pro Ile Glu Lys Thr Ile Ser Lys Ala Lys Gly Gln Pro Arg Glu
    370                 375                 380
Pro Gln Val Tyr Thr Leu Pro Pro Ser Arg Asp Glu Leu Thr Lys Asn
385                 390                 395                 400
Gln Val Ser Leu Thr Cys Leu Val Lys Gly Phe Tyr Pro Ser Asp Ile
                405                 410                 415
Ala Val Glu Trp Glu Ser Asn Gly Gln Pro Glu Asn Asn Tyr Lys Thr
            420                 425                 430
Thr Pro Pro Val Leu Asp Ser Asp Gly Ser Phe Phe Leu Tyr Ser Lys
        435                 440                 445
Leu Thr Val Asp Lys Ser Arg Trp Gln Gln Gly Asn Val Phe Ser Cys
    450                 455                 460
Ser Val Met His Glu Ala Leu His Asn His Tyr Thr Gln Lys Ser Leu
465                 470                 475                 480
Ser Leu Ser Pro Gly Lys Ser Arg Thr Val Ala Ala Pro Ser Val Phe
                485                 490                 495
Ile Phe Pro Pro Ser Asp Glu Gln Leu Lys Ser Gly Thr Ala Ser Val
            500                 505                 510
Val Cys Leu Leu Asn Tyr Phe Tyr Pro Arg Glu Ala Lys Val Gln Trp

515                 520                 525
Lys Val Asp Asn Ala Leu Gln Ser Gly Asn Ser Gln Glu Ser Ala Thr
    530                 535                 540
Glu Gln Asp Ser Lys Asp Ser Thr Tyr Ser Leu Ser Ser Glu Leu Thr
545                 550                 555                 560
Leu Ser Lys Ala Asp Tyr Glu Lys His Lys Val Tyr Ala Cys Glu Val
                565                 570                 575
Thr His Gln Gly Leu Ser Ser Pro Val Thr Lys Ser Phe Asn Arg Gly
            580                 585                 590
Glu

<210> 61
<211> 684
<212> PRT
<213> Artificial Sequence

<220>
<223> TSC008 mature protein comprising : G19-4
      (anti-CD3) scFv, altered human IgE CH2 hinge (SEQ
      ID NO:757), human IgG1 CH2, human IgG1 CH3, and
      human Ck(YAE)

<400> 61
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Ser Arg Asp Phe Thr Pro Pro
                245                 250                 255
Thr Val Lys Ile Leu Gln Ser Ser Ser Asp Gly Gly Gly His Phe Pro
            260                 265                 270
Pro Thr Ile Gln Leu Leu Cys Leu Val Ser Gly Tyr Thr Pro Gly Thr
        275                 280                 285
Ile Asn Ile Thr Trp Leu Glu Asp Gly Gln Val Met Asp Val Asp Leu
    290                 295                 300

Ser Thr Ala Ser Thr Thr Gln Glu Gly Glu Leu Ala Ser Thr Gln Ser
305                 310                 315                 320
Glu Leu Thr Leu Ser Gln Lys His Trp Leu Ser Asp Arg Thr Tyr Thr
                325                 330                 335
Cys Gln Val Thr Tyr Gln Gly His Thr Phe Glu Asp Ser Thr Lys Lys
            340                 345                 350
Ser Ala Cys Pro Pro Cys Ser Gly Ala Pro Glu Leu Leu Gly Gly Pro
        355                 360                 365
Ser Val Phe Leu Phe Pro Pro Lys Pro Lys Asp Thr Leu Met Ile Ser
    370                 375                 380
Arg Thr Pro Glu Val Thr Cys Val Val Val Asp Val Ser His Glu Asp
385                 390                 395                 400
Pro Glu Val Lys Phe Asn Trp Tyr Val Asp Gly Val Glu Val His Asn
                405                 410                 415
Ala Lys Thr Lys Pro Arg Glu Glu Gln Tyr Asn Ser Thr Tyr Arg Val
            420                 425                 430
Val Ser Val Leu Thr Val Leu His Gln Asp Trp Leu Asn Gly Lys Glu
        435                 440                 445
Tyr Lys Cys Lys Val Ser Asn Lys Ala Leu Pro Ala Pro Ile Glu Lys
    450                 455                 460
Thr Ile Ser Lys Ala Lys Gly Gln Pro Arg Glu Pro Gln Val Tyr Thr
465                 470                 475                 480
Leu Pro Pro Ser Arg Asp Glu Leu Thr Lys Asn Gln Val Ser Leu Thr
                485                 490                 495
Cys Leu Val Lys Gly Phe Tyr Pro Ser Asp Ile Ala Val Glu Trp Glu
            500                 505                 510
Ser Asn Gly Gln Pro Glu Asn Asn Tyr Lys Thr Thr Pro Pro Val Leu
        515                 520                 525
Asp Ser Asp Gly Ser Phe Phe Leu Tyr Ser Lys Leu Thr Val Asp Lys
    530                 535                 540
Ser Arg Trp Gln Gln Gly Asn Val Phe Ser Cys Ser Val Met His Glu
545                 550                 555                 560
Ala Leu His Asn His Tyr Thr Gln Lys Ser Leu Ser Leu Ser Pro Gly
                565                 570                 575
Lys Ser Arg Thr Val Ala Ala Pro Ser Val Phe Ile Phe Pro Pro Ser
            580                 585                 590
Asp Glu Gln Leu Lys Ser Gly Thr Ala Ser Val Val Cys Leu Leu Asn
        595                 600                 605
Tyr Phe Tyr Pro Arg Glu Ala Lys Val Gln Trp Lys Val Asp Asn Ala
    610                 615                 620
Leu Gln Ser Gly Asn Ser Gln Glu Ser Ala Thr Glu Gln Asp Ser Lys
625                 630                 635                 640
Asp Ser Thr Tyr Ser Leu Ser Ser Glu Leu Thr Leu Ser Lys Ala Asp
                645                 650                 655
Tyr Glu Lys His Lys Val Tyr Ala Cys Glu Val Thr His Gln Gly Leu
            660                 665                 670
Ser Ser Pro Val Thr Lys Ser Phe Asn Arg Gly Glu
        675                 680

<210> 62
<211> 691
<212> PRT
<213> Artificial Sequence

<220>
<223> TSC009 mature protein comprising : G19-4
      (anti-CD3) scFv, altered human IgM CH2 hinge,
      human IgG1 CH2, human IgG1 CH3, and human Ck(YAE)

<400> 62

<210> 63
<211> 592
<212> PRT
<213> Artificial Sequence

<220>
<223> TSC010 mature protein comprising : HD37
      (anti-CD19) scFv, altered human IgA1 hinge, human
      IgG1 CH2, human IgG1 CH3, and human CH1

<400> 63
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala

210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Pro Ser Thr Pro
                245                 250                 255
Pro Thr Pro Ser Pro Ser Thr Pro Pro Thr Pro Ser Pro Ser Cys Pro
            260                 265                 270
Pro Cys Pro Ala Pro Glu Leu Leu Gly Gly Pro Ser Val Phe Leu Phe
        275                 280                 285
Pro Pro Lys Pro Lys Asp Thr Leu Met Ile Ser Arg Thr Pro Glu Val
    290                 295                 300
Thr Cys Val Val Val Asp Val Ser His Glu Asp Pro Glu Val Lys Phe
305                 310                 315                 320
Asn Trp Tyr Val Asp Gly Val Glu Val His Asn Ala Lys Thr Lys Pro
                325                 330                 335
Arg Glu Glu Gln Tyr Asn Ser Thr Tyr Arg Val Val Ser Val Leu Thr
            340                 345                 350
Val Leu His Gln Asp Trp Leu Asn Gly Lys Glu Tyr Lys Cys Lys Val
        355                 360                 365
Ser Asn Lys Ala Leu Pro Ala Pro Ile Glu Lys Thr Ile Ser Lys Ala
    370                 375                 380
Lys Gly Gln Pro Arg Glu Pro Gln Val Tyr Thr Leu Pro Pro Ser Arg
385                 390                 395                 400
Asp Glu Leu Thr Lys Asn Gln Val Ser Leu Thr Cys Leu Val Lys Gly
                405                 410                 415
Phe Tyr Pro Ser Asp Ile Ala Val Glu Trp Glu Ser Asn Gly Gln Pro
            420                 425                 430
Glu Asn Asn Tyr Lys Thr Thr Pro Pro Val Leu Asp Ser Asp Gly Ser
        435                 440                 445
Phe Phe Leu Tyr Ser Lys Leu Thr Val Asp Lys Ser Arg Trp Gln Gln
    450                 455                 460
Gly Asn Val Phe Ser Cys Ser Val Met His Glu Ala Leu His Asn His
465                 470                 475                 480
Tyr Thr Gln Lys Ser Leu Ser Leu Ser Pro Gly Lys Ser Arg Ala Ser
                485                 490                 495
Thr Lys Gly Pro Ser Val Phe Pro Leu Ala Pro Ser Ser Lys Ser Thr
            500                 505                 510
Ser Gly Gly Thr Ala Ala Leu Gly Cys Leu Val Lys Asp Tyr Phe Pro
        515                 520                 525
Glu Pro Val Thr Val Ser Trp Asn Ser Gly Ala Leu Thr Ser Gly Val
    530                 535                 540
His Thr Phe Pro Ala Val Leu Gln Ser Ser Gly Leu Tyr Ser Leu Ser
545                 550                 555                 560
Ser Val Val Thr Val Pro Ser Ser Ser Leu Gly Thr Gln Thr Tyr Ile
                565                 570                 575
Cys Asn Val Asn His Lys Pro Ser Asn Thr Lys Val Asp Lys Lys Val
            580                 585                 590

<210> 64
<211> 579
<212> PRT
<213> Artificial Sequence

<220>
<223> TSC011 mature protein comprising : HD37
      (anti-CD19) scFv, altered human IgA2 hinge, human
      IgG1 CH2, human IgG1 CH3, and human CH1

<400> 64
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp

                20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Pro Pro Pro Pro
                245                 250                 255
Pro Cys Pro Pro Cys Pro Ala Pro Glu Leu Leu Gly Gly Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Glu Tyr Lys
            340                 345                 350
Cys Lys Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Ala Ser Thr Lys Gly Pro Ser Val Phe Pro Leu Ala Pro Ser Ser
                485                 490                 495
Lys Ser Thr Ser Gly Gly Thr Ala Ala Leu Gly Cys Leu Val Lys Asp
            500                 505                 510
Tyr Phe Pro Glu Pro Val Thr Val Ser Trp Asn Ser Gly Ala Leu Thr
        515                 520                 525

Ser Gly Val His Thr Phe Pro Ala Val Leu Gln Ser Ser Gly Leu Tyr
    530                 535                 540
Ser Leu Ser Ser Val Val Thr Val Pro Ser Ser Ser Leu Gly Thr Gln
545                 550                 555                 560
Thr Tyr Ile Cys Asn Val Asn His Lys Pro Ser Asn Thr Lys Val Asp
                565                 570                 575
Lys Lys Val

<210> 65
<211> 589
<212> PRT
<213> Artificial Sequence

<220>
<223> TSC012 mature protein comprising : HD37
      (anti-CD19) scFv, altered human IgG1 hinge,  human
      IgG1 CH2, human IgG1 CH3, and human CH1

<400> 65
Asp Ile Val Leu Thr Gln Ser Pro Ala Ser Leu Ala Val Ser Leu Gly
 1               5                  10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Ser Pro Pro Ser Pro Cys Pro Pro Cys Pro
            260                 265                 270
Ala Pro Glu Leu Leu Gly Gly Pro Ser Val Phe Leu Phe Pro Pro Lys
        275                 280                 285
Pro Lys Asp Thr Leu Met Ile Ser Arg Thr Pro Glu Val Thr Cys Val
    290                 295                 300
Val Val Asp Val Ser His Glu Asp Pro Glu Val Lys Phe Asn Trp Tyr
305                 310                 315                 320
Val Asp Gly Val Glu Val His Asn Ala Lys Thr Lys Pro Arg Glu Glu
                325                 330                 335

Gln Tyr Asn Ser Thr Tyr Arg Val Val Ser Val Leu Thr Val Leu His
                340                 345                 350
Gln Asp Trp Leu Asn Gly Lys Glu Tyr Lys Cys Lys Val Ser Asn Lys
            355                 360                 365
Ala Leu Pro Ala Pro Ile Glu Lys Thr Ile Ser Lys Ala Lys Gly Gln
        370                 375                 380
Pro Arg Glu Pro Gln Val Tyr Thr Leu Pro Pro Ser Arg Asp Glu Leu
385                 390                 395                 400
Thr Lys Asn Gln Val Ser Leu Thr Cys Leu Val Lys Gly Phe Tyr Pro
                405                 410                 415
Ser Asp Ile Ala Val Glu Trp Glu Ser Asn Gly Gln Pro Glu Asn Asn
                420                 425                 430
Tyr Lys Thr Thr Pro Pro Val Leu Asp Ser Asp Gly Ser Phe Phe Leu
            435                 440                 445
Tyr Ser Lys Leu Thr Val Asp Lys Ser Arg Trp Gln Gln Gly Asn Val
    450                 455                 460
Phe Ser Cys Ser Val Met His Glu Ala Leu His Asn His Tyr Thr Gln
465                 470                 475                 480
Lys Ser Leu Ser Leu Ser Pro Gly Lys Ser Arg Ala Ser Thr Lys Gly
                485                 490                 495
Pro Ser Val Phe Pro Leu Ala Pro Ser Ser Lys Ser Thr Ser Gly Gly
                500                 505                 510
Thr Ala Ala Leu Gly Cys Leu Val Lys Asp Tyr Phe Pro Glu Pro Val
            515                 520                 525
Thr Val Ser Trp Asn Ser Gly Ala Leu Thr Ser Gly Val His Thr Phe
    530                 535                 540
Pro Ala Val Leu Gln Ser Ser Gly Leu Tyr Ser Leu Ser Ser Val Val
545                 550                 555                 560
Thr Val Pro Ser Ser Ser Leu Gly Thr Gln Thr Tyr Ile Cys Asn Val
                565                 570                 575
Asn His Lys Pro Ser Asn Thr Lys Val Asp Lys Lys Val
                580                 585

<210> 66
<211> 589
<212> PRT
<213> Artificial Sequence

<220>
<223> TSC013 mature protein comprising : HD37
      (anti-CD19) scFv, altered human IgG3 hinge, human
      IgG1 CH2, human IgG1 CH3, and human CH1

<400> 66
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140

Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Thr Pro Pro Pro Ser Pro Arg Ser Pro Cys Pro Pro Cys Pro
            260                 265                 270
Ala Pro Glu Leu Leu Gly Gly Pro Ser Val Phe Leu Phe Pro Pro Lys
        275                 280                 285
Pro Lys Asp Thr Leu Met Ile Ser Arg Thr Pro Glu Val Thr Cys Val
    290                 295                 300
Val Val Asp Val Ser His Glu Asp Pro Glu Val Lys Phe Asn Trp Tyr
305                 310                 315                 320
Val Asp Gly Val Glu Val His Asn Ala Lys Thr Lys Pro Arg Glu Glu
                325                 330                 335
Gln Tyr Asn Ser Thr Tyr Arg Val Val Ser Val Leu Thr Val Leu His
            340                 345                 350
Gln Asp Trp Leu Asn Gly Lys Glu Tyr Lys Cys Lys Val Ser Asn Lys
        355                 360                 365
Ala Leu Pro Ala Pro Ile Glu Lys Thr Ile Ser Lys Ala Lys Gly Gln
    370                 375                 380
Pro Arg Glu Pro Gln Val Tyr Thr Leu Pro Pro Ser Arg Asp Glu Leu
385                 390                 395                 400
Thr Lys Asn Gln Val Ser Leu Thr Cys Leu Val Lys Gly Phe Tyr Pro
                405                 410                 415
Ser Asp Ile Ala Val Glu Trp Glu Ser Asn Gly Gln Pro Glu Asn Asn
            420                 425                 430
Tyr Lys Thr Thr Pro Pro Val Leu Asp Ser Asp Gly Ser Phe Phe Leu
        435                 440                 445
Tyr Ser Lys Leu Thr Val Asp Lys Ser Arg Trp Gln Gln Gly Asn Val
    450                 455                 460
Phe Ser Cys Ser Val Met His Glu Ala Leu His Asn His Tyr Thr Gln
465                 470                 475                 480
Lys Ser Leu Ser Leu Ser Pro Gly Lys Ser Arg Ala Ser Thr Lys Gly
                485                 490                 495
Pro Ser Val Phe Pro Leu Ala Pro Ser Ser Lys Ser Thr Ser Gly Gly
            500                 505                 510
Thr Ala Ala Leu Gly Cys Leu Val Lys Asp Tyr Phe Pro Glu Pro Val
        515                 520                 525
Thr Val Ser Trp Asn Ser Gly Ala Leu Thr Ser Gly Val His Thr Phe
    530                 535                 540
Pro Ala Val Leu Gln Ser Ser Gly Leu Tyr Ser Leu Ser Ser Val Val
545                 550                 555                 560
Thr Val Pro Ser Ser Ser Leu Gly Thr Gln Thr Tyr Ile Cys Asn Val
                565                 570                 575
Asn His Lys Pro Ser Asn Thr Lys Val Asp Lys Lys Val
            580                 585

<210> 67
<211> 608
<212> PRT
<213> Artificial Sequence

<220>
<223> TSC014 mature protein comprising : HD37
      (anti-CD19) scFv, altered human IgD hinge, human
      IgG1 CH2, human IgG1 CH3, and human CH1

<400> 67
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Ser Pro Lys
                245                 250                 255
Ala Gln Ala Ser Ser Val Pro Thr Ala Gln Pro Gln Ala Glu Gly Ser
            260                 265                 270
Leu Ala Lys Ala Thr Thr Ala Pro Ala Thr Thr Arg Asn Thr Cys Pro
        275                 280                 285
Pro Cys Pro Ala Pro Glu Leu Leu Gly Gly Pro Ser Val Phe Leu Phe
    290                 295                 300
Pro Pro Lys Pro Lys Asp Thr Leu Met Ile Ser Arg Thr Pro Glu Val
305                 310                 315                 320
Thr Cys Val Val Val Asp Val Ser His Glu Asp Pro Glu Val Lys Phe
                325                 330                 335
Asn Trp Tyr Val Asp Gly Val Glu Val His Asn Ala Lys Thr Lys Pro
            340                 345                 350
Arg Glu Glu Gln Tyr Asn Ser Thr Tyr Arg Val Val Ser Val Leu Thr
        355                 360                 365
Val Leu His Gln Asp Trp Leu Asn Gly Lys Glu Tyr Lys Cys Lys Val
    370                 375                 380
Ser Asn Lys Ala Leu Pro Ala Pro Ile Glu Lys Thr Ile Ser Lys Ala
385                 390                 395                 400
Lys Gly Gln Pro Arg Glu Pro Gln Val Tyr Thr Leu Pro Pro Ser Arg
                405                 410                 415
Asp Glu Leu Thr Lys Asn Gln Val Ser Leu Thr Cys Leu Val Lys Gly
            420                 425                 430
Phe Tyr Pro Ser Asp Ile Ala Val Glu Trp Glu Ser Asn Gly Gln Pro
        435                 440                 445
Glu Asn Asn Tyr Lys Thr Thr Pro Pro Val Leu Asp Ser Asp Gly Ser

450                 455                 460
Phe Phe Leu Tyr Ser Lys Leu Thr Val Asp Lys Ser Arg Trp Gln Gln
465                 470                 475                 480
Gly Asn Val Phe Ser Cys Ser Val Met His Glu Ala Leu His Asn His
                485                 490                 495
Tyr Thr Gln Lys Ser Leu Ser Leu Ser Pro Gly Lys Ser Arg Ala Ser
            500                 505                 510
Thr Lys Gly Pro Ser Val Phe Pro Leu Ala Pro Ser Ser Lys Ser Thr
        515                 520                 525
Ser Gly Gly Thr Ala Ala Leu Gly Cys Leu Val Lys Asp Tyr Phe Pro
    530                 535                 540
Glu Pro Val Thr Val Ser Trp Asn Ser Gly Ala Leu Thr Ser Gly Val
545                 550                 555                 560
His Thr Phe Pro Ala Val Leu Gln Ser Ser Gly Leu Tyr Ser Leu Ser
                565                 570                 575
Ser Val Val Thr Val Pro Ser Ser Ser Leu Gly Thr Gln Thr Tyr Ile
            580                 585                 590
Cys Asn Val Asn His Lys Pro Ser Asn Thr Lys Val Asp Lys Lys Val
        595                 600                 605

<210> 68
<211> 680
<212> PRT
<213> Artificial Sequence

<220>
<223> TSC015 mature protein comprising : HD37
      (anti-CD19) scFv, altered human IgE CH2 hinge,
      human IgG1 CH2, human IgG1 CH3, and human CH1

<400> 68
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Ser Arg Asp Phe

                    245                     250                     255
Thr Pro Pro Thr Val Lys Ile Leu Gln Ser Ser Ser Asp Gly Gly Gly
            260                     265                     270
His Phe Pro Pro Thr Ile Gln Leu Leu Cys Leu Val Ser Gly Tyr Thr
        275                     280                     285
Pro Gly Thr Ile Asn Ile Thr Trp Leu Glu Asp Gly Gln Val Met Asp
    290                     295                     300
Val Asp Leu Ser Thr Ala Ser Thr Thr Gln Glu Gly Glu Leu Ala Ser
305                     310                     315                     320
Thr Gln Ser Glu Leu Thr Leu Ser Gln Lys His Trp Leu Ser Asp Arg
                    325                     330                     335
Thr Tyr Thr Cys Gln Val Thr Tyr Gln Gly His Thr Phe Glu Asp Ser
            340                     345                     350
Thr Lys Lys Ser Ala Cys Pro Pro Cys Ser Gly Ala Pro Glu Leu Leu
        355                     360                     365
Gly Gly Pro Ser Val Phe Leu Phe Pro Pro Lys Pro Lys Asp Thr Leu
    370                     375                     380
Met Ile Ser Arg Thr Pro Glu Val Thr Cys Val Val Val Asp Val Ser
385                     390                     395                     400
His Glu Asp Pro Glu Val Lys Phe Asn Trp Tyr Val Asp Gly Val Glu
                    405                     410                     415
Val His Asn Ala Lys Thr Lys Pro Arg Glu Glu Gln Tyr Asn Ser Thr
            420                     425                     430
Tyr Arg Val Val Ser Val Leu Thr Val Leu His Gln Asp Trp Leu Asn
        435                     440                     445
Gly Lys Glu Tyr Lys Cys Lys Val Ser Asn Lys Ala Leu Pro Ala Pro
    450                     455                     460
Ile Glu Lys Thr Ile Ser Lys Ala Lys Gly Gln Pro Arg Glu Pro Gln
465                     470                     475                     480
Val Tyr Thr Leu Pro Pro Ser Arg Asp Glu Leu Thr Lys Asn Gln Val
                    485                     490                     495
Ser Leu Thr Cys Leu Val Lys Gly Phe Tyr Pro Ser Asp Ile Ala Val
            500                     505                     510
Glu Trp Glu Ser Asn Gly Gln Pro Glu Asn Asn Tyr Lys Thr Thr Pro
        515                     520                     525
Pro Val Leu Asp Ser Asp Gly Ser Phe Phe Leu Tyr Ser Lys Leu Thr
    530                     535                     540
Val Asp Lys Ser Arg Trp Gln Gln Gly Asn Val Phe Ser Cys Ser Val
545                     550                     555                     560
Met His Glu Ala Leu His Asn His Tyr Thr Gln Lys Ser Leu Ser Leu
                    565                     570                     575
Ser Pro Gly Lys Ser Arg Ala Ser Thr Lys Gly Pro Ser Val Phe Pro
            580                     585                     590
Leu Ala Pro Ser Ser Lys Ser Thr Ser Gly Gly Thr Ala Ala Leu Gly
        595                     600                     605
Cys Leu Val Lys Asp Tyr Phe Pro Glu Pro Val Thr Val Ser Trp Asn
    610                     615                     620
Ser Gly Ala Leu Thr Ser Gly Val His Thr Phe Pro Ala Val Leu Gln
625                     630                     635                     640
Ser Ser Gly Leu Tyr Ser Leu Ser Ser Val Val Thr Val Pro Ser Ser
                    645                     650                     655
Ser Leu Gly Thr Gln Thr Tyr Ile Cys Asn Val Asn His Lys Pro Ser
            660                     665                     670
Asn Thr Lys Val Asp Lys Lys Val
        675                     680


<210> 69
<211> 687
<212> PRT
<213> Artificial Sequence

<220>

<223> TSC016 mature protein comprising : HD37
      (anti-CD19) scFv, altered human IgM CH2 hinge,
      human IgG1 CH2, human IgG1 CH3, and human CH1

<400> 69
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Ile Ala Glu Leu
                245                 250                 255
Pro Pro Lys Val Ser Val Phe Val Pro Pro Arg Asp Gly Phe Phe Gly
            260                 265                 270
Asn Pro Arg Lys Ser Lys Leu Ile Cys Gln Ala Thr Gly Phe Ser Pro
        275                 280                 285
Arg Gln Ile Gln Val Ser Trp Leu Arg Glu Gly Lys Gln Val Gly Ser
    290                 295                 300
Gly Val Thr Thr Asp Gln Val Gln Ala Glu Ala Lys Glu Ser Gly Pro
305                 310                 315                 320
Thr Thr Tyr Lys Val Thr Ser Thr Leu Thr Ile Lys Glu Ser Asp Trp
                325                 330                 335
Leu Ser Gln Ser Met Phe Thr Cys Arg Val Asp His Arg Gly Leu Thr
            340                 345                 350
Phe Gln Gln Asn Ala Ser Ser Met Ser Val Pro Cys Pro Pro Cys Pro
        355                 360                 365
Ser Gly Ala Pro Glu Leu Leu Gly Gly Pro Ser Val Phe Leu Phe Pro
    370                 375                 380
Pro Lys Pro Lys Asp Thr Leu Met Ile Ser Arg Thr Pro Glu Val Thr
385                 390                 395                 400
Cys Val Val Val Asp Val Ser His Glu Asp Pro Glu Val Lys Phe Asn
                405                 410                 415
Trp Tyr Val Asp Gly Val Glu Val His Asn Ala Lys Thr Lys Pro Arg
            420                 425                 430
Glu Glu Gln Tyr Asn Ser Thr Tyr Arg Val Val Ser Val Leu Thr Val
        435                 440                 445
Leu His Gln Asp Trp Leu Asn Gly Lys Glu Tyr Lys Cys Lys Val Ser
    450                 455                 460

Asn Lys Ala Leu Pro Ala Pro Ile Glu Lys Thr Ile Ser Lys Ala Lys
465                 470                 475                 480
Gly Gln Pro Arg Glu Pro Gln Val Tyr Thr Leu Pro Pro Ser Arg Asp
                485                 490                 495
Glu Leu Thr Lys Asn Gln Val Ser Leu Thr Cys Leu Val Lys Gly Phe
            500                 505                 510
Tyr Pro Ser Asp Ile Ala Val Glu Trp Glu Ser Asn Gly Gln Pro Glu
        515                 520                 525
Asn Asn Tyr Lys Thr Thr Pro Pro Val Leu Asp Ser Asp Gly Ser Phe
    530                 535                 540
Phe Leu Tyr Ser Lys Leu Thr Val Asp Lys Ser Arg Trp Gln Gln Gly
545                 550                 555                 560
Asn Val Phe Ser Cys Ser Val Met His Glu Ala Leu His Asn His Tyr
                565                 570                 575
Thr Gln Lys Ser Leu Ser Leu Ser Pro Gly Lys Ser Arg Ala Ser Thr
            580                 585                 590
Lys Gly Pro Ser Val Phe Pro Leu Ala Pro Ser Ser Lys Ser Thr Ser
        595                 600                 605
Gly Gly Thr Ala Ala Leu Gly Cys Leu Val Lys Asp Tyr Phe Pro Glu
    610                 615                 620
Pro Val Thr Val Ser Trp Asn Ser Gly Ala Leu Thr Ser Gly Val His
625                 630                 635                 640
Thr Phe Pro Ala Val Leu Gln Ser Ser Gly Leu Tyr Ser Leu Ser Ser
                645                 650                 655
Val Val Thr Val Pro Ser Ser Ser Leu Gly Thr Gln Thr Tyr Ile Cys
            660                 665                 670
Asn Val Asn His Lys Pro Ser Asn Thr Lys Val Asp Lys Lys Val
        675                 680                 685

<210> 70
<211> 575
<212> PRT
<213> Artificial Sequence

<220>
<223> TSC017 mature protein comprising : P2C2
      (anti-CD79b) scFv, human IgG1 SCC-P hinge, human
      IgG1 CH2, human IgG1 CH3 and human CH1

<400> 70
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser
    50                  55                  60
Asp Arg Phe Ser Gly Ser Gly Ser Gly Thr Asp Phe Thr Leu Glu Ile
65                  70                  75                  80
Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110
Gly Gly Gly Gly Ser Gly Gly Gly Gly Ser Gly Gly Gly Gly Ser Gln
        115                 120                 125
Ile Gln Leu Val Gln Ser Gly Pro Glu Leu Lys Lys Pro Gly Glu Thr
    130                 135                 140
Val Lys Ile Ser Cys Lys Ala Ser Gly Tyr Thr Phe Thr Asp Phe Ser
145                 150                 155                 160
Met His Trp Val Arg Gln Ala Pro Gly Lys Gly Leu Lys Trp Met Gly
                165                 170                 175

Trp Ile Asn Thr Glu Thr Gly Glu Pro Thr Tyr Ala Asp Asp Phe Arg
            180                 185                 190
Gly Arg Phe Ala Phe Ser Leu Glu Thr Ser Ala Ser Thr Ala Tyr Leu
        195                 200                 205
Gln Ile Asn Asn Leu Lys Asn Glu Asp Thr Ala Thr Tyr Phe Cys Thr
    210                 215                 220
Trp Ser Ala Tyr Trp Gly Gln Gly Thr Leu Val Thr Val Ser Ala Ser
225                 230                 235                 240
Ser Glu Pro Lys Ser Ser Asp Lys Thr His Thr Cys Pro Pro Cys Pro
                245                 250                 255
Ser Gly Ala Pro Glu Leu Leu Gly Gly Pro Ser Val Phe Leu Phe Pro
            260                 265                 270
Pro Lys Pro Lys Asp Thr Leu Met Ile Ser Arg Thr Pro Glu Val Thr
        275                 280                 285
Cys Val Val Val Asp Val Ser His Glu Asp Pro Glu Val Lys Phe Asn
    290                 295                 300
Trp Tyr Val Asp Gly Val Glu Val His Asn Ala Lys Thr Lys Pro Arg
305                 310                 315                 320
Glu Glu Gln Tyr Asn Ser Thr Tyr Arg Val Val Ser Val Leu Thr Val
                325                 330                 335
Leu His Gln Asp Trp Leu Asn Gly Lys Glu Tyr Lys Cys Lys Val Ser
            340                 345                 350
Asn Lys Ala Leu Pro Ala Pro Ile Glu Lys Thr Ile Ser Lys Ala Lys
        355                 360                 365
Gly Gln Pro Arg Glu Pro Gln Val Tyr Thr Leu Pro Pro Ser Arg Asp
    370                 375                 380
Glu Leu Thr Lys Asn Gln Val Ser Leu Thr Cys Leu Val Lys Gly Phe
385                 390                 395                 400
Tyr Pro Ser Asp Ile Ala Val Glu Trp Glu Ser Asn Gly Gln Pro Glu
                405                 410                 415
Asn Asn Tyr Lys Thr Thr Pro Pro Val Leu Asp Ser Asp Gly Ser Phe
            420                 425                 430
Phe Leu Tyr Ser Lys Leu Thr Val Asp Lys Ser Arg Trp Gln Gln Gly
        435                 440                 445
Asn Val Phe Ser Cys Ser Val Met His Glu Ala Leu His Asn His Tyr
    450                 455                 460
Thr Gln Lys Ser Leu Ser Leu Ser Pro Gly Lys Ser Arg Ala Ser Thr
465                 470                 475                 480
Lys Gly Pro Ser Val Phe Pro Leu Ala Pro Ser Ser Lys Ser Thr Ser
                485                 490                 495
Gly Gly Thr Ala Ala Leu Gly Cys Leu Val Lys Asp Tyr Phe Pro Glu
            500                 505                 510
Pro Val Thr Val Ser Trp Asn Ser Gly Ala Leu Thr Ser Gly Val His
        515                 520                 525
Thr Phe Pro Ala Val Leu Gln Ser Ser Gly Leu Tyr Ser Leu Ser Ser
    530                 535                 540
Val Val Thr Val Pro Ser Ser Ser Leu Gly Thr Gln Thr Tyr Ile Cys
545                 550                 555                 560
Asn Val Asn His Lys Pro Ser Asn Thr Lys Val Asp Lys Lys Val
                565                 570                 575

<210> 71
<211> 583
<212> PRT
<213> Artificial Sequence

<220>
<223> TSC018 mature protein comprising : M0042
      (anti-HLA-DR) scFv, human IgG1 SCC-P hinge, human
      IgG1 CH2, human IgG1 CH3, and human CH1

<400> 71

Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Val
        35                  40                  45
Tyr Ser Val Ser Ser Leu Gln Ser Gly Val Pro Ser Arg Phe Ser Gly
    50                  55                  60
Ser Gly Ser Gly Thr Asp Phe Thr Leu Thr Ile Asn Ser Leu Gln Pro
65                  70                  75                  80
Glu Asp Phe Ala Thr Tyr Tyr Cys Gln Gln Ser Tyr Ser Thr Pro Phe
                85                  90                  95
Thr Phe Gly Pro Gly Thr Lys Val Asp Ile Lys Gly Gly Gly Gly Ser
            100                 105                 110
Gly Gly Gly Gly Ser Gly Gly Gly Gly Ser Gly Gly Gly Gly Ser Glu
        115                 120                 125
Val Gln Leu Leu Glu Ser Gly Gly Gly Leu Val Gln Pro Gly Gly Ser
    130                 135                 140
Leu Arg Leu Ser Cys Ala Ala Ser Gly Phe Thr Phe Ser His Tyr Tyr
145                 150                 155                 160
Met Ser Trp Val Arg Gln Ala Pro Gly Lys Gly Leu Glu Trp Val Ser
                165                 170                 175
Tyr Ile Gly Pro Ser Gly Gly Phe Thr Ala Tyr Ala Asp Ser Val Lys
            180                 185                 190
Gly Arg Phe Thr Ile Ser Arg Asp Asn Ser Lys Asn Thr Leu Tyr Leu
        195                 200                 205
Gln Met Asn Ser Leu Arg Ala Glu Asp Thr Ala Val Tyr Tyr Cys Ala
    210                 215                 220
Arg Asp Gly Trp Val Gly Ser Glu Asn Tyr Ser Gly Met Asp Val Trp
225                 230                 235                 240
Gly Gln Gly Thr Thr Val Ser Ser Ser Glu Pro Lys Ser Ser Asp Lys
                245                 250                 255
Thr His Thr Cys Pro Pro Cys Pro Ser Gly Ala Pro Glu Leu Leu Gly
            260                 265                 270
Gly Pro Ser Val Phe Leu Phe Pro Pro Lys Pro Lys Asp Thr Leu Met
        275                 280                 285
Ile Ser Arg Thr Pro Glu Val Thr Cys Val Val Val Asp Val Ser His
    290                 295                 300
Glu Asp Pro Glu Val Lys Phe Asn Trp Tyr Val Asp Gly Val Glu Val
305                 310                 315                 320
His Asn Ala Lys Thr Lys Pro Arg Glu Glu Gln Tyr Asn Ser Thr Tyr
                325                 330                 335
Arg Val Val Ser Val Leu Thr Val Leu His Gln Asp Trp Leu Asn Gly
            340                 345                 350
Lys Glu Tyr Lys Cys Lys Val Ser Asn Lys Ala Leu Pro Ala Pro Ile
        355                 360                 365
Glu Lys Thr Ile Ser Lys Ala Lys Gly Gln Pro Arg Glu Pro Gln Val
    370                 375                 380
Tyr Thr Leu Pro Pro Ser Arg Asp Glu Leu Thr Lys Asn Gln Val Ser
385                 390                 395                 400
Leu Thr Cys Leu Val Lys Gly Phe Tyr Pro Ser Asp Ile Ala Val Glu
                405                 410                 415
Trp Glu Ser Asn Gly Gln Pro Glu Asn Asn Tyr Lys Thr Thr Pro Pro
            420                 425                 430
Val Leu Asp Ser Asp Gly Ser Phe Phe Leu Tyr Ser Lys Leu Thr Val
        435                 440                 445
Asp Lys Ser Arg Trp Gln Gln Gly Asn Val Phe Ser Cys Ser Val Met
    450                 455                 460
His Glu Ala Leu His Asn His Tyr Thr Gln Lys Ser Leu Ser Leu Ser
465                 470                 475                 480
Pro Gly Lys Ser Arg Ala Ser Thr Lys Gly Pro Ser Val Phe Pro Leu
                485                 490                 495
Ala Pro Ser Ser Lys Ser Thr Ser Gly Gly Thr Ala Ala Leu Gly Cys

                    500                     505                     510
Leu Val Lys Asp Tyr Phe Pro Glu Pro Val Thr Val Ser Trp Asn Ser
        515                     520                     525
Gly Ala Leu Thr Ser Gly Val His Thr Phe Pro Ala Val Leu Gln Ser
    530                     535                     540
Ser Gly Leu Tyr Ser Leu Ser Ser Val Val Thr Val Pro Ser Ser Ser
545                     550                     555                     560
Leu Gly Thr Gln Thr Tyr Ile Cys Asn Val Asn His Lys Pro Ser Asn
                    565                     570                     575
Thr Lys Val Asp Lys Lys Val
                580

<210> 72
<211> 590
<212> PRT
<213> Artificial Sequence

<220>
<223> TSC019 mature protein comprising : G19-4
      (anti-CD3) scFv, human IgG1 SCC-P hinge, human
      CH2, human CH3, and human Ck(YAE)

<400> 72
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Glu Pro Lys Ser Ser Asp Lys
                245                 250                 255
Thr His Thr Cys Pro Pro Cys Pro Ser Gly Ala Pro Glu Leu Leu Gly
            260                 265                 270
Gly Pro Ser Val Phe Leu Phe Pro Pro Lys Pro Lys Asp Thr Leu Met
        275                 280                 285
Ile Ser Arg Thr Pro Glu Val Thr Cys Val Val Val Asp Val Ser His
    290                 295                 300
Glu Asp Pro Glu Val Lys Phe Asn Trp Tyr Val Asp Gly Val Glu Val

305                 310                 315                 320
His Asn Ala Lys Thr Lys Pro Arg Glu Glu Gln Tyr Asn Ser Thr Tyr
                325                 330                 335
Arg Val Val Ser Val Leu Thr Val Leu His Gln Asp Trp Leu Asn Gly
            340                 345                 350
Lys Glu Tyr Lys Cys Lys Val Ser Asn Lys Ala Leu Pro Ala Pro Ile
        355                 360                 365
Glu Lys Thr Ile Ser Lys Ala Lys Gly Gln Pro Arg Glu Pro Gln Val
    370                 375                 380
Tyr Thr Leu Pro Pro Ser Arg Asp Glu Leu Thr Lys Asn Gln Val Ser
385                 390                 395                 400
Leu Thr Cys Leu Val Lys Gly Phe Tyr Pro Ser Asp Ile Ala Val Glu
                405                 410                 415
Trp Glu Ser Asn Gly Gln Pro Glu Asn Asn Tyr Lys Thr Thr Pro Pro
            420                 425                 430
Val Leu Asp Ser Asp Gly Ser Phe Phe Leu Tyr Ser Lys Leu Thr Val
        435                 440                 445
Asp Lys Ser Arg Trp Gln Gln Gly Asn Val Phe Ser Cys Ser Val Met
    450                 455                 460
His Glu Ala Leu His Asn His Tyr Thr Gln Lys Ser Leu Ser Leu Ser
465                 470                 475                 480
Pro Gly Lys Ser Arg Thr Val Ala Ala Pro Ser Val Phe Ile Phe Pro
                485                 490                 495
Pro Ser Asp Glu Gln Leu Lys Ser Gly Thr Ala Ser Val Val Cys Leu
            500                 505                 510
Leu Asn Tyr Phe Tyr Pro Arg Glu Ala Lys Val Gln Trp Lys Val Asp
        515                 520                 525
Asn Ala Leu Gln Ser Gly Asn Ser Gln Glu Ser Ala Thr Glu Gln Asp
    530                 535                 540
Ser Lys Asp Ser Thr Tyr Ser Leu Ser Ser Glu Leu Thr Leu Ser Lys
545                 550                 555                 560
Ala Asp Tyr Glu Lys His Lys Val Tyr Ala Cys Glu Val Thr His Gln
                565                 570                 575
Gly Leu Ser Ser Pro Val Thr Lys Ser Phe Asn Arg Gly Glu
            580                 585                 590

<210> 73
<211> 2292
<212> DNA
<213> Artificial Sequence

<220>
<223> 4C04 (anti-RON) x G19-4 (anti-CD3) Scorpion DNA
      sequence (SO266)

<400> 73
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt gggaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gcgagcccaa atcttctgac aaaactcaca catgcccacc gtgcccagca 840
cctgaactcc tgggtggacc gtcagtcttc ctcttccccc caaaacccaa ggacaccctc 900
atgatctccc ggacccctga ggtcacatgc gtggtggtgg acgtgagcca cgaagaccct 960

gaggtcaagt tcaactggta cgtggacggc gtggaggtgc ataatgccaa gacaaagccg 1020
cgggaggagc agtacaacag cacgtaccgt gtggtcagcg tcctcaccgt cctgcaccag 1080
gactggctga atggcaagga gtacaagtgc aaggtctcca acaaagccct cccagccccc 1140
atcgagaaaa ccatctccaa agccaaaggg cagccccgag aaccacaggt gtacaccctg 1200
cccccatccc gggatgagct gaccaagaac caggtcagcc tgacctgcct ggtcaaaggc 1260
ttctatccaa gcgacatcgc cgtggagtgg gagagcaatg ggcagccgga gaacaactac 1320
aagaccacgc ctcccgtgct ggactccgac ggctccttct tcctctacag caagctcacc 1380
gtggacaaga gcaggtggca gcaggggaac gtcttctcat gctccgtgat gcatgaggct 1440
ctgcacaacc actacacgca gaagagcctc tccctgtctc cgggtcagag gcacaacaat 1500
tcttccctga atacaggaac tcagatggca ggtcattctc cgaattctga ggtccagctg 1560
caacagtctg gacctgaact ggtgaagcct ggagcttcaa tgaagatttc ctgcaaggcc 1620
tctggttact cattcactgg ctacatcgtg aactggctga agcagagcca tggaaagaac 1680
cttgagtgga ttggacttat taatccatac aaaggtctta ctacctacaa ccagaaattc 1740
aagggcaagg ccacattaac tgtagacaag tcatccagca cagcctacat ggagctcctc 1800
agtctgacat ctgaagactc tgcagtctat tactgtgcaa gatctgggta ctatggtgac 1860
tcggactggt acttcgatgt ctggggcgca gggaccacgg tcaccgtctc aagcggtggc 1920
ggagggtctg ggggtggcgg atccggaggt ggtggctctg cacaagacat ccagatgaca 1980
cagactacat cctccctgtc tgcctctctg ggagacagag tcaccatcag ttgcagggca 2040
agtcaggaca ttcgcaatta tttaaactgg tatcagcaga aaccagatgg aactgttaaa 2100
ctcctgatct actacacatc aagattacac tcaggagtcc catcaaggtt cagtggcagt 2160
gggtctggaa cagattattc tctcaccatt gccaacctgc aaccagaaga tattgccact 2220
tacttttgcc aacagggtaa tacgcttccg tggacgttcg gtggaggcac caaactggta 2280
accaaacgat aa 2292

<210> 74
<211> 743
<212> PRT
<213> Artificial Sequence

<220>
<223> 4C04 (anti-RON) x G19-4 (anti-CD3) Scorpion mature
      protein sequence (SO266)

<400> 74
Asp Ile Gln Met Thr Gln Ser Pro Ala Ser Gln Ser Ala Ser Leu Gly
1               5                   10                  15
Glu Ser Val Thr Ile Thr Cys Leu Ala Ser Gln Thr Ile Gly Thr Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Ala
    50                  55                  60
Ser Gly Ser Gly Thr Lys Phe Ser Phe Lys Ile Asp Ser Leu Gln Ser
65                  70                  75                  80
Glu Asp Phe Val Ser Tyr Tyr Cys Gln Gln Leu Tyr Asn Thr Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Ile Gln Leu Gln Gln
        115                 120                 125
Ser Gly Pro Glu Leu Val Lys Pro Gly Thr Ser Val Lys Ile Ser Cys
    130                 135                 140
Lys Ala Ser Asp Tyr Thr Leu Thr Asp Phe Tyr Met Asn Trp Val Arg
145                 150                 155                 160
Gln Lys Pro Gly Gln Gly Leu Glu Trp Ile Gly Arg Ile Tyr Pro Gly
                165                 170                 175
Thr Asp Lys Thr Arg Tyr Asn Glu Lys Phe Arg Asp Lys Ala Ile Leu
            180                 185                 190
Thr Val Asp Thr Ser Ser Ser Thr Ala Tyr Met Gln Leu Ser Ser Leu
        195                 200                 205
Thr Ser Glu Asp Thr Ala Val Tyr Phe Cys Ala Arg Ser Ala Tyr Tyr
    210                 215                 220
Gly Asn Tyr Val Ala Met Asp Tyr Trp Gly Gln Gly Thr Ser Val Thr

225                 230                 235                 240
Val Ser Ser Ser Glu Pro Lys Ser Ser Asp Lys Thr His Thr Cys Pro
                245                 250                 255
Pro Cys Pro Ala Pro Glu Leu Leu Gly Gly Pro Ser Val Phe Leu Phe
            260                 265                 270
Pro Pro Lys Pro Lys Asp Thr Leu Met Ile Ser Arg Thr Pro Glu Val
        275                 280                 285
Thr Cys Val Val Val Asp Val Ser His Glu Asp Pro Glu Val Lys Phe
    290                 295                 300
Asn Trp Tyr Val Asp Gly Val Glu Val His Asn Ala Lys Thr Lys Pro
305                 310                 315                 320
Arg Glu Glu Gln Tyr Asn Ser Thr Tyr Arg Val Val Ser Val Leu Thr
                325                 330                 335
Val Leu His Gln Asp Trp Leu Asn Gly Lys Glu Tyr Lys Cys Lys Val
            340                 345                 350
Ser Asn Lys Ala Leu Pro Ala Pro Ile Glu Lys Thr Ile Ser Lys Ala
        355                 360                 365
Lys Gly Gln Pro Arg Glu Pro Gln Val Tyr Thr Leu Pro Pro Ser Arg
    370                 375                 380
Asp Glu Leu Thr Lys Asn Gln Val Ser Leu Thr Cys Leu Val Lys Gly
385                 390                 395                 400
Phe Tyr Pro Ser Asp Ile Ala Val Glu Trp Glu Ser Asn Gly Gln Pro
                405                 410                 415
Glu Asn Asn Tyr Lys Thr Thr Pro Pro Val Leu Asp Ser Asp Gly Ser
            420                 425                 430
Phe Phe Leu Tyr Ser Lys Leu Thr Val Asp Lys Ser Arg Trp Gln Gln
        435                 440                 445
Gly Asn Val Phe Ser Cys Ser Val Met His Glu Ala Leu His Asn His
    450                 455                 460
Tyr Thr Gln Lys Ser Leu Ser Leu Ser Pro Gly Gln Arg His Asn Asn
465                 470                 475                 480
Ser Ser Leu Asn Thr Gly Thr Gln Met Ala Gly His Ser Pro Asn Ser
                485                 490                 495
Glu Val Gln Leu Gln Gln Ser Gly Pro Glu Leu Val Lys Pro Gly Ala
            500                 505                 510
Ser Met Lys Ile Ser Cys Lys Ala Ser Gly Tyr Ser Phe Thr Gly Tyr
        515                 520                 525
Ile Val Asn Trp Leu Lys Gln Ser His Gly Lys Asn Leu Glu Trp Ile
    530                 535                 540
Gly Leu Ile Asn Pro Tyr Lys Gly Leu Thr Thr Tyr Asn Gln Lys Phe
545                 550                 555                 560
Lys Gly Lys Ala Thr Leu Thr Val Asp Lys Ser Ser Ser Thr Ala Tyr
                565                 570                 575
Met Glu Leu Leu Ser Leu Thr Ser Glu Asp Ser Ala Val Tyr Tyr Cys
            580                 585                 590
Ala Arg Ser Gly Tyr Tyr Gly Asp Ser Asp Trp Tyr Phe Asp Val Trp
        595                 600                 605
Gly Ala Gly Thr Thr Val Thr Val Ser Ser Gly Gly Gly Gly Ser Gly
    610                 615                 620
Gly Gly Gly Ser Gly Gly Gly Gly Ser Ala Gln Asp Ile Gln Met Thr
625                 630                 635                 640
Gln Thr Thr Ser Ser Leu Ser Ala Ser Leu Gly Asp Arg Val Thr Ile
                645                 650                 655
Ser Cys Arg Ala Ser Gln Asp Ile Arg Asn Tyr Leu Asn Trp Tyr Gln
            660                 665                 670
Gln Lys Pro Asp Gly Thr Val Lys Leu Leu Ile Tyr Tyr Thr Ser Arg
        675                 680                 685
Leu His Ser Gly Val Pro Ser Arg Phe Ser Gly Ser Gly Ser Gly Thr
    690                 695                 700
Asp Tyr Ser Leu Thr Ile Ala Asn Leu Gln Pro Glu Asp Ile Ala Thr
705                 710                 715                 720
Tyr Phe Cys Gln Gln Gly Asn Thr Leu Pro Trp Thr Phe Gly Gly Gly
                725                 730                 735

Thr Lys Leu Val Thr Lys Arg
            740

<210> 75

<400> 75
 000

<210> 76

<400> 76
 000

<210> 77
<211> 510
<212> PRT
<213> Artificial Sequence

<220>
<223> Mutated M0039

<220>
<221> SIGNAL
<222> (1)...(22)

<400> 77
Met Asp Phe Gln Val Gln Ile Phe Ser Phe Leu Leu Ile Ser Ala Ser
        -20                 -15                 -10
Val Ile Met Ser Arg Gly Val Gln Val Gln Leu Lys Glu Ser Gly Pro
    -5                  1               5                   10
Gly Leu Val Ala Pro Ser Gln Ser Leu Ser Ile Thr Cys Thr Val Ser
                15                  20                  25
Gly Phe Ser Leu Thr Gly Tyr Gly Val Asn Trp Val Arg Gln Pro Pro
            30                  35                  40
Gly Lys Gly Leu Glu Trp Leu Gly Met Ile Trp Gly Asp Gly Ser Thr
        45                  50                  55
Asp Tyr Asn Ser Ala Leu Lys Ser Arg Leu Ser Ile Thr Lys Asp Asn
    60                  65                  70
Ser Lys Ser Gln Val Phe Leu Lys Met Asn Ser Leu Gln Thr Asp Asp
75                  80                  85                  90
Thr Ala Arg Tyr Tyr Cys Ala Arg Asp Gly Tyr Ser Asn Phe His Tyr
                95                  100                 105
Tyr Val Met Asp Tyr Trp Gly Gln Gly Thr Ser Val Thr Val Ser Ser
            110                 115                 120
Gly Gly Gly Gly Ser Gly Gly Gly Gly Ser Gly Gly Gly Gly Ser Gly
        125                 130                 135
Gly Gly Gly Ser Asp Ile Val Leu Thr Gln Ser Pro Ala Ser Leu Ala
    140                 145                 150
Val Ser Leu Gly Gln Arg Ala Thr Ile Ser Cys Arg Ala Ser Glu Ser
155                 160                 165                 170
Val Glu Tyr Tyr Val Thr Ser Leu Met Gln Trp Tyr Gln Gln Lys Pro
                175                 180                 185
Gly Gln Pro Pro Lys Leu Leu Ile Ser Ala Ala Ser Asn Val Glu Ser
            190                 195                 200
Gly Val Pro Ala Arg Phe Ser Gly Ser Gly Ser Gly Thr Asp Phe Ser
        205                 210                 215
Leu Asn Ile His Pro Val Glu Glu Asp Asp Ile Ala Met Tyr Phe Cys
    220                 225                 230
Gln Gln Ser Arg Lys Val Pro Trp Thr Phe Gly Gly Gly Thr Lys Leu
235                 240                 245                 250
Glu Ile Lys Arg Asp Gln Glu Pro Lys Ser Ser Asp Lys Thr His Thr
                255                 260                 265

Ser Pro Pro Ser Pro Ala Pro Glu Leu Leu Gly Gly Pro Ser Val Phe
            270                 275                 280
Leu Phe Pro Pro Lys Pro Lys Asp Thr Leu Met Ile Ser Arg Thr Pro
        285                 290                 295
Glu Val Thr Cys Val Val Val Asp Val Ser His Glu Asp Pro Glu Val
    300                 305                 310
Lys Phe Asn Trp Tyr Val Asp Gly Val Glu Val His Asn Ala Lys Thr
315                 320                 325                 330
Lys Pro Arg Glu Glu Gln Tyr Asn Ser Thr Tyr Arg Val Val Ser Val
                335                 340                 345
Leu Thr Val Leu His Gln Asp Trp Leu Asn Gly Lys Glu Tyr Lys Cys
            350                 355                 360
Lys Val Ser Asn Lys Ala Leu Pro Ala Pro Ile Glu Lys Thr Ile Ser
        365                 370                 375
Lys Ala Lys Gly Gln Pro Arg Glu Pro Gln Val Tyr Thr Leu Pro Pro
    380                 385                 390
Ser Arg Asp Glu Leu Thr Lys Asn Gln Val Ser Leu Thr Cys Leu Val
395                 400                 405                 410
Lys Gly Phe Tyr Pro Ser Asp Ile Ala Val Glu Trp Glu Ser Asn Gly
                415                 420                 425
Gln Pro Glu Asn Asn Tyr Lys Thr Thr Pro Pro Val Leu Asp Ser Asp
            430                 435                 440
Gly Ser Phe Phe Leu Tyr Ser Lys Leu Thr Val Asp Lys Ser Arg Trp
        445                 450                 455
Gln Gln Gly Asn Val Phe Ser Cys Ser Val Met His Glu Ala Leu His
    460                 465                 470
Asn His Tyr Thr Gln Lys Ser Leu Ser Leu Ser Pro Gly Lys
475                 480                 485


<210> 78
<211> 20
<212> PRT
<213> Artificial Sequence

<220>
<223> H68 linker (scorpion linker derived from NKG2D)

<400> 78
Asn Ser Leu Phe Asn Gln Glu Val Gln Ile Pro Leu Thr Glu Ser Tyr
1               5                   10                  15
Ser Pro Asn Ser
            20


<210> 79
<211> 366
<212> DNA
<213> Artificial Sequence

<220>
<223> VH of A2

<400> 79
gaagttcaat tgttagagtc tggtggcggt cttgttcagc ctggtggttc tttacgtctt 60
tcttgcgctg cttccggatt cactttctct ttttaccaga tgcattgggt tcgccaagct 120
cctggtaaag gtttggagtg ggtttctggt atctatcctt ctggtggcta tactaagtat 180
gctgactccg ttaaaggtcg cttcactatc tctagagaca actctaagaa tactctctac 240
ttgcagatga acagcttaag ggctgaggac actgcagtct actattgtgc gagagatcta 300
gggtacggca gtagctggta ctactttgac tactggggcc agggaaccct ggtcaccgtc 360
tcaagc                                                            366

<210> 80

<211> 122
<212> PRT
<213> Artificial Sequence

<220>
<223> VH of A2

<400> 80
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Phe Tyr
            20                  25                  30
Gln Met His Trp Val Arg Gln Ala Pro Gly Lys Gly Leu Glu Trp Val
        35                  40                  45
Ser Gly Ile Tyr Pro Ser Gly Gly Tyr Thr Lys Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Leu Gly Tyr Gly Ser Ser Trp Tyr Tyr Phe Asp Tyr Trp
            100                 105                 110
Gly Gln Gly Thr Leu Val Thr Val Ser Ser
        115                 120


<210> 81
<400> 81
000

<210> 82
<400> 82
000

<210> 83
<211> 330
<212> DNA
<213> Artificial Sequence

<220>
<223> VL of A2

<400> 83
cagagcgctt tgactcagcc tgcctccgtg tctgggtctc ctggacagtc gatcaccatc 60
tcctgcagtg gaaccgacag tgacgttggt ggttataacc atgtctcctg gtaccaacaa 120
cacccaggca aggcccccaa actcataatt tatgatgtcg atcatcggcc ctcagggatc 180
tctaaccgtt tctctggctc caagtctggc aacacggcct ccctgaccat ctctgggctc 240
caggctgagg acgaggctga ttattactgc agctcatata gaagcggtag cacttatgtc 300
ttcggaactg ggaccaaggt caccgtccta                                  330

<210> 84
<211> 110
<212> PRT
<213> Artificial Sequence

<220>
<223> VL of A2

<400> 84
Gln Ser Ala Leu Thr Gln Pro Ala Ser Val Ser Gly Ser Pro Gly Gln
1               5                   10                  15
Ser Ile Thr Ile Ser Cys Ser Gly Thr Asp Ser Asp Val Gly Gly Tyr
            20                  25                  30

Asn His Val Ser Trp Tyr Gln Gln His Pro Gly Lys Ala Pro Lys Leu
        35                  40                  45
Ile Ile Tyr Asp Val Asp His Arg Pro Ser Gly Ile Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Arg Ser Gly
                85                  90                  95
Ser Thr Tyr Val Phe Gly Thr Gly Thr Lys Val Thr Val Leu
            100                 105                 110

<210> 85
<211> 741
<212> DNA
<213> Artificial Sequence

<220>
<223> A2 scFv

<400> 85
gaagttcaat tgttagagtc tggtggcggt cttgttcagc ctggtggttc tttacgtctt 60
tcttgcgctg cttccggatt cactttctct ttttaccaga tgcattgggt tcgccaagct 120
cctggtaaag gtttggagtg ggtttctggt atctatcctt ctggtggcta tactaagtat 180
gctgactccg ttaaaggtcg cttcactatc tctagagaca actctaagaa tactctctac 240
ttgcagatga acagcttaag ggctgaggac actgcagtct actattgtgc gagagatcta 300
gggtacggca gtagctggta ctactttgac tactggggcc agggaaccct ggtcaccgtc 360
tcaagcggtg gcggcggttc ggggggtggc ggaagtggag gtggagggag tcagagcgct 420
ttgactcagc ctgcctccgt gtctgggtct cctggacagt cgatcaccat ctcctgcagt 480
ggaaccgaca gtgacgttgg tggttataac catgtctcct ggtaccaaca acacccaggc 540
aaggccccca aactcataat ttatgatgtc gatcatcggc cctcagggat ctctaaccgt 600
ttctctggct ccaagtctgg caacacggcc tccctgacca tctctgggct ccaggctgag 660
gacgaggctg attattactg cagctcatat agaagcggta gcacttatgt cttcggaact 720
gggaccaagg tcaccgtcct a                                           741

<210> 86
<211> 247
<212> PRT
<213> Artificial Sequence

<220>
<223> A2 scFv

<400> 86
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Phe Tyr
            20                  25                  30
Gln Met His Trp Val Arg Gln Ala Pro Gly Lys Gly Leu Glu Trp Val
        35                  40                  45
Ser Gly Ile Tyr Pro Ser Gly Gly Tyr Thr Lys Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Leu Gly Tyr Gly Ser Ser Trp Tyr Tyr Phe Asp Tyr Trp
            100                 105                 110
Gly Gln Gly Thr Leu Val Thr Val Ser Ser Gly Gly Gly Gly Ser Gly
        115                 120                 125
Gly Gly Gly Ser Gly Gly Gly Gly Ser Gln Ser Ala Leu Thr Gln Pro
    130                 135                 140
Ala Ser Val Ser Gly Ser Pro Gly Gln Ser Ile Thr Ile Ser Cys Ser

145                 150                 155                 160
Gly Thr Asp Ser Asp Val Gly Gly Tyr Asn His Val Ser Trp Tyr Gln
                165                 170                 175
Gln His Pro Gly Lys Ala Pro Lys Leu Ile Ile Tyr Asp Val Asp His
            180                 185                 190
Arg Pro Ser Gly Ile Ser Asn Arg Phe Ser Gly Ser Lys Ser Gly Asn
        195                 200                 205
Thr Ala Ser Leu Thr Ile Ser Gly Leu Gln Ala Glu Asp Glu Ala Asp
    210                 215                 220
Tyr Tyr Cys Ser Ser Tyr Arg Ser Gly Ser Thr Tyr Val Phe Gly Thr
225                 230                 235                 240
Gly Thr Lys Val Thr Val Leu
                245

<210> 87
<211> 603
<212> DNA
<213> Artificial Sequence

<220>
<223> PLD ECD

<400> 87
ttattcacag tgacagtccc taaggaactg tacataatag agcatggcag caatgtgacc 60
ctggaatgca actttgacac tggaagtcat gtgaaccttg gagcaataac agccagtttg 120
caaaaggtgg aaaatgatac atccccacac cgtgaaagag ccactttgct ggaggagcag 180
ctgcccttag ggaaggcctc gttccacata cctcaagtcc aagtgaggga cgaaggacag 240
taccaatgca taatcatcta tggggtcgcc tgggactaca agtacctgac tctgaaagtc 300
aaagcttcct acaggaaaat aaacactcac atcctaaagg ttccagaaac agatgaggta 360
gagctcacct gccaggctac aggttatcct ctggcagaag tatcctggcc aaacgtcagc 420
gttcctgcca acaccagcca ctccaggacc cctgaaggcc tctaccaggt caccagtgtt 480
ctgcgcctaa agccaccccc tggcagaaac ttcagctgtg tgttctggaa tactcacgtg 540
agggaactta ctttggccag cattgacctt caaagtcaga tggaacccag gacccatcca 600
acc                                                               603

<210> 88
<211> 201
<212> PRT
<213> Artificial Sequence

<220>
<223> PLD ECD

<400> 88
Leu Phe Thr Val Thr Val Pro Lys Glu Leu Tyr Ile Ile Glu His Gly
1               5                   10                  15
Ser Asn Val Thr Leu Glu Cys Asn Phe Asp Thr Gly Ser His Val Asn
            20                  25                  30
Leu Gly Ala Ile Thr Ala Ser Leu Gln Lys Val Glu Asn Asp Thr Ser
        35                  40                  45
Pro His Arg Glu Arg Ala Thr Leu Leu Glu Glu Gln Leu Pro Leu Gly
    50                  55                  60
Lys Ala Ser Phe His Ile Pro Gln Val Gln Val Arg Asp Glu Gly Gln
65                  70                  75                  80
Tyr Gln Cys Ile Ile Ile Tyr Gly Val Ala Trp Asp Tyr Lys Tyr Leu
                85                  90                  95
Thr Leu Lys Val Lys Ala Ser Tyr Arg Lys Ile Asn Thr His Ile Leu
            100                 105                 110
Lys Val Pro Glu Thr Asp Glu Val Glu Leu Thr Cys Gln Ala Thr Gly
        115                 120                 125
Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160
Leu Arg Leu Lys Pro Pro Pro Gly Arg Asn Phe Ser Cys Val Phe Trp
                165                 170                 175
Asn Thr His Val Arg Glu Leu Thr Leu Ala Ser Ile Asp Leu Gln Ser
            180                 185                 190
Gln Met Glu Pro Arg Thr His Pro Thr
        195                 200

<210> 89
<211> 498
<212> DNA
<213> Artificial Sequence

<220>
<223> monoIL10

<400> 89
agcccaggcc agggcaccca gtctgagaac agctgcaccc acttcccagg caacctgcct 60
aacatgcttc gagatctccg agatgccttc agcagagtga agactttctt tcaaatgaag 120
gatcagctgg acaacttgtt gttaaaggag tccttgctgg aggactttaa gggttacctg 180
ggttgccaag ccttgtctga gatgatccag ttttacctgg aggaggtgat gccccaagct 240
gagaaccaag acccagacat caaggcgcat gtgaactccc tgggggagaa cctgaagacc 300
ctcaggctga ggctacggcg ctgtcatcga tttcttccct gtgaaaacgg tggtggatcc 360
ggcggtaaga gcaaggccgt ggagcaggtg aagaatgcct ttaataagct ccaagagaaa 420
ggcatctaca aagccatgag tgagtttgac atcttcatca actacataga agcctacatt 480
acaatgaaga tacgaaac                                                498

<210> 90
<211> 166
<212> PRT
<213> Artificial Sequence

<220>
<223> monoIL10

<400> 90
Ser Pro Gly Gln Gly Thr Gln Ser Glu Asn Ser Cys Thr His Phe Pro
1               5                   10                  15
Gly Asn Leu Pro Asn Met Leu Arg Asp Leu Arg Asp Ala Phe Ser Arg
            20                  25                  30
Val Lys Thr Phe Phe Gln Met Lys Asp Gln Leu Asp Asn Leu Leu Leu
        35                  40                  45
Lys Glu Ser Leu Leu Glu Asp Phe Lys Gly Tyr Leu Gly Cys Gln Ala
    50                  55                  60
Leu Ser Glu Met Ile Gln Phe Tyr Leu Glu Glu Val Met Pro Gln Ala
65                  70                  75                  80
Glu Asn Gln Asp Pro Asp Ile Lys Ala His Val Asn Ser Leu Gly Glu
                85                  90                  95
Asn Leu Lys Thr Leu Arg Leu Arg Leu Arg Arg Cys His Arg Phe Leu
            100                 105                 110
Pro Cys Glu Asn Gly Gly Gly Ser Gly Gly Lys Ser Lys Ala Val Glu
        115                 120                 125
Gln Val Lys Asn Ala Phe Asn Lys Leu Gln Glu Lys Gly Ile Tyr Lys
    130                 135                 140
Ala Met Ser Glu Phe Asp Ile Phe Ile Asn Tyr Ile Glu Ala Tyr Ile
145                 150                 155                 160
Thr Met Lys Ile Arg Asn
                165

<210> 91

<211> 348
<212> DNA
<213> Artificial Sequence

<220>
<223> VH of 3D1

<400> 91
caggtccagc tgcagcagtc tgggcctgag ctggtgaggc ctggggaatc agtgaagatt 60
tcctgcaagg gttccggcta cacattcact gattatgcta tacagtgggt gaagcagagt 120
catgcaaaga gtctagagtg gattggagtt attaatattt actatgataa tacaaactac 180
aaccagaagt ttaagggcaa ggccacaatg actgtagaca aatcctccag cacagcctat 240
atggaacttg ccagattgac atctgaggat tctgccatct attactgtgc aagagcggcc 300
tggtatatgg actactgggg tcaaggaacc tcagtcaccg tctcctca 348

<210> 92
<211> 116
<212> PRT
<213> Artificial Sequence

<220>
<223> VH of 3D1

<400> 92
Gln Val Gln Leu Gln Gln Ser Gly Pro Glu Leu Val Arg Pro Gly Glu
1               5                   10                  15
Ser Val Lys Ile Ser Cys Lys Gly Ser Gly Tyr Thr Phe Thr Asp Tyr
            20                  25                  30
Ala Ile Gln Trp Val Lys Gln Ser His Ala Lys Ser Leu Glu Trp Ile
        35                  40                  45
Gly Val Ile Asn Ile Tyr Tyr Asp Asn Thr Asn Tyr Asn Gln Lys Phe
    50                  55                  60
Lys Gly Lys Ala Thr Met Thr Val Asp Lys Ser Ser Ser Thr Ala Tyr
65                  70                  75                  80
Met Glu Leu Ala Arg Leu Thr Ser Glu Asp Ser Ala Ile Tyr Tyr Cys
                85                  90                  95
Ala Arg Ala Ala Trp Tyr Met Asp Tyr Trp Gly Gln Gly Thr Ser Val
            100                 105                 110
Thr Val Ser Ser
        115

<210> 93
<400> 93
000

<210> 94
<400> 94
000

<210> 95
<211> 336
<212> DNA
<213> Artificial Sequence

<220>
<223> VL of 3D1

<400> 95
gacattgtgc tgtcacagtc tccatcctcc ctggctgtgt cagcaggaga gaaggtcact 60
atgagctgca aatccagtca gagtctgctc aacagtagaa cccgagagaa ctacttggct 120
tggtaccagc agaaaccagg gcagtctcct aaactgctga tctactgggc atccactagg 180
gaatctgggg tccctgatcg cttcacaggc agtggatctg ggacagattt cactctcacc 240

atcagcagtg tgcaggctga agacctggca gtttattact gcacgcaatc ttataatctt 300
tacacgttcg gaggggggac caagctggaa ataaaa 336

<210> 96
<211> 112
<212> PRT
<213> Artificial Sequence

<220>
<223> VL of 3D1

<400> 96
Asp Ile Val Leu Ser Gln Ser Pro Ser Ser Leu Ala Val Ser Ala Gly
1               5                   10                  15
Glu Lys Val Thr Met Ser Cys Lys Ser Ser Gln Ser Leu Leu Asn Ser
            20                  25                  30
Arg Thr Arg Glu Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Thr Gln
                85                  90                  95
Ser Tyr Asn Leu Tyr Thr Phe Gly Gly Gly Thr Lys Leu Glu Ile Lys
            100                 105                 110

<210> 97
<211> 744
<212> DNA
<213> Artificial Sequence

<220>
<223> 3D1 scFv

<400> 97
caggtccagc tgcagcagtc tgggcctgag ctggtgaggc ctggggaatc agtgaagatt 60
tcctgcaagg gttccggcta cacattcact gattatgcta tacagtgggt gaagcagagt 120
catgcaaaga gtctagagtg gattggagtt attaatattt actatgataa tacaaactac 180
aaccagaagt ttaagggcaa ggccacaatg actgtagaca aatcctccag cacagcctat 240
atggaacttg ccagattgac atctgaggat tctgccatct attactgtgc aagagcggcc 300
tggtatatgg actactgggg tcaaggaacc tcagtcaccg tctcctcagg gggtggaggc 360
tctggtggcg gtggctctgg cggaggtgga tccggtggcg gcggatctga cattgtgctg 420
tcacagtctc catcctccct ggctgtgtca gcaggagaga aggtcactat gagctgcaaa 480
tccagtcaga gtctgctcaa cagtagaacc cgagagaact acttggcttg gtaccagcag 540
aaaccagggc agtctcctaa actgctgatc tactgggcat ccactaggga atctggggtc 600
cctgatcgct tcacaggcag tggatctggg acagatttca ctctcaccat cagcagtgtg 660
caggctgaag acctggcagt ttattactgc acgcaatctt ataatcttta cacgttcgga 720
ggggggacca agctggaaat aaaa 744

<210> 98
<211> 248
<212> PRT
<213> Artificial Sequence

<220>
<223> 3D1 scFv

<400> 98
Gln Val Gln Leu Gln Gln Ser Gly Pro Glu Leu Val Arg Pro Gly Glu
1               5                   10                  15
Ser Val Lys Ile Ser Cys Lys Gly Ser Gly Tyr Thr Phe Thr Asp Tyr

                20                  25                  30
Ala Ile Gln Trp Val Lys Gln Ser His Ala Lys Ser Leu Glu Trp Ile
            35                  40                  45
Gly Val Ile Asn Ile Tyr Tyr Asp Asn Thr Asn Tyr Asn Gln Lys Phe
    50                  55                  60
Lys Gly Lys Ala Thr Met Thr Val Asp Lys Ser Ser Ser Thr Ala Tyr
65                  70                  75                  80
Met Glu Leu Ala Arg Leu Thr Ser Glu Asp Ser Ala Ile Tyr Tyr Cys
                85                  90                  95
Ala Arg Ala Ala Trp Tyr Met Asp Tyr Trp Gly Gln Gly Thr Ser Val
            100                 105                 110
Thr Val Ser Ser Gly Gly Gly Gly Ser Gly Gly Gly Gly Ser Gly Gly
        115                 120                 125
Gly Gly Ser Gly Gly Gly Gly Ser Asp Ile Val Leu Ser Gln Ser Pro
    130                 135                 140
Ser Ser Leu Ala Val Ser Ala Gly Glu Lys Val Thr Met Ser Cys Lys
145                 150                 155                 160
Ser Ser Gln Ser Leu Leu Asn Ser Arg Thr Arg Glu Asn Tyr Leu Ala
                165                 170                 175
Trp Tyr Gln Gln Lys Pro Gly Gln Ser Pro Lys Leu Leu Ile Tyr Trp
            180                 185                 190
Ala Ser Thr Arg Glu Ser Gly Val Pro Asp Arg Phe Thr Gly Ser Gly
        195                 200                 205
Ser Gly Thr Asp Phe Thr Leu Thr Ile Ser Ser Val Gln Ala Glu Asp
    210                 215                 220
Leu Ala Val Tyr Tyr Cys Thr Gln Ser Tyr Asn Leu Tyr Thr Phe Gly
225                 230                 235                 240
Gly Gly Thr Lys Leu Glu Ile Lys
                245

<210> 99
<211> 247
<212> PRT
<213> Artificial Sequence

<220>
<223> MET021 scFv

<400> 99
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Arg Asn Tyr
            20                  25                  30
Leu Asn Trp Tyr Gln Gln Glu Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Ile Leu Thr Ile Ser Ser Leu Gln Pro
65                  70                  75                  80
Glu Asp Phe Ala Thr Tyr Tyr Cys Gln Gln Ser Tyr Val Thr Pro Leu
                85                  90                  95
Thr Phe Gly Gly Gly Thr Lys Val Glu Ile Lys Gly Gly Gly Gly Ser
            100                 105                 110
Gly Gly Gly Gly Ser Gly Gly Gly Gly Ser Gly Gly Gly Gly Ser Glu
        115                 120                 125
Val Gln Leu Leu Glu Ser Gly Gly Gly Leu Val Gln Pro Gly Gly Ser
    130                 135                 140
Leu Arg Leu Ser Cys Ala Ala Ser Gly Phe Thr Phe Ser Lys Tyr Asp
145                 150                 155                 160
Met Leu Trp Val Arg Gln Ala Pro Gly Lys Gly Leu Glu Trp Val Ser
                165                 170                 175
Tyr Ile Tyr Pro Ser Gly Gly Leu Thr Glu Tyr Ala Asp Ser Val Lys

                180                     185                     190
Gly Arg Phe Thr Ile Ser Arg Asp Asn Ser Lys Asn Thr Leu Tyr Leu
        195                     200                     205
Gln Met Asn Ser Leu Arg Ala Glu Asp Thr Ala Val Tyr Tyr Cys Ala
    210                     215                     220
Arg Arg Ala Pro Arg Ser Leu Ser Phe Asp Ile Trp Gly Gln Gly Thr
225                     230                     235                     240
Met Val Thr Val Ser Ser Ser
                    245


<210> 100
<211> 120
<212> PRT
<213> Artificial Sequence

<220>
<223> VH of MET021 scFv

<400> 100
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Lys Tyr
            20                  25                  30
Asp Met Leu Trp Val Arg Gln Ala Pro Gly Lys Gly Leu Glu Trp Val
        35                  40                  45
Ser Tyr Ile Tyr Pro Ser Gly Gly Leu Thr Glu Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Arg Ala Pro Arg Ser Leu Ser Phe Asp Ile Trp Gly Gln Gly
            100                 105                 110
Thr Met Val Thr Val Ser Ser Ser
        115                 120


<210> 101
<211> 107
<212> PRT
<213> Artificial Sequence

<220>
<223> VL of MET021 scFv

<400> 101
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Arg Asn Tyr
            20                  25                  30
Leu Asn Trp Tyr Gln Gln Glu Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Ile Leu Thr Ile Ser Ser Leu Gln Pro
65                  70                  75                  80
Glu Asp Phe Ala Thr Tyr Tyr Cys Gln Gln Ser Tyr Val Thr Pro Leu
                85                  90                  95
Thr Phe Gly Gly Gly Thr Lys Val Glu Ile Lys
            100                 105

<210> 102
<211> 249
<212> PRT
<213> Artificial Sequence

<220>
<223> G19-4 scFv

<400> 102
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser
                245


<210> 103
<211> 122
<212> PRT
<213> Artificial Sequence

<220>
<223> VH of G19-4

<400> 103
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys

85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser
        115                 120

<210> 104
<211> 112
<212> PRT
<213> Artificial Sequence

<220>
<223> VL of G19-4

<400> 104
Ala Gln Asp Ile Gln Met Thr Gln Thr Thr Ser Ser Leu Ser Ala Ser
1               5                   10                  15
Leu Gly Asp Arg Val Thr Ile Ser Cys Arg Ala Ser Gln Asp Ile Arg
            20                  25                  30
Asn Tyr Leu Asn Trp Tyr Gln Gln Lys Pro Asp Gly Thr Val Lys Leu
        35                  40                  45
Leu Ile Tyr Tyr Thr Ser Arg Leu His Ser Gly Val Pro Ser Arg Phe
    50                  55                  60
Ser Gly Ser Gly Ser Gly Thr Asp Tyr Ser Leu Thr Ile Ala Asn Leu
65                  70                  75                  80
Gln Pro Glu Asp Ile Ala Thr Tyr Phe Cys Gln Gln Gly Asn Thr Leu
                85                  90                  95
Pro Trp Thr Phe Gly Gly Gly Thr Lys Leu Val Thr Lys Arg Ser Ser
            100                 105                 110

<210> 105
<211> 252
<212> PRT
<213> Artificial Sequence

<220>
<223> HD37 scFv

<400> 105
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175

Gln Ile Trp Pro Gly Asp Gly Asp Thr Asn Tyr Asn Gly Lys Phe Lys
                180                 185                 190
Gly Lys Ala Thr Leu Thr Ala Asp Glu Ser Ser Ser Thr Ala Tyr Met
            195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser
                245                 250

<210> 106
<211> 121
<212> PRT
<213> Artificial Sequence

<220>
<223> VH of 2E12 scFv

<400> 106
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala
                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser
        115                 120

<210> 107
<211> 111
<212> PRT
<213> Artificial Sequence

<220>
<223> VL of 2E12 scFv

<400> 107
Asp Ile Val Leu Thr Gln Ser Pro Ala Ser Leu Ala Val Ser Leu Gly
1               5                   10                  15
Gln Arg Ala Thr Ile Ser Cys Arg Ala Ser Glu Ser Val Glu Tyr Tyr
            20                  25                  30
Val Thr Ser Leu Met Gln Trp Tyr Gln Gln Lys Pro Gly Gln Pro Pro
        35                  40                  45
Lys Leu Leu Ile Ser Ala Ala Ser Asn Val Glu Ser Gly Val Pro Ala
    50                  55                  60
Arg Phe Ser Gly Ser Gly Ser Gly Thr Asp Phe Ser Leu Asn Ile His
65                  70                  75                  80
Pro Val Glu Glu Asp Asp Ile Ala Met Tyr Phe Cys Gln Gln Ser Arg
                85                  90                  95
Lys Val Pro Trp Thr Phe Gly Gly Gly Thr Lys Leu Glu Ile Lys
            100                 105                 110

<210> 108
<211> 20
<212> PRT
<213> Artificial Sequence

<220>
<223> Linker of 2E12 scFv

<400> 108
Gly Gly Gly Gly Ser Gly Gly Gly Gly Ser Gly Gly Gly Gly Ser Gly
1               5                   10                  15
Gly Gly Gly Ser
            20


<210> 109
<211> 252
<212> PRT
<213> Artificial Sequence

<220>
<223> full length 2E12 scFv

<400> 109
Gln Val Gln Leu Lys Glu Ser Gly Pro Gly Ser Val Ala Pro Ser Gln
1               5                   10                  15
Ser Leu Ser Ile Thr Cys Thr Val Ser Gly Phe Ser Leu Thr Gly Tyr
            20                  25                  30
Gly Val Asn Trp Val Arg Gln Pro Pro Gly Lys Gly Leu Glu Trp Leu
        35                  40                  45
Gly Met Ile Trp Gly Asp Gly Ser Thr Asp Tyr Asn Ser Ala Leu Lys
    50                  55                  60
Ser Arg Leu Ser Ile Thr Lys Asp Asn Ser Lys Ser Gln Val Phe Leu
65                  70                  75                  80
Lys Met Asn Ser Leu Gln Thr Asp Asp Thr Ala Arg Tyr Tyr Cys Ala
                85                  90                  95
Arg Asp Gly Tyr Ser Asn Phe His Tyr Tyr Val Met Asp Tyr Trp Gly
            100                 105                 110
Gln Gly Thr Ser Val Thr Val Ser Ser Gly Gly Gly Gly Ser Gly Gly
        115                 120                 125
Gly Gly Ser Gly Gly Gly Gly Ser Gly Gly Gly Gly Ser Asp Ile Val
    130                 135                 140
Leu Thr Gln Ser Pro Ala Ser Leu Ala Val Ser Leu Gly Gln Arg Ala
145                 150                 155                 160
Thr Ile Ser Cys Arg Ala Ser Glu Ser Val Glu Tyr Tyr Val Thr Ser
                165                 170                 175
Leu Met Gln Trp Tyr Gln Gln Lys Pro Gly Gln Pro Pro Lys Leu Leu
            180                 185                 190
Ile Ser Ala Ala Ser Asn Val Glu Ser Gly Val Pro Ala Arg Phe Ser
        195                 200                 205
Gly Ser Gly Ser Gly Thr Asp Phe Ser Leu Asn Ile His Pro Val Glu
    210                 215                 220
Glu Asp Asp Ile Ala Met Tyr Phe Cys Gln Gln Ser Arg Lys Val Pro
225                 230                 235                 240
Trp Thr Phe Gly Gly Gly Thr Lys Leu Glu Ile Lys
                245                 250


<210> 110
<211> 22
<212> PRT
<213> Artificial Sequence

<220>
<223> Signal peptide of 2E12 scFv

<400> 110
Met Asp Phe Gln Val Gln Ile Phe Ser Phe Leu Leu Ile Ser Ala Ser
1               5                   10                  15
Val Ile Met Ser Arg Gly
            20

<210> 111
<211> 20
<212> PRT
<213> Artificial Sequence

<220>
<223> Signal peptide in various signle chain fusion
      proteins (X01xx)

<400> 111
Met Glu Ala Pro Ala Gln Leu Leu Phe Leu Leu Leu Leu Trp Leu Pro
1               5                   10                  15
Asp Thr Thr Gly
            20

<210> 112
<211> 107
<212> PRT
<213> Homo sapiens

<400> 112

<210> 113
<211> 106
<212> PRT
<213> Homo sapiens

<400> 113
Gly Gln Pro Lys Ala Asn Pro Thr Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Gly Ser Pro
        35                  40                  45
Val Lys Ala Gly Val Glu Thr Thr Lys Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 114
<211> 98
<212> PRT
<213> Homo sapiens

<400> 114
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val

<210> 115
<211> 110
<212> PRT
<213> Homo sapiens

<400> 115
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Ala Lys
            100                 105                 110

<210> 116
<211> 107
<212> PRT
<213> Homo sapiens

<400> 116
Gly Gln Pro Arg Glu Pro Gln Val Tyr Thr Leu Pro Pro Ser Arg Asp
1               5                   10                  15
Glu Leu Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu

35                  40                  45
Asn Asn Tyr Lys Thr Thr Pro Pro Val Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Pro Gly Lys
            100                 105

<210> 117
<211> 125
<212> PRT
<213> Artificial Sequence

<220>
<223> VH of HD37

<400> 117
Gln Val Gln Leu Gln Gln Ser Gly Ala Glu Leu Val Arg Pro Gly Ser
1               5                   10                  15
Ser Val Lys Ile Ser Cys Lys Ala Ser Gly Tyr Ala Phe Ser Ser Tyr
            20                  25                  30
Trp Met Asn Trp Val Lys Gln Arg Pro Gly Gln Gly Leu Glu Trp Ile
        35                  40                  45
Gly Gln Ile Trp Pro Gly Asp Gly Asp Thr Asn Tyr Asn Gly Lys Phe
    50                  55                  60
Lys Gly Lys Ala Thr Leu Thr Ala Asp Glu Ser Ser Ser Thr Ala Tyr
65                  70                  75                  80
Met Gln Leu Ser Ser Leu Ala Ser Glu Asp Ser Ala Val Tyr Phe Cys
                85                  90                  95
Ala Arg Arg Glu Thr Thr Thr Val Gly Arg Tyr Tyr Tyr Ala Met Asp
            100                 105                 110
Tyr Trp Gly Gln Gly Thr Ser Val Thr Val Ser Ser Ser
        115                 120                 125

<210> 118
<400> 118
000

<210> 119
<211> 111
<212> PRT
<213> Artificial Sequence

<220>
<223> VL of HD37

<400> 119
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr

85                  90                  95
Glu Asp Pro Trp Thr Phe Gly Gly Gly Thr Lys Leu Glu Ile Lys
            100                 105                 110

<210> 120
<211> 249
<212> PRT
<213> Artificial Sequence

<220>
<223> M0042 scFv (VL-VH)

<400> 120
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Val
        35                  40                  45
Tyr Ser Val Ser Ser Leu Gln Ser Gly Val Pro Ser Arg Phe Ser Gly
    50                  55                  60
Ser Gly Ser Gly Thr Asp Phe Thr Leu Thr Ile Asn Ser Leu Gln Pro
65                  70                  75                  80
Glu Asp Phe Ala Thr Tyr Tyr Cys Gln Gln Ser Tyr Ser Thr Pro Phe
                85                  90                  95
Thr Phe Gly Pro Gly Thr Lys Val Asp Ile Lys Gly Gly Gly Gly Ser
            100                 105                 110
Gly Gly Gly Gly Ser Gly Gly Gly Gly Ser Gly Gly Gly Gly Ser Glu
        115                 120                 125
Val Gln Leu Leu Glu Ser Gly Gly Gly Leu Val Gln Pro Gly Gly Ser
    130                 135                 140
Leu Arg Leu Ser Cys Ala Ala Ser Gly Phe Thr Phe Ser His Tyr Tyr
145                 150                 155                 160
Met Ser Trp Val Arg Gln Ala Pro Gly Lys Gly Leu Glu Trp Val Ser
                165                 170                 175
Tyr Ile Gly Pro Ser Gly Gly Phe Thr Ala Tyr Ala Asp Ser Val Lys
            180                 185                 190
Gly Arg Phe Thr Ile Ser Arg Asp Asn Ser Lys Asn Thr Leu Tyr Leu
        195                 200                 205
Gln Met Asn Ser Leu Arg Ala Glu Asp Thr Ala Val Tyr Tyr Cys Ala
    210                 215                 220
Arg Asp Gly Trp Val Gly Ser Glu Asn Tyr Ser Gly Met Asp Val Trp
225                 230                 235                 240
Gly Gln Gly Thr Thr Val Ser Ser Ser
                245

<210> 121
<211> 122
<212> PRT
<213> Artificial Sequence

<220>
<223> VH of M0042

<400> 121
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser His Tyr
            20                  25                  30
Tyr Met Ser Trp Val Arg Gln Ala Pro Gly Lys Gly Leu Glu Trp Val
        35                  40                  45

Ser Tyr Ile Gly Pro Ser Gly Gly Phe Thr Ala Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Trp Val Gly Ser Glu Asn Tyr Ser Gly Met Asp Val
            100                 105                 110
Trp Gly Gln Gly Thr Thr Val Ser Ser Ser
        115                 120

<210> 122
<211> 107
<212> PRT
<213> Artificial Sequence

<220>
<223> VL of M0042

<400> 122
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Val
        35                  40                  45
Tyr Ser Val Ser Ser Leu Gln Ser Gly Val Pro Ser Arg Phe Ser Gly
    50                  55                  60
Ser Gly Ser Gly Thr Asp Phe Thr Leu Thr Ile Asn Ser Leu Gln Pro
65                  70                  75                  80
Glu Asp Phe Ala Thr Tyr Tyr Cys Gln Gln Ser Tyr Ser Thr Pro Phe
                85                  90                  95
Thr Phe Gly Pro Gly Thr Lys Val Asp Ile Lys
            100                 105

<210> 123
<400> 123
000

<210> 124
<400> 124
000

<210> 125
<211> 20
<212> PRT
<213> Artificial Sequence

<220>
<223> NKG2D Linker

<400> 125
Asn Ser Leu Phe Asn Gln Glu Val Gln Ile Pro Leu Thr Glu Ser Tyr
1               5                   10                  15
Ser Pro Asn Ser
            20

<210> 126
<400> 126
000

<210> 127

<400> 127
 000

<210> 128
<400> 128
 000

<210> 129
<400> 129
 000

<210> 130
<400> 130
 000

<210> 131
<400> 131
 000

<210> 132
<400> 132
 000

<210> 133
<400> 133
 000

<210> 134
<400> 134
 000

<210> 135
<400> 135
 000

<210> 136
<400> 136
 000

<210> 137
<400> 137
 000

<210> 138
<400> 138
 000

<210> 139
<400> 139
 000

<210> 140
<211> 105
<212> PRT
<213> Artificial Sequence

<220>
<223> Altered human Cl sequence used in constructing
      Interceptors

<400> 140
Gln Pro Lys Ala Asn Pro Thr Val Thr Leu Phe Pro Pro Ser Ser Glu

1                   5                   10                  15
Glu Leu Gln Ala Asn Lys Ala Thr Leu Val Cys Leu Ile Ser Asp Phe
                20                  25                  30
Tyr Pro Gly Ala Val Thr Val Ala Trp Lys Ala Asp Gly Ser Pro Val
            35                  40                  45
Lys Ala Gly Val Glu Thr Thr Lys Pro Ser Lys Gln Ser Asn Asn Lys
    50                  55                  60
Tyr Ala Ala Ser Ser Tyr Leu Ser Leu Thr Pro Glu Gln Trp Lys Ser
65                  70                  75                  80
His Arg Ser Tyr Ser Cys Gln Val Thr His Glu Gly Ser Thr Val Glu
                85                  90                  95
Lys Thr Val Ala Pro Thr Glu Ser Ser
                100                 105

<210> 141
<211> 105
<212> PRT
<213> Artificial Sequence

<220>
<223> Altered human Ck sequence used in constructing
      Interceptors

<400> 141
Thr Val Ala Ala Pro Ser Val Phe Ile Phe Pro Pro Ser Asp Glu Gln
1                   5                   10                  15
Leu Lys Ser Gly Thr Ala Ser Val Val Cys Leu Leu Asn Asn Phe Tyr
                20                  25                  30
Pro Arg Glu Ala Lys Val Gln Trp Lys Val Asp Asn Ala Leu Gln Ser
            35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
                100                 105

<210> 142
<211> 105
<212> PRT
<213> Artificial Sequence

<220>
<223> Altered human Ck with N29A substitution

<400> 142
Thr Val Ala Ala Pro Ser Val Phe Ile Phe Pro Pro Ser Asp Glu Gln
1                   5                   10                  15
Leu Lys Ser Gly Thr Ala Ser Val Val Cys Leu Leu Ala Asn Phe Tyr
                20                  25                  30
Pro Arg Glu Ala Lys Val Gln Trp Lys Val Asp Asn Ala Leu Gln Ser
            35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu

            100                 105

<210> 143
<211> 105
<212> PRT
<213> Artificial Sequence

<220>
<223> Altered human Ck with N30A substitution

<400> 143
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 144
<211> 105
<212> PRT
<213> Artificial Sequence

<220>
<223> Altered human Ck with Q52A substitution

<400> 144
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Ala Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 145
<211> 105
<212> PRT
<213> Artificial Sequence

<220>
<223> Altered human Ck with V55A substitution

<400> 145
Thr Val Ala Ala Pro Ser Val Phe Ile Phe Pro Pro Ser Asp Glu Gln

1                   5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 146
<211> 105
<212> PRT
<213> Artificial Sequence

<220>
<223> Altered human Ck with T56A substitution

<400> 146
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Ala Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 147
<211> 105
<212> PRT
<213> Artificial Sequence

<220>
<223> Altered human Ck with S68A substitution

<400> 147
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ala Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 148
<211> 105
<212> PRT
<213> Artificial Sequence

<220>
<223> Altered human Ck with T70A substitution

<400> 148
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 149
<211> 105
<212> PRT
<213> Artificial Sequence

<220>
<223> Altered human Ck with V55A N29A substitution

<400> 149
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 150
<211> 105
<212> PRT
<213> Artificial Sequence

<220>
<223> Altered human Ck with V55A N30A substitution

<400> 150
Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Ala Phe Tyr
                20                  25                  30
Pro Arg Glu Ala Lys Val Gln Trp Lys Val Asp Asn Ala Leu Gln Ser
            35                  40                  45
Gly Asn Ser Gln Glu Ser Ala Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
                100                 105

<210> 151
<211> 105
<212> PRT
<213> Artificial Sequence

<220>
<223> Altered human Ck with V55A Q52A substitution

<400> 151
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Asn Phe Tyr
                20                  25                  30
Pro Arg Glu Ala Lys Val Gln Trp Lys Val Asp Asn Ala Leu Gln Ser
            35                  40                  45
Gly Asn Ser Ala Glu Ser Ala Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
                100                 105

<210> 152
<211> 105
<212> PRT
<213> Artificial Sequence

<220>
<223> Altered human Ck with V55A S68A substitution

<400> 152
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Asn Phe Tyr
                20                  25                  30
Pro Arg Glu Ala Lys Val Gln Trp Lys Val Asp Asn Ala Leu Gln Ser
            35                  40                  45
Gly Asn Ser Gln Glu Ser Ala Thr Glu Gln Asp Ser Lys Asp Ser Thr
    50                  55                  60
Tyr Ser Leu Ala Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
                100                 105

<210> 153
<211> 105
<212> PRT
<213> Artificial Sequence

<220>
<223> Altered human Ck with V55A T70A substitution

<400> 153
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 154
<211> 105
<212> PRT
<213> Artificial Sequence

<220>
<223> Altered human Ck with V55A N29A N30A substitution

<400> 154
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 155
<211> 105
<212> PRT
<213> Artificial Sequence

<220>
<223> Altered human Ck with V55A N29A S68A substitution

<400> 155
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Asn Phe Tyr

                20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ala Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 156
<211> 105
<212> PRT
<213> Artificial Sequence

<220>
<223> Altered human Ck with V55A S68A T70A substitution

<400> 156
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ala Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 157
<211> 105
<212> PRT
<213> Artificial Sequence

<220>
<223> Altered human Ck with V55A Q52A S68A substitution

<400> 157
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Ala Glu Ser Ala Thr Glu Gln Asp Ser Lys Asp Ser Thr
    50                  55                  60
Tyr Ser Leu Ala Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 158
<211> 105
<212> PRT
<213> Artificial Sequence

<220>
<223> Altered human Ck with N29R V55A T70A substitution

<400> 158
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Arg Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 159
<211> 105
<212> PRT
<213> Artificial Sequence

<220>
<223> Altered human Ck with N29AK V55A T70A substitution

<400> 159
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Lys Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 160
<211> 105
<212> PRT
<213> Artificial Sequence

<220>
<223> Altered human Ck with N29W V55A T70A substitution

<400> 160
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Trp Asn Phe Tyr
            20                  25                  30

Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 161
<211> 105
<212> PRT
<213> Artificial Sequence

<220>
<223> Altered human Ck with N29Y V55A T70A substitution

<400> 161
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Tyr Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 162
<211> 105
<212> PRT
<213> Artificial Sequence

<220>
<223> Altered human Ck with S68K V55A substitution

<400> 162
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Lys Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 163

<211> 105
<212> PRT
<213> Artificial Sequence

<220>
<223> Altered human Ck with S68E V55A substitution

<400> 163
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Glu Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 164
<211> 105
<212> PRT
<213> Artificial Sequence

<220>
<223> Altered human Ck with S68Q V55A substitution

<400> 164
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Gln Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 165
<211> 105
<212> PRT
<213> Artificial Sequence

<220>
<223> Altered human Ck with T70E V55A substitution

<400> 165
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser

35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 166
<211> 105
<212> PRT
<213> Artificial Sequence

<220>
<223> Altered human Ck with Q52L N29A N30A substitution

<400> 166
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Leu Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 167
<211> 105
<212> PRT
<213> Artificial Sequence

<220>
<223> Altered human Ck with N30R V55A T70A substitution

<400> 167
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Arg Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 168
<211> 105

<212> PRT
<213> Artificial Sequence

<220>
<223> Altered human Ck with N30K V55A T70A substitution

<400> 168
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Lys Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 169
<211> 105
<212> PRT
<213> Artificial Sequence

<220>
<223> Altered human Ck with N30W V55A T70A substitution

<400> 169
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Trp Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 170
<211> 105
<212> PRT
<213> Artificial Sequence

<220>
<223> Altered human Ck with N30E V55A T70A substitution

<400> 170
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Glu Phe Tyr
            20                  25                  30
Pro Arg Glu Ala Lys Val Gln Trp Lys Val Asp Asn Ala Leu Gln Ser
        35                  40                  45

Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 171
<211> 105
<212> PRT
<213> Artificial Sequence

<220>
<223> Altered human Ck with N30G V55A T70A substitution

<400> 171
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Gly Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Ala Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 172
<211> 105
<212> PRT
<213> Artificial Sequence

<220>
<223> Altered human Ck with V55R N29A N30A substitution

<400> 172
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Arg Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 173
<211> 105
<212> PRT

<213> Artificial Sequence

<220>
<223> Altered human Ck with V55W N29A N30A substitution

<400> 173
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Trp Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 174
<211> 105
<212> PRT
<213> Artificial Sequence

<220>
<223> Altered human Ck with V55E N29A N30A substitution

<400> 174
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Glu Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 175
<211> 105
<212> PRT
<213> Artificial Sequence

<220>
<223> Altered human Ck with N29W N30Y V55A T70E (WYAE)
      substitution

<400> 175
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Trp Tyr Phe Tyr
            20                  25                  30
Pro Arg Glu Ala Lys Val Gln Trp Lys Val Asp Asn Ala Leu Gln Ser
        35                  40                  45

Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 176
<211> 105
<212> PRT
<213> Artificial Sequence

<220>
<223> Altered human Ck with N29Y N30Y V55A T70E (YYAE)
      substitution

<400> 176
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Tyr Tyr Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 177
<211> 105
<212> PRT
<213> Artificial Sequence

<220>
<223> Altered human Ck with N30E V55A T70E (EAE)
      substitution

<400> 177
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Glu Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 178

<211> 105
<212> PRT
<213> Artificial Sequence

<220>
<223> Altered human Ck with N30Y V55A T70E (YAE)
      substitution

<400> 178
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Tyr Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105


<210> 179
<400> 179
000

<210> 180
<400> 180
000


<210> 181
<211> 4
<212> PRT
<213> Artificial Sequence

<220>
<223> Portion of human IgG1 hinge

<400> 181
Cys Pro Pro Cys
1


<210> 182
<211> 112
<212> PRT
<213> Artificial Sequence

<220>
<223> VL of P2C2 scFv

<400> 182
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser

50                  55                  60
Asp Arg Phe Ser Gly Ser Gly Ser Gly Thr Asp Phe Thr Leu Glu Ile
65                  70                  75                  80
Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110

<210> 183
<211> 15
<212> PRT
<213> Artificial Sequence

<220>
<223> Linker of P2C2 scFv

<400> 183
Gly Gly Gly Gly Ser Gly Gly Gly Gly Ser Gly Gly Gly Gly Ser
1               5                   10                  15

<210> 184
<211> 112
<212> PRT
<213> Artificial Sequence

<220>
<223> VH of P2C2 scFv

<400> 184
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Asp Phe
            20                  25                  30
Ser Met His Trp Val Arg Gln Ala Pro Gly Lys Gly Leu Lys Trp Met
        35                  40                  45
Gly Trp Ile Asn Thr Glu Thr Gly Glu Pro Thr Tyr Ala Asp Asp Phe
    50                  55                  60
Arg Gly Arg Phe Ala Phe Ser Leu Glu Thr Ser Ala Ser Thr Ala Tyr
65                  70                  75                  80
Leu Gln Ile Asn Asn Leu Lys Asn Glu Asp Thr Ala Thr Tyr Phe Cys
                85                  90                  95
Thr Trp Ser Ala Tyr Trp Gly Gln Gly Thr Leu Val Thr Val Ser Ala
            100                 105                 110

<210> 185
<211> 239
<212> PRT
<213> Artificial Sequence

<220>
<223> full length of P2C2 scFv

<400> 185
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser

50                  55                  60
Asp Arg Phe Ser Gly Ser Gly Ser Gly Thr Asp Phe Thr Leu Glu Ile
65                  70                  75                  80
Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110
Gly Gly Gly Gly Ser Gly Gly Gly Gly Ser Gly Gly Gly Gly Ser Gln
        115                 120                 125
Ile Gln Leu Val Gln Ser Gly Pro Glu Leu Lys Lys Pro Gly Glu Thr
    130                 135                 140
Val Lys Ile Ser Cys Lys Ala Ser Gly Tyr Thr Phe Thr Asp Phe Ser
145                 150                 155                 160
Met His Trp Val Arg Gln Ala Pro Gly Lys Gly Leu Lys Trp Met Gly
                165                 170                 175
Trp Ile Asn Thr Glu Thr Gly Glu Pro Thr Tyr Ala Asp Asp Phe Arg
            180                 185                 190
Gly Arg Phe Ala Phe Ser Leu Glu Thr Ser Ala Ser Thr Ala Tyr Leu
        195                 200                 205
Gln Ile Asn Asn Leu Lys Asn Glu Asp Thr Ala Thr Tyr Phe Cys Thr
    210                 215                 220
Trp Ser Ala Tyr Trp Gly Gln Gly Thr Leu Val Thr Val Ser Ala
225                 230                 235

<210> 186
<211> 102
<212> PRT
<213> Homo sapiens

<400> 186
Ala Ser Pro Thr Ser Pro Lys Val Phe Pro Leu Ser Leu Cys Ser Thr
1               5                   10                  15
Gln Pro Asp Gly Asn Val Val Ile Ala Cys Leu Val Gln Gly Phe Phe
            20                  25                  30
Pro Gln Glu Pro Leu Ser Val Thr Trp Ser Glu Ser Gly Gln Gly Val
        35                  40                  45
Thr Ala Arg Asn Phe Pro Pro Ser Gln Asp Ala Ser Gly Asp Leu Tyr
    50                  55                  60
Thr Thr Ser Ser Gln Leu Thr Leu Pro Ala Thr Gln Cys Leu Ala Gly
65                  70                  75                  80
Lys Ser Val Thr Cys His Val Lys His Tyr Thr Asn Pro Ser Gln Asp
                85                  90                  95
Val Thr Val Pro Cys Pro
            100

<210> 187
<211> 102
<212> PRT
<213> Homo sapiens

<400> 187
Ala Ser Pro Thr Ser Pro Lys Val Phe Pro Leu Ser Leu Asp Ser Thr
1               5                   10                  15
Pro Gln Asp Gly Asn Val Val Val Ala Cys Leu Val Gln Gly Phe Phe
            20                  25                  30
Pro Gln Glu Pro Leu Ser Val Thr Trp Ser Glu Ser Gly Gln Asn Val
        35                  40                  45
Thr Ala Arg Asn Phe Pro Pro Ser Gln Asp Ala Ser Gly Asp Leu Tyr
    50                  55                  60
Thr Thr Ser Ser Gln Leu Thr Leu Pro Ala Thr Gln Cys Pro Asp Gly
65                  70                  75                  80

Lys Ser Val Thr Cys His Val Lys His Tyr Thr Asn Pro Ser Gln Asp
                85                  90                  95
Val Thr Val Pro Cys Pro
            100

<210> 188
<211> 101
<212> PRT
<213> Homo sapiens

<400> 188
Ala Pro Thr Lys Ala Pro Asp Val Phe Pro Ile Ile Ser Gly Cys Arg
 1               5                  10                  15
His Pro Lys Asp Asn Ser Pro Val Val Leu Ala Cys Leu Ile Thr Gly
            20                  25                  30
Tyr His Pro Thr Ser Val Thr Val Thr Trp Tyr Met Gly Thr Gln Ser
        35                  40                  45
Gln Pro Gln Arg Thr Phe Pro Glu Ile Gln Arg Arg Asp Ser Tyr Tyr
    50                  55                  60
Met Thr Ser Ser Gln Leu Ser Thr Pro Leu Gln Gln Trp Arg Gln Gly
65                  70                  75                  80
Glu Tyr Lys Cys Val Val Gln His Thr Ala Ser Lys Ser Lys Lys Glu
                85                  90                  95
Ile Phe Arg Trp Pro
            100

<210> 189
<211> 103
<212> PRT
<213> Homo sapiens

<400> 189
Ala Ser Thr Gln Ser Pro Ser Val Phe Pro Leu Thr Arg Cys Cys Lys
 1               5                  10                  15
Asn Ile Pro Ser Asn Ala Thr Ser Val Thr Leu Gly Cys Leu Ala Thr
            20                  25                  30
Gly Tyr Phe Pro Glu Pro Val Met Val Thr Cys Asp Thr Gly Ser Leu
        35                  40                  45
Asn Gly Thr Thr Met Thr Leu Pro Ala Thr Thr Leu Thr Leu Ser Gly
    50                  55                  60
His Tyr Ala Thr Ile Ser Leu Leu Thr Val Ser Gly Ala Trp Ala Lys
65                  70                  75                  80
Gln Met Phe Thr Cys Arg Val Ala His Thr Pro Ser Ser Thr Asp Trp
                85                  90                  95
Val Asp Asn Lys Thr Phe Ser
            100

<210> 190
<211> 98
<212> PRT
<213> Homo sapiens

<400> 190
Ala Ser Thr Lys Gly Pro Ser Val Phe Pro Leu Ala Pro Cys Ser Arg
 1               5                  10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser

50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val

<210> 191
<211> 98
<212> PRT
<213> Homo sapiens

<400> 191
Ala Ser Thr Lys Gly Pro Ser Val Phe Pro Leu Ala Pro Cys Ser Arg
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Thr Cys Asn Val Asn His Lys Pro Ser Asn Thr Lys Val Asp Lys
                85                  90                  95
Arg Val

<210> 192
<211> 98
<212> PRT
<213> Homo sapiens

<400> 192
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val

<210> 193
<400> 193
000

<210> 194
<211> 104
<212> PRT
<213> Homo sapiens

<400> 194

Gly Ser Ala Ser Ala Pro Thr Leu Phe Pro Leu Val Ser Cys Glu Asn
1               5                   10                  15
Ser Pro Ser Asp Thr Ser Ser Val Ala Val Gly Cys Leu Ala Gln Asp
            20                  25                  30
Phe Leu Pro Asp Ser Ile Thr Leu Ser Trp Lys Tyr Lys Asn Asn Ser
        35                  40                  45
Asp Ile Ser Ser Thr Arg Gly Phe Pro Ser Val Leu Arg Gly Gly Lys
    50                  55                  60
Tyr Ala Ala Thr Ser Gln Val Leu Leu Pro Ser Lys Asp Val Met Gln
65                  70                  75                  80
Gly Thr Asp Glu His Val Val Cys Lys Val Gln His Pro Asn Gly Asn
                85                  90                  95
Lys Glu Lys Asn Val Pro Leu Pro
            100

<210> 195
<211> 101
<212> PRT
<213> Homo sapiens

<400> 195
Cys Cys His Pro Arg Leu Ser Leu His Arg Pro Ala Leu Glu Asp Leu
1               5                   10                  15
Leu Leu Gly Ser Glu Ala Asn Leu Thr Cys Thr Leu Thr Gly Leu Arg
            20                  25                  30
Asp Ala Ser Gly Val Thr Phe Thr Trp Thr Pro Ser Ser Gly Lys Ser
        35                  40                  45
Ala Val Gln Gly Pro Pro Glu Arg Asp Leu Cys Gly Cys Tyr Ser Val
    50                  55                  60
Ser Ser Val Leu Pro Gly Cys Ala Glu Pro Trp Asn His Gly Lys Thr
65                  70                  75                  80
Phe Thr Cys Thr Ala Ala Tyr Pro Glu Ser Lys Thr Pro Leu Thr Ala
                85                  90                  95
Thr Leu Ser Lys Ser
            100

<210> 196
<211> 101
<212> PRT
<213> Homo sapiens

<400> 196
Cys Cys His Pro Arg Leu Ser Leu His Arg Pro Ala Leu Glu Asp Leu
1               5                   10                  15
Leu Leu Gly Ser Glu Ala Asn Leu Thr Cys Thr Leu Thr Gly Leu Arg
            20                  25                  30
Asp Ala Ser Gly Ala Thr Phe Thr Trp Thr Pro Ser Ser Gly Lys Ser
        35                  40                  45
Ala Val Gln Gly Pro Pro Glu Arg Asp Leu Cys Gly Cys Tyr Ser Val
    50                  55                  60
Ser Ser Val Leu Pro Gly Cys Ala Gln Pro Trp Asn His Gly Glu Thr
65                  70                  75                  80
Phe Thr Cys Thr Ala Ala His Pro Glu Leu Lys Thr Pro Leu Thr Ala
                85                  90                  95
Asn Ile Thr Lys Ser
            100

<210> 197
<211> 108
<212> PRT

<213> Homo sapiens

<400> 197
Glu Cys Pro Ser His Thr Gln Pro Leu Gly Val Tyr Leu Leu Thr Pro
1               5                   10                  15
Ala Val Gln Asp Leu Trp Leu Arg Asp Lys Ala Thr Phe Thr Cys Phe
            20                  25                  30
Val Val Gly Ser Asp Leu Lys Asp Ala His Leu Thr Trp Glu Val Ala
        35                  40                  45
Gly Lys Val Pro Thr Gly Gly Val Glu Glu Gly Leu Leu Glu Arg His
    50                  55                  60
Ser Asn Gly Ser Gln Ser Gln His Ser Arg Leu Thr Leu Pro Arg Ser
65                  70                  75                  80
Leu Trp Asn Ala Gly Thr Ser Val Thr Cys Thr Leu Asn His Pro Ser
                85                  90                  95
Leu Pro Pro Gln Arg Leu Met Ala Leu Arg Glu Pro
            100                 105


<210> 198
<211> 107
<212> PRT
<213> Homo sapiens

<400> 198
Val Cys Ser Arg Asp Phe Thr Pro Pro Thr Val Lys Ile Leu Gln Ser
1               5                   10                  15
Ser Cys Asp Gly Gly Gly His Phe Pro Pro Thr Ile Gln Leu Leu Cys
            20                  25                  30
Leu Val Ser Gly Tyr Thr Pro Gly Thr Ile Asn Ile Thr Trp Leu Glu
        35                  40                  45
Asp Gly Gln Val Met Asp Val Asp Leu Ser Thr Ala Ser Thr Thr Gln
    50                  55                  60
Glu Gly Glu Leu Ala Ser Thr Gln Ser Glu Leu Thr Leu Ser Gln Lys
65                  70                  75                  80
His Trp Leu Ser Asp Arg Thr Tyr Thr Cys Gln Val Thr Tyr Gln Gly
                85                  90                  95
His Thr Phe Glu Asp Ser Thr Lys Lys Cys Ala
            100                 105


<210> 199
<211> 109
<212> PRT
<213> Homo sapiens

<400> 199
Ala Pro Pro Val Ala Gly Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser His Glu Asp Pro Glu Val Gln Phe Asn Trp Tyr Val
        35                  40                  45
Asp Gly Val Glu Val His Asn Ala Lys Thr Lys Pro Arg Glu Glu Gln
    50                  55                  60
Phe Asn Ser Thr Phe Arg Val Val Ser Val Leu Thr Val Val His Gln
65                  70                  75                  80
Asp Trp Leu Asn Gly Lys Glu Tyr Lys Cys Lys Val Ser Asn Lys Gly
                85                  90                  95
Leu Pro Ala Pro Ile Glu Lys Thr Ile Ser Lys Thr Lys
            100                 105

<210> 200
<211> 110
<212> PRT
<213> Homo sapiens

<400> 200
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Gln Phe Lys Trp Tyr
        35                  40                  45
Val Asp Gly Val Glu Val His Asn Ala Lys Thr Lys Pro Arg Glu Glu
    50                  55                  60
Gln Tyr Asn Ser Thr Phe Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Thr Lys
            100                 105                 110


<210> 201
<211> 110
<212> PRT
<213> Homo sapiens

<400> 201
Ala Pro Glu Phe Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
        35                  40                  45
Val Asp Gly Val Glu Val His Asn Ala Lys Thr Lys Pro Arg Glu Glu
    50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110


<210> 202
<211> 105
<212> PRT
<213> Artificial Sequence

<220>
<223> Altered human Ck region + T70E, N29A, N30A, V55A

<400> 202
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Ala Ala Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 203
<211> 112
<212> PRT
<213> Homo sapiens

<400> 203
Val Ile Ala Glu Leu Pro Pro Lys Val Ser Val Phe Val Pro Pro Arg
 1               5                  10                  15
Asp Gly Phe Phe Gly Asn Pro Arg Lys Ser Lys Leu Ile Cys Gln Ala
            20                  25                  30
Thr Gly Phe Ser Pro Arg Gln Ile Gln Val Ser Trp Leu Arg Glu Gly
        35                  40                  45
Lys Gln Val Gly Ser Gly Val Thr Thr Asp Gln Val Gln Ala Glu Ala
    50                  55                  60
Lys Glu Ser Gly Pro Thr Thr Tyr Lys Val Thr Ser Thr Leu Thr Ile
65                  70                  75                  80
Lys Glu Ser Asp Trp Leu Gly Gln Ser Met Phe Thr Cys Arg Val Asp
                85                  90                  95
His Arg Gly Leu Thr Phe Gln Gln Asn Ala Ser Ser Met Cys Val Pro
            100                 105                 110

<210> 204
<211> 131
<212> PRT
<213> Homo sapiens

<400> 204
Gly Asn Thr Phe Arg Pro Glu Val His Leu Leu Pro Pro Pro Ser Glu
 1               5                  10                  15
Glu Leu Ala Leu Asn Glu Leu Val Thr Leu Thr Cys Leu Ala Arg Gly
            20                  25                  30
Phe Ser Pro Lys Asp Val Leu Val Arg Trp Leu Gln Gly Ser Gln Glu
        35                  40                  45
Leu Pro Arg Glu Lys Tyr Leu Thr Trp Ala Ser Arg Gln Glu Pro Ser
    50                  55                  60
Gln Gly Thr Thr Thr Phe Ala Val Thr Ser Ile Leu Arg Val Ala Ala
65                  70                  75                  80
Glu Asp Trp Lys Lys Gly Asp Thr Phe Ser Cys Met Val Gly His Glu
                85                  90                  95
Ala Leu Pro Leu Ala Phe Thr Gln Lys Thr Ile Asp Arg Leu Ala Gly
            100                 105                 110
Lys Pro Thr His Val Asn Val Ser Val Val Met Ala Glu Val Asp Gly
        115                 120                 125
Thr Cys Tyr
    130

<210> 205
<211> 131
<212> PRT
<213> Homo sapiens

<400> 205
Gly Asn Thr Phe Arg Pro Glu Val His Leu Leu Pro Pro Pro Ser Glu
 1               5                  10                  15
Glu Leu Ala Leu Asn Glu Leu Val Thr Leu Thr Cys Leu Ala Arg Gly

20                  25                  30
Phe Ser Pro Lys Asp Val Leu Val Arg Trp Leu Gln Gly Ser Gln Glu
        35                  40                  45
Leu Pro Arg Glu Lys Tyr Leu Thr Trp Ala Ser Arg Gln Glu Pro Ser
    50                  55                  60
Gln Gly Thr Thr Thr Phe Ala Val Thr Ser Ile Leu Arg Val Ala Ala
65                  70                  75                  80
Glu Asp Trp Lys Lys Gly Asp Thr Phe Ser Cys Met Val Gly His Glu
                85                  90                  95
Ala Leu Pro Leu Ala Phe Thr Gln Lys Thr Ile Asp Arg Leu Ala Gly
            100                 105                 110
Lys Pro Thr His Val Asn Val Ser Val Val Met Ala Glu Val Asp Gly
        115                 120                 125
Thr Cys Tyr
    130

<210> 206
<211> 117
<212> PRT
<213> Homo sapiens

<400> 206
Ala Ala Gln Ala Pro Val Lys Leu Ser Leu Asn Leu Leu Ala Ser Ser
 1               5                  10                  15
Asp Pro Pro Glu Ala Ala Ser Trp Leu Leu Cys Glu Val Ser Gly Phe
            20                  25                  30
Ser Pro Pro Asn Ile Leu Leu Met Trp Leu Glu Asp Gln Arg Glu Val
        35                  40                  45
Asn Thr Ser Gly Phe Ala Pro Ala Arg Pro Pro Pro Gln Pro Arg Ser
    50                  55                  60
Thr Thr Phe Trp Ala Trp Ser Val Leu Arg Val Pro Ala Pro Pro Ser
65                  70                  75                  80
Pro Gln Pro Ala Thr Tyr Thr Cys Val Val Ser His Glu Asp Ser Arg
                85                  90                  95
Thr Leu Leu Asn Ala Ser Arg Ser Leu Glu Val Ser Tyr Val Thr Asp
            100                 105                 110
His Gly Pro Met Lys
        115

<210> 207
<211> 108
<212> PRT
<213> Homo sapiens

<400> 207
Asp Ser Asn Pro Arg Gly Val Ser Ala Tyr Leu Ser Arg Pro Ser Pro
 1               5                  10                  15
Phe Asp Leu Phe Ile Arg Lys Ser Pro Thr Ile Thr Cys Leu Val Val
            20                  25                  30
Asp Leu Ala Pro Ser Lys Gly Thr Val Asn Leu Thr Trp Ser Arg Ala
        35                  40                  45
Ser Gly Lys Pro Val Asn His Ser Thr Arg Lys Glu Glu Lys Gln Arg
    50                  55                  60
Asn Gly Thr Leu Thr Val Thr Ser Thr Leu Pro Val Gly Thr Arg Asp
65                  70                  75                  80
Trp Ile Glu Gly Glu Thr Tyr Gln Cys Arg Val Thr His Pro His Leu
                85                  90                  95
Pro Arg Ala Leu Met Arg Ser Thr Thr Lys Thr Ser
            100                 105

<210> 208
<211> 107
<212> PRT
<213> Homo sapiens

<400> 208
Gly Gln Pro Arg Glu Pro Gln Val Tyr Thr Leu Pro Pro Ser Arg Glu
1               5                   10                  15
Glu Met Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Met Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Pro Gly Lys
            100                 105


<210> 209
<211> 107
<212> PRT
<213> Homo sapiens

<400> 209
Gly Gln Pro Arg Glu Pro Gln Val Tyr Thr Leu Pro Pro Ser Arg Glu
1               5                   10                  15
Glu Met Thr Lys Asn Gln Val Ser Leu Thr Cys Leu Val Lys Gly Phe
            20                  25                  30
Tyr Pro Ser Asp Ile Ala Val Glu Trp Glu Ser Ser Gly Gln Pro Glu
        35                  40                  45
Asn Asn Tyr Asn Thr Thr Pro Pro Met Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Ile Phe Ser Cys Ser Val Met His Glu Ala Leu His Asn Arg Phe
                85                  90                  95
Thr Gln Lys Ser Leu Ser Leu Ser Pro Gly Lys
            100                 105


<210> 210
<211> 107
<212> PRT
<213> Homo sapiens

<400> 210
Gly Gln Pro Arg Glu Pro Gln Val Tyr Thr Leu Pro Pro Ser Gln Glu
1               5                   10                  15
Glu Met Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Val Leu Asp Ser Asp Gly Ser Phe
    50                  55                  60
Phe Leu Tyr Ser Arg Leu Thr Val Asp Lys Ser Arg Trp Gln Glu Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Leu Gly Lys

100                 105

<210> 211
<211> 7
<212> PRT
<213> Homo sapiens

<400> 211
Glu Arg Lys Cys Cys Val Glu
1               5

<210> 212
<211> 106
<212> PRT
<213> Homo sapiens

<400> 212
Asp Gln Asp Thr Ala Ile Arg Val Phe Ala Ile Pro Pro Ser Phe Ala
1               5                   10                  15
Ser Ile Phe Leu Thr Lys Ser Thr Lys Leu Thr Cys Leu Val Thr Asp
            20                  25                  30
Leu Thr Thr Tyr Asp Ser Val Thr Ile Ser Trp Thr Arg Gln Asn Gly
        35                  40                  45
Glu Ala Val Lys Thr His Thr Asn Ile Ser Glu Ser His Pro Asn Ala
    50                  55                  60
Thr Phe Ser Ala Val Gly Glu Ala Ser Ile Cys Glu Asp Asp Trp Asn
65                  70                  75                  80
Ser Gly Glu Arg Phe Thr Cys Thr Val Thr His Thr Asp Leu Pro Ser
                85                  90                  95
Pro Leu Lys Gln Thr Ile Ser Arg Pro Lys
            100                 105

<210> 213
<211> 110
<212> PRT
<213> Homo sapiens

<400> 213
Gly Pro Arg Ala Ala Pro Glu Val Tyr Ala Phe Ala Thr Pro Glu Trp
1               5                   10                  15
Pro Gly Ser Arg Asp Lys Arg Thr Leu Ala Cys Leu Ile Gln Asn Phe
            20                  25                  30
Met Pro Glu Asp Ile Ser Val Gln Trp Leu His Asn Glu Val Gln Leu
        35                  40                  45
Pro Asp Ala Arg His Ser Thr Thr Gln Pro Arg Lys Thr Lys Gly Ser
    50                  55                  60
Gly Phe Phe Val Phe Ser Arg Leu Glu Val Thr Arg Ala Glu Trp Glu
65                  70                  75                  80
Gln Lys Asp Glu Phe Ile Cys Arg Ala Val His Glu Ala Ala Ser Pro
                85                  90                  95
Ser Gln Thr Val Gln Arg Ala Val Ser Val Asn Pro Gly Lys
            100                 105                 110

<210> 214
<211> 131
<212> PRT
<213> Homo sapiens

<400> 214

Gly Val Ala Leu His Arg Pro Asp Val Tyr Leu Leu Pro Pro Ala Arg
1               5                   10                  15
Glu Gln Leu Asn Leu Arg Glu Ser Ala Thr Ile Thr Cys Leu Val Thr
            20                  25                  30
Gly Phe Ser Pro Ala Asp Val Phe Val Gln Trp Met Gln Arg Gly Gln
        35                  40                  45
Pro Leu Ser Pro Glu Lys Tyr Val Thr Ser Ala Pro Met Pro Glu Pro
    50                  55                  60
Gln Ala Pro Gly Arg Tyr Phe Ala His Ser Ile Leu Thr Val Ser Glu
65                  70                  75                  80
Glu Glu Trp Asn Thr Gly Glu Thr Tyr Thr Cys Val Val Ala His Glu
                85                  90                  95
Ala Leu Pro Asn Arg Val Thr Glu Arg Thr Val Asp Lys Ser Thr Gly
            100                 105                 110
Lys Pro Thr Leu Tyr Asn Val Ser Leu Val Met Ser Asp Thr Ala Gly
        115                 120                 125
Thr Cys Tyr
    130

<210> 215
<211> 19
<212> PRT
<213> Homo sapiens

<400> 215
Val Pro Ser Thr Pro Pro Thr Pro Ser Pro Ser Thr Pro Pro Thr Pro
1               5                   10                  15
Ser Pro Ser

<210> 216
<211> 6
<212> PRT
<213> Homo sapiens

<400> 216
Val Pro Pro Pro Pro Pro
1               5

<210> 217
<211> 58
<212> PRT
<213> Homo sapiens

<400> 217
Glu Ser Pro Lys Ala Gln Ala Ser Ser Val Pro Thr Ala Gln Pro Gln
1               5                   10                  15
Ala Glu Gly Ser Leu Ala Lys Ala Thr Thr Ala Pro Ala Thr Thr Arg
            20                  25                  30
Asn Thr Gly Arg Gly Gly Glu Glu Lys Lys Lys Glu Lys Glu Lys Glu
        35                  40                  45
Glu Gln Glu Glu Arg Glu Thr Lys Thr Pro
    50                  55

<210> 218
<400> 218
000

<210> 219
<211> 12
<212> PRT
<213> Homo sapiens

<400> 219
Glu Arg Lys Cys Cys Val Glu Cys Pro Pro Cys Pro
1               5                   10


<210> 220
<211> 62
<212> PRT
<213> Homo sapiens

<400> 220
Glu Leu Lys Thr Pro Leu Gly Asp Thr Thr His Thr Cys Pro Arg Cys
1               5                   10                  15
Pro Glu Pro Lys Ser Cys Asp Thr Pro Pro Pro Cys Pro Arg Cys Pro
            20                  25                  30
Glu Pro Lys Ser Cys Asp Thr Pro Pro Pro Cys Pro Arg Cys Pro Glu
        35                  40                  45
Pro Lys Ser Cys Asp Thr Pro Pro Pro Cys Pro Arg Cys Pro
    50                  55                  60


<210> 221
<211> 12
<212> PRT
<213> Homo sapiens

<400> 221
Glu Ser Lys Tyr Gly Pro Pro Cys Pro Ser Cys Pro
1               5                   10


<210> 222
<211> 34
<212> PRT
<213> Homo sapiens

<400> 222
Glu Ser Pro Lys Ala Gln Ala Ser Ser Val Pro Thr Ala Gln Pro Gln
1               5                   10                  15
Ala Glu Gly Ser Leu Ala Lys Ala Thr Thr Ala Pro Ala Thr Thr Arg
            20                  25                  30
Asn Thr


<210> 223
<211> 106
<212> PRT
<213> Homo sapiens

<400> 223
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn

50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 224
<211> 106
<212> PRT
<213> Homo sapiens

<400> 224
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Lys Ser Tyr Ser Cys Gln Val Thr His Glu Gly Ser Thr Val
                85                  90                  95
Glu Lys Thr Val Ala Pro Thr Glu Cys Ser
            100                 105

<210> 225
<211> 106
<212> PRT
<213> Homo sapiens

<400> 225
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Lys Val Ala Trp Lys Ala Asp Gly Ser Pro
        35                  40                  45
Val Asn Thr Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Ala Glu Cys Ser
            100                 105

<210> 226
<211> 106
<212> PRT
<213> Homo sapiens

<400> 226
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Val Ser Asp
            20                  25                  30

Phe Tyr Pro Gly Ala Val Thr Val Ala Trp Lys Ala Asp Gly Ser Pro
        35                  40                  45
Val Lys Val Gly Val Glu Thr Thr Lys Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Arg Val Thr His Glu Gly Ser Thr Val
                85                  90                  95
Glu Lys Thr Val Ala Pro Ala Glu Cys Ser
            100                 105

<210> 227
<211> 10
<212> PRT
<213> Homo sapiens

<400> 227
Glu Pro Lys Ser Cys Asp Lys Thr His Thr
1               5                   10

<210> 228
<211> 5
<212> PRT
<213> Homo sapiens

<400> 228
Cys Pro Pro Cys Pro
1               5

<210> 229
<400> 229
000

<210> 230
<400> 230
000

<210> 231
<400> 231
000

<210> 232
<211> 15
<212> PRT
<213> Artificial Sequence

<220>
<223> Altered human IgG1 hinge (CSC-P)

<400> 232
Glu Pro Lys Ser Cys Asp Lys Thr His Thr Ser Pro Pro Cys Pro
1               5                   10                  15

<210> 233
<400> 233
000

<210> 234
<211> 15
<212> PRT
<213> Artificial Sequence

<220>
<223> Altered human IgG1 hinge (CCS-P)

<400> 234
Glu Pro Lys Ser Cys Asp Lys Thr His Thr Cys Pro Pro Ser Pro
1               5                   10                  15


<210> 235
<400> 235
000


<210> 236
<400> 236
000


<210> 237
<400> 237
000


<210> 238
<400> 238
000


<210> 239
<400> 239
000


<210> 240
<211> 15
<212> PRT
<213> Artificial Sequence

<220>
<223> Altered human IgG1 hinge (CCS-S)

<400> 240
Glu Pro Lys Ser Cys Asp Lys Thr His Thr Cys Pro Pro Ser Ser
1               5                   10                  15


<210> 241
<211> 12
<212> PRT
<213> Artificial Sequence

<220>
<223> Stalk  region of CD69

<400> 241
Gln Tyr Asn Cys Pro Gly Gln Tyr Thr Phe Ser Met

1                 5                   10


<210> 242
<211> 11
<212> PRT
<213> Artificial Sequence

<220>
<223> Stalk  region of CD72

<400> 242
Pro Phe Phe Thr Cys Gly Ser Ala Asp Thr Cys
1                 5                   10


<210> 243
<211> 18
<212> PRT
<213> Artificial Sequence

<220>
<223> Stalk  region of CD94

<400> 243
Glu Pro Ala Phe Thr Pro Gly Pro Asn Ile Glu Leu Gln Lys Asp Ser
1                 5                   10                  15
Asp Cys


<210> 244
<211> 18
<212> PRT
<213> Artificial Sequence

<220>
<223> Stalk  region of NKG2A

<400> 244
Gln Arg His Asn Asn Ser Ser Leu Asn Thr Arg Thr Gln Lys Ala Arg
1                 5                   10                  15
His Cys


<210> 245
<211> 12
<212> PRT
<213> Homo sapiens

<400> 245
Glu Leu Lys Thr Pro Leu Gly Asp Thr Thr His Thr
1                 5                   10


<210> 246
<211> 10
<212> PRT
<213> Homo sapiens

<400> 246
Glu Pro Lys Ser Cys Asp Thr Pro Pro Pro

1               5                   10


<210> 247
<211> 7
<212> PRT
<213> Homo sapiens

<400> 247
Glu Ser Lys Tyr Gly Pro Pro
1               5


<210> 248
<211> 5
<212> PRT
<213> Homo sapiens

<400> 248
Cys Pro Arg Cys Pro
1               5


<210> 249
<211> 5
<212> PRT
<213> Homo sapiens

<400> 249
Cys Pro Ser Cys Pro
1               5


<210> 250
<211> 17
<212> PRT
<213> Homo sapiens

<400> 250
Glu Leu Lys Thr Pro Leu Gly Asp Thr Thr His Thr Cys Pro Arg Cys
1               5                   10                  15
Pro


<210> 251
<211> 15
<212> PRT
<213> Homo sapiens

<400> 251
Glu Pro Lys Ser Cys Asp Thr Pro Pro Pro Cys Pro Arg Cys Pro
1               5                   10                  15


<210> 252
<211> 24
<212> PRT
<213> Homo sapiens

<400> 252
Gly Arg Gly Gly Glu Glu Lys Lys Lys Glu Lys Glu Lys Glu Glu Gln
1               5                   10                  15

Glu Glu Arg Glu Thr Lys Thr Pro
            20

<210> 253
<211> 107
<212> PRT
<213> Homo sapiens

<400> 253
Val Cys Ser Arg Asp Phe Thr Pro Pro Thr Val Lys Ile Leu Gln Ser
1               5                   10                  15
Ser Ser Asp Gly Gly Gly His Phe Pro Pro Thr Ile Gln Leu Leu Cys
            20                  25                  30
Leu Val Ser Gly Tyr Thr Pro Gly Thr Ile Asn Ile Thr Trp Leu Glu
        35                  40                  45
Asp Gly Gln Val Met Asp Val Asp Leu Ser Thr Ala Ser Thr Thr Gln
    50                  55                  60
Glu Gly Glu Leu Ala Ser Thr Gln Ser Glu Leu Thr Leu Ser Gln Lys
65                  70                  75                  80
His Trp Leu Ser Asp Arg Thr Tyr Thr Cys Gln Val Thr Tyr Gln Gly
                85                  90                  95
His Thr Phe Glu Asp Ser Thr Lys Lys Cys Ala
            100                 105

<210> 254
<211> 112
<212> PRT
<213> Homo sapiens

<400> 254
Val Ile Ala Glu Leu Pro Pro Lys Val Ser Val Phe Val Pro Pro Arg
1               5                   10                  15
Asp Gly Phe Phe Gly Asn Pro Arg Lys Ser Lys Leu Ile Cys Gln Ala
            20                  25                  30
Thr Gly Phe Ser Pro Arg Gln Ile Gln Val Ser Trp Leu Arg Glu Gly
        35                  40                  45
Lys Gln Val Gly Ser Gly Val Thr Thr Asp Gln Val Gln Ala Glu Ala
    50                  55                  60
Lys Glu Ser Gly Pro Thr Thr Tyr Lys Val Thr Ser Thr Leu Thr Ile
65                  70                  75                  80
Lys Glu Ser Asp Trp Leu Gly Gln Ser Met Phe Thr Cys Arg Val Asp
                85                  90                  95
His Arg Gly Leu Thr Phe Gln Gln Asn Ala Ser Ser Met Cys Val Pro
            100                 105                 110

<210> 255
<211> 107
<212> PRT
<213> Artificial Sequence

<220>
<223> Altered human CH2 upper hinge like sequence of IgE

<400> 255
Val Cys Ser Arg Asp Phe Thr Pro Pro Thr Val Lys Ile Leu Gln Ser
1               5                   10                  15
Ser Ser Asp Gly Gly Gly His Phe Pro Pro Thr Ile Gln Leu Leu Cys
            20                  25                  30
Leu Val Ser Gly Tyr Thr Pro Gly Thr Ile Asn Ile Thr Trp Leu Glu
        35                  40                  45

Asp Gly Gln Val Met Asp Val Asp Leu Ser Thr Ala Ser Thr Thr Gln
    50                  55                  60
Glu Gly Glu Leu Ala Ser Thr Gln Ser Glu Leu Thr Leu Ser Gln Lys
65                  70                  75                  80
His Trp Leu Ser Asp Arg Thr Tyr Thr Cys Gln Val Thr Tyr Gln Gly
                85                  90                  95
His Thr Phe Glu Asp Ser Thr Lys Lys Ser Ala
            100                 105

<210> 256
<211> 268
<212> PRT
<213> Artificial Sequence

<220>
<223> 5D5 scFv (VLVH)

<220>
<221> SIGNAL
<222> (10)...(20)

<400> 256
Met Glu Ala Pro Ala Gln Leu Leu Phe Leu Leu Leu Leu Trp Leu Pro
-20                 -15                 -10                 -5
Asp Thr Thr Gly Asp Phe Met Met Ser Gln Ser Pro Ser Ser Leu Thr
                1               5                   10
Val Ser Val Gly Glu Lys Val Thr Val Ser Cys Lys Ser Ser Gln Ser
        15                  20                  25
Leu Leu Tyr Thr Ser Ser Gln Lys Asn Tyr Leu Ala Trp Tyr Gln Gln
    30                  35                  40
Lys Pro Gly Gln Ser Pro Lys Leu Leu Ile Tyr Trp Ala Ser Thr Arg
45                  50                  55                  60
Glu Ser Gly Val Pro Asp Arg Phe Thr Gly Ser Gly Ser Gly Thr Asp
                65                  70                  75
Phe Thr Leu Thr Ile Thr Ser Val Lys Ala Asp Asp Leu Ala Val Tyr
            80                  85                  90
Tyr Cys Gln Gln Tyr Tyr Ala Tyr Pro Trp Thr Phe Gly Gly Gly Thr
        95                  100                 105
Lys Leu Glu Leu Lys Gly Gly Gly Gly Ser Gly Gly Gly Gly Ser Gly
    110                 115                 120
Gly Gly Gly Ser Gln Val Gln Leu Gln Gln Ser Gly Pro Glu Leu Val
125                 130                 135                 140
Arg Pro Gly Ala Ser Val Lys Met Ser Cys Arg Ala Ser Gly Tyr Thr
                145                 150                 155
Phe Thr Ser Tyr Trp Leu His Trp Val Lys Gln Arg Pro Gly Gln Gly
            160                 165                 170
Leu Glu Trp Ile Gly Met Ile Asp Pro Ser Asn Ser Asp Thr Arg Phe
        175                 180                 185
Asn Pro Asn Phe Lys Asp Lys Ala Thr Leu Asn Val Asp Arg Ser Ser
    190                 195                 200
Asn Thr Ala Tyr Met Leu Leu Ser Ser Leu Thr Ser Ala Asp Ser Ala
205                 210                 215                 220
Val Tyr Tyr Cys Ala Thr Tyr Gly Ser Tyr Val Ser Pro Leu Asp Tyr
                225                 230                 235
Trp Gly Gln Gly Thr Ser Val Thr Val Ser Ser Ser
            240                 245

<210> 257
<211> 248
<212> PRT
<213> Artificial Sequence

<220>
<223> 5D5 scFv (VLVH) mature protein without the signal
      peptide

<400> 257
Asp Phe Met Met Ser Gln Ser Pro Ser Ser Leu Thr Val Ser Val Gly
1               5                   10                  15
Glu Lys Val Thr Val Ser Cys Lys Ser Ser Gln Ser Leu Leu Tyr Thr
            20                  25                  30
Ser Ser Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Thr Ser Val Lys Ala Asp Asp Leu Ala Val Tyr Tyr Cys Gln Gln
                85                  90                  95
Tyr Tyr Ala Tyr Pro Trp Thr Phe Gly Gly Gly Thr Lys Leu Glu Leu
            100                 105                 110
Lys Gly Gly Gly Gly Ser Gly Gly Gly Gly Ser Gly Gly Gly Gly Ser
        115                 120                 125
Gln Val Gln Leu Gln Gln Ser Gly Pro Glu Leu Val Arg Pro Gly Ala
    130                 135                 140
Ser Val Lys Met Ser Cys Arg Ala Ser Gly Tyr Thr Phe Thr Ser Tyr
145                 150                 155                 160
Trp Leu His Trp Val Lys Gln Arg Pro Gly Gln Gly Leu Glu Trp Ile
                165                 170                 175
Gly Met Ile Asp Pro Ser Asn Ser Asp Thr Arg Phe Asn Pro Asn Phe
            180                 185                 190
Lys Asp Lys Ala Thr Leu Asn Val Asp Arg Ser Ser Asn Thr Ala Tyr
        195                 200                 205
Met Leu Leu Ser Ser Leu Thr Ser Ala Asp Ser Ala Val Tyr Tyr Cys
    210                 215                 220
Ala Thr Tyr Gly Ser Tyr Val Ser Pro Leu Asp Tyr Trp Gly Gln Gly
225                 230                 235                 240
Thr Ser Val Thr Val Ser Ser Ser
                245


<210> 258
<211> 120
<212> PRT
<213> Artificial Sequence

<220>
<223> 5D5 VH

<400> 258
Gln Val Gln Leu Gln Gln Ser Gly Pro Glu Leu Val Arg Pro Gly Ala
1               5                   10                  15
Ser Val Lys Met Ser Cys Arg Ala Ser Gly Tyr Thr Phe Thr Ser Tyr
            20                  25                  30
Trp Leu His Trp Val Lys Gln Arg Pro Gly Gln Gly Leu Glu Trp Ile
        35                  40                  45
Gly Met Ile Asp Pro Ser Asn Ser Asp Thr Arg Phe Asn Pro Asn Phe
    50                  55                  60
Lys Asp Lys Ala Thr Leu Asn Val Asp Arg Ser Ser Asn Thr Ala Tyr
65                  70                  75                  80
Met Leu Leu Ser Ser Leu Thr Ser Ala Asp Ser Ala Val Tyr Tyr Cys
                85                  90                  95
Ala Thr Tyr Gly Ser Tyr Val Ser Pro Leu Asp Tyr Trp Gly Gln Gly
            100                 105                 110
Thr Ser Val Thr Val Ser Ser Ser

115                 120


<210> 259
<211> 113
<212> PRT
<213> Artificial Sequence

<220>
<223> 5D5 VL

<400> 259
Asp Phe Met Met Ser Gln Ser Pro Ser Ser Leu Thr Val Ser Val Gly
1               5                   10                  15
Glu Lys Val Thr Val Ser Cys Lys Ser Ser Gln Ser Leu Leu Tyr Thr
            20                  25                  30
Ser Ser Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Thr Ser Val Lys Ala Asp Asp Leu Ala Val Tyr Tyr Cys Gln Gln
                85                  90                  95
Tyr Tyr Ala Tyr Pro Trp Thr Phe Gly Gly Gly Thr Lys Leu Glu Leu
            100                 105                 110
Lys



<210> 260
<211> 792
<212> DNA
<213> Artificial Sequence

<220>
<223> 4C04 anti-Ron scFv with signal peptide and
      junction amino acids SSS at carboxy-terminus

<400> 260
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt gggaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gc                                                     792

<210> 261
<211> 244
<212> PRT
<213> Artificial Sequence

<220>
<223> 4C04 anti-Ron scFv with signal peptide and
      junction amino acids SSS at carboxy-terminus

<400> 261
Asp Ile Gln Met Thr Gln Ser Pro Ala Ser Gln Ser Ala Ser Leu Gly
1               5                   10                  15
Glu Ser Val Thr Ile Thr Cys Leu Ala Ser Gln Thr Ile Gly Thr Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Ala
    50                  55                  60
Ser Gly Ser Gly Thr Lys Phe Ser Phe Lys Ile Asp Ser Leu Gln Ser
65                  70                  75                  80
Glu Asp Phe Val Ser Tyr Tyr Cys Gln Gln Leu Tyr Asn Thr Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Ile Gln Leu Gln Gln
        115                 120                 125
Ser Gly Pro Glu Leu Val Lys Pro Gly Thr Ser Val Lys Ile Ser Cys
    130                 135                 140
Lys Ala Ser Asp Tyr Thr Leu Thr Asp Phe Tyr Met Asn Trp Val Arg
145                 150                 155                 160
Gln Lys Pro Gly Gln Gly Leu Glu Trp Ile Gly Arg Ile Tyr Pro Gly
                165                 170                 175
Thr Asp Lys Thr Arg Tyr Asn Glu Lys Phe Arg Asp Lys Ala Ile Leu
            180                 185                 190
Thr Val Asp Thr Ser Ser Ser Thr Ala Tyr Met Gln Leu Ser Ser Leu
        195                 200                 205
Thr Ser Glu Asp Thr Ala Val Tyr Phe Cys Ala Arg Ser Ala Tyr Tyr
    210                 215                 220
Gly Asn Tyr Val Ala Met Asp Tyr Trp Gly Gln Gly Thr Ser Val Thr
225                 230                 235                 240
Val Ser Ser Ser

<210> 262
<211> 2115
<212> DNA
<213> Artificial Sequence

<220>
<223> 4C04 anti-Ron single chain polypeptide (4C04
      scFv-CH1-CH2-CH3-Ck with signal peptide)

<400> 262
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt ggaaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gcgcctccac caagggccca tcggtcttcc ccctggcacc ctcctccaag 840
agcacctctg ggggcacagc ggccctgggc tgcctggtca aggactactt ccccgagccg 900
gtgacggtgt cgtggaactc aggcgccctg accagcggcg tgcacacctt cccggctgtc 960
ctacagtcct caggactcta ctccctcagc agcgtggtga ccgtgccctc cagcagcttg 1020

ggcacccaga cctacatctg caacgtgaat cacaagccca gcaacaccaa ggtggacaag 1080
aaagttcgta cggagcccaa atcttctgac aaaactcaca catgcccacc gtgcccatcc 1140
ggagcacctg aactcctggg tggaccgtca gtcttcctct tccccccaaa acccaaggac 1200
accctcatga tctcccggac ccctgaggtc acatgcgtgg tggtggacgt gagccacgaa 1260
gaccctgagg tcaagttcaa ctggtacgtg gacggcgtgg aggtgcataa tgccaagaca 1320
aagccgcggg aggagcagta caacagcacg taccgtgtgg tcagcgtcct caccgtcctg 1380
caccaggact ggctgaatgg caaggagtac aagtgcaagg tctccaacaa agccctccca 1440
gcccccatcg agaaaaccat ctccaaagcc aaagggcagc cccgagaacc acaggtgtac 1500
accctgcccc catcccggga tgagctgacc aagaaccagg tcagcctgac ctgcctggtc 1560
aaaggcttct atccaagcga catcgccgtg gagtgggaga gcaatgggca gccggagaac 1620
aactacaaga ccacgcctcc cgtgctggac tccgacggct ccttcttcct ctacagcaag 1680
ctcaccgtgg acaagagcag gtggcagcag gggaacgtct tctcatgctc cgtgatgcat 1740
gaggctctgc acaaccacta cacgcagaag agcctctccc tgtctccggg taaatctaga 1800
actgtggctg caccatctgt cttcatcttc ccgccatctg atgagcagtt gaaatctgga 1860
actgcctctg ttgtgtgcct gctgaataac ttctatccca gagaggccaa agtacagtgg 1920
aaggtggata acgccctcca atcgggtaac tcccaggaga gtgtcacaga gcaggacagc 1980
aaggacagca cctacagcct cagcagcacc ctgacgctga gcaaagcaga ctacgagaaa 2040
cacaaagtct acgcctgcga agtcacccat cagggcctga gctcgcccgt cacaaagagc 2100
ttcaacaggg gagag 2115

<210> 263
<400> 263
000

<210> 264
<400> 264
000

<210> 265
<211> 240
<212> PRT
<213> Artificial Sequence

<220>
<223> 11H09 anti-RON scFv mature protein without the
signal peptide but with junction amino acids SSS
at carboxy-terminus

<400> 265
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Ser Ser
            20                  25                  30
Leu Ser Trp Leu Gln Gln Glu Pro Asp Gly Thr Ile Lys Arg Leu Ile
        35                  40                  45
Tyr Ser Thr Ser Ser Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Ser Ser Pro Trp
                85                  90                  95
Thr Phe Gly Gly Gly Thr Lys Leu Glu Ile Lys Gly Gly Gly Gly Ser
            100                 105                 110
Gly Gly Gly Gly Ser Gly Gly Gly Gly Ser Gln Val Gln Leu Gln Gln
        115                 120                 125
Ser Gly Ala Glu Leu Ala Lys Pro Gly Ala Ser Val Lys Met Ser Cys
    130                 135                 140
Lys Ser Ser Gly Tyr Thr Phe Thr Ser Tyr Trp Ile His Trp Val Lys
145                 150                 155                 160
Glu Arg Pro Gly Gln Gly Leu Glu Trp Ile Gly Tyr Ile Tyr Pro Thr
                165                 170                 175
Thr Gly Tyr Thr Glu Ser Asn Gln Lys Phe Lys Asp Lys Ala Thr Leu

180                 185                 190
Thr Ala Asp Lys Ser Ser Ser Thr Ala Tyr Met Gln Leu Ser Ser Leu
        195                 200                 205
Thr Ser Glu Asp Ser Ala Val Tyr Tyr Cys Phe Leu Leu Arg Pro Trp
    210                 215                 220
Tyr Phe Asp Val Trp Gly Ala Gly Thr Thr Val Thr Val Ser Ser Ser
225                 230                 235                 240

<210> 266
<400> 266
 000

<210> 267
<400> 267
 000

<210> 268
<400> 268
 000

<210> 269
<400> 269
 000

<210> 270
<400> 270
 000

<210> 271
<400> 271
 000

<210> 272
<400> 272
 000

<210> 273
<400> 273
 000

<210> 274
<400> 274
 000

<210> 275
<400> 275
 000

<210> 276
<400> 276
 000

<210> 277

<400> 277
000

<210> 278
<400> 278
000

<210> 279
<400> 279
000

<210> 280
<400> 280
000

<210> 281
<400> 281
000

<210> 282
<400> 282
000

<210> 283
<400> 283
000

<210> 284
<400> 284
000

<210> 285
<400> 285
000

<210> 286
<400> 286
000

<210> 287
<400> 287
000

<210> 288
<400> 288
000

<210> 289
<400> 289
000

<210> 290
<400> 290
 000

<210> 291
<400> 291
 000

<210> 292
<400> 292
 000

<210> 293
<400> 293
 000

<210> 294
<400> 294
 000

<210> 295
<400> 295
 000

<210> 296
<211> 11
<212> PRT
<213> Artificial Sequence

<220>
<223> Anti-c-Met Light Chain Complementarity Determining
      Region

<400> 296
Arg Ala Ser Gln Ser Ile Arg Asn Tyr Leu Asn
 1               5                   10

<210> 297
<211> 7
<212> PRT
<213> Artificial Sequence

<220>
<223> Anti-c-Met Light Chain Complementarity Determining
      Region

<400> 297
Ala Ala Ser Ser Leu Gln Ser
 1               5

<210> 298
<211> 9
<212> PRT
<213> Artificial Sequence

<220>
<223> Anti-c-Met Light Chain Complementarity Determining
      Region

<400> 298
Gln Gln Ser Tyr Val Thr Pro Leu Thr
1               5

<210> 299
<400> 299
000

<210> 300
<400> 300
000

<210> 301
<211> 119
<212> PRT
<213> Artificial Sequence

<220>
<223> OKT3 VH amino acid sequence

<400> 301
Gln Val Gln Leu Gln Gln Ser Gly Ala Glu Leu Ala Arg Pro Gly Ala
1               5                   10                  15
Ser Val Lys Met Ser Cys Lys Ala Ser Gly Tyr Thr Phe Thr Arg Tyr
            20                  25                  30
Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Val Thr Val Ser Ser
        115

<210> 302
<211> 106
<212> PRT
<213> Artificial Sequence

<220>
<223> OKT3 VL amino acid sequence

<400> 302

<210> 303
<211> 123
<212> PRT
<213> Artificial Sequence

<220>
<223> BC3 VH amino acid sequence

<400> 303
Gln Val Gln Leu Gln Gln Ser Ala Ala Glu Leu Ala Arg Pro Gly Ala
1               5                   10                  15
Ser Val Lys Met Ser Cys Lys Ala Ser Gly Tyr Thr Phe Thr Arg Tyr
            20                  25                  30
Thr Met Gln Trp Val Lys Gln Arg Pro Gly Gln Gly Leu Glu Trp Ile
        35                  40                  45
Gly Tyr Ile Asn Pro Ser Ser Gly Tyr Ile Gly Tyr Ser Gln Lys Phe
    50                  55                  60
Lys Asp Lys Thr Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Ser Lys Val Tyr Tyr Asp Tyr Asp Val Tyr Ser Met Asp Tyr
            100                 105                 110
Trp Gly Gln Gly Thr Ser Val Thr Val Ser Ser
        115                 120

<210> 304
<211> 106
<212> PRT
<213> Artificial Sequence

<220>
<223> BC3 VL amino acid sequence

<400> 304
Gln Ile Ile Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Leu Thr
                85                  90                  95
Phe Gly Ala Gly Thr Lys Leu Glu Leu Lys
            100                 105

<210> 305
<211> 217
<212> PRT
<213> Artificial Sequence

<220>
<223> G1 N297A  CH2-CH3 (N297ST - A297ST)

<400> 305
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Ala Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Ala Lys Gly Gln
            100                 105                 110
Pro Arg Glu Pro Gln Val Tyr Thr Leu Pro Pro Ser Arg Asp Glu Leu
        115                 120                 125
Thr Lys Asn Gln Val Ser Leu Thr Cys Leu Val Lys Gly Phe Tyr Pro
    130                 135                 140
Ser Asp Ile Ala Val Glu Trp Glu Ser Asn Gly Gln Pro Glu Asn Asn
145                 150                 155                 160
Tyr Lys Thr Thr Pro Pro Val Leu Asp Ser Asp Gly Ser Phe Phe Leu
                165                 170                 175
Tyr Ser Lys Leu Thr Val Asp Lys Ser Arg Trp Gln Gln Gly Asn Val
            180                 185                 190
Phe Ser Cys Ser Val Met His Glu Ala Leu His Asn His Tyr Thr Gln
        195                 200                 205
Lys Ser Leu Ser Leu Ser Pro Gly Lys
    210                 215


<210> 306
<211> 216
<212> PRT
<213> Artificial Sequence

<220>
<223> IgG1 AA N297A CH2CH3 (Ala substitutions at 234,
      235, 237 and 297; 236 deleted)

<400> 306
Ala Pro Glu Ala Ala Ala Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser His Glu Asp Pro Glu Val Lys Phe Asn Trp Tyr Val
        35                  40                  45
Asp Gly Val Glu Val His Asn Ala Lys Thr Lys Pro Arg Glu Glu Gln
    50                  55                  60
Tyr Ala Ser Thr Tyr Arg Val Val Ser Val Leu Thr Val Leu His Gln
65                  70                  75                  80
Asp Trp Leu Asn Gly Lys Glu Tyr Lys Cys Lys Val Ser Asn Lys Ala
                85                  90                  95

Leu Pro Ala Pro Ile Glu Lys Thr Ile Ser Lys Ala Lys Gly Gln Pro
            100                 105                 110
Arg Glu Pro Gln Val Tyr Thr Leu Pro Pro Ser Arg Asp Glu Leu Thr
        115                 120                 125
Lys Asn Gln Val Ser Leu Thr Cys Leu Val Lys Gly Phe Tyr Pro Ser
    130                 135                 140
Asp Ile Ala Val Glu Trp Glu Ser Asn Gly Gln Pro Glu Asn Asn Tyr
145                 150                 155                 160
Lys Thr Thr Pro Pro Val Leu Asp Ser Asp Gly Ser Phe Phe Leu Tyr
                165                 170                 175
Ser Lys Leu Thr Val Asp Lys Ser Arg Trp Gln Gln Gly Asn Val Phe
            180                 185                 190
Ser Cys Ser Val Met His Glu Ala Leu His Asn His Tyr Thr Gln Lys
        195                 200                 205
Ser Leu Ser Leu Ser Pro Gly Lys
    210                 215


<210> 307
<211> 216
<212> PRT
<213> Artificial Sequence

<220>
<223> G2 AA N297A CH2CH3 (Ala substitution at 234, 236
      and 297)

<400> 307
Ala Pro Glu Ala Ala Ala Pro Ser Val Phe Leu Phe Pro Pro Lys Pro
 1               5                  10                  15
Lys Asp Thr Leu Met Ile Ser Arg Thr Pro Glu Val Thr Cys Val Val
            20                  25                  30
Val Asp Val Ser His Glu Asp Pro Glu Val Gln Phe Asn Trp Tyr Val
        35                  40                  45
Asp Gly Met Glu Val His Asn Ala Lys Thr Lys Pro Arg Glu Glu Gln
    50                  55                  60
Phe Ala Ser Thr Phe Arg Val Val Ser Val Leu Thr Val Val His Gln
65                  70                  75                  80
Asp Trp Leu Asn Gly Lys Glu Tyr Lys Cys Lys Val Ser Asn Lys Gly
                85                  90                  95
Leu Pro Ala Pro Ile Glu Lys Thr Ile Ser Lys Thr Lys Gly Gln Pro
            100                 105                 110
Arg Glu Pro Gln Val Tyr Thr Leu Pro Pro Ser Arg Glu Glu Met Thr
        115                 120                 125
Lys Asn Gln Val Ser Leu Thr Cys Leu Val Lys Gly Phe Tyr Pro Ser
    130                 135                 140
Asp Ile Ala Val Glu Trp Glu Ser Asn Gly Gln Pro Glu Asn Asn Tyr
145                 150                 155                 160
Lys Thr Thr Pro Pro Met Leu Asp Ser Asp Gly Ser Phe Phe Leu Tyr
                165                 170                 175
Ser Lys Leu Thr Val Asp Lys Ser Arg Trp Gln Gln Gly Asn Val Phe
            180                 185                 190
Ser Cys Ser Val Met His Glu Ala Leu His Asn His Tyr Thr Gln Lys
        195                 200                 205
Ser Leu Ser Leu Ser Pro Gly Lys
    210                 215


<210> 308
<211> 216
<212> PRT
<213> Artificial Sequence

<220>
<223> G4 AA N297A CH2CH3 (Ala substitutions at 234, 235,
      237 and 297; 236 deleted)

<400> 308
Ala Pro Glu Ala Ala Ala Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser Gln Glu Asp Pro Glu Val Gln Phe Asn Trp Tyr Val
        35                  40                  45
Asp Gly Val Glu Val His Asn Ala Lys Thr Lys Pro Arg Glu Glu Gln
    50                  55                  60
Phe Ala Ser Thr Tyr Arg Val Val Ser Val Leu Thr Val Leu His Gln
65                  70                  75                  80
Asp Trp Leu Asn Gly Lys Glu Tyr Lys Cys Lys Val Ser Asn Lys Gly
                85                  90                  95
Leu Pro Ser Ser Ile Glu Lys Thr Ile Ser Lys Ala Lys Gly Gln Pro
            100                 105                 110
Arg Glu Pro Gln Val Tyr Thr Leu Pro Pro Ser Gln Glu Glu Met Thr
        115                 120                 125
Lys Asn Gln Val Ser Leu Thr Cys Leu Val Lys Gly Phe Tyr Pro Ser
    130                 135                 140
Asp Ile Ala Val Glu Trp Glu Ser Asn Gly Gln Pro Glu Asn Asn Tyr
145                 150                 155                 160
Lys Thr Thr Pro Pro Val Leu Asp Ser Asp Gly Ser Phe Phe Leu Tyr
                165                 170                 175
Ser Arg Leu Thr Val Asp Lys Ser Arg Trp Gln Glu Gly Asn Val Phe
            180                 185                 190
Ser Cys Ser Val Met His Glu Ala Leu His Asn His Tyr Thr Gln Lys
        195                 200                 205
Ser Leu Ser Leu Ser Pro Gly Lys
    210                 215

<210> 309
<211> 153
<212> PRT
<213> Artificial Sequence

<220>
<223> DeltaCH2 (g1CH3 only)

<400> 309
Gly Gln Pro Arg Glu Pro Gln Val Tyr Thr Leu Pro Pro Ser Arg Asp
1               5                   10                  15
Glu Leu Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Val Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Pro Gly Lys Thr Gly Leu Asn Asp
            100                 105                 110
Ile Phe Glu Ala Gln Lys Ile Glu Trp His Glu Asp Tyr Lys Asp Asp
        115                 120                 125
Asp Asp Lys Asp Tyr Lys Asp Asp Asp Lys Asp Tyr Lys Asp Asp
    130                 135                 140

Asp Asp Lys His His His His His His
145                 150

<210> 310
<211> 246
<212> PRT
<213> Artificial Sequence

<220>
<223> BC3-VH-VL amino acid sequence (includes g4s
      linker)

<400> 310
Gln Val Gln Leu Gln Gln Ser Ala Ala Glu Leu Ala Arg Pro Gly Ala
1               5                   10                  15
Ser Val Lys Met Ser Cys Lys Ala Ser Gly Tyr Thr Phe Thr Arg Tyr
            20                  25                  30
Thr Met Gln Trp Val Lys Gln Arg Pro Gly Gln Gly Leu Glu Trp Ile
        35                  40                  45
Gly Tyr Ile Asn Pro Ser Ser Gly Tyr Ile Gly Tyr Ser Gln Lys Phe
    50                  55                  60
Lys Asp Lys Thr Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Ser Lys Val Tyr Tyr Asp Tyr Asp Val Tyr Ser Met Asp Tyr
            100                 105                 110
Trp Gly Gln Gly Thr Ser Val Thr Val Ser Ser Gly Gly Gly Gly Ser
        115                 120                 125
Gly Gly Gly Gly Ser Gly Gly Gly Gly Ser Ala Gln Gln Ile Ile Leu
    130                 135                 140
Thr Gln Ser Pro Ala Ile Met Ser Ala Ser Pro Gly Glu Lys Val Thr
145                 150                 155                 160
Met Thr Cys Ser Ala Ser Ser Ser Val Ser Tyr Met His Trp Tyr Gln
                165                 170                 175
Gln Lys Ser Gly Thr Ser Pro Lys Arg Trp Ile Tyr Asp Thr Ser Lys
            180                 185                 190
Leu Ala Ser Gly Val Pro Ala Arg Phe Ser Gly Ser Gly Ser Gly Thr
        195                 200                 205
Ser Tyr Ser Leu Thr Ile Ser Ser Met Glu Ala Glu Asp Ala Ala Thr
    210                 215                 220
Tyr Tyr Cys Gln Gln Trp Ser Ser Asn Pro Leu Thr Phe Gly Ala Gly
225                 230                 235                 240
Thr Lys Leu Glu Leu Lys
                245

<210> 311
<211> 242
<212> PRT
<213> Artificial Sequence

<220>
<223> OKT3-VH-VL amino acid sequence (includes g4s
      linker)

<400> 311
Gln Val Gln Leu Gln Gln Ser Gly Ala Glu Leu Ala Arg Pro Gly Ala
1               5                   10                  15
Ser Val Lys Met Ser Cys Lys Ala Ser Gly Tyr Thr Phe Thr Arg Tyr
            20                  25                  30
Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Val Thr Val Ser Ser Gly Gly Gly Gly Ser Gly Gly Gly Gly
        115                 120                 125
Ser Gly Gly Gly Gly Ser Ala Gln Gln Ile Val Leu Thr Gln Ser Pro
    130                 135                 140
Ala Ile Met Ser Ala Ser Pro Gly Glu Lys Val Thr Met Thr Cys Ser
145                 150                 155                 160
Ala Ser Ser Ser Val Ser Tyr Met Asn Trp Tyr Gln Gln Lys Ser Gly
                165                 170                 175
Thr Ser Pro Lys Arg Trp Ile Tyr Asp Thr Ser Lys Leu Ala Ser Gly
            180                 185                 190
Val Pro Ala His Phe Arg Gly Ser Gly Ser Gly Thr Ser Tyr Ser Leu
        195                 200                 205
Thr Ile Ser Gly Met Glu Ala Glu Asp Ala Ala Thr Tyr Tyr Cys Gln
    210                 215                 220
Gln Trp Ser Ser Asn Pro Phe Thr Phe Gly Ser Gly Thr Lys Leu Glu
225                 230                 235                 240
Ile Asn


<210> 312
<211> 244
<212> PRT
<213> Artificial Sequence

<220>
<223> H57 VH-Linker-VL (without leader)

<400> 312
Glu Val Tyr Leu Val Glu Ser Gly Gly Asp Leu Val Gln Pro Gly Ser
1               5                   10                  15
Ser Leu Lys Val Ser Cys Ala Ala Ser Gly Phe Thr Phe Ser Asp Phe
            20                  25                  30
Trp Met Tyr Trp Val Arg Gln Ala Pro Gly Lys Gly Leu Glu Trp Val
        35                  40                  45
Gly Arg Ile Lys Asn Lys Pro Asn Asn Tyr Ala Thr Glu Tyr Ala Asp
    50                  55                  60
Ser Val Arg Gly Arg Phe Thr Ile Ser Arg Asp Asp Ser Arg Asn Ser
65                  70                  75                  80
Ile Tyr Leu Gln Met Asn Arg Leu Arg Val Asp Asp Thr Ala Ile Tyr
                85                  90                  95
Tyr Cys Thr Arg Ala Gly Arg Phe Asp His Phe Asp Tyr Trp Gly Gln
            100                 105                 110
Gly Thr Met Val Thr Val Ser Ser Gly Gly Gly Gly Ser Gly Gly Gly
        115                 120                 125
Gly Ser Gly Gly Gly Gly Ser Ala Gln Tyr Glu Leu Ile Gln Pro Ser
    130                 135                 140
Ser Ala Ser Val Thr Val Gly Glu Thr Val Lys Ile Thr Cys Ser Gly
145                 150                 155                 160
Asp Gln Leu Pro Lys Asn Phe Ala Tyr Trp Phe Gln Gln Lys Ser Asp
                165                 170                 175
Lys Asn Ile Leu Leu Leu Ile Tyr Met Asp Asn Lys Arg Pro Ser Gly
            180                 185                 190
Ile Pro Glu Arg Phe Ser Gly Ser Thr Ser Gly Thr Thr Ala Thr Leu
        195                 200                 205
Thr Ile Ser Gly Ala Gln Pro Glu Asp Glu Ala Ala Tyr Tyr Cys Leu

210                 215                 220
Ser Ser Tyr Gly Asp Asn Asn Asp Leu Val Phe Gly Ser Gly Thr Gln
225                 230                 235                 240
Leu Thr Val Leu

<210> 313
<211> 120
<212> PRT
<213> Artificial Sequence

<220>
<223> H57 Null2 SMIP - VH amino acid

<400> 313
Glu Val Tyr Leu Val Glu Ser Gly Gly Asp Leu Val Gln Pro Gly Ser
1               5                   10                  15
Ser Leu Lys Val Ser Cys Ala Ala Ser Gly Phe Thr Phe Ser Asp Phe
            20                  25                  30
Trp Met Tyr Trp Val Arg Gln Ala Pro Gly Lys Gly Leu Glu Trp Val
        35                  40                  45
Gly Arg Ile Lys Asn Lys Pro Asn Asn Tyr Ala Thr Glu Tyr Ala Asp
    50                  55                  60
Ser Val Arg Gly Arg Phe Thr Ile Ser Arg Asp Asp Ser Arg Asn Ser
65                  70                  75                  80
Ile Tyr Leu Gln Met Asn Arg Leu Arg Val Asp Asp Thr Ala Ile Tyr
                85                  90                  95
Tyr Cys Thr Arg Ala Gly Arg Phe Asp His Phe Asp Tyr Trp Gly Gln
            100                 105                 110
Gly Thr Met Val Thr Val Ser Ser
        115                 120

<210> 314
<211> 110
<212> PRT
<213> Artificial Sequence

<220>
<223> Mouse IGHG2c mutated CH2 (alanine substitutions at
      positions L234, L235, G237, E318, K320 and K322)

<400> 314
Ala Pro Asp Ala Ala Gly Ala Pro Ser Val Phe Ile Phe Pro Pro Lys
1               5                   10                  15
Ile Lys Asp Val Leu Met Ile Ser Leu Ser Pro Met Val Thr Cys Val
            20                  25                  30
Val Val Asp Val Ser Glu Asp Asp Pro Asp Val Gln Ile Ser Trp Phe
        35                  40                  45
Val Asn Asn Val Glu Val His Thr Ala Gln Thr Gln Thr His Arg Glu
    50                  55                  60
Asp Tyr Asn Ser Thr Leu Arg Val Val Ser Ala Leu Pro Ile Gln His
65                  70                  75                  80
Gln Asp Trp Met Ser Gly Lys Ala Phe Ala Cys Ala Val Asn Asn Arg
                85                  90                  95
Ala Leu Pro Ser Pro Ile Glu Lys Thr Ile Ser Lys Pro Arg
            100                 105                 110

<210> 315
<211> 107
<212> PRT

<213> Artificial Sequence

<220>
<223> H57 Null2 SMIP - VL amino acid sequence

<400> 315
Tyr Glu Leu Ile Gln Pro Ser Ser Ala Ser Val Thr Val Gly Glu Thr
1               5                   10                  15
Val Lys Ile Thr Cys Ser Gly Asp Gln Leu Pro Lys Asn Phe Ala Tyr
            20                  25                  30
Trp Phe Gln Gln Lys Ser Asp Lys Asn Ile Leu Leu Leu Ile Tyr Met
        35                  40                  45
Asp Asn Lys Arg Pro Ser Gly Ile Pro Glu Arg Phe Ser Gly Ser Thr
    50                  55                  60
Ser Gly Thr Thr Ala Thr Leu Thr Ile Ser Gly Ala Gln Pro Glu Asp
65                  70                  75                  80
Glu Ala Ala Tyr Tyr Cys Leu Ser Ser Tyr Gly Asp Asn Asn Asp Leu
                85                  90                  95
Val Phe Gly Ser Gly Thr Gln Leu Thr Val Leu
            100                 105

<210> 316
<400> 316
000

<210> 317
<211> 116
<212> PRT
<213> Artificial Sequence

<220>
<223> 2C11 Null2 SMIP - VH amino acid

<400> 317
Glu Val Gln Leu Val Glu Ser Gly Gly Gly Leu Val Gln Pro Gly Lys
1               5                   10                  15
Ser Leu Lys Leu Ser Cys Glu Ala Ser Gly Phe Thr Phe Ser Gly Tyr
            20                  25                  30
Gly Met His Trp Val Arg Gln Ala Pro Gly Arg Gly Leu Glu Ser Val
        35                  40                  45
Ala Tyr Ile Thr Ser Ser Ser Ile Asn Ile Lys Tyr Ala Asp Ala Val
    50                  55                  60
Lys Gly Arg Phe Thr Val Ser Arg Asp Asn Ala Lys Asn Leu Leu Phe
65                  70                  75                  80
Leu Gln Met Asn Ile Leu Lys Ser Glu Asp Thr Ala Met Tyr Tyr Cys
                85                  90                  95
Ala Arg Phe Asp Trp Asp Lys Asn Tyr Trp Gly Gln Gly Thr Met Val
            100                 105                 110
Thr Val Ser Ser
        115

<210> 318
<211> 107
<212> PRT
<213> Artificial Sequence

<220>
<223> 2C11 Null2 SMIP - VL amino acid

<400> 318
Asp Ile Gln Met Thr Gln Ser Pro Ser Ser Leu Pro Ala Ser Leu Gly
1               5                   10                  15
Asp Arg Val Thr Ile Asn Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Tyr Thr Asn Lys Leu Ala Asp Gly Val Pro Ser Arg Phe Ser Gly
    50                  55                  60
Ser Gly Ser Gly Arg Asp Ser Ser Phe Thr Ile Ser Ser Leu Glu Ser
65                  70                  75                  80
Glu Asp Ile Gly Ser Tyr Tyr Cys Gln Gln Tyr Tyr Asn Tyr Pro Trp
                85                  90                  95
Thr Phe Gly Pro Gly Thr Lys Leu Glu Ile Lys
            100                 105

<210> 319
<211> 240
<212> PRT
<213> Artificial Sequence

<220>
<223> 2C11 VH-Linker-VL (without leader)

<400> 319
Glu Val Gln Leu Val Glu Ser Gly Gly Gly Leu Val Gln Pro Gly Lys
1               5                   10                  15
Ser Leu Lys Leu Ser Cys Glu Ala Ser Gly Phe Thr Phe Ser Gly Tyr
            20                  25                  30
Gly Met His Trp Val Arg Gln Ala Pro Gly Arg Gly Leu Glu Ser Val
        35                  40                  45
Ala Tyr Ile Thr Ser Ser Ser Ile Asn Ile Lys Tyr Ala Asp Ala Val
    50                  55                  60
Lys Gly Arg Phe Thr Val Ser Arg Asp Asn Ala Lys Asn Leu Leu Phe
65                  70                  75                  80
Leu Gln Met Asn Ile Leu Lys Ser Glu Asp Thr Ala Met Tyr Tyr Cys
                85                  90                  95
Ala Arg Phe Asp Trp Asp Lys Asn Tyr Trp Gly Gln Gly Thr Met Val
            100                 105                 110
Thr Val Ser Ser Gly Gly Gly Gly Ser Gly Gly Gly Gly Ser Gly Gly
        115                 120                 125
Gly Gly Ser Ala Gln Asp Ile Gln Met Thr Gln Ser Pro Ser Ser Leu
    130                 135                 140
Pro Ala Ser Leu Gly Asp Arg Val Thr Ile Asn Cys Gln Ala Ser Gln
145                 150                 155                 160
Asp Ile Ser Asn Tyr Leu Asn Trp Tyr Gln Gln Lys Pro Gly Lys Ala
                165                 170                 175
Pro Lys Leu Leu Ile Tyr Tyr Thr Asn Lys Leu Ala Asp Gly Val Pro
            180                 185                 190
Ser Arg Phe Ser Gly Ser Gly Ser Gly Arg Asp Ser Ser Phe Thr Ile
        195                 200                 205
Ser Ser Leu Glu Ser Glu Asp Ile Gly Ser Tyr Tyr Cys Gln Gln Tyr
    210                 215                 220
Tyr Asn Tyr Pro Trp Thr Phe Gly Pro Gly Thr Lys Leu Glu Ile Lys
225                 230                 235                 240

<210> 320
<211> 110

<212> PRT
<213> Mus musculus

<220>
<223> Human IGHG2c CH2 domain

<400> 320
Ala Pro Asp Leu Leu Gly Gly Pro Ser Val Phe Ile Phe Pro Pro Lys
1               5                   10                  15
Ile Lys Asp Val Leu Met Ile Ser Leu Ser Pro Met Val Thr Cys Val
            20                  25                  30
Val Val Asp Val Ser Glu Asp Asp Pro Asp Val Gln Ile Ser Trp Phe
        35                  40                  45
Val Asn Asn Val Glu Val His Thr Ala Gln Thr Gln Thr His Arg Glu
    50                  55                  60
Asp Tyr Asn Ser Thr Leu Arg Val Val Ser Ala Leu Pro Ile Gln His
65                  70                  75                  80
Gln Asp Trp Met Ser Gly Lys Glu Phe Lys Cys Lys Val Asn Asn Arg
                85                  90                  95
Ala Leu Pro Ser Pro Ile Glu Lys Thr Ile Ser Lys Pro Arg
            100                 105                 110

<210> 321
<211> 213
<212> PRT
<213> Artificial Sequence

<220>
<223> HM1 (IgM CH3:IgG1 CH3) without C-terminal tail

<400> 321
Asp Gln Asp Thr Ala Ile Arg Val Phe Ala Ile Pro Pro Ser Phe Ala
1               5                   10                  15
Ser Ile Phe Leu Thr Lys Ser Thr Lys Leu Thr Cys Leu Val Thr Asp
            20                  25                  30
Leu Thr Thr Tyr Asp Ser Val Thr Ile Ser Trp Thr Arg Gln Asn Gly
        35                  40                  45
Glu Ala Val Lys Thr His Thr Asn Ile Ser Glu Ser His Pro Asn Ala
    50                  55                  60
Thr Phe Ser Ala Val Gly Glu Ala Ser Ile Cys Glu Asp Asp Trp Asn
65                  70                  75                  80
Ser Gly Glu Arg Phe Thr Cys Thr Val Thr His Thr Asp Leu Pro Ser
                85                  90                  95
Pro Leu Lys Gln Thr Ile Ser Arg Pro Lys Gly Gln Pro Arg Glu Pro
            100                 105                 110
Gln Val Tyr Thr Leu Pro Pro Ser Arg Asp Glu Leu Thr Lys Asn Gln
        115                 120                 125
Val Ser Leu Thr Cys Leu Val Lys Gly Phe Tyr Pro Ser Asp Ile Ala
    130                 135                 140
Val Glu Trp Glu Ser Asn Gly Gln Pro Glu Asn Asn Tyr Lys Thr Thr
145                 150                 155                 160
Pro Pro Val Leu Asp Ser Asp Gly Ser Phe Phe Leu Tyr Ser Lys Leu
                165                 170                 175
Thr Val Asp Lys Ser Arg Trp Gln Gln Gly Asn Val Phe Ser Cys Ser
            180                 185                 190
Val Met His Glu Ala Leu His Asn His Tyr Thr Gln Lys Ser Leu Ser
        195                 200                 205
Leu Ser Pro Gly Lys
    210

<210> 322

<211> 109
<212> PRT
<213> Artificial Sequence

<220>
<223> IgG4 AA CH2

<400> 322
Ala Pro Glu Ala Ala Ala Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser Gln Glu Asp Pro Glu Val Gln Phe Asn Trp Tyr Val
        35                  40                  45
Asp Gly Val Glu Val His Asn Ala Lys Thr Lys Pro Arg Glu Glu Gln
    50                  55                  60
Phe Ala Ser Thr Tyr Arg Val Val Ser Val Leu Thr Val Leu His Gln
65                  70                  75                  80
Asp Trp Leu Asn Gly Lys Glu Tyr Lys Cys Lys Val Ser Asn Lys Gly
                85                  90                  95
Leu Pro Ser Ser Ile Glu Lys Thr Ile Ser Lys Ala Lys
            100                 105

<210> 323
<211> 217
<212> PRT
<213> Artificial Sequence

<220>
<223> Human IgG1 CH2 CH3 Null2

<400> 323
Ala Pro Glu Ala Ala Gly Ala Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Ala Tyr Ala Cys Ala Val Ser Asn Lys
                85                  90                  95
Ala Leu Pro Ala Pro Ile Glu Lys Thr Ile Ser Lys Ala Lys Gly Gln
            100                 105                 110
Pro Arg Glu Pro Gln Val Tyr Thr Leu Pro Pro Ser Arg Asp Glu Leu
        115                 120                 125
Thr Lys Asn Gln Val Ser Leu Thr Cys Leu Val Lys Gly Phe Tyr Pro
    130                 135                 140
Ser Asp Ile Ala Val Glu Trp Glu Ser Asn Gly Gln Pro Glu Asn Asn
145                 150                 155                 160
Tyr Lys Thr Thr Pro Pro Val Leu Asp Ser Asp Gly Ser Phe Phe Leu
                165                 170                 175
Tyr Ser Lys Leu Thr Val Asp Lys Ser Arg Trp Gln Gln Gly Asn Val
            180                 185                 190
Phe Ser Cys Ser Val Met His Glu Ala Leu His Asn His Tyr Thr Gln
        195                 200                 205
Lys Ser Leu Ser Leu Ser Pro Gly Lys
    210                 215

<210> 324
<211> 110
<212> PRT
<213> Artificial Sequence

<220>
<223> IgG1 N297A CH2

<400> 324
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Ala Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Ala Lys
            100                 105                 110


<210> 325
<211> 109
<212> PRT
<213> Artificial Sequence

<220>
<223> IgG1 AA CH2

<400> 325
Ala Pro Glu Ala Ala Ala Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser His Glu Asp Pro Glu Val Lys Phe Asn Trp Tyr Val
        35                  40                  45
Asp Gly Val Glu Val His Asn Ala Lys Thr Lys Pro Arg Glu Glu Gln
    50                  55                  60
Tyr Ala Ser Thr Tyr Arg Val Val Ser Val Leu Thr Val Leu His Gln
65                  70                  75                  80
Asp Trp Leu Asn Gly Lys Glu Tyr Lys Cys Lys Val Ser Asn Lys Ala
                85                  90                  95
Leu Pro Ala Pro Ile Glu Lys Thr Ile Ser Lys Ala Lys
            100                 105


<210> 326
<211> 108
<212> PRT
<213> Artificial Sequence

<220>
<223> IgG2 AA CH2

<400> 326
Ala Pro Glu Ala Ala Ala Pro Ser Val Phe Leu Phe Pro Pro Lys Pro
1               5                   10                  15
Lys Asp Thr Leu Met Ile Ser Arg Thr Pro Glu Val Thr Cys Val Val

                20                  25                  30
Val Asp Val Ser His Glu Asp Pro Glu Val Gln Phe Asn Trp Tyr Val
        35                  40                  45
Asp Gly Met Glu Val His Asn Ala Lys Thr Lys Pro Arg Glu Glu Gln
    50                  55                  60
Phe Ala Ser Thr Phe Arg Val Val Ser Val Leu Thr Val Val His Gln
65                  70                  75                  80
Asp Trp Leu Asn Gly Lys Glu Tyr Lys Cys Lys Val Ser Asn Lys Gly
                85                  90                  95
Leu Pro Ala Pro Ile Glu Lys Thr Ile Ser Lys Thr
            100                 105

<210> 327
<211> 121
<212> PRT
<213> Artificial Sequence

<220>
<223> Cris7-VH murine hybridoma Amino Acid

<220>
<221> ACT_SITE
<222> (31)...(35)
<223> complementarity-determining region

<220>
<221> ACT_SITE
<222> (50)...(66)
<223> complementarity-determining region

<220>
<221> ACT_SITE
<222> (100)...(110)
<223> complementarity-determining region

<400> 327
Gln Val Gln Leu Gln Gln Ser Gly Ala Glu Leu Ala Arg Pro Gly Ala
 1               5                  10                  15
Ser Val Lys Met Ser Cys Lys Ala Ser Gly Tyr Thr Phe Thr Arg Ser
            20                  25                  30
Thr Met His Trp Val Lys Gln Arg Pro Gly Gln Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Ser Pro Gln Val His Tyr Asp Tyr Asn Gly Phe Pro Tyr Trp Gly
            100                 105                 110
Gln Gly Thr Leu Val Thr Val Ser Ala
        115                 120

<210> 328
<211> 107
<212> PRT
<213> Artificial Sequence

<220>
<223> Cris7-VL murine hybridoma Amino Acid

<220>
<221> ACT_SITE
<222> (24)...(33)
<223> complementarity-determining region

<220>
<221> ACT_SITE
<222> (49)...(55)
<223> complementarity-determining region

<220>
<221> ACT_SITE
<222> (88)...(96)
<223> complementarity-determining region

<400> 328
Gln Val Val Leu Thr Gln Ser Pro Ala Ile Met Ser Ala Phe Pro Gly
1               5                   10                  15
Glu Lys Val Thr Met Thr Cys Ser Ala Ser Ser Ser Val Ser Tyr Met
            20                  25                  30
Asn Trp Tyr Gln Gln Lys Ser Gly Thr Ser Pro Lys Arg Trp Ile Tyr
        35                  40                  45
Asp Ser Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Thr Glu
65                  70                  75                  80
Asp Ala Ala Thr Tyr Tyr Cys Gln Gln Trp Ser Arg Asn Pro Pro Thr
                85                  90                  95
Phe Gly Gly Gly Thr Lys Leu Gln Ile Thr Arg
            100                 105

<210> 329
<211> 107
<212> PRT
<213> Artificial Sequence

<220>
<223> HuCris7 VL.1

<220>
<221> ACT_SITE
<222> (24)...(33)
<223> complementarity-determining region

<220>
<221> ACT_SITE
<222> (49)...(55)
<223> complementarity-determining region

<220>
<221> ACT_SITE
<222> (88)...(96)
<223> complementarity-determining region

<400> 329
Asp Ile Gln Met Thr Gln Ser Pro Ser Ser Leu Ser Ala Ser Val Gly
1               5                   10                  15
Asp Arg Val Thr Met Thr Cys Ser Ala Ser Ser Ser Val Ser Tyr Met
            20                  25                  30
Asn Trp Tyr Gln Gln Lys Pro Gly Lys Ala Pro Lys Arg Trp Ile Tyr
        35                  40                  45

Asp Ser Ser Lys Leu Ala Ser Gly Val Pro Ala Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Trp Ser Arg Asn Pro Pro Thr
                85                  90                  95
Phe Gly Gly Gly Thr Lys Leu Gln Ile Thr Arg
            100                 105

<210> 330
<211> 107
<212> PRT
<213> Artificial Sequence

<220>
<223> HuCris7 VL.2

<400> 330
Asp Ile Gln Met Thr Gln Ser Pro Ser Ser Leu Ser Ala Ser Val Gly
 1               5                  10                  15
Asp Arg Val Thr Ile Thr Cys Ser Ala Ser Ser Ser Val Ser Tyr Met
            20                  25                  30
Asn Trp Tyr Gln Gln Thr Pro Gly Lys Ala Pro Lys Arg Trp Ile Tyr
        35                  40                  45
Asp Ser Ser Lys Leu Ala Ser Gly Val Pro Ser Arg Phe Ser Gly Ser
    50                  55                  60
Gly Ser Gly Thr Asp Phe Thr Leu Thr Ile Ser Ser Leu Gln Pro Glu
65                  70                  75                  80
Asp Ile Ala Thr Tyr Tyr Cys Gln Gln Trp Ser Arg Asn Pro Pro Thr
                85                  90                  95
Phe Gly Gln Gly Thr Lys Leu Gln Ile Thr Arg
            100                 105

<210> 331
<211> 121
<212> PRT
<213> Artificial Sequence

<220>
<223> HuCris7 VH.1

<220>
<221> ACT_SITE
<222> (31)...(35)
<223> complementarity-determining region

<220>
<221> ACT_SITE
<222> (50)...(66)
<223> complementarity-determining region

<220>
<221> ACT_SITE
<222> (100)...(110)
<223> complementarity-determining region

<400> 331
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser
        115                 120

<210> 332
<211> 121
<212> PRT
<213> Artificial Sequence

<220>
<223> HuCris7 VH.2

<400> 332
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Lys Asn Thr Ala Tyr
65                  70                  75                  80
Met Glu Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser
        115                 120

<210> 333
<211> 121
<212> PRT
<213> Artificial Sequence

<220>
<223> HuCRIS7 VH.3

<400> 333
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly

100                 105                 110
Gln Gly Thr Pro Val Thr Val Ser Ser
        115                 120

<210> 334
<211> 245
<212> PRT
<213> Artificial Sequence

<220>
<223> Cris7-(VH-VL)

<220>
<221> SITE
<222> (122)...(138)
<223> Linker

<400> 334
Gln Val Gln Leu Gln Gln Ser Gly Ala Glu Leu Ala Arg Pro Gly Ala
 1               5                  10                  15
Ser Val Lys Met Ser Cys Lys Ala Ser Gly Tyr Thr Phe Thr Arg Ser
            20                  25                  30
Thr Met His Trp Val Lys Gln Arg Pro Gly Gln Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Ser Pro Gln Val His Tyr Asp Tyr Asn Gly Phe Pro Tyr Trp Gly
            100                 105                 110
Gln Gly Thr Leu Val Thr Val Ser Ala Gly Gly Gly Gly Ser Gly Gly
        115                 120                 125
Gly Gly Ser Gly Gly Gly Gly Ser Ala Gln Gln Val Val Leu Thr Gln
    130                 135                 140
Ser Pro Ala Ile Met Ser Ala Phe Pro Gly Glu Lys Val Thr Met Thr
145                 150                 155                 160
Cys Ser Ala Ser Ser Ser Val Ser Tyr Met Asn Trp Tyr Gln Gln Lys
                165                 170                 175
Ser Gly Thr Ser Pro Lys Arg Trp Ile Tyr Asp Ser Ser Lys Leu Ala
            180                 185                 190
Ser Gly Val Pro Ala Arg Phe Ser Gly Ser Gly Ser Gly Thr Ser Tyr
        195                 200                 205
Ser Leu Thr Ile Ser Ser Met Glu Thr Glu Asp Ala Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg
                245

<210> 335
<211> 245
<212> PRT
<213> Artificial Sequence

<220>
<223> HuCris7-(VH1-VL1)

<220>

<221> SITE
<222> (122)...(138)
<223> Linker

<400> 335
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Arg Ser
            20                  25                  30
Thr Met His Trp Val Lys Gln Ala Pro Gly Gln Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg
                245


<210> 336
<211> 245
<212> PRT
<213> Artificial Sequence

<220>
<223> HuCris7-(VH1-VL2)

<220>
<221> SITE
<222> (122)...(138)
<223> Linker

<400> 336
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Arg Ser
            20                  25                  30
Thr Met His Trp Val Lys Gln Ala Pro Gly Gln Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Ser Ser Thr Ala Tyr
65                  70                  75                  80

Met Gln Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Ile Thr
145                 150                 155                 160
Cys Ser Ala Ser Ser Ser Val Ser Tyr Met Asn Trp Tyr Gln Gln Thr
                165                 170                 175
Pro Gly Lys Ala Pro Lys Arg Trp Ile Tyr Asp Ser Ser Lys Leu Ala
            180                 185                 190
Ser Gly Val Pro Ser Arg Phe Ser Gly Ser Gly Ser Gly Thr Asp Phe
        195                 200                 205
Thr Leu Thr Ile Ser Ser Leu Gln Pro Glu Asp Ile Ala Thr Tyr Tyr
    210                 215                 220
Cys Gln Gln Trp Ser Arg Asn Pro Pro Thr Phe Gly Gln Gly Thr Lys
225                 230                 235                 240
Leu Gln Ile Thr Arg
                245

<210> 337
<211> 245
<212> PRT
<213> Artificial Sequence

<220>
<223> HuCris7-(VH2-VL1)

<220>
<221> SITE
<222> (122)...(138)
<223> Linker

<400> 337
Gln Val Gln Leu Val Gln Ser Gly Gly Gly Val Val Gln Pro Gly Arg
 1               5                  10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Lys Asn Thr Ala Tyr
65                  70                  75                  80
Met Glu Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr

195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg
                245

<210> 338
<211> 245
<212> PRT
<213> Artificial Sequence

<220>
<223> HuCris7-(VH2-VL2)

<220>
<221> SITE
<222> (122)...(138)
<223> Linker

<400> 338
Gln Val Gln Leu Val Gln Ser Gly Gly Gly Val Val Gln Pro Gly Arg
 1               5                  10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Lys Ala Thr Leu Thr Ala Asp Lys Ser Lys Asn Thr Ala Tyr
65                  70                  75                  80
Met Glu Leu Ser Ser Leu Arg Ser Glu Asp Thr Ala Val Tyr Tyr Cys
                85                  90                  95
Ala Arg Pro Gln Val His Tyr Asp Tyr Asn Gly Phe Pro Tyr Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Ile Thr
145                 150                 155                 160
Cys Ser Ala Ser Ser Ser Val Ser Tyr Met Asn Trp Tyr Gln Gln Thr
                165                 170                 175
Pro Gly Lys Ala Pro Lys Arg Trp Ile Tyr Asp Ser Ser Lys Leu Ala
            180                 185                 190
Ser Gly Val Pro Ser Arg Phe Ser Gly Ser Gly Ser Gly Thr Asp Phe
        195                 200                 205
Thr Leu Thr Ile Ser Ser Leu Gln Pro Glu Asp Ile Ala Thr Tyr Tyr
    210                 215                 220
Cys Gln Gln Trp Ser Arg Asn Pro Pro Thr Phe Gly Gln Gly Thr Lys
225                 230                 235                 240
Leu Gln Ile Thr Arg
                245

<210> 339
<211> 245
<212> PRT
<213> Artificial Sequence

<220>

<223> HuCris7-(VH3-VL1)

<220>
<221> SITE
<222> (122)...(138)
<223> Liniker

<400> 339
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg
                245


<210> 340
<211> 245
<212> PRT
<213> Artificial Sequence

<220>
<223> HuCris7-(VH3-VL2)

<220>
<221> SITE
<222> (122)...(138)
<223> Linker

<400> 340
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe

50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Ile Thr
145                 150                 155                 160
Cys Ser Ala Ser Ser Ser Val Ser Tyr Met Asn Trp Tyr Gln Gln Thr
                165                 170                 175
Pro Gly Lys Ala Pro Lys Arg Trp Ile Tyr Asp Ser Ser Lys Leu Ala
            180                 185                 190
Ser Gly Val Pro Ser Arg Phe Ser Gly Ser Gly Ser Gly Thr Asp Phe
        195                 200                 205
Thr Leu Thr Ile Ser Ser Leu Gln Pro Glu Asp Ile Ala Thr Tyr Tyr
    210                 215                 220
Cys Gln Gln Trp Ser Arg Asn Pro Pro Thr Phe Gly Gln Gly Thr Lys
225                 230                 235                 240
Leu Gln Ile Thr Arg
                245

<210> 341
<211> 216
<212> PRT
<213> Artificial Sequence

<220>
<223> Half null CH2-CH3 regions

<400> 341
Pro Asp Ala Ala Gly Ala Pro Ser Val Phe Ile Phe Pro Pro Lys Ile
1               5                   10                  15
Lys Asp Val Leu Met Ile Ser Leu Ser Pro Met Val Thr Cys Val Val
            20                  25                  30
Val Asp Val Ser Glu Asp Asp Pro Asp Val Gln Ile Ser Trp Phe Val
        35                  40                  45
Asn Asn Val Glu Val His Thr Ala Gln Thr Gln Thr His Arg Glu Asp
    50                  55                  60
Tyr Ala Ser Thr Leu Arg Val Val Ser Ala Leu Pro Ile Gln His Gln
65                  70                  75                  80
Asp Trp Met Ser Gly Lys Glu Phe Lys Cys Lys Val Asn Asn Arg Ala
                85                  90                  95
Leu Pro Ser Pro Ile Glu Lys Thr Ile Ser Lys Pro Arg Gly Pro Val
            100                 105                 110
Arg Ala Pro Gln Val Tyr Val Leu Pro Pro Pro Ala Glu Glu Met Thr
        115                 120                 125
Lys Lys Glu Phe Ser Leu Thr Cys Met Ile Thr Gly Phe Leu Pro Ala
    130                 135                 140
Glu Ile Ala Val Asp Trp Thr Ser Asn Gly Arg Thr Glu Gln Asn Tyr
145                 150                 155                 160
Lys Asn Thr Ala Thr Val Leu Asp Ser Asp Gly Ser Tyr Phe Met Tyr
                165                 170                 175
Ser Lys Leu Arg Val Gln Lys Ser Thr Trp Glu Arg Gly Ser Leu Phe
            180                 185                 190
Ala Cys Ser Val Val His Glu Gly Leu His Asn His Leu Thr Thr Lys
        195                 200                 205

Thr Ile Ser Arg Ser Leu Gly Lys
    210                 215

<210> 342
<211> 213
<212> PRT
<213> Artificial Sequence

<220>
<223> HM2 (mouse CH3mu-CH3gamma) without C-termial tail

<400> 342
Ser Pro Ser Thr Asp Ile Leu Thr Phe Thr Ile Pro Pro Ser Phe Ala
1               5                   10                  15
Asp Ile Phe Leu Ser Lys Ser Ala Asn Leu Thr Cys Leu Val Ser Asn
            20                  25                  30
Leu Ala Thr Tyr Glu Thr Leu Asn Ile Ser Trp Ala Ser Gln Ser Gly
        35                  40                  45
Glu Pro Leu Glu Thr Lys Ile Lys Ile Met Glu Ser His Pro Asn Gly
    50                  55                  60
Thr Phe Ser Ala Lys Gly Val Ala Ser Val Cys Val Glu Asp Trp Asn
65                  70                  75                  80
Asn Arg Lys Glu Phe Val Cys Thr Val Thr His Arg Asp Leu Pro Ser
                85                  90                  95
Pro Gln Lys Lys Phe Ile Ser Lys Pro Asn Gly Pro Val Arg Ala Pro
            100                 105                 110
Gln Val Tyr Val Leu Pro Pro Pro Ala Glu Glu Met Thr Lys Lys Glu
        115                 120                 125
Phe Ser Leu Thr Cys Met Ile Thr Gly Phe Leu Pro Ala Glu Ile Ala
    130                 135                 140
Val Asp Trp Thr Ser Asn Gly Arg Thr Glu Gln Asn Tyr Lys Asn Thr
145                 150                 155                 160
Ala Thr Val Leu Asp Ser Asp Gly Ser Tyr Phe Met Tyr Ser Lys Leu
                165                 170                 175
Arg Val Gln Lys Ser Thr Trp Glu Arg Gly Ser Leu Phe Ala Cys Ser
            180                 185                 190
Val Val His Glu Gly Leu His Asn His Leu Thr Thr Lys Thr Ile Ser
        195                 200                 205
Arg Ser Leu Gly Lys
    210

<210> 343
<211> 110
<212> PRT
<213> Artificial Sequence

<220>
<223> huamn IgG4 AA CH2 with alanine substitutions at
      F234 and N297

<400> 343
Ala Pro Glu Ala Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
        35                  40                  45
Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110


<210> 344
<211> 110
<212> PRT
<213> Artificial Sequence

<220>
<223> human IgG4 AA CH2 with alanine substitutions at
      L235 and N297

<400> 344
Ala Pro Glu Phe Ala Gly Gly Pro Ser Val Phe Leu Phe Pro Pro Lys
 1               5                  10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
        35                  40                  45
Val Asp Gly Val Glu Val His Asn Ala Lys Thr Lys Pro Arg Glu Glu
    50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110


<210> 345
<211> 110
<212> PRT
<213> Artificial Sequence

<220>
<223> human IgG4 AA CH2 with alanine substitutions at
      G236 and N297

<400> 345
Ala Pro Glu Phe Leu Ala Gly Pro Ser Val Phe Leu Phe Pro Pro Lys
 1               5                  10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
        35                  40                  45
Val Asp Gly Val Glu Val His Asn Ala Lys Thr Lys Pro Arg Glu Glu
    50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110


<210> 346
<211> 110
<212> PRT

<213> Artificial Sequence

<220>
<223> human IgG4 AA CH2 with alanine substitutions at
      G237 and N297

<400> 346
Ala Pro Glu Phe Leu Gly Ala Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
        35                  40                  45
Val Asp Gly Val Glu Val His Asn Ala Lys Thr Lys Pro Arg Glu Glu
    50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110


<210> 347
<400> 347
000

<210> 348
<400> 348
000

<210> 349
<400> 349
000

<210> 350
<400> 350
000

<210> 351
<400> 351
000

<210> 352
<400> 352
000

<210> 353
<400> 353
000

<210> 354
<400> 354
000

<210> 355
<400> 355
000

<210> 356
<400> 356
000

<210> 357
<400> 357
 000

<210> 358
<400> 358
 000

<210> 359
<400> 359
 000

<210> 360
<400> 360
 000

<210> 361
<400> 361
 000

<210> 362
<400> 362
 000

<210> 363
<400> 363
 000

<210> 364
<400> 364
 000

<210> 365
<400> 365
 000

<210> 366
<400> 366
 000

<210> 367
<400> 367
 000

<210> 368
<400> 368
 000

<210> 369
<400> 369
 000

<210> 370
<400> 370
 000

<210> 371
<400> 371
 000

<210> 372
<400> 372
 000

<210> 373
<400> 373
 000

<210> 374
<400> 374
 000

<210> 375
<400> 375
 000

<210> 376
<400> 376
 000

<210> 377
<400> 377
 000

<210> 378
<400> 378
 000

<210> 379
<211> 15
<212> PRT
<213> Artificial Sequence

<220>
<223> Linker 134

<400> 379
Glu Pro Met Ser Cys Asp Lys Thr His Thr Cys Pro Pro Cys Pro
1               5                   10                  15

<210> 380
<211> 15
<212> PRT
<213> Artificial Sequence

<220>
<223> Linker 135

<400> 380
Glu Pro Met Ser Ser Asp Lys Thr His Thr Cys Pro Pro Cys Pro
1               5                   10                  15

<210> 381
<211> 15
<212> PRT
<213> Artificial Sequence

<220>
<223> Linker 136

<400> 381
Glu Pro Met Ser Cys Asp Lys Thr His Thr Ser Pro Pro Cys Pro
1               5                   10                  15

<210> 382
<211> 15
<212> PRT
<213> Artificial Sequence

<220>
<223> Linker 137

<400> 382
Glu Pro Met Ser Cys Asp Lys Thr His Thr Cys Pro Pro Ser Pro
1               5                   10                  15


<210> 383
<211> 15
<212> PRT
<213> Artificial Sequence

<220>
<223> Linker 138

<400> 383
Glu Pro Met Ser Ser Asp Lys Thr His Thr Ser Pro Pro Cys Pro
1               5                   10                  15


<210> 384
<211> 15
<212> PRT
<213> Artificial Sequence

<220>
<223> Linker 139

<400> 384
Glu Pro Met Ser Ser Asp Lys Thr His Thr Cys Pro Pro Ser Pro
1               5                   10                  15


<210> 385
<211> 15
<212> PRT
<213> Artificial Sequence

<220>
<223> Linker 140

<400> 385
Glu Pro Met Ser Cys Asp Lys Thr His Thr Ser Pro Pro Ser Pro
1               5                   10                  15


<210> 386
<211> 15
<212> PRT
<213> Artificial Sequence

<220>
<223> Linker 141

<400> 386

Glu Pro Met Ser Cys Asp Lys Thr His Thr Cys Pro Pro Cys Ser
1               5                   10                  15

<210> 387
<211> 15
<212> PRT
<213> Artificial Sequence

<220>
<223> Linker 142

<400> 387
Glu Pro Met Ser Ser Asp Lys Thr His Thr Cys Pro Pro Cys Ser
1               5                   10                  15

<210> 388
<211> 15
<212> PRT
<213> Artificial Sequence

<220>
<223> Linker 143

<400> 388
Glu Pro Met Ser Cys Asp Lys Thr His Thr Ser Pro Pro Cys Ser
1               5                   10                  15

<210> 389
<211> 15
<212> PRT
<213> Artificial Sequence

<220>
<223> Linker 144

<400> 389
Glu Pro Met Ser Cys Asp Lys Thr His Thr Cys Pro Pro Ser Ser
1               5                   10                  15

<210> 390
<211> 15
<212> PRT
<213> Artificial Sequence

<220>
<223> Linker 145

<400> 390
Glu Pro Met Ser Ser Asp Lys Thr His Thr Ser Pro Pro Cys Ser
1               5                   10                  15

<210> 391
<211> 15
<212> PRT
<213> Artificial Sequence

<220>
<223> Linker 146

<400> 391
Glu Pro Met Ser Ser Asp Lys Thr His Thr Cys Pro Pro Ser Pro
1               5                   10                  15

<210> 392
<211> 15
<212> PRT
<213> Artificial Sequence

<220>
<223> Linker 147

<400> 392
Glu Pro Met Ser Cys Asp Lys Thr His Thr Ser Pro Pro Ser Ser
1               5                   10                  15

<210> 393
<211> 15
<212> PRT
<213> Artificial Sequence

<220>
<223> Linker 148

<400> 393
Glu Pro Thr Ser Cys Asp Lys Thr His Thr Cys Pro Pro Cys Pro
1               5                   10                  15

<210> 394
<211> 15
<212> PRT
<213> Artificial Sequence

<220>
<223> Linker 149

<400> 394
Glu Pro Thr Ser Ser Asp Lys Thr His Thr Cys Pro Pro Cys Pro
1               5                   10                  15

<210> 395
<211> 15
<212> PRT
<213> Artificial Sequence

<220>
<223> Linker 150

<400> 395
Glu Pro Thr Ser Cys Asp Lys Thr His Thr Ser Pro Pro Cys Pro
1               5                   10                  15

<210> 396
<211> 15
<212> PRT
<213> Artificial Sequence

<220>
<223> Linker 151

<400> 396
Glu Pro Thr Ser Cys Asp Lys Thr His Thr Cys Pro Pro Ser Pro
1               5                   10                  15


<210> 397
<211> 15
<212> PRT
<213> Artificial Sequence

<220>
<223> Linker 152

<400> 397
Glu Pro Thr Ser Ser Asp Lys Thr His Thr Ser Pro Pro Cys Pro
1               5                   10                  15


<210> 398
<211> 15
<212> PRT
<213> Artificial Sequence

<220>
<223> Linker 153

<400> 398
Glu Pro Thr Ser Ser Asp Lys Thr His Thr Cys Pro Pro Ser Pro
1               5                   10                  15


<210> 399
<211> 15
<212> PRT
<213> Artificial Sequence

<220>
<223> Linker 154

<400> 399
Glu Pro Thr Ser Cys Asp Lys Thr His Thr Ser Pro Pro Ser Pro
1               5                   10                  15


<210> 400
<211> 15
<212> PRT
<213> Artificial Sequence

<220>
<223> Linker 155

<400> 400
Glu Pro Thr Ser Cys Asp Lys Thr His Thr Cys Pro Pro Cys Ser
1               5                   10                  15


<210> 401
<211> 15
<212> PRT

<213> Artificial Sequence

<220>
<223> Linker 156

<400> 401
Glu Pro Thr Ser Ser Asp Lys Thr His Thr Cys Pro Pro Cys Ser
1               5                   10                  15


<210> 402
<211> 15
<212> PRT
<213> Artificial Sequence

<220>
<223> Linker 157

<400> 402
Glu Pro Thr Ser Cys Asp Lys Thr His Thr Ser Pro Pro Cys Ser
1               5                   10                  15


<210> 403
<211> 15
<212> PRT
<213> Artificial Sequence

<220>
<223> Linker 158

<400> 403
Glu Pro Thr Ser Cys Asp Lys Thr His Thr Cys Pro Pro Ser Ser
1               5                   10                  15


<210> 404
<211> 15
<212> PRT
<213> Artificial Sequence

<220>
<223> Linker 159

<400> 404
Glu Pro Thr Ser Ser Asp Lys Thr His Thr Ser Pro Pro Cys Ser
1               5                   10                  15


<210> 405
<211> 15
<212> PRT
<213> Artificial Sequence

<220>
<223> Linker 160

<400> 405
Glu Pro Thr Ser Ser Asp Lys Thr His Thr Cys Pro Pro Ser Ser
1               5                   10                  15


<210> 406

<211> 15
<212> PRT
<213> Artificial Sequence

<220>
<223> Linker 161

<400> 406
Glu Pro Thr Ser Cys Asp Lys Thr His Thr Ser Pro Pro Ser Ser
1               5                   10                  15


<210> 407
<211> 15
<212> PRT
<213> Artificial Sequence

<220>
<223> Linker 162

<400> 407
Glu Pro Ala Ser Cys Asp Lys Thr His Thr Cys Pro Pro Cys Pro
1               5                   10                  15


<210> 408
<211> 15
<212> PRT
<213> Artificial Sequence

<220>
<223> Linker 163

<400> 408
Glu Pro Ala Ser Ser Asp Lys Thr His Thr Cys Pro Pro Cys Pro
1               5                   10                  15


<210> 409
<211> 15
<212> PRT
<213> Artificial Sequence

<220>
<223> Linker 164

<400> 409
Glu Pro Ala Ser Cys Asp Lys Thr His Thr Ser Pro Pro Cys Pro
1               5                   10                  15


<210> 410
<211> 15
<212> PRT
<213> Artificial Sequence

<220>
<223> Linker 165

<400> 410
Glu Pro Ala Ser Cys Asp Lys Thr His Thr Cys Pro Pro Ser Pro
1               5                   10                  15

<210> 411
<211> 15
<212> PRT
<213> Artificial Sequence

<220>
<223> Linker 166

<400> 411
Glu Pro Ala Ser Ser Asp Lys Thr His Thr Ser Pro Pro Cys Pro
1               5                   10                  15


<210> 412
<211> 15
<212> PRT
<213> Artificial Sequence

<220>
<223> Linker 167

<400> 412
Glu Pro Ala Ser Ser Asp Lys Thr His Thr Cys Pro Pro Ser Pro
1               5                   10                  15


<210> 413
<211> 15
<212> PRT
<213> Artificial Sequence

<220>
<223> Linker 168

<400> 413
Glu Pro Ala Ser Cys Asp Lys Thr His Thr Ser Pro Pro Ser Pro
1               5                   10                  15


<210> 414
<211> 15
<212> PRT
<213> Artificial Sequence

<220>
<223> Linker 169

<400> 414
Glu Pro Ala Ser Cys Asp Lys Thr His Thr Cys Pro Pro Cys Ser
1               5                   10                  15


<210> 415
<211> 15
<212> PRT
<213> Artificial Sequence

<220>
<223> Linker 170

<400> 415
Glu Pro Ala Ser Ser Asp Lys Thr His Thr Cys Pro Pro Cys Ser

1               5                   10                  15


<210> 416
<211> 15
<212> PRT
<213> Artificial Sequence

<220>
<223> Linker 171

<400> 416
Glu Pro Ala Ser Cys Asp Lys Thr His Thr Ser Pro Pro Cys Ser
1               5                   10                  15


<210> 417
<211> 15
<212> PRT
<213> Artificial Sequence

<220>
<223> Linker 172

<400> 417
Glu Pro Ala Ser Cys Asp Lys Thr His Thr Cys Pro Pro Ser Ser
1               5                   10                  15


<210> 418
<211> 15
<212> PRT
<213> Artificial Sequence

<220>
<223> Linker 173

<400> 418
Glu Pro Ala Ser Ser Asp Lys Thr His Thr Ser Pro Pro Cys Ser
1               5                   10                  15


<210> 419
<211> 15
<212> PRT
<213> Artificial Sequence

<220>
<223> Linker 174

<400> 419
Glu Pro Ala Ser Ser Asp Lys Thr His Thr Cys Pro Pro Ser Ser
1               5                   10                  15


<210> 420
<211> 15
<212> PRT
<213> Artificial Sequence

<220>
<223> Linker 175

<400> 420
Glu Pro Ala Ser Cys Asp Lys Thr His Thr Ser Pro Pro Ser Ser
1               5                   10                  15


<210> 421
<211> 14
<212> PRT
<213> Artificial Sequence

<220>
<223> Linker 176

<400> 421
Glu Pro Ser Cys Asp Lys Thr His Thr Cys Pro Pro Cys Pro
1               5                   10


<210> 422
<211> 14
<212> PRT
<213> Artificial Sequence

<220>
<223> Linker 177

<400> 422
Glu Pro Ser Ser Asp Lys Thr His Thr Cys Pro Pro Cys Pro
1               5                   10


<210> 423
<211> 14
<212> PRT
<213> Artificial Sequence

<220>
<223> Linker 178

<400> 423
Glu Pro Ser Cys Asp Lys Thr His Thr Ser Pro Pro Cys Pro
1               5                   10


<210> 424
<211> 14
<212> PRT
<213> Artificial Sequence

<220>
<223> Linker 179

<400> 424
Glu Pro Ser Cys Asp Lys Thr His Thr Cys Pro Pro Ser Pro
1               5                   10


<210> 425
<211> 14
<212> PRT
<213> Artificial Sequence

<220>

<223> Linker 180

<400> 425
Glu Pro Ser Ser Asp Lys Thr His Thr Ser Pro Pro Cys Pro
1               5                   10


<210> 426
<211> 14
<212> PRT
<213> Artificial Sequence

<220>
<223> Linker 181

<400> 426
Glu Pro Ser Ser Asp Lys Thr His Thr Cys Pro Pro Ser Pro
1               5                   10


<210> 427
<211> 14
<212> PRT
<213> Artificial Sequence

<220>
<223> Linker 182

<400> 427
Glu Pro Ser Cys Asp Lys Thr His Thr Ser Pro Pro Ser Pro
1               5                   10


<210> 428
<211> 14
<212> PRT
<213> Artificial Sequence

<220>
<223> Linker 183

<400> 428
Glu Pro Ser Cys Asp Lys Thr His Thr Cys Pro Pro Cys Ser
1               5                   10


<210> 429
<211> 14
<212> PRT
<213> Artificial Sequence

<220>
<223> Linker 184

<400> 429
Glu Pro Ser Ser Asp Lys Thr His Thr Cys Pro Pro Cys Ser
1               5                   10


<210> 430
<211> 14
<212> PRT
<213> Artificial Sequence

<220>
<223> Linker 185

<400> 430
Glu Pro Ser Cys Asp Lys Thr His Thr Ser Pro Pro Cys Ser
1               5                   10


<210> 431
<211> 14
<212> PRT
<213> Artificial Sequence

<220>
<223> Linker 186

<400> 431
Glu Pro Ser Cys Asp Lys Thr His Thr Cys Pro Pro Ser Ser
1               5                   10


<210> 432
<211> 14
<212> PRT
<213> Artificial Sequence

<220>
<223> Linker 187

<400> 432
Glu Pro Ser Ser Asp Lys Thr His Thr Ser Pro Pro Cys Ser
1               5                   10


<210> 433
<211> 14
<212> PRT
<213> Artificial Sequence

<220>
<223> Linker 188

<400> 433
Glu Pro Ser Ser Asp Lys Thr His Thr Cys Pro Pro Ser Ser
1               5                   10


<210> 434
<211> 14
<212> PRT
<213> Artificial Sequence

<220>
<223> Linker 189

<400> 434
Glu Pro Ser Cys Asp Lys Thr His Thr Ser Pro Pro Ser Ser
1               5                   10

<210> 435
<400> 435
000

<210> 436
<400> 436
000

<210> 437
<400> 437
000

<210> 438
<400> 438
000

<210> 439
<400> 439
000

<210> 440
<400> 440
000

<210> 441
<400> 441
000

<210> 442
<400> 442
000

<210> 443
<400> 443
000

<210> 444
<400> 444
000

<210> 445
<400> 445
000

<210> 446
<400> 446
000

<210> 447
<400> 447
000

<210> 448
<400> 448
000

<210> 449
<400> 449
 000

<210> 450
<400> 450
 000

<210> 451
<400> 451
 000

<210> 452
<400> 452
 000

<210> 453
<400> 453
 000

<210> 454
<400> 454
 000

<210> 455
<400> 455
 000

<210> 456
<400> 456
 000

<210> 457
<400> 457
 000

<210> 458
<400> 458
 000

<210> 459
<400> 459
 000

<210> 460
<400> 460
 000

<210> 461
<400> 461
 000

<210> 462
<400> 462
 000

<210> 463
<400> 463
 000


<210> 464
<211> 5
<212> PRT
<213> Artificial Sequence

<220>
<223> Anti-c-Met Heavy Chain Complementarity Determining
      Region

<400> 464
Lys Tyr Asp Met Leu
 1               5


<210> 465
<211> 17
<212> PRT
<213> Artificial Sequence

<220>
<223> Anti-c-Met Heavy Chain Complementarity Determining
      Region

<400> 465
Tyr Ile Tyr Pro Ser Gly Gly Leu Thr Glu Tyr Ala Asp Ser Val Lys
 1               5                   10                  15
Gly


<210> 466
<211> 10
<212> PRT
<213> Artificial Sequence

<220>
<223> Anti-c-Met Heavy Chain Complementarity Determining
      Region

<400> 466
Arg Ala Pro Arg Ser Leu Ser Phe Asp Ile
 1               5                   10

<210> 467
<400> 467
 000


<210> 468
<400> 468
 000


<210> 469
<400> 469
 000

<210> 470
<400> 470
 000

<210> 471
<400> 471
 000

<210> 472
<400> 472
 000

<210> 473
<400> 473
 000

<210> 474
<400> 474
 000

<210> 475
<400> 475
 000

<210> 476
<400> 476
 000

<210> 477
<400> 477
 000

<210> 478
<400> 478
 000

<210> 479
<400> 479
 000

<210> 480
<400> 480
 000

<210> 481
<400> 481
 000

<210> 482
<400> 482
 000

<210> 483

<400> 483
 000

<210> 484
<400> 484
 000

<210> 485
<400> 485
 000

<210> 486
<400> 486
 000

<210> 487
<400> 487
 000

<210> 488
<400> 488
 000

<210> 489
<400> 489
 000

<210> 490
<400> 490
 000

<210> 491
<400> 491
 000

<210> 492
<400> 492
 000

<210> 493
<400> 493
 000

<210> 494
<400> 494
 000

<210> 495
<400> 495
 000

<210> 496
<400> 496
 000

<210> 497
<400> 497
 000

<210> 498
<400> 498
 000

<210> 499
<400> 499
 000

<210> 500
<400> 500
 000

<210> 501
<400> 501
 000

<210> 502
<400> 502
 000

<210> 503
<400> 503
 000

<210> 504
<400> 504
 000

<210> 505
<400> 505
 000

<210> 506
<400> 506
 000

<210> 507
<400> 507
 000

<210> 508
<400> 508
 000

<210> 509
<400> 509
 000

<210> 510
<400> 510

000

<210> 511
<400> 511
000

<210> 512
<400> 512
000

<210> 513
<400> 513
000

<210> 514
<400> 514
000

<210> 515
<400> 515
000

<210> 516
<400> 516
000

<210> 517
<400> 517
000

<210> 518
<400> 518
000

<210> 519
<400> 519
000

<210> 520
<400> 520
000

<210> 521
<400> 521
000

<210> 522
<400> 522
000

<210> 523
<400> 523
000

<210> 524

<400> 524
 000

<210> 525
<400> 525
 000

<210> 526
<400> 526
 000

<210> 527
<400> 527
 000

<210> 528
<400> 528
 000

<210> 529
<400> 529
 000

<210> 530
<400> 530
 000

<210> 531
<400> 531
 000

<210> 532
<400> 532
 000

<210> 533
<400> 533
 000

<210> 534
<400> 534
 000

<210> 535
<400> 535
 000

<210> 536
<400> 536
 000

<210> 537
<400> 537
 000

<210> 538
<400> 538
 000

<210> 539
<400> 539
 000

<210> 540
<400> 540
 000

<210> 541
<400> 541
 000

<210> 542
<400> 542
 000

<210> 543
<400> 543
 000

<210> 544
<400> 544
 000

<210> 545
<400> 545
 000

<210> 546
<400> 546
 000

<210> 547
<400> 547
 000

<210> 548
<400> 548
 000

<210> 549
<400> 549
 000

<210> 550
<400> 550
 000

<210> 551

<400> 551
 000

<210> 552
<400> 552
 000

<210> 553
<400> 553
 000

<210> 554
<400> 554
 000

<210> 555
<400> 555
 000

<210> 556
<400> 556
 000

<210> 557
<400> 557
 000

<210> 558
<400> 558
 000

<210> 559
<400> 559
 000

<210> 560
<400> 560
 000

<210> 561
<400> 561
 000

<210> 562
<400> 562
 000

<210> 563
<400> 563
 000

<210> 564
<400> 564
 000

<210> 565
<400> 565
 000

<210> 566
<400> 566
 000

<210> 567
<400> 567
 000

<210> 568
<400> 568
 000

<210> 569
<400> 569
 000

<210> 570
<400> 570
 000

<210> 571
<400> 571
 000

<210> 572
<400> 572
 000

<210> 573
<400> 573
 000

<210> 574
<400> 574
 000

<210> 575
<400> 575
 000

<210> 576
<400> 576
 000

<210> 577
<400> 577
 000

<210> 578
<400> 578

000

<210> 579
<400> 579
000

<210> 580
<400> 580
000

<210> 581
<400> 581
000

<210> 582
<400> 582
000

<210> 583
<400> 583
000

<210> 584
<400> 584
000

<210> 585
<400> 585
000

<210> 586
<400> 586
000

<210> 587
<400> 587
000

<210> 588
<400> 588
000

<210> 589
<400> 589
000

<210> 590
<400> 590
000

<210> 591
<400> 591
000

<210> 592
<400> 592
 000

<210> 593
<400> 593
 000

<210> 594
<400> 594
 000

<210> 595
<211> 110
<212> PRT
<213> Artificial Sequence

<220>
<223> Human IgG1 CH2 with L235A, E318A, K320A and K322A
      substitutions

<400> 595
Ala Pro Glu Leu Ala Gly Gly Pro Ser Val Phe Leu Phe Pro Pro Lys
 1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Ala Tyr Ala Cys Ala Val Ser Asn Lys
                85                  90                  95
Ala Leu Pro Ala Pro Ile Glu Lys Thr Ile Ser Lys Ala Lys
            100                 105                 110

<210> 596
<211> 110
<212> PRT
<213> Artificial Sequence

<220>
<223> Human IgG1 CH2 with L234A, L235A, G237A, E318A,
      K320A and K322A

<400> 596
Ala Pro Glu Ala Ala Gly Ala Pro Ser Val Phe Leu Phe Pro Pro Lys
 1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Ala Tyr Ala Cys Ala Val Ser Asn Lys
                85                  90                  95
Ala Leu Pro Ala Pro Ile Glu Lys Thr Ile Ser Lys Ala Lys

            100                 105                 110

<210> 597
<400> 597
000

<210> 598
<211> 3
<212> PRT
<213> Artificial Sequence

<220>
<223> Junction amino acids

<400> 598
Arg Asp Gln
1

<210> 599
<211> 4
<212> PRT
<213> Artificial Sequence

<220>
<223> Junction amino acids

<400> 599
Ser Ala Ser Ser
1

<210> 600
<211> 3
<212> PRT
<213> Artificial Sequence

<220>
<223> Junction amino acids

<400> 600
Ser Ser Ser
1

<210> 601
<211> 17
<212> PRT
<213> Artificial Sequence

<220>
<223> NKG2D Linker

<400> 601
Asn Ser Leu Phe Asn Gln Glu Val Gln Ile Pro Leu Thr Glu Ser Tyr
1               5                   10                  15
Ser

<210> 602

<211> 107
<212> PRT
<213> Artificial Sequence

<220>
<223> Anti-RON mAb 4C04 light chain

<400> 602
Asp Ile Gln Met Thr Gln Ser Pro Ala Ser Gln Ser Ala Ser Leu Gly
1               5                   10                  15
Glu Ser Val Thr Ile Thr Cys Leu Ala Ser Gln Thr Ile Gly Thr Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Ala
    50                  55                  60
Ser Gly Ser Gly Thr Lys Phe Ser Phe Lys Ile Asp Ser Leu Gln Ser
65                  70                  75                  80
Glu Asp Phe Val Ser Tyr Tyr Cys Gln Gln Leu Tyr Asn Thr Pro Trp
                85                  90                  95
Thr Phe Gly Gly Gly Thr Lys Leu Glu Ile Lys
            100                 105


<210> 603
<211> 121
<212> PRT
<213> Artificial Sequence

<220>
<223> Anti-RON mAb 4C04 heavy chain

<400> 603
Gln Ile Gln Leu Gln Gln Ser Gly Pro Glu Leu Val Lys Pro Gly Thr
1               5                   10                  15
Ser Val Lys Ile Ser Cys Lys Ala Ser Asp Tyr Thr Leu Thr Asp Phe
            20                  25                  30
Tyr Met Asn Trp Val Arg Gln Lys Pro Gly Gln Gly Leu Glu Trp Ile
        35                  40                  45
Gly Arg Ile Tyr Pro Gly Thr Asp Lys Thr Arg Tyr Asn Glu Lys Phe
    50                  55                  60
Arg Asp Lys Ala Ile Leu Thr Val Asp Thr Ser Ser Ser Thr Ala Tyr
65                  70                  75                  80
Met Gln Leu Ser Ser Leu Thr Ser Glu Asp Thr Ala Val Tyr Phe Cys
                85                  90                  95
Ala Arg Ser Ala Tyr Tyr Gly Asn Tyr Val Ala Met Asp Tyr Trp Gly
            100                 105                 110
Gln Gly Thr Ser Val Thr Val Ser Ser
        115                 120


<210> 604
<211> 6
<212> PRT
<213> Artificial Sequence

<220>
<223> Anti-RON mAb 4C04 light chain CDR1

<400> 604
Gln Thr Ile Gly Thr Trp
1               5

<210> 605
<211> 3
<212> PRT
<213> Artificial Sequence

<220>
<223> Anti-RON mAb 4C04 light chain CDR2

<400> 605
Ala Ala Thr
1

<210> 606
<211> 9
<212> PRT
<213> Artificial Sequence

<220>
<223> Anti-RON mAb 4C04 light chain CDR3

<400> 606
Gln Gln Leu Tyr Asn Thr Pro Trp Thr
1               5

<210> 607
<211> 8
<212> PRT
<213> Artificial Sequence

<220>
<223> Anti-RON mAb 4C04 heavy chain CDR1

<400> 607
Asp Tyr Thr Leu Thr Asp Phe Tyr
1               5

<210> 608
<211> 8
<212> PRT
<213> Artificial Sequence

<220>
<223> Anti-RON mAb 4C04 heavy chain CDR2

<400> 608
Ile Tyr Pro Gly Thr Asp Lys Thr
1               5

<210> 609
<211> 14
<212> PRT
<213> Artificial Sequence

<220>
<223> Anti-RON mAb 4C04 heavy chain CDR3

<400> 609
Ala Arg Ser Ala Tyr Tyr Gly Asn Tyr Val Ala Met Asp Tyr

1               5                   10


<210> 610
<211> 107
<212> PRT
<213> Artificial Sequence

<220>
<223> Anti-RON mAb 11H09 light chain

<400> 610
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Ser Ser
            20                  25                  30
Leu Ser Trp Leu Gln Gln Glu Pro Asp Gly Thr Ile Lys Arg Leu Ile
        35                  40                  45
Tyr Ser Thr Ser Ser Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Ser Ser Pro Trp
                85                  90                  95
Thr Phe Gly Gly Gly Thr Lys Leu Glu Ile Lys
            100                 105


<210> 611
<211> 117
<212> PRT
<213> Artificial Sequence

<220>
<223> Anti-RON mAb 11H09 heavy chain

<400> 611
Gln Val Gln Leu Gln Gln Ser Gly Ala Glu Leu Ala Lys Pro Gly Ala
1               5                   10                  15
Ser Val Lys Met Ser Cys Lys Ser Ser Gly Tyr Thr Phe Thr Ser Tyr
            20                  25                  30
Trp Ile His Trp Val Lys Glu Arg Pro Gly Gln Gly Leu Glu Trp Ile
        35                  40                  45
Gly Tyr Ile Tyr Pro Thr Thr Gly Tyr Thr Glu Ser Asn Gln Lys Phe
    50                  55                  60
Lys Asp Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Phe Leu Leu Arg Pro Trp Tyr Phe Asp Val Trp Gly Ala Gly Thr Thr
            100                 105                 110
Val Thr Val Ser Ser
        115


<210> 612
<211> 6
<212> PRT
<213> Artificial Sequence

<220>
<223> Anti-RON mAb 11H09 light chain CDR1

<400> 612
Gln Asp Ile Gly Ser Ser
1               5

<210> 613
<211> 3
<212> PRT
<213> Artificial Sequence

<220>
<223> Anti-RON mAb 11H09 light chain CDR2

<400> 613
Ser Thr Ser
1

<210> 614
<211> 9
<212> PRT
<213> Artificial Sequence

<220>
<223> Anti-RON mAb 11H09 light chain CDR3

<400> 614
Leu Gln Tyr Ala Ser Ser Pro Trp Thr
1               5

<210> 615
<211> 8
<212> PRT
<213> Artificial Sequence

<220>
<223> Anti-RON mAb 11H09 heavy chain CDR1

<400> 615
Gly Tyr Thr Phe Thr Ser Tyr Trp
1               5

<210> 616
<211> 8
<212> PRT
<213> Artificial Sequence

<220>
<223> Anti-RON mAb 11H09 heavy chain CDR2

<400> 616
Ile Tyr Pro Thr Thr Gly Tyr Thr
1               5

<210> 617
<211> 10
<212> PRT
<213> Artificial Sequence

<220>

<223> Anti-RON mAb 11H09 heavy chain CDR3

<400> 617
Phe Leu Leu Arg Pro Trp Tyr Phe Asp Val
1               5                   10

<210> 618
<211> 2
<212> PRT
<213> Artificial Sequence

<220>
<223> Made in a lab

<400> 618
Asn Ser
1

<210> 619
<211> 6
<212> PRT
<213> Artificial Sequence

<220>
<223> Made in a lab

<400> 619
Ser Cys Pro Pro Cys Pro
1               5

<210> 620
<211> 8
<212> PRT
<213> Artificial Sequence

<220>
<223> Made in a lab

<400> 620
Gly Gly Gly Gly Ser Gly Asn Ser
1               5

<210> 621
<211> 8
<212> PRT
<213> Artificial Sequence

<220>
<223> Made in a lab

<400> 621
Gly Cys Pro Pro Cys Pro Asn Ser
1               5

<210> 622
<211> 8
<212> PRT
<213> Artificial Sequence

<220>
<223> Made in a lab

<400> 622

Gly Ser Pro Pro Ser Pro Asn Ser
1               5

<210> 623
<211> 8
<212> PRT
<213> Artificial Sequence

<220>
<223> Made in a lab

<400> 623
Gly Ser Pro Pro Ser Pro Asn Ser
1               5

<210> 624
<211> 8
<212> PRT
<213> Artificial Sequence

<220>
<223> Made in a lab

<400> 624
Gly Cys Pro Pro Cys Pro Asn Ser
1               5

<210> 625
<211> 8
<212> PRT
<213> Artificial Sequence

<220>
<223> Made in a lab

<400> 625
Gly Cys Pro Pro Cys Pro Asn Ser
1               5

<210> 626
<211> 9
<212> PRT
<213> Artificial Sequence

<220>
<223> Made in a lab

<400> 626
Gly Cys Pro Pro Cys Pro Gly Asn Ser
1               5

<210> 627
<211> 9
<212> PRT
<213> Artificial Sequence

<220>
<223> Made in a lab

<400> 627
Gly Cys Pro Pro Cys Pro Ala Asn Ser
1               5

<210> 628
<211> 9
<212> PRT
<213> Artificial Sequence

<220>
<223> Made in a lab

<400> 628
Gly Cys Pro Pro Cys Pro Ala Asn Ser
1               5

<210> 629
<211> 9
<212> PRT
<213> Artificial Sequence

<220>
<223> Made in a lab

<400> 629
Glu Glu Glu Glu Asp Glu Gly Asn Ser
1               5

<210> 630
<211> 10
<212> PRT
<213> Artificial Sequence

<220>
<223> Made in a lab

<400> 630
Asn Tyr Gly Gly Gly Gly Ser Gly Asn Ser
1               5                   10

<210> 631
<211> 10
<212> PRT
<213> Artificial Sequence

<220>
<223> Made in a lab

<400> 631
Val Ser Glu Arg Pro Phe Pro Pro Asn Ser
1               5                   10

<210> 632
<211> 11
<212> PRT
<213> Artificial Sequence

<220>
<223> Made in a lab

<400> 632
Glu Pro Lys Ser Cys Asp Lys Thr Cys Cys Pro
1               5                   10

<210> 633
<211> 11
<212> PRT

<213> Artificial Sequence

<220>
<223> Made in a lab

<400> 633
Ser Gln Pro Glu Ile Val Pro Ile Ser Asn Ser
1               5                   10

<210> 634
<211> 12
<212> PRT
<213> Artificial Sequence

<220>
<223> Made in a lab

<400> 634
Gly Gly Gly Gly Ser Cys Pro Pro Cys Pro Asn Ser
1               5                   10

<210> 635
<211> 12
<212> PRT
<213> Artificial Sequence

<220>
<223> Made in a lab

<400> 635
Lys Ala Asp Phe Leu Thr Pro Ser Ile Gly Asn Ser
1               5                   10

<210> 636
<211> 12
<212> PRT
<213> Artificial Sequence

<220>
<223> Made in a lab

<400> 636
Gln Met Asn Ser Glu Leu Ser Val Leu Ala Asn Ser
1               5                   10

<210> 637
<211> 13
<212> PRT
<213> Artificial Sequence

<220>
<223> Made in a lab

<400> 637
Glu Pro Lys Ser Cys Asp Lys Thr His Thr Cys Cys Pro
1               5                   10

<210> 638
<211> 13
<212> PRT
<213> Artificial Sequence

<220>

<223> Made in a lab

<400> 638
Glu Pro Lys Ser Cys Asp Lys Thr Cys Pro Pro Cys Pro
1               5                   10

<210> 639
<211> 13
<212> PRT
<213> Artificial Sequence

<220>
<223> Made in a lab

<400> 639
Gly Gly Gly Gly Ser Gly Gly Gly Gly Ser Gly Asn Ser
1               5                   10

<210> 640
<211> 13
<212> PRT
<213> Artificial Sequence

<220>
<223> Made in a lab

<400> 640
Gly Cys Pro Pro Cys Pro Gly Gly Gly Gly Ser Asn Ser
1               5                   10

<210> 641
<211> 13
<212> PRT
<213> Artificial Sequence

<220>
<223> Made in a lab

<400> 641
Gly Gly Gly Gly Ser Cys Pro Pro Cys Pro Gly Asn Ser
1               5                   10

<210> 642
<211> 13
<212> PRT
<213> Artificial Sequence

<220>
<223> Made in a lab

<400> 642
Gly Cys Pro Pro Cys Pro Gly Gly Gly Gly Ser Asn Ser
1               5                   10

<210> 643
<211> 13
<212> PRT
<213> Artificial Sequence

<220>
<223> Made in a lab

<400> 643

Gly Gly Gly Ala Ser Cys Pro Pro Cys Pro Gly Asn Ser
1               5                   10

<210> 644
<211> 13
<212> PRT
<213> Artificial Sequence

<220>
<223> Made in a lab

<400> 644
Gly Gly Gly Ala Ser Cys Pro Pro Cys Ala Gly Asn Ser
1               5                   10

<210> 645
<211> 13
<212> PRT
<213> Artificial Sequence

<220>
<223> Made in a lab

<400> 645
Gly Gly Gly Ala Ser Cys Pro Pro Cys Ala Gly Asn Ser
1               5                   10

<210> 646
<211> 15
<212> PRT
<213> Artificial Sequence

<220>
<223> Made in a lab

<400> 646
Asn Tyr Gly Gly Gly Gly Ser Gly Gly Gly Gly Ser Gly Asn Ser
1               5                   10                  15

<210> 647
<211> 15
<212> PRT
<213> Artificial Sequence

<220>
<223> Made in a lab

<400> 647
Leu Ser Val Lys Ala Asp Phe Leu Thr Pro Ser Ile Gly Asn Ser
1               5                   10                  15

<210> 648
<211> 15
<212> PRT
<213> Artificial Sequence

<220>
<223> Made in a lab

<400> 648
Leu Ser Val Leu Ala Asn Phe Ser Gln Pro Glu Ile Gly Asn Ser
1               5                   10                  15

<210> 649
<211> 15
<212> PRT
<213> Artificial Sequence

<220>
<223> Made in a lab

<400> 649
Leu Lys Ile Gln Glu Arg Val Ser Lys Pro Lys Ile Ser Asn Ser
1               5                   10                  15

<210> 650
<211> 15
<212> PRT
<213> Artificial Sequence

<220>
<223> Made in a lab

<400> 650
Leu Asp Val Ser Glu Arg Pro Phe Pro Pro His Ile Gln Asn Ser
1               5                   10                  15

<210> 651
<211> 15
<212> PRT
<213> Artificial Sequence

<220>
<223> Made in a lab

<400> 651
Arg Glu Gln Leu Ala Glu Val Thr Leu Ser Leu Lys Ala Asn Ser
1               5                   10                  15

<210> 652
<211> 15
<212> PRT
<213> Artificial Sequence

<220>
<223> Made in a lab

<400> 652
Arg Ile His Gln Met Asn Ser Glu Leu Ser Val Leu Ala Asn Ser
1               5                   10                  15

<210> 653
<211> 15
<212> PRT
<213> Artificial Sequence

<220>
<223> Made in a lab

<400> 653
Asp Thr Lys Gly Lys Asn Val Leu Glu Lys Ile Phe Ser Asn Ser
1               5                   10                  15

<210> 654
<211> 15
<212> PRT

<213> Artificial Sequence

<220>
<223> Made in a lab

<400> 654
Leu Pro Pro Glu Thr Gln Glu Ser Gln Glu Val Thr Leu Asn Ser
1               5                   10                  15

<210> 655
<211> 15
<212> PRT
<213> Artificial Sequence

<220>
<223> Made in a lab

<400> 655
Arg Ile His Leu Asn Val Ser Glu Arg Pro Phe Pro Pro Asn Ser
1               5                   10                  15

<210> 656
<211> 15
<212> PRT
<213> Artificial Sequence

<220>
<223> Made in a lab

<400> 656
Leu Ser Val Lys Ala Asp Phe Leu Thr Pro Ser Ile Gly Asn Ser
1               5                   10                  15

<210> 657
<211> 15
<212> PRT
<213> Artificial Sequence

<220>
<223> Made in a lab

<400> 657
Leu Ser Val Leu Ala Asn Phe Ser Gln Pro Glu Ile Gly Asn Ser
1               5                   10                  15

<210> 658
<211> 15
<212> PRT
<213> Artificial Sequence

<220>
<223> Made in a lab

<400> 658
Leu Ser Val Leu Ala Asn Phe Ser Gln Pro Glu Ile Gly Asn Ser
1               5                   10                  15

<210> 659
<211> 15
<212> PRT
<213> Artificial Sequence

<220>

<223> Made in a lab

<400> 659
Arg Ile His Gln Met Asn Ser Glu Leu Ser Val Leu Ala Asn Ser
1               5                   10                  15

<210> 660
<211> 15
<212> PRT
<213> Artificial Sequence

<220>
<223> Made in a lab

<400> 660
Lys Pro Phe Phe Thr Cys Gly Ser Ala Asp Thr Cys Pro Asn Ser
1               5                   10                  15

<210> 661
<211> 15
<212> PRT
<213> Artificial Sequence

<220>
<223> Made in a lab

<400> 661
Lys Pro Phe Phe Thr Cys Gly Ser Ala Asp Thr Cys Pro Asn Ser
1               5                   10                  15

<210> 662
<211> 15
<212> PRT
<213> Artificial Sequence

<220>
<223> Made in a lab

<400> 662
Gln Tyr Asn Cys Pro Gly Gln Tyr Thr Phe Ser Met Pro Asn Ser
1               5                   10                  15

<210> 663
<211> 15
<212> PRT
<213> Artificial Sequence

<220>
<223> Made in a lab

<400> 663
Gly Gly Gly Gly Ser Gly Gly Gly Gly Ser Gly Gly Gly Gly Ser
1               5                   10                  15

<210> 664
<211> 15
<212> PRT
<213> Artificial Sequence

<220>
<223> Made in a lab

<400> 664

Glu Pro Lys Ser Ser Asp Lys Thr His Thr Cys Pro Pro Cys Pro
1               5                   10                  15

<210> 665
<211> 15
<212> PRT
<213> Artificial Sequence

<220>
<223> Made in a lab

<400> 665
Glu Pro Lys Ser Ser Asp Lys Thr His Thr Cys Pro Pro Cys Ser
1               5                   10                  15

<210> 666
<211> 15
<212> PRT
<213> Artificial Sequence

<220>
<223> Made in a lab

<400> 666
Glu Pro Lys Ser Cys Asp Lys Thr His Thr Ser Pro Pro Cys Ser
1               5                   10                  15

<210> 667
<211> 15
<212> PRT
<213> Artificial Sequence

<220>
<223> Made in a lab

<400> 667
Glu Pro Lys Ser Cys Asp Lys Thr His Thr Cys Pro Pro Cys Pro
1               5                   10                  15

<210> 668
<211> 15
<212> PRT
<213> Artificial Sequence

<220>
<223> Made in a lab

<400> 668
Glu Pro Lys Ser Cys Asp Lys Thr His Thr Ser Pro Pro Ser Ser
1               5                   10                  15

<210> 669
<211> 15
<212> PRT
<213> Artificial Sequence

<220>
<223> Made in a lab

<400> 669
Pro Arg Gly Pro Thr Ile Lys Pro Cys Pro Pro Cys Lys Cys Pro
1               5                   10                  15

<210> 670
<211> 15
<212> PRT
<213> Artificial Sequence

<220>
<223> Made in a lab

<400> 670
Glu Pro Lys Ser Ser Asp Lys Thr His Thr Ser Pro Pro Ser Ser
1               5                   10                  15

<210> 671
<211> 15
<212> PRT
<213> Artificial Sequence

<220>
<223> Made in a lab

<400> 671
Glu Pro Lys Ser Ser Asp Lys Thr His Thr Ser Pro Pro Cys Ser
1               5                   10                  15

<210> 672
<211> 15
<212> PRT
<213> Artificial Sequence

<220>
<223> Made in a lab

<400> 672
Glu Pro Lys Ser Ser Asp Lys Thr His Thr Cys Pro Pro Ser Ser
1               5                   10                  15

<210> 673
<211> 15
<212> PRT
<213> Artificial Sequence

<220>
<223> Made in a lab

<400> 673
Glu Pro Lys Ser Cys Asp Lys Thr His Thr Ser Pro Pro Cys Pro
1               5                   10                  15

<210> 674
<211> 15
<212> PRT
<213> Artificial Sequence

<220>
<223> Made in a lab

<400> 674
Glu Pro Lys Ser Ser Asp Lys Thr His Thr Ser Pro Pro Ser Pro
1               5                   10                  15

<210> 675
<211> 15
<212> PRT

<213> Artificial Sequence

<220>
<223> Made in a lab

<400> 675
Glu Pro Lys Ser Ser Asp Lys Thr His Thr Ser Pro Pro Cys Pro
1               5                   10                  15

<210> 676
<211> 15
<212> PRT
<213> Artificial Sequence

<220>
<223> Made in a lab

<400> 676
Glu Pro Lys Ser Cys Asp Lys Thr His Thr Ser Pro Pro Ser Pro
1               5                   10                  15

<210> 677
<211> 15
<212> PRT
<213> Artificial Sequence

<220>
<223> Made in a lab

<400> 677
Glu Pro Lys Ser Ser Asp Lys Thr His Thr Cys Pro Pro Ser Pro
1               5                   10                  15

<210> 678
<211> 15
<212> PRT
<213> Artificial Sequence

<220>
<223> Made in a lab

<400> 678
Gly Gly Gly Gly Cys Asp Lys Thr His Thr Cys Pro Pro Cys Pro
1               5                   10                  15

<210> 679
<211> 15
<212> PRT
<213> Artificial Sequence

<220>
<223> Made in a lab

<400> 679
Glu Pro Lys Ser Cys Gly Gly Gly Gly Gly Cys Pro Pro Cys Pro
1               5                   10                  15

<210> 680
<211> 15
<212> PRT
<213> Artificial Sequence

<220>

<223> Made in a lab

<400> 680
Glu Pro Lys Ser Cys Asp Lys Thr His Thr Cys Gly Gly Cys Pro
1               5                   10                  15

<210> 681
<211> 15
<212> PRT
<213> Artificial Sequence

<220>
<223> Made in a lab

<400> 681
Glu Pro Lys Ser Cys Asp Lys Thr His Thr Cys Pro Pro Cys Gly
1               5                   10                  15

<210> 682
<211> 15
<212> PRT
<213> Artificial Sequence

<220>
<223> Made in a lab

<400> 682
Glu Pro Lys Ser Cys Asp Lys Thr His Thr Cys Pro Pro Ser Pro
1               5                   10                  15

<210> 683
<211> 15
<212> PRT
<213> Artificial Sequence

<220>
<223> Made in a lab

<400> 683
Glu Pro Lys Ser Cys Asp Lys Cys His Thr Cys Pro Pro Cys Pro
1               5                   10                  15

<210> 684
<211> 15
<212> PRT
<213> Artificial Sequence

<220>
<223> Made in a lab

<400> 684
Glu Pro Lys Ser Cys Asp Lys Thr Cys Cys Cys Pro Pro Cys Pro
1               5                   10                  15

<210> 685
<211> 15
<212> PRT
<213> Artificial Sequence

<220>
<223> Made in a lab

<400> 685

Glu Pro Lys Ser Cys Pro Pro Pro Pro Pro Cys Pro Pro Cys Pro
1               5                   10                  15

<210> 686
<211> 15
<212> PRT
<213> Artificial Sequence

<220>
<223> Made in a lab

<400> 686
Pro Pro Pro Pro Cys Asp Lys Thr His Thr Cys Pro Pro Cys Pro
1               5                   10                  15

<210> 687
<211> 15
<212> PRT
<213> Artificial Sequence

<220>
<223> Made in a lab

<400> 687
Glu Pro Lys Ser Cys Asp Lys Thr His Thr Cys Trp Trp Cys Pro
1               5                   10                  15

<210> 688
<211> 15
<212> PRT
<213> Artificial Sequence

<220>
<223> Made in a lab

<400> 688
Glu Pro Lys Ser Cys Asp Trp Trp His Thr Cys Pro Pro Cys Pro
1               5                   10                  15

<210> 689
<211> 15
<212> PRT
<213> Artificial Sequence

<220>
<223> Made in a lab

<400> 689
Glu Pro Lys Cys Ser Asp Lys Thr His Thr Cys Pro Pro Cys Pro
1               5                   10                  15

<210> 690
<211> 15
<212> PRT
<213> Artificial Sequence

<220>
<223> Made in a lab

<400> 690
Glu Pro Lys Ser Asp Cys Lys Thr His Thr Cys Pro Pro Cys Pro
1               5                   10                  15

<210> 691
<211> 15
<212> PRT
<213> Artificial Sequence

<220>
<223> Made in a lab

<400> 691
Glu Pro Lys Ser Asp Cys Trp Trp His Thr Cys Pro Pro Cys Pro
1               5                   10                  15

<210> 692
<211> 15
<212> PRT
<213> Artificial Sequence

<220>
<223> Made in a lab

<400> 692
Glu Pro Lys Ser Cys Asp Phe Phe His Thr Cys Pro Pro Cys Pro
1               5                   10                  15

<210> 693
<211> 15
<212> PRT
<213> Artificial Sequence

<220>
<223> Made in a lab

<400> 693
Glu Pro Lys Ser Cys Asp Trp Trp Trp Thr Cys Pro Pro Cys Pro
1               5                   10                  15

<210> 694
<211> 15
<212> PRT
<213> Artificial Sequence

<220>
<223> Made in a lab

<400> 694
Glu Pro Lys Ser Cys Trp Trp Thr His Thr Cys Pro Pro Cys Pro
1               5                   10                  15

<210> 695
<211> 15
<212> PRT
<213> Artificial Sequence

<220>
<223> Made in a lab

<400> 695
Glu Pro Trp Trp Cys Asp Lys Thr His Thr Cys Pro Pro Cys Pro
1               5                   10                  15

<210> 696
<211> 16
<212> PRT

<213> Artificial Sequence

<220>
<223> Made in a lab

<400> 696
Ser Gln Pro Glu Ile Val Pro Ile Ser Cys Pro Pro Cys Pro Asn Ser
1               5                   10                  15

<210> 697
<211> 17
<212> PRT
<213> Artificial Sequence

<220>
<223> Made in a lab

<400> 697
Thr Gly Glu Pro Lys Ser Ser Asp Lys Thr His Thr Cys Pro Pro Cys
1               5                   10                  15
Pro

<210> 698
<211> 17
<212> PRT
<213> Artificial Sequence

<220>
<223> Made in a lab

<400> 698
Glu Pro Lys Ser Thr Asp Lys Thr His Thr Cys Pro Pro Cys Pro Asn
1               5                   10                  15
Ser

<210> 699
<211> 17
<212> PRT
<213> Artificial Sequence

<220>
<223> Made in a lab

<400> 699
Glu Pro Lys Ser Thr Asp Lys Thr His Thr Ser Pro Pro Ser Pro Asn
1               5                   10                  15
Ser

<210> 700
<211> 17
<212> PRT
<213> Artificial Sequence

<220>
<223> Made in a lab

<400> 700
Glu Pro Lys Ser Thr Asp Lys Thr His Thr Cys Pro Pro Cys Pro Asn
1               5                   10                  15
Ser

<210> 701
<211> 17
<212> PRT
<213> Artificial Sequence

<220>
<223> Made in a lab

<400> 701
Glu Pro Lys Ser Ser Asp Lys Thr His Thr Cys Pro Pro Cys Pro Asn
1               5                   10                  15
Ser


<210> 702
<211> 18
<212> PRT
<213> Artificial Sequence

<220>
<223> Made in a lab

<400> 702
Glu Pro Lys Ser Cys Asp Lys Thr His Thr Cys Pro Gly Gly Gly Pro
1               5                   10                  15
Cys Pro


<210> 703
<211> 18
<212> PRT
<213> Artificial Sequence

<220>
<223> Made in a lab

<400> 703
Glu Pro Lys Ser Cys Asp Gly Gly Gly Lys Thr His Thr Cys Pro Pro
1               5                   10                  15
Cys Pro


<210> 704
<211> 18
<212> PRT
<213> Artificial Sequence

<220>
<223> Made in a lab

<400> 704
Glu Pro Lys Ser Cys Asp Pro Pro Pro Lys Thr His Thr Cys Pro Pro
1               5                   10                  15
Cys Pro


<210> 705
<211> 18
<212> PRT
<213> Artificial Sequence

<220>
<223> Made in a lab

<400> 705
Glu Pro Lys Ser Cys Asp Lys Thr His Thr Cys Pro Pro Pro Pro Pro
1               5                   10                  15
Cys Pro


<210> 706
<211> 18
<212> PRT
<213> Artificial Sequence

<220>
<223> Made in a lab

<400> 706
Gly Gly Gly Gly Ser Gly Gly Gly Gly Ser Gly Gly Gly Gly Ser Gly
1               5                   10                  15
Asn Ser


<210> 707
<211> 19
<212> PRT
<213> Artificial Sequence

<220>
<223> Made in a lab

<400> 707
Asn Tyr Gly Gly Gly Gly Ser Gly Gly Gly Gly Ser Gly Gly Gly Gly
1               5                   10                  15
Ser Asn Ser


<210> 708
<211> 19
<212> PRT
<213> Artificial Sequence

<220>
<223> Made in a lab

<400> 708
Leu Ser Val Lys Ala Asp Phe Leu Thr Pro Ser Ile Ser Pro Pro Cys
1               5                   10                  15
Pro Asn Ser


<210> 709
<211> 19
<212> PRT
<213> Artificial Sequence

<220>
<223> Made in a lab

<400> 709
Ser Val Leu Ala Asn Phe Ser Gln Pro Glu Ile Ser Cys Pro Pro Cys
1               5                   10                  15
Pro Asn Ser

<210> 710
<211> 20
<212> PRT
<213> Artificial Sequence

<220>
<223> Made in a lab

<400> 710
Gly Gln Arg His Asn Asn Ser Ser Leu Asn Thr Arg Thr Gln Lys Ala
1               5                   10                  15
Arg His Ser Pro
            20

<210> 711
<211> 20
<212> PRT
<213> Artificial Sequence

<220>
<223> Made in a lab

<400> 711
Leu Ser Val Leu Ala Asn Phe Ser Gln Pro Glu Ile Ser Cys Pro Pro
1               5                   10                  15
Cys Pro Asn Ser
            20

<210> 712
<211> 20
<212> PRT
<213> Artificial Sequence

<220>
<223> Made in a lab

<400> 712
Leu Lys Ile Gln Glu Arg Val Ser Lys Pro Lys Ile Ser Cys Pro Pro
1               5                   10                  15
Cys Pro Asn Ser
            20

<210> 713
<211> 20
<212> PRT
<213> Artificial Sequence

<220>
<223> Made in a lab

<400> 713
Arg Glu Gln Leu Ala Glu Val Thr Leu Ser Leu Lys Ala Cys Pro Pro
1               5                   10                  15
Cys Pro Asn Ser
            20

<210> 714
<211> 20
<212> PRT
<213> Artificial Sequence

<220>
<223> Made in a lab

<400> 714
Arg Ile His Gln Met Asn Ser Glu Leu Ser Val Leu Ala Cys Pro Pro
1               5                   10                  15
Cys Pro Asn Ser
            20

<210> 715
<211> 20
<212> PRT
<213> Artificial Sequence

<220>
<223> Made in a lab

<400> 715
Arg Ile His Leu Asn Val Ser Glu Arg Pro Phe Pro Pro Cys Pro Pro
1               5                   10                  15
Cys Pro Asn Ser
            20

<210> 716
<211> 20
<212> PRT
<213> Artificial Sequence

<220>
<223> Made in a lab

<400> 716
Asn Ser Leu Phe Asn Gln Glu Val Gln Ile Pro Leu Thr Glu Ser Tyr
1               5                   10                  15
Cys Pro Asn Ser
            20

<210> 717
<211> 20
<212> PRT
<213> Artificial Sequence

<220>
<223> Made in a lab

<400> 717
Glu Glu Glu Glu Asp Glu Glu Asp Glu Glu Asp Glu Glu Glu Glu Glu
1               5                   10                  15
Asp Gly Asn Ser
            20

<210> 718
<211> 21
<212> PRT
<213> Artificial Sequence

<220>
<223> Made in a lab

<400> 718
Leu Asp Val Ser Glu Arg Pro Phe Pro Pro His Ile Gln Ser Cys Pro
1               5                   10                  15
Pro Cys Pro Asn Ser

            20

<210> 719
<211> 21
<212> PRT
<213> Artificial Sequence

<220>
<223> Made in a lab

<400> 719
Asp Thr Lys Gly Lys Asn Val Leu Glu Lys Ile Phe Asp Ser Cys Pro
1               5                   10                  15
Pro Cys Pro Asn Ser
            20

<210> 720
<211> 21
<212> PRT
<213> Artificial Sequence

<220>
<223> Made in a lab

<400> 720
Leu Pro Pro Glu Thr Gln Glu Ser Gln Glu Val Thr Leu Ser Cys Pro
1               5                   10                  15
Pro Cys Pro Asn Ser
            20

<210> 721
<211> 21
<212> PRT
<213> Artificial Sequence

<220>
<223> Made in a lab

<400> 721
Glu Pro Ala Phe Thr Pro Gly Pro Asn Ile Glu Leu Gln Lys Asp Ser
1               5                   10                  15
Asp Cys Pro Asn Ser
            20

<210> 722
<211> 21
<212> PRT
<213> Artificial Sequence

<220>
<223> Made in a lab

<400> 722
Gln Arg His Asn Asn Ser Ser Leu Asn Thr Arg Thr Gln Lys Ala Arg
1               5                   10                  15
His Cys Pro Asn Ser
            20

<210> 723
<211> 21
<212> PRT
<213> Artificial Sequence

<220>
<223> Made in a lab

<400> 723
Gln Arg His Asn Asn Ser Ser Leu Asn Thr Arg Thr Gln Lys Ala Arg
1               5                   10                  15
His Ser Pro Asn Ser
            20

<210> 724
<211> 36
<212> PRT
<213> Artificial Sequence

<220>
<223> Made in a lab

<400> 724
Asn Tyr Gly Gly Gly Gly Ser Gly Gly Gly Gly Ser Gly Gly Gly Gly
1               5                   10                  15
Ser Asn Tyr Gly Gly Gly Gly Ser Gly Gly Gly Gly Ser Gly Gly Gly
            20                  25                  30
Gly Ser Asn Ser
        35

<210> 725
<211> 122
<212> PRT
<213> Artificial Sequence

<220>
<223> Made in a lab

<400> 725
Arg Thr Arg Tyr Leu Gln Val Ser Gln Gln Leu Gln Gln Thr Asn Arg
1               5                   10                  15
Val Leu Glu Val Thr Asn Ser Ser Leu Arg Gln Gln Leu Arg Leu Lys
            20                  25                  30
Ile Thr Gln Leu Gly Gln Ser Ala Glu Asp Leu Gln Gly Ser Arg Arg
        35                  40                  45
Glu Leu Ala Gln Ser Gln Glu Ala Leu Gln Val Glu Gln Arg Ala His
    50                  55                  60
Gln Ala Ala Glu Gly Gln Leu Gln Ala Cys Gln Ala Asp Arg Gln Lys
65                  70                  75                  80
Thr Lys Glu Thr Leu Gln Ser Glu Glu Gln Gln Arg Arg Ala Leu Glu
                85                  90                  95
Gln Lys Leu Ser Asn Met Glu Asn Arg Leu Lys Pro Phe Phe Thr Cys
            100                 105                 110
Gly Ser Ala Asp Thr Cys Cys Pro Asn Ser
        115                 120

<210> 726
<211> 13
<212> PRT
<213> Artificial Sequence

<220>
<223> Made in a lab

<400> 726
Gly Gly Gly Ala Ser Cys Pro Pro Cys Ala Gly Asn Ser
1               5                   10

<210> 727
<211> 39
<212> PRT
<213> Artificial Sequence

<220>
<223> Made in a lab

<400> 727
Asn Asn Tyr Gly Gly Gly Gly Ser Gly Gly Gly Gly Ser Gly Gly Gly
1               5                   10                  15
Gly Ser Gly Asn Tyr Gly Gly Gly Gly Ser Gly Gly Gly Gly Ser Gly
            20                  25                  30
Gly Gly Gly Ser Gly Asn Ser
        35

<210> 728
<211> 20
<212> PRT
<213> Artificial Sequence

<220>
<223> Made in a lab

<400> 728
Asn Tyr Gly Gly Gly Gly Ser Gly Gly Gly Gly Ser Gly Gly Gly Gly
1               5                   10                  15
Ser Gly Asn Ser
            20

<210> 729
<211> 7
<212> PRT
<213> Artificial Sequence

<220>
<223> Made in a lab

<400> 729
Ser Pro Pro Ser Pro Asn Ser
1               5

<210> 730
<211> 17
<212> PRT
<213> Artificial Sequence

<220>
<223> Made in a lab

<400> 730
Glu Pro Thr Ser Thr Asp Lys Thr His Thr Ser Pro Pro Ser Pro Asn
1               5                   10                  15
Ser

<210> 731
<211> 17
<212> PRT
<213> Artificial Sequence

<220>
<223> Made in a lab

<400> 731
Glu Pro Thr Ser Thr Asp Lys Thr His Thr Cys Pro Pro Cys Pro Asn
1               5                   10                  15
Ser

<210> 732
<211> 19
<212> PRT
<213> Artificial Sequence

<220>
<223> Made in a lab

<400> 732
Leu Ser Val Lys Ala Asp Phe Leu Thr Pro Ser Ile Ser Pro Pro Cys
1               5                   10                  15
Pro Asn Ser

<210> 733
<211> 12
<212> PRT
<213> Artificial Sequence

<220>
<223> Made in a lab

<400> 733
Gly Gly Gly Ala Ser Cys Pro Pro Cys Ala Asn Ser
1               5                   10

<210> 734
<211> 22
<212> PRT
<213> Artificial Sequence

<220>
<223> Made in a lab

<400> 734
Arg Thr Gln Arg His Asn Asn Ser Ser Leu Asn Thr Arg Thr Gln Lys
1               5                   10                  15
Ala Arg His Ser Gly His
            20

<210> 735
<211> 118
<212> PRT
<213> Artificial Sequence

<220>
<223> Made in a lab

<400> 735
Arg Thr Arg Tyr Leu Gln Val Ser Gln Gln Leu Gln Gln Thr Asn Arg
1               5                   10                  15
Val Leu Glu Val Thr Asn Ser Ser Leu Arg Gln Gln Leu Arg Leu Lys
            20                  25                  30
Ile Thr Gln Leu Gly Gln Ser Ala Glu Asp Leu Gln Gly Ser Arg Arg
        35                  40                  45
Glu Leu Ala Gln Ser Gln Glu Ala Leu Gln Val Glu Gln Arg Ala His

50                  55                  60
Gln Ala Ala Glu Gly Gln Leu Gln Ala Cys Gln Ala Asp Arg Gln Lys
65                  70                  75                  80
Thr Lys Glu Thr Leu Gln Ser Glu Glu Gln Gln Arg Arg Ala Leu Glu
                85                  90                  95
Gln Lys Leu Ser Asn Met Glu Asn Arg Leu Lys Pro Phe Phe Thr Cys
            100                 105                 110
Gly Ser Ala Asp Thr Cys
        115

<210> 736
<211> 35
<212> PRT
<213> Artificial Sequence

<220>
<223> Made in a lab

<400> 736
Arg Thr Gln Ser Glu Glu Gln Gln Arg Arg Ala Leu Glu Gln Lys Leu
1               5                   10                  15
Ser Asn Met Glu Asn Arg Leu Lys Pro Phe Phe Thr Cys Gly Ser Ala
            20                  25                  30
Asp Thr Cys
        35

<210> 737
<211> 25
<212> PRT
<213> Artificial Sequence

<220>
<223> Made in a lab

<400> 737
Arg Thr Glu Gln Lys Leu Ser Asn Met Glu Asn Arg Leu Lys Pro Phe
1               5                   10                  15
Phe Thr Cys Gly Ser Ala Asp Thr Cys
            20                  25

<210> 738
<211> 26
<212> PRT
<213> Artificial Sequence

<220>
<223> Made in a lab

<400> 738
Ser Gly Gly Gly Gly Ser Gly Gly Gly Gly Ser Gly Gly Gly Gly Ser
1               5                   10                  15
Gly Gly Gly Gly Ser Gly Gly Gly Gly Ser
            20                  25

<210> 739
<211> 16
<212> PRT
<213> Artificial Sequence

<220>
<223> Made in a lab

<400> 739

Gly Gly Gly Gly Ser Gly Gly Gly Gly Ser Gly Gly Gly Gly Ala Ser
1               5                   10                  15

<210> 740
<211> 16
<212> PRT
<213> Artificial Sequence

<220>
<223> Made in a lab

<400> 740
Ser Glu Pro Lys Ser Ser Asp Lys Thr His Thr Cys Pro Pro Cys Pro
1               5                   10                  15

<210> 741
<211> 18
<212> PRT
<213> Artificial Sequence

<220>
<223> Made in a lab

<400> 741
Gly Asp Gln Glu Pro Lys Ser Ser Asp Lys Thr His Thr Ser Pro Pro
1               5                   10                  15
Cys Pro


<210> 742
<211> 21
<212> PRT
<213> Artificial Sequence

<220>
<223> Made in a lab

<400> 742
Gln Arg His Asn Asn Ser Ser Leu Asn Thr Gly Thr Gln Met Ala Gly
1               5                   10                  15
His Ser Pro Asn Ser
            20

<210> 743
<211> 21
<212> PRT
<213> Artificial Sequence

<220>
<223> Made in a lab

<400> 743
Gln Arg His Asn Asn Ser Ser Leu Asn Thr Gly Thr Gln Lys Ala Arg
1               5                   10                  15
His Ser Pro Asn Ser
            20

<210> 744
<211> 21
<212> PRT
<213> Artificial Sequence

<220>

<223> Made in a lab

<400> 744
Gln Arg His Asn Asn Ser Ser Leu Asn Thr Gly Thr Gln Met Ala Arg
1               5                   10                  15
His Ser Pro Asn Ser
            20

<210> 745
<211> 21
<212> PRT
<213> Artificial Sequence

<220>
<223> Made in a lab

<400> 745
Gln Arg His Asn Asn Ser Ser Leu Asn Thr Arg Thr Gln Lys Ala Gly
1               5                   10                  15
His Ser Pro Asn Ser
            20

<210> 746
<211> 21
<212> PRT
<213> Artificial Sequence

<220>
<223> Made in a lab

<400> 746
Gln Arg His Asn Asn Ser Ser Leu Asn Thr Arg Thr Gln Met Ala Gly
1               5                   10                  15
His Ser Pro Asn Ser
            20

<210> 747
<211> 21
<212> PRT
<213> Artificial Sequence

<220>
<223> Made in a lab

<400> 747
Gln Arg His Asn Asn Ser Ser Leu Asn Thr Arg Thr Gln Met Ala Arg
1               5                   10                  15
His Ser Pro Asn Ser
            20

<210> 748
<211> 10
<212> PRT
<213> Artificial Sequence

<220>
<223> Hinge sequence

<400> 748
Pro Pro Pro Pro Pro Cys Pro Pro Cys Pro
1               5                   10

<210> 749
<211> 16
<212> PRT
<213> Artificial Sequence

<220>
<223> Hinge sequence

<400> 749
Thr Glu Pro Lys Ser Ser Asp Lys Thr His Thr Cys Pro Pro Cys Pro
1               5                   10                  15


<210> 750
<211> 20
<212> PRT
<213> Artificial Sequence

<220>
<223> Hinge sequence

<400> 750
Glu Pro Lys Ser Ser Asp Lys Thr His Thr Ser Pro Pro Ser Pro Cys
1               5                   10                  15
Pro Pro Cys Pro
            20


<210> 751
<211> 20
<212> PRT
<213> Artificial Sequence

<220>
<223> Hinge sequence

<400> 751
Glu Pro Lys Ser Ser Asp Thr Pro Pro Pro Ser Pro Arg Ser Pro Cys
1               5                   10                  15
Pro Pro Cys Pro
            20


<210> 752
<211> 23
<212> PRT
<213> Artificial Sequence

<220>
<223> Hinge sequence

<400> 752
Pro Ser Thr Pro Pro Thr Pro Ser Pro Ser Thr Pro Pro Thr Pro Ser
1               5                   10                  15
Pro Ser Cys Pro Pro Cys Pro
            20


<210> 753
<211> 23
<212> PRT
<213> Artificial Sequence

<220>
<223> Hinge sequence

<400> 753
Arg Thr Glu Pro Arg Val Pro Ile Thr Gln Asn Pro Cys Pro Pro Leu
1               5                   10                  15
Lys Glu Cys Pro Pro Cys Ala
            20


<210> 754
<211> 39
<212> PRT
<213> Artificial Sequence

<220>
<223> Hinge sequence

<400> 754
Glu Ser Pro Lys Ala Gln Ala Ser Ser Val Pro Thr Ala Gln Pro Gln
1               5                   10                  15
Ala Glu Gly Ser Leu Ala Lys Ala Thr Thr Ala Pro Ala Thr Thr Arg
            20                  25                  30
Asn Thr Cys Pro Pro Cys Pro
        35


<210> 755
<211> 50
<212> PRT
<213> Artificial Sequence

<220>
<223> Hinge sequence

<400> 755
Gln Glu Lys Glu Ala Ile Glu Arg Leu Lys Ala Ala Gly Ala Pro Glu
1               5                   10                  15
Ser Leu Val Ile Gln Ala Tyr Phe Ala Ser Glu Lys Asn Glu Asn Leu
            20                  25                  30
Ala Ala Asn Phe Leu Leu Ser Gln Asn Phe Asp Asp Glu Cys Pro Pro
        35                  40                  45
Cys Pro
    50


<210> 756
<211> 52
<212> PRT
<213> Artificial Sequence

<220>
<223> Hinge sequence

<400> 756
Gln Glu Lys Glu Ala Ile Glu Arg Leu Lys Ala Ala Gly Ala Pro Glu
1               5                   10                  15
Ser Leu Val Ile Gln Ala Tyr Phe Ala Ser Glu Lys Asn Glu Asn Leu
            20                  25                  30
Ala Ala Asn Phe Leu Leu Ser Gln Asn Phe Asp Asp Glu Cys Pro Pro
        35                  40                  45
Cys Pro Ser Gly
    50

<210> 757
<211> 110
<212> PRT
<213> Artificial Sequence

<220>
<223> Hinge sequence

<400> 757
Ser Arg Asp Phe Thr Pro Pro Thr Val Lys Ile Leu Gln Ser Ser Ser
1               5                   10                  15
Asp Gly Gly Gly His Phe Pro Pro Thr Ile Gln Leu Leu Cys Leu Val
            20                  25                  30
Ser Gly Tyr Thr Pro Gly Thr Ile Asn Ile Thr Trp Leu Glu Asp Gly
        35                  40                  45
Gln Val Met Asp Val Asp Leu Ser Thr Ala Ser Thr Thr Gln Glu Gly
    50                  55                  60
Glu Leu Ala Ser Thr Gln Ser Glu Leu Thr Leu Ser Gln Lys His Trp
65                  70                  75                  80
Leu Ser Asp Arg Thr Tyr Thr Cys Gln Val Thr Tyr Gln Gly His Thr
                85                  90                  95
Phe Glu Asp Ser Thr Lys Lys Ser Ala Cys Pro Pro Cys Pro
            100                 105                 110


<210> 758
<211> 111
<212> PRT
<213> Artificial Sequence

<220>
<223> Hinge sequence

<400> 758
Ser Arg Asp Phe Thr Pro Pro Thr Val Lys Ile Leu Gln Ser Ser Ser
1               5                   10                  15
Asp Gly Gly Gly His Phe Pro Pro Thr Ile Gln Leu Leu Cys Leu Val
            20                  25                  30
Ser Gly Tyr Thr Pro Gly Thr Ile Asn Ile Thr Trp Leu Glu Asp Gly
        35                  40                  45
Gln Val Met Asp Val Asp Leu Ser Thr Ala Ser Thr Thr Gln Glu Gly
    50                  55                  60
Glu Leu Ala Ser Thr Gln Ser Glu Leu Thr Leu Ser Gln Lys His Trp
65                  70                  75                  80
Leu Ser Asp Arg Thr Tyr Thr Cys Gln Val Thr Tyr Gln Gly His Thr
                85                  90                  95
Phe Glu Asp Ser Thr Lys Lys Ser Ala Cys Pro Pro Cys Ser Gly
            100                 105                 110


<210> 759
<211> 116
<212> PRT
<213> Artificial Sequence

<220>
<223> Hinge sequence

<400> 759
Ile Ala Glu Leu Pro Pro Lys Val Ser Val Phe Val Pro Pro Arg Asp
1               5                   10                  15

Gly Phe Phe Gly Asn Pro Arg Lys Ser Lys Leu Ile Cys Gln Ala Thr
            20                  25                  30
Gly Phe Ser Pro Arg Gln Ile Gln Val Ser Trp Leu Arg Glu Gly Lys
        35                  40                  45
Gln Val Gly Ser Gly Val Thr Thr Asp Gln Val Gln Ala Glu Ala Lys
    50                  55                  60
Glu Ser Gly Pro Thr Thr Tyr Lys Val Thr Ser Thr Leu Thr Ile Lys
65                  70                  75                  80
Glu Ser Asp Trp Leu Gly Gln Ser Met Phe Thr Cys Arg Val Asp His
                85                  90                  95
Arg Gly Leu Thr Phe Gln Gln Asn Ala Ser Ser Met Ser Val Pro Cys
            100                 105                 110
Pro Pro Cys Pro
        115

<210> 760
<211> 6
<212> PRT
<213> Artificial Sequence

<220>
<223> linker sequence of monoIL-10

<400> 760
Gly Gly Gly Ser Gly Gly
1               5


<210> 761
<211> 106
<212> PRT
<213> Homo sapiens

<400> 761

<210> 762
<211> 216
<212> PRT
<213> Artificial Sequence

<220>
<223> IgG1 CH2 CH3 Null2 desLys

<400> 762
Ala Pro Glu Ala Ala Gly Ala Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr

35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Ala Tyr Ala Cys Ala Val Ser Asn Lys
                85                  90                  95
Ala Leu Pro Ala Pro Ile Glu Lys Thr Ile Ser Lys Ala Lys Gly Gln
            100                 105                 110
Pro Arg Glu Pro Gln Val Tyr Thr Leu Pro Pro Ser Arg Asp Glu Leu
        115                 120                 125
Thr Lys Asn Gln Val Ser Leu Thr Cys Leu Val Lys Gly Phe Tyr Pro
    130                 135                 140
Ser Asp Ile Ala Val Glu Trp Glu Ser Asn Gly Gln Pro Glu Asn Asn
145                 150                 155                 160
Tyr Lys Thr Thr Pro Pro Val Leu Asp Ser Asp Gly Ser Phe Phe Leu
                165                 170                 175
Tyr Ser Lys Leu Thr Val Asp Lys Ser Arg Trp Gln Gln Gly Asn Val
            180                 185                 190
Phe Ser Cys Ser Val Met His Glu Ala Leu His Asn His Tyr Thr Gln
        195                 200                 205
Lys Ser Leu Ser Leu Ser Pro Gly
    210                 215

<210> 763
<211> 7
<212> PRT
<213> Artificial Sequence

<220>
<223> Peptide linker

<400> 763
Ser Pro Pro Ser Pro Asn Ser
1               5

<210> 764
<211> 17
<212> PRT
<213> Artificial Sequence

<220>
<223> Peptide linker

<400> 764
Glu Pro Thr Ser Thr Asp Lys Thr His Thr Ser Pro Pro Ser Pro Asn
1               5                   10                  15
Ser

<210> 765
<211> 17
<212> PRT
<213> Artificial Sequence

<220>
<223> Peptide linker

<400> 765
Glu Pro Thr Ser Thr Asp Lys Thr His Thr Cys Pro Pro Cys Pro Asn

1               5                   10                  15
Ser



<210> 766
<211> 16
<212> PRT
<213> Artificial Sequence

<220>
<223> Peptide linker

<400> 766
Asn Gln Glu Val Gln Ile Pro Leu Thr Glu Ser Tyr Ser Pro Asn Ser
1               5                   10                  15



<210> 767
<211> 20
<212> PRT
<213> Artificial Sequence

<220>
<223> Peptide linker

<400> 767
Asn Ser Gly Ala Asn Gln Glu Val Gln Ile Pro Leu Thr Glu Ser Tyr
1               5                   10                  15
Ser Pro Asn Ser
            20



<210> 768
<211> 14
<212> PRT
<213> Artificial Sequence

<220>
<223> Peptide linker

<400> 768
Glu Val Gln Ile Pro Leu Thr Glu Ser Tyr Ser Pro Asn Ser
1               5                   10



<210> 769
<211> 20
<212> PRT
<213> Artificial Sequence

<220>
<223> Peptide linker

<400> 769
Asn Ser Gly Phe Asn Gln Glu Val Gln Ile Pro Leu Thr Glu Ser Tyr
1               5                   10                  15
Ser Pro Asn Ser
            20



<210> 770
<211> 20

<212> PRT
<213> Artificial Sequence

<220>
<223> Peptide linker

<400> 770
Asn Ser Leu Ala Asn Gln Glu Val Gln Ile Pro Leu Thr Glu Ser Tyr
1               5                   10                  15
Ser Pro Asn Ser
            20


<210> 771
<211> 20
<212> PRT
<213> Artificial Sequence

<220>
<223> Peptide linker

<400> 771
Asn Ser Leu Phe Ser Gln Glu Val Gln Ile Pro Leu Thr Glu Ser Tyr
1               5                   10                  15
Ser Pro Asn Ser
            20


<210> 772
<211> 20
<212> PRT
<213> Artificial Sequence

<220>
<223> Peptide linker

<400> 772
Asn Ser Leu Ala Ser Gln Glu Val Gln Ile Pro Leu Thr Glu Ser Tyr
1               5                   10                  15
Ser Pro Asn Ser
            20


<210> 773
<211> 26
<212> PRT
<213> Artificial Sequence

<220>
<223> Peptide linker

<400> 773
Glu Gln Lys Leu Ser Asn Met Glu Asn Arg Leu Lys Pro Phe Phe Thr
1               5                   10                  15
Ser Gly Ser Ala Asp Thr Ser Pro Asn Ser
            20                  25


<210> 774
<211> 21
<212> PRT
<213> Artificial Sequence

<220>
<223> Peptide linker

<400> 774
Gln Arg His Ala Asn Ser Ala Leu Asn Thr Gly Thr Gln Met Ala Gly
1               5                   10                  15
His Ser Pro Asn Ser
            20



<210> 775
<211> 21
<212> PRT
<213> Artificial Sequence

<220>
<223> Peptide linker

<400> 775
Gln Arg His Asn Ala Ala Ser Leu Asn Thr Gly Thr Gln Met Ala Gly
1               5                   10                  15
His Ser Pro Asn Ser
            20



<210> 776
<211> 21
<212> PRT
<213> Artificial Sequence

<220>
<223> Peptide linker

<400> 776
Gln Arg His Ala Ala Ser Ser Leu Asn Thr Gly Thr Gln Met Ala Gly
1               5                   10                  15
His Ser Pro Asn Ser
            20



<210> 777
<211> 19
<212> PRT
<213> Artificial Sequence

<220>
<223> Peptide linker

<400> 777
Gln Arg His Ser Ser Leu Asn Thr Gly Thr Gln Met Ala Gly His Ser
1               5                   10                  15
Pro Asn Ser



<210> 778
<211> 21
<212> PRT
<213> Artificial Sequence

<220>
<223> Peptide linker

<400> 778
Gln Arg His Gly Gly Ser Ser Leu Asn Thr Gly Thr Gln Met Ala Gly
1               5                   10                  15
His Ser Pro Asn Ser
            20

<210> 779
<211> 11
<212> PRT
<213> Artificial Sequence

<220>
<223> Peptide linker

<400> 779
His Asp Gly Ser Ser Leu Asn Thr Pro Asn Ser
1               5                   10

<210> 780
<211> 16
<212> PRT
<213> Artificial Sequence

<220>
<223> Peptide linker

<400> 780
Ser Ser Leu Asn Thr Gly Thr Gln Met Ala Gly His Ser Pro Asn Ser
1               5                   10                  15

<210> 781
<211> 16
<212> PRT
<213> Artificial Sequence

<220>
<223> Peptide linker

<400> 781
Ser Ser Leu Asn Thr Arg Thr Gln Met Ala Gly His Ser Pro Asn Ser
1               5                   10                  15

<210> 782
<211> 16
<212> PRT
<213> Artificial Sequence

<220>
<223> Peptide linker

<400> 782
Ser Ser Leu Asn Thr Gly Thr Gln Met Ala Arg His Ser Pro Asn Ser
1               5                   10                  15

<210> 783
<211> 16
<212> PRT
<213> Artificial Sequence

<220>
<223> Peptide linker

<400> 783
Ser Ser Leu Asn Thr Gly Thr Gln Lys Ala Gly His Ser Pro Asn Ser
1               5                   10                  15


<210> 784
<211> 16
<212> PRT
<213> Artificial Sequence

<220>
<223> Peptide linker

<400> 784
Ser Ser Leu Asn Thr Gly Thr Gln Lys Ala Arg His Ser Pro Asn Ser
1               5                   10                  15


<210> 785
<211> 16
<212> PRT
<213> Artificial Sequence

<220>
<223> Peptide linker

<400> 785
Ser Ser Leu Asn Thr Arg Thr Gln Met Ala Arg His Ser Pro Asn Ser
1               5                   10                  15


<210> 786
<211> 16
<212> PRT
<213> Artificial Sequence

<220>
<223> Peptide linker

<400> 786
Ser Ser Leu Asn Thr Arg Thr Gln Lys Ala Gly His Ser Pro Asn Ser
1               5                   10                  15


<210> 787
<211> 17
<212> PRT
<213> Artificial Sequence

<220>
<223> Peptide linker

<400> 787
Asn Ser Leu Phe Asn Gln Glu Val Gln Ile Pro Leu Thr Glu Pro Asn
1               5                   10                  15
Ser

<210> 788
<211> 14
<212> PRT
<213> Artificial Sequence

<220>
<223> Peptide linker

<400> 788
Asn Ser Leu Phe Asn Gln Glu Val Gln Ile Pro Pro Asn Ser
1               5                   10


<210> 789
<211> 11
<212> PRT
<213> Artificial Sequence

<220>
<223> Peptide linker

<400> 789
Asn Ser Leu Phe Asn Gln Glu Val Pro Asn Ser
1               5                   10


<210> 790
<211> 13
<212> PRT
<213> Artificial Sequence

<220>
<223> Peptide linker

<400> 790
Gln Arg His Asn Asn Ser Ser Leu Asn Thr Pro Asn Ser
1               5                   10


<210> 791
<211> 19
<212> PRT
<213> Artificial Sequence

<220>
<223> Peptide linker

<400> 791
Ser Gly Gly Gly Gly Ser Gly Gly Gly Gly Ser Gly Gly Gly Gly Ser
1               5                   10                  15
Pro Asn Ser


<210> 792
<211> 2730
<212> DNA
<213> Artificial Sequence

<220>
<223> TSC038 comprises: G19-4 (anti-CD3) scFv, human
      IgG1 SCC-P hinge, human IgG1 CH2, human IgG1 CH3,
      human Ck(YAE), H75 linker and 3D1 (anti-CD86) scFv

<400> 792
acgactcact atagggagac ccaagcttgc cgccatggaa gcaccagcgc agcttctctt 60
cctcctgcta ctctggctcc cagataccac cggtgaggtc cagctgcaac agtctggacc 120
tgaactggtg aagcctggag cttcaatgaa gatttcctgc aaggcctctg gttactcatt 180
cactggctac atcgtgaact ggctgaagca gagccatgga aagaaccttg agtggattgg 240
acttattaat ccatacaaag gtcttactac ctacaaccag aaattcaagg gcaaggccac 300
attaactgta gacaagtcat ccagcacagc ctacatggag ctcctcagtc tgacatctga 360
agactctgca gtctattact gtgcaagatc tgggtactat ggtgactcgg actggtactt 420
cgatgtctgg ggcgcaggga ccacggtcac cgtctcaagc ggtggcggag ggtctggggg 480
tggcggatcc ggaggtggtg gctctgcaca agacatccag atgacacaga ctacatcctc 540
cctgtctgcc tctctgggag acagagtcac catcagttgc agggcaagtc aggacattcg 600
caattattta aactggtatc agcagaaacc agatggaact gttaaactcc tgatctacta 660
cacatcaaga ttacactcag gagtcccatc aaggttcagt ggcagtgggt ctggaacaga 720
ttattctctc accattgcca acctgcaacc agaagatatt gccacttact tttgccaaca 780
gggtaatacg cttccgtgga cgttcggtgg aggcaccaaa ctggtaacca aacgctcgag 840
cgagcccaaa tcttctgaca aaactcacac atgcccaccg tgcccatccg gagcacctga 900
actcctgggt ggaccgtcag tcttcctctt ccccccaaaa cccaaggaca ccctcatgat 960
ctcccggacc cctgaggtca catgcgtggt ggtggacgtg agccacgaag accctgaggt 1020
caagttcaac tggtacgtgg acggcgtgga ggtgcataat gccaagacaa agccgcggga 1080
ggagcagtac aacagcacgt accgtgtggt cagcgtcctc accgtcctgc accaggactg 1140
gctgaatggc aaggagtaca agtgcaaggt ctccaacaaa gccctcccag cccccatcga 1200
gaaaaccatc tccaaagcca aagggcagcc ccgagaacca caggtgtaca ccctgccccc 1260
atcccgggat gagctgacca agaaccaggt cagcctgacc tgcctggtca aaggcttcta 1320
tccaagcgac atcgccgtgg agtgggagag caatgggcag ccggagaaca actacaagac 1380
cacgcctccc gtgctggact ccgacggctc cttcttcctc tacagcaagc tcaccgtgga 1440
caagagcagg tggcagcagg ggaacgtctt ctcatgctcc gtgatgcatg aggctctgca 1500
caaccactac acgcagaaga gcctctccct gtctccgggt aaatctagaa ctgtggctgc 1560
accatctgtc ttcatcttcc cgccatctga tgagcagttg aaatctggaa ctgcctctgt 1620
tgtgtgcctg ctgaattact tctatcccag agaggccaaa gtacagtgga aggtggataa 1680
cgccctccaa tcgggtaact cccaggagag tgccacagag caggacagca aggacagcac 1740
ctacagcctc agcagcgagc tgacgctgag caaagcagac tacgagaaac acaaagtcta 1800
cgcctgcgaa gtcacccatc agggcctgag ctcgcccgtc acaaagagct tcaacagggg 1860
agagcagagg cacaacaatt cttccctgaa tacaggaact cagatggcag gtcattctcc 1920
gaattctcag gtccagctgc agcagtctgg gcctgagctg gtgaggcctg gggaatcagt 1980
gaagatttcc tgcaagggtt ccggctacac attcactgat tatgctatac agtgggtgaa 2040
gcagagtcat gcaaagagtc tagagtggat tggagttatt aatatttact atgataatac 2100
aaactacaac cagaagttta agggcaaggc cacaatgact gtagacaaat cctccagcac 2160
agcctatatg gaacttgcca gattgacatc tgaggattct gccatctatt actgtgcaag 2220
agcggcctgg tatatggact actggggtca aggaacctca gtcaccgtct cctcaggggg 2280
tggaggctct ggtggcggtg gctctggcgg aggtggatcc ggtggcggcg gatctgacat 2340
tgtgctgtca cagtctccat cctccctggc tgtgtcagca ggagagaagg tcactatgag 2400
ctgcaaatcc agtcagagtc tgctcaacag tagaacccga gagaactact tggcttggta 2460
ccagcagaaa ccagggcagt ctcctaaact gctgatctac tgggcatcca ctagggaatc 2520
tggggtccct gatcgcttca caggcagtgg atctgggaca gatttcactc tcaccatcag 2580
cagtgtgcag gctgaagacc tggcagttta ttactgcacg caatcttata atctttacac 2640
gttcggaggg gggaccaagc tggaaataaa ataatctaga gcggccgctc tagagggccc 2700
tattctatag tgtcacctaa atgctagagg 2730

<210> 793
<211> 1821
<212> DNA
<213> Artificial Sequence

<220>
<223> TSC039 comprises: BMA031 (anti-TCR) scFv, human
      IgG1 SCC-P hinge, human IgG1 CH2, human IgG1 CH3,
      and human Ck(YAE)

<400> 793
atggaagcac cagcgcagct tctcttcctc ctgctactct ggctcccaga taccaccggt 60
ggacaggtcc aactagttca gtctggagca gaggttaaga agcctgggag ctcagtgaag 120
gtttcctgca aggcttccgg atataaattc actagctatg ttatgcactg ggtgaagcag 180
gcacctggcc aggggctcga gtggattgga tatattaatc cttacaatga tgttactaag 240

tacaatgaga agttcaaagg caaggccaca cttacggccg acgagtccac taatacagcc 300
tacatggagc tgagcagcct gagatctgag gacactgcgg tccattactg tgcaagaggg 360
agctactatg attacgacgg gtttgtttac tggggccaag gtaccctggt caccgtctca 420
agcggtggcg gagggtctgg gggtggcgga tccggaggtg gtggctctgc acaagatatc 480
cagatgaccc aatctccatc gaccctgagt gcatctgtcg gggacagagt caccatgacc 540
tgcagtgcca cctcaagtgt aagttacatg cactggtatc agcagaagcc cgggaaggct 600
cccaaaagat ggatctatga cacatccaaa ctggcttctg gagtccctgc gcgcttcatt 660
ggcagtgggt ctgggaccga gttcacgctc acaatcagca gcctgcagcc agatgatttc 720
gctacgtatt actgccagca gtggagtagt aacccgctca cgttcggtgg aggtaccaag 780
gtcgagatta aatcctcgag cgagcccaaa tcttctgaca aaactcacac atgcccaccg 840
tgcccagcac ctgaactcct gggtggaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggag tacaagtgca aggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat tacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 794
<211> 1821
<212> DNA
<213> Artificial Sequence

<220>
<223> TSC041 comprises: CRIS7 (anti-CD3) scFv, human
IgG1 SCC-P hinge, human IgG1 CH2, human IgG1 CH3,
and human Ck(YAE)

<400> 794
atggaagcac cagcgcagct tctcttcctc ctgctactct ggctcccaga taccaccggt 60
caggtccagc tggtgcagtc tggggcggaa gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag cgagcccaaa tcttctgaca aaactcacac atgcccaccg 840
tgcccagcac ctgaactcct gggtggaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggag tacaagtgca aggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440

catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat tacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 795
<211> 1773
<212> DNA
<213> Artificial Sequence

<220>
<223> TSC043 comprises: OKT3-M (Micromet variant
      anti-CD3, see also US 7,635,472) scFv, human IgG1
      SCC-P hinge, human IgG1 CH2, human IgG1 CH3, and
      human Ck(YAE)

<400> 795
atggaagcac cagcgcagct tctcttcctc ctgctactct ggctcccaga taccaccggt 60
gatatcaaac tgcagcagtc aggggctgaa ctggcaagac ctggggcctc agtgaagatg 120
tcctgcaaga cttctggcta cacctttact aggtacacga tgcactgggt aaaacagagg 180
cctggacagg gtctggaatg gattggatac attaatccta gccgtggtta tactaattac 240
aatcagaagt tcaaggacaa ggccacattg actacagaca aatcctccag cacagcctac 300
atgcaactga gcagcctgac atctgaggac tctgcagtct attactgtgc aagatattat 360
gatgatcatt actgccttga ctactggggc caaggcacca ctctcacagt ctcctcagtc 420
gaaggtggaa gtggaggttc tggtggaagt ggaggttcag gtggagtcga cgacattcag 480
ctgacccagt ctccagcaat catgtctgca tctccagggg agaaggtcac catgacctgc 540
agagccagtt caagtgtaag ttacatgaac tggtaccagc agaagtcagg cacctccccc 600
aaaagatgga tttatgacac atccaaagtg gcttctggag tcccttatcg cttcagtggc 660
agtgggtctg ggacctcata ctctctcaca atcagcagca tggaggctga agatgctgcc 720
acttattact gccaacagtg gagtagctcg agcgagccca aatcttctga caaaactcac 780
acatgcccac cgtgcccagc acctgaactc ctgggtggac cgtcagtctt cctcttcccc 840
ccaaaaccca aggacaccct catgatctcc cggacccctg aggtcacatg cgtggtggtg 900
gacgtgagcc acgaagaccc tgaggtcaag ttcaactggt acgtggacgg cgtggaggtg 960
cataatgcca agacaaagcc gcgggaggag cagtacaaca gcacgtaccg tgtggtcagc 1020
gtcctcaccg tcctgcacca ggactggctg aatggcaagg agtacaagtg caaggtctcc 1080
aacaaagccc tcccagcccc catcgagaaa accatctcca aagccaaagg gcagccccga 1140
gaaccacagg tgtacaccct gcccccatcc cgggatgagc tgaccaagaa ccaggtcagc 1200
ctgacctgcc tggtcaaagg cttctatcca agcgacatcg ccgtggagtg ggagagcaat 1260
gggcagccgg agaacaacta caagaccacg cctcccgtgc tggactccga cggctccttc 1320
ttcctctaca gcaagctcac cgtggacaag agcaggtggc agcaggggaa cgtcttctca 1380
tgctccgtga tgcatgaggc tctgcacaac cactacacgc agaagagcct ctccctgtct 1440
ccgggtaaat ctagaactgt ggctgcacca tctgtcttca tcttcccgcc atctgatgag 1500
cagttgaaat ctggaactgc ctctgttgtg tgcctgctga attacttcta tcccagagag 1560
gccaaagtac agtggaaggt ggataacgcc ctccaatcgg gtaactccca ggagagtgcc 1620
acagagcagg acagcaagga cagcacctac agcctcagca gcgagctgac gctgagcaaa 1680
gcagactacg agaaacacaa agtctacgcc tgcgaagtca cccatcaggg cctgagctcg 1740
cccgtcacaa agagcttcaa caggggagag tga 1773

<210> 796
<211> 2088
<212> DNA
<213> Artificial Sequence

<220>
<223> TSC049 comprises: HD37 (anti-CD19) scFv, human
      IgG1 SCC-P hinge, human IgG1 CH2(ADCC/CDC null),
      human IgG1 CH3, and human IgG1 CH1

<400> 796

cgaaattaat acgactcact atagggagac ccaagcttgc cgccatggaa gcaccagcgc 60
agcttctctt cctcctgcta ctctggctcc cagataccac cggtgacatt gtgctgaccc 120
aatctccagc ttctttggct gtgtctctag ggcagagggc caccatctcc tgcaaggcca 180
gccaaagtgt tgattatgat ggtgatagtt atttgaactg gtaccaacag attccaggac 240
agccacccaa actcctcatc tatgatgcat ccaatctagt ttctgggatc ccacccaggt 300
ttagtggcag tgggtctggg acagacttca ccctcaacat ccatcctgtg gagaaggtgg 360
atgctgcaac ctatcactgc cagcaaagta ctgaggatcc gtggacgttc ggtggaggca 420
ccaagctgga aatcaaaggt ggcggtggtt cgggcggtgg tgggtcgggt ggcggcggag 480
ctagccaggt tcagctgcag cagtctgggg ctgagctggt gaggcctggg tcctcagtga 540
agatttcctg caaggcttct ggctatgcat tcagtagcta ctggatgaac tgggtgaagc 600
agaggcctgg acagggtctt gagtggattg gacagatttg gcctggagat ggtgatacta 660
actacaatgg aaagttcaag ggtaaagcca ctctgactgc agacgaatcc tccagcacag 720
cctacatgca actcagcagc ctagcatctg aggactctgc ggtctatttc tgtgcaagac 780
gggagactac gacggtaggc cgttattact atgctatgga ctactggggt caaggaacct 840
cagtcaccgt ctcctcgagt gagcccaaat cttctgacaa aactcacaca tgcccaccgt 900
gcccagcacc tgaagccgcg ggtgcaccgt cagtcttcct cttcccccca aaacccaagg 960
acaccctcat gatctcccgg acccctgagg tcacatgcgt ggtggtggac gtgagccacg 1020
aagaccctga ggtcaagttc aactggtacg tggacggcgt ggaggtgcat aatgccaaga 1080
caaagccgcg ggaggagcag tacaacagca cgtaccgtgt ggtcagcgtc ctcaccgtcc 1140
tgcaccagga ctggctgaat ggcaaggcgt acgcgtgcgc ggtctccaac aaagccctcc 1200
cagcccccat cgagaaaacc atctccaaag ccaaagggca gccccgagaa ccacaggtgt 1260
acaccctgcc cccatcccgg gatgagctga ccaagaacca ggtcagcctg acctgcctgg 1320
tcaaaggctt ctatccaagc gacatcgccg tggagtggga gagcaatggg cagccggaga 1380
acaactacaa gaccacgcct cccgtgctgg actccgacgg ctccttcttc ctctacagca 1440
agctcaccgt ggacaagagc aggtggcagc aggggaacgt cttctcatgc tccgtgatgc 1500
atgaggctct gcacaaccac tacacgcaga agagcctctc cctgtctccg ggtaaatcta 1560
gagcctccac caagggccca tcggtcttcc ccctggcacc ctcctccaag agcacctctg 1620
ggggcacagc ggccctgggc tgcctggtca aggactactt ccccgagccg gtgacggtgt 1680
cgtggaactc aggcgccctg accagcggcg tgcacacctt cccggctgtc ctacagtcct 1740
caggactcta ctccctcagc agcgtggtga ccgtgccctc cagcagcttg ggcacccaga 1800
cctacatctg caacgtgaat cacaagccca gcaacaccaa ggtggacaag aaagtttgat 1860
accactacat atcgcggccg ctctagaggg ccctattcta tagtgtcacc taaatgctag 1920
agctcgctga tctgcctcga ctgtgccttc tagttgccag ccatctgttg tttgcccctc 1980
ccccgtgcct tccttgaccc tggaaggtgc cactcccact gtcctttcct aataaaatga 2040
ggaaattgca tcgcattgtc tgagtaggtg tcattctatt ctgggggg 2088

<210> 797
<211> 2067
<212> DNA
<213> Artificial Sequence

<220>
<223> TSC050 comprises: 2H7 (anti-CD20) scFv, human IgG1
      SCC-P hinge, human IgG1 CH2(ADCC/CDC null), human
      IgG1 CH3, and human IgG1 CH1

<400> 797
cgaaattaat acgactcact atagggagac ccaagcttgc cgccatggaa gcaccagcgc 60
agcttctctt cctcctgcta ctctggctcc cagataccac cggtcaaatt gttctctccc 120
agtctccagc aatcctgtct gcatctccag gggagaaggt cacaatgact tgcagggcca 180
gctcaagtgt aagttacatg cactggtacc agcagaagcc aggatcctcc cccaaaccct 240
ggatttatgc cccatccaac ctggcttctg gagtccctgc tcgcttcagt ggcagtgggt 300
ctgggacctc ttactctctc acaatcagca gagtggaggc tgaagatgct gccacttatt 360
actgccagca gtggagtttt aacccaccca cgttcggtgc tgggaccaag ctggagctga 420
aagatggcgg tggctcgggc ggtggtggat ctggaggagg tggggctagc caggcttatc 480
tacagcagtc tggggctgag tcggtgaggc ctggggcctc agtgaagatg tcctgcaagg 540
cttctggcta cacatttacc agttacaata tgcactgggt aaagcagaca cctagacagg 600
gcctggaatg gattggagct atttatccag gaaatggtga tacttcctac aatcagaagt 660
tcaagggcaa ggccacactg actgtagaca aatcctccag cacagcctac atgcagctca 720
gcagcctgac atctgaagac tctgcggtct atttctgtgc aagagtggtg tactatagta 780
actcttactg gtacttcgat gtctggggca cagggaccac ggtcaccgtc tcctcgagcg 840
agcccaaatc ttctgacaaa actcacacat gcccaccgtg cccagcacct gaagccgcgg 900
gtgcaccgtc agtcttcctc ttccccccaa aacccaagga caccctcatg atctcccgga 960

cccctgaggt cacatgcgtg gtggtggacg tgagccacga agaccctgag gtcaagttca 1020
actggtacgt ggacggcgtg gaggtgcata atgccaagac aaagccgcgg gaggagcagt 1080
acaacagcac gtaccgtgtg gtcagcgtcc tcaccgtcct gcaccaggac tggctgaatg 1140
gcaaggcgta cgcgtgcgcg gtctccaaca aagccctccc agcccccatc gagaaaacca 1200
tctccaaagc caaagggcag ccccgagaac cacaggtgta caccctgccc ccatcccggg 1260
atgagctgac caagaaccag gtcagcctga cctgcctggt caaaggcttc tatccaagcg 1320
acatcgccgt ggagtgggag agcaatgggc agccggagaa caactacaag accacgcctc 1380
ccgtgctgga ctccgacggc tccttcttcc tctacagcaa gctcaccgtg gacaagagca 1440
ggtggcagca ggggaacgtc ttctcatgct ccgtgatgca tgaggctctg cacaaccact 1500
acacgcagaa gagcctctcc ctgtctccgg gtaaatctag agcctccacc aagggcccat 1560
cggtcttccc cctggcaccc tcctccaaga gcacctctgg gggcacagcg gccctgggct 1620
gcctggtcaa ggactacttc cccgagccgg tgacggtgtc gtggaactca ggcgccctga 1680
ccagcggcgt gcacaccttc ccggctgtcc tacagtcctc aggactctac tccctcagca 1740
gcgtggtgac cgtgccctcc agcagcttgg gcacccagac ctacatctgc aacgtgaatc 1800
acaagcccag caacaccaag gtggacaaga aagtttgata ccactacata tcgcggccgc 1860
tctagagggc cctattctat agtgtcacct aaatgctaga gctcgctgat ctgcctcgac 1920
tgtgccttct agttgccagc catctgttgt ttgcccctcc cccgtgcctt ccttgaccct 1980
ggaaggtgcc actcccactg tcctttccta ataaaatgag gaaattgcat cgcattgtct 2040
gagtaggtgt cattctattc tggggg 2067

<210> 798
<211> 2055
<212> DNA
<213> Artificial Sequence

<220>
<223> TSC051 comprises: P2C2 (anti-CD79) scFv, human
      IgG1 SCC-P hinge, human IgG1 CH2(ADCC/CDC null),
      human IgG1 CH3, and human IgG1 CH1

<400> 798
cgaaattaat acgactcact atagggagac ccaagcttgc cgccatggaa gcaccagcgc 60
agcttctctt cctcctgcta ctctggctcc cagataccac cggtgatatt gtgatgatcc 120
aggatgaact ctccaatcct gtcagttctg gagaatcagt ttccatctcc tgtaggtcta 180
gtaagagtct cctagataag gaagggaaga catacttgaa ttggtttctg cagagaccag 240
gacaatctcc tcagctcctg atctatctga tgtccatgcg tgaatcagga gtctcagacc 300
ggtttagtgg cagtgggtca ggaacagatt tcaccctgga aatcagtaga gtgaaggctg 360
aggatgtggg tgtgtattac tgtcaacaac ttgtagagta tccgtggacg ttcggtggag 420
gcaccaagct ggaaatcaaa ggtggcggtg gctctggcgg aggtggatcc ggtggcggcg 480
gctctcagat ccagttggtg cagtctggac ctgagctgaa gaagcctgga gagacagtca 540
agatctcctg caaggcttct ggttatacct tcacagactt ttcaatgcac tgggtgaggc 600
aggctccagg aaagggttta aagtggatgg gctggataaa cactgagact ggtgagccaa 660
catatgcaga tgacttcagg ggacggtttg ccttctcttt ggaaacttct gccagcactg 720
cctatttgca gatcaacaac ctcaaaaatg aggacacggc tacatatttt tgtacctggt 780
ctgcttactg gggccaaggg actctggtca ctgtctctgc ctcgagcgag cccaaatctt 840
ctgacaaaac tcacacatgc ccaccgtgcc cagcacctga agccgcgggt gcaccgtcag 900
tcttcctctt ccccccaaaa cccaaggaca ccctcatgat ctcccggacc cctgaggtca 960
catgcgtggt ggtggacgtg agccacgaag accctgaggt caagttcaac tggtacgtgg 1020
acggcgtgga ggtgcataat gccaagacaa agccgcggga ggagcagtac aacagcacgt 1080
accgtgtggt cagcgtcctc accgtcctgc accaggactg gctgaatggc aaggcgtacg 1140
cgtgcgcggt ctccaacaaa gccctcccag cccccatcga gaaaaccatc tccaaagcca 1200
aagggcagcc ccgagaacca caggtgtaca ccctgccccc atcccgggat gagctgacca 1260
agaaccaggt cagcctgacc tgcctggtca aaggcttcta tccaagcgac atcgccgtgg 1320
agtgggagag caatgggcag ccggagaaca actacaagac cacgcctccc gtgctggact 1380
ccgacggctc cttcttcctc tacagcaagc tcaccgtgga caagagcagg tggcagcagg 1440
ggaacgtctt ctcatgctcc gtgatgcatg aggctctgca caaccactac acgcagaaga 1500
gcctctccct gtctccgggt aaatctagag cctccaccaa gggcccatcg gtcttccccc 1560
tggcaccctc ctccaagagc acctctgggg gcacagcggc cctgggctgc ctggtcaagg 1620
actacttccc cgagccggtg acggtgtcgt ggaactcagg cgccctgacc agcggcgtgc 1680
acaccttccc ggctgtccta cagtcctcag gactctactc cctcagcagc gtggtgaccg 1740
tgccctccag cagcttgggc acccagacct acatctgcaa cgtgaatcac aagcccagca 1800
acaccaaggt ggacaagaaa gtttgatacc actacatatc gcggccgctc tagagggccc 1860
tattctatag tgtcacctaa atgctagagc tcgctgatct gcctcgactg tgccttctag 1920

ttgccagcca tctgttgttt gcccctcccc cgtgccttcc ttgaccctgg aaggtgccac 1980
tcccactgtc ctttcctaat aaaatgagga aattgcatcg cattgtctga gtaggtgtca 2040
ttctattctg ggggg 2055

<210> 799
<211> 1870
<212> DNA
<213> Artificial Sequence

<220>
<223> TSC052 comprises: 5D5 (anti-cMet) scFv, human IgG1
      SCC-P hinge, human IgG1 CH2(ADCC/CDC null), human
      IgG1 CH3, and human IgG1 CH1

<400> 799
cttatcgaat taatacgact cactataggg agacccaagc ttgccgccat ggaagcacca 60
gcgcagcttc tcttcctcct gctactctgg ctcccagata ccaccggtga ctttatgatg 120
tcacagtctc catcctccct aactgtgtca gttggagaga aggttactgt gagctgcaag 180
tccagtcagt cccttttata tactagcagt cagaagaact acttggcctg gtaccagcag 240
aaaccaggtc agtctcctaa actgctgatt tactgggcat ccactaggga atctggggtc 300
cctgatcgct tcacaggcag tggatctggg acagatttca ctctcaccat caccagtgtg 360
aaggctgacg acctggcagt ttattactgt cagcaatatt atgcctatcc gtggacgttc 420
ggtggaggca ccaagttgga gctcaaaggt ggcggtggct ctggcggagg tggatccggt 480
ggcggcggct ctcaggtcca actgcagcag tctgggcctg agctggtgag gcctggggct 540
tcagtgaaga tgtcctgcag ggcttcgggc tataccttca ccagctactg gttgcactgg 600
gttaaacaga ggcctggaca aggccttgag tggattggca tgattgatcc ttccaatagt 660
gacactaggt ttaatccgaa cttcaaggac aaggccacat tgaatgtaga cagatcttcc 720
aacacagcct acatgctgct cagcagcctg acatctgctg actctgcagt ctattactgt 780
gccacatatg gtagctacgt ttcccctctg gactactggg gtcaaggaac ctcagtcacc 840
gtctcctcga gcgagcccaa atcttctgac aaaactcaca catgcccacc gtgcccagca 900
cctgaagccg cgggtgcacc gtcagtcttc ctcttccccc caaaacccaa ggacaccctc 960
atgatctccc ggacccctga ggtcacatgc gtggtggtgg acgtgagcca cgaagaccct 1020
gaggtcaagt tcaactggta cgtggacggc gtggaggtgc ataatgccaa gacaaagccg 1080
cgggaggagc agtacaacag cacgtaccgt gtggtcagcg tcctcaccgt cctgcaccag 1140
gactggctga atggcaaggc gtacgcgtgc gcggtctcca acaaagccct cccagccccc 1200
atcgagaaaa ccatctccaa agccaaaggg cagccccgag aaccacaggt gtacaccctg 1260
cccccatccc gggatgagct gaccaagaac caggtcagcc tgacctgcct ggtcaaaggc 1320
ttctatccaa gcgacatcgc cgtggagtgg gagagcaatg ggcagccgga gaacaactac 1380
aagaccacgc ctcccgtgct ggactccgac ggctccttct tcctctacag caagctcacc 1440
gtggacaaga gcaggtggca gcaggggaac gtcttctcat gctccgtgat gcatgaggct 1500
ctgcacaacc actacacgca gaagagcctc tccctgtctc cgggtaaatc tagagcctcc 1560
accaagggcc catcggtctt ccccctggca ccctcctcca agagcacctc tgggggcaca 1620
gcggccctgg gctgcctggt caaggactac ttccccgagc cggtgacggt gtcgtggaac 1680
tcaggcgccc tgaccagcgg cgtgcacacc ttcccggctg tcctacagtc ctcaggactc 1740
tactccctca gcagcgtggt gaccgtgccc tccagcagct tgggcaccca gacctacatc 1800
tgcaacgtga atcacaagcc cagcaacacc aaggtggaca agaaagtttg ataccactac 1860
atatcgcggc 1870

<210> 800
<211> 1827
<212> DNA
<213> Artificial Sequence

<220>
<223> TSC053 comprises: G19-4 (anti CD3) scFv, human
      IgG1 SCC-P hinge, human IgG1 CH2(ADCC/CDC null),
      human IgG1 CH3, and human Ck(YAE)

<400> 800
atggaagcac cagcgcagct tctcttcctc ctgctactct ggctcccaga taccaccggt 60
gaggtccagc tgcaacagtc tggacctgaa ctggtgaagc ctggagcttc aatgaagatt 120
tcctgcaagg cctctggtta ctcattcact ggctacatcg tgaactggct gaagcagagc 180

catggaaaga accttgagtg gattggactt attaatccat acaaaggtct tactacctac 240
aaccagaaat tcaagggcaa ggccacatta actgtagaca agtcatccag cacagcctac 300
atggagctcc tcagtctgac atctgaagac tctgcagtct attactgtgc aagatctggg 360
tactatggtg actcggactg gtacttcgat gtctggggcg cagggaccac ggtcaccgtc 420
tcaagcggtg gcggagggtc tgggggtggc ggatccggag gtggtggctc tgcacaagac 480
atccagatga cacagactac atcctccctg tctgcctctc tgggagacag agtcaccatc 540
agttgcaggg caagtcagga cattcgcaat tatttaaact ggtatcagca gaaaccagat 600
ggaactgtta aactcctgat ctactacaca tcaagattac actcaggagt cccatcaagg 660
ttcagtggca gtgggtctgg aacagattat tctctcacca ttgccaacct gcaaccagaa 720
gatattgcca cttacttttg ccaacagggt aatacgcttc cgtggacgtt cggtggaggc 780
accaaactgg taaccaaacg ctcgagcgag cccaaatctt ctgacaaaac tcacacatgc 840
ccaccgtgcc cagcacctga agccgcgggt gcaccgtcag tcttcctctt ccccccaaaa 900
cccaaggaca ccctcatgat ctcccggacc cctgaggtca catgcgtggt ggtggacgtg 960
agccacgaag accctgaggt caagttcaac tggtacgtgg acggcgtgga ggtgcataat 1020
gccaagacaa agccgcggga ggagcagtac aacagcacgt accgtgtggt cagcgtcctc 1080
accgtcctgc accaggactg gctgaatggc aaggcgtacg cgtgcgcggt ctccaacaaa 1140
gccctcccag cccccatcga gaaaaccatc tccaaagcca aagggcagcc ccgagaacca 1200
caggtgtaca ccctgccccc atcccgggat gagctgacca agaaccaggt cagcctgacc 1260
tgcctggtca aaggcttcta tccaagcgac atcgccgtgg agtgggagag caatgggcag 1320
ccggagaaca actacaagac cacgcctccc gtgctggact ccgacggctc cttcttcctc 1380
tacagcaagc tcaccgtgga caagagcagg tggcagcagg ggaacgtctt ctcatgctcc 1440
gtgatgcatg aggctctgca caaccactac acgcagaaga gcctctccct gtctccgggt 1500
aaatctagaa ctgtggctgc accatctgtc ttcatcttcc cgccatctga tgagcagttg 1560
aaatctggaa ctgcctctgt tgtgtgcctg ctgaattact tctatcccag agaggccaaa 1620
gtacagtgga aggtggataa cgccctccaa tcgggtaact cccaggagag tgccacagag 1680
caggacagca aggacagcac ctacagcctc agcagcgagc tgacgctgag caaagcagac 1740
tacgagaaac acaaagtcta cgcctgcgaa gtcacccatc agggcctgag ctcgcccgtc 1800
acaaagagct tcaacagggg agagtga 1827

<210> 801
<211> 2707
<212> DNA
<213> Artificial Sequence

<220>
<223> TSC064 comprises: G19-4 (anti-CD3) scFv, human
      IgG1 SCC-P hinge, human IgG1 CH2, human IgG1 CH3,
      human Ck(YAE), H75 linker, and Met021 (anti-c-Met)
      scFv

<400> 801
atacgactca ctatagggag acccaagctt gccgccatgg aagcaccagc gcagcttctc 60
ttcctcctgc tactctggct cccagatacc accggtgagg tccagctgca acagtctgga 120
cctgaactgg tgaagcctgg agcttcaatg aagatttcct gcaaggcctc tggttactca 180
ttcactggct acatcgtgaa ctggctgaag cagagccatg gaaagaacct tgagtggatt 240
ggacttatta atccatacaa aggtcttact acctacaacc agaaattcaa gggcaaggcc 300
acattaactg tagacaagtc atccagcaca gcctacatgg agctcctcag tctgacatct 360
gaagactctg cagtctatta ctgtgcaaga tctgggtact atggtgactc ggactggtac 420
ttcgatgtct ggggcgcagg gaccacggtc accgtctcaa gcggtggcgg agggtctggg 480
ggtggcggat ccggaggtgg tggctctgca caagacatcc agatgacaca gactacatcc 540
tccctgtctg cctctctggg agacagagtc accatcagtt gcagggcaag tcaggacatt 600
cgcaattatt taaactggta tcagcagaaa ccagatggaa ctgttaaact cctgatctac 660
tacacatcaa gattacactc aggagtccca tcaaggttca gtggcagtgg gtctggaaca 720
gattattctc tcaccattgc caacctgcaa ccagaagata ttgccactta cttttgccaa 780
cagggtaata cgcttccgtg gacgttcggt ggaggcacca aactggtaac caaacgctcg 840
agcgagccca aatcttctga caaaactcac acatgcccac cgtgcccagc acctgaactc 900
ctgggtggac cgtcagtctt cctcttcccc ccaaaaccca aggacaccct catgatctcc 960
cggacccctg aggtcacatg cgtggtggtg gacgtgagcc acgaagaccc tgaggtcaag 1020
ttcaactggt acgtggacgg cgtggaggtg cataatgcca agacaaagcc gcgggaggag 1080
cagtacaaca gcacgtaccg tgtggtcagc gtcctcaccg tcctgcacca ggactggctg 1140
aatggcaagg agtacaagtg caaggtctcc aacaaagccc tcccagcccc catcgagaaa 1200
accatctcca aagccaaagg gcagccccga gaaccacagg tgtacaccct gcccccatcc 1260
cgggatgagc tgaccaagaa ccaggtcagc ctgacctgcc tggtcaaagg cttctatcca 1320

agcgacatcg ccgtggagtg ggagagcaat gggcagccgg agaacaacta caagaccacg 1380
cctcccgtgc tggactccga cggctccttc ttcctctaca gcaagctcac cgtggacaag 1440
agcaggtggc agcaggggaa cgtcttctca tgctccgtga tgcatgaggc tctgcacaac 1500
cactacacgc agaagagcct ctccctgtct ccgggtaaat ctagaactgt ggctgcacca 1560
tctgtcttca tcttcccgcc atctgatgag cagttgaaat ctggaactgc ctctgttgtg 1620
tgcctgctga attacttcta tcccagagag gccaaagtac agtggaaggt ggataacgcc 1680
ctccaatcgg gtaactccca ggagagtgcc acagagcagg acagcaagga cagcacctac 1740
agcctcagca gcgagctgac gctgagcaaa gcagactacg agaaacacaa agtctacgcc 1800
tgcgaagtca cccatcaggg cctgagctcg cccgtcacaa agagcttcaa caggggagag 1860
cagaggcaca acaattcttc cctgaataca ggaactcaga tggcaggtca ttctccgaat 1920
tctgacatcc agatgaccca gtctccatcc tccctgtctg catctgtagg agacagagtc 1980
accatcactt gtcgggcaag tcagagcatt aggaactatt taaattggta tcagcaggaa 2040
ccagggaaag cccctaagct cctgatctat gctgcatcca gtttgcaaag tggggtccca 2100
tcaaggttca gtggcagtgg atctgggaca gacttcattc tcaccatcag cagtctgcag 2160
cctgaagatt ttgcaactta ctactgtcaa cagagttacg ttaccccgct cactttcggc 2220
ggagggacca aggtggagat caaagggggt ggaggctctg gtggcggtgg ctctggcgga 2280
ggtggatccg gtggcggcgg ctctgaagtt caattgttag agtctggtgg cggtcttgtt 2340
cagcctggtg gttctttacg tctttcttgc gctgcttccg gattcacttt ctctaagtac 2400
gatatgcttt gggttcgcca agctcctggt aaaggtttgg agtgggtttc ttatatctat 2460
ccttctggtg gccttactga gtatgctgac tccgttaaag gtcgcttcac tatctctaga 2520
gacaactcta agaatactct ctacttgcag atgaacagct taagggctga ggacacggcc 2580
gtgtattact gtgcgagacg ggctccccgg tccctttctt ttgatatctg gggccaaggg 2640
acaatggtca ccgtctaagc ggccgctcta gagggcccta ttctatagtg tcacctaaat 2700
gctagag 2707

<210> 802
<211> 1812
<212> DNA
<213> Artificial Sequence

<220>
<223> TSC076 comprises: OKT3 (anti-CD3) scFv, human IgG1
      SCC-P hinge, human IgG1 CH2(ADCC/CDC null), human
      IgG1 CH3, and human Ck(YAE)

<400> 802
atggaagcac cagcgcagct tctcttcctc ctgctactct ggctcccaga taccaccggt 60
caggtccagc tgcagcagtc tggggctgaa ctggcaagac ctggggcctc agtgaagatg 120
tcctgcaagg cttctggcta cacctttact aggtacacga tgcactgggt aaaacagagg 180
cctggacagg gtctggaatg gattggatac attaatccta gccgtggtta tactaattac 240
aatcagaagt tcaaggacaa ggccacattg actacagaca aatcctccag cacagcctac 300
atgcaactga gcagcctgac atctgaggac tctgcagtct attactgtgc aagatattat 360
gatgatcatt actgccttga ctactggggc caaggcacca cggtcaccgt ctcaagcggt 420
ggcggagggt ctgggggtgg cggatccgga ggtggtggct ctgcacaaca aattgttctc 480
acccagtctc cagcaatcat gtctgcatct ccaggggaga aggtcaccat gacctgcagt 540
gccagctcaa gtgtaagtta catgaactgg taccagcaga agtcaggcac ctcccccaaa 600
agatggattt atgacacatc caaactggct tctggagtcc ctgctcactt caggggcagt 660
gggtctggga cctcttactc tctcacaatc agcggcatgg aggctgaaga tgctgccact 720
tattactgcc agcagtggag tagtaaccca ttcacgttcg gctcggggac aaagttggaa 780
ataaactcga gtgagcccaa atcttctgac aaaactcaca catgcccacc gtgcccagca 840
cctgaagccg cgggtgcacc gtcagtcttc ctcttccccc caaaacccaa ggacaccctc 900
atgatctccc ggacccctga ggtcacatgc gtggtggtgg acgtgagcca cgaagaccct 960
gaggtcaagt tcaactggta cgtggacggc gtggaggtgc ataatgccaa gacaaagccg 1020
cgggaggagc agtacaacag cacgtaccgt gtggtcagcg tcctcaccgt cctgcaccag 1080
gactggctga atggcaaggc gtacgcgtgc gcggtctcca acaaagccct cccagccccc 1140
atcgagaaaa ccatctccaa agccaaaggg cagccccgag aaccacaggt gtacaccctg 1200
cccccatccc gggatgagct gaccaagaac caggtcagcc tgacctgcct ggtcaaaggc 1260
ttctatccaa gcgacatcgc cgtggagtgg gagagcaatg ggcagccgga gaacaactac 1320
aagaccacgc ctcccgtgct ggactccgac ggctccttct tcctctacag caagctcacc 1380
gtggacaaga gcaggtggca gcaggggaac gtcttctcat gctccgtgat gcatgaggct 1440
ctgcacaacc actacacgca gaagagcctc tccctgtctc cgggtaaatc tagaactgtg 1500
gctgcaccat ctgtcttcat cttcccgcca tctgatgagc agttgaaatc tggaactgcc 1560
tctgttgtgt gcctgctgaa ttacttctat cccagagagg ccaaagtaca gtggaaggtg 1620

gataacgccc tccaatcggg taactcccag gagagtgcca cagagcagga cagcaaggac 1680
agcacctaca gcctcagcag cgagctgacg ctgagcaaag cagactacga gaaacacaaa 1740
gtctacgcct gcgaagtcac ccatcagggc ctgagctcgc ccgtcacaaa gagcttcaac 1800
aggggagagt ga 1812

<210> 803
<211> 1821
<212> DNA
<213> Artificial Sequence

<220>
<223> TSC077 comprises: HuM291 (anti-CD3) scFv, human
      IgG1 SCC-P hinge, human IgG1 CH2(ADCC/CDC null),
      human IgG1 CH3, and human Ck(YAE)

<400> 803
atggaagcac cagcgcagct tctcttcctc ctgctactct ggctcccaga taccaccggt 60
caggtccagc ttgtccagtc tggggctgaa gtcaagaaac ctggcgccag cgtgaaggtc 120
tcctgcaagg cttctggcta cacctttatt agttacacga tgcattgggt aaggcaggcc 180
cctggacagg gtctggaatg gatgggatat attaatccga gaagtgggta tactcattac 240
aatcagaagt taaaggacaa ggcaacactt accgcggaca aatccgcgag cacagcctac 300
atggaactga gcagcctgag atctgaggac accgcagtct attactgtgc aagatcggcc 360
tactatgatt atgacggctt tgcttactgg ggccaaggaa ccctggtcac cgtctcaagc 420
ggtggcggag ggtctggggg tggcggatcc ggaggtggtg gctctgcaca agatattcag 480
atgacccagt ctccatcttc cctctctgct agcgtcgggg atagggtcac cataacctgc 540
tctgccagct caagtgtaag ttacatgaac tggtaccagc agaagccagg caaagctccc 600
aagagactaa tttatgacac atccaaactg gcttctggag tcccttctag gttcagtggc 660
agtggatctg ggaccgattt caccctcaca atcagctctc tgcagccaga agatttcgcc 720
acttattact gccagcaatg gagtagtaac ccacccacgt tcggtggagg gaccaaggtg 780
gagatcaaac gaacctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat tacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 804
<211> 1821
<212> DNA
<213> Artificial Sequence

<220>
<223> TSC093 comprises from its amino to carboxy
      terminus: CRIS7 (anti-CD3) scFv, human IgG1 SCC-P
      hinge, human IgG1 CH2(ADCC/CDC null), human IgG1
      CH3, and human Ck(YAE)

<400> 804
atggaagcac cagcgcagct tctcttcctc ctgctactct ggctcccaga taccaccggt 60
caggtccagc tggtgcagtc tgggggcgga gtggtgcagc ctggccggtc actgaggctg 120
tcctgcaagg cttctggcta cacctttact agatctacga tgcactgggt aaggcaggcc 180

cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat tacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 805
<211> 1773
<212> DNA
<213> Artificial Sequence

<220>
<223> TSC094 comprises: OKT3-M (Micromet variant
      anti-CD3) scFv, human IgG1 SCC-P hinge, human IgG1
      CH2(ADCC/CDC null), human IgG1 CH3, human Ck(YAE)

<400> 805
atggaagcac cagcgcagct tctcttcctc ctgctactct ggctcccaga taccaccggt 60
gatatcaaac tgcagcagtc aggggctgaa ctggcaagac ctggggcctc agtgaagatg 120
tcctgcaaga cttctggcta cacctttact aggtacacga tgcactgggt aaaacagagg 180
cctggacagg gtctggaatg gattggatac attaatccta gccgtggtta tactaattac 240
aatcagaagt tcaaggacaa ggccacattg actacagaca aatcctccag cacagcctac 300
atgcaactga gcagcctgac atctgaggac tctgcagtct attactgtgc aagatattat 360
gatgatcatt actgccttga ctactggggc caaggcacca ctctcacagt ctcctcagtc 420
gaaggtggaa gtggaggttc tggtggaagt ggaggttcag gtggagtcga cgacattcag 480
ctgacccagt ctccagcaat catgtctgca tctccagggg agaaggtcac catgacctgc 540
agagccagtt caagtgtaag ttacatgaac tggtaccagc agaagtcagg cacctccccc 600
aaaagatgga tttatgacac atccaaagtg gcttctggag tcccttatcg cttcagtggc 660
agtgggtctg ggacctcata ctctctcaca atcagcagca tggaggctga agatgctgcc 720
acttattact gccaacagtg gagtagctcg agtgagccca aatcttctga caaaactcac 780
acatgcccac cgtgcccagc acctgaagcc gcgggtgcac cgtcagtctt cctcttcccc 840
ccaaaaccca aggacaccct catgatctcc cggacccctg aggtcacatg cgtggtggtg 900
gacgtgagcc acgaagaccc tgaggtcaag ttcaactggt acgtggacgg cgtggaggtg 960
cataatgcca agacaaagcc gcgggaggag cagtacaaca gcacgtaccg tgtggtcagc 1020
gtcctcaccg tcctgcacca ggactggctg aatggcaagg cgtacgcgtg cgcggtctcc 1080
aacaaagccc tcccagcccc catcgagaaa accatctcca aagccaaagg gcagccccga 1140
gaaccacagg tgtacaccct gcccccatcc cgggatgagc tgaccaagaa ccaggtcagc 1200
ctgacctgcc tggtcaaagg cttctatcca agcgacatcg ccgtggagtg ggagagcaat 1260
gggcagccgg agaacaacta caagaccacg cctcccgtgc tggactccga cggctccttc 1320
ttcctctaca gcaagctcac cgtggacaag agcaggtggc agcaggggaa cgtcttctca 1380

tgctccgtga tgcatgaggc tctgcacaac cactacacgc agaagagcct ctccctgtct 1440
ccgggtaaat ctagaactgt ggctgcacca tctgtcttca tcttcccgcc atctgatgag 1500
cagttgaaat ctggaactgc ctctgttgtg tgcctgctga attacttcta tcccagagag 1560
gccaaagtac agtggaaggt ggataacgcc ctccaatcgg gtaactccca ggagagtgcc 1620
acagagcagg acagcaagga cagcacctac agcctcagca gcgagctgac gctgagcaaa 1680
gcagactacg agaaacacaa agtctacgcc tgcgaagtca cccatcaggg cctgagctcg 1740
cccgtcacaa agagcttcaa caggggagag tga 1773

<210> 806
<211> 1821
<212> DNA
<213> Artificial Sequence

<220>
<223> TSC095 comprises: BMA031 (anti-TCR) scFv, human
      IgG1 SCC-P hinge, human IgG1 CH2(ADCC/CDC null),
      human IgG1 CH3, human Ck(YAE)

<400> 806
atggaagcac cagcgcagct tctcttcctc ctgctactct ggctcccaga taccaccggt 60
ggacaggtcc aactagttca gtctggagca gaggttaaga agcctgggag ctcagtgaag 120
gtttcctgca aggcttccgg atataaattc actagctatg ttatgcactg ggtgaagcag 180
gcacctggcc aggggctcga gtggattgga tatattaatc cttacaatga tgttactaag 240
tacaatgaga agttcaaagg caaggccaca cttacggccg acgagtccac taatacagcc 300
tacatggagc tgagcagcct gagatctgag gacactgcgg tccattactg tgcaagaggg 360
agctactatg attacgacgg gtttgtttac tggggccaag gtaccctggt caccgtctca 420
agcggtggcg gagggtctgg gggtggcgga tccggaggtg gtggctctgc acaagatatc 480
cagatgaccc aatctccatc gaccctgagt gcatctgtcg gggacagagt caccatgacc 540
tgcagtgcca cctcaagtgt aagttacatg cactggtatc agcagaagcc cgggaaggct 600
cccaaaagat ggatctatga cacatccaaa ctggcttctg gagtccctgc gcgcttcatt 660
ggcagtgggt ctgggaccga gttcacgctc acaatcagca gcctgcagcc agatgatttc 720
gctacgtatt actgccagca gtggagtagt aacccgctca cgttcggtgg aggtaccaag 780
gtcgagatta aatcctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat tacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 807
<211> 1836
<212> DNA
<213> Artificial Sequence

<220>
<223> TSC096 comprises: HD37 (anti-CD19) scFv, human
      IgG1 SCC-P hinge, human IgG1 CH2 (ADCC/CDC null),
        human IgG1 CH3, human Ck(YAE)

<400> 807
atggaagcac cagcgcagct tctcttcctc ctgctactct ggctcccaga taccaccggt 60

gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagtgagc ccaaatcttc tgacaaaact 840
cacacatgcc caccgtgccc agcacctgaa gccgcgggtg caccgtcagt cttcctcttc 900
cccccaaaac ccaaggacac cctcatgatc tcccggaccc ctgaggtcac atgcgtggtg 960
gtggacgtga gccacgaaga ccctgaggtc aagttcaact ggtacgtgga cggcgtggag 1020
gtgcataatg ccaagacaaa gccgcgggag gagcagtaca acagcacgta ccgtgtggtc 1080
agcgtcctca ccgtcctgca ccaggactgg ctgaatggca aggcgtacgc gtgcgcggtc 1140
tccaacaaag ccctcccagc ccccatcgag aaaaccatct ccaaagccaa agggcagccc 1200
cgagaaccac aggtgtacac cctgccccca tcccgggatg agctgaccaa gaaccaggtc 1260
agcctgacct gcctggtcaa aggcttctat ccaagcgaca tcgccgtgga gtgggagagc 1320
aatgggcagc cggagaacaa ctacaagacc acgcctcccg tgctggactc cgacggctcc 1380
ttcttcctct acagcaagct caccgtggac aagagcaggt ggcagcaggg gaacgtcttc 1440
tcatgctccg tgatgcatga ggctctgcac aaccactaca cgcagaagag cctctccctg 1500
tctccgggta aatctagaac tgtggctgca ccatctgtct tcatcttccc gccatctgat 1560
gagcagttga aatctggaac tgcctctgtt gtgtgcctgc tgaattactt ctatcccaga 1620
gaggccaaag tacagtggaa ggtggataac gccctccaat cgggtaactc ccaggagagt 1680
gccacagagc aggacagcaa ggacagcacc tacagcctca gcagcgagct gacgctgagc 1740
aaagcagact acgagaaaca caaagtctac gcctgcgaag tcacccatca gggcctgagc 1800
tcgcccgtca caaagagctt caacagggga gagtga 1836

<210> 808
<211> 1812
<212> DNA
<213> Artificial Sequence

<220>
<223> TSC097 comprises: 4C04 (anti-RON) scFv, human IgG1
      SCC-P hinge, human IgG1 CH2(ADCC/CDC null), human
      IgG1 CH3, and human Ck(YAE)

<400> 808
atggaagcac cagcgcagct tctcttcctc ctgctactct ggctcccaga taccaccggt 60
gacattcaga tgacccagtc tcctgcctcc cagtctgcat ctctgggaga aagtgtcacc 120
atcacatgcc tggcaagtca gaccattggt acatggttag catggtatca gcagaaacca 180
gggaaatctc ctcagctcct gatttatgct gcaaccagct tggcagatgg ggtcccatca 240
aggttcagtg ctagtggatc tggcacaaaa ttttctttca agatcgacag cctacagtct 300
gaagattttg taagttatta ctgtcaacaa ctttacaata ctccgtggac gttcggtgga 360
ggcaccaagc tggaaatcaa aggtggcggt ggctctggcg gaggtggatc cggtggcggc 420
ggctctcaga tccagctgca gcagtctgga cctgagctgg tgaagcctgg gacttcagtg 480
aagatatcct gcaaggcttc tgactacaca ctcactgact tctatatgaa ctgggtgagg 540
cagaagcctg gacagggact tgagtggatt gggaggattt atcctggaac cgataaaact 600
agatacaatg agaaattcag ggacaaggcc atactgaccg tggacacgtc ctccagcaca 660
gcctacatgc agctcagcag cctgacatca gaggacactg ctgtctattt ctgtgcaaga 720
tccgcctact atggtaacta cgttgctatg gactactggg gtcaaggaac ctcagtcacc 780
gtctcctcga gtgagcccaa atcttctgac aaaactcaca catgcccacc gtgcccagca 840
cctgaagccg cgggtgcacc gtcagtcttc ctcttccccc caaaacccaa ggacaccctc 900
atgatctccc ggacccctga ggtcacatgc gtggtggtgg acgtgagcca cgaagaccct 960
gaggtcaagt tcaactggta cgtggacggc gtggaggtgc ataatgccaa gacaaagccg 1020
cgggaggagc agtacaacag cacgtaccgt gtggtcagcg tcctcaccgt cctgcaccag 1080
gactggctga atggcaaggc gtacgcgtgc gcggtctcca acaaagccct cccagccccc 1140
atcgagaaaa ccatctccaa agccaaaggg cagccccgag aaccacaggt gtacaccctg 1200
cccccatccc gggatgagct gaccaagaac caggtcagcc tgacctgcct ggtcaaaggc 1260

ttctatccaa gcgacatcgc cgtggagtgg gagagcaatg ggcagccgga gaacaactac 1320
aagaccacgc ctcccgtgct ggactccgac ggctccttct tcctctacag caagctcacc 1380
gtggacaaga gcaggtggca gcaggggaac gtcttctcat gctccgtgat gcatgaggct 1440
ctgcacaacc actacacgca gaagagcctc tccctgtctc cgggtaaatc tagaactgtg 1500
gctgcaccat ctgtcttcat cttcccgcca tctgatgagc agttgaaatc tggaactgcc 1560
tctgttgtgt gcctgctgaa ttacttctat cccagagagg ccaaagtaca gtggaaggtg 1620
gataacgccc tccaatcggg taactcccag gagagtgcca cagagcagga cagcaaggac 1680
agcacctaca gcctcagcag cgagctgacg ctgagcaaag cagactacga gaaacacaaa 1740
gtctacgcct gcgaagtcac ccatcagggc ctgagctcgc ccgtcacaaa gagcttcaac 1800
aggggagagt ga 1812

<210> 809
<211> 1608
<212> DNA
<213> Artificial Sequence

<220>
<223> Bispecific T-Cell Engaging molecule (BiTE) known
      as bsc19x3

<400> 809
aagcttgccg ccatgggatg gagctgtatc atcctcttct tggtagcaac agctacaggt 60
gtccactccg actacaaaga tgatgacgat aaggatatcc agctgaccca gtctccagct 120
tctttggctg tgtctctagg gcagagggcc accatctcct gcaaggccag ccaaagtgtt 180
gattatgatg gtgatagtta tttgaactgg taccaacaga ttccaggaca gccacccaaa 240
ctcctcatct atgatgcatc caatctagtt tctgggatcc cacccaggtt tagtggcagt 300
gggtctggga cagacttcac cctcaacatc catcctgtgg agaaggtgga tgctgcaacc 360
tatcactgtc agcaaagtac tgaggatccg tggacgttcg gtggagggac caagctcgag 420
atcaaaggtg gtggtggttc tggcggcggc ggctccggtg gtggtggttc tcaggtgcag 480
ctgcagcagt ctggggctga gctggtgagg cctgggtcct cagtgaagat ttcctgcaag 540
gcttctggct atgcattcag tagctactgg atgaactggg tgaagcagag gcctggacag 600
ggtcttgagt ggattggaca gatttggcct ggagatggtg atactaacta caatggaaag 660
ttcaagggta aagccactct gactgcagac gaatcctcca gcacagccta catgcaactc 720
agcagcctag catctgagga ctctgcggtc tatttctgtg caagacggga gactacgacg 780
gtaggccgtt attactatgc tatggactac tggggccaag ggaccacggt caccgtctcc 840
tccggaggtg gtggatccga tatcaaactg cagcagtcag gggctgaact ggcaagacct 900
ggggcctcag tgaagatgtc ctgcaagact tctggctaca cctttactag gtacacgatg 960
cactgggtaa aacagaggcc tggacagggt ctggaatgga ttggatacat taatcctagc 1020
cgtggttata ctaattacaa tcagaagttc aaggacaagg ccacattgac tacagacaaa 1080
tcctccagca cagcctacat gcaactgagc agcctgacat ctgaggactc tgcagtctat 1140
tactgtgcaa gatattatga tgatcattac tgccttgact actggggcca aggcaccact 1200
ctcacagtct cctcagtcga aggtggaagt ggaggttctg gtggaagtgg aggttcaggt 1260
ggagtcgacg acattcagct gacccagtct ccagcaatca tgtctgcatc tccaggggag 1320
aaggtcacca tgacctgcag agccagttca agtgtaagtt acatgaactg gtaccagcag 1380
aagtcaggca cctcccccaa aagatggatt tatgacacat ccaaagtggc ttctggagtc 1440
ccttatcgct tcagtggcag tgggtctggg acctcatact ctctcacaat cagcagcatg 1500
gaggctgaag atgctgccac ttattactgc caacagtgga gtagtaaccc gctcacgttc 1560
ggtgctggga ccaagctgga gctgaaacat catcaccatc atcattag 1608

<210> 810
<211> 859
<212> PRT
<213> Artificial Sequence

<220>
<223> TSC038 comprises: G19-4 (anti-CD3) scFv, human
      IgG1 SCC-P hinge, human IgG1 CH2, human IgG1 CH3,
      human Ck(YAE), H75 linker and 3D1 (anti-CD86) scFv

<400> 810
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr

20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr
        195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Glu Pro Lys Ser Ser Asp Lys
                245                 250                 255
Thr His Thr Cys Pro Pro Cys Pro Ser Gly Ala Pro Glu Leu Leu Gly
            260                 265                 270
Gly Pro Ser Val Phe Leu Phe Pro Pro Lys Pro Lys Asp Thr Leu Met
        275                 280                 285
Ile Ser Arg Thr Pro Glu Val Thr Cys Val Val Val Asp Val Ser His
    290                 295                 300
Glu Asp Pro Glu Val Lys Phe Asn Trp Tyr Val Asp Gly Val Glu Val
305                 310                 315                 320
His Asn Ala Lys Thr Lys Pro Arg Glu Glu Gln Tyr Asn Ser Thr Tyr
                325                 330                 335
Arg Val Val Ser Val Leu Thr Val Leu His Gln Asp Trp Leu Asn Gly
            340                 345                 350
Lys Glu Tyr Lys Cys Lys Val Ser Asn Lys Ala Leu Pro Ala Pro Ile
        355                 360                 365
Glu Lys Thr Ile Ser Lys Ala Lys Gly Gln Pro Arg Glu Pro Gln Val
    370                 375                 380
Tyr Thr Leu Pro Pro Ser Arg Asp Glu Leu Thr Lys Asn Gln Val Ser
385                 390                 395                 400
Leu Thr Cys Leu Val Lys Gly Phe Tyr Pro Ser Asp Ile Ala Val Glu
                405                 410                 415
Trp Glu Ser Asn Gly Gln Pro Glu Asn Asn Tyr Lys Thr Thr Pro Pro
            420                 425                 430
Val Leu Asp Ser Asp Gly Ser Phe Phe Leu Tyr Ser Lys Leu Thr Val
        435                 440                 445
Asp Lys Ser Arg Trp Gln Gln Gly Asn Val Phe Ser Cys Ser Val Met
    450                 455                 460
His Glu Ala Leu His Asn His Tyr Thr Gln Lys Ser Leu Ser Leu Ser
465                 470                 475                 480
Pro Gly Lys Ser Arg Thr Val Ala Ala Pro Ser Val Phe Ile Phe Pro
                485                 490                 495
Pro Ser Asp Glu Gln Leu Lys Ser Gly Thr Ala Ser Val Val Cys Leu
            500                 505                 510
Leu Asn Tyr Phe Tyr Pro Arg Glu Ala Lys Val Gln Trp Lys Val Asp
        515                 520                 525

Asn Ala Leu Gln Ser Gly Asn Ser Gln Glu Ser Ala Thr Glu Gln Asp
    530                 535                 540
Ser Lys Asp Ser Thr Tyr Ser Leu Ser Ser Glu Leu Thr Leu Ser Lys
545                 550                 555                 560
Ala Asp Tyr Glu Lys His Lys Val Tyr Ala Cys Glu Val Thr His Gln
                565                 570                 575
Gly Leu Ser Ser Pro Val Thr Lys Ser Phe Asn Arg Gly Glu Gln Arg
            580                 585                 590
His Asn Asn Ser Ser Leu Asn Thr Gly Thr Gln Met Ala Gly His Ser
        595                 600                 605
Pro Asn Ser Gln Val Gln Leu Gln Gln Ser Gly Pro Glu Leu Val Arg
    610                 615                 620
Pro Gly Glu Ser Val Lys Ile Ser Cys Lys Gly Ser Gly Tyr Thr Phe
625                 630                 635                 640
Thr Asp Tyr Ala Ile Gln Trp Val Lys Gln Ser His Ala Lys Ser Leu
                645                 650                 655
Glu Trp Ile Gly Val Ile Asn Ile Tyr Tyr Asp Asn Thr Asn Tyr Asn
            660                 665                 670
Gln Lys Phe Lys Gly Lys Ala Thr Met Thr Val Asp Lys Ser Ser Ser
        675                 680                 685
Thr Ala Tyr Met Glu Leu Ala Arg Leu Thr Ser Glu Asp Ser Ala Ile
    690                 695                 700
Tyr Tyr Cys Ala Arg Ala Ala Trp Tyr Met Asp Tyr Trp Gly Gln Gly
705                 710                 715                 720
Thr Ser Val Thr Val Ser Ser Gly Gly Gly Gly Ser Gly Gly Gly Gly
                725                 730                 735
Ser Gly Gly Gly Gly Ser Gly Gly Gly Gly Ser Asp Ile Val Leu Ser
            740                 745                 750
Gln Ser Pro Ser Ser Leu Ala Val Ser Ala Gly Glu Lys Val Thr Met
        755                 760                 765
Ser Cys Lys Ser Ser Gln Ser Leu Leu Asn Ser Arg Thr Arg Glu Asn
    770                 775                 780
Tyr Leu Ala Trp Tyr Gln Gln Lys Pro Gly Gln Ser Pro Lys Leu Leu
785                 790                 795                 800
Ile Tyr Trp Ala Ser Thr Arg Glu Ser Gly Val Pro Asp Arg Phe Thr
                805                 810                 815
Gly Ser Gly Ser Gly Thr Asp Phe Thr Leu Thr Ile Ser Ser Val Gln
            820                 825                 830
Ala Glu Asp Leu Ala Val Tyr Tyr Cys Thr Gln Ser Tyr Asn Leu Tyr
        835                 840                 845
Thr Phe Gly Gly Gly Thr Lys Leu Glu Ile Lys
    850                 855

<210> 811
<211> 585
<212> PRT
<213> Artificial Sequence

<220>
<223> TSC039 comprises from its amino to carboxyl
      terminus: BMA031 (anti-TCR) scFv, human IgG1 SCC-P
      hinge, human IgG1 CH2, human IgG1 CH3, and human
      Ck(YAE)

<400> 811
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Lys Phe Thr Ser Tyr
            20                  25                  30
Val Met His Trp Val Lys Gln Ala Pro Gly Gln Gly Leu Glu Trp Ile
        35                  40                  45
Gly Tyr Ile Asn Pro Tyr Asn Asp Val Thr Lys Tyr Asn Glu Lys Phe

50                  55                  60
Lys Gly Lys Ala Thr Leu Thr Ala Asp Glu Ser Thr Asn Thr Ala Tyr
65                  70                  75                  80
Met Glu Leu Ser Ser Leu Arg Ser Glu Asp Thr Ala Val His Tyr Cys
                85                  90                  95
Ala Arg Gly Ser Tyr Tyr Asp Tyr Asp Gly Phe Val Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Ala Gln Asp Ile Gln Met Thr Gln Ser
    130                 135                 140
Pro Ser Thr Leu Ser Ala Ser Val Gly Asp Arg Val Thr Met Thr Cys
145                 150                 155                 160
Ser Ala Thr Ser Ser Val Ser Tyr Met His Trp Tyr Gln Gln Lys Pro
                165                 170                 175
Gly Lys Ala Pro Lys Arg Trp Ile Tyr Asp Thr Ser Lys Leu Ala Ser
            180                 185                 190
Gly Val Pro Ala Arg Phe Ile Gly Ser Gly Ser Gly Thr Glu Phe Thr
        195                 200                 205
Leu Thr Ile Ser Ser Leu Gln Pro Asp Asp Phe Ala Thr Tyr Tyr Cys
    210                 215                 220
Gln Gln Trp Ser Ser Asn Pro Leu Thr Phe Gly Gly Gly Thr Lys Val
225                 230                 235                 240
Glu Ile Lys Ser Ser Ser Glu Pro Lys Ser Ser Asp Lys Thr His Thr
                245                 250                 255
Cys Pro Pro Cys Pro Ala Pro Glu Leu Leu Gly Gly Pro Ser Val Phe
            260                 265                 270
Leu Phe Pro Pro Lys Pro Lys Asp Thr Leu Met Ile Ser Arg Thr Pro
        275                 280                 285
Glu Val Thr Cys Val Val Val Asp Val Ser His Glu Asp Pro Glu Val
    290                 295                 300
Lys Phe Asn Trp Tyr Val Asp Gly Val Glu Val His Asn Ala Lys Thr
305                 310                 315                 320
Lys Pro Arg Glu Glu Gln Tyr Asn Ser Thr Tyr Arg Val Val Ser Val
                325                 330                 335
Leu Thr Val Leu His Gln Asp Trp Leu Asn Gly Lys Glu Tyr Lys Cys
            340                 345                 350
Lys Val Ser Asn Lys Ala Leu Pro Ala Pro Ile Glu Lys Thr Ile Ser
        355                 360                 365
Lys Ala Lys Gly Gln Pro Arg Glu Pro Gln Val Tyr Thr Leu Pro Pro
    370                 375                 380
Ser Arg Asp Glu Leu Thr Lys Asn Gln Val Ser Leu Thr Cys Leu Val
385                 390                 395                 400
Lys Gly Phe Tyr Pro Ser Asp Ile Ala Val Glu Trp Glu Ser Asn Gly
                405                 410                 415
Gln Pro Glu Asn Asn Tyr Lys Thr Thr Pro Pro Val Leu Asp Ser Asp
            420                 425                 430
Gly Ser Phe Phe Leu Tyr Ser Lys Leu Thr Val Asp Lys Ser Arg Trp
        435                 440                 445
Gln Gln Gly Asn Val Phe Ser Cys Ser Val Met His Glu Ala Leu His
    450                 455                 460
Asn His Tyr Thr Gln Lys Ser Leu Ser Leu Ser Pro Gly Lys Ser Arg
465                 470                 475                 480
Thr Val Ala Ala Pro Ser Val Phe Ile Phe Pro Pro Ser Asp Glu Gln
                485                 490                 495
Leu Lys Ser Gly Thr Ala Ser Val Val Cys Leu Leu Asn Tyr Phe Tyr
            500                 505                 510
Pro Arg Glu Ala Lys Val Gln Trp Lys Val Asp Asn Ala Leu Gln Ser
        515                 520                 525
Gly Asn Ser Gln Glu Ser Ala Thr Glu Gln Asp Ser Lys Asp Ser Thr
    530                 535                 540
Tyr Ser Leu Ser Ser Glu Leu Thr Leu Ser Lys Ala Asp Tyr Glu Lys
545                 550                 555                 560

His Lys Val Tyr Ala Cys Glu Val Thr His Gln Gly Leu Ser Ser Pro
                565                 570                 575
Val Thr Lys Ser Phe Asn Arg Gly Glu
            580                 585

<210> 812
<211> 586
<212> PRT
<213> Artificial Sequence

<220>
<223> polypeptide TSC041 comprises from its amino to
      carboxy terminus: CRIS7 (anti-CD3) scFv, human
      IgG1 SCC-P hinge, human IgG1 CH2, human IgG1 CH3,
      and human Ck(YAE)

<400> 812
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Cys Pro Pro Cys Pro Ala Pro Glu Leu Leu Gly Gly Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Glu Tyr Lys
            340                 345                 350
Cys Lys Val Ser Asn Lys Ala Leu Pro Ala Pro Ile Glu Lys Thr Ile

355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Thr Val Ala Ala Pro Ser Val Phe Ile Phe Pro Pro Ser Asp Glu
                485                 490                 495
Gln Leu Lys Ser Gly Thr Ala Ser Val Val Cys Leu Leu Asn Tyr Phe
            500                 505                 510
Tyr Pro Arg Glu Ala Lys Val Gln Trp Lys Val Asp Asn Ala Leu Gln
        515                 520                 525
Ser Gly Asn Ser Gln Glu Ser Ala Thr Glu Gln Asp Ser Lys Asp Ser
    530                 535                 540
Thr Tyr Ser Leu Ser Ser Glu Leu Thr Leu Ser Lys Ala Asp Tyr Glu
545                 550                 555                 560
Lys His Lys Val Tyr Ala Cys Glu Val Thr His Gln Gly Leu Ser Ser
                565                 570                 575
Pro Val Thr Lys Ser Phe Asn Arg Gly Glu
            580                 585


<210> 813
<211> 570
<212> PRT
<213> Artificial Sequence

<220>
<223> TSC043 comprises from its amino to carboxy
      terminus: OKT3-M (Micromet variant anti-CD3, see
      also US 7,635,472) scFv, human IgG1 SCC-P hinge,
      human IgG1 CH2, human IgG1 CH3, and human Ck(YAE)

<400> 813
Asp Ile Lys Leu Gln Gln Ser Gly Ala Glu Leu Ala Arg Pro Gly Ala
1               5                   10                  15
Ser Val Lys Met Ser Cys Lys Thr Ser Gly Tyr Thr Phe Thr Arg Tyr
            20                  25                  30
Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Leu Thr Val Ser Ser Val Glu Gly Gly Ser Gly Gly Ser Gly
        115                 120                 125
Gly Ser Gly Gly Ser Gly Gly Val Asp Asp Ile Gln Leu Thr Gln Ser
    130                 135                 140
Pro Ala Ile Met Ser Ala Ser Pro Gly Glu Lys Val Thr Met Thr Cys
145                 150                 155                 160

Arg Ala Ser Ser Ser Val Ser Tyr Met Asn Trp Tyr Gln Gln Lys Ser
                165                 170                 175
Gly Thr Ser Pro Lys Arg Trp Ile Tyr Asp Thr Ser Lys Val Ala Ser
            180                 185                 190
Gly Val Pro Tyr Arg Phe Ser Gly Ser Gly Ser Gly Thr Ser Tyr Ser
        195                 200                 205
Leu Thr Ile Ser Ser Met Glu Ala Glu Asp Ala Ala Thr Tyr Tyr Cys
    210                 215                 220
Gln Gln Trp Ser Ser Ser Ser Glu Pro Lys Ser Ser Asp Lys Thr His
225                 230                 235                 240
Thr Cys Pro Pro Cys Pro Ala Pro Glu Leu Leu Gly Gly Pro Ser Val
                245                 250                 255
Phe Leu Phe Pro Pro Lys Pro Lys Asp Thr Leu Met Ile Ser Arg Thr
            260                 265                 270
Pro Glu Val Thr Cys Val Val Val Asp Val Ser His Glu Asp Pro Glu
        275                 280                 285
Val Lys Phe Asn Trp Tyr Val Asp Gly Val Glu Val His Asn Ala Lys
    290                 295                 300
Thr Lys Pro Arg Glu Glu Gln Tyr Asn Ser Thr Tyr Arg Val Val Ser
305                 310                 315                 320
Val Leu Thr Val Leu His Gln Asp Trp Leu Asn Gly Lys Glu Tyr Lys
                325                 330                 335
Cys Lys Val Ser Asn Lys Ala Leu Pro Ala Pro Ile Glu Lys Thr Ile
            340                 345                 350
Ser Lys Ala Lys Gly Gln Pro Arg Glu Pro Gln Val Tyr Thr Leu Pro
        355                 360                 365
Pro Ser Arg Asp Glu Leu Thr Lys Asn Gln Val Ser Leu Thr Cys Leu
    370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
                405                 410                 415
Asp Gly Ser Phe Phe Leu Tyr Ser Lys Leu Thr Val Asp Lys Ser Arg
            420                 425                 430
Trp Gln Gln Gly Asn Val Phe Ser Cys Ser Val Met His Glu Ala Leu
        435                 440                 445
His Asn His Tyr Thr Gln Lys Ser Leu Ser Leu Ser Pro Gly Lys Ser
    450                 455                 460
Arg Thr Val Ala Ala Pro Ser Val Phe Ile Phe Pro Pro Ser Asp Glu
465                 470                 475                 480
Gln Leu Lys Ser Gly Thr Ala Ser Val Val Cys Leu Leu Asn Tyr Phe
                485                 490                 495
Tyr Pro Arg Glu Ala Lys Val Gln Trp Lys Val Asp Asn Ala Leu Gln
            500                 505                 510
Ser Gly Asn Ser Gln Glu Ser Ala Thr Glu Gln Asp Ser Lys Asp Ser
        515                 520                 525
Thr Tyr Ser Leu Ser Ser Glu Leu Thr Leu Ser Lys Ala Asp Tyr Glu
    530                 535                 540
Lys His Lys Val Tyr Ala Cys Glu Val Thr His Gln Gly Leu Ser Ser
545                 550                 555                 560
Pro Val Thr Lys Ser Phe Asn Arg Gly Glu
                565                 570


<210> 814
<211> 584
<212> PRT
<213> Artificial Sequence

<220>
<223> TSC049 comprises from its amino to carboxy
      terminus: HD37 (anti-CD19) scFv, human IgG1 SCC-P
      hinge, human IgG1 CH2(ADCC/CDC null), human IgG1

CH3, and human IgG1 CH1

<400> 814
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480

Ser Pro Gly Lys Ser Arg Ala Ser Thr Lys Gly Pro Ser Val Phe Pro
                485                 490                 495
Leu Ala Pro Ser Ser Lys Ser Thr Ser Gly Gly Thr Ala Ala Leu Gly
            500                 505                 510
Cys Leu Val Lys Asp Tyr Phe Pro Glu Pro Val Thr Val Ser Trp Asn
        515                 520                 525
Ser Gly Ala Leu Thr Ser Gly Val His Thr Phe Pro Ala Val Leu Gln
    530                 535                 540
Ser Ser Gly Leu Tyr Ser Leu Ser Ser Val Val Thr Val Pro Ser Ser
545                 550                 555                 560
Ser Leu Gly Thr Gln Thr Tyr Ile Cys Asn Val Asn His Lys Pro Ser
                565                 570                 575
Asn Thr Lys Val Asp Lys Lys Val
            580

<210> 815
<211> 577
<212> PRT
<213> Artificial Sequence

<220>
<223> TSC050 comprises from its amino to carboxy
      terminus: 2H7 (anti-CD20) scFv, human IgG1 SCC-P
      hinge, human IgG1 CH2(ADCC/CDC null), human IgG1
      CH3, and human IgG1 CH1

<400> 815
Gln Ile Val Leu Ser Gln Ser Pro Ala Ile Leu Ser Ala Ser Pro Gly
1               5                   10                  15
Glu Lys Val Thr Met Thr Cys Arg Ala Ser Ser Ser Val Ser Tyr Met
            20                  25                  30
His Trp Tyr Gln Gln Lys Pro Gly Ser Ser Pro Lys Pro Trp Ile Tyr
        35                  40                  45
Ala Pro Ser Asn Leu Ala Ser Gly Val Pro Ala Arg Phe Ser Gly Ser
    50                  55                  60
Gly Ser Gly Thr Ser Tyr Ser Leu Thr Ile Ser Arg Val Glu Ala Glu
65                  70                  75                  80
Asp Ala Ala Thr Tyr Tyr Cys Gln Gln Trp Ser Phe Asn Pro Pro Thr
                85                  90                  95
Phe Gly Ala Gly Thr Lys Leu Glu Leu Lys Asp Gly Gly Gly Ser Gly
            100                 105                 110
Gly Gly Gly Ser Gly Gly Gly Gly Ala Ser Gln Ala Tyr Leu Gln Gln
        115                 120                 125
Ser Gly Ala Glu Ser Val Arg Pro Gly Ala Ser Val Lys Met Ser Cys
    130                 135                 140
Lys Ala Ser Gly Tyr Thr Phe Thr Ser Tyr Asn Met His Trp Val Lys
145                 150                 155                 160
Gln Thr Pro Arg Gln Gly Leu Glu Trp Ile Gly Ala Ile Tyr Pro Gly
                165                 170                 175
Asn Gly Asp Thr Ser Tyr Asn Gln Lys Phe Lys Gly Lys Ala Thr Leu
            180                 185                 190
Thr Val Asp Lys Ser Ser Ser Thr Ala Tyr Met Gln Leu Ser Ser Leu
        195                 200                 205
Thr Ser Glu Asp Ser Ala Val Tyr Phe Cys Ala Arg Val Val Tyr Tyr
    210                 215                 220
Ser Asn Ser Tyr Trp Tyr Phe Asp Val Trp Gly Thr Gly Thr Thr Val
225                 230                 235                 240
Thr Val Ser Ser Ser Glu Pro Lys Ser Ser Asp Lys Thr His Thr Cys
                245                 250                 255
Pro Pro Cys Pro Ala Pro Glu Ala Ala Gly Ala Pro Ser Val Phe Leu
            260                 265                 270
Phe Pro Pro Lys Pro Lys Asp Thr Leu Met Ile Ser Arg Thr Pro Glu

275                 280                 285
Val Thr Cys Val Val Val Asp Val Ser His Glu Asp Pro Glu Val Lys
    290                 295                 300
Phe Asn Trp Tyr Val Asp Gly Val Glu Val His Asn Ala Lys Thr Lys
305                 310                 315                 320
Pro Arg Glu Glu Gln Tyr Asn Ser Thr Tyr Arg Val Val Ser Val Leu
                325                 330                 335
Thr Val Leu His Gln Asp Trp Leu Asn Gly Lys Ala Tyr Ala Cys Ala
            340                 345                 350
Val Ser Asn Lys Ala Leu Pro Ala Pro Ile Glu Lys Thr Ile Ser Lys
        355                 360                 365
Ala Lys Gly Gln Pro Arg Glu Pro Gln Val Tyr Thr Leu Pro Pro Ser
    370                 375                 380
Arg Asp Glu Leu Thr Lys Asn Gln Val Ser Leu Thr Cys Leu Val Lys
385                 390                 395                 400
Gly Phe Tyr Pro Ser Asp Ile Ala Val Glu Trp Glu Ser Asn Gly Gln
                405                 410                 415
Pro Glu Asn Asn Tyr Lys Thr Thr Pro Pro Val Leu Asp Ser Asp Gly
            420                 425                 430
Ser Phe Phe Leu Tyr Ser Lys Leu Thr Val Asp Lys Ser Arg Trp Gln
        435                 440                 445
Gln Gly Asn Val Phe Ser Cys Ser Val Met His Glu Ala Leu His Asn
    450                 455                 460
His Tyr Thr Gln Lys Ser Leu Ser Leu Ser Pro Gly Lys Ser Arg Ala
465                 470                 475                 480
Ser Thr Lys Gly Pro Ser Val Phe Pro Leu Ala Pro Ser Ser Lys Ser
                485                 490                 495
Thr Ser Gly Gly Thr Ala Ala Leu Gly Cys Leu Val Lys Asp Tyr Phe
            500                 505                 510
Pro Glu Pro Val Thr Val Ser Trp Asn Ser Gly Ala Leu Thr Ser Gly
        515                 520                 525
Val His Thr Phe Pro Ala Val Leu Gln Ser Ser Gly Leu Tyr Ser Leu
    530                 535                 540
Ser Ser Val Val Thr Val Pro Ser Ser Ser Leu Gly Thr Gln Thr Tyr
545                 550                 555                 560
Ile Cys Asn Val Asn His Lys Pro Ser Asn Thr Lys Val Asp Lys Lys
                565                 570                 575
Val

<210> 816
<211> 573
<212> PRT
<213> Artificial Sequence

<220>
<223> TSC051 comprises from its amino to carboxy
      terminus: P2C2 (anti-CD79) scFv, human IgG1 SCC-P
      hinge, human IgG1 CH2(ADCC/CDC null), human IgG1
      CH3, and human IgG1 CH1

<400> 816
Asp Ile Val Met Ile Gln Asp Glu Leu Ser Asn Pro Val Ser Ser Gly
1               5                   10                  15
Glu Ser Val Ser Ile Ser Cys Arg Ser Ser Lys Ser Leu Leu Asp Lys
            20                  25                  30
Glu Gly Lys Thr Tyr Leu Asn Trp Phe Leu Gln Arg Pro Gly Gln Ser
        35                  40                  45
Pro Gln Leu Leu Ile Tyr Leu Met Ser Met Arg Glu Ser Gly Val Ser
    50                  55                  60
Asp Arg Phe Ser Gly Ser Gly Ser Gly Thr Asp Phe Thr Leu Glu Ile
65                  70                  75                  80

Ser Arg Val Lys Ala Glu Asp Val Gly Val Tyr Tyr Cys Gln Gln Leu
                85                  90                  95
Val Glu Tyr Pro Trp Thr Phe Gly Gly Gly Thr Lys Leu Glu Ile Lys
            100                 105                 110
Gly Gly Gly Gly Ser Gly Gly Gly Gly Ser Gly Gly Gly Gly Ser Gln
        115                 120                 125
Ile Gln Leu Val Gln Ser Gly Pro Glu Leu Lys Lys Pro Gly Glu Thr
    130                 135                 140
Val Lys Ile Ser Cys Lys Ala Ser Gly Tyr Thr Phe Thr Asp Phe Ser
145                 150                 155                 160
Met His Trp Val Arg Gln Ala Pro Gly Lys Gly Leu Lys Trp Met Gly
                165                 170                 175
Trp Ile Asn Thr Glu Thr Gly Glu Pro Thr Tyr Ala Asp Asp Phe Arg
            180                 185                 190
Gly Arg Phe Ala Phe Ser Leu Glu Thr Ser Ala Ser Thr Ala Tyr Leu
        195                 200                 205
Gln Ile Asn Asn Leu Lys Asn Glu Asp Thr Ala Thr Tyr Phe Cys Thr
    210                 215                 220
Trp Ser Ala Tyr Trp Gly Gln Gly Thr Leu Val Thr Val Ser Ala Ser
225                 230                 235                 240
Ser Glu Pro Lys Ser Ser Asp Lys Thr His Thr Cys Pro Pro Cys Pro
                245                 250                 255
Ala Pro Glu Ala Ala Gly Ala Pro Ser Val Phe Leu Phe Pro Pro Lys
            260                 265                 270
Pro Lys Asp Thr Leu Met Ile Ser Arg Thr Pro Glu Val Thr Cys Val
        275                 280                 285
Val Val Asp Val Ser His Glu Asp Pro Glu Val Lys Phe Asn Trp Tyr
    290                 295                 300
Val Asp Gly Val Glu Val His Asn Ala Lys Thr Lys Pro Arg Glu Glu
305                 310                 315                 320
Gln Tyr Asn Ser Thr Tyr Arg Val Val Ser Val Leu Thr Val Leu His
                325                 330                 335
Gln Asp Trp Leu Asn Gly Lys Ala Tyr Ala Cys Ala Val Ser Asn Lys
            340                 345                 350
Ala Leu Pro Ala Pro Ile Glu Lys Thr Ile Ser Lys Ala Lys Gly Gln
        355                 360                 365
Pro Arg Glu Pro Gln Val Tyr Thr Leu Pro Pro Ser Arg Asp Glu Leu
    370                 375                 380
Thr Lys Asn Gln Val Ser Leu Thr Cys Leu Val Lys Gly Phe Tyr Pro
385                 390                 395                 400
Ser Asp Ile Ala Val Glu Trp Glu Ser Asn Gly Gln Pro Glu Asn Asn
                405                 410                 415
Tyr Lys Thr Thr Pro Pro Val Leu Asp Ser Asp Gly Ser Phe Phe Leu
            420                 425                 430
Tyr Ser Lys Leu Thr Val Asp Lys Ser Arg Trp Gln Gln Gly Asn Val
        435                 440                 445
Phe Ser Cys Ser Val Met His Glu Ala Leu His Asn His Tyr Thr Gln
    450                 455                 460
Lys Ser Leu Ser Leu Ser Pro Gly Lys Ser Arg Ala Ser Thr Lys Gly
465                 470                 475                 480
Pro Ser Val Phe Pro Leu Ala Pro Ser Ser Lys Ser Thr Ser Gly Gly
                485                 490                 495
Thr Ala Ala Leu Gly Cys Leu Val Lys Asp Tyr Phe Pro Glu Pro Val
            500                 505                 510
Thr Val Ser Trp Asn Ser Gly Ala Leu Thr Ser Gly Val His Thr Phe
        515                 520                 525
Pro Ala Val Leu Gln Ser Ser Gly Leu Tyr Ser Leu Ser Ser Val Val
    530                 535                 540
Thr Val Pro Ser Ser Ser Leu Gly Thr Gln Thr Tyr Ile Cys Asn Val
545                 550                 555                 560
Asn His Lys Pro Ser Asn Thr Lys Val Asp Lys Lys Val
                565                 570

<210> 817
<211> 580
<212> PRT
<213> Artificial Sequence

<220>
<223> TSC052 comprises from its amino to carboxy
      terminus: 5D5 (anti-cMet) scFv, human IgG1 SCC-P
      hinge, human IgG1 CH2(ADCC/CDC null), human IgG1
      CH3, and human IgG1 CH1

<400> 817
Asp Phe Met Met Ser Gln Ser Pro Ser Ser Leu Thr Val Ser Val Gly
1               5                   10                  15
Glu Lys Val Thr Val Ser Cys Lys Ser Ser Gln Ser Leu Leu Tyr Thr
            20                  25                  30
Ser Ser Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Thr Ser Val Lys Ala Asp Asp Leu Ala Val Tyr Tyr Cys Gln Gln
                85                  90                  95
Tyr Tyr Ala Tyr Pro Trp Thr Phe Gly Gly Gly Thr Lys Leu Glu Leu
            100                 105                 110
Lys Gly Gly Gly Gly Ser Gly Gly Gly Gly Ser Gly Gly Gly Gly Ser
        115                 120                 125
Gln Val Gln Leu Gln Gln Ser Gly Pro Glu Leu Val Arg Pro Gly Ala
    130                 135                 140
Ser Val Lys Met Ser Cys Arg Ala Ser Gly Tyr Thr Phe Thr Ser Tyr
145                 150                 155                 160
Trp Leu His Trp Val Lys Gln Arg Pro Gly Gln Gly Leu Glu Trp Ile
                165                 170                 175
Gly Met Ile Asp Pro Ser Asn Ser Asp Thr Arg Phe Asn Pro Asn Phe
            180                 185                 190
Lys Asp Lys Ala Thr Leu Asn Val Asp Arg Ser Ser Asn Thr Ala Tyr
        195                 200                 205
Met Leu Leu Ser Ser Leu Thr Ser Ala Asp Ser Ala Val Tyr Tyr Cys
    210                 215                 220
Ala Thr Tyr Gly Ser Tyr Val Ser Pro Leu Asp Tyr Trp Gly Gln Gly
225                 230                 235                 240
Thr Ser Val Thr Val Ser Ser Ser Glu Pro Lys Ser Ser Asp Lys Thr
                245                 250                 255
His Thr Cys Pro Pro Cys Pro Ala Pro Glu Ala Ala Gly Ala Pro Ser
            260                 265                 270
Val Phe Leu Phe Pro Pro Lys Pro Lys Asp Thr Leu Met Ile Ser Arg
        275                 280                 285
Thr Pro Glu Val Thr Cys Val Val Val Asp Val Ser His Glu Asp Pro
    290                 295                 300
Glu Val Lys Phe Asn Trp Tyr Val Asp Gly Val Glu Val His Asn Ala
305                 310                 315                 320
Lys Thr Lys Pro Arg Glu Glu Gln Tyr Asn Ser Thr Tyr Arg Val Val
                325                 330                 335
Ser Val Leu Thr Val Leu His Gln Asp Trp Leu Asn Gly Lys Ala Tyr
            340                 345                 350
Ala Cys Ala Val Ser Asn Lys Ala Leu Pro Ala Pro Ile Glu Lys Thr
        355                 360                 365
Ile Ser Lys Ala Lys Gly Gln Pro Arg Glu Pro Gln Val Tyr Thr Leu
    370                 375                 380
Pro Pro Ser Arg Asp Glu Leu Thr Lys Asn Gln Val Ser Leu Thr Cys
385                 390                 395                 400

Leu Val Lys Gly Phe Tyr Pro Ser Asp Ile Ala Val Glu Trp Glu Ser
                405                 410                 415
Asn Gly Gln Pro Glu Asn Asn Tyr Lys Thr Thr Pro Pro Val Leu Asp
            420                 425                 430
Ser Asp Gly Ser Phe Phe Leu Tyr Ser Lys Leu Thr Val Asp Lys Ser
        435                 440                 445
Arg Trp Gln Gln Gly Asn Val Phe Ser Cys Ser Val Met His Glu Ala
    450                 455                 460
Leu His Asn His Tyr Thr Gln Lys Ser Leu Ser Leu Ser Pro Gly Lys
465                 470                 475                 480
Ser Arg Ala Ser Thr Lys Gly Pro Ser Val Phe Pro Leu Ala Pro Ser
                485                 490                 495
Ser Lys Ser Thr Ser Gly Gly Thr Ala Ala Leu Gly Cys Leu Val Lys
            500                 505                 510
Asp Tyr Phe Pro Glu Pro Val Thr Val Ser Trp Asn Ser Gly Ala Leu
        515                 520                 525
Thr Ser Gly Val His Thr Phe Pro Ala Val Leu Gln Ser Ser Gly Leu
    530                 535                 540
Tyr Ser Leu Ser Ser Val Val Thr Val Pro Ser Ser Ser Leu Gly Thr
545                 550                 555                 560
Gln Thr Tyr Ile Cys Asn Val Asn His Lys Pro Ser Asn Thr Lys Val
                565                 570                 575
Asp Lys Lys Val
            580


<210> 818
<211> 588
<212> PRT
<213> Artificial Sequence

<220>
<223> TSC053 comprises from its amino to carboxy
      terminus: G19-4 (anti CD3) scFv, human IgG1 SCC-P
      hinge, human IgG1 CH2(ADCC/CDC null), human IgG1
      CH3, and human Ck(YAE)

<400> 818
Glu Val Gln Leu Gln Gln Ser Gly Pro Glu Leu Val Lys Pro Gly Ala
1               5                   10                  15
Ser Met Lys Ile Ser Cys Lys Ala Ser Gly Tyr Ser Phe Thr Gly Tyr
            20                  25                  30
Ile Val Asn Trp Leu Lys Gln Ser His Gly Lys Asn Leu Glu Trp Ile
        35                  40                  45
Gly Leu Ile Asn Pro Tyr Lys Gly Leu Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Leu Ser Leu Thr Ser Glu Asp Ser Ala Val Tyr Tyr Cys
                85                  90                  95
Ala Arg Ser Gly Tyr Tyr Gly Asp Ser Asp Trp Tyr Phe Asp Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Ala Gln Asp Ile Gln Met Thr
    130                 135                 140
Gln Thr Thr Ser Ser Leu Ser Ala Ser Leu Gly Asp Arg Val Thr Ile
145                 150                 155                 160
Ser Cys Arg Ala Ser Gln Asp Ile Arg Asn Tyr Leu Asn Trp Tyr Gln
                165                 170                 175
Gln Lys Pro Asp Gly Thr Val Lys Leu Leu Ile Tyr Tyr Thr Ser Arg
            180                 185                 190
Leu His Ser Gly Val Pro Ser Arg Phe Ser Gly Ser Gly Ser Gly Thr

195                 200                 205
Asp Tyr Ser Leu Thr Ile Ala Asn Leu Gln Pro Glu Asp Ile Ala Thr
    210                 215                 220
Tyr Phe Cys Gln Gln Gly Asn Thr Leu Pro Trp Thr Phe Gly Gly Gly
225                 230                 235                 240
Thr Lys Leu Val Thr Lys Arg Ser Ser Glu Pro Lys Ser Ser Asp Lys
                245                 250                 255
Thr His Thr Cys Pro Pro Cys Pro Ala Pro Glu Ala Ala Gly Ala Pro
            260                 265                 270
Ser Val Phe Leu Phe Pro Pro Lys Pro Lys Asp Thr Leu Met Ile Ser
        275                 280                 285
Arg Thr Pro Glu Val Thr Cys Val Val Val Asp Val Ser His Glu Asp
    290                 295                 300
Pro Glu Val Lys Phe Asn Trp Tyr Val Asp Gly Val Glu Val His Asn
305                 310                 315                 320
Ala Lys Thr Lys Pro Arg Glu Glu Gln Tyr Asn Ser Thr Tyr Arg Val
                325                 330                 335
Val Ser Val Leu Thr Val Leu His Gln Asp Trp Leu Asn Gly Lys Ala
            340                 345                 350
Tyr Ala Cys Ala Val Ser Asn Lys Ala Leu Pro Ala Pro Ile Glu Lys
        355                 360                 365
Thr Ile Ser Lys Ala Lys Gly Gln Pro Arg Glu Pro Gln Val Tyr Thr
    370                 375                 380
Leu Pro Pro Ser Arg Asp Glu Leu Thr Lys Asn Gln Val Ser Leu Thr
385                 390                 395                 400
Cys Leu Val Lys Gly Phe Tyr Pro Ser Asp Ile Ala Val Glu Trp Glu
                405                 410                 415
Ser Asn Gly Gln Pro Glu Asn Asn Tyr Lys Thr Thr Pro Pro Val Leu
            420                 425                 430
Asp Ser Asp Gly Ser Phe Phe Leu Tyr Ser Lys Leu Thr Val Asp Lys
        435                 440                 445
Ser Arg Trp Gln Gln Gly Asn Val Phe Ser Cys Ser Val Met His Glu
    450                 455                 460
Ala Leu His Asn His Tyr Thr Gln Lys Ser Leu Ser Leu Ser Pro Gly
465                 470                 475                 480
Lys Ser Arg Thr Val Ala Ala Pro Ser Val Phe Ile Phe Pro Pro Ser
                485                 490                 495
Asp Glu Gln Leu Lys Ser Gly Thr Ala Ser Val Val Cys Leu Leu Asn
            500                 505                 510
Tyr Phe Tyr Pro Arg Glu Ala Lys Val Gln Trp Lys Val Asp Asn Ala
        515                 520                 525
Leu Gln Ser Gly Asn Ser Gln Glu Ser Ala Thr Glu Gln Asp Ser Lys
    530                 535                 540
Asp Ser Thr Tyr Ser Leu Ser Ser Glu Leu Thr Leu Ser Lys Ala Asp
545                 550                 555                 560
Tyr Glu Lys His Lys Val Tyr Ala Cys Glu Val Thr His Gln Gly Leu
                565                 570                 575
Ser Ser Pro Val Thr Lys Ser Phe Asn Arg Gly Glu
            580                 585

<210> 819
<211> 853
<212> PRT
<213> Artificial Sequence

<220>
<223> TSC064 comprises from its amino to carboxy
      terminus: G19-4 (anti-CD3) scFv, human IgG1 SCC-P
      hinge, human IgG1 CH2, human IgG1 CH3, human
      Ck(YAE), H75 linker, and Met021 (anti-c-Met) scFv

<400> 819

<210> 820
<211> 583
<212> PRT
<213> Artificial Sequence

<220>
<223> TSC076 comprises from its amino to carboxy
      terminus: OKT3 (anti-CD3) scFv, human IgG1 SCC-P
      hinge, human IgG1 CH2(ADCC/CDC null), human IgG1
      CH3, and human Ck(YAE)

<400> 820
Gln Val Gln Leu Gln Gln Ser Gly Ala Glu Leu Ala Arg Pro Gly Ala
1               5                   10                  15
Ser Val Lys Met Ser Cys Lys Ala Ser Gly Tyr Thr Phe Thr Arg Tyr
            20                  25                  30

Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Val Thr Val Ser Ser Gly Gly Gly Gly Ser Gly Gly Gly Gly
        115                 120                 125
Ser Gly Gly Gly Gly Ser Ala Gln Gln Ile Val Leu Thr Gln Ser Pro
    130                 135                 140
Ala Ile Met Ser Ala Ser Pro Gly Glu Lys Val Thr Met Thr Cys Ser
145                 150                 155                 160
Ala Ser Ser Ser Val Ser Tyr Met Asn Trp Tyr Gln Gln Lys Ser Gly
                165                 170                 175
Thr Ser Pro Lys Arg Trp Ile Tyr Asp Thr Ser Lys Leu Ala Ser Gly
            180                 185                 190
Val Pro Ala His Phe Arg Gly Ser Gly Ser Gly Thr Ser Tyr Ser Leu
        195                 200                 205
Thr Ile Ser Gly Met Glu Ala Glu Asp Ala Ala Thr Tyr Tyr Cys Gln
    210                 215                 220
Gln Trp Ser Ser Asn Pro Phe Thr Phe Gly Ser Gly Thr Lys Leu Glu
225                 230                 235                 240
Ile Asn Ser Ser Glu Pro Lys Ser Ser Asp Lys Thr His Thr Cys Pro
                245                 250                 255
Pro Cys Pro Ala Pro Glu Ala Ala Gly Ala Pro Ser Val Phe Leu Phe
            260                 265                 270
Pro Pro Lys Pro Lys Asp Thr Leu Met Ile Ser Arg Thr Pro Glu Val
        275                 280                 285
Thr Cys Val Val Val Asp Val Ser His Glu Asp Pro Glu Val Lys Phe
    290                 295                 300
Asn Trp Tyr Val Asp Gly Val Glu Val His Asn Ala Lys Thr Lys Pro
305                 310                 315                 320
Arg Glu Glu Gln Tyr Asn Ser Thr Tyr Arg Val Val Ser Val Leu Thr
                325                 330                 335
Val Leu His Gln Asp Trp Leu Asn Gly Lys Ala Tyr Ala Cys Ala Val
            340                 345                 350
Ser Asn Lys Ala Leu Pro Ala Pro Ile Glu Lys Thr Ile Ser Lys Ala
        355                 360                 365
Lys Gly Gln Pro Arg Glu Pro Gln Val Tyr Thr Leu Pro Pro Ser Arg
    370                 375                 380
Asp Glu Leu Thr Lys Asn Gln Val Ser Leu Thr Cys Leu Val Lys Gly
385                 390                 395                 400
Phe Tyr Pro Ser Asp Ile Ala Val Glu Trp Glu Ser Asn Gly Gln Pro
                405                 410                 415
Glu Asn Asn Tyr Lys Thr Thr Pro Pro Val Leu Asp Ser Asp Gly Ser
            420                 425                 430
Phe Phe Leu Tyr Ser Lys Leu Thr Val Asp Lys Ser Arg Trp Gln Gln
        435                 440                 445
Gly Asn Val Phe Ser Cys Ser Val Met His Glu Ala Leu His Asn His
    450                 455                 460
Tyr Thr Gln Lys Ser Leu Ser Leu Ser Pro Gly Lys Ser Arg Thr Val
465                 470                 475                 480
Ala Ala Pro Ser Val Phe Ile Phe Pro Pro Ser Asp Glu Gln Leu Lys
                485                 490                 495
Ser Gly Thr Ala Ser Val Val Cys Leu Leu Asn Tyr Phe Tyr Pro Arg
            500                 505                 510
Glu Ala Lys Val Gln Trp Lys Val Asp Asn Ala Leu Gln Ser Gly Asn
        515                 520                 525
Ser Gln Glu Ser Ala Thr Glu Gln Asp Ser Lys Asp Ser Thr Tyr Ser

530                 535                 540
Leu Ser Ser Glu Leu Thr Leu Ser Lys Ala Asp Tyr Glu Lys His Lys
545                 550                 555                 560
Val Tyr Ala Cys Glu Val Thr His Gln Gly Leu Ser Ser Pro Val Thr
                565                 570                 575
Lys Ser Phe Asn Arg Gly Glu
            580

<210> 821
<211> 586
<212> PRT
<213> Artificial Sequence

<220>
<223> TSC077 comprises: HuM291 (anti-CD3) scFv, human
      IgG1 SCC-P hinge, human IgG1 CH2(ADCC/CDC null),
      human IgG1 CH3, and human Ck(YAE)

<400> 821
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Ile Ser Tyr
            20                  25                  30
Thr Met His Trp Val Arg Gln Ala Pro Gly Gln Gly Leu Glu Trp Met
        35                  40                  45
Gly Tyr Ile Asn Pro Arg Ser Gly Tyr Thr His Tyr Asn Gln Lys Leu
    50                  55                  60
Lys Asp Lys Ala Thr Leu Thr Ala Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ala Tyr Tyr Asp Tyr Asp Gly Phe Ala Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Ala Gln Asp Ile Gln Met Thr Gln Ser
    130                 135                 140
Pro Ser Ser Leu Ser Ala Ser Val Gly Asp Arg Val Thr Ile Thr Cys
145                 150                 155                 160
Ser Ala Ser Ser Ser Val Ser Tyr Met Asn Trp Tyr Gln Gln Lys Pro
                165                 170                 175
Gly Lys Ala Pro Lys Arg Leu Ile Tyr Asp Thr Ser Lys Leu Ala Ser
            180                 185                 190
Gly Val Pro Ser Arg Phe Ser Gly Ser Gly Ser Gly Thr Asp Phe Thr
        195                 200                 205
Leu Thr Ile Ser Ser Leu Gln Pro Glu Asp Phe Ala Thr Tyr Tyr Cys
    210                 215                 220
Gln Gln Trp Ser Ser Asn Pro Pro Thr Phe Gly Gly Gly Thr Lys Val
225                 230                 235                 240
Glu Ile Lys Arg Thr Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala

340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Thr Val Ala Ala Pro Ser Val Phe Ile Phe Pro Pro Ser Asp Glu
                485                 490                 495
Gln Leu Lys Ser Gly Thr Ala Ser Val Val Cys Leu Leu Asn Tyr Phe
            500                 505                 510
Tyr Pro Arg Glu Ala Lys Val Gln Trp Lys Val Asp Asn Ala Leu Gln
        515                 520                 525
Ser Gly Asn Ser Gln Glu Ser Ala Thr Glu Gln Asp Ser Lys Asp Ser
    530                 535                 540
Thr Tyr Ser Leu Ser Ser Glu Leu Thr Leu Ser Lys Ala Asp Tyr Glu
545                 550                 555                 560
Lys His Lys Val Tyr Ala Cys Glu Val Thr His Gln Gly Leu Ser Ser
                565                 570                 575
Pro Val Thr Lys Ser Phe Asn Arg Gly Glu
            580                 585

<210> 822
<211> 586
<212> PRT
<213> Artificial Sequence

<220>
<223> TSC093 comprises from its amino to carboxy
      terminus: CRIS7 (anti-CD3) scFv, human IgG1 SCC-P
      hinge, human IgG1 CH2(ADCC/CDC null), human IgG1
      CH3, and human Ck(YAE)

<400> 822
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140

Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Thr Val Ala Ala Pro Ser Val Phe Ile Phe Pro Pro Ser Asp Glu
                485                 490                 495
Gln Leu Lys Ser Gly Thr Ala Ser Val Val Cys Leu Leu Asn Tyr Phe
            500                 505                 510
Tyr Pro Arg Glu Ala Lys Val Gln Trp Lys Val Asp Asn Ala Leu Gln
        515                 520                 525
Ser Gly Asn Ser Gln Glu Ser Ala Thr Glu Gln Asp Ser Lys Asp Ser
    530                 535                 540
Thr Tyr Ser Leu Ser Ser Glu Leu Thr Leu Ser Lys Ala Asp Tyr Glu
545                 550                 555                 560
Lys His Lys Val Tyr Ala Cys Glu Val Thr His Gln Gly Leu Ser Ser
                565                 570                 575
Pro Val Thr Lys Ser Phe Asn Arg Gly Glu
            580                 585

<210> 823
<211> 570
<212> PRT
<213> Artificial Sequence

<220>
<223> TSC094 comprises from its amino to carboxy
      terminus: OKT3-M (Micromet variant anti-CD3) scFv,
      human IgG1 SCC-P hinge, human IgG1 CH2(ADCC/CDC
      null), human IgG1 CH3, human Ck(YAE)

<400> 823
Asp Ile Lys Leu Gln Gln Ser Gly Ala Glu Leu Ala Arg Pro Gly Ala
1               5                   10                  15
Ser Val Lys Met Ser Cys Lys Thr Ser Gly Tyr Thr Phe Thr Arg Tyr
            20                  25                  30
Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Leu Thr Val Ser Ser Val Glu Gly Gly Ser Gly Gly Ser Gly
        115                 120                 125
Gly Ser Gly Gly Ser Gly Gly Val Asp Asp Ile Gln Leu Thr Gln Ser
    130                 135                 140
Pro Ala Ile Met Ser Ala Ser Pro Gly Glu Lys Val Thr Met Thr Cys
145                 150                 155                 160
Arg Ala Ser Ser Ser Val Ser Tyr Met Asn Trp Tyr Gln Gln Lys Ser
                165                 170                 175
Gly Thr Ser Pro Lys Arg Trp Ile Tyr Asp Thr Ser Lys Val Ala Ser
            180                 185                 190
Gly Val Pro Tyr Arg Phe Ser Gly Ser Gly Ser Gly Thr Ser Tyr Ser
        195                 200                 205
Leu Thr Ile Ser Ser Met Glu Ala Glu Asp Ala Ala Thr Tyr Tyr Cys
    210                 215                 220
Gln Gln Trp Ser Ser Ser Ser Glu Pro Lys Ser Ser Asp Lys Thr His
225                 230                 235                 240
Thr Cys Pro Pro Cys Pro Ala Pro Glu Ala Ala Gly Ala Pro Ser Val
                245                 250                 255
Phe Leu Phe Pro Pro Lys Pro Lys Asp Thr Leu Met Ile Ser Arg Thr
            260                 265                 270
Pro Glu Val Thr Cys Val Val Val Asp Val Ser His Glu Asp Pro Glu
        275                 280                 285
Val Lys Phe Asn Trp Tyr Val Asp Gly Val Glu Val His Asn Ala Lys
    290                 295                 300
Thr Lys Pro Arg Glu Glu Gln Tyr Asn Ser Thr Tyr Arg Val Val Ser
305                 310                 315                 320
Val Leu Thr Val Leu His Gln Asp Trp Leu Asn Gly Lys Ala Tyr Ala
                325                 330                 335
Cys Ala Val Ser Asn Lys Ala Leu Pro Ala Pro Ile Glu Lys Thr Ile
            340                 345                 350
Ser Lys Ala Lys Gly Gln Pro Arg Glu Pro Gln Val Tyr Thr Leu Pro
        355                 360                 365
Pro Ser Arg Asp Glu Leu Thr Lys Asn Gln Val Ser Leu Thr Cys Leu
    370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
                405                 410                 415
Asp Gly Ser Phe Phe Leu Tyr Ser Lys Leu Thr Val Asp Lys Ser Arg
            420                 425                 430
Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys Ser
    450                 455                 460
Arg Thr Val Ala Ala Pro Ser Val Phe Ile Phe Pro Pro Ser Asp Glu
465                 470                 475                 480
Gln Leu Lys Ser Gly Thr Ala Ser Val Val Cys Leu Leu Asn Tyr Phe
                485                 490                 495
Tyr Pro Arg Glu Ala Lys Val Gln Trp Lys Val Asp Asn Ala Leu Gln
            500                 505                 510
Ser Gly Asn Ser Gln Glu Ser Ala Thr Glu Gln Asp Ser Lys Asp Ser
        515                 520                 525
Thr Tyr Ser Leu Ser Ser Glu Leu Thr Leu Ser Lys Ala Asp Tyr Glu
    530                 535                 540
Lys His Lys Val Tyr Ala Cys Glu Val Thr His Gln Gly Leu Ser Ser
545                 550                 555                 560
Pro Val Thr Lys Ser Phe Asn Arg Gly Glu
                565                 570


<210> 824
<211> 586
<212> PRT
<213> Artificial Sequence

<220>
<223> TSC095 comprises from its amino to carboxy
      terminus: BMA031 (anti-TCR) scFv, human IgG1 SCC-P
      hinge, human IgG1 CH2(ADCC/CDC null), human IgG1
      CH3, human Ck(YAE)

<400> 824
Gly Gln Val Gln Leu Val Gln Ser Gly Ala Glu Val Lys Lys Pro Gly
1               5                   10                  15
Ser Ser Val Lys Val Ser Cys Lys Ala Ser Gly Tyr Lys Phe Thr Ser
            20                  25                  30
Tyr Val Met His Trp Val Lys Gln Ala Pro Gly Gln Gly Leu Glu Trp
        35                  40                  45
Ile Gly Tyr Ile Asn Pro Tyr Asn Asp Val Thr Lys Tyr Asn Glu Lys
    50                  55                  60
Phe Lys Gly Lys Ala Thr Leu Thr Ala Asp Glu Ser Thr Asn Thr Ala
65                  70                  75                  80
Tyr Met Glu Leu Ser Ser Leu Arg Ser Glu Asp Thr Ala Val His Tyr
                85                  90                  95
Cys Ala Arg Gly Ser Tyr Tyr Asp Tyr Asp Gly Phe Val Tyr Trp Gly
            100                 105                 110
Gln Gly Thr Leu Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Thr Leu Ser Ala Ser Val Gly Asp Arg Val Thr Met Thr
145                 150                 155                 160
Cys Ser Ala Thr Ser Ser Val Ser Tyr Met His Trp Tyr Gln Gln Lys
                165                 170                 175
Pro Gly Lys Ala Pro Lys Arg Trp Ile Tyr Asp Thr Ser Lys Leu Ala
            180                 185                 190
Ser Gly Val Pro Ala Arg Phe Ile Gly Ser Gly Ser Gly Thr Glu Phe
        195                 200                 205
Thr Leu Thr Ile Ser Ser Leu Gln Pro Asp Asp Phe Ala Thr Tyr Tyr
    210                 215                 220
Cys Gln Gln Trp Ser Ser Asn Pro Leu Thr Phe Gly Gly Gly Thr Lys
225                 230                 235                 240
Val Glu Ile Lys Ser Ser Ser Glu Pro Lys Ser Ser Asp Lys Thr His
                245                 250                 255
Thr Cys Pro Pro Cys Pro Ala Pro Glu Ala Ala Gly Ala Pro Ser Val

260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Thr Val Ala Ala Pro Ser Val Phe Ile Phe Pro Pro Ser Asp Glu
                485                 490                 495
Gln Leu Lys Ser Gly Thr Ala Ser Val Val Cys Leu Leu Asn Tyr Phe
            500                 505                 510
Tyr Pro Arg Glu Ala Lys Val Gln Trp Lys Val Asp Asn Ala Leu Gln
        515                 520                 525
Ser Gly Asn Ser Gln Glu Ser Ala Thr Glu Gln Asp Ser Lys Asp Ser
    530                 535                 540
Thr Tyr Ser Leu Ser Ser Glu Leu Thr Leu Ser Lys Ala Asp Tyr Glu
545                 550                 555                 560
Lys His Lys Val Tyr Ala Cys Glu Val Thr His Gln Gly Leu Ser Ser
                565                 570                 575
Pro Val Thr Lys Ser Phe Asn Arg Gly Glu
            580                 585

<210> 825
<211> 591
<212> PRT
<213> Artificial Sequence

<220>
<223> TSC096 comprises: HD37 (anti-CD19) scFv, human
      IgG1 SCC-P hinge, human IgG1 CH2 (ADCC/CDC null),
        human IgG1 CH3, human Ck(YAE)

<400> 825
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His

65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Lys Ser Arg Thr Val Ala Ala Pro Ser Val Phe Ile Phe
                485                 490                 495
Pro Pro Ser Asp Glu Gln Leu Lys Ser Gly Thr Ala Ser Val Val Cys
            500                 505                 510
Leu Leu Asn Tyr Phe Tyr Pro Arg Glu Ala Lys Val Gln Trp Lys Val
        515                 520                 525
Asp Asn Ala Leu Gln Ser Gly Asn Ser Gln Glu Ser Ala Thr Glu Gln
    530                 535                 540
Asp Ser Lys Asp Ser Thr Tyr Ser Leu Ser Ser Glu Leu Thr Leu Ser
545                 550                 555                 560
Lys Ala Asp Tyr Glu Lys His Lys Val Tyr Ala Cys Glu Val Thr His
                565                 570                 575

Gln Gly Leu Ser Ser Pro Val Thr Lys Ser Phe Asn Arg Gly Glu
            580                 585                 590

<210> 826
<211> 583
<212> PRT
<213> Artificial Sequence

<220>
<223> TSC097 comprises from its amino to carboxy
      terminus: 4C04 (anti-RON) scFv, human IgG1 SCC-P
      hinge, human IgG1 CH2(ADCC/CDC null), human IgG1
      CH3, and human Ck(YAE)

<400> 826


<210> 827
<211> 512
<212> PRT
<213> Artificial Sequence

<220>
<223> Bispecific T-Cell Engaging molecule (BiTE) known
      as bsc19x3

<400> 827
Asp Tyr Lys Asp Asp Asp Asp Lys Asp Ile Gln Leu Thr Gln Ser Pro
1               5                   10                  15
Ala Ser Leu Ala Val Ser Leu Gly Gln Arg Ala Thr Ile Ser Cys Lys
            20                  25                  30
Ala Ser Gln Ser Val Asp Tyr Asp Gly Asp Ser Tyr Leu Asn Trp Tyr
        35                  40                  45
Gln Gln Ile Pro Gly Gln Pro Pro Lys Leu Leu Ile Tyr Asp Ala Ser
    50                  55                  60
Asn Leu Val Ser Gly Ile Pro Pro Arg Phe Ser Gly Ser Gly Ser Gly
65                  70                  75                  80
Thr Asp Phe Thr Leu Asn Ile His Pro Val Glu Lys Val Asp Ala Ala
                85                  90                  95
Thr Tyr His Cys Gln Gln Ser Thr Glu Asp Pro Trp Thr Phe Gly Gly
            100                 105                 110
Gly Thr Lys Leu Glu Ile Lys Gly Gly Gly Gly Ser Gly Gly Gly Gly
        115                 120                 125
Ser Gly Gly Gly Gly Ser Gln Val Gln Leu Gln Gln Ser Gly Ala Glu
    130                 135                 140
Leu Val Arg Pro Gly Ser Ser Val Lys Ile Ser Cys Lys Ala Ser Gly
145                 150                 155                 160
Tyr Ala Phe Ser Ser Tyr Trp Met Asn Trp Val Lys Gln Arg Pro Gly
                165                 170                 175
Gln Gly Leu Glu Trp Ile Gly Gln Ile Trp Pro Gly Asp Gly Asp Thr
            180                 185                 190

Asn Tyr Asn Gly Lys Phe Lys Gly Lys Ala Thr Leu Thr Ala Asp Glu
        195                 200                 205
Ser Ser Ser Thr Ala Tyr Met Gln Leu Ser Ser Leu Ala Ser Glu Asp
    210                 215                 220
Ser Ala Val Tyr Phe Cys Ala Arg Arg Glu Thr Thr Thr Val Gly Arg
225                 230                 235                 240
Tyr Tyr Tyr Ala Met Asp Tyr Trp Gly Gln Gly Thr Thr Val Thr Val
                245                 250                 255
Ser Ser Gly Gly Gly Gly Ser Asp Ile Lys Leu Gln Gln Ser Gly Ala
            260                 265                 270
Glu Leu Ala Arg Pro Gly Ala Ser Val Lys Met Ser Cys Lys Thr Ser
        275                 280                 285
Gly Tyr Thr Phe Thr Arg Tyr Thr Met His Trp Val Lys Gln Arg Pro
    290                 295                 300
Gly Gln Gly Leu Glu Trp Ile Gly Tyr Ile Asn Pro Ser Arg Gly Tyr
305                 310                 315                 320
Thr Asn Tyr Asn Gln Lys Phe Lys Asp Lys Ala Thr Leu Thr Thr Asp
                325                 330                 335
Lys Ser Ser Ser Thr Ala Tyr Met Gln Leu Ser Ser Leu Thr Ser Glu
            340                 345                 350
Asp Ser Ala Val Tyr Tyr Cys Ala Arg Tyr Tyr Asp Asp His Tyr Cys
        355                 360                 365
Leu Asp Tyr Trp Gly Gln Gly Thr Thr Leu Thr Val Ser Ser Val Glu
    370                 375                 380
Gly Gly Ser Gly Gly Ser Gly Gly Ser Gly Gly Ser Gly Gly Val Asp
385                 390                 395                 400
Asp Ile Gln Leu Thr Gln Ser Pro Ala Ile Met Ser Ala Ser Pro Gly
                405                 410                 415
Glu Lys Val Thr Met Thr Cys Arg Ala Ser Ser Ser Val Ser Tyr Met
            420                 425                 430
Asn Trp Tyr Gln Gln Lys Ser Gly Thr Ser Pro Lys Arg Trp Ile Tyr
        435                 440                 445
Asp Thr Ser Lys Val Ala Ser Gly Val Pro Tyr Arg Phe Ser Gly Ser
    450                 455                 460
Gly Ser Gly Thr Ser Tyr Ser Leu Thr Ile Ser Ser Met Glu Ala Glu
465                 470                 475                 480
Asp Ala Ala Thr Tyr Tyr Cys Gln Gln Trp Ser Ser Asn Pro Leu Thr
                485                 490                 495
Phe Gly Ala Gly Thr Lys Leu Glu Leu Lys His His His His His His
            500                 505                 510

<210> 828
<211> 120
<212> PRT
<213> Homo sapiens

<220>
<223> VH region of BMA031 (anti-TCR) scFv

<400> 828
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Lys Phe Thr Ser Tyr
            20                  25                  30
Val Met His Trp Val Lys Gln Ala Pro Gly Gln Gly Leu Glu Trp Ile
        35                  40                  45
Gly Tyr Ile Asn Pro Tyr Asn Asp Val Thr Lys Tyr Asn Glu Lys Phe
    50                  55                  60
Lys Gly Lys Ala Thr Leu Thr Ala Asp Glu Ser Thr Asn Thr Ala Tyr
65                  70                  75                  80
Met Glu Leu Ser Ser Leu Arg Ser Glu Asp Thr Ala Val His Tyr Cys
                85                  90                  95

Ala Arg Gly Ser Tyr Tyr Asp Tyr Asp Gly Phe Val Tyr Trp Gly Gln
            100                 105                 110
Gly Thr Leu Val Thr Val Ser Ser
        115                 120

<210> 829
<211> 111
<212> PRT
<213> Homo sapiens

<220>
<223> VL region of BMA031 (anti-TCR) scFv

<400> 829
Ala Gln Asp Ile Gln Met Thr Gln Ser Pro Ser Thr Leu Ser Ala Ser
 1               5                  10                  15
Val Gly Asp Arg Val Thr Met Thr Cys Ser Ala Thr Ser Ser Val Ser
            20                  25                  30
Tyr Met His Trp Tyr Gln Gln Lys Pro Gly Lys Ala Pro Lys Arg Trp
        35                  40                  45
Ile Tyr Asp Thr Ser Lys Leu Ala Ser Gly Val Pro Ala Arg Phe Ile
    50                  55                  60
Gly Ser Gly Ser Gly Thr Glu Phe Thr Leu Thr Ile Ser Ser Leu Gln
65                  70                  75                  80
Pro Asp Asp Phe Ala Thr Tyr Tyr Cys Gln Gln Trp Ser Ser Asn Pro
                85                  90                  95
Leu Thr Phe Gly Gly Gly Thr Lys Val Glu Ile Lys Ser Ser Ser
            100                 105                 110

<210> 830
<211> 246
<212> PRT
<213> Homo sapiens

<220>
<223> BMA031 scFv

<400> 830
Gln Val Gln Leu Val Gln Ser Gly Ala Glu Val Lys Lys Pro Gly Ser
 1               5                  10                  15
Ser Val Lys Val Ser Cys Lys Ala Ser Gly Tyr Lys Phe Thr Ser Tyr
            20                  25                  30
Val Met His Trp Val Lys Gln Ala Pro Gly Gln Gly Leu Glu Trp Ile
        35                  40                  45
Gly Tyr Ile Asn Pro Tyr Asn Asp Val Thr Lys Tyr Asn Glu Lys Phe
    50                  55                  60
Lys Gly Lys Ala Thr Leu Thr Ala Asp Glu Ser Thr Asn Thr Ala Tyr
65                  70                  75                  80
Met Glu Leu Ser Ser Leu Arg Ser Glu Asp Thr Ala Val His Tyr Cys
                85                  90                  95
Ala Arg Gly Ser Tyr Tyr Asp Tyr Asp Gly Phe Val Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Ala Gln Asp Ile Gln Met Thr Gln Ser
    130                 135                 140
Pro Ser Thr Leu Ser Ala Ser Val Gly Asp Arg Val Thr Met Thr Cys
145                 150                 155                 160
Ser Ala Thr Ser Ser Val Ser Tyr Met His Trp Tyr Gln Gln Lys Pro
                165                 170                 175
Gly Lys Ala Pro Lys Arg Trp Ile Tyr Asp Thr Ser Lys Leu Ala Ser

180                 185                 190
Gly Val Pro Ala Arg Phe Ile Gly Ser Gly Ser Gly Thr Glu Phe Thr
        195                 200                 205
Leu Thr Ile Ser Ser Leu Gln Pro Asp Asp Phe Ala Thr Tyr Tyr Cys
    210                 215                 220
Gln Gln Trp Ser Ser Asn Pro Leu Thr Phe Gly Gly Gly Thr Lys Val
225                 230                 235                 240
Glu Ile Lys Ser Ser Ser
                245

<210> 831
<211> 119
<212> PRT
<213> Homo sapiens

<220>
<223> VH region of OKT3-M (anti-CD3) scFv

<400> 831
Asp Ile Lys Leu Gln Gln Ser Gly Ala Glu Leu Ala Arg Pro Gly Ala
1               5                   10                  15
Ser Val Lys Met Ser Cys Lys Thr Ser Gly Tyr Thr Phe Thr Arg Tyr
            20                  25                  30
Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Leu Thr Val Ser Ser
        115

<210> 832
<211> 18
<212> PRT
<213> Artificial Sequence

<220>
<223> OKT3-M Linker

<400> 832
Val Glu Gly Gly Ser Gly Gly Ser Gly Gly Ser Gly Gly Ser Gly Gly
1               5                   10                  15
Val Asp

<210> 833
<211> 94
<212> PRT
<213> Homo sapiens

<220>
<223> VL region of OKT3-M (anti-CD3) scFv

<400> 833
Asp Ile Gln Leu Thr Gln Ser Pro Ala Ile Met Ser Ala Ser Pro Gly

1                   5                   10                  15
Glu Lys Val Thr Met Thr Cys Arg Ala Ser Ser Ser Val Ser Tyr Met
                20                  25                  30
Asn Trp Tyr Gln Gln Lys Ser Gly Thr Ser Pro Lys Arg Trp Ile Tyr
            35                  40                  45
Asp Thr Ser Lys Val Ala Ser Gly Val Pro Tyr Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Ser Ser
                85                  90

<210> 834
<211> 231
<212> PRT
<213> Homo sapiens

<220>
<223> OKT3-M scFv

<400> 834
Asp Ile Lys Leu Gln Gln Ser Gly Ala Glu Leu Ala Arg Pro Gly Ala
1               5                   10                  15
Ser Val Lys Met Ser Cys Lys Thr Ser Gly Tyr Thr Phe Thr Arg Tyr
                20                  25                  30
Thr Met His Trp Val Lys Gln Arg Pro Gly Gln Gly Leu Glu Trp Ile
            35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Leu Thr Val Ser Ser Val Glu Gly Gly Ser Gly Gly Ser Gly
        115                 120                 125
Gly Ser Gly Gly Ser Gly Gly Val Asp Asp Ile Gln Leu Thr Gln Ser
    130                 135                 140
Pro Ala Ile Met Ser Ala Ser Pro Gly Glu Lys Val Thr Met Thr Cys
145                 150                 155                 160
Arg Ala Ser Ser Ser Val Ser Tyr Met Asn Trp Tyr Gln Gln Lys Ser
                165                 170                 175
Gly Thr Ser Pro Lys Arg Trp Ile Tyr Asp Thr Ser Lys Val Ala Ser
            180                 185                 190
Gly Val Pro Tyr Arg Phe Ser Gly Ser Gly Ser Gly Thr Ser Tyr Ser
        195                 200                 205
Leu Thr Ile Ser Ser Met Glu Ala Glu Asp Ala Ala Thr Tyr Tyr Cys
    210                 215                 220
Gln Gln Trp Ser Ser Ser Ser
225                 230

<210> 835
<211> 120
<212> PRT
<213> Homo sapiens

<220>
<223> VH region of HuM291 (anti-CD3) scFv

<400> 835

<210> 836
<211> 112
<212> PRT
<213> Homo sapiens

<220>
<223> VL region of HuM291 (anti-CD3) scFv

<400> 836
Ala Gln Asp Ile Gln Met Thr Gln Ser Pro Ser Ser Leu Ser Ala Ser
1               5                   10                  15
Val Gly Asp Arg Val Thr Ile Thr Cys Ser Ala Ser Ser Ser Val Ser
            20                  25                  30
Tyr Met Asn Trp Tyr Gln Gln Lys Pro Gly Lys Ala Pro Lys Arg Leu
        35                  40                  45
Ile Tyr Asp Thr Ser Lys Leu Ala Ser Gly Val Pro Ser Arg Phe Ser
    50                  55                  60
Gly Ser Gly Ser Gly Thr Asp Phe Thr Leu Thr Ile Ser Ser Leu Gln
65                  70                  75                  80
Pro Glu Asp Phe Ala Thr Tyr Tyr Cys Gln Gln Trp Ser Ser Asn Pro
                85                  90                  95
Pro Thr Phe Gly Gly Gly Thr Lys Val Glu Ile Lys Arg Thr Ser Ser
            100                 105                 110

<210> 837
<211> 247
<212> PRT
<213> Homo sapiens

<220>
<223> HuM291 scFv

<400> 837
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Ile Ser Tyr
            20                  25                  30
Thr Met His Trp Val Arg Gln Ala Pro Gly Gln Gly Leu Glu Trp Met
        35                  40                  45
Gly Tyr Ile Asn Pro Arg Ser Gly Tyr Thr His Tyr Asn Gln Lys Leu
    50                  55                  60
Lys Asp Lys Ala Thr Leu Thr Ala Asp Lys Ser Ala Ser Thr Ala Tyr
65                  70                  75                  80
Met Glu Leu Ser Ser Leu Arg Ser Glu Asp Thr Ala Val Tyr Tyr Cys

85                          90                          95
Ala Arg Ser Ala Tyr Tyr Asp Tyr Asp Gly Phe Ala Tyr Trp Gly Gln
                100                         105                         110
Gly Thr Leu Val Thr Val Ser Ser Gly Gly Gly Gly Ser Gly Gly Gly
        115                         120                         125
Gly Ser Gly Gly Gly Gly Ser Ala Gln Asp Ile Gln Met Thr Gln Ser
    130                         135                         140
Pro Ser Ser Leu Ser Ala Ser Val Gly Asp Arg Val Thr Ile Thr Cys
145                         150                         155                         160
Ser Ala Ser Ser Ser Val Ser Tyr Met Asn Trp Tyr Gln Gln Lys Pro
                        165                         170                         175
Gly Lys Ala Pro Lys Arg Leu Ile Tyr Asp Thr Ser Lys Leu Ala Ser
                180                         185                         190
Gly Val Pro Ser Arg Phe Ser Gly Ser Gly Ser Gly Thr Asp Phe Thr
        195                         200                         205
Leu Thr Ile Ser Ser Leu Gln Pro Glu Asp Phe Ala Thr Tyr Tyr Cys
    210                         215                         220
Gln Gln Trp Ser Ser Asn Pro Pro Thr Phe Gly Gly Gly Thr Lys Val
225                         230                         235                         240
Glu Ile Lys Arg Thr Ser Ser
                245

<210> 838
<211> 105
<212> PRT
<213> Artificial Sequence

<220>
<223> mutated human Ck DAE

<400> 838
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asp Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 839
<211> 105
<212> PRT
<213> Artificial Sequence

<220>
<223> mutated human Ck MAE

<400> 839
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Met Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60

Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 840
<211> 105
<212> PRT
<213> Artificial Sequence

<220>
<223> mutated human Ck SAE

<400> 840
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Ser Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 841
<211> 105
<212> PRT
<213> Artificial Sequence

<220>
<223> mutated human FAE

<400> 841
Thr Val Ala Ala Pro Ser Val Phe Ile Phe Pro Pro Ser Asp Glu Gln
 1               5                  10                  15
Leu Lys Ser Gly Thr Ala Ser Val Val Cys Leu Leu Asn Phe Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Ala Thr Glu Gln Asp Ser Lys Asp Ser Thr
    50                  55                  60
Tyr Ser Leu Ser Ser Glu Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 842
<211> 105
<212> PRT
<213> Artificial Sequence

<220>
<223> mutated Ck L29E

<400> 842
Thr Val Ala Ala Pro Ser Val Phe Ile Phe Pro Pro Ser Asp Glu Gln
1               5                   10                  15
Leu Lys Ser Gly Thr Ala Ser Val Val Cys Glu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 843
<211> 105
<212> PRT
<213> Artificial Sequence

<220>
<223> mutated Ck L29K

<400> 843
Thr Val Ala Ala Pro Ser Val Phe Ile Phe Pro Pro Ser Asp Glu Gln
1               5                   10                  15
Leu Lys Ser Gly Thr Ala Ser Val Val Cys Lys Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu
            100                 105

<210> 844
<211> 98
<212> PRT
<213> Artificial Sequence

<220>
<223> mutated CH1 V68E

<400> 844
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Glu Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val

<210> 845
<211> 98
<212> PRT
<213> Artificial Sequence

<220>
<223> mutated CH1 V68K

<400> 845
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Lys Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val


<210> 846
<211> 3
<212> PRT
<213> Artificial Sequence

<220>
<223> CH3-CH1 linker or CH3-Ck linker

<400> 846
Lys Ser Arg
1

<210> 847
<211> 5
<212> PRT
<213> Artificial Sequence

<220>
<223> CH3-CH1 linker

<400> 847
Gly Gly Gly Ser Ser
1               5

<210> 848
<211> 6
<212> PRT
<213> Artificial Sequence

<220>
<223> CH3-CH1 linker

<400> 848
Ser Tyr Ser Pro Asn Ser
1               5

<210> 849
<211> 8

<212> PRT
<213> Artificial Sequence

<220>
<223> CH3-CH1 linker

<400> 849
Ser Ser Leu Asn Thr Pro Asn Ser
 1               5

<210> 850
<211> 5
<212> PRT
<213> Artificial Sequence

<220>
<223> CH3-Ck linker

<400> 850
Gly Gly Gly Ser Arg
 1               5

<210> 851
<211> 7
<212> PRT
<213> Artificial Sequence

<220>
<223> CH3-Ck linker

<400> 851
Ser Tyr Ser Pro Asn Ser Arg
 1               5

<210> 852
<211> 9
<212> PRT
<213> Artificial Sequence

<220>
<223> CH3-Ck linker

<400> 852
Ser Ser Leu Asn Thr Pro Asn Ser Arg
 1               5

<210> 853
<211> 1806
<212> DNA
<213> Artificial Sequence

<220>
<223> TSC145 (Cris7 scFv-hinge-CH2-CH3-GGGSS linker-CH1)

<400> 853
atggaagcac cagcgcagct tctcttcctc ctgctactct ggctcccaga taccaccggt      60
caggtccagc tggtgcagtc tgggggcgga gtggtgcagc ctgggcggtc actgaggctg      120
tcctgcaagg cttctggcta cacctttact agatctacga tgcactgggt aaggcaggcc      180
cctggaaagg gtctggaatg gattggatac attaatccta gcagtgctta tactaattac      240
aatcagaaat tcaaggacag gttcacaatc agcgcagaca aatccaagag cacagccttc      300
ctgcagatgg acagcctgag gcccgaggac accggcgtct atttctgtgc acggccccaa      360
gtccactatg attacaacgg gtttccttac tggggccaag ggactcccgt cactgtctct      420
agcggtggcg gagggtctgg gggtggcgga tccggaggtg gtggctctgc acaagacatc      480

cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtggcgga 1500
ggctcctcag cctccaccaa gggcccatcg gtcttccccc tggcaccctc ctccaagagc 1560
acctctgggg gcacagcggc cctgggctgc ctggtcaagg actacttccc cgagccggtg 1620
acggtgtcgt ggaactcagg cgccctgacc agcggcgtgc acaccttccc ggctgtccta 1680
cagtcctcag gactctactc cctcagcagc gtggtgaccg tgccctccag cagcttgggc 1740
acccagacct acatctgcaa cgtgaatcac aagcccagca acaccaaggt ggacaagaaa 1800
gtttga 1806

<210> 854
<211> 1809
<212> DNA
<213> Artificial Sequence

<220>
<223> TSC146 (Cris7 scFv-hinge-CH2-CH3-SYSPNS
      linker-CH1)

<400> 854
atggaagcac cagcgcagct tctcttcctc ctgctactct ggctcccaga taccaccggt 60
caggtccagc tggtgcagtc tggggcggga gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtagttac 1500
agcccgaact ctgcctccac caagggccca tcggtcttcc ccctggcacc ctcctccaag 1560
agcacctctg ggggcacagc ggccctgggc tgcctggtca aggactactt ccccgagccg 1620
gtgacggtgt cgtggaactc aggcgccctg accagcggcg tgcacacctt cccggctgtc 1680
ctacagtcct caggactcta ctccctcagc agcgtggtga ccgtgccctc cagcagcttg 1740

ggcacccaga cctacatctg caacgtgaat cacaagccca gcaacaccaa ggtggacaag 1800
aaagtttga 1809

<210> 855
<211> 1815
<212> DNA
<213> Artificial Sequence

<220>
<223> TSC147 (Cris7 scFv-hinge-CH2-CH3-SSLNTPNS
      linker-CH1)

<400> 855
atggaagcac cagcgcagct tctcttcctc ctgctactct ggctcccaga taccaccggt 60
caggtccagc tggtgcagtc tgggggcgga gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggttcttcc 1500
ctgaatacac cgaactctgc ctccaccaag ggcccatcgg tcttccccct ggcaccctcc 1560
tccaagagca cctctggggg cacagcggcc ctgggctgcc tggtcaagga ctacttcccc 1620
gagccggtga cggtgtcgtg gaactcaggc gccctgacca gcggcgtgca caccttcccg 1680
gctgtcctac agtcctcagg actctactcc ctcagcagcg tggtgaccgt gccctccagc 1740
agcttgggca cccagaccta catctgcaac gtgaatcaca agcccagcaa caccaaggtg 1800
gacaagaaag tttga 1815

<210> 856
<211> 1842
<212> DNA
<213> Artificial Sequence

<220>
<223> TSC148 (HD37 scFv-hinge-CH2-CH3-GGGSR linker-Ck)

<400> 856
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540

atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagtgagc ccaaatcttc tgacaaaact 840
cacacatgcc caccgtgccc agcacctgaa gccgcgggtg caccgtcagt cttcctcttc 900
cccccaaaac ccaaggacac cctcatgatc tcccggaccc ctgaggtcac atgcgtggtg 960
gtggacgtga gccacgaaga ccctgaggtc aagttcaact ggtacgtgga cggcgtggag 1020
gtgcataatg ccaagacaaa gccgcgggag gagcagtaca acagcacgta ccgtgtggtc 1080
agcgtcctca ccgtcctgca ccaggactgg ctgaatggca aggcgtacgc gtgcgcggtc 1140
tccaacaaag ccctcccagc ccccatcgag aaaaccatct ccaaagccaa agggcagccc 1200
cgagaaccac aggtgtacac cctgccccca tcccgggatg agctgaccaa gaaccaggtc 1260
agcctgacct gcctggtcaa aggcttctat ccaagcgaca tcgccgtgga gtgggagagc 1320
aatgggcagc cggagaacaa ctacaagacc acgcctcccg tgctggactc cgacggctcc 1380
ttcttcctct acagcaagct caccgtggac aagagcaggt ggcagcaggg gaacgtcttc 1440
tcatgctccg tgatgcatga ggctctgcac aaccactaca cgcagaagag cctctccctg 1500
tctccgggtg gcggaggctc caggactgtg gctgcaccat ctgtcttcat cttcccgcca 1560
tctgatgagc agttgaaatc tggaactgcc tctgttgtgt gcctgctgaa ttacttctat 1620
cccagagagg ccaaagtaca gtggaaggtg gataacgccc tccaatcggg taactcccag 1680
gagagtgcca cagagcagga cagcaaggac agcacctaca gcctcagcag cgagctgacg 1740
ctgagcaaag cagactacga gaaacacaaa gtctacgcct gcgaagtcac ccatcagggc 1800
ctgagctcgc ccgtcacaaa gagcttcaac aggggagagt ga 1842

<210> 857
<211> 1848
<212> DNA
<213> Artificial Sequence

<220>
<223> TSC149 (HD37 scFv-hinge-CH2-CH3-SYSPNSR linker-Ck)

<400> 857
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagtgagc ccaaatcttc tgacaaaact 840
cacacatgcc caccgtgccc agcacctgaa gccgcgggtg caccgtcagt cttcctcttc 900
cccccaaaac ccaaggacac cctcatgatc tcccggaccc ctgaggtcac atgcgtggtg 960
gtggacgtga gccacgaaga ccctgaggtc aagttcaact ggtacgtgga cggcgtggag 1020
gtgcataatg ccaagacaaa gccgcgggag gagcagtaca acagcacgta ccgtgtggtc 1080
agcgtcctca ccgtcctgca ccaggactgg ctgaatggca aggcgtacgc gtgcgcggtc 1140
tccaacaaag ccctcccagc ccccatcgag aaaaccatct ccaaagccaa agggcagccc 1200
cgagaaccac aggtgtacac cctgccccca tcccgggatg agctgaccaa gaaccaggtc 1260
agcctgacct gcctggtcaa aggcttctat ccaagcgaca tcgccgtgga gtgggagagc 1320
aatgggcagc cggagaacaa ctacaagacc acgcctcccg tgctggactc cgacggctcc 1380
ttcttcctct acagcaagct caccgtggac aagagcaggt ggcagcaggg gaacgtcttc 1440
tcatgctccg tgatgcatga ggctctgcac aaccactaca cgcagaagag cctctccctg 1500
tctccgggta gttacagccc gaactctagg actgtggctg caccatctgt cttcatcttc 1560
ccgccatctg atgagcagtt gaaatctgga actgcctctg ttgtgtgcct gctgaattac 1620
ttctatccca gagaggccaa agtacagtgg aaggtggata acgccctcca atcgggtaac 1680
tcccaggaga gtgccacaga gcaggacagc aaggacagca cctacagcct cagcagcgag 1740
ctgacgctga gcaaagcaga ctacgagaaa cacaaagtct acgcctgcga agtcacccat 1800
cagggcctga gctcgcccgt cacaaagagc ttcaacaggg gagagtga 1848

<210> 858
<211> 1854
<212> DNA
<213> Artificial Sequence

<220>
<223> TSC150 (HD37 scFv-hinge-CH2-CH3-SSLNTPNSR
      linker-Ck)

<400> 858
atggaagcac cagcgcagct tctcttcctc ctgctactct ggctcccaga taccaccggt      60
gacattgtgc tgacccaatc tccagcttct ttggctgtgt ctctagggca gagggccacc     120
atctcctgca aggccagcca aagtgttgat tatgatggtg atagttattt gaactggtac     180
caacagattc caggacagcc acccaaactc ctcatctatg atgcatccaa tctagtttct     240
gggatcccac ccaggtttag tggcagtggg tctgggacag acttcaccct caacatccat     300
cctgtggaga aggtggatgc tgcaacctat cactgccagc aaagtactga ggatccgtgg     360
acgttcggtg gaggcaccaa gctggaaatc aaaggtggcg gtggttcggg cggtggtggg     420
tcgggtggcg gcggagctag ccaggttcag ctgcagcagt ctggggctga gctggtgagg     480
cctgggtcct cagtgaagat ttcctgcaag gcttctggct atgcattcag tagctactgg     540
atgaactggg tgaagcagag gcctggacag ggtcttgagt ggattggaca gatttggcct     600
ggagatggtg atactaacta caatggaaag ttcaagggta aagccactct gactgcagac     660
gaatcctcca gcacagccta catgcaactc agcagcctag catctgagga ctctgcggtc     720
tatttctgtg caagacggga gactacgacg gtaggccgtt attactatgc tatggactac     780
tggggtcaag gaacctcagt caccgtctcc tcgagtgagc ccaaatcttc tgacaaaact     840
cacacatgcc caccgtgccc agcacctgaa gccgcgggtg caccgtcagt cttcctcttc     900
cccccaaaac ccaaggacac cctcatgatc tcccggaccc ctgaggtcac atgcgtggtg     960
gtggacgtga gccacgaaga ccctgaggtc aagttcaact ggtacgtgga cggcgtggag    1020
gtgcataatg ccaagacaaa gccgcgggag gagcagtaca acagcacgta ccgtgtggtc    1080
agcgtcctca ccgtcctgca ccaggactgg ctgaatggca aggcgtacgc gtgcgcggtc    1140
tccaacaaag ccctcccagc ccccatcgag aaaaccatct ccaaagccaa agggcagccc    1200
cgagaaccac aggtgtacac cctgccccca tcccgggatg agctgaccaa gaaccaggtc    1260
agcctgacct gcctggtcaa aggcttctat ccaagcgaca tcgccgtgga gtgggagagc    1320
aatgggcagc cggagaacaa ctacaagacc acgcctcccg tgctggactc cgacggctcc    1380
ttcttcctct acagcaagct caccgtggac aagagcaggt ggcagcaggg gaacgtcttc    1440
tcatgctccg tgatgcatga ggctctgcac aaccactaca cgcagaagag cctctccctg    1500
tctccgggtt cttccctgaa tacaccgaac tctaggactg tggctgcacc atctgtcttc    1560
atcttcccgc catctgatga gcagttgaaa tctggaactg cctctgttgt gtgcctgctg    1620
aattacttct atcccagaga ggccaaagta cagtggaagg tggataacgc cctccaatcg    1680
ggtaactccc aggagagtgc cacagagcag gacagcaagg acagcaccta cagcctcagc    1740
agcgagctga cgctgagcaa agcagactac gagaaacaca aagtctacgc ctgcgaagtc    1800
acccatcagg gcctgagctc gcccgtcaca aagagcttca acaggggaga gtga          1854

<210> 859
<211> 581
<212> PRT
<213> Artificial Sequence

<220>
<223> TSC145 (Cris7 scFv-hinge-CH2-CH3-GGGSS linker-CH1)

<400> 859
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys

                    85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Gly Gly
465                 470                 475                 480
Gly Ser Ser Ala Ser Thr Lys Gly Pro Ser Val Phe Pro Leu Ala Pro
                485                 490                 495
Ser Ser Lys Ser Thr Ser Gly Gly Thr Ala Ala Leu Gly Cys Leu Val
            500                 505                 510
Lys Asp Tyr Phe Pro Glu Pro Val Thr Val Ser Trp Asn Ser Gly Ala
        515                 520                 525
Leu Thr Ser Gly Val His Thr Phe Pro Ala Val Leu Gln Ser Ser Gly
    530                 535                 540
Leu Tyr Ser Leu Ser Ser Val Val Thr Val Pro Ser Ser Ser Leu Gly
545                 550                 555                 560
Thr Gln Thr Tyr Ile Cys Asn Val Asn His Lys Pro Ser Asn Thr Lys
                565                 570                 575
Val Asp Lys Lys Val
            580

<210> 860
<211> 582
<212> PRT
<213> Artificial Sequence

<220>
<223> TSC146 (Cris7 scFv-hinge-CH2-CH3-SYSPNS
      linker-CH1)

<400> 860
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415

Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Ser Tyr
465                 470                 475                 480
Ser Pro Asn Ser Ala Ser Thr Lys Gly Pro Ser Val Phe Pro Leu Ala
                485                 490                 495
Pro Ser Ser Lys Ser Thr Ser Gly Gly Thr Ala Ala Leu Gly Cys Leu
            500                 505                 510
Val Lys Asp Tyr Phe Pro Glu Pro Val Thr Val Ser Trp Asn Ser Gly
        515                 520                 525
Ala Leu Thr Ser Gly Val His Thr Phe Pro Ala Val Leu Gln Ser Ser
    530                 535                 540
Gly Leu Tyr Ser Leu Ser Ser Val Val Thr Val Pro Ser Ser Ser Leu
545                 550                 555                 560
Gly Thr Gln Thr Tyr Ile Cys Asn Val Asn His Lys Pro Ser Asn Thr
                565                 570                 575
Lys Val Asp Lys Lys Val
            580

<210> 861
<211> 584
<212> PRT
<213> Artificial Sequence

<220>
<223> TSC147 (Cris7 scFv-hinge-CH2-CH3-SSLNTPNS
linker-CH1)

<400> 861
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240

Leu Gln Ile Thr Arg Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Ser Ser
465                 470                 475                 480
Leu Asn Thr Pro Asn Ser Ala Ser Thr Lys Gly Pro Ser Val Phe Pro
                485                 490                 495
Leu Ala Pro Ser Ser Lys Ser Thr Ser Gly Gly Thr Ala Ala Leu Gly
            500                 505                 510
Cys Leu Val Lys Asp Tyr Phe Pro Glu Pro Val Thr Val Ser Trp Asn
        515                 520                 525
Ser Gly Ala Leu Thr Ser Gly Val His Thr Phe Pro Ala Val Leu Gln
    530                 535                 540
Ser Ser Gly Leu Tyr Ser Leu Ser Ser Val Val Thr Val Pro Ser Ser
545                 550                 555                 560
Ser Leu Gly Thr Gln Thr Tyr Ile Cys Asn Val Asn His Lys Pro Ser
                565                 570                 575
Asn Thr Lys Val Asp Lys Lys Val
            580

<210> 862
<211> 593
<212> PRT
<213> Artificial Sequence

<220>
<223> TSC148 (HD37 scFv-hinge-CH2-CH3-GGGSR linker-Ck)

<400> 862
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His

65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Gly Gly Gly Ser Arg Thr Val Ala Ala Pro Ser Val Phe
                485                 490                 495
Ile Phe Pro Pro Ser Asp Glu Gln Leu Lys Ser Gly Thr Ala Ser Val
            500                 505                 510
Val Cys Leu Leu Asn Tyr Phe Tyr Pro Arg Glu Ala Lys Val Gln Trp
        515                 520                 525
Lys Val Asp Asn Ala Leu Gln Ser Gly Asn Ser Gln Glu Ser Ala Thr
    530                 535                 540
Glu Gln Asp Ser Lys Asp Ser Thr Tyr Ser Leu Ser Ser Glu Leu Thr
545                 550                 555                 560
Leu Ser Lys Ala Asp Tyr Glu Lys His Lys Val Tyr Ala Cys Glu Val
                565                 570                 575

Thr His Gln Gly Leu Ser Ser Pro Val Thr Lys Ser Phe Asn Arg Gly
            580                 585                 590
Glu

<210> 863
<211> 595
<212> PRT
<213> Artificial Sequence

<220>
<223> TSC149 (HD37 scFv-hinge-CH2-CH3-SYSPNSR linker-Ck)

<400> 863
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val

385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Ser Tyr Ser Pro Asn Ser Arg Thr Val Ala Ala Pro Ser
                485                 490                 495
Val Phe Ile Phe Pro Pro Ser Asp Glu Gln Leu Lys Ser Gly Thr Ala
            500                 505                 510
Ser Val Val Cys Leu Leu Asn Tyr Phe Tyr Pro Arg Glu Ala Lys Val
        515                 520                 525
Gln Trp Lys Val Asp Asn Ala Leu Gln Ser Gly Asn Ser Gln Glu Ser
    530                 535                 540
Ala Thr Glu Gln Asp Ser Lys Asp Ser Thr Tyr Ser Leu Ser Ser Glu
545                 550                 555                 560
Leu Thr Leu Ser Lys Ala Asp Tyr Glu Lys His Lys Val Tyr Ala Cys
                565                 570                 575
Glu Val Thr His Gln Gly Leu Ser Ser Pro Val Thr Lys Ser Phe Asn
            580                 585                 590
Arg Gly Glu
        595

<210> 864
<211> 597
<212> PRT
<213> Artificial Sequence

<220>
<223> TSC150 (HD37 scFv-hinge-CH2-CH3-SSLNTPNSR
      linker-Ck)

<400> 864
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met

195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Ser Ser Leu Asn Thr Pro Asn Ser Arg Thr Val Ala Ala
                485                 490                 495
Pro Ser Val Phe Ile Phe Pro Pro Ser Asp Glu Gln Leu Lys Ser Gly
            500                 505                 510
Thr Ala Ser Val Val Cys Leu Leu Asn Tyr Phe Tyr Pro Arg Glu Ala
        515                 520                 525
Lys Val Gln Trp Lys Val Asp Asn Ala Leu Gln Ser Gly Asn Ser Gln
    530                 535                 540
Glu Ser Ala Thr Glu Gln Asp Ser Lys Asp Ser Thr Tyr Ser Leu Ser
545                 550                 555                 560
Ser Glu Leu Thr Leu Ser Lys Ala Asp Tyr Glu Lys His Lys Val Tyr
                565                 570                 575
Ala Cys Glu Val Thr His Gln Gly Leu Ser Ser Pro Val Thr Lys Ser
            580                 585                 590
Phe Asn Arg Gly Glu
        595

<210> 865
<211> 1800
<212> DNA
<213> Artificial Sequence

<220>
<223> TSC125 (Cris7 scFv-CH2-CH3-CH1)

<400> 865
atggaagcac cagcgcagct tctcttcctc ctgctactct ggctcccaga taccaccggt      60
caggtccagc tggtgcagtc tgggggcgga gtggtgcagc ctgggcggtc actgaggctg      120

tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag tgagcccaaa tcttctgaca aaactcacac atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agagcctcca ccaagggccc atcggtcttc cccctggcac cctcctccaa gagcacctct 1560
gggggcacag cggccctggg ctgcctggtc aaggactact tccccgagcc ggtgacggtg 1620
tcgtggaact caggcgccct gaccagcggc gtgcacacct tcccggctgt cctacagtcc 1680
tcaggactct actccctcag cagcgtggtg accgtgccct ccagcagctt gggcacccag 1740
acctacatct gcaacgtgaa tcacaagccc agcaacacca aggtggacaa gaaagtttga 1800

<210> 866
<211> 1785
<212> DNA
<213> Artificial Sequence

<220>
<223> TSC157 (Cris7 scFv-IgA2UH-CH2-CH3-CH1)

<400> 866
   atggaagcac cagcgcagct tctcttcctc ctgctactct ggctcccaga taccaccggt 60
   caggtccagc tggtgcagtc tgggggcgga gtggtgcagc ctgggcggtc actgaggctg 120
   tcctgcaagg cttctggcta cacctttact agatctacga tgcactgggt aaggcaggcc 180
   cctggaaagg gtctggaatg gattggatac attaatccta gcagtgctta tactaattac 240
   aatcagaaat tcaaggacag gttcacaatc agcgcagaca aatccaagag cacagccttc 300
   ctgcagatgg acagcctgag gcccgaggac accggcgtct atttctgtgc acggccccaa 360
   gtccactatg attacaacgg gtttccttac tggggccaag ggactcccgt cactgtctct 420
   agcggtggcg gagggtctgg gggtggcgga tccggaggtg gtggctctgc acaagacatc 480
   cagatgaccc agtctccaag cagcctgtct gcaagcgtgg gggacagggt caccatgacc 540
   tgcagtgcca gctcaagtgt aagttacatg aactggtacc agcagaagcc cggcaaggcc 600
   cccaaaagat ggatttatga ctcatccaaa ctggcttctg gagtccctgc tcgcttcagt 660
   ggcagtgggt ctgggaccga ctataccctc acaatcagca gcctgcagcc cgaagatttc 720
   gccacttatt actgccagca gtggagtcgt aacccaccca cgttcggagg ggggaccaag 780
   ctacaaatta cacgctcgag ccccccacct cccccatgcc caccgtgccc agcacctgaa 840
   gccgcgggtg caccgtcagt cttcctcttc cccccaaaac ccaaggacac cctcatgatc 900
   tcccggaccc ctgaggtcac atgcgtggtg gtggacgtga gccacgaaga ccctgaggtc 960
   aagttcaact ggtacgtgga cggcgtggag gtgcataatg ccaagacaaa gccgcgggag 1020
   gagcagtaca acagcacgta ccgtgtggtc agcgtcctca ccgtcctgca ccaggactgg 1080
   ctgaatggca aggcgtacgc gtgcgcggtc tccaacaaag ccctcccagc ccccatcgag 1140
   aaaaccatct ccaaagccaa agggcagccc cgagaaccac aggtgtacac cctgccccca 1200
   tcccgggatg agctgaccaa gaaccaggtc agcctgacct gcctggtcaa aggcttctat 1260
   ccaagcgaca tcgccgtgga gtgggagagc aatgggcagc cggagaacaa ctacaagacc 1320
   acgcctcccg tgctggactc cgacggctcc ttcttcctct acagcaagct caccgtggac 1380
   aagagcaggt ggcagcaggg gaacgtcttc tcatgctccg tgatgcatga ggctctgcac 1440
   aaccactaca cgcagaagag cctctccctg tctccgggta aatctagagc ctccaccaag 1500
   ggcccatcgg tcttccccct ggcaccctcc tccaagagca cctctggggg cacagcggcc 1560
   ctgggctgcc tggtcaagga ctacttcccc gagccggtga cggtgtcgtg gaactcaggc 1620
   gccctgacca gcggcgtgca caccttcccg gctgtcctac agtcctcagg actctactcc 1680
   ctcagcagcg tggtgaccgt gccctccagc agcttgggca cccagaccta catctgcaac 1740
   gtgaatcaca agcccagcaa caccaaggtg gacaagaaag tttga 1785

<210> 867
<211> 1815
<212> DNA
<213> Artificial Sequence

<220>
<223> TSC158 (Cris7 scFv-IgG1miniUH-CH2-CH3-CH1)

<400> 867
atggaagcac cagcgcagct tctcttcctc ctgctactct ggctcccaga taccaccggt 60
caggtccagc tggtgcagtc tgggggcgga gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag cgagcccaaa tcttctgaca aaactcacac aagcccaccg 840
agcccatgcc caccgtgccc agcacctgaa gccgcgggtg caccgtcagt cttcctcttc 900
cccccaaaac ccaaggacac cctcatgatc tcccggaccc ctgaggtcac atgcgtggtg 960
gtggacgtga gccacgaaga ccctgaggtc aagttcaact ggtacgtgga cggcgtggag 1020
gtgcataatg ccaagacaaa gccgcgggag gagcagtaca acagcacgta ccgtgtggtc 1080
agcgtcctca ccgtcctgca ccaggactgg ctgaatggca aggcgtacgc gtgcgcggtc 1140
tccaacaaag ccctcccagc ccccatcgag aaaaccatct ccaaagccaa agggcagccc 1200
cgagaaccac aggtgtacac cctgccccca tcccgggatg agctgaccaa gaaccaggtc 1260
agcctgacct gcctggtcaa aggcttctat ccaagcgaca tcgccgtgga gtgggagagc 1320
aatgggcagc cggagaacaa ctacaagacc acgcctcccg tgctggactc cgacggctcc 1380
ttcttcctct acagcaagct caccgtggac aagagcaggt ggcagcaggg gaacgtcttc 1440
tcatgctccg tgatgcatga ggctctgcac aaccactaca cgcagaagag cctctccctg 1500
tctccgggta aatctagagc ctccaccaag ggcccatcgg tcttccccct ggcaccctcc 1560
tccaagagca cctctggggg cacagcggcc ctgggctgcc tggtcaagga ctacttcccc 1620
gagccggtga cggtgtcgtg gaactcaggc gccctgacca gcggcgtgca caccttcccg 1680
gctgtcctac agtcctcagg actctactcc ctcagcagcg tggtgaccgt gccctccagc 1740
agcttgggca cccagaccta catctgcaac gtgaatcaca agcccagcaa caccaaggtg 1800
gacaagaaag tttga 1815

<210> 868
<211> 1815
<212> DNA
<213> Artificial Sequence

<220>
<223> TSC159 (Cris7 scFv-IgG3UH-CH2-CH3-CH1)

<400> 868
atggaagcac cagcgcagct tctcttcctc ctgctactct ggctcccaga taccaccggt 60
caggtccagc tggtgcagtc tgggggcgga gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420

agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag cgagcccaaa tctagcgaca cacctccccc aagcccacgg 840
tccccatgcc caccgtgccc agcacctgaa gccgcgggtg caccgtcagt cttcctcttc 900
cccccaaaac ccaaggacac cctcatgatc tcccggaccc ctgaggtcac atgcgtggtg 960
gtggacgtga gccacgaaga ccctgaggtc aagttcaact ggtacgtgga cggcgtggag 1020
gtgcataatg ccaagacaaa gccgcgggag gagcagtaca acagcacgta ccgtgtggtc 1080
agcgtcctca ccgtcctgca ccaggactgg ctgaatggca aggcgtacgc gtgcgcggtc 1140
tccaacaaag ccctcccagc ccccatcgag aaaaccatct ccaaagccaa agggcagccc 1200
cgagaaccac aggtgtacac cctgccccca tcccgggatg agctgaccaa gaaccaggtc 1260
agcctgacct gcctggtcaa aggcttctat ccaagcgaca tcgccgtgga gtgggagagc 1320
aatgggcagc cggagaacaa ctacaagacc acgcctcccg tgctggactc cgacggctcc 1380
ttcttcctct acagcaagct caccgtggac aagagcaggt ggcagcaggg gaacgtcttc 1440
tcatgctccg tgatgcatga ggctctgcac aaccactaca cgcagaagag cctctccctg 1500
tctccgggta aatctagagc ctccaccaag ggcccatcgg tcttccccct ggcaccctcc 1560
tccaagagca cctctggggg cacagcggcc ctgggctgcc tggtcaagga ctacttcccc 1620
gagccggtga cggtgtcgtg gaactcaggc gccctgacca gcggcgtgca caccttcccg 1680
gctgtcctac agtcctcagg actctactcc ctcagcagcg tggtgaccgt gccctccagc 1740
agcttgggca cccagaccta catctgcaac gtgaatcaca agcccagcaa caccaaggtg 1800
gacaagaaag tttga 1815

<210> 869
<211> 2103
<212> DNA
<213> Artificial Sequence

<220>
<223> TSC160 (Cris7 scFv-IgMCH2UH-CH2-CH3-CH1)

<400> 869
atggaagcac cagcgcagct tctcttcctc ctgctactct ggctcccaga taccaccggt 60
caggtccagc tggtgcagtc tgggggcgga gtggtgcagc ctgggcggtc actgaggctg 120
tcctgcaagg cttctggcta cacctttact agatctacga tgcactgggt aaggcaggcc 180
cctggaaagg gtctggaatg gattggatac attaatccta gcagtgctta tactaattac 240
aatcagaaat tcaaggacag gttcacaatc agcgcagaca aatccaagag cacagccttc 300
ctgcagatgg acagcctgag gcccgaggac accggcgtct atttctgtgc acggccccaa 360
gtccactatg attacaacgg gtttccttac tggggccaag ggactcccgt cactgtctct 420
agcggtggcg gagggtctgg gggtggcgga tccggaggtg gtggctctgc acaagacatc 480
cagatgaccc agtctccaag cagcctgtct gcaagcgtgg gggacagggt caccatgacc 540
tgcagtgcca gctcaagtgt aagttacatg aactggtacc agcagaagcc cggcaaggcc 600
cccaaaagat ggatttatga ctcatccaaa ctggcttctg gagtccctgc tcgcttcagt 660
ggcagtgggt ctgggaccga ctataccctc acaatcagca gcctgcagcc cgaagatttc 720
gccacttatt actgccagca gtggagtcgt aacccaccca cgttcggagg ggggaccaag 780
ctacaaatta cacgctcgag cattgccgag ctgcctccca aagtgagcgt cttcgtccca 840
ccccgcgacg gcttcttcgg caacccccgc aagtccaagc tcatctgcca ggccacgggt 900
ttcagtcccc ggcagattca ggtgtcctgg ctgcgcgagg ggaagcaggt ggggtctggc 960
gtcaccacgg accaggtgca ggctgaggcc aaagagtctg ggcccacgac ctacaaggtg 1020
accagcacac tgaccatcaa agagagcgac tggctcagcc agagcatgtt cacctgccgc 1080
gtggatcaca ggggcctgac cttccagcag aatgcgtcct ccatgagtgt cccctgccca 1140
ccgtgcccag cacctgaagc cgcgggtgca ccgtcagtct tcctcttccc cccaaaaccc 1200
aaggacaccc tcatgatctc ccggacccct gaggtcacat gcgtggtggt ggacgtgagc 1260
cacgaagacc ctgaggtcaa gttcaactgg tacgtggacg gcgtggaggt gcataatgcc 1320
aagacaaagc cgcgggagga gcagtacaac agcacgtacc gtgtggtcag cgtcctcacc 1380
gtcctgcacc aggactggct gaatggcaag gcgtacgcgt gcgcggtctc caacaaagcc 1440
ctcccagccc ccatcgagaa aaccatctcc aaagccaaag ggcagccccg agaaccacag 1500
gtgtacaccc tgcccccatc ccgggatgag ctgaccaaga accaggtcag cctgacctgc 1560
ctggtcaaag gcttctatcc aagcgacatc gccgtggagt gggagagcaa tgggcagccg 1620
gagaacaact acaagaccac gcctcccgtg ctggactccg acggctcctt cttcctctac 1680
agcaagctca ccgtggacaa gagcaggtgg cagcagggga acgtcttctc atgctccgtg 1740

atgcatgagg ctctgcacaa ccactacacg cagaagagcc tctccctgtc tccgggtaaa 1800
tctagagcct ccaccaaggg cccatcggtc ttccccctgg caccctcctc caagagcacc 1860
tctgggggca cagcggccct gggctgcctg gtcaaggact acttccccga gccggtgacg 1920
gtgtcgtgga actcaggcgc cctgaccagc ggcgtgcaca ccttcccggc tgtcctacag 1980
tcctcaggac tctactccct cagcagcgtg gtgaccgtgc cctccagcag cttgggcacc 2040
cagacctaca tctgcaacgt gaatcacaag cccagcaaca ccaaggtgga caagaaagtt 2100
tga 2103

<210> 870
<211> 1821
<212> DNA
<213> Artificial Sequence

<220>
<223> TSC161 (HD37 scFv-IgA2UH-CH2-CH3-Ck)

<400> 870
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagccccc cacctccccc atgcccaccg 840
tgcccagcac ctgaagccgc gggtgcaccg tcagtcttcc tcttcccccc aaaacccaag 900
gacaccctca tgatctcccg gacccctgag gtcacatgcg tggtggtgga cgtgagccac 960
gaagaccctg aggtcaagtt caactggtac gtggacggcg tggaggtgca taatgccaag 1020
acaaagccgc gggaggagca gtacaacagc acgtaccgtg tggtcagcgt cctcaccgtc 1080
ctgcaccagg actggctgaa tggcaaggcg tacgcgtgcg cggtctccaa caaagccctc 1140
ccagccccca tcgagaaaac catctccaaa gccaaagggc agccccgaga accacaggtg 1200
tacaccctgc ccccatcccg ggatgagctg accaagaacc aggtcagcct gacctgcctg 1260
gtcaaaggct tctatccaag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc gggtaaatct 1500
agaactgtgg ctgcaccatc tgtcttcatc ttcccgccat ctgatgagca gttgaaatct 1560
ggaactgcct ctgttgtgtg cctgctgaat aacttctatc ccagagaggc caaagtacag 1620
tggaaggtgg ataacgccct ccaatcgggt aactcccagg agagtgccac agagcaggac 1680
agcaaggaca gcacctacag cctcagcagc gagctgacgc tgagcaaagc agactacgag 1740
aaacacaaag tctacgcctg cgaagtcacc catcagggcc tgagctcgcc cgtcacaaag 1800
agcttcaaca ggggagagtg a 1821

<210> 871
<211> 1851
<212> DNA
<213> Artificial Sequence

<220>
<223> TSC162 (HD37 scFv-IgG1miniUH-CH2-CH3-Ck)

<400> 871
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300

cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcgagc ccaaatcttc tgacaaaact 840
cacacaagcc caccgagccc atgcccaccg tgcccagcac ctgaagccgc gggtgcaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacaacagc 1080
acgtaccgtg tggtcagcgt cctcaccgtc ctgcaccagg actggctgaa tggcaaggcg 1140
tacgcgtgcg cggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaaatct agaactgtgg ctgcaccatc tgtcttcatc 1560
ttcccgccat ctgatgagca gttgaaatct ggaactgcct ctgttgtgtg cctgctgaat 1620
aacttctatc ccagagaggc caaagtacag tggaaggtgg ataacgccct ccaatcgggt 1680
aactcccagg agagtgtcac agagcaggac agcaaggaca gcacctacag cctcagcagc 1740
accctgacgc tgagcaaagc agactacgag aaacacaaag tctacgcctg cgaagtcacc 1800
catcagggcc tgagctcgcc cgtcacaaag agcttcaaca ggggagagtg a 1851

<210> 872
<211> 1851
<212> DNA
<213> Artificial Sequence

<220>
<223> TSC163 (HD37 scFv-IgG3UH-CH2-CH3-Ck)

<400> 872
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcgagc ccaaatctag cgacacacct 840
cccccaagcc cacggtcccc atgcccaccg tgcccagcac ctgaagccgc gggtgcaccg 900
tcagtcttcc tcttcccccc aaaacccaag gacaccctca tgatctcccg gacccctgag 960
gtcacatgcg tggtggtgga cgtgagccac gaagaccctg aggtcaagtt caactggtac 1020
gtggacggcg tggaggtgca taatgccaag acaaagccgc gggaggagca gtacaacagc 1080
acgtaccgtg tggtcagcgt cctcaccgtc ctgcaccagg actggctgaa tggcaaggcg 1140
tacgcgtgcg cggtctccaa caaagccctc ccagccccca tcgagaaaac catctccaaa 1200
gccaaagggc agccccgaga accacaggtg tacaccctgc ccccatcccg ggatgagctg 1260
accaagaacc aggtcagcct gacctgcctg gtcaaaggct tctatccaag cgacatcgcc 1320
gtggagtggg agagcaatgg gcagccggag aacaactaca agaccacgcc tcccgtgctg 1380
gactccgacg gctccttctt cctctacagc aagctcaccg tggacaagag caggtggcag 1440
caggggaacg tcttctcatg ctccgtgatg catgaggctc tgcacaacca ctacacgcag 1500
aagagcctct ccctgtctcc gggtaaatct agaactgtgg ctgcaccatc tgtcttcatc 1560
ttcccgccat ctgatgagca gttgaaatct ggaactgcct ctgttgtgtg cctgctgaat 1620

tacttctatc ccagagaggc caaagtacag tggaaggtgg ataacgccct ccaatcgggt 1680
aactcccagg agagtgccac agagcaggac agcaaggaca gcacctacag cctcagcagc 1740
gagctgacgc tgagcaaagc agactacgag aaacacaaag tctacgcctg cgaagtcacc 1800
catcagggcc tgagctcgcc cgtcacaaag agcttcaaca ggggagagtg a 1851

<210> 873
<211> 2139
<212> DNA
<213> Artificial Sequence

<220>
<223> TSC164 (HD37 scFv-IgMCH2UH-CH2-CH3-Ck)

<400> 873
atggaagcac cagcgcagct tctcttcctc ctgctactct ggctcccaga taccaccggt 60
gacattgtgc tgacccaatc tccagcttct ttggctgtgt ctctagggca gagggccacc 120
atctcctgca aggccagcca aagtgttgat tatgatggtg atagttattt gaactggtac 180
caacagattc caggacagcc acccaaactc ctcatctatg atgcatccaa tctagtttct 240
gggatcccac ccaggtttag tggcagtggg tctgggacag acttcaccct caacatccat 300
cctgtggaga aggtggatgc tgcaacctat cactgccagc aaagtactga ggatccgtgg 360
acgttcggtg gaggcaccaa gctggaaatc aaaggtggcg gtggttcggg cggtggtggg 420
tcgggtggcg gcggagctag ccaggttcag ctgcagcagt ctggggctga gctggtgagg 480
cctgggtcct cagtgaagat ttcctgcaag gcttctggct atgcattcag tagctactgg 540
atgaactggg tgaagcagag gcctggacag ggtcttgagt ggattggaca gatttggcct 600
ggagatggtg atactaacta caatggaaag ttcaagggta aagccactct gactgcagac 660
gaatcctcca gcacagccta catgcaactc agcagcctag catctgagga ctctgcggtc 720
tatttctgtg caagacggga gactacgacg gtaggccgtt attactatgc tatggactac 780
tggggtcaag gaacctcagt caccgtctcc tcgagcattg ccgagctgcc tcccaaagtg 840
agcgtcttcg tcccaccccg cgacggcttc ttcggcaacc cccgcaagtc caagctcatc 900
tgccaggcca cgggtttcag tccccggcag attcaggtgt cctggctgcg cgaggggaag 960
caggtggggt ctggcgtcac cacggaccag gtgcaggctg aggccaaaga gtctgggccc 1020
acgacctaca aggtgaccag cacactgacc atcaaagaga gcgactggct cagccagagc 1080
atgttcacct gccgcgtgga tcacaggggc ctgaccttcc agcagaatgc gtcctccatg 1140
agtgtcccct gcccaccgtg cccagcacct gaagccgcgg gtgcaccgtc agtcttcctc 1200
ttccccccaa aacccaagga caccctcatg atctcccgga cccctgaggt cacatgcgtg 1260
gtggtggacg tgagccacga agaccctgag gtcaagttca actggtacgt ggacggcgtg 1320
gaggtgcata atgccaagac aaagccgcgg gaggagcagt acaacagcac gtaccgtgtg 1380
gtcagcgtcc tcaccgtcct gcaccaggac tggctgaatg gcaaggcgta cgcgtgcgcg 1440
gtctccaaca aagccctccc agcccccatc gagaaaacca tctccaaagc caaagggcag 1500
ccccgagaac cacaggtgta caccctgccc ccatcccggg atgagctgac caagaaccag 1560
gtcagcctga cctgcctggt caaaggcttc tatccaagcg acatcgccgt ggagtgggag 1620
agcaatgggc agccggagaa caactacaag accacgcctc ccgtgctgga ctccgacggc 1680
tccttcttcc tctacagcaa gctcaccgtg gacaagagca ggtggcagca ggggaacgtc 1740
ttctcatgct ccgtgatgca tgaggctctg cacaaccact acacgcagaa gagcctctcc 1800
ctgtctccgg gtaaatctag aactgtggct gcaccatctg tcttcatctt cccgccatct 1860
gatgagcagt tgaaatctgg aactgcctct gttgtgtgcc tgctgaatta cttctatccc 1920
agagaggcca aagtacagtg gaaggtggat aacgccctcc aatcgggtaa ctcccaggag 1980
agtgccacag agcaggacag caaggacagc acctacagcc tcagcagcga gctgacgctg 2040
agcaaagcag actacgagaa acacaaagtc tacgcctgcg aagtcaccca tcagggcctg 2100
agctcgcccg tcacaaagag cttcaacagg ggagagtga 2139

<210> 874
<211> 579
<212> PRT
<213> Artificial Sequence

<220>
<223> TSC125 (Cris7 scFv-CH2-CH3-CH1)

<400> 874
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser

20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Ala Ser Thr Lys Gly Pro Ser Val Phe Pro Leu Ala Pro Ser Ser
                485                 490                 495
Lys Ser Thr Ser Gly Gly Thr Ala Ala Leu Gly Cys Leu Val Lys Asp
            500                 505                 510
Tyr Phe Pro Glu Pro Val Thr Val Ser Trp Asn Ser Gly Ala Leu Thr
        515                 520                 525

Ser Gly Val His Thr Phe Pro Ala Val Leu Gln Ser Ser Gly Leu Tyr
    530                 535                 540
Ser Leu Ser Ser Val Val Thr Val Pro Ser Ser Ser Leu Gly Thr Gln
545                 550                 555                 560
Thr Tyr Ile Cys Asn Val Asn His Lys Pro Ser Asn Thr Lys Val Asp
                565                 570                 575
Lys Lys Val

<210> 875
<211> 574
<212> PRT
<213> Artificial Sequence

<220>
<223> TSC157 (Cris7 scFv-IgA2UH-CH2-CH3-CH1)

<400> 875
Gln Val Gln Leu Val Gln Ser Gly Gly Gly Val Val Gln Pro Gly Arg
 1               5                  10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Pro Pro Pro Pro Pro Cys Pro Pro Cys
                245                 250                 255
Pro Ala Pro Glu Ala Ala Gly Ala Pro Ser Val Phe Leu Phe Pro Pro
            260                 265                 270
Lys Pro Lys Asp Thr Leu Met Ile Ser Arg Thr Pro Glu Val Thr Cys
        275                 280                 285
Val Val Val Asp Val Ser His Glu Asp Pro Glu Val Lys Phe Asn Trp
    290                 295                 300
Tyr Val Asp Gly Val Glu Val His Asn Ala Lys Thr Lys Pro Arg Glu
305                 310                 315                 320
Glu Gln Tyr Asn Ser Thr Tyr Arg Val Val Ser Val Leu Thr Val Leu
                325                 330                 335
His Gln Asp Trp Leu Asn Gly Lys Ala Tyr Ala Cys Ala Val Ser Asn
            340                 345                 350
Lys Ala Leu Pro Ala Pro Ile Glu Lys Thr Ile Ser Lys Ala Lys Gly

                355                 360                 365
Gln Pro Arg Glu Pro Gln Val Tyr Thr Leu Pro Pro Ser Arg Asp Glu
    370                 375                 380
Leu Thr Lys Asn Gln Val Ser Leu Thr Cys Leu Val Lys Gly Phe Tyr
385                 390                 395                 400
Pro Ser Asp Ile Ala Val Glu Trp Glu Ser Asn Gly Gln Pro Glu Asn
                405                 410                 415
Asn Tyr Lys Thr Thr Pro Pro Val Leu Asp Ser Asp Gly Ser Phe Phe
            420                 425                 430
Leu Tyr Ser Lys Leu Thr Val Asp Lys Ser Arg Trp Gln Gln Gly Asn
        435                 440                 445
Val Phe Ser Cys Ser Val Met His Glu Ala Leu His Asn His Tyr Thr
    450                 455                 460
Gln Lys Ser Leu Ser Leu Ser Pro Gly Lys Ser Arg Ala Ser Thr Lys
465                 470                 475                 480
Gly Pro Ser Val Phe Pro Leu Ala Pro Ser Ser Lys Ser Thr Ser Gly
                485                 490                 495
Gly Thr Ala Ala Leu Gly Cys Leu Val Lys Asp Tyr Phe Pro Glu Pro
            500                 505                 510
Val Thr Val Ser Trp Asn Ser Gly Ala Leu Thr Ser Gly Val His Thr
        515                 520                 525
Phe Pro Ala Val Leu Gln Ser Ser Gly Leu Tyr Ser Leu Ser Ser Val
    530                 535                 540
Val Thr Val Pro Ser Ser Ser Leu Gly Thr Gln Thr Tyr Ile Cys Asn
545                 550                 555                 560
Val Asn His Lys Pro Ser Asn Thr Lys Val Asp Lys Lys Val
                565                 570

<210> 876
<211> 584
<212> PRT
<213> Artificial Sequence

<220>
<223> TSC158 (Cris7 scFv-IgG1miniUH-CH2-CH3-CH1)

<400> 876
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205

Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Lys Thr His
                245                 250                 255
Thr Ser Pro Pro Ser Pro Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Lys Ser Arg Ala Ser Thr Lys Gly Pro Ser Val Phe Pro
                485                 490                 495
Leu Ala Pro Ser Ser Lys Ser Thr Ser Gly Gly Thr Ala Ala Leu Gly
            500                 505                 510
Cys Leu Val Lys Asp Tyr Phe Pro Glu Pro Val Thr Val Ser Trp Asn
        515                 520                 525
Ser Gly Ala Leu Thr Ser Gly Val His Thr Phe Pro Ala Val Leu Gln
    530                 535                 540
Ser Ser Gly Leu Tyr Ser Leu Ser Ser Val Val Thr Val Pro Ser Ser
545                 550                 555                 560
Ser Leu Gly Thr Gln Thr Tyr Ile Cys Asn Val Asn His Lys Pro Ser
                565                 570                 575
Asn Thr Lys Val Asp Lys Lys Val
            580

<210> 877
<211> 584
<212> PRT
<213> Artificial Sequence

<220>
<223> TSC159 (Cris7 scFv-IgG3UH-CH2-CH3-CH1)

<400> 877
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile

35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Glu Pro Lys Ser Ser Asp Thr Pro Pro
                245                 250                 255
Pro Ser Pro Arg Ser Pro Cys Pro Pro Cys Pro Ala Pro Glu Ala Ala
            260                 265                 270
Gly Ala Pro Ser Val Phe Leu Phe Pro Pro Lys Pro Lys Asp Thr Leu
        275                 280                 285
Met Ile Ser Arg Thr Pro Glu Val Thr Cys Val Val Val Asp Val Ser
    290                 295                 300
His Glu Asp Pro Glu Val Lys Phe Asn Trp Tyr Val Asp Gly Val Glu
305                 310                 315                 320
Val His Asn Ala Lys Thr Lys Pro Arg Glu Glu Gln Tyr Asn Ser Thr
                325                 330                 335
Tyr Arg Val Val Ser Val Leu Thr Val Leu His Gln Asp Trp Leu Asn
            340                 345                 350
Gly Lys Ala Tyr Ala Cys Ala Val Ser Asn Lys Ala Leu Pro Ala Pro
        355                 360                 365
Ile Glu Lys Thr Ile Ser Lys Ala Lys Gly Gln Pro Arg Glu Pro Gln
    370                 375                 380
Val Tyr Thr Leu Pro Pro Ser Arg Asp Glu Leu Thr Lys Asn Gln Val
385                 390                 395                 400
Ser Leu Thr Cys Leu Val Lys Gly Phe Tyr Pro Ser Asp Ile Ala Val
                405                 410                 415
Glu Trp Glu Ser Asn Gly Gln Pro Glu Asn Asn Tyr Lys Thr Thr Pro
            420                 425                 430
Pro Val Leu Asp Ser Asp Gly Ser Phe Phe Leu Tyr Ser Lys Leu Thr
        435                 440                 445
Val Asp Lys Ser Arg Trp Gln Gln Gly Asn Val Phe Ser Cys Ser Val
    450                 455                 460
Met His Glu Ala Leu His Asn His Tyr Thr Gln Lys Ser Leu Ser Leu
465                 470                 475                 480
Ser Pro Gly Lys Ser Arg Ala Ser Thr Lys Gly Pro Ser Val Phe Pro
                485                 490                 495
Leu Ala Pro Ser Ser Lys Ser Thr Ser Gly Gly Thr Ala Ala Leu Gly
            500                 505                 510
Cys Leu Val Lys Asp Tyr Phe Pro Glu Pro Val Thr Val Ser Trp Asn
        515                 520                 525
Ser Gly Ala Leu Thr Ser Gly Val His Thr Phe Pro Ala Val Leu Gln
    530                 535                 540

Ser Ser Gly Leu Tyr Ser Leu Ser Ser Val Val Thr Val Pro Ser Ser
545                 550                 555                 560
Ser Leu Gly Thr Gln Thr Tyr Ile Cys Asn Val Asn His Lys Pro Ser
                565                 570                 575
Asn Thr Lys Val Asp Lys Lys Val
            580

<210> 878
<211> 680
<212> PRT
<213> Artificial Sequence

<220>
<223> TSC160 (Cris7 scFv-IgMCH2UH-CH2-CH3-CH1)

<400> 878
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Ser
            20                  25                  30
Thr Met His Trp Val Arg Gln Ala Pro Gly Lys Gly Leu Glu Trp Ile
        35                  40                  45
Gly Tyr Ile Asn Pro Ser Ser Ala Tyr Thr Asn Tyr Asn Gln Lys Phe
    50                  55                  60
Lys Asp Arg Phe Thr Ile Ser Ala Asp Lys Ser Lys Ser Thr Ala Phe
65                  70                  75                  80
Leu Gln Met Asp Ser Leu Arg Pro Glu Asp Thr Gly Val Tyr Phe Cys
                85                  90                  95
Ala Arg Pro Gln Val His Tyr Asp Tyr Asn Gly Phe Pro Tyr Trp Gly
            100                 105                 110
Gln Gly Thr Pro Val Thr Val Ser Ser Gly Gly Gly Gly Ser Gly Gly
        115                 120                 125
Gly Gly Ser Gly Gly Gly Gly Ser Ala Gln Asp Ile Gln Met Thr Gln
    130                 135                 140
Ser Pro Ser Ser Leu Ser Ala Ser Val Gly Asp Arg Val Thr Met Thr
145                 150                 155                 160
Cys Ser Ala Ser Ser Ser Val Ser Tyr Met Asn Trp Tyr Gln Gln Lys
                165                 170                 175
Pro Gly Lys Ala Pro Lys Arg Trp Ile Tyr Asp Ser Ser Lys Leu Ala
            180                 185                 190
Ser Gly Val Pro Ala Arg Phe Ser Gly Ser Gly Ser Gly Thr Asp Tyr
        195                 200                 205
Thr Leu Thr Ile Ser Ser Leu Gln Pro Glu Asp Phe Ala Thr Tyr Tyr
    210                 215                 220
Cys Gln Gln Trp Ser Arg Asn Pro Pro Thr Phe Gly Gly Gly Thr Lys
225                 230                 235                 240
Leu Gln Ile Thr Arg Ser Ser Ile Ala Glu Leu Pro Pro Lys Val Ser
                245                 250                 255
Val Phe Val Pro Pro Arg Asp Gly Phe Phe Gly Asn Pro Arg Lys Ser
            260                 265                 270
Lys Leu Ile Cys Gln Ala Thr Gly Phe Ser Pro Arg Gln Ile Gln Val
        275                 280                 285
Ser Trp Leu Arg Glu Gly Lys Gln Val Gly Ser Gly Val Thr Thr Asp
    290                 295                 300
Gln Val Gln Ala Glu Ala Lys Glu Ser Gly Pro Thr Thr Tyr Lys Val
305                 310                 315                 320
Thr Ser Thr Leu Thr Ile Lys Glu Ser Asp Trp Leu Ser Gln Ser Met
                325                 330                 335
Phe Thr Cys Arg Val Asp His Arg Gly Leu Thr Phe Gln Gln Asn Ala
            340                 345                 350
Ser Ser Met Ser Val Pro Cys Pro Pro Cys Pro Ala Pro Glu Ala Ala
        355                 360                 365
Gly Ala Pro Ser Val Phe Leu Phe Pro Pro Lys Pro Lys Asp Thr Leu

370                 375                 380
Met Ile Ser Arg Thr Pro Glu Val Thr Cys Val Val Val Asp Val Ser
385                 390                 395                 400
His Glu Asp Pro Glu Val Lys Phe Asn Trp Tyr Val Asp Gly Val Glu
                405                 410                 415
Val His Asn Ala Lys Thr Lys Pro Arg Glu Glu Gln Tyr Asn Ser Thr
            420                 425                 430
Tyr Arg Val Val Ser Val Leu Thr Val Leu His Gln Asp Trp Leu Asn
        435                 440                 445
Gly Lys Ala Tyr Ala Cys Ala Val Ser Asn Lys Ala Leu Pro Ala Pro
    450                 455                 460
Ile Glu Lys Thr Ile Ser Lys Ala Lys Gly Gln Pro Arg Glu Pro Gln
465                 470                 475                 480
Val Tyr Thr Leu Pro Pro Ser Arg Asp Glu Leu Thr Lys Asn Gln Val
                485                 490                 495
Ser Leu Thr Cys Leu Val Lys Gly Phe Tyr Pro Ser Asp Ile Ala Val
            500                 505                 510
Glu Trp Glu Ser Asn Gly Gln Pro Glu Asn Asn Tyr Lys Thr Thr Pro
        515                 520                 525
Pro Val Leu Asp Ser Asp Gly Ser Phe Phe Leu Tyr Ser Lys Leu Thr
    530                 535                 540
Val Asp Lys Ser Arg Trp Gln Gln Gly Asn Val Phe Ser Cys Ser Val
545                 550                 555                 560
Met His Glu Ala Leu His Asn His Tyr Thr Gln Lys Ser Leu Ser Leu
                565                 570                 575
Ser Pro Gly Lys Ser Arg Ala Ser Thr Lys Gly Pro Ser Val Phe Pro
            580                 585                 590
Leu Ala Pro Ser Ser Lys Ser Thr Ser Gly Gly Thr Ala Ala Leu Gly
        595                 600                 605
Cys Leu Val Lys Asp Tyr Phe Pro Glu Pro Val Thr Val Ser Trp Asn
    610                 615                 620
Ser Gly Ala Leu Thr Ser Gly Val His Thr Phe Pro Ala Val Leu Gln
625                 630                 635                 640
Ser Ser Gly Leu Tyr Ser Leu Ser Ser Val Val Thr Val Pro Ser Ser
                645                 650                 655
Ser Leu Gly Thr Gln Thr Tyr Ile Cys Asn Val Asn His Lys Pro Ser
            660                 665                 670
Asn Thr Lys Val Asp Lys Lys Val
        675                 680

<210> 879
<211> 586
<212> PRT
<213> Artificial Sequence

<220>
<223> TSC161 (HD37 scFv-IgA2UH-CH2-CH3-Ck)

<400> 879
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110

Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Pro Pro Pro Pro
                245                 250                 255
Pro Cys Pro Pro Cys Pro Ala Pro Glu Ala Ala Gly Ala Pro Ser Val
            260                 265                 270
Phe Leu Phe Pro Pro Lys Pro Lys Asp Thr Leu Met Ile Ser Arg Thr
        275                 280                 285
Pro Glu Val Thr Cys Val Val Val Asp Val Ser His Glu Asp Pro Glu
    290                 295                 300
Val Lys Phe Asn Trp Tyr Val Asp Gly Val Glu Val His Asn Ala Lys
305                 310                 315                 320
Thr Lys Pro Arg Glu Glu Gln Tyr Asn Ser Thr Tyr Arg Val Val Ser
                325                 330                 335
Val Leu Thr Val Leu His Gln Asp Trp Leu Asn Gly Lys Ala Tyr Ala
            340                 345                 350
Cys Ala Val Ser Asn Lys Ala Leu Pro Ala Pro Ile Glu Lys Thr Ile
        355                 360                 365
Ser Lys Ala Lys Gly Gln Pro Arg Glu Pro Gln Val Tyr Thr Leu Pro
    370                 375                 380
Pro Ser Arg Asp Glu Leu Thr Lys Asn Gln Val Ser Leu Thr Cys Leu
385                 390                 395                 400
Val Lys Gly Phe Tyr Pro Ser Asp Ile Ala Val Glu Trp Glu Ser Asn
                405                 410                 415
Gly Gln Pro Glu Asn Asn Tyr Lys Thr Thr Pro Pro Val Leu Asp Ser
            420                 425                 430
Asp Gly Ser Phe Phe Leu Tyr Ser Lys Leu Thr Val Asp Lys Ser Arg
        435                 440                 445
Trp Gln Gln Gly Asn Val Phe Ser Cys Ser Val Met His Glu Ala Leu
    450                 455                 460
His Asn His Tyr Thr Gln Lys Ser Leu Ser Leu Ser Pro Gly Lys Ser
465                 470                 475                 480
Arg Thr Val Ala Ala Pro Ser Val Phe Ile Phe Pro Pro Ser Asp Glu
                485                 490                 495
Gln Leu Lys Ser Gly Thr Ala Ser Val Val Cys Leu Leu Asn Tyr Phe
            500                 505                 510
Tyr Pro Arg Glu Ala Lys Val Gln Trp Lys Val Asp Asn Ala Leu Gln
        515                 520                 525
Ser Gly Asn Ser Gln Glu Ser Ala Thr Glu Gln Asp Ser Lys Asp Ser
    530                 535                 540
Thr Tyr Ser Leu Ser Ser Glu Leu Thr Leu Ser Lys Ala Asp Tyr Glu
545                 550                 555                 560
Lys His Lys Val Tyr Ala Cys Glu Val Thr His Gln Gly Leu Ser Ser
                565                 570                 575
Pro Val Thr Lys Ser Phe Asn Arg Gly Glu
            580                 585

<210> 880
<211> 596

<212> PRT
<213> Artificial Sequence

<220>
<223> TSC162 (HD37 scFv-IgG1miniUH-CH2-CH3-Ck)

<400> 880
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Lys Thr His Thr Ser Pro Pro Ser Pro Cys Pro Pro Cys Pro
            260                 265                 270
Ala Pro Glu Ala Ala Gly Ala Pro Ser Val Phe Leu Phe Pro Pro Lys
        275                 280                 285
Pro Lys Asp Thr Leu Met Ile Ser Arg Thr Pro Glu Val Thr Cys Val
    290                 295                 300
Val Val Asp Val Ser His Glu Asp Pro Glu Val Lys Phe Asn Trp Tyr
305                 310                 315                 320
Val Asp Gly Val Glu Val His Asn Ala Lys Thr Lys Pro Arg Glu Glu
                325                 330                 335
Gln Tyr Asn Ser Thr Tyr Arg Val Val Ser Val Leu Thr Val Leu His
            340                 345                 350
Gln Asp Trp Leu Asn Gly Lys Ala Tyr Ala Cys Ala Val Ser Asn Lys
        355                 360                 365
Ala Leu Pro Ala Pro Ile Glu Lys Thr Ile Ser Lys Ala Lys Gly Gln
    370                 375                 380
Pro Arg Glu Pro Gln Val Tyr Thr Leu Pro Pro Ser Arg Asp Glu Leu
385                 390                 395                 400
Thr Lys Asn Gln Val Ser Leu Thr Cys Leu Val Lys Gly Phe Tyr Pro
                405                 410                 415
Ser Asp Ile Ala Val Glu Trp Glu Ser Asn Gly Gln Pro Glu Asn Asn
            420                 425                 430
Tyr Lys Thr Thr Pro Pro Val Leu Asp Ser Asp Gly Ser Phe Phe Leu
        435                 440                 445

Tyr Ser Lys Leu Thr Val Asp Lys Ser Arg Trp Gln Gln Gly Asn Val
    450                 455                 460
Phe Ser Cys Ser Val Met His Glu Ala Leu His Asn His Tyr Thr Gln
465                 470                 475                 480
Lys Ser Leu Ser Leu Ser Pro Gly Lys Ser Arg Thr Val Ala Ala Pro
                485                 490                 495
Ser Val Phe Ile Phe Pro Pro Ser Asp Glu Gln Leu Lys Ser Gly Thr
            500                 505                 510
Ala Ser Val Val Cys Leu Leu Asn Tyr Phe Tyr Pro Arg Glu Ala Lys
        515                 520                 525
Val Gln Trp Lys Val Asp Asn Ala Leu Gln Ser Gly Asn Ser Gln Glu
    530                 535                 540
Ser Ala Thr Glu Gln Asp Ser Lys Asp Ser Thr Tyr Ser Leu Ser Ser
545                 550                 555                 560
Glu Leu Thr Leu Ser Lys Ala Asp Tyr Glu Lys His Lys Val Tyr Ala
                565                 570                 575
Cys Glu Val Thr His Gln Gly Leu Ser Ser Pro Val Thr Lys Ser Phe
            580                 585                 590
Asn Arg Gly Glu
        595

<210> 881
<211> 596
<212> PRT
<213> Artificial Sequence

<220>
<223> TSC163 (HD37 scFv-IgG3UH-CH2-CH3-Ck)

<400> 881
Asp Ile Val Leu Thr Gln Ser Pro Ala Ser Leu Ala Val Ser Leu Gly
 1               5                  10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80
Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Glu Pro Lys Ser
                245                 250                 255
Ser Asp Thr Pro Pro Pro Ser Pro Arg Ser Pro Cys Pro Pro Cys Pro

260                 265                 270
Ala Pro Glu Ala Ala Gly Ala Pro Ser Val Phe Leu Phe Pro Pro Lys
        275                 280                 285
Pro Lys Asp Thr Leu Met Ile Ser Arg Thr Pro Glu Val Thr Cys Val
    290                 295                 300
Val Val Asp Val Ser His Glu Asp Pro Glu Val Lys Phe Asn Trp Tyr
305                 310                 315                 320
Val Asp Gly Val Glu Val His Asn Ala Lys Thr Lys Pro Arg Glu Glu
                325                 330                 335
Gln Tyr Asn Ser Thr Tyr Arg Val Val Ser Val Leu Thr Val Leu His
            340                 345                 350
Gln Asp Trp Leu Asn Gly Lys Ala Tyr Ala Cys Ala Val Ser Asn Lys
        355                 360                 365
Ala Leu Pro Ala Pro Ile Glu Lys Thr Ile Ser Lys Ala Lys Gly Gln
    370                 375                 380
Pro Arg Glu Pro Gln Val Tyr Thr Leu Pro Pro Ser Arg Asp Glu Leu
385                 390                 395                 400
Thr Lys Asn Gln Val Ser Leu Thr Cys Leu Val Lys Gly Phe Tyr Pro
                405                 410                 415
Ser Asp Ile Ala Val Glu Trp Glu Ser Asn Gly Gln Pro Glu Asn Asn
            420                 425                 430
Tyr Lys Thr Thr Pro Pro Val Leu Asp Ser Asp Gly Ser Phe Phe Leu
        435                 440                 445
Tyr Ser Lys Leu Thr Val Asp Lys Ser Arg Trp Gln Gln Gly Asn Val
    450                 455                 460
Phe Ser Cys Ser Val Met His Glu Ala Leu His Asn His Tyr Thr Gln
465                 470                 475                 480
Lys Ser Leu Ser Leu Ser Pro Gly Lys Ser Arg Thr Val Ala Ala Pro
                485                 490                 495
Ser Val Phe Ile Phe Pro Pro Ser Asp Glu Gln Leu Lys Ser Gly Thr
            500                 505                 510
Ala Ser Val Val Cys Leu Leu Asn Tyr Phe Tyr Pro Arg Glu Ala Lys
        515                 520                 525
Val Gln Trp Lys Val Asp Asn Ala Leu Gln Ser Gly Asn Ser Gln Glu
    530                 535                 540
Ser Ala Thr Glu Gln Asp Ser Lys Asp Ser Thr Tyr Ser Leu Ser Ser
545                 550                 555                 560
Glu Leu Thr Leu Ser Lys Ala Asp Tyr Glu Lys His Lys Val Tyr Ala
                565                 570                 575
Cys Glu Val Thr His Gln Gly Leu Ser Ser Pro Val Thr Lys Ser Phe
            580                 585                 590
Asn Arg Gly Glu
        595

<210> 882
<211> 692
<212> PRT
<213> Artificial Sequence

<220>
<223> TSC164 (HD37 scFv-IgMCH2UH-CH2-CH3-Ck)

<400> 882
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Leu Asn Trp Tyr Gln Gln Ile Pro Gly Gln Pro Pro
        35                  40                  45
Lys Leu Leu Ile Tyr Asp Ala Ser Asn Leu Val Ser Gly Ile Pro Pro
    50                  55                  60
Arg Phe Ser Gly Ser Gly Ser Gly Thr Asp Phe Thr Leu Asn Ile His
65                  70                  75                  80

Pro Val Glu Lys Val Asp Ala Ala Thr Tyr His Cys Gln Gln Ser Thr
                85                  90                  95
Glu Asp Pro Trp Thr Phe Gly Gly Gly Thr Lys Leu Glu Ile Lys Gly
            100                 105                 110
Gly Gly Gly Ser Gly Gly Gly Gly Ser Gly Gly Gly Gly Ala Ser Gln
        115                 120                 125
Val Gln Leu Gln Gln Ser Gly Ala Glu Leu Val Arg Pro Gly Ser Ser
    130                 135                 140
Val Lys Ile Ser Cys Lys Ala Ser Gly Tyr Ala Phe Ser Ser Tyr Trp
145                 150                 155                 160
Met Asn Trp Val Lys Gln Arg Pro Gly Gln Gly Leu Glu Trp Ile Gly
                165                 170                 175
Gln Ile Trp Pro Gly Asp Gly Asp Thr Asn Tyr Asn Gly Lys Phe Lys
            180                 185                 190
Gly Lys Ala Thr Leu Thr Ala Asp Glu Ser Ser Ser Thr Ala Tyr Met
        195                 200                 205
Gln Leu Ser Ser Leu Ala Ser Glu Asp Ser Ala Val Tyr Phe Cys Ala
    210                 215                 220
Arg Arg Glu Thr Thr Thr Val Gly Arg Tyr Tyr Tyr Ala Met Asp Tyr
225                 230                 235                 240
Trp Gly Gln Gly Thr Ser Val Thr Val Ser Ser Ser Ile Ala Glu Leu
                245                 250                 255
Pro Pro Lys Val Ser Val Phe Val Pro Pro Arg Asp Gly Phe Phe Gly
            260                 265                 270
Asn Pro Arg Lys Ser Lys Leu Ile Cys Gln Ala Thr Gly Phe Ser Pro
        275                 280                 285
Arg Gln Ile Gln Val Ser Trp Leu Arg Glu Gly Lys Gln Val Gly Ser
    290                 295                 300
Gly Val Thr Thr Asp Gln Val Gln Ala Glu Ala Lys Glu Ser Gly Pro
305                 310                 315                 320
Thr Thr Tyr Lys Val Thr Ser Thr Leu Thr Ile Lys Glu Ser Asp Trp
                325                 330                 335
Leu Ser Gln Ser Met Phe Thr Cys Arg Val Asp His Arg Gly Leu Thr
            340                 345                 350
Phe Gln Gln Asn Ala Ser Ser Met Ser Val Pro Cys Pro Pro Cys Pro
        355                 360                 365
Ala Pro Glu Ala Ala Gly Ala Pro Ser Val Phe Leu Phe Pro Pro Lys
    370                 375                 380
Pro Lys Asp Thr Leu Met Ile Ser Arg Thr Pro Glu Val Thr Cys Val
385                 390                 395                 400
Val Val Asp Val Ser His Glu Asp Pro Glu Val Lys Phe Asn Trp Tyr
                405                 410                 415
Val Asp Gly Val Glu Val His Asn Ala Lys Thr Lys Pro Arg Glu Glu
            420                 425                 430
Gln Tyr Asn Ser Thr Tyr Arg Val Val Ser Val Leu Thr Val Leu His
        435                 440                 445
Gln Asp Trp Leu Asn Gly Lys Ala Tyr Ala Cys Ala Val Ser Asn Lys
    450                 455                 460
Ala Leu Pro Ala Pro Ile Glu Lys Thr Ile Ser Lys Ala Lys Gly Gln
465                 470                 475                 480
Pro Arg Glu Pro Gln Val Tyr Thr Leu Pro Pro Ser Arg Asp Glu Leu
                485                 490                 495
Thr Lys Asn Gln Val Ser Leu Thr Cys Leu Val Lys Gly Phe Tyr Pro
            500                 505                 510
Ser Asp Ile Ala Val Glu Trp Glu Ser Asn Gly Gln Pro Glu Asn Asn
        515                 520                 525
Tyr Lys Thr Thr Pro Pro Val Leu Asp Ser Asp Gly Ser Phe Phe Leu
    530                 535                 540
Tyr Ser Lys Leu Thr Val Asp Lys Ser Arg Trp Gln Gln Gly Asn Val
545                 550                 555                 560
Phe Ser Cys Ser Val Met His Glu Ala Leu His Asn His Tyr Thr Gln
                565                 570                 575
Lys Ser Leu Ser Leu Ser Pro Gly Lys Ser Arg Thr Val Ala Ala Pro

                580                 585                 590
Ser Val Phe Ile Phe Pro Pro Ser Asp Glu Gln Leu Lys Ser Gly Thr
        595                 600                 605
Ala Ser Val Val Cys Leu Leu Asn Tyr Phe Tyr Pro Arg Glu Ala Lys
    610                 615                 620
Val Gln Trp Lys Val Asp Asn Ala Leu Gln Ser Gly Asn Ser Gln Glu
625                 630                 635                 640
Ser Ala Thr Glu Gln Asp Ser Lys Asp Ser Thr Tyr Ser Leu Ser Ser
                645                 650                 655
Glu Leu Thr Leu Ser Lys Ala Asp Tyr Glu Lys His Lys Val Tyr Ala
            660                 665                 670
Cys Glu Val Thr His Gln Gly Leu Ser Ser Pro Val Thr Lys Ser Phe
        675                 680                 685
Asn Arg Gly Glu
    690

## Description

### CROSS-REFERENCE(S) TO RELATED APPLICATION(S)

This application claims the benefit under 35 U.S.C. § 119(e) of U.S. Provisional Patent Application No. 61/290,840, filed December 29, 2009, and U.S. Provisional Patent Application No. 61/365,266, filed July 16, 2010, each of which is herein incorporated by reference in its entirety.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is 910180_422PC_SEQUENCE_LISTING.txt. The text file is 839 KB, was created on December 29, 2010, and is being submitted electronically via EFS-Web, concurrent with the filing of the specification.

### BACKGROUND

### Technical Field

The present disclosure generally provides polypeptide heterodimers, compositions thereof, and methods for making and using such polypeptide heterodimers. More specifically, the polypeptide heterodimers provided herein are formed, in part, via natural heterodimerization between an immunoglobulin CH1 region and an immunoglobulin light chain constant region (CL). In addition, the polypeptide heterodimers provided herein comprise two or more binding domains that specifically bind one or more targets. Furthermore, both single chain polypeptides of the polypeptide heterodimers provided herein each comprise an Fc region portion (e.g., immunoglobulin CH2 and CH3 domains).

### Description of the Related Art

The process of signal transduction often involves receptor proteins that have extracellular domains, transmembrane domains, and intracellular domains. During ligand binding, cell surface receptor molecules often oligomerize or multimerize (also referred to as "cross-link") to effectively transmit a signal to the intracellular compartment of the cell. The stimulation or blockade of this interaction between a receptor and a ligand or of the subsequent oligomerization or multimerization of receptors has important therapeutic implications for a wide variety of diseases.

Exemplary molecules useful in modulating receptor and ligand interactions include antibodies or molecules derived from antibodies. For instance, an antibody or its derivative may function as a receptor antagonist that binds to a cell surface receptor and inactivates it by blocking the binding site of an activating ligand or preventing receptor dimerization or multimerization required for activation. In certain other cases, an antibody or its derivative may function as an agonist by binding to and cross-linking multiple membrane receptors, mimicking the function of a natural ligand. Another example is a bispecific antibody derivative that may be used to direct cytotoxic agents or immune effector cells to target sites, such as tumors.

Bispecific antibodies are antibody-based molecules that can simultaneously bind two separate and distinct antigens (or different epitopes of the same antigen). One use of bispecific antibodies has been to redirect cytotoxic immune effector cells for enhanced killing of tumor cells by antibody dependent cellular cytotoxicity (ADCC). In this context, one arm of the bispecific antibody binds an antigen on the tumor cell, and the other binds a determinant expressed on effector cells. By cross-linking tumor and effector cells, the bispecific antibody not only brings the effector cells within the proximity of the tumor cells but also simultaneously triggers their activation, leading to effective tumor cell-killing. Bispecific antibodies have also been used to enrich chemo- or radiotherapeutic agents in tumor tissues to minimize detrimental effects to normal tissue. In this setting, one arm of the bispecific antibody binds an antigen expressed on the cell targeted for destruction, and the other arm delivers a chemotherapeutic drug, radioisotope, or toxin.

A major obstacle in the general development of bispecific antibodies has been the difficulty of producing materials of sufficient quality and quantity for both preclinical and clinical studies. Initially, the main route to the production of bispecific antibodies was by co-expression of both the light chains and both the heavy chains of two parent antibodies of different specificities in a single cell. However, the desired binding-competent bispecific antibodies are a minor product, and purification from the other products is very difficult. Another traditional method for bispecific antibody production is chemical conjugation of two antibodies or their fragments having different specificities. However, this method is also complicated, and the chemical modification process may inactivate the antibody or promote aggregation. Because purification from undesired products remains difficult, the resulting low yield and poor quality of bispecific antibody make this process unsuitable for the large scale production required for clinical development.

Recently, various heterodimerization techniques have been used to improve the production of bispecific antibodies. However, fusion of simple heterodimerization domains like the Jun/Fos coiled-coil to scFv domains lead to a mixture of homo- and heterodimers and need to be assembled by refolding (de Kruif and Logtenberg, J. Biol. Chem. 271: 7630-4, 1996). Fusion of scFv fragments to whole antibodies was also used as a dimerization device (Coloma and Morrison, Nat. Biotechnol. 15:159-63, 1997). However, such fusion results in a large molecule with poor solid tissue penetration capabilities. Fusion of two scFv fragments together has also been used to generate bispecific proteins (e.g., BITE® antibodies by Micromet Inc., Bethesda, MD, U.S. Patent No. 7,635,472). However, such proteins do not contain Fc regions, and thus do not allow manipulation of their activities via Fc regions. In addition, these proteins are small (∼55 kDa) and thus have relatively short half lives in serum.

To date, immunoglobulin fusion technology has not provided commercially viable heterodimeric proteins or methods for making them. Thus, there remains a need in the art for alternative multispecific heterodimeric proteins as well as efficient methods for producing the same.

### BRIEF SUMMARY

The present disclosure provides polypeptide heterodimers formed between two different single chain polypeptides via natural heterodimerization of an immunoglobulin CH1 region and an immunoglobulin light chain constant region (CL). The present disclosure also provides nucleic acids, vectors, host cells and methods for making polypeptide heterodimers as well as methods for using such polypeptide heterodimers, such as in directed T cell activation, inhibiting solid malignancy growth, treating autoimmune or inflammatory conditions, or treating B-cell associated disorders or diseases.

In one aspect, the present disclosure provides a polypeptide heterodimer that comprises (a) a first single chain polypeptide (SCP-I) comprising from one to four binding domains that specifically bind from one to four targets, a hinge (H-I), an immunoglobulin heterodimerization domain (HD-I), and an Fc region portion (FRP-I); and (b) a second single chain polypeptide (SCP-II) comprising from zero to four binding domains that specifically bind from zero to four targets, a hinge (H-II), an immunoglobulin heterodimerization domain (HD-II), and an Fc region portion (FRP-II); wherein (i) the immunoglobulin HD-I and the immunoglobulin HD-II preferentially associate with each other to form a polypeptide heterodimer comprised of SCP-I and SCP-II, and (1) the immunoglobulin heterodimerization domain of the first single chain polypeptide (HD-I) comprises a first immunoglobulin CH1 region, and the immunoglobulin heterodimerization domain of the second single chain polypeptide (HD-II) comprises a first immunoglobulin CL region, or (2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region; and (ii) the Fc region portion of SCP-I and the Fc region portion of SCP-II comprise an immunoglobulin CH2 and CH3 domain of IgG1, IgG2, IgG3, IgG4, IgA1, IgA2, IgD, or any combination thereof; one or two immunoglobulin CH3 domains of IgG1, IgG2, IgG3, IgG4, IgA1, IgA2, IgD, IgE, IgM, or any combination thereof; or an immunoglobulin CH3 and CH4 domain of IgE, IgM, or any combination thereof, provided that the polypeptide heterodimer comprises at least two binding domains that specifically bind a target, for instance, at least two different targets.

In certain embodiments, the binding domains of the polypeptide heterodimers are single chain Fv (scFv) polypeptides.

In certain embodiments, the polypeptide heterodimer comprises two binding domains (BD1 and BD2). In one embodiment, the two binding domains (BD1 and BD2) are both on the first single chain polypeptide (SCP-I) and wherein the HD-I and FRP-I are disposed between BD1 and BD2. In another embodiment, the first binding domain (BD1) is on the first single chain polypeptide (SCP-I) and the second binding domain (BD2) is on the second single chain polypeptide (SCP-II). For example, the first binding domain (BD1) may be amino terminal to the Fc region portion of the first single chain polypeptide (FRP-I), and the second binding domain (BD2) may amino terminal to the Fc region portion of the second single chain polypeptide (FRP-II). Alternatively, the first binding domain (BD1) may be amino terminal to the Fc region portion of the first single chain polypeptide (FRP-I), and the second binding domain (BD2) may be carboxyl terminal to the Fc region portion of the second single chain polypeptide (FRP-II). Also alternatively, the first binding domain (BD1) may be carboxyl terminal to the Fc region portion of the first single chain polypeptide (FRP-I), and the second binding domain (BD2) may be carboxyl terminal to the Fc region portion of the second single chain polypeptide (FRP-II).

In certain embodiments, the polypeptide heterodimer comprises three binding domains (BD1, BD2 and BD3). In one embodiment, the HD-I and FRP-I are disposed between BD1 and BD2, and the third binding domain (BD3) is amino terminal to the Fc region portion of the second single chain polypeptide (FRP-II). In an alternative embodiment, the HD-I and FRP-I are disposed between BD1 and BD2, and the third binding domain (BD3) is carboxyl terminal to the Fc region portion of the second single chain polypeptide (FRP-II).

In certain embodiments, the polypeptide heterodimer comprises four binding domains (BD1, BD2, BD3, and BD4). For instance, the HD-I and FRP-I may be disposed between BD1 and BD2, and the HD-II and FRP-II may be disposed between BD3 and BD4.

In certain embodiments, the polypeptide heterodimer comprises five to eight binding domains *(e.g.,* 5, 6, 7 or 8 binding domains).

In certain embodiments, at least one of the binding domains of the polypeptide heterodimers provided herein specifically binds to, or is an antagonist of, TCRα, TCRβ, CD3γ, CD3δ, CD3ε, CD28, CD79b, hyperIL-6, monoIL-10, CD86, CD20, PSMA, CD 19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACI, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL, , HGF, MSP, EGF (including epiregulin, herregulin, β-regulin, neuregulin), HIF-1α, VEGFA, VEGFB, VEGFC, VEGFD, TNFα, Wnt, sHH, TGFβ, PDGF, TWEAK, EpCAM, CEA, PCTA-1, STEAP-1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, ganglioside GD3, 9-O-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD137, LIFRβ, TLR7 or TLR9.

In certain other embodiments, at least one of the binding domains of the polypeptide heterodimer is an agonist of IL-10, HLA-G, HGF, IL-35, PD-1, BTLA, TNFR1, TNFR2, DR4, DR5, TWEAKR, or FAS.

In certain polypeptide heterodimers provided herein, at least one binding domain specifically binds a TCR complex or a component thereof, and at least another binding domain specifically binds to PSMA, CD79b, CD19, HLA-DR, CD20, RON, c-Met, or CEACAM-6.

In certain other polypeptide heterodimers provided herein, at least one binding domain specifically binds to CD28, and at least another binding domain specifically binds to, or is an antagonist of, CD79b, hyperIL-6, PDL2, monoIL-10, CD86, LIGHT, GITRL, CD40, PDL1, HVEM, or LTBR.

In certain other polypeptide heterodimers provided herein, at least one binding domain specifically binds to CD28, and at least another binding domain is an agonist of IL-10, HLA-G, HGF, IL-35, PD-1, or BTLA.

In certain embodiments, the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region. In one embodiment, the first CH1 region is amino terminal to the Fc region portion of the first single chain polypeptide, and the first CL region is amino terminal to the Fc region portion of the second single chain polypeptide. In another embodiment, the first CH1 region is carboxyl terminal to the Fc region portion of the first single chain polypeptide, and the first CL region is carboxyl terminal to the Fc region portion of the second single chain polypeptide.

In certain embodiments in which the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region, the first single chain polypeptide further comprises a second CH1 region and the second single chain polypeptide further comprises a second CL region, and the second CH1 region of the first single chain polypeptide and the second CL region of the second single chain polypeptide associate with each other in the polypeptide heterodimer. For instance, the Fc region portion of the first single chain polypeptide may be disposed between the first and second CH1 regions, and the Fc region portion of the second single chain polypeptide may be disposed between the first and second CL regions. Alternatively, both the first and second CH1 regions may be amino terminal to the Fc region portion of the first single chain polypeptide, and both the first and second CL regions may be amino terminal to the Fc region portion of the second single chain polypeptide. Also alternatively, both the first and second CH1 regions may be carboxyl terminal to the Fc region portion of the first single chain polypeptide, and both the first and second CL regions may be carboxyl terminal to the Fc region portion of the second single chain polypeptide.

In certain other embodiments in which the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region, the first single chain polypeptide further comprises a second CL region and the second single chain polypeptide further comprises a second CH1 region, and the second CL region of the first single chain polypeptide and the second CH1 region of the second single chain polypeptide associate with each other in the polypeptide heterodimer. For instance, in one embodiment, in the first single chain polypeptide, the first CH1 region is amino terminal to the Fc region portion and the second CL region is carboxyl terminal to the Fc region portion; and in the second single chain polypeptide, the first CL region is amino terminal to the Fc region portion, and the second CH1 region is carboxyl terminal to the Fc region portion. In another embodiment, in the first single chain polypeptide, the first CH1 region is carboxyl terminal to the Fc region portion, and the second CL region is amino terminal to the Fc region portion; and in the second single chain polypeptide, the first CL region is carboxyl terminal to the Fc region portion, and the second CH1 region is amino terminal to the Fc region portion. In yet another embodiment, in the first single chain polypeptide, both the first CH1 region and the second CL regions are amino terminal to the Fc region portion, and the first CH1 region is amino terminal to the second CL region; and in the second single chain polypeptide, both the first CL region and the second CH1 region are amino terminal to the Fc region portion, and the first CL region is amino terminal to the second CH1 region. In yet another embodiment, in the first single chain polypeptide, both the first CH1 region and the second CL regions are amino terminal to the Fc region portion, and the second CL region is amino terminal to the first CH1 region; and in the second single chain polypeptide, both the first CL region and the second CH1 region are amino terminal to the Fc region portion, and the second CH1 region is amino terminal to the first CL region. In a further embodiment, in the first single chain polypeptide, both the first CH1 region and the second CL regions are carboxyl terminal to the Fc region portion, and the first CH1 region is amino terminal to the second CL region; and in the second single chain polypeptide, both the first CL region and the second CH1 region are carboxyl terminal to the Fc region portion, and the first CL region is amino terminal to the second CH1 region. In another embodiment, in the first single chain polypeptide, both the first CH1 region and the second CL regions are carboxyl terminal to the Fc region portion, and the second CL region is amino terminal to the first CH1 region; and in the second single chain polypeptide, both the first CL region and the second CH1 region are carboxyl terminal to the Fc region portion, and the second CH1 region is amino terminal to the first CL region.

In certain embodiments, the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region. In one embodiment, the first CL region is amino terminal to the Fc region portion of the first single chain polypeptide, and the first CH1 region is amino terminal to the Fc region portion of the second single chain polypeptide. In another embodiment, the first CL region is carboxyl terminal to the Fc region portion of the first single chain polypeptide, and the first CH1 region is carboxyl terminal to the Fc region portion of the second single chain polypeptide. In yet another embodiment, the first single chain polypeptide further comprises a second CL region and the second single chain polypeptide further comprises a second CH1 region, and the second CL region of the first single chain polypeptide and the second CH1 region of the second single chain polypeptide associate with each other in the polypeptide heterodimer. For instance, the Fc region portion of the first single chain polypeptide may be disposed between the first and second CL regions, and the Fc region portion of the second single chain polypeptide may be disposed between the first and second CH1 regions. Alternatively, both the first and second CL regions may be amino terminal to the Fc region portion of the first single chain polypeptide, and both the first and second CH1 regions may be amino terminal to the Fc region portion of the second single chain polypeptide. Also alternatively, both the first and second CL regions may be carboxyl terminal to the Fc region portion of the first single chain polypeptide, and both the first and second CH1 regions may be carboxyl terminal to the Fc region portion of the second single chain polypeptide.

In certain embodiments, the first CL region is a Cκ region. In other embodiments, the first CL region is a Cλ region.

In certain embodiments, the second CL region is a Cκ region. In other embodiments, the second CL region is a Cλ region.

In certain embodiments, the Cκ region is a wild type human immunoglobulin Cκ region. In certain other embodiments, the Cκ region is an altered human immunoglobulin Cκ region with one or more amino acids of a wild type human Cκ region substituted at N29, N30, Q52, V55, T56, T56, S68, or T70. For example, the one or more amino acid substitutions may be selected from Ala (A), Arg (R), Trp (W), Tyr (Y), Glu (E), Gln (Q), Lys (K), Asp (D), Met (M), Ser (S), and Phe (F).

In certain embodiments, the CH1 region is an altered human immunoglobulin CH1 region comprising an amino acid substitution by which Val (V) at position 68 is substituted by Lys (K), Arg (R) or His (H), and wherein the Ck region is an altered human immunoglobulin Ck region comprising an amino acid substitution by which Leu (L) at position 29 is substituted by Asp (D) or Glu (E). In certain other embodiments, the CH1 region is an altered human immunoglobulin CH1 region comprising an amino acid substitution by which Val (V) at position 68 is changed to Asp (D) or Glu (E), and wherein the Ck region is an altered human immunoglobulin Ck region comprising an amino acid substitution by which Leu (L) at position 29 is changed to Lys (K), Arg (R) or His (H).

In certain embodiments, the Cλ region is a wild type human immunoglobulin Cλ region.

In certain embodiments, the first CH1 region or the second CH1 region when present is a wild type human immunoglobulin CH1 region, such as a wild type human IgG1 CH1 region.

In certain embodiments, the first CH1 region or the second CH1 region when present is an altered human immunoglobulin CH1 region, such as an altered human IgG1 CH1 region, with the cysteine of a wild type human immunoglobulin CH1 region that is involved in forming a disulfide bond with a wild type human immunoglobulin CL region deleted or substituted.

In certain embodiments, the Cκ region is an altered human immunoglobulin Ck region, such as an with the cysteine residue of a wild type human Cκ region that is involved in forming a disulfide bond with a wild type human immunoglobulin CH1 region deleted or substituted.

In certain embodiments, the Cλ region is an altered human immunoglobulin Cλ region with the cysteine residue of a wild type human Cλ region that is involved in forming a disulfide bond with a wild type human immunoglobulin CH1 region deleted or substituted.

In certain embodiments, the first CH1 region and the second CH1 region when present is a polypeptide comprising SEQ ID NO:114, 844 or 845.

In certain embodiments, the Ck region when present is selected from any one of the polypeptides comprising SEQ ID NOS: 141-178, 202, and 838-843.

In certain embodiments, the Cλ region when present is a polypeptide comprising SEQ ID NO:140.

In certain embodiments, the Fc region portion of the first single chain polypeptide (FRP-I) and the Fc region portion of the second single chain polypeptide (FRP-II) each comprise an immunoglobulin CH2 domain, such as an IgG1 CH2 domain or an IgG2, IgG3, IgG4, IgA1, IgA2, or IgD CH2 domain.

In certain embodiments, the Fc region portion of the first single chain polypeptide (FRP-I) and the Fc region portion of the second single chain polypeptide (FRP-II) each comprise an immunoglobulin CH3 domain, such as an IgG1 CH3 domain or an IgG2, IgG3, IgG4, IgA1, IgA2, IgD, IgE or IgM CH3 domain.

In certain embodiments, the Fc region portion of the first single chain polypeptide (FRP-I) and the Fc region portion of the second single chain polypeptide (FRP-II) each comprise an immunoglobulin CH2 domain and an immunoglobulin CH3 domain, such as are IgG1, IgG2, IgG3, IgG4, IgA1, IgA2, or IgD CH2 and CH3 domains.

In some embodiments in which the Fc region portion comprises an immunoglobulin CH3 domain that is immediately amino terminal to an immunoglobulin heterodimerization domain (*e.g.,* a CH1 domain or a Ck domain), the immunoglobulin CH3 domain is linked to the CH1 domain immediately carboxyl terminal to the immunoglobulin CH3 domain in one single chain polypeptide via a peptide comprising SEQ ID NOS:846, 847, 848, or 849; and the immunoglobulin CH3 domain is linked to the Ck domain immediately carboxyl terminal to the immunoglobulin CH3 domain in the other single chain polypeptide via a peptide comprising SEQ ID NOs:846, 850, 951, or 852.

In certain embodiments, the Fc region portion of the first single chain polypeptide (FRP-I) and the Fc region portion of the second chain polypeptide (FRP-II) comprise IgM or IgE CH3 and CH4 domains.

In certain embodiments in which the Fc region portion comprises an immunoglobulin CH2 domain, the CH2 domain may be an altered human IgG1, IgG2, IgG3, or IgG4 CH2 domain. Exemplary altered human IgG1, IgG2, IgG3 or IgG4 CH2 domain include those that comprise (a) an amino acid substitution at position 297 and at least one additional substitution or deletion at positions 234 to 238; (b) one or more amino acid mutations at positions 234-238 and at least one substitution at position 253, 310, 318, 320, 322, or 331; or (c) an amino acid substitution at the asparagine of position 297, one or more substitutions or deletions at positions 234 to 238, and at least one substitution at position 253, 310, 318, 320, 322, or 331. Another exemplary CH2 domain is an altered human IgG1 CH2 domain that comprises amino acid substitutions at positions L234, L235, G237, E318, K320 and K322.

In certain embodiments, the CH3 domain of the first single chain polypeptide is an altered human IgG1, IgG2, IgG3, or IgG4 CH3 domain that comprises a T366W, and the CH3 domain of the second single chain polypeptide is an altered human IgG1, IgG2, IgG3 or IgG4 CH3 domain that comprises a Y407A substitution. In certain other embodiments, the CH3 domain of the first single chain polypeptide is an altered human IgG1, IgG2, IgG3, or IgG4 CH3 domain that comprises a T366Y substitution, and the CH3 domain of the second single chain polypeptide is an altered human IgG1, IgG2, IgG3 or IgG4 CH3 domain that comprises a Y407T substitution. In certain other embodiments, the CH3 domain of the first single chain polypeptide is an altered human IgG1, IgG2, IgG3, or IgG4 CH3 domain that comprises a T366W substitution, and the CH3 domain of the second single chain polypeptide is an altered human IgG1, IgG2, IgG3 or IgG4 CH3 domain that comprises T366S, L368A and Y407V substitutions. In certain other embodiments, the CH3 domain of the first single chain polypeptide is an altered human IgG1, IgG2, IgG3, or IgG4 CH3 domain that comprises a Y407A substitution, and the CH3 domain of the second single chain polypeptide is an altered human IgG1, IgG2, IgG3 or IgG4 CH3 domain that comprises a T366W substitution. In certain other embodiments, the CH3 domain of the first single chain polypeptide is an altered human IgG1, IgG2, IgG3, or IgG4 CH3 domain that comprises a Y407T substitution, and the CH3 domain of the second single chain polypeptide is an altered human IgG1, IgG2, IgG3 or IgG4 CH3 domain that comprises a T366Y substitution. In certain other embodiments, the CH3 domain of the first single chain polypeptide is an altered human IgG1, IgG2, IgG3, or IgG4 CH3 domain that comprises T366S, L368A and Y407W substitutions, and the CH3 domain of the second single chain polypeptide is an altered human IgG1, IgG2, IgG3 or IgG4 CH3 domain that comprises a T366W substitution.

In certain embodiments, the hinge of both the first and second single chain polypeptides is an immunoglobulin hinge region, such as an IgG1, IgG2, IgG3, IgG4, IgA1, IgA2, IgD, or IgE hinge. In certain embodiments, the immunoglobulin hinge is a wild type immunoglobulin hinge. In certain other embodiments, the immunoglobulin hinge is an altered immunoglobulin hinge selected from SEQ ID NOS:232, 234, 240, 664-673, 675 and 676.

In certain embodiments, the hinge region is (a) at the amino terminal to the Fc region portion, (b) disposed between the binding domain and the immunoglobulin heterodimerization domain, (c) disposed between the immunoglobulin heterodimerization domain and the Fc region portion, (d) at the amino terminus of the first or second single chain polypeptide, (e) disposed between the Fc region portion and a binding domain, or (f) at the carboxyl terminus of the first or second single chain polypeptide.

In certain embodiments, at least one of the first and second single chain polypeptide hinges is a C-type lectin hinge region, such as an NKg2A or NKg2D peptide, or a derivative thereof.

In certain embodiments, the hinges of the first and second single chain polypeptides are identical. In certain other embodiments, the hinges of the first and second single chain polypeptides are different.

In certain embodiments, the first single chain polypeptide comprises a binding domain that specifically binds a TCR complex or a component thereof, and the second single chain polypeptide comprises a binding domain that specifically binds CD 19, CD79b, HLA-DR or CD20.

In certain embodiments, the first single chain polypeptide comprises a binding domain that specifically binds CD28, and the second single chain polypeptide comprises a binding domain (a) that specifically binds CD79b, hyperIL-6, or CD86 or (b) that comprises a PDL ectodomain or a monoIL-10.

In certain embodiments, the first single chain polypeptide comprises a binding domain that specifically binds c-Met, and the second single chain polypeptide comprises a binding domain that specifically binds RON.

In certain embodiments, the first and second single chain polypeptides comprise SEQ ID NOS: SEQ ID NOS:2 and 4, SEQ ID NOS:6 and 8, SEQ ID NOS:10 and 12, SEQ ID NOS: 14 and 16, SEQ ID NOS:18 and 20, SEQ ID NOS:20 and 22, SEQ ID NOS:20 and 24, SEQ ID NOS:30 and 32, SEQ ID NOS:29 and 31, SEQ ID NOS:29 and 32, SEQ ID NOS:30 and 72, SEQ ID NOS:53 and 72, SEQ ID NOS:54 and 72, SEQ ID NOS:55 and 72, SEQ ID NOS:70 and 72, SEQ ID NOS:71 and 72, SEQ ID NOS:63 and 56, SEQ ID NOS:64 and 57, SEQ ID NOS:65 and 60, SEQ ID NOS:66 and 58, SEQ ID NOS:67 and 59, SEQ ID NOS:68 and 61, SEQ ID NOS:69 and 62, SEQ ID NOS:54 and 811, SEQ ID NOS:54 and 812, SEQ ID NOS:54 and 813, SEQ ID NOS:814 and 818, SEQ ID NOS:815 and 818, SEQ ID NOS:816 and 818, SEQ ID NOS:817 and 818, SEQ ID NOS:814 and 820, SEQ ID NOS:814 and 821, SEQ ID NOS:54 and 819, SEQ ID NOS:814 and 826, SEQ ID NOS:814 and 822, SEQ ID NOS:814 and 823, SEQ ID NOS:814 and 824, SEQ ID NOS:859 and 862, SEQ ID NOS:860 and 863, SEQ ID NOS:861 and 864, SEQ ID NOS:874 and 825, SEQ ID NOS:875 and 879, SEQ ID NOS:876 and 880, SEQ ID NOS:877 and 881, or SEQ ID NOS:878 and 882.

In another aspect, the present disclosure provides a composition comprising a polypeptide heterodimer provided herein and a pharmaceutically acceptable excipient.

In another aspect, the present disclosure provides an expression vector capable of expressing a polypeptide heterodimer provided herein, comprising a first polynucleotide encoding the first single chain polypeptide and a second polynucleotide encoding the second single chain polypeptide.

In another aspect, the present disclosure provides a host cell comprising the above expression vector.

In another aspect, the present disclosure provides a host cell comprising first and second expression vectors capable of expressing the first and second single chain polypeptides, respectively, of the polypeptide heterodimer provided herein.

In another aspect, the present disclosure provides a method for making a polypeptide heterodimer, comprising (a) culturing a host cell provided herein under conditions suitable to express first and second single chain polypeptides, and (b) optionally isolating or purifying the heterodimers formed from the first and second single chain polypeptides from the culture.

In another aspect, the present disclosure provides a method for directing T cell activation, comprising administering to a patient in need thereof an effective amount of a polypeptide heterodimer that comprises a binding domain that specifically binds TCRα, TCRβ, CD3γ, CD3δ, CD3ε, or a combination thereof, and a second binding domain that specifically binds a different target, for instance, a tumor-specific antigen or other antigen of choice at a site or cell where T cell activation is desired.

In another aspect, the present disclosure provides a method for inhibiting growth, metastatis, or metastatic growth of a malignancy, comprising administering to a patient in need thereof an effective amount of a polypeptide heterodimer that comprises a binding domain that specifically binds TCRα, TCRβ, CD3γ, CD3δ, CD3ε, c-Met, RON, or a combination thereof. In certain embodiments, the method further comprises administering to a patient in need thereof a chemotherapeutic agent or ionizing radiation.

In another aspect, the present disclosure provides a method for treating an autoimmune or inflammatory condition, comprising administering to a patient in need thereof an effective amount of a polypeptide heterodimer that comprises a binding domain that specifically binds TCRα, TCRβ, CD3γ, CD3δ, CD3ε, or CD28.

In another aspect, the present disclosure provides a method for treating a B-cell associated disorder or disease comprising administering to a patient in need thereof an effective amount of a polypeptide heterodimer that comprises a binding domain that specifically binds TCRα, TCRβ, CD3γ, CD3δ, or CD3ε, and a second binding domain that specifically binds to CD 19, CD20, CD79b or HLA-DR.

In certain embodiments, the methods for using the polypeptide heterodimers provided herein may further comprise administering to a patient in need thereof a second active agent, such as a second polypeptide heterodimer, a monoclonal antibody, or an immunoglobulin-derived fusion protein.

### BRIEF DESCRIPTION OF THE DRAWINGS

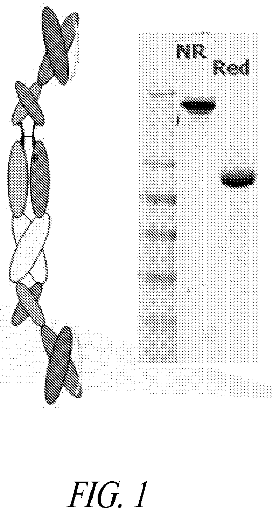

Figure 1 is a schematic representation of bivalent anti-CD28 polypeptide heterodimer X0172 (left) and SDS-PAGE analysis of X0172 (right). "NR" stands for "Non-Reduced," and "Red" stands for "Reduced."
Figure 2 shows that both a monovalent (X0124) and a bivalent (X0172) anti-CD28 polypeptide heterodimer synergize with a suboptimal concentration of PMA in stimulating purified human T cells when compared to an anti-CD28 scFv (2E12 scFv) but less than a bivalent anti-CD28 SMIP protein (2E12 SMIP).
Figure 3 shows that bivalent polypeptide heterodimer X0172 binds to CD4⁺ T cells better than 2E12 scFv and monovalent polypeptide heterodimer X0124.
Figure 4 shows cation exchange chromatography of polypeptide heterodimers X0251, X0252 and X0253 (left) and SDS-PAGE electrophoresis analysis of the same polypeptide heterodimers under non-reduced (NR) and reduced (Red) conditions (right).
Figure 5 shows the mass spectra of polypeptide heterodimer X0252, which demonstrates that the heterodimer is the predominant species.
Figure 6 shows SDS-PAGE electrophoresis analysis of polypeptide heterodimers X0283 and X0284 under non-reduced (NR) and reduced (Red) conditions (left) and cation exchange chromatography analysis of polypeptide heterodimer X0283.
Figure 7 shows direct binding to CD86 by polypeptide heterodimer X0283 as examined by BIACORE® analysis, with response units (Ru) plotted against time (left) and a schematic representation of X0283.
Figures 8A and 8B show binding of bispecific anti-RON and anti-CD3 constructs (polypeptide heterodimer S0268 and Scorpion protein S0266) to MDA-MB-453 cells (A) and to isolated T cells (B).
Figures 9A and 9B show specificity of binding to (A) Rec1 (CD19⁺, CD20⁺) cells or (B) Jurkat (CD3⁺) cells by bispecific heterodimers having either anti-CD19 and anti-CD3 binding domains (TSC020) or having anti-CD20 and anti-CD3 binding domains (TSC021).
Figures 10A-10D show proliferation of CD4+ and CD8+ T-cells in response to bispecific polypeptide heterodimers TSC054, TSC078, TSC079, and bsc19x3 (TSC036) with (A and B) Daudi (CD19⁺) cells or (C and D) MDA-MB-453 (CD19⁻) cells.
Figures 11A and 11B shows T-cell directed cytotoxicity induced by bispecific polypeptide heterodimers TSC054, TSC078, TSC079, and S0268 in a chromium (⁵¹Cr) release assay with (A) Daudi (RON⁻, CD19⁺) cells or (B) BxPC-3 (RON⁺, CD19⁻) cells.
Figure 12 shows target-dependent T-cell proliferation induced by a CD 19+ cell line (Daudi) using bispecific polypeptide heterodimers TSC165, TSC166, TSC167, TSC168 and TSC100 at concentrations from 0.1 pM to 10000 pM.
Figure 13 shows target-dependent T-cell proliferation induced by a CD19+ cell line (Daudi) using bispecific polypeptide heterodimers TSC127 and TS165 with bsc19x3 BiTE as a control at concentrations from 0.001 pM to 1000 pM.
Figure 14 shows target-dependent redirected T-cell cytotoxicity on a CD19+ cell line (Daudi) using bispecific polypeptide heterodimers TSC100, TSC165, TC166, TSC167, and TSC168.

### DETAILED DESCRIPTION

The present disclosure provides polypeptide heterodimers formed between two different single chain polypeptides via natural heterodimerization of an immunoglobulin CH1 region and an immunoglobulin light chain constant region (CL). The polypeptide heterodimer has two or more binding domains that specifically bind one or more targets (*e.g.*, an antigen, a receptor, or a ligand). In addition, both chains of a heterodimer each further comprise an Fc region portion (*e.g*., immunoglobulin CH2 and/or CH3 domains). The present disclosure also provides nucleic acids, vectors, host cells and methods for making polypeptide heterodimers.

The heterodimerization technology described herein has one or more of the following advantages: (1) potential for minimal immunogenicity of the polypeptide heterodimers because the dimers are formed via natural heterodimerization of an immunoglobulin CH1 region and an immunoglobulin CL region; (2) efficient production and purification of polypeptide heterodimers of the present disclosure is possible by co-expressing the two different single chain polypeptides, as shown in the examples; (3) the ability to mediate Fc effector functions (*e.g*., CDC, ADCC, ADCP), which can be modulated up or down by mutagenesis, and a longer serum half life because each chain of a polypeptide heterodimer according to the present disclosure has an Fc region portion (*e.g.*, immunoglobulin CH2 and CH3 domains); and (4) polypeptide heterodimers of the present disclosure have a size that is typically smaller than an antibody molecule, which can allow for better tissue penetration, such as into a solid malignancy.

The polypeptide heterodimers provided herein are useful to direct therapeutic agents or immune effector cells to target cells. For instance, in certain embodiments, the polypeptide heterodimers may comprise a binding domain that specifically binds a TCR complex or a component thereof *(e.g.,* TCRα, TCRβ, CD3γ, CD3δ, and CD3ε) and another binding domain that specifically binds a second different target, such as an oncology target (*e.g.*, c-Met, RON, CEACAM-6, and PSMA) or a B-cell target (*e.g.,* CD19, CD79b, HLA-DR and CD20). The binding domain specific for the second different target may have a higher affinity for its target than the affinity of the binding domain for the TCR complex or component thereof, such as CD3. Such polypeptide heterodimers will preferably bind to the oncology target or the B-cell target first and subsequently recruit T cells to the tumor or cancer cells expressing the oncology target or B-cell target and are useful in inhibiting growth, metastasis or metastatic growth of a malignancy (including B-cell cancers). Additional uses of polypeptide heterodimers provided herein include directed T cell activation and treating autoimmune or inflammatory conditions.

The section headings used herein are for organizational purposes only and are not to be construed as limiting the subject matter described. All documents, or portions of documents, cited herein, including but not limited to patents, patent applications, articles, books, and treatises, are hereby expressly incorporated by reference in their entirety for any purpose. In the event that one or more of the incorporated documents or portions of documents defines a term that contradicts that term's definition in the application, the definition that appears in this application controls.

In the present description, any concentration range, percentage range, ratio range, or integer range is to be understood to include the value of any integer within the recited range and, when appropriate, fractions thereof (such as one tenth and one hundredth of an integer), unless otherwise indicated. As used herein, "about" means ± 20% of the indicated range, value, sequence, or structure, unless otherwise indicated. It should be understood that the terms "a" and "an" as used herein refer to "one or more" of the enumerated components unless otherwise indicated or dictated by its context. The use of the alternative (e.g., "or") should be understood to mean either one, both, or any combination thereof of the alternatives. As used herein, the terms "include" and "comprise" are used synonymously. In addition, it should be understood that the individual single chain polypeptides or heterodimers derived from various combinations of the structures and substituents (*e.g*., domains, regions, hinges, and linkers) described herein are disclosed by the present application to the same extent as if each single chain polypeptide or heterodimer were set forth individually. Thus, selection of particular components to form individual single chain polypeptides or heterodimers is within the scope of the present disclosure.

As used herein, a protein "consists essentially of" several domains (*e.g.*, a binding domain that specifically binds a target, a hinge, an immunoglobulin heterodimerization domain, and an Fc region portion) if the other portions of the protein (*e.g.*, amino acids at the amino- or carboxy-terminus or between two domains), in combination, contribute to at most 20% (*e.g.,* at most 15%, 10%, 8%, 6%, 5%, 4%, 3%, 2% or 1%) of the length of the protein and do not substantially affect (*i.e.,* do not reduce the activity by more than 50%, such as more than 40%, 30%, 25%, 20%, 15%, 10%, or 5%) the activities of various domains (*e.g.*, the target binding affinity of the binding domain, the activities of the Fc region portion, and the capability of the immunoglobulin heterodimerization domain in facilitating heterodimerization). In certain embodiments, a protein (*e.g.,* a single chain polypeptide) consists essentially of a binding domain that specifically binds a target, an immunoglobulin heterodimerization domain, a hinge, and an Fc region portion may comprise junction amino acids at the amino- and/or carboxy-terminus of the protein and/or between two different domains (*e.g.*, between the binding domain and the immunoglobulin heterodimerization domain, between the immunoglobulin heterodimerization domain and the hinge, and/or between the hinge and the Fc region portion).

A "polypeptide heterodimer" or "heterodimer," as used herein, refers to a dimer formed from two different single chain polypeptides. This term does not include an antibody formed from four single chain polypeptides (*i.e.,* two light chains and two heavy chains). A "dimer" refers to a biological entity that consists of two subunits associated with each other via one or more forms of intramolecular forces, including covalent bonds (*e.g.,* disulfide bonds) and other interactions (*e.g.,* electrostatic interactions, salt bridges, hydrogen bonding, and hydrophobic interactions), and is stable under appropriate conditions (*e.g.*, under physiological conditions, in an aqueous solution suitable for expressing, purifying, and/or storing recombinant proteins, or under conditions for non-denaturing and/or non-reducing electrophoresis).

A "single chain polypeptide" is a single, linear and contiguous arrangement of covalently linked amino acids. It does not include two polypeptide chains that link together in a non-linear fashion, such as via an interchain disulfide bond *(e.g.,* a half immunoglobulin molecule in which a light chain links with a heavy chain via a disulfide bond). In certain embodiments, a single chain polypeptide may have or form one or more intrachain disulfide bonds.

An "immunoglobulin heterodimerization domain," as used herein, refers to an immunoglobulin domain of a single chain polypeptide that preferentially interacts or associates with a different immunoglobulin domain of another single chain polypeptide wherein the interaction of the different heterodimerization domains substantially contributes to or efficiently promotes heterodimerization of the first and second single chain polypeptides (*i.e.,* the formation of a dimer between two different single chain polypeptides, which is also referred to as a "heterodimer"). The interaction(s) between heterodimerization domains "substantially contributes to or efficiently promotes" the heterodimerization of first and second single chain polypeptides if there is a statistically significant reduction in the dimerization between the first and second single chain polypeptides in the absence of the heterodimerization domain of the first single chain polypeptide (HD-I) and/or the heterodimerization domain of the second single chain polypeptide (HD-II). In certain embodiments, when the first and second single chain polypeptides are co-expressed, at least 60%, at least about 60% to about 70%, at least about 70% to about 80%, at least about 80% to about 90%, 91%, 92%, 93%, 94%, 95%, 96%, 97%, 98%, 99%, or 100%, and at least about 90% to about 92%, 93%, 94%, 95%, 96%, 97%, 98%, or 99% of the first and second single chain polypeptides form heterodimers with each other. Representative immunoglobulin heterodimerization domains of the present disclosure include an immunoglobulin CH1 region, an immunoglobulin CL region (*e.g.,* Cκ or Cλ isotypes), or derivatives thereof, including wild type immunoglobulin CH1 and CL regions and altered (or mutated) immunoglobulin CH1 and CL regions, as provided herein.

A "binding domain" or "binding region," as used herein, refers to a protein, polypeptide, oligopeptide, or peptide that possesses the ability to specifically recognize and bind to a target (*e.g.,* CD3, TCR, CD28, c-Met, RON). A binding domain includes any naturally occurring, synthetic, semi-synthetic, or recombinantly produced binding partner for a biological molecule or another target of interest. Exemplary binding domains include single chain antibody variable regions (*e.g.*, domain antibodies, sFv, scFv, Fab), receptor ectodomains (*e.g.*, c-Met, RON), or ligands (*e.g.*, cytokines, chemokines). A variety of assays are known for identifying binding domains of the present disclosure that specifically bind a particular target, including Western blot, ELISA, and Biacore analysis.

A binding domain and a fusion protein thereof "specifically binds" a target if it binds the target with an affinity or Ka (*i.e.,* an equilibrium association constant of a particular binding interaction with units of 1/M) equal to or greater than 10⁵ M⁻¹, while not significantly binding other components present in a test sample. Binding domains (or fusion proteins thereof) may be classified as "high affinity" binding domains (or fusion proteins thereof) and "low affinity" binding domains (or fusion proteins thereof). "High affinity" binding domains refer to those binding domains with a Kₐ of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. "Low affinity" binding domains refer to those binding domains with a Kₐ of up to 10⁷ M⁻¹, up to 10⁶ M⁻¹, up to 10⁵ M⁻¹. Alternatively, affinity may be defined as an equilibrium dissociation constant (K_{d}) of a particular binding interaction with units of M (*e.g.,* 10⁻⁵ M to 10⁻¹³ M). Affinities of binding domain polypeptides and single chain polyepeptides according to the present disclosure can be readily determined using conventional techniques *(see, e.g.,* Scatchard et al. (1949) Ann. N.Y. Acad. Sci. 51:660; and U.S. Patent Nos. 5,283,173, 5,468,614, or the equivalent).

"T cell receptor" (TCR) is a molecule found on the surface of T cells that, along with CD3, is generally responsible for recognizing antigens bound to major histocompatibility complex (MHC) molecules. It consists of a disulfide-linked heterodimer of the highly variable α and β chains in most T cells. In other T cells, an alternative receptor made up of variable γ and δ chains is expressed. Each chain of the TCR is a member of the immunoglobulin superfamily and possesses one N-terminal immunoglobulin variable domain, one immunoglobulin constant domain, a transmembrane region, and a short cytoplasmic tail at the C-terminal end *(see,* Abbas and Lichtman, Cellular and Molecular Immunology (5th Ed.), Editor: Saunders, Philadelphia, 2003; Janeway et al., Immunobiology: The Immune System in Health and Disease, 4th Ed., Current Biology Publications, p148, 149, and 172, 1999). TCR as used in the present disclosure may be from various animal species, including human, mouse, rat, or other mammals.

"CD3" is known in the art as a multi-protein complex of six chains *(see,* Abbas and Lichtman, 2003; Janeway *et al.,* p. 172 and 178, 1999). In mammals, the complex comprises a CD3γ chain, a CD3δ chain, two CD3ε chains, and a homodimer of CD3ζ chains. The CD3γ, CD3δ, and CD3ε chains are highly related cell surface proteins of the immunoglobulin superfamily containing a single immunoglobulin domain. The transmembrane regions of the CD3γ, CD3δ, and CD3ε chains are negatively charged, which is a characteristic that allows these chains to associate with the positively charged T cell receptor chains. The intracellular tails of the CD3γ, CD3δ, and CD3ε chains each contain a single conserved motif known as an immunoreceptor tyrosine-based activation motif or ITAM, whereas each CD3ζ chain has three. It is believed the ITAMs are important for the signaling capacity of a TCR complex. CD3 as used in the present disclosure may be from various animal species, including human, mouse, rat, or other mammals.

"TCR complex," as used herein, refers to a complex formed by the association of CD3 with TCR. For example, a TCR complex can be composed of a CD3γ chain, a CD3δ chain, two CD3ε chains, a homodimer of CD3ζ chains, a TCRα chain, and a TCRβ chain. Alternatively, a TCR complex can be composed of a CD3γ chain, a CD3δ chain, two CD3ε chains, a homodimer of CD3ζ chains, a TCRγ chain, and a TCRδ chain.

"A component of a TCR complex," as used herein, refers to a TCR chain (*i.e.,* TCRα, TCRβ, TCRγ or TCRδ), a CD3 chain (*i.e.,* CD3γ, CD3δ, CD3ε or CD3ζ), or a complex formed by two or more TCR chains or CD3 chains *(e.g.,* a complex of TCRα and TCRβ, a complex of TCRγ and TCRδ, a complex of CD3ε and CD3δ, a complex of CD3γ and CD3ε, or a sub-TCR complex of TCRα, TCRβ, CD3γ, CD3δ, and two CD3ε chains).

Terms understood by those in the art of antibody technology are each given the meaning acquired in the art, unless expressly defined differently herein. Antibodies are known to have variable regions, a hinge region, and constant domains. Immunoglobulin structure and function are reviewed, for example, in Harlow et al., Eds., Antibodies: A Laboratory Manual, Chapter 14 (Cold Spring Harbor Laboratory, Cold Spring Harbor, 1988).

For example, the terms "VL" and "VH" refer to the variable binding region from an antibody light and heavy chain, respectively. The variable binding regions are made up of discrete, well-defined sub-regions known as "complementarity determining regions" (CDRs) and "framework regions" (FRs). The term "CL" refers to an "immunoglobulin light chain constant region" or a "light chain constant region," *i.e.,* a constant region from an antibody light heavy chain. The term "CH" refers to an "immunoglobulin heavy chain constant region" or a "heavy chain constant region," which is further divisible, depending on the antibody isotype into CH1, CH2, and CH3 (IgA, IgD, IgG), or CH1, CH2, CH3, and CH4 domains (IgE, IgM). A "Fab" (fragment antigen binding) is the part of an antibody that binds to antigens and includes the variable region and CH1 of the heavy chain linked to the light chain via an inter-chain disulfide bond.

As used herein, "an Fc region constant domain portion" or "Fc region portion" refers to the heavy chain constant region segment of the Fc fragment (the "fragment crystallizable" region or Fc region) from an antibody, which can include one or more constant domains, such as CH2, CH3, CH4, or any combination thereof. By way of background, the Fc region is responsible for the effector functions of an immunoglobulin, such as ADCC (antibody-dependent cell-mediated cytotoxicity), ADCP (antibody-dependent cellular phagocytosis), CDC (complement-dependent cytotoxicity) and complement fixation, binding to Fc receptors (*e.g.*, CD16, CD32, FcRn), greater half-life *in vivo* relative to a polypeptide lacking an Fc region, protein A binding, and perhaps even placental transfer *(see* Capon et al., Nature, 337:525 (1989)).

In addition, antibodies have a hinge sequence that is typically situated between the Fab and Fc region (but a lower section of the hinge may include an amino-terminal portion of the Fc region). By way of background, an immunoglobulin hinge acts as a flexible spacer to allow the Fab portion to move freely in space. In contrast to the constant regions, hinges are structurally diverse, varying in both sequence and length between immunoglobulin classes and even among subclasses. For example, a human IgG1 hinge region is freely flexible, which allows the Fab fragments to rotate about their axes of symmetry and move within a sphere centered at the first of two inter-heavy chain disulfide bridges. By comparison, a human IgG2 hinge is relatively short and contains a rigid poly-proline double helix stabilized by four inter-heavy chain disulfide bridges, which restricts the flexibility. A human IgG3 hinge differs from the other subclasses by its unique extended hinge region (about four times as long as the IgG1 hinge), containing 62 amino acids (including 21 prolines and 11 cysteines), forming an inflexible poly-proline double helix and providing greater flexibility because the Fab fragments are relatively far away from the Fc fragment. A human IgG4 hinge is shorter than IgG1 but has the same length as IgG2, and its flexibility is intermediate between that of IgG1 and IgG2.

According to crystallographic studies, an IgG hinge domain can be functionally and structurally subdivided into three regions: the upper, the core or middle, and the lower hinge regions (Shin et al., Immunological Reviews 130:87 (1992)). Exemplary upper hinge regions include EPKSCDKTHT (SEQ ID NO:227) as found in IgG1, ERKCCVE (SEQ ID NO:211) as found in IgG2, ELKTPLGDTT HT (SEQ ID NO:245) or EPKSCDTPPP (SEQ ID NO:246) as found in IgG3, and ESKYGPP (SEQ ID NO:247) as found in IgG4. Exemplary middle or core hinge regions include CPPCP (SEQ ID NO:228) as found in IgG1 and IgG2, CPRCP (SEQ ID NO:248) as found in IgG3, and CPSCP (SEQ ID NO:249) as found in IgG4. While IgG1, IgG2, and IgG4 antibodies each appear to have a single upper and middle hinge, IgG3 has four in tandem - one being ELKTPLGDTTHTCPRCP (SEQ ID NO:250) and three being EPKSCDTPPP CPRCP (SEQ ID NO:251).

IgA and IgD antibodies appear to lack an IgG-like core region, and IgD appears to have two upper hinge regions in tandem *(see* SEQ ID NOS:222 and 252). Exemplary wild type upper hinge regions found in IgA1 and IgA2 antibodies are set forth in SEQ ID NOS:215 and 216.

IgE and IgM antibodies, in contrast, lack a typical hinge region and instead have a CH2 domain with hinge-like properties. Exemplary wild-type CH2 upper hinge-like sequences of IgE and IgM are set forth in SEQ ID NO:253 (VCSRDFTPPTVKILQSSSDGGGHFPPTIQLLCLVSGYTPGTINITWLEDG QVMDVDLSTASTTQEGELASTQSELTLSQKHWLSDRTYTCQVTYQGHTFE DSTKKCA) and SEQ ID NO:254 (VIAELPPKVSVFVPPRDGFFGNPRKSKLIC QATGFSPRQIQVSWLREGKQVGSGVTTDQVQAEAKESGPTTYKVTSTLTI KESDWLGQSMFTCRVDHRGLTFQQNASSMCVP), respectively.

As used herein, a "hinge region" or a "hinge" refers to (a) an immunoglobulin hinge region (made up of, for example, upper and core regions) or a functional variant thereof, including wild type and altered immunoglobulin hinges, (b) a lectin interdomain region or a functional variant thereof, (c) a cluster of differentiation (CD) molecule stalk region or a functional variant thereof, or (d) a portion of a cell surface receptor (interdomain region) that connects immunoglobulin V-like or immunoglobulin C-like domains.

As used herein, a "wild type immunoglobulin hinge region" refers to a naturally occurring upper and middle hinge amino acid sequences interposed between and connecting the CH1 and CH2 domains (for IgG, IgA, and IgD) or interposed between and connecting the CH1 and CH3 domains (for IgE and IgM) found in the heavy chain of an antibody. In certain embodiments, a wild type immunoglobulin hinge region sequence is human, and may comprises a human IgG hinge region. Exemplary human wild type immunoglobulin hinge regions are set forth in SEQ ID NOS:215 (IgA1 hinge), 216 (IgA2 hinge), 217 (IgD hinge), 667 (IgG1 hinge), 219 (IgG2 hinge), 220 (IgG3 hinge) and 221 (IgG4 hinge).

An "altered wild type immunoglobulin hinge region" or "altered immunoglobulin hinge region" refers to (a) a wild type immunoglobulin hinge region with up to 30% amino acid changes (*e.g.*, up to 25%, 20%, 15%, 10%, or 5% amino acid substitutions or deletions), or (b) a portion of a wild type immunoglobulin hinge region that has a length of about 5 amino acids (*e.g.*, about 5, 6, 7, 8, 9, 10, 11, 12, 13, 14, 15, 16, 17, 18, 19, or 20 amino acids) up to about 120 amino acids (for instance, having a length of about 10 to about 40 amino acids or about 15 to about 30 amino acids or about 15 to about 20 amino acids or about 20 to about 25 amino acids), has up to about 30% amino acid changes (*e.g.*, up to about 25%, 20%, 15%, 10%, 5%, 4%, 3%, 2%, or 1% amino acid substitutions or deletions or a combination thereof), and has an IgG core hinge region as set forth in SEQ ID NOS:228, 248, or 249.

A "peptide linker" refers to an amino acid sequence that connects a heavy chain variable region to a light chain variable region and provides a spacer function compatible with interaction of the two sub-binding domains so that the resulting polypeptide retains a specific binding affinity to the same target molecule as an antibody that comprises the same light and heavy chain variable regions. In certain embodiments, a linker is comprised of five to about 35 amino acids, for instance, about 15 to about 25 amino acids.

"Junction amino acids" or "junction amino acid residues" refer to one or more (*e.g.*, about 2-10) amino acid residues between two adjacent regions or domains of a single chain polypeptide, such as between a hinge and an adjacent Fc region portion or between a hinge and an adjacent binding domain or between a peptide linker that links two immunoglobulin variable domains and an adjacent immunoglobulin variable domain. Junction amino acids may result from the construct design of a single chain polypeptide (*e.g.*, amino acid residues resulting from the use of a restriction enzyme site during the construction of a nucleic acid molecule encoding a single chain polypeptide).

A "linker between CH3 and CH1 or CL" refers to one or more (*e.g.,* about 2-12) amino acid residues between the C- terminus of a CH3 domain (*e.g.,* a wild type CH3 or a mutated CH3) and the N-terminus of a CH1 domain or CL domain (*e.g*., Ck).

A "wild type immunoglobulin region" or "wild type immunoglobulin domain" refers to a naturally occurring immunoglobulin region or domain *(e.g.,* a naturally occurring VL, VH, hinge, CL, CH1, CH2, CH3, or CH4) from various immunoglobulin classes or subclasses (including, for example, IgG1, IgG2, IgG3, IgG4, IgA1, IgA2, IgD, IgE, and IgM) and from various species (including, for example, human, sheep, mouse, rat, and other mammals). Exemplary wild type human CH1 regions are set forth in SEQ ID NOS:114, 186-192 and 194, wild type human Cκ region in SEQ ID NO:112, wild type human Cλ regions in SEQ ID NO:113 and 224-226, wild type human CH2 domains in SEQ ID NOS:115, 195-201 and 203, wild type human CH3 domains in SEQ ID NOS:116, 204-210 and 212, and wild type human CH4 domains in SEQ ID NO:213 and 214.

An "altered immunoglobulin region," "altered immunoglobulin domain," "mutated immunoglobulin domain," or the like, refers to an immunoglobulin region with a sequence identity to a wild type immunoglobulin region or domain *(e.g.,* a wild type VL, VH, hinge, CL, CH1, CH2, CH3, or CH4) of at least 75% *(e.g.,* 80%, 82%, 84%, 86%, 88%, 90%, 91%, 92%, 93%, 94%, 95%, 96%, 97%, 98%, 99%, or 99.5%). For example, an "altered immunoglobulin CH1 region" or "altered CH1 region" refers to a CH1 region with a sequence identity to a wild type immunoglobulin CH1 region (*e.g.,* a human CH1) of at least 75% *(e.g.,* 80%, 82%, 84%, 86%, 88%, 90%, 91%, 92%, 93%, 94%, 95%, 96%, 97%, 98%, 99%, or 99.5%). Similarly, an "altered immunoglobulin CH2 domain" or "altered CH2 domain" refers to a CH2 domain with a sequence identity to a wild type immunoglobulin CH2 region *(e.g.,* a human CH2) of at least 75% *(e.g.,* 80%, 82%, 84%, 86%, 88%, 90%, 91%, 92%, 93%, 94%, 95%, 96%, 97%, 98%, 99%, or 99.5%).

"Sequence identity," as used herein, refers to the percentage of amino acid residues in one sequence that are identical with the amino acid residues in another reference polypeptide sequence after aligning the sequences and introducing gaps, if necessary, to achieve the maximum percent sequence identity, and not considering any conservative substitutions as part of the sequence identity. The percentage sequence identity values are generated by the NCBI BLAST2.0 software as defined by Altschul et al. (1997) "Gapped BLAST and PSI-BLAST: a new generation of protein database search programs," Nucleic Acids Res. 25:3389-3402, with the parameters set to default values.

In certain embodiments, an altered immunoglobulin domain only contains conservative amino acid substitutions of a wild type immunoglobulin domain. In certain other embodiments, an altered immunoglobulin domain only contains non-conservative amino acid substitutions of a wild type immunoglobulin domain. In yet other embodiments, an altered immunoglobulin domain contains both conservative and non-conservative amino acid substitutions.

A "conservative substitution" is recognized in the art as a substitution of one amino acid for another amino acid that has similar properties. Exemplary conservative substitutions are well known in the art *(see, e.g.,* WO 97/09433, page 10, published March 13, 1997; Lehninger, Biochemistry, Second Edition; Worth Publishers, Inc. NY:NY (1975), pp.71-77; Lewin, Genes IV, Oxford University Press, NY and Cell Press, Cambridge, MA (1990), p. 8). In certain embodiments, a conservative substitution includes a leucine to serine substitution.

As used herein, the term "derivative" refers to a modification of one or more amino acid residues of a peptide by chemical or biological means, either with or without an enzyme, *e.g*., by glycosylation, alkylation, acylation, ester formation, or amide formation. Generally, a "derivative" differs from an "analogue" in that a parent polypeptide may be the starting material to generate a "derivative," whereas the parent polypeptide may not necessarily be used as the starting material to generate an "analogue." A derivative may have different chemical, biological or physical properties of the parent polypeptide. For example, a derivative may be more hydrophilic or it may have altered reactivity (*e.g.*, a CDR having an amino acid change that alters its affinity for a target) as compared to the parent polypeptide.

As used herein, unless otherwise provided, a position of an amino acid residue in a variable region of an immunoglobulin molecule is numbered according to the Kabat numbering convention (Kabat, Sequences of Proteins of Immunological Interest, 5th ed. Bethesda, MD: Public Health Service, National Institutes of Health (1991)), and a position of an amino acid residue in a constant region of an immunoglobulin molecule is numbered according to EU nomenclature (Ward et al., 1995 Therap. Immunol. 2:77-94).

A "receptor" is a protein molecule present in the plasma membrane or in the cytoplasm of a cell to which a signal molecule (*i.e.,* a ligand, such as a hormone, a neurotransmitter, a toxin, a cytokine) may attach. The binding of the signal molecule to the receptor results in a conformational change of the receptor, which ordinarily initiates a cellular response. However, some ligands merely block receptors without inducing any response (*e.g*., antagonists). Some receptor proteins are peripheral membrane proteins. Many hormone and neurotransmitter receptors are transmembrane proteins that are embedded in the phospholipid bilayer of cell membranes, and another major class of receptors are intracellular proteins such as those for steroid and intracrine peptide hormone receptors.

"B-cell associated disorder or disease" or "a disease or disorder associated with aberrant B-cell activity" refers to a disease or disorder associated with (e.g., causing or resulting from) aberrant B-cell activity or activity that deviates from the normal, proper, or expected course. For example, a B-cell associated disorder or disease may include inappropriate proliferation of B-cells that have damaged or defective DNA or other cellular components. Aberrant B-cell activity may include cell proliferation characterized by inappropriately high levels of B-cell division, inappropriately low levels of B-cell apoptosis, or both. Such diseases may have, for example, single or multiple local abnormal proliferations of B-cells, groups of B-cells or tissue(s), whether cancerous or non-cancerous, benign or malignant. A B-cell associated disorder or disease may also include aberrant antibody production, such as production of autoantibodies, or overproduction of antibodies more desirable when produced at normal levels. It is also contemplated herein that aberrant B-cell activity may occur in certain subpopulations of B-cells and not in other subpopulations, or may include inappropriate stimulation of T-cells, such as by inappropriate antigen presentation to T-cells or by other B-cell pathways. Examples of B-cell associated disorders or diseases include a B-cell malignancy or B-cell cancer *(e.g.,* B-cell lymphoma, B-cell leukemia or B-cell myeloma), a disease characterized by autoantibody production (*e.g*., autoimmune diseases) or inflammation or a disease characterized by inappropriate T-cell stimulation caused by inappropriate B-cell antigen presentation to T-cells or caused by other pathways involving B-cells.

"Treatment," "treating" or "ameliorating" refers to either a therapeutic treatment or prophylactic/preventative treatment. A treatment is therapeutic if at least one symptom of disease in an individual receiving treatment improves or a treatment may delay worsening of a progressive disease in an individual, or prevent onset of additional associated diseases.

A "therapeutically effective amount (or dose)" or "effective amount (or dose)" of a specific binding molecule or compound refers to that amount of the compound sufficient to result in amelioration of one or more symptoms of the disease being treated in a statistically significant manner. When referring to an individual active ingredient, administered alone, a therapeutically effective dose refers to that ingredient alone. When referring to a combination, a therapeutically effective dose refers to combined amounts of the active ingredients that result in the therapeutic effect, whether administered serially or simultaneously (in the same formulation or concurrently in separate formulations).

The term "pharmaceutically acceptable" refers to molecular entities and compositions that do not produce allergic or other serious adverse reactions when administered using routes well known in the art.

A "patient in need" refers to a patient at risk of, or suffering from, a disease, disorder or condition that is amenable to treatment or amelioration with a polypeptide heterodimer or a composition thereof provided herein.

The term "immunoglobulin-derived fusion protein," as used herein, refers to a fusion protein that comprises at least one immunoglobulin region, such as a VL, VH, CL, CH1, CH2, CH3, and CH4 domain. The immunoglobulin region may be a wild type immunoglobulin region or an altered immunoglobulin region. Exemplary immunoglobulin-derived fusion proteins include single chain variable antibody fragment (scFv) (*see, e.g.,* Huston et al., Proc. Natl. Acad. Sci. USA 85: 5879-83, 1988), SMIP™ proteins *(see,* U.S. Patent Publication Nos. 2003/0133939, 2003/0118592, and 2005/0136049), PIMS proteins *(see,* PCT Application Publication No. WO 2009/023386), and multi-functional binding proteins (such as SCORPION™ and Xceptor proteins) *(see,* PCT Application Publication No. WO 2007/146968, U.S. Patent Application Publication No. 2006/0051844, and U.S. Patent No. 7,166,707).

Additional definitions are provided throughout the present disclosure.

### Polypeptide Heterodimers

In one aspect, the present disclosure provides a polypeptide heterodimer formed by the association of two different single chain polypeptides. The first single chain polypeptide (SCP-I) comprises, consists essentially of, or consists of from one to four binding domains that specifically bind from one to four targets, a hinge (H-I), an immunoglobulin heterodimerization domain (HD-I), and an Fc region portion (FRP-I), whereas the second single chain polypeptide (SCP-II) comprises, consists essentially of, or consists of from zero to four binding domains that specifically bind from zero to four targets, a hinge (H-II), an immunoglobulin heterodimerization domain (HD-II), and an Fc region portion (FRP-II), provided that the polypeptide heterodimer comprises at least two binding domains that specifically bind to one or more targets, for instance, at least two different targets. The H-I and H-II may have the same sequence, but may be different. The HD-I may comprise an immunoglobulin CH1 region, and the HD-II may comprise an immunoglobulin CL region. Alternatively, the HD-I may comprise an immunoglobulin CL region, and the HD-II may comprise an immunoglobulin CH1 region. The FRP-I and FRP-II may have the same sequence, but may be different. The individual components of the polypeptide heterodimers of the present disclosure are described in detail herein.

### Binding Domains

As indicated above, the polypeptide heterodimer of the present disclosure comprises two single chain polypeptides: One single chain polypeptide comprises from one to four binding domains that specifically bind from one to four targets, and the other single chain polypeptide of the polypeptide heterodimer comprises from zero to four binding domains that bind from zero to four targets. The total number of binding domains of the polypeptide heterodimer ranges from about two to eight, and the total number of different targets that the binding domains bind ranges from about one to eight, for instance, from two to eight, two to four, two to three or two targets.

If a single chain polypeptide of a polypeptide heterodimer comprises a single binding domain, the binding domain may be located either amino or carboxyl terminal to the Fc region portion of the single chain polypeptide. For example, a single chain polypeptide comprising two binding domains may have one binding domain located amino terminal and the other carboxyl terminal to the Fc region portion of the single chain polypeptide, or both binding domains may be amino terminal or both carboxyl terminal to the Fc region portion. In another example, a single chain polypeptide may comprise three binding domains wherein (a) two binding domains are amino terminal on different single chain proteins and the third binding domain is carboxyl terminal to the Fc region portion on either SCP-I or SCP-II, (b) two binding domains are carboxyl terminal on different single chain proteins and the third binding domain is amino terminal to the Fc region portion on either SCP-I or SCP-II. In still a further example, a polypeptide heterodimer may comprise four binding domains, wherein two binding domains are located amino terminal to the Fc region portion on different chains and the other two binding domains are located carboxyl terminal to the Fc region portion on different chains. Alternatively, in any of these embodiments, two binding domains may be linked to each other in tandem and located on either SCP-I or SCP-II or both, depending on the number of binding domains present - the tandem stacking is used when five to eight binding domains combined are present in SCP-I and SCP-II.

Binding of a target by a binding domain modulates the interaction between the target *(e.g.,* a receptor or a ligand) and another molecule. In certain embodiments, the binding of a target *(e.g.,* a receptor) by a binding domain stimulates certain functions of the target (*e.g*., signal transduction) or brings different targets closer together for a biological effect (*e.g.*, directing T cells to a tumor which in turn activates the T cells). In certain other embodiments, the binding of a target by a binding domain blocks the interaction between the target and another molecule and thus interferes, reduces or eliminates certain functions of the target.

A target molecule, which is specifically bound by a binding domain contained in a polypeptide heterodimer of the present disclosure, may be found on or in association with a cell of interest ("target cell"). Exemplary target cells include cancer cells, cells associated with an autoimmune disease or disorder or with an inflammatory disease or disorder, B-cells and T-cells. A target molecule may also not be associated with a cell. Exemplary target molecules not associated with a cell include soluble proteins, secreted proteins, deposited proteins, and extracellular structural (matrix) proteins.

In certain embodiments, binding domains of polypeptide heterodimers of the present disclosure specifically bind a target selected from T-cell targets, tumor antigens, B-cell targets, pro-inflammatory cytokines or chemokines, pro-oncogenic cytokines or growth factors, angiogenic agents, Fc receptors, transferrin receptor, receptor tyrosine kinases (RTKs), TNFSFRs, or any combination thereof. For instance, polypeptide heterodimers of the present disclosure specifically bind a T-cell target and a tumor target.

In certain embodiments, a binding domain of a polypeptide heterodimer of the present disclosure specifically binds a T-cell target, such as a TCR complex or a component thereof (*e.g.*, TCRα, TCRβ, CD3γ, CD3δ, and CD3ε), CD28, PD-1, HVEM, BTLA, CD80, CD86, GITR, and TGFBR1.

In certain embodiments, a binding domain of polypeptide heterodimer of the present disclosure specifically binds a TCR complex or a component thereof. For example, in certain embodiments, a binding domain specifically binds to an individual human CD3 chain (*e.g*., human CD3γ chain, human CD3δ chain, and human CD3ε chain) or a combination of two or more of the individual human CD3 chains *(e.g.,* a complex of human CD3γ and human CD3ε or a complex of human CD3δ and human CD3ε). In certain embodiments, a binding domain specifically binds to a human CD3ε chain. In certain other embodiments, a binding domain specifically binds to one or more of human TCRα, human TCRβ, or a heterodimer formed from human TCRα and human TCRβ. In certain other embodiments, a binding domain of the present disclosure binds to a complex formed from one or more human CD3 chains with one or more human TCR chains, such as a complex formed from a human CD3γ chain, a human CD3δ chain, or a human CD3ε chain with a human TCRα chain or a human TCRβ chain. In any of these embodiments, a polypeptide heterodimer of the present disclosure may also bind a tumor target.

In certain embodiments, one or more binding domains of a polypeptide heterodimer of the present disclosure specifically bind a tumor target, including RON, c-Met, CEACAM-6, PSMA, EpCAM, CEA, PCTA-1, STEAP-1, PSCA, ALCAM (CD166), EphA2, CD151, CA-125, MUC-1, MAGE-1, TROP2, IGF1R, CD44v6, CD151, TGFBR2, GHRHR, GHR, IL-6R, gp130, TNFR2, OSMRβ, Patched-1, Frizzled, Robo1, LTβT, CD80, CD81, CD86, CCR5, HER-3, HER-4, EGFR, CEA, MUC2, MUC3, MUC4, MUC5_{AC}, MUC5_{b}, MUC7, βhCG, Lewis-Y, CD33, CD30, ganglioside GD3, 9-O-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP-2, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, B7-H3, or any combination thereof.

In certain embodiments, one or more binding domains of a polypeptide heterodimer of the present disclosure specifically bind a B-cell target, such as CD 19, CD20, CD21, CD22, CD30, CD33, CD37, CD38, CD70, CD79b, HLA-DR, or any combination thereof.

In certain embodiments, one or more binding domains of a polypeptide heterodimer of the present disclosure specifically bind a pro-inflammatory cytokine or chemokine, such as TNFα, IL-6, hyperIL-6, IL-2, IL-1, IL-8, IP-10, IFNγ, IFNα, RANKL, FASL, TGFβ, IL7, IL10, IL17A/F, TWEAK, CSF2, IGF1, IGF2 or BLyS/APRIL, or any combination thereof.

In certain embodiments, one or more binding domains of a polypeptide heterodimer of the present disclosure specifically bind a pro-oncogenic cytokine or growth factor, such as HGF, MSP, EGF (including epiregulin, herregulin, β-regulin, neuregulin), HIF-1α, VEGFA, VEGFB, VEGFC, VEGFD, TNFα, IL-6, hyperIL-6, IL-8, Wnt, sHH, TGFβ, PDGF, or any combination thereof.

In certain embodiments, one or more binding domains of a polypeptide heterodimer of the present disclosure specifically bind an angiogenic agent, such as PDGFR, VEGFR1-4, NRP1, Angiopoietin 2, c-Met or any combination thereof.

In certain embodiments, one or more binding domains of a polypeptide heterodimer of the present disclosure specifically bind an Fc receptor, such as CD64, CD32A, CD32B, CD16, FcRn, or any combination thereof.

In certain embodiments, a binding domain of a polypeptide heterodimer of the present disclosure specifically binds a transferrin receptor, such as CD71.

In certain embodiments, one or more binding domains of a polypeptide heterodimer of the present disclosure specifically binds a receptor tyrosine kinase, such as EGFR, EGFRvIII, ErbB2, ErbB3, ErbB4, IGF1R, EphA2, c-Met, RON, or any combination thereof.

In certain embodiments, one or more binding domains of a polypeptide heterodimer of the present disclosure specifically binds a TNFSFR, such as TNFR1, TNFR2, TWEAKR, TACI, BAFF-R, BCMA, FAS, OX40, GITR, 4-1-BB, LTbetaR, HVEM, RANK, or any combination thereof.

In certain embodiments, one or more binding domains of a polypeptide heterodimer of the present disclosure specifically bind hyperIL-6, IL-10, LIGHT, CD40, PDL1, PDL2, or any combination thereof.

In certain embodiments, one or more binding domains of a polypeptide heterodimer of the present disclosure are an agonist of one of the following molecules: IL-10, HLA-G, HGF, IL-35, PD-1, BTLA, TNFR1, TNFR2, DR4, DR5, TWEAKR, FAS, or any combination thereof.

A binding domain may be any peptide that specifically binds a target of interest. Sources of binding domains include antibody variable regions from various species (which can be formatted as antibodies, sFvs, scFvs, Fabs, or soluble VH domain or domain antibodies), including human, rodent, avian, and ovine. Additional sources of binding domains include variable regions of antibodies from other species, such as camelid (from camels, dromedaries, or llamas; Ghahroudi et al. (1997) FEBS Letters 414(3):521-526; Vincke et al. (2009) Journal of Biological Chemistry (2009) 284:3273-3284; Hamers-Casterman et al. (1993) Nature, 363:446 and Nguyen et al. (1998) J. Mol. Biol., 275:413), nurse sharks (Roux et al. (1998) Proc. Nat'l. Acad. Sci. (USA) 95:11804), spotted ratfish (Nguyen et al. (2002) Immunogenetics, 54:39), or lamprey (Herrin et al., (2008) Proc. Nat'l. Acad. Sci. (USA) 105:2040-2045 and Alder et al. (2008) Nature Immunology 9:319-327). These antibodies can apparently form antigen-binding regions using only heavy chain variable region, *i.e.,* these functional antibodies are homodimers of heavy chains only (referred to as "heavy chain antibodies") (Jespers et al. (2004) Nature Biotechnology 22:1161-1165; Cortez-Retamozo et al. (2004) Cancer Research 64:2853-2857; Baral et al. (2006) Nature Medicine 12:580-584, and Barthelemy et al. (2008) Journal of Biological Chemistry 283:3639-3654).

Exemplary anti-CD3 antibodies from which the binding domain of this disclosure may be derived include Cris-7 monoclonal antibody (Reinherz, E. L. et al. (eds.), Leukocyte typing II., Springer Verlag, New York, (1986)), BC3 monoclonal antibody (Anasetti et al. (1990) J. Exp. Med. 172:1691), OKT3 (Ortho multicenter Transplant Study Group (1985) N. Engl. J. Med. 313:337) and derivatives thereof such as OKT3 ala-ala (Herold et al. (2003) J. Clin. Invest. 11:409), visilizumab (Carpenter et al. (2002) Blood 99:2712), and 145-2C11 monoclonal antibody (Hirsch et al. (1988) J. Immunol. 140: 3766). An exemplary anti-TCR antibody is H57 monoclonal antibody (Lavasani et al. (2007) Scandinavian Journal of Immunology 65:39-47).

An alternative source of binding domains of this disclosure includes sequences that encode random peptide libraries or sequences that encode an engineered diversity of amino acids in loop regions of alternative non-antibody scaffolds, such as fibrinogen domains *(see, e.g.,* Weisel et al. (1985) Science 230:1388), Kunitz domains *(see, e.g.,* US Patent No. 6,423,498), ankyrin repeat proteins (Binz et al. (2003) Journal of Molecular Biology 332:489-503 and Binz et al. (2004) Nature Biotechnology 22(5):575-582), fibronectin binding domains (Richards et al. (2003) Journal of Molecular Biology 326:1475-1488; Parker et al. (2005) Protein Engineering Design and Selection 18(9):435-444 and Hackel et al. (2008) Journal of Molecular Biology 381:1238-1252), cysteine-knot miniproteins (Vita et al. (1995) Proc. Nat'l. Acad. Sci. (USA) 92:6404-6408; Martin et al. (2002) Nature Biotechnology 21:71-76 and Huang et al. (2005) Structure 13:755-768), tetratricopeptide repeat domains (Main et al. (2003) Structure 11:497-508 and Cortajarena et al. (2008) ACS Chemical Biology 3:161-166), leucine-rich repeat domains (Stumpp et al. (2003) Journal of Molecular Biology 332:471-487), lipocalin domains (*see, e.g.,* WO 2006/095164, Beste et al. (1999) Proc. Nat'l. Acad. Sci. (USA) 96:1898-1903 and Schönfeld et al. (2009) Proc. Nat'l. Acad. Sci. (USA) 106:8198-8203), V-like domains (*see, e.g.,* US Patent Application Publication No. 2007/0065431), C-type lectin domains (Zelensky and Gready (2005) FEBS J. 272:6179; Beavil et al. (1992) Proc. Nat'l. Acad. Sci. (USA) 89:753-757 and Sato et al. (2003) Proc. Nat'l. Acad. Sci. (USA) 100:7779-7784), mAb² or FCAB™ *(see, e.g.,* PCT Patent Application Publication Nos. WO 2007/098934; WO 2006/072620), or the like (Nord et al. (1995) Protein Engineering 8(6):601-608; Nord et al. (1997) Nature Biotechnology 15:772-777; Nord et al. (2001) European Journal of Biochemistry 268(15):4269-4277 and Binz et al. (2005) Nature Biotechnology 23:1257-1268).

Binding domains of this disclosure can be generated as described herein or by a variety of methods known in the art *(see, e.g.,* U.S. Patent Nos. 6,291,161 and 6,291,158). For example, binding domains of this disclosure may be identified by screening a Fab phage library for Fab fragments that specifically bind to a target of interest *(see* Hoet et al. (2005) Nature Biotechnol. 23:344). Additionally, traditional strategies for hybridoma development using a target of interest as an immunogen in convenient systems (*e.g.*, mice, HUMAB MOUSE®, TC MOUSE™, KM-MOUSE^{®}, llamas, chicken, rats, hamsters, rabbits, *etc.*) can be used to develop binding domains of this disclosure.

In certain embodiments, a polypeptide heterodimer comprises a CD86 binding domain, such as a CTLA4 ectodomain, a CD28 ectodomain, or an immunoglobulin variable region binding domain (such as a scFv) specific for CD86 *(e.g.,* from monoclonal antibodies 3D1 or FUN1). In some embodiments, less than an entire ectodomain is used. For example, domains within the CTLA4 ectodomain that bind CD86 and prevent binding of CD86 to CD28 can be used. The CD86 binding domain can block binding of CD86 to CD28 and thereby downregulate T-cell activation.

In certain embodiments, a polypeptide heterodimer comprises an IL-10 agonist, such as IL-10, monoIL-10, or a functional region thereof. "MonoIL-10" refers to an IL-10 molecule having a short linker (GGGSGG, SEQ ID NO:760) that separates the two subdomains of the molecule (amino and carboxyl end domains) so that these subdomains can form an intramolecular dimer.

In certain embodiments, a polypeptide heterodimer comprises an HLA-G agonist, such as an HLA-G5, an HLA-G1, an HLA-G mutein, or a functional region thereof; an ectodomain of an HLA-G5, an HLA-G1 or an HLA-G mutein; or an immunoglobulin variable region binding domain (such as an scFv) specific for ILT2, ILT4 or KIR2DL4.

In certain embodiments, a polypeptide heterodimer comprises an HGF agonist, such as an HGF or a sub-domain thereof.

In certain embodiments, a polypeptide heterodimer comprises an IL35 agonist, such as an immunoglobulin variable region binding domain (such as an scFv) specific for IL35R or IL35, or a functional region thereof.

In certain embodiments, a polypeptide heterodimer comprises a LIGHT antagonist, such as an immunoglobulin variable region binding domain (such as an scFv) specific for LIGHT, or a HVEM ectodomain or a functional region thereof.

In certain embodiments, a polypeptide heterodimer comprises a PD-1 agonist, such as an immunoglobulin variable region binding domain (such as an scFv) specific for PD-1, or a PD-1 ligand (e.g. PD-L1 or PD-L2) or a functional region thereof.

In certain embodiments, a polypeptide heterodimer comprises a BTLA agonist, such as an immunoglobulin-like variable region binding domain (such as an scFv) specific for BTLA, or a HVEM ectodomain or a functional region thereof.

In certain embodiments, a polypeptide heterodimer comprises a GITRL antagonist, such as an immunoglobulin-like variable region binding domain (such as an scFv) specific for GITRL, or a GITR ectodomain, soluble GITR, or a functional region thereof.

In certain embodiments, a polypeptide heterodimer comprises a CD40 antagonist, such as an immunoglobulin-like variable region binding domain (such as an scFv) specific for CD40.

In some embodiments, a binding domain is a single chain Fv fragment (scFv) that comprises VH and VL regions specific for a target of interest. In certain embodiments, the V_{H} and V_{L} domains are human. Exemplary VH regions include the VH region of 2E12 (anti-CD28) scFv as set forth in SEQ ID NO:106, the VH region of P2C2 (anti-CD79b) scFv as set forth in SEQ ID NO:184, the VH region of 5D5 (anti-c-Met) scFv as set forth in SEQ ID NO:258, the VH region of A2 (anti-hyperIL-6) scFv as set forth in SEQ ID NO:80, the VH region of 3D1 (anti-CD86) scFv as set forth in SEQ ID NO:92, the VH region of MET021 (anti-c-Met) scFv as set forth in SEQ ID NO:100, the VH region of G19-4 (anti-CD3) scFv as set forth in SEQ ID NO:103, the VH region of HD37 (anti-CD 19) scFv as set forth in SEQ ID NO:117, the VH region of M0042 (anti-HLA-DR) scFv as set forth in SEQ ID NO:121, the VH region of BMA031 (anti-TCR) scFv as set forth in SEQ ID NO:828, the VH region of OKT3-M (anti-CD3) scFv as set forth in SEQ ID NO:831, and the VH region of HuM291 (anti-CD3) scFv as set forth in SEQ ID NO:835. The nucleotide sequences encoding the VH regions of the A2 (anti-hyperIL-6) and 3D1 (anti-CD86) scFv's are set forth in SEQ ID NOS:79 and 91, respectively.

Exemplary VL domains are the VL region of 2E12 (anti-CD28) scFv as set forth in SEQ ID NO:107, the VL region of P2C2 (anti-CD79b) scFv as set forth in SEQ ID NO:182, the VL region of 5D5 (anti-c-Met) scFv as set forth in SEQ ID NO:259, the VL region of A2 (anti-hyper IL-6) scFv as set forth in SEQ ID NO:84, the VL region of 3D1 (anti-CD86) scFv as set forth in SEQ ID NO:96, the VL region of MET021 (anti-c-Met) scFv as set forth in SEQ ID NO:101, the VL region of G19-4 (anti-CD3) scFv as set forth in SEQ ID NO:104, the VL region of HD37 (anti-CD19) scFv as set forth in SEQ ID NO:119, the VL region of M0042 (anti-HLA-DR) scFv as set forth in SEQ ID NO:122, the VL region of BMA031 (anti-TCR) scFv as set forth in SEQ ID NO:829, the VL region of OKT3-M (anti-CD3) scFv as set forth in SEQ ID NO:833, and the VL region of HuM291 (anti-CD3) scFv as set forth in SEQ ID NO:836. The nucleotide sequences encoding the VL regions of the A2 (anti-hyperIL-6) and 3D1 (anti-CD86) scFv's are set forth in SEQ ID NOS:83 and 95, respectively.

In some embodiments, a binding domain is a single chain Fv fragment (scFv) that comprises V_{H} and V_{L} domains specific for a TCR complex or a component thereof. In certain embodiments, the V_{H} and V_{L} domains are human or humanized V_{H} and V_{L} domains. Exemplary V_{H} domains include BC3 (anti-CD3) V_{H}, OKT3 (anti-CD3) V_{H}, H57 (anti-TCR) V_{H}, and 2C11 (anti-CD3) V_{H} domains as set forth in SEQ ID NOS:301, 303, 313, and 317, respectively. Further exemplary V_{H} domains include Cris-7 (anti-CD3) V_{H} domains, such as those set forth in SEQ ID NOS:327 and 331-333. Exemplary V_{L} domains are BC3 (anti-CD3) V_{L}, OKT3 (anti-CD3) V_{L}, H57 (anti-TCR) V_{L}, and 2C11 (anti-CD3) V_{L} domains as set forth in SEQ ID NOS:302, 304, 315 and 318, respectively. Further exemplary V_{L} domains include Cris-7 (anti-CD3) V_{L} domains, such as those set forth in SEQ ID NOS:328, 329 and 330.

In certain embodiments, a binding domain comprises or is a sequence that is at least 90%, at least 91%, at least 92%, at least 93%, at least 94%, at least 95%, at least 96%, at least 97%, at least 98%, at least 99%, at least 99.5%, or 100% identical to an amino acid sequence of a light chain variable region (V_{L}) or to a heavy chain variable region (V_{H}), or both, wherein each CDR comprises zero changes or at most one, two, or three changes, from a monoclonal antibody or fragment or derivative thereof that specifically binds to a target of interest (*e.g.,* c-Met, RON, CD28, CD79b, CD3ε, TCRα, TCRβ, hyper IL-6, CD86, CD19, and HLA-DR) and such mutant or derivative still binds its target.

In certain embodiments, a binding domain VH or VL region of the present disclosure can be derived from or based on a VH or VL of a known monoclonal antibody and contains one or more *(e.g.,* 2, 3, 4, 5, 6, 7, 8, 9, 10) insertions, one or more *(e.g.,* 2, 3, 4, 5, 6, 7, 8, 9, 10) deletions, one or more *(e.g.,* 2, 3, 4, 5, 6, 7, 8, 9, 10) amino acid substitutions (*e.g.,* conservative amino acid substitutions or non-conservative amino acid substitutions), or a combination of the above-noted changes, when compared with the VH or VL of a known monoclonal antibody. The insertion(s), deletion(s) or substitution(s) may be anywhere in the VH region, including at the amino- or carboxy-terminus or both ends of this region, provided that each CDR comprises zero changes or at most one, two, or three changes and provided that a binding domain containing the modified VH or VL region can still specifically bind its target with an affinity about the same as the wild type binding domain.

The VH and VL domains may be arranged in either orientation (*i.e.,* from amino-terminus to carboxyl terminus, VH-VL or VL-VH) and may be joined by an amino acid sequence (*e.g.,* having a length of about five to about 35 amino acids) capable of providing a spacer function such that the two sub-binding domains can interact to form a functional binding domain. In certain embodiments, an amino acid sequence that joins the VH and VL domains (also referred to herein as a "linker") includes those belonging to the (GlyₙSer) family, such as (Gly₃Ser)ₙ(Gly₄Ser)₁, (Gly₃Ser)₁(Gly₄Ser)ₙ, (Gly₃Ser)ₙ(Gly₄Ser)ₙ, or (Gly₄Ser)ₙ, wherein n is an integer of 1 to 5. In certain embodiments, the linker is GGGGSGGGGS GGGGS (SEQ ID NO:183) or GGGGSGGGGSGGGGSGGGGS (SEQ ID NO:108). An additional exemplary linker is GGGGSGGGGSGGGGAS (SEQ ID NO:739). In certain embodiments, these (GlyₙSer)-based linkers are used to link the VH and VL domains in a binding domain, but are not used to link a binding domain to an immunoglobulin heterodimerization domain or to an Fc region portion.

Exemplary binding domains specific for CD28 include a 2E12 scFv as set forth in SEQ ID NO:109, binding domains specific for CD79b include a P2C2 scFv as set forth in SEQ ID NO:185; binding domains specific for c-Met include a 5D5 scFv as set forth in SEQ ID NO:257; binding domains specific for RON include a 4C04 scFv as set forth in SEQ ID NO:261 and a 11H09 scFv as set forth in SEQ ID NO:265; binding domains specific for hyperIL-6 include an A2 scFv as set forth in SEQ ID NO:86; binding domains specific for CD86 include a 3D1 scFv as set forth in SEQ ID NO:98; binding domains specific for HLA-DR include an M0042 scFv as set forth in SEQ ID NO:120; binding domains specific for CD3 include a G19-4 scFv as set forth in SEQ ID NO:102, an OKT3-M scFv as set forth in SEQ ID NO:834, and a HuM291 scFv as set forth in SEQ ID NO:837; binding domain specific for CD19 include a H37 scFv as set forth in SEQ ID NO:105; and binding domains specific for c-Met include an MET021 scFv as set forth in SEQ ID NO:120 (with the light chain CDR1, CDR2, CDR3 and heavy chain CDR1, CDR2 and CDR3 as set forth in SEQ ID NOS:296-298 and 464-466, respectively). The nucleotide sequences encoding the A2 (anti-hyperIL6) and 3D1 (anti-CD86) scFv's are set forth in SEQ ID NOS:85 and 97, respectively. Exemplary binding domains that bind a TCR complex or a component thereof include a BMA031 scFv as set forth in SEQ ID NO:830 and other scFv's as set forth in SEQ ID NOS:310, 311, 312, 319, and 334-340.

Additional exemplary binding domains include a PDL2 ectodomain as set forth in SEQ ID NO:88 and monoIL-10 as set forth in SEQ ID NO:90. The nucleotide sequences encoding the PDL2 ectodomain and monoIL-10 are set forth in SEQ ID NOS:87 and 89, respectively.

The light chain amino acid sequence of the 4C04 (anti-RON) scFv is set forth in SEQ ID NO:602, and its CDR1, CDR2, and CDR3 are set forth in SEQ ID NOS:604-606, respectively. The heavy chain amino acid sequence of the 4C04 (anti-RON) scFv is set forth in SEQ ID NO:603, and its CDR1, CDR2, and CDR3 are set forth in SEQ ID NOS:607-609, respectively.

The light chain amino acid sequence of the 11H09 (anti-RON) scFv is set forth in SEQ ID NO:610, and its CDR1, CDR2, and CDR3 are set forth in SEQ ID NOS:612-614, respectively. The heavy chain amino acid sequence of the 11H09 (anti-RON) scFv is set forth in SEQ ID NO:611, and its CDR1, CDR2, and CDR3 are set forth in SEQ ID NOS:615-617, respectively.

A binding domain may be located either at amino terminal or carboxyl terminal to the Fc region portion of a single chain polypeptide of the present disclosure. In certain embodiments, the binding domain is located at the amino terminus of a single chain polypeptide. In certain other embodiments, the binding domain is located at the carboxyl terminus of a single chain polypeptide. In certain other embodiments, a binding domain is located at both the amino and carboxyl terminus of a single chain polypeptide.

### Heterodimerization Domains

As indicated above, a polypeptide heterodimer of the present disclosure comprises an immunoglobulin heterodimerization domain in each polypeptide chain. The immunoglobulin heterodimerization domains in the two single chain polypeptides of a polypeptide heterodimer are different from each other and thus may be differentially modified to facilitate heterodimerization of both chains and to minimize homodimerization of either chain. As shown in the examples, immunoglobulin heterodimerization domains provided herein allow for efficient heterodimerization between different polypeptides and facilitate purification of the resulting polypeptide heterodimers.

As provided herein, immunoglobulin heterodimerization domains useful for promoting heterodimerization of two different single chain polypeptides (e.g., one short and one long) according to the present disclosure include immunoglobulin CH1 and CL domains, for instance, human CH1 and CL domains. In certain embodiments, an immunoglobulin heterodimerization domain is a wild type CH1 region, such as a wild type IgG1, IgG2, IgG3, IgG4, IgA1, IgA2, IgD, IgE, or IgM CH1 region. In further embodiments, an immunoglobulin heterodimerization domain is a wild type human IgG1, IgG2, IgG3, IgG4, IgA1, IgA2, IgD, IgE, or IgM CH1 region as set forth in SEQ ID NOS:114, 186-192 and 194, respectively. In certain embodiments, an immunoglobulin heterodimerization domain is a wild type human IgG1 CH1 region as set forth in SEQ ID NO:114.

In further embodiments, an immunoglobulin heterodimerization domain is an altered immunoglobulin CH1 region, such as an altered IgG1, IgG2, IgG3, IgG4, IgA1, IgA2 IgD, IgE, or IgM CH1 region. In certain embodiments, an immunoglobulin heterodimerization domain is an altered human IgG1, IgG2, IgG3, IgG4, IgA1, IgA2, IgD, IgE, or IgM CH1 region. In still further embodiments, a cysteine residue of a wild type CH1 region (*e.g.,* a human CH1) involved in forming a disulfide bond with a wild type immunoglobulin CL domain (*e.g.,* a human CL) is deleted or substituted in the altered immunoglobulin CH1 region such that a disulfide bond is not formed between the altered CH1 region and the wild type CL domain.

In certain embodiments, an immunoglobulin heterodimerization domain is a wild type CL domain, such as a wild type Cκ domain or a wild type Cλ domain. In particular embodiments, an immunoglobulin heterodimerization domain is a wild type human Cκ or human Cλ domain as set forth in SEQ ID NOS:112 and 113, respectively. In further embodiments, an immunoglobulin heterodimerization domain is an altered immunoglobulin CL domain, such as an altered Cκ or Cλ domain, for instance, an altered human Cκ or human Cλ domain.

In certain embodiments, a cysteine residue of a wild type CL domain *(e.g.,* a human CL) involved in forming a disulfide bond with a wild type immunoglobulin CH1 region *(e.g.,* a human CH1) is deleted or substituted in the altered immunoglobulin CL domain. Such altered CL domains may further comprise an amino acid deletion at their amino termini. An exemplary Cκ domain is set forth in SEQ ID NO:141, in which the first arginine and the last cysteine of the wild type human Ck domain are both deleted. In certain embodiments, only the last cysteine of the wild type human Ck domain is deleted in the altered Ck domain because the first arginine deleted from the wild type human Ck domain may be provided by a linker that has an arginine at its carboxyl terminus and links the amino terminus of the altered Ck domain with another domain (*e.g*., an Fc region portion). An exemplary Cλ domain is set forth in SEQ ID NO:140, in which the first arginine of a wild type human Cλ domain is deleted and the cysteine involved in forming a disulfide bond with a cysteine in a CH1 region is substituted by a serine.

In further embodiments, an immunoglobulin heterodimerization domain is an altered Cκ domain that contains one or more amino acid substitutions, as compared to a wild type Cκ domain, at positions that may be involved in forming the interchain-hydrogen bond network at a Cκ-Cκ interface. For example, in certain embodiments, an immunoglobulin heterodimerization domain is an altered human Cκ domain having one or more amino acids at positions N29, N30, Q52, V55, T56, S68 or T70 that are substituted with a different amino acid. The numbering of the amino acids is based on their positions in the altered human Cκ sequence as set forth in SEQ ID NO:141. In certain embodiments, an immunoglobulin heterodimerization domain is an altered human Cκ domain having one, two, three or four amino acid substitutions at positions N29, N30, V55, or T70. The amino acid used as a substitute at the above-noted positions may be an alanine, or an amino acid residue with a bulk side chain moiety such as arginine, tryptophan, tyrosine, glutamate, glutamine, or lysine. Additional amino acid residues that may be used to substitute amino acid residues of the wild type human Ck sequence at the above noted positions (*e.g.*, N30) include aspartate, methionine, serine and phenyalanine. Exemplary altered human Cκ domains are set forth in SEQ ID NOS: 142-178. Altered human Cκ domains are those that facilitate heterodimerization with a CH1 region, but minimize homodimerization with another Cκ domain. Representative altered human Cκ domains are set forth in SEQ ID NOS:160 (N29W V55A T70A), 161 (N29Y V55A T70A), 202 (T70E N29A N30A V55A), 167 (N30R V55A T70A), 168 (N30K V55A T70A), 170 (N30E V55A T70A), 172 (V55R N29A N30A), 175 (N29W N30Y V55A T70E), 176 (N29Y N30Y V55A T70E), 177 (N30E V55A T70E), 178 (N30Y V55A T70E), 838 (N30D V55A T70E), 839 (N30M V55A T70E), 840 (N30S V55A T70E), and 841 (N30F V55A T70E).

In certain embodiments, in addition to or alternative to the mutations in Ck domains described herein, both the immunoglobulin heterodimerization domains (*i.e.,* immunoglobulin CH1 and CL domains) of a polypeptide heterodimer have mutations so that the resulting immunoglobulin heterodimerization domains form salt bridges (*i.e.,* ionic interactions) between the amino acid residues at the mutated sites. For example, the immunoglobulin heterodimerization domains of a polypeptide heterodimer may be a mutated CH1 domain in combination with a mutated Ck domain. In the mutated CH1 domain, valine at position 68 (V68) of the wild type human CH1 domain is substituted by an amino acid residue having a negative charge (*e.g.*, aspartate or glutamate), whereas leucine at position 29 (L29) of a mutated human Ck domain in which the first arginine and the last cysteine have been deleted is substituted by an amino acid residue having a positive charge (*e.g*., lysine, arginine or histidine). The charge-charge interaction between the amino acid residue having a negative charge of the resulting mutated CH1 domain and the amino acid residue having a positive charge of the resulting mutated Ck domain forms a salt bridge, which stabilizes the heterodimeric interface between the mutated CH1 and Ck domains. Alternatively, V68 of the wild type CH1 may be substituted by an amino acid residue having a positive charge, whereas L29 of a mutated human Ck domain in which the first arginine and the last cysteine have been deleted may be substituted by an amino acid residue having a negative charge. Exemplary mutated CH1 sequences in which V68 is substituted by an amino acid with either a negative or positive charge are set forth in SEQ ID NOS:844 and 845. Exemplary mutated Ck sequences in which L29 is substituted by an amino acid with either a negative or positive charge are set forth in SEQ ID NOS:842 and 843.

Positions other than V68 of human CH1 domain and L29 of human Ck domain may be substituted with amino acids having opposite charges to produce ionic interactions between the amino acids in addition or alternative to the mutations in V68 of CH1 domain and L29 of Ck domain. Such positions can be identified by any suitable method, including random mutagenesis, analysis of the crystal structure of the CH1-Ck pair to identify amino acid residues at the CH1-Ck interface, and further identifying suitable positions among the amino acid residues at the CH1-Ck interface using a set of criteria (*e.g.*, propensity to engage in ionic interactions, proximity to a potential partner residue, *etc.*).

In certain embodiments, polypeptide heterodimers of the present disclosure contain only one pair of immunoglobulin heterodimerization domains. For example, a first chain of a polypeptide heterodimer may comprise a CH1 region as an immunoglobulin heterodimerization domain, while a second chain may comprise a CL domain *(e.g.,* a Cκ or Cλ) as an immunoglobulin heterodimerization domain. Alternatively, a first chain may comprise a CL region *(e.g.,* a Cκ or Cλ) as an immunoglobulin heterodimerization domain, while a second chain may comprise a CH1 region as an immunoglobulin heterodimerization domain. As set forth herein, the immunoglobulin heterodimerization domains of the first and second chains are capable of associating to form a polypeptide heterodimer of this disclosure.

In certain other embodiments, polypeptide heterodimers of the present disclosure may have two pairs of immunoglobulin heterodimerization domains. For example, a first chain of a polypeptide heterodimer may comprise two CH1 regions, while a second chain may have two CL domains that associate with the two CH1 regions in the first chain. Alternatively, a first chain may comprise two CL domains, while a second chain may have two CH1 regions that associate with the two CL domains in the first chain. In certain embodiments, a first chain polypeptide comprises a CH1 region and a CL domain, while a second chain polypeptide comprises a CL domain and a CH1 region that associate with the CH1 region and the CL domain, respectively, of the first chain polypeptide.

In the embodiments where a polypeptide heterodimer comprises only one heterodimerization pair (*i.e.,* one immunoglobulin heterodimerization domain in each chain), the immunoglobulin heterodimerization domain of each chain may be located amino terminal to the Fc region portion of that chain. Alternatively, the immunoglobulin heterodimerization domain in each chain may be located carboxyl terminal to the Fc region portion of that chain.

In the embodiments where a polypeptide heterodimer comprises two heterodimerization pairs (*i.e.,* two immunoglobulin heterodimerization domains in each chain), both immunoglobulin heterodimerization domains in each chain may be located amino terminal to the Fc region portion of that chain. Alternatively, both immunoglobulin heterodimerization domains in each chain may be located carboxyl terminal to the Fc region portion of that chain. In further embodiments, one immunoglobulin heterodimerization domain in each chain may be located amino terminal to the Fc region portion of that chain, while the other immunoglobulin heterodimerization domain of each chain may be located carboxyl terminal to the Fc region portion of that chain. In other words, in those embodiments, the Fc region portion is interposed between the two immunoglobulin heterodimerization domains of each chain.

### Fc region portion

As indicated herein, polypeptide heterodimers of the present disclosure comprise an Fc region constant domain portion (also referred to as an Fc region portion) in each polypeptide chain. The inclusion of an Fc region portion slows clearance of the heterodimers from circulation after administration to a subject. By mutations or other alterations, the Fc region portion further enables relatively easy modulation of heterodimer polypeptide effector functions (*e.g.*, ADCC, ADCP, CDC, complement fixation and binding to Fc receptors), which can either be increased or decreased depending on the disease being treated, as known in the art and described herein. In certain embodiments, an Fc region portion of polypeptide heterodimers of the present disclosure will be capable of mediating one or more of these effector functions.

An Fc region portion present in single chain polypeptides that form part of the polypeptide heterodimers of the present disclosure may comprise a CH2 domain, a CH3 domain, a CH4 domain or any combination thereof. For example, an Fc region portion may comprise a CH2 domain, a CH3 domain, both CH2 and CH3 domains, both CH3 and CH4 domains, two CH3 domains, a CH4 domain, or two CH4 domains.

A CH2 domain that may form an Fc region portion of a single chain polypeptide of a heterodimer of the present disclosure may be a wild type immunoglobulin CH2 domain or an altered immunoglobulin CH2 domain thereof from certain immunoglobulin classes or subclasses (*e.g.*, IgG1, IgG2, IgG3, IgG4, IgA1, IgA2, or IgD) and from various species (including human, mouse, rat, and other mammals).

In certain embodiments, a CH2 domain is a wild type human immunoglobulin CH2 domain, such as wild type CH2 domains of human IgG1, IgG2, IgG3, IgG4, IgA1, IgA2, or IgD, as set forth in SEQ ID NOS:115, 199-201 and 195-197, respectively. In certain embodiments, the CH2 domain is a wild type human IgG1 CH2 domain as set forth in SEQ ID NO:115.

In certain embodiments, a CH2 domain is an altered immunoglobulin CH2 region *(e.g.,* an altered human IgG1 CH2 domain) that comprises an amino acid substitution at the asparagine of position 297 (*e.g.*, asparagine to alanine). Such an amino acid substitution reduces or eliminates glycosylation at this site and abrogates efficient Fc binding to FcγR and C1q. The sequence of an altered human IgG1 CH2 domain with an Asn to Ala substitution at position 297 is set forth in SEQ ID NO:324.

In certain embodiments, a CH2 domain is an altered immunoglobulin CH2 region *(e.g.,* an altered human IgG1 CH2 domain) that comprises at least one substitution or deletion at positions 234 to 238. For example, an immunoglobulin CH2 region can comprise a substitution at position 234, 235, 236, 237 or 238, positions 234 and 235, positions 234 and 236, positions 234 and 237, positions 234 and 238, positions 234-236, positions 234, 235 and 237, positions 234, 236 and 238, positions 234, 235, 237, and 238, positions 236-238, or any other combination of two, three, four, or five amino acids at positions 234-238. In addition or alternatively, an altered CH2 region may comprise one or more (*e.g.*, two, three, four or five) amino acid deletions at positions 234-238, for instance, at one of position 236 or position 237 while the other position is substituted. The above-noted mutation(s) decrease or eliminate the antibody-dependent cell-mediated cytotoxicity (ADCC) activity or Fc receptor-binding capability of a polypeptide heterodimer that comprises the altered CH2 domain. In certain embodiments, the amino acid residues at one or more of positions 234-238 has been replaced with one or more alanine residues. In further embodiments, only one of the amino acid residues at positions 234-238 have been deleted while one or more of the remaining amino acids at positions 234-238 can be substituted with another amino acid (*e.g.*, alanine or serine).

In certain other embodiments, a CH2 domain is an altered immunoglobulin CH2 region *(e.g.,* an altered human IgG1 CH2 domain) that comprises one or more amino acid substitutions at positions 253, 310, 318, 320, 322, and 331. For example, an immunoglobulin CH2 region can comprise a substitution at position 253, 310, 318, 320, 322, or 331, positions 318 and 320, positions 318 and 322, positions 318, 320 and 322, or any other combination of two, three, four, five or six amino acids at positions 253, 310, 318, 320, 322, and 331. The above-noted mutation(s) decrease or eliminate the complement-dependent cytotoxicity (CDC) of a polypeptide heterodimer that comprises the altered CH2 domain.

In certain other embodiments, in addition to the amino acid substitution at position 297, an altered CH2 region *(e.g.,* an altered human IgG1 CH2 domain) can further comprise one or more (*e.g*., two, three, four, or five) additional substitutions at positions 234-238. For example, an immunoglobulin CH2 region can comprise a substitution at positions 234 and 297, positions 234, 235, and 297, positions 234, 236 and 297, positions 234-236 and 297, positions 234, 235, 237 and 297, positions 234, 236, 238 and 297, positions 234, 235, 237, 238 and 297, positions 236-238 and 297, or any combination of two, three, four, or five amino acids at positions 234-238 in addition to position 297. In addition or alternatively, an altered CH2 region may comprise one or more (*e.g*., two, three, four or five) amino acid deletions at positions 234-238, such as at position 236 or position 237. The additional mutation(s) decreases or eliminates the antibody-dependent cell-mediated cytotoxicity (ADCC) activity or Fc receptor-binding capability of a polypeptide heterodimer that comprises the altered CH2 domain. In certain embodiments, the amino acid residues at one or more of positions 234-238 have been replaced with one or more alanine residues. In further embodiments, only one of the amino acid residues at positions 234-238 has been deleted while one or more of the remaining amino acids at positions 234-238 can be substituted with another amino acid (*e.g.*, alanine or serine).

In certain embodiments, in addition to one or more *(e.g.,* 2, 3, 4, or 5) amino acid substitutions at positions 234-238, a mutated CH2 region *(e.g.,* an altered human IgG1 CH2 domain) in a fusion protein of the present disclosure may contain one or more *(e.g.,* 2, 3, 4, 5, or 6) additional amino acid substitutions *(e.g.,* substituted with alanine) at one or more positions involved in complement fixation (*e.g.*, at positions 1253, H310, E318, K320, K322, or P331). Examples of mutated immunoglobulin CH2 regions include human IgG1, IgG2, IgG4 and mouse IgG2a CH2 regions with alanine substitutions at positions 234, 235, 237 (if present), 318, 320 and 322. An exemplary mutated immunoglobulin CH2 region is mouse IGHG2c CH2 region with alanine substitutions at L234, L235, G237, E318, K320, and K322 (SEQ ID NO:314).

In still further embodiments, in addition to the amino acid substitution at position 297 and the additional deletion(s) or substitution(s) at positions 234-238, an altered CH2 region *(e.g.,* an altered human IgG1 CH2 domain) can further comprise one or more (*e.g.*, two, three, four, five, or six) additional substitutions at positions 253, 310, 318, 320, 322, and 331. For example, an immunoglobulin CH2 region can comprise a (1) substitution at position 297, (2) one or more substitutions or deletions or a combination thereof at positions 234-238, and one or more *(e.g.,* 2, 3, 4, 5, or 6) amino acid substitutions at positions 1253, H310, E318, K320, K322, and P331, such as one, two, three substitutions at positions E318, K320 and K322. The amino acids at the above-noted positions may be substituted by alanine or serine.

In certain embodiments, an immunoglobulin CH2 region polypeptide comprises: (i) an amino acid substitution at the asparagines of position 297 and one amino acid substitution at position 234, 235, 236 or 237; (ii) an amino acid substitution at the asparagine of position 297 and amino acid substitutions at two of positions 234-237; (iii) an amino acid substitution at the asparagine of position 297 and amino acid substitutions at three of positions 234-237; (iv) an amino acid substitution at the asparagine of position 297, amino acid substitutions at positions 234, 235 and 237, and an amino acid deletion at position 236; (v) amino acid substitutions at three of positions 234-237 and amino acid substitutions at positions 318, 320 and 322; or (vi) amino acid substitutions at three of positions 234-237, an amino acid deletion at position 236, and amino acid substitutions at positions 318, 320 and 322.

Exemplary altered immunoglobulin CH2 regions with amino acid substitutions at the asparagine of position 297 include: human IgG1 CH2 region with alanine substitutions at L234, L235, G237 and N297 and a deletion at G236 (SEQ ID NO:325), human IgG2 CH2 region with alanine substitutions at V234, G236, and N297 (SEQ ID NO:326), human IgG4 CH2 region with alanine substitutions at F234, L235, G237 and N297 and a deletion of G236 (SEQ ID NO:322), human IgG4 CH2 region with alanine substitutions at F234 and N297 (SEQ ID NO:343), human IgG4 CH2 region with alanine substitutions at L235 and N297 (SEQ ID NO:344), human IgG4 CH2 region with alanine substitutions at G236 and N297 (SEQ ID NO:345), and human IgG4 CH2 region with alanine substitutions at G237 and N297 (SEQ ID NO:346).

In certain embodiments, in addition to the amino acid substitutions described above, an altered CH2 region *(e.g.,* an altered human IgG1 CH2 domain) may contain one or more additional amino acid substitutions at one or more positions other than the above-noted positions. Such amino acid substitutions may be conservative or non-conservative amino acid substitutions. For example, in certain embodiments, P233 may be changed to E233 in an altered IgG2 CH2 region *(see, e.g.,* SEQ ID NO:326). In addition or alternatively, in certain embodiments, the altered CH2 region may contain one or more amino acid insertions, deletions, or both. The insertion(s), deletion(s) or substitution(s) may anywhere in an immunoglobulin CH2 region, such as at the N- or C-terminus of a wild type immunoglobulin CH2 region resulting from linking the CH2 region with another region *(e.g.,* a binding domain or an immunoglobulin heterodimerization domain) via a hinge.

In certain embodiments, an altered CH2 region in a polypeptide heterodimer of the present disclosure comprises or is a sequence that is at least 90%, at least 91%, at least 92%, at least 93%, at least 94%, at least 95%, at least 96%, at least 97%, at least 98%, at least 99% identical to a wild type immunoglobulin CH2 region, such as the CH2 region of wild type human IgG1, IgG2, or IgG4, or mouse IgG2a (*e.g.,* IGHG2c).

An altered immunoglobulin CH2 region in a polypeptide heterodimer of the present disclosure may be derived from a CH2 region of various immunoglobulin isotypes, such as IgG1, IgG2, IgG3, IgG4, IgA1, IgA2, and IgD, from various species (including human, mouse, rat, and other mammals). In certain embodiments, an altered immunoglobulin CH2 region in a fusion protein of the present disclosure may be derived from a CH2 region of human IgG1, IgG2 or IgG4, or mouse IgG2a (*e.g.,* IGHG2c), whose sequences are set forth in SEQ ID NOS:115, 199, 201 and 320.

In certain embodiments, an altered CH2 domain is a human IgG1 CH2 domain with alanine substitutions at positions 235, 318, 320, and 322 (*i.e.,* a human IgG1 CH2 domain with L235A, E318A, K320A and K322A substitutions) (SEQ ID NO:595), and optionally an N297 mutation (*e.g.*, to alanine). In certain other embodiments, an altered CH2 domain is a human IgG1 CH2 domain with alanine substitutions at positions 234, 235, 237, 318, 320 and 322 (*i.e.,* a human IgG1 CH2 domain with L234A, L235A, G237A, E318A, K320A and K322A substitutions) (SEQ ID NO:596), and optionally an N297 mutation (*e.g.*, to alanine).

In certain embodiments, an altered CH2 domain is an altered human IgG1 CH2 domain with mutations known in the art that enhance immunological activities such as ADCC, ADCP, CDC, complement fixation, Fc receptor binding, or any combination thereof.

The CH3 domain that may form an Fc region portion of a single chain polypeptide of a heterodimer of the present disclosure may be a wild type immunoglobulin CH3 domain or an altered immunoglobulin CH3 domain thereof from certain immunoglobulin classes or subclasses (*e.g.,* IgG1, IgG2, IgG3, IgG4, IgA1, IgA2, IgD, IgE, IgM) of various species (including human, mouse, rat, and other mammals). In certain embodiments, a CH3 domain is a wild type human immunoglobulin CH3 domain, such as wild type CH3 domains of human IgG1, IgG2, IgG3, IgG4, IgA1, IgA2, IgD, IgE, or IgM as set forth in SEQ ID NOS:116, 208-210, 204-207, and 212, respectively. In certain embodiments, the CH3 domain is a wild type human IgG1 CH3 domain as set forth in SEQ ID NO:116. In certain embodiments, a CH3 domain is an altered human immunoglobulin CH3 domain, such as an altered CH3 domain based on or derived from a wild-type CH3 domain of human IgG1, IgG2, IgG3, IgG4, IgA1, IgA2, IgD, IgE, or IgM antibodies. For example, an altered CH3 domain may be a human IgG1 CH3 domain with one or two mutations at positions H433 and N434 (positions are numbered according to EU numbering). The mutations in such positions may be involved in complement fixation. In certain other embodiments, an altered CH3 domain may be a human IgG1 CH3 domain but with one or two amino acid substitutions at position F405 or Y407. The amino acids at such positions are involved in interacting with another CH3 domain. In certain embodiments, an altered CH3 domain may be an altered human IgG1 CH3 domain with its last lysine deleted. The sequence of this altered CH3 domain is set forth in SEQ ID NO:761.

In certain embodiments, a polypeptide heterodimer comprises a CH3 pair that comprises so called "knobs-into-holes" mutations (*see,* Marvin and Zhu, Acta Pharmacologica Sinica 26:649-58, 2005; Ridgway et al., Protein Engineering 9:617-21, 1966). More specifically, mutations may be introduced into each of the two CH3 domains so that the steric complementarity required for CH3/CH3 association obligates these two CH3 domains to pair with each other. For example, a CH3 domain in one single chain polypeptide of a polypeptide heterodimer may contain a T366W mutation (a "knob" mutation, which substitutes a small amino acid with a larger one), and a CH3 domain in the other single chain polypeptide of the polypeptide heterodimer may contain a Y407A mutation (a "hole" mutation, which substitutes a large amino acid with a smaller one). Other exemplary knobs-into-holes mutations include (1) a T366Y mutation in one CH3 domain and a Y407T in the other CH3 domain, and (2) a T366W mutation in one CH3 domain and T366S, L368A and Y407V mutations in the other CH3 domain.

The CH4 domain that may form an Fc region portion of a single chain polypeptide of a heterodimer of the present disclosure may be a wild type immunoglobulin CH4 domain or an altered immunoglobulin CH4 domain thereof from IgE or IgM molecules. In certain embodiments, the CH4 domain is a wild type human immunoglobulin CH4 domain, such as wild type CH4 domains of human IgE and IgM molecules as set forth in SEQ ID NOS:213 and 214, respectively. In certain embodiments, a CH4 domain is an altered human immunoglobulin CH4 domain, such as an altered CH4 domain based on or derived from a CH4 domain of human IgE or IgM molecules, which have mutations that increase or decrease an immunological activity known to be associated with an IgE or IgM Fc region.

In certain embodiments, an Fc region constant domain portion in heterodimers of the present disclosure comprises a combination of CH2, CH3 or CH4 domains (*i.e.,* more than one constant sub-domain selected from CH2, CH3 and CH4). For example, the Fc region portion may comprise CH2 and CH3 domains or CH3 and CH4 domains. In certain other embodiments, the Fc region portion may comprise two CH3 domains and no CH2 or CH4 domains (*i.e.,* only two or more CH3). The multiple constant sub-domains that form an Fc region portion may be based on or derived from the same immunoglobulin molecule, or the same class or subclass immunoglobulin molecules. In certain embodiments, the Fc region portion is an IgG CH2CH3 (*e.g.,* IgG1 CH2CH3, IgG2 CH2CH3, and IgG4 CH2CH3) and may be a human (e.g., human IgG1, IgG2, and IgG4) CH2CH3. For example, in certain embodiments, the Fc region portion comprises (1) wild type human IgG1 CH2 and CH3 domains, (2) human IgG1 CH2 with N297A substitution (*i.e.,* CH2(N297A)) and wild type human IgG1 CH3, or (3) human IgG1 CH2(N297A) and an altered human IgG1 CH3 with the last lysine deleted.

Alternatively, the multiple constant sub-domains may be based on or derived from different immunoglobulin molecules, or different classes or subclasses immunoglobulin molecules. For example, in certain embodiments, an Fc region portion comprises both human IgM CH3 domain and human IgG1 CH3 domain. The multiple constant sub-domains that form an Fc region portion may be directly linked together or may be linked to each other via one or more (e.g., about 2-10) amino acids.

Exemplary Fc region portions are set forth in SEQ ID NOS:305-309, 321, 323, 341, 342, and 762.

In certain embodiments, the Fc region portions of both single chain polypeptides of a polypeptide heterodimer are identical to each other. In certain other embodiments, the Fc region portion of one single chain polypeptide of a polypeptide heterodimer is different from the Fc region portion of the other single chain polypeptide of the heterodimer. For example, one Fc region portion may contain a CH3 domain with a "knob" mutation, whereas the other Fc region portion may contain a CH3 domain with a "hole" mutation.

### Hinge

A hinge region contained in a single chain polypeptide of a polypeptide heterodimer according to the present disclosure may be located (a) immediately amino terminal to an Fc region portion (*e.g.,* depending on the isotype, amino terminal to a CH2 domain wherein the Fc region portion is a CH2CH3, or amino terminal to a CH3 domain wherein the Fc region portion is a CH3CH4), (b) interposed between and connecting a binding domain (*e.g.,* scFv) and an immunoglobulin heterodimerization domain, (c) interposed between and connecting an immunoglobulin heterodimerization domain and an Fc region portion (*e.g.,* wherein the Fc region portion is a CH2CH3 or a CH3CH4, depending on the isotype or isotypes), (d) interposed between and connecting an Fc region portion and a binding domain, (e) at the amino terminus of the single chain polypeptide, or (f) at the carboxyl terminus of the single chain polypeptide. The single chain polypeptide comprising a hinge region as described herein will be capable of associating with a different single chain fusion polypeptide to form a polypeptide heterodimer provided herein, and the polypeptide heterodimer formed will contain a binding domain that retains its target specificity or its specific target binding affinity.

In certain embodiments, a hinge present in a single chain polypeptide that forms a polypeptide heterodimer with another single chain polypeptide may be an immunoglobulin hinge region, such as a wild type immunoglobulin hinge region or an altered immunoglobulin hinge region thereof.

In certain embodiments, a hinge is a wild type human immunoglobulin hinge region (*e.g.,* human immunoglobulin hinge regions as set forth in SEQ ID NOS:215-221). In certain other embodiments, one or more amino acid residues may be added at the amino- or carboxy- terminus of a wild type immunoglobulin hinge region as part of a fusion protein construct design. For example, additional junction amino acid residues at the hinge amino-terminus can be "RT," "RSS," "TG," or "T", or at the hinge carboxy-terminus can be "SG", or a hinge deletion can be combined with an addition, such as ΔP with "SG" added at the carboxyl terminus.

In certain embodiments, a hinge is an altered immunoglobulin hinge in which one or more cysteine residues in a wild type immunoglobulin hinge region is substituted with one or more other amino acid residues (*e.g.,* serine or alanine). For example, a hinge may be an altered immunoglobulin hinge based on or derived from a wild type human IgG1 hinge as set forth in SEQ ID NO:667, which from amino terminus to carboxyl terminus comprises the upper hinge region (EPKSCDKTHT, SEQ ID NO:227) and the core hinge region (CPPCP, SEQ ID NO:228). Exemplary altered immunoglobulin hinges include an immunoglobulin human IgG1 hinge region having one, two or three cysteine residues found in a wild type human IgG1 hinge substituted by one, two or three different amino acid residues (*e.g*., serine or alanine). An altered immunoglobulin hinge may additionally have a proline substituted with another amino acid *(e.g.,* serine or alanine). For example, the above-described altered human IgG1 hinge may additionally have a proline located carboxyl terminal to the three cysteines of wild type human IgG1 hinge region substituted by another amino acid residue (*e.g.,* serine, alanine). In one embodiment, the prolines of the core hinge region are not substituted. Exemplary altered immunoglobulin hinges are set forth in SEQ ID NOS: 229-240, 255, 664-677, and 748-759. An example of an altered IgG1 hinge is an altered human IgG1 hinge in which the first cysteine is substituted by serine. The sequence of this altered IgG1 hinge is set forth in SEQ ID NO:664, and is referred to as the " human IgG1 SCC-P hinge" or "SCC-P hinge." In certain embodiments, one or more amino acid residues (*e.g.,* "RT," "RSS," or "T") may be added at the amino-or carboxy-terminus of a mutated immunoglobulin hinge region as part of a fusion protein construct design.

In certain embodiments, a hinge polypeptide comprises or is a sequence that is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identical to a wild type immunoglobulin hinge region, such as a wild type human IgG1 hinge, a wild type human IgG2 hinge, or a wild type human IgG4 hinge.

In further embodiments, a hinge present in a single chain polypeptide that forms a polypeptide heterodimer with another single chain polypeptide may be a hinge that is not based on or derived from an immunoglobulin hinge (*i.e.,* not a wild type immunoglobulin hinge or an altered immunoglobulin hinge). These types of non-immunoglobulin based hinges may be used on or near the carboxyl end *(e.g.,* located carboxyl terminal to Fc region portions) of the single chain polypeptides that form the polypeptide heterodimers. Examples for such hinges include peptides of about five to about 150 amino acids of the interdomain or stalk region of type II C-lectins or CD molecules, for instance, peptides of about eight to 25 amino acids and peptides of about seven to 18 amino acids, and derivatives thereof.

The "interdomain or stalk region" of a type II C-lectin or CD molecule refers to the portion of the extracellular domain of the type II C-lectin or CD molecule that is located between the C-type lectin-like domain (CTLD; *e.g.,* similar to CTLD of natural killer cell receptors) and the transmembrane domain. For example, in the human CD94 molecule (GenBank Accession No. AAC50291.1, PRI November 30, 1995), the extracellular domain corresponds to amino acid residues 34-179, whereas the CTLD corresponds to amino acid residues 61-176. Accordingly, the interdomain or stalk region of the human CD94 molecule includes amino acid residues 34-60, which is found between the membrane and the CTLD (*see* Boyington et al., Immunity 10:75, 1999; for descriptions of other stalk regions, *see also* Beavil et al., Proc. Nat'l. Acad. Sci. USA 89:753, 1992; and Figdor et al., Nature Rev. Immunol. 2:77, 2002). These type II C-lectin or CD molecules may also have from six to 10 junction amino acids between the stalk region and the transmembrane region or the CTLD. In another example, the 233 amino acid human NKG2A protein (GenBank Accession No. P26715.1, PRI June 15, 2010) has a transmembrane domain ranging from amino acids 71-93 and an extracellular domain ranging from amino acids 94-233. The CTLD is comprised of amino acids 119-231, and the stalk region comprises amino acids 99-116, which is flanked by junctions of five and two amino acids. Other type II C-lectin or CD molecules, as well as their extracellular ligand-bind domains, interdomain or stalk regions, and CTLDs are known in the art (*see, e.g.,* GenBank Accession Nos. NP_001993.2; AAH07037.1, PRI July 15, 2006; NP_001773.1, PRI June 20, 1010; AAL65234.1, PRI January 17, 2002, and CAA04925.1, PRI November 14, 2006, for the sequences of human CD23, CD69, CD72, NKG2A and NKG2D and their descriptions, respectively).

A "derivative" of an interdomain or stalk region, or fragment thereof, of a type II C-lectin or CD molecule includes an about an eight to about 150 amino acid sequence in which one, two, or three amino acids of the stalk region of a wild type type II C-lectin or CD molecule have a deletion, insertion, substitution, or any combination thereof, for instance, the one or more changes are substitutions or the one or more mutations include only one deletion. In further embodiments, a derivative of an interdomain or stalk region is more resistant to proteolytic cleavage as compared to the wild-type interdomain or stalk region sequence, such as those derived from about eight to about 20 amino acids of NKG2A, NKG2D, CD23, CD64, CD72, or CD94.

In certain embodiments, interdomain or stalk region hinges have seven to 18 amino acids and can form an α-helical coiled coil structure. In certain embodiments, interdomain or stalk region hinges contain 0, 1, 2, 3, or 4 cysteines. Exemplary interdomain or stalk region hinges are peptide fragments of the interdomain or stalk regions, such as ten to 150 amino acid fragments from the stalk regions of CD69, CD72, CD94, NKG2A and NKG2D, as set forth in SEQ ID NOS:241-244, 716 and 601. Additional exemplary stalk region or interdomain hinges include those as set forth in SEQ ID NOS:78, 734-737, 742-747, and 766-790.

Alternative hinges that can be used in single chain polypeptides of polypeptide heterodimers are from portions of cell surface receptors (interdomain regions) that connect immunoglobulin V-like or immunoglobulin C-like domains. Regions between Ig V-like domains where the cell surface receptor contains multiple Ig V-like domains in tandem and between Ig C-like domains where the cell surface receptor contains multiple tandem Ig C-like regions are also contemplated as hinges useful in single chain polypeptides of polypeptide heterodimers. In certain embodiments, hinge sequences comprised of cell surface receptor interdomain regions may further contain a naturally occurring or added motif, such as an IgG core hinge sequence that confers one or more disulfide bonds to stabilize the polypeptide heterodimer formation. Examples of hinges include interdomain regions between the Ig V-like and Ig C-like regions of CD2, CD4, CD22, CD33, CD48, CD58, CD66, CD80, CD86, CD150, CD166, and CD244.

In certain embodiments, hinge sequences have about 5 to 150 amino acids, 5 to 10 amino acids, 10 to 20 amino acids, 20 to 30 amino acids, 30 to 40 amino acids, 40 to 50 amino acids, 50 to 60 amino acids, 5 to 60 amino acids, 5 to 40 amino acids, 8 to 20 amino acids, or 10 to 15 amino acids. The hinge may be primarily flexible, but may also provide more rigid characteristics or may contain primarily α-helical structure with minimal β-sheet structure. The lengths or the sequences of the hinges may affect the binding affinities of the binding domains to which the hinges are directly or indirectly (via another region or domain, such as an immunoglobulin heterodimerization domain) connected as well as one or more activities of the Fc region portions to which the hinges are directly or indirectly connected (*see,* Examples 9 and 10).

In certain embodiments, hinge sequences are stable in plasma and serum and are resistant to proteolytic cleavage. The first lysine in the IgG1 upper hinge region may be mutated to minimize proteolytic cleavage, for instance, the lysine may be substituted with methionine, threonine, alanine or glycine, or is deleted (*see, e.g.,* SEQ ID NOS:379-434, which may include junction amino acids at the amino terminus such as RT).

In some embodiments, hinge sequences may contain a naturally occurring or added motif such as an immunoglobulin hinge core structure CPPCP (SEQ ID NO:228) that confers the capacity to form a disulfide bond or multiple disulfide bonds to stabilize the carboxy-terminus of a molecule. In other embodiments, hinge sequences may contain one or more glycosylation sites.

Exemplary hinges, including altered immunoglobulin hinges, are set forth in SEQ ID NOS:379-434, 618-749, and 763-791.

In certain embodiments, a single chain polypeptide of a polypeptide heterodimer according to the present disclosure comprises more than one hinge. For example, a single chain polypeptide having two binding domains, one of which at the amino terminus and the other at the carboxyl terminus, may have two hinges. One hinge may be directly or indirectly (*e.g.,* via an immunoglobulin heterodimerization domain) connected to the binding domain at or near the amino terminus, and the other hinge may be connected (*e.g.,* directly connected) to the other binding domain at or near the carboxyl terminus. In certain embodiments, even if a single chain polypeptide has only one binding domain, it may have more than one hinge, for example, at its amino or carboxyl terminus. Such a hinge may interact with a corresponding hinge in the other chain of the heterodimer, such as forming one or more interchain disulfide bonds, to facilitate or enhance the heterodimerization of the two chains. A hinge (H-I) of a SCP-I of a polypeptide heterodimer "corresponds to" a hinge (H-II) of a SCP-II of the heterodimer when H-I and H-II are located on the same end of the Fc region portion of their respective single chain polypeptide. For example, a polypeptide heterodimer may comprise the following two single chain polypeptides: A first chain polypeptide from amino to carboxyl terminus comprises a first binding domain, CH1, hinge, CH2, and CH3, and a second chain polypeptide from amino to carboxyl terminus comprises a CK, first hinge, CH2, CH3, second hinge, and a second binding domain. The hinge in the first chain would be regarded as "corresponding" to the first hinge of the second chain because both are amino terminal to the Fc region portions to which they are connected.

In certain embodiments where a single chain polypeptide of a polypeptide heterodimer comprises a binding domain at or near its carboxyl terminus, a hinge may be present to link the binding domain with another portion of the single chain polypeptide (*e.g.,* an Fc region portion or an immunoglobulin heterodimerization domain). In one embodiment, such a hinge is a non-immunoglobulin hinge (*i.e.,* a hinge not based on or derived from a wild type immunoglobulin hinge) and may be a stalk region of a type II C-lectin or CD molecule, an interdomain region that connect IgV-like or IgC-like domains of a cell surface receptor, or a derivative or functional variant thereof. Exemplary carboxyl terminal hinges, sometimes referred to as "back-end" hinges, includes those set forth in SEQ ID NOS:78, 734-737, 742-747, and 766-790.

In certain embodiments, a hinge of one single chain polypeptide of a polypeptide heterodimer is identical to a corresponding hinge of the other single chain polypeptide of the heterodimer. In certain other embodiments, a hinge of one chain is different from that of the other chain (in their length or sequence). The different hinges in the different chains allow different manipulation of the binding affinities of the binding domains to which the hinges are connected, so that the heterodimer is able to preferentially bind to the target of one binding domain over the target of the other binding domain. For example, in certain embodiments, a polypeptide heterodimer has a CD3 or TCR binding domain in one chain and a tumor antigen binding domain in another chain. Having two different hinges in the two chains may allow the heterodimer to bind to the tumor antigen first, and then to a CD3 or TCR molecule second. Thus, the heterodimer may recruit CD3⁺ T cells to tumor cells carrying the tumor antigen, which in turn may damage or destroy the tumor cells.

### Other components or modifications

In certain embodiments, a single chain polypeptide that forms a heterodimer with another single chain polypeptide may contain one or more additional domains or regions. Such additional regions may be a leader sequence (also referred to as "signal peptide") at the amino-terminus for secretion of an expressed single chain polypeptide. Exemplary leader peptides of this disclosure include natural leader sequences or others, such as those as set forth in SEQ ID NOS:110 and 111.

Additional regions may also be sequences at the carboxy-terminus for identifying or purifying single chain polypeptides (*e.g.,* epitope tags for detection or purification, such as a histidine tag, biotin, a FLAG® epitope, or any combination thereof).

Further optional regions may be additional amino acid residues (referred to as "junction amino acids" or "junction amino acid residues") having a length of 1 to about 10 amino acids (*e.g.,* about 2 to 5 amino acids), which may be resulted from use of specific expression systems or construct design for the single chain polypeptides of the present disclosure. Such additional amino acid residues (for instance, one, two, three, four or five additional amino acids) may be present at the amino or carboxyl terminus or between various regions or domains of a single chain polypeptide, such as between a binding domain and an immunoglobulin heterodimerization domain, between an immunoglobulin heterodimerization domain and a hinge, between a hinge and an Fc region portion, between domains of an Fc region portion (*e.g.,* between CH2 and CH3 domains or between two CH3 domains), between a binding domain and a hinge, between an Fc region portion and an immunoglobulin heterodimerization domain, or between a variable domain and a linker. Exemplary junction amino acids amino-terminal to a hinge include RDQ (SEQ ID NO:598), RT, SS, SASS (SEQ ID NO:599) and SSS (SEQ ID NO:600). Exemplary junction amino acids carboxy-terminal to a hinge include amino acids SG. Additional exemplary junction amino acids include SR.

In certain embodiments, junction amino acids are present between an Fc region portion that comprises CH2 and CH3 domains and an immunoglobulin heterodimerization domain (CH1 or CL). These junction amino acids are also referred to as a "linker between CH3 and CH1 or CL" if they are present between the C-terminus of CH3 and the N-terminus of CH1 or CL. Such a linker may be about 2-12 amino acids in length. In certain embodiments, the Fc region portion comprises human IgG1 CH2 and CH3 domains in which the C-terminal lysine residue of human IgG1 CH3 is deleted. Exemplary linkers between CH3 and CH1 include those set forth in SEQ ID NO:847-849. Exemplary linkers between CH3 and Ck include those set forth in SEQ ID NOS:850-852 (in which the carboxyl terminal arginine in the linkers may alternatively be regarded as the first arginine of Ck). In certain embodiments, the presence of such linkers or linker pairs *(e.g.,* SEQ ID NO:847 as a CH3-CH1 linker in one single chain polypeptide of a heterodimer and SEQ ID NO:850 as a CH3-Ck linker in the other single chain polypeptide of the heterodimer; SEQ ID NO:848 as a CH3-CH1 linker and SEQ ID NO:851 as a CH3-Ck linker; and SEQ ID NO:849 as a CH3-CH1 linker and SEQ ID NO:852 as a CH3-Ck linker) improves the production of heterodimer compared the presence of a reference linker as set forth in SEQ ID NO:846 (in which the last lysine of CH3 is included as part of the linker) in both single chain polypeptides of a heterodimer.

In certain embodiments, an immunoglobulin Fc region *(e.g.,* CH2, CH3, and/or CH4 regions) of a polypeptide heterodimer of the present disclosure may have an altered glycosylation pattern relative to an immunoglobulin reference sequence. For example, any of a variety of genetic techniques may be employed to alter one or more particular amino acid residues that form a glycosylation site (*see* Co *et al.* (1993) Mol. Immunol. 30:1361; Jacquemon *et al.* (2006) J. Thromb. Haemost. 4:1047; Schuster *et al.* (2005) Cancer Res. 65:7934; Warnock *et al.* (2005) Biotechnol. Bioeng. 92:831), such as N297 of the CH2 domain (EU numbering). Alternatively, the host cells producing polypeptide heterodimers of this disclosure may be engineered to produce an altered glycosylation pattern. One method known in the art, for example, provides altered glycosylation in the form of bisected, non-fucosylated variants that increase ADCC. The variants result from expression in a host cell containing an oligosaccharide-modifying enzyme. Alternatively, the POTELLIGENT® technology of BioWa/Kyowa Hakko is contemplated to reduce the fucose content of glycosylated molecules according to this disclosure. In one known method, a CHO host cell for recombinant immunoglobulin production is provided that modifies the glycosylation pattern of the immunoglobulin Fc region, through production of GDP-fucose.

Alternatively, chemical techniques are used to alter the glycosylation pattern of polypeptide heterodimers of this disclosure. For example, a variety of glycosidase and/or mannosidase inhibitors provide one or more of desired effects of increasing ADCC activity, increasing Fc receptor binding, and altering glycosylation pattern. In certain embodiments, cells expressing polypeptide heterodimers of the instant disclosure are grown in a culture medium comprising a carbohydrate modifier at a concentration that increases the ADCC of immunoglycoprotein molecules produced by said host cell, wherein said carbohydrate modifier is at a concentration of less than 800 µM. In one embodiment, the cells expressing these polypeptide heterodimers are grown in a culture medium comprising castanospermine or kifunensine, for instance, castanospermine at a concentration of 100-800 µM, such as 100 µM, 200 µM, 300 µM, 400 µM, 500 µM, 600 µM, 700 µM, or 800 µM. Methods for altering glycosylation with a carbohydrate modifier such as castanospermine are provided in U.S. Patent No. 7,846,434 or PCT Publication No. WO 2008/052030.

### Structural Arrangements and Exemplary Heterodimers

To form a polypeptide heterodimer according to the present invention, two single chain polypeptides are designed so that the immunoglobulin heterodimerization domain of the first single chain polypeptide is properly aligned and interacts with the immunoglobulin heterodimerization domain of the second single chain polypeptide. In certain embodiments, the heterodimer may comprise a second immunoglobulin heterodimerization domain pair to facilitate or enhance the heterodimerization of the two chains. In certain embodiments, in addition to the interaction between the two immunoglobulin heterodimerization domains, an Fc region portion *(e.g.,* a CH3 domain) in the first chain may interact with an Fc region in the second chain to enhance heterodimerization (*e.g.,* via interaction between two wild type CH3 domains or between a CH3 domain pair with "knobs-into-holes" mutations). Moreover, in certain embodiments, the hinge in the first chain (*e.g.,* an altered human IgG1 hinge with two cysteine residues as set forth in SEQ ID NO:664) may interact with the hinge in the second chain (*e.g.,* the same altered human IgG1 hinge as set forth in SEQ ID NO:664) to form, for example, disulfide bonds, which may further strengthen the interaction between the first and second single chain polypeptides to form a polypeptide heterodimer of the present disclosure. Furthermore, in certain embodiments, the first and second chain may comprise a second hinge pair *(e.g.,* at the carboxyl termini of the two chains) to further enhance the interactions between the two chains.

In certain embodiments, a polypeptide heterodimer of the present disclosure comprises two binding domains (BD1 and BD2), wherein the binding domains bind to two different target molecules. In certain embodiments, the two binding domains (BD1 and BD2) are both on the SCP-I with the immunoglobulin heterodimerization domain (HD-I) and the Fc region portion (FRP-1) disposed between BD1 and BD2. In certain other embodiments, the first binding domain (BD1) is on the SCP-I and the second binding domain (BD2) is on the SCP-II. In certain embodiments, both BD1 and BD2 are amino terminal to the Fc region portion of the SCP-I and SCP-II, respectively. In certain other embodiments, BD1 is amino terminal to the Fc region portion of the SCP-I and BD2 is carboxyl terminal to the Fc region portion of the SCP-II. In certain other embodiments, BD1 is carboxyl terminal to the Fc region portion of the SCP-I and BD2 is amino terminal to the Fc region portion of the SCP-II. In certain other embodiments, both BD1 and BD2 are carboxyl terminal to the Fc region portion of the SCP-I and SCP-II, respectively.

In certain embodiments, a polypeptide heterodimer comprises three binding domains (BD1, BD2 and BD3), wherein the binding domains bind to two or three different target molecules. For example, BD1, BD2, and BD3 each bind a different target, or BD 1 and BD2 bind a first target while BD3 binds a second target, or BD1 and BD3 bind a first target while BD2 binds a second target, or BD2 and BD3 bind a first target while BD1 binds a second target. In certain embodiments, the immunoglobulin heterodimerization domain and the Fc region portion are disposed between BD1 and BD2 on SCP-I, and BD3 is amino terminal to the Fc region portion of the SCP-II. In further embodiments, the immunoglobulin heterodimerization domain and the Fc region portion are disposed between BD1 and BD2 on SCP-II, and BD3 is carboxyl terminal to the Fc region portion of the SCP-I.

In certain embodiments, a polypeptide heterodimer comprises four binding domains (BD1, BD2, BD3 and BD4), wherein the binding domains bind to two to four different target molecules. In certain embodiments, the immunoglobulin heterodimerization domain and Fc region portion of the SCP-I are disposed between BD1 and BD2, and the immunoglobulin heterodimerization domain and Fc region portion of SCP-II are disposed between BD3 and BD4.

In certain embodiments, a polypeptide heterodimer comprises five binding domains (BD1, BD2, BD3, BD4 and BD5), wherein the binding domains bind to two to four different target molecules. In certain embodiments, SCP-I comprises three binding domains (BD1, BD2 and BD3), and SCP-II comprises two binding domains (BD4 and BD5). In further embodiments, BD1 and BD2 may be, for example, linked in tandem to each other (either directly or via a peptide linker of about two to eight amino acids) at the amino (or carboxyl) terminus of SCP-I with BD3 located at the carboxyl (or amino) terminus of SCP-I or SCP-II, wherein BD4 and BD5 are on SCP-II if BD3 is on SCP-I, or BD4 or BD5 are on SCP-I if BD3 is on SCP-II.

In still further embodiments, the polypeptide heterodimer comprises six binding domains (BD1-BD6). In certain embodiments, SCP-I and SCP-II each comprise three binding domains (*e.g.,* BD1-BD3 on SCP-I, and BD4-BD6 on SCP-II). In such embodiments, for example, BD1 and BD2 may be linked in tandem and located at the SCP-I amino (or carboxyl) terminus, and BD3 may be located at the SCP-I carboxyl (or amino) terminus. Similarly, BD4 and BD5 may be linked in tandem and located at the SCP-II amino (or carboxyl) terminus, and BD6 may be located at the SCP-II carboxyl (or amino) terminus. In certain other embodiments, the first single chain polypeptide (SCP-I) comprises four binding domains (BD1-BD4) and the second single chain polypeptide (SCP-II) comprises two binding domains (BD5 and BD6). In such embodiments, BD1 and BD2 may be linked in tandem and located at or near amino terminus of SCP-I, BD3 and BD4 may be linked in tandem and located at or near carboxyl terminus of SCP-I, and BD5 and BD6 may be located at or near the amino and carboxyl termini of SCP-II, respectively.

In certain embodiments, the polypeptide heterodimer comprises seven binding domains (BD1-BD7). In such embodiments, SCP-I may comprise four binding domains (BD1-BD4), and SCP-II may comprise the other three binding domains (BD5-BD7). For example, BD 1 and BD2 may be linked in tandem and located at or near the amino terminus of SCP-I, and BD3 and BD4 may be linked in tandem and located at or near the carboxyl terminus of SCP-II. BD5 and BD6 may be linked in tandem and located at or near the amino (or carboxyl) terminus of SCP-II, and BD7 may be located at or near the carboxyl (or amino) terminus of SCP-II.

In certain embodiments, the polypeptide heterodimer comprises eight binding domains (BD1-BD8). In such embodiments, the first and second single chain polypeptides may each comprise four binding domains. In each chain, two binding domains may be located at or near the amino terminus, and the other two binding domains located at or near the carboxyl terminus.

To simplify the description of how various components can be arranged to make first and second single chain polypeptides that form polypeptide heterodimers of the present disclosure, arrangements in exemplary embodiments (1) to (50) are provided below in which only two binding domains are included in each heterodimer.
In embodiment (1), a polypeptide heterodimer comprises the following two single chain polypeptides: from amino terminus to carboxyl terminus, a first single chain polypeptide comprising a first binding domain, a CH1 region, a hinge, and an Fc region portion; and a second single chain polypeptide comprising a second binding domain, a CL region (*e.g.,* Cκ, Cλ), a hinge, and an Fc region portion.
In embodiment (2), a polypeptide heterodimer comprises the following two single chain polypeptides: from amino terminus to carboxyl terminus, a first single chain polypeptide comprising a first binding domain, a hinge, an Fc region portion, and a CH1 region; and a second single chain polypeptide comprising a second binding domain, a hinge, an Fc region portion, and a CL region.
In embodiment (3), a polypeptide heterodimer comprises the following two single chain polypeptides: from amino terminus to carboxyl terminus, a first single chain polypeptide comprising a first binding domain, a CH1 region, a hinge, an Fc region portion, and a second CH1 region; and a second single chain polypeptide comprising a second binding domain, a CL region, a hinge, an Fc region portion, and a second CL region.
In embodiment (4), a polypeptide heterodimer comprises the following two single chain polypeptides: from amino terminus to carboxyl terminus, a first single chain polypeptide comprising a first binding domain, a CH1 region, a second CH1 region, a hinge, and an Fc region portion; and a second single chain polypeptide comprising a second binding domain, a CL region, a second CL region, a hinge and an Fc region portion.
In embodiment (5), a polypeptide heterodimer comprises the following two single chain polypeptides: from amino terminus to carboxyl terminus, a first single chain polypeptide comprising a first binding domain, a hinge, an Fc region portion, a CH1 region, and a second CH1 region; and a second single chain polypeptide comprising a second binding domain, a hinge, an Fc region portion, a CL region and a second CL region.
In embodiment (6), a polypeptide heterodimer comprises the following two single chain polypeptides: from amino terminus to carboxyl terminus, a first single chain polypeptide comprising a CH1 region, a hinge, an Fc region portion, a second hinge, and a first binding domain; and a second single chain polypeptide comprising a CL region, a hinge, an Fc region portion, a second hinge, and a second binding domain.
In embodiment (7), a polypeptide heterodimer is formed from the following two single chain polypeptides: from amino terminus to carboxyl terminus, a first single chain polypeptide comprising a hinge, an Fc region portion, a CH1 region, a second hinge, and a first binding domain; and a second single chain polypeptide comprising a hinge, an Fc region portion, a CL region, a second hinge, and a second binding domain.
In embodiment (8), a polypeptide heterodimer comprises the following two single chain polypeptides: from amino terminus to carboxyl terminus, a first single chain polypeptide comprising a CH1 region, a hinge, an Fc region portion, a second CH1 region, a second hinge, and a first binding domain; and a second single chain polypeptide comprising a CL region, a hinge, an Fc region portion, a second CL region, a second hinge, and a second binding domain.
In embodiment (9), a polypeptide heterodimer comprises the following two single chain polypeptides: from amino terminus to carboxyl terminus, a first single chain polypeptide comprising a CH1 region, a second CH1 region, a hinge, an Fc region portion, a second hinge, and a first binding domain; and a second single chain polypeptide comprising a CL region, a second CL region, a hinge, an Fc region portion, a second hinge, and a second binding domain.
In embodiment (10), a polypeptide heterodimer comprises the following two single chain polypeptides: from amino terminus to carboxyl terminus, a first single chain polypeptide comprising a hinge, an Fc region portion, a CH1 region, a second CH1 region, a second hinge, and a first binding domain; and a second single chain polypeptide comprising a hinge, an Fc region portion, a CL region, a second CL region, a second hinge, and a second binding domain.
In embodiment (11), a polypeptide heterodimer comprises the following two single chain polypeptides: from amino terminus to carboxyl terminus, a first single chain polypeptide comprising a first binding domain, a CL region, a hinge, and an Fc region portion; and a second single chain polypeptide comprising a second binding domain, a CH1 region, a hinge, and an Fc region portion.
In embodiment (12), a polypeptide heterodimer comprises the following two single chain polypeptides: from amino terminus to carboxyl terminus, a first single chain polypeptide comprising a first binding domain, a hinge, an Fc region portion, and a CL region; and a second single chain polypeptide comprising a second binding domain, a hinge, an Fc region portion, and a CH1 region.
In embodiment (13), a polypeptide heterodimer comprises the following two single chain polypeptides: from amino terminus to carboxyl terminus, a first single chain polypeptide comprising a first binding domain, a CL region, a hinge, an Fc region portion, and a second CL region; and a second single chain polypeptide comprising a second binding domain, a CH1 region, a hinge, an Fc region portion, and a second CH1 region.
In embodiment (14), a polypeptide heterodimer comprises the following two single chain polypeptides: from amino terminus to carboxyl terminus, a first single chain polypeptide comprising a first binding domain, a CL region, a second CL region, a hinge, and an Fc region portion; and a second single chain polypeptide comprising a second binding domain, a CH1 region, a second CH1 region, a hinge, and an Fc region portion.
In embodiment (15), a polypeptide heterodimer comprises the following two single chain polypeptides: from amino terminus to carboxyl terminus, a first single chain polypeptide comprising a first binding domain, a hinge, an Fc region portion, a CL region, and a second CL region; and a second single chain polypeptide comprising a second binding domain, a hinge, an Fc region portion, a CH1 region, and a second CH1 region.
In embodiment (16), a polypeptide heterodimer comprises the following two single chain polypeptides: from amino terminus to carboxyl terminus, a first single chain polypeptide comprising a CL region, a hinge, an Fc region portion, a second hinge, and a first binding domain; and a second single chain polypeptide comprising a CH1 region, a hinge, an Fc region portion, a second hinge, and a second binding domain.
In embodiment (17), a polypeptide heterodimer comprises the following two single chain polypeptides: from amino terminus to carboxyl terminus, a first single chain polypeptide comprising a hinge, an Fc region portion, a CL region, a second hinge, and a first binding domain; and a second single chain polypeptide comprising a hinge, an Fc region portion, a CH1 region, a second hinge, and a second binding domain.
In embodiment (18), a polypeptide heterodimer comprises the following two single chain polypeptides: from amino terminus to carboxyl terminus, a first single chain polypeptide comprising a CL region, a hinge, an Fc region portion, a second CL region, a second hinge, and a first binding domain; and a second single chain polypeptide comprising a CH1 region, a hinge, an Fc region portion, a second CH1 region, a second hinge, and a second binding domain.
In embodiment (19), a polypeptide heterodimer comprises the following two single chain polypeptides: from amino terminus to carboxyl terminus, a first single chain polypeptide comprising a CL region, a second CL region, a hinge, an Fc region portion, a second hinge, and a first binding domain; and a second single chain polypeptide comprising a CH1 region, a second CH1 region, a hinge, an Fc region portion, a second hinge, and a second binding domain.
In embodiment (20), a polypeptide heterodimer comprises the following two single chain polypeptides: from amino terminus to carboxyl terminus, a first single chain polypeptide comprising a hinge, an Fc region portion, a CL region, a second CL region, a second hinge, and a first binding domain; and a second single chain polypeptide comprising a hinge, an Fc region portion, a CH1 region, a second CH1 region, a second hinge, and a second binding domain.
In embodiment (21), a polypeptide heterodimer comprises the following two single chain polypeptides: from amino terminus to carboxyl terminus, a first single chain polypeptide comprising a first binding domain, a CH1 region, a hinge, an Fc region portion, and a CL region; and a second single chain polypeptide comprising a second binding domain, a CL region, a hinge, an Fc region portion, and a CH1 region.
In embodiment (22), a polypeptide heterodimer comprises the following two single chain polypeptides: from amino terminus to carboxyl terminus, a first single chain polypeptide comprising a first binding domain, a CL region, a hinge, an Fc region portion, and a CH1 region; and a second single chain polypeptide comprising a second binding domain, a CH1 region, a hinge, an Fc region portion, and a CL region.
In embodiment (23), a polypeptide heterodimer comprises the following two single chain polypeptides: from amino terminus to carboxyl terminus, a first single chain polypeptide comprising a first binding domain, a CH1 region, a CL region, a hinge, and an Fc region portion; and a second single chain polypeptide comprising a second binding domain, a CL region, a CH1 region, a hinge and an Fc region portion.
In embodiment (24), a polypeptide heterodimer comprises the following two single chain polypeptides: from amino terminus to carboxyl terminus, a first single chain polypeptide comprising a first binding domain, a CL region, a CH1 region, a hinge, and an Fc region portion; and a second single chain polypeptide comprising a second binding domain, a CH1 region, a CL region, a hinge and an Fc region portion.
In embodiment (25), a polypeptide heterodimer comprises the following two single chain polypeptides: from amino terminus to carboxyl terminus, a first single chain polypeptide comprising a first binding domain, a hinge, an Fc region portion, a CH1 region and a CL region; and a second single chain polypeptide comprising a second binding domain, a hinge, an Fc region portion, a CL region and a CH1 region.
In embodiment (26), a polypeptide heterodimer comprises the following two single chain polypeptides: from amino terminus to carboxyl terminus, a first single chain polypeptide comprising a first binding domain, a hinge, an Fc region portion, a CL region and a CH1 region; and a second single chain polypeptide comprising a second binding domain, a hinge, an Fc region portion, a CH1 region and a CL region.
In embodiment (27), a polypeptide heterodimer comprises the following two single chain polypeptides: from amino terminus to carboxyl terminus, a first single chain polypeptide comprising a CH1 region, a hinge, an Fc region portion, a CL region, a second hinge, and a first binding domain; and a second single chain polypeptide comprising a CL region, a hinge, an Fc region portion, a CH1 region, a second hinge, and a second binding domain.
In embodiment (28), a polypeptide heterodimer comprises the following two single chain polypeptides: from amino terminus to carboxyl terminus, a first single chain polypeptide comprising a CL region, a hinge, an Fc region portion, a CH1 region, a second hinge, and a first binding domain; and a second single chain polypeptide comprising a CH1 region, a hinge, an Fc region portion, a CL region, a second hinge, and a second binding domain.
In embodiment (29), a polypeptide heterodimer comprises the following two single chain polypeptides: from amino terminus to carboxyl terminus, a first single chain polypeptide comprising a CH1 region, a CL region, a hinge, an Fc region portion, a second hinge, and a first binding domain; and a second single chain polypeptide comprising a CL region, a CH1 region, a hinge, an Fc region portion, a second hinge, and a second binding domain.
In embodiment (30), a polypeptide heterodimer comprises the following two single chain polypeptides: from amino terminus to carboxyl terminus, a first single chain polypeptide comprising a CL region, a CH1 region, a hinge, an Fc region portion, a second hinge, and a first binding domain; and a second single chain polypeptide comprising a CH1 region, a CL region, a hinge, an Fc region portion, a second hinge, and a second binding domain.
In embodiment (31), a polypeptide heterodimer comprises the following two single chain polypeptides: from amino terminus to carboxyl terminus, a first single chain polypeptide comprising a hinge, an Fc region portion, a CH1 region, a CL region, a second hinge, and a first binding domain; and a second single chain polypeptide comprising a hinge, an Fc region portion, a CL region, a CH1 region, a second hinge, and a second binding domain.
In embodiment (32), a polypeptide heterodimer comprises the following two single chain polypeptides: from amino terminus to carboxyl terminus, a first single chain polypeptide comprising a hinge, an Fc region portion, a CL region, a CH1 region, a second hinge, and a first binding domain; and a second single chain polypeptide comprising a hinge, an Fc region portion, a CH1 region, a CL region, a second hinge, and a second binding domain.
In embodiment (33), a polypeptide heterodimer comprises the following two single chain polypeptides: from amino to carboxyl terminus, a first single chain polypeptide comprising a first binding domain, a CH1 region, a hinge, an Fc region portion, a second hinge, and a second binding domain; and a second single chain polypeptide comprising a CL region, a hinge, and an Fc region.
In embodiment (34), a polypeptide heterodimer comprises the following two single chain polypeptides: from amino to carboxyl terminus, a first single chain polypeptide comprising a first binding domain, a CL region, a hinge, an Fc region portion, a second hinge, and a second binding domain; and a second single chain polypeptide comprising a CH1 region, a hinge and an Fc region portion.
In embodiment (35), a polypeptide heterodimer comprises the following two single chain polypeptides: from amino to carboxyl terminus, a first single chain polypeptide comprising a first binding domain, a CH1 region, a hinge and an Fc region portion; and a second single chain polypeptide comprising a CL region, a hinge, an Fc region portion, a second hinge and a second binding domain.
In embodiment (36), a polypeptide heterodimer comprises the following two single chain polypeptides: from amino to carboxyl terminus, a first single chain polypeptide comprising a CH1 region, a hinge, an Fc region portion, a second hinge and a first binding domain; and a second single chain polypeptide comprising a second binding domain, a CL region, a hinge and an Fc region portion.
In embodiment (37), a polypeptide heterodimer comprises the following two single chain polypeptides: from amino to carboxyl terminus, a first single chain polypeptide comprising a first binding domain, a CH1 region, a hinge, an Fc region portion, and a second hinge; and a second single chain polypeptide comprising a second binding domain, a CL region, a hinge, an Fc region portion, and a second hinge.
In embodiment (38), a polypeptide heterodimer comprises the following two single chain polypeptides: from amino to carboxyl terminus, a first single chain polypeptide comprising a first binding domain, a hinge, an Fc region portion, a CH1 region, a second hinge, and a second binding domain; and the second single chain polypeptide comprising a hinge, an Fc region portion, and a CL region.
In embodiment (39), a polypeptide heterodimer comprises the following two single chain polypeptides: from amino to carboxyl terminus, a first single chain polypeptide comprising a hinge, an Fc region portion, and a CH1 region; and a second single chain polypeptide comprising a first binding domain, a hinge, an Fc region portion, a CL region, a second hinge, and a second binding domain.
In embodiment (40), a polypeptide heterodimer comprises the following two single chain polypeptides: from amino to carboxyl terminus, a first single chain polypeptide comprising a first binding domain, a hinge, an Fc region portion, and a CH1 region; and a second single chain polypeptide comprising a hinge, an Fc region portion, a CL region, a second hinge and a second binding domain.
In embodiment (41), a polypeptide heterodimer comprises the following two single chain polypeptides: from amino to carboxyl terminus, a first single chain polypeptide comprising a hinge, an Fc region portion, a CH1 region, a second hinge and a first binding domain; and the second single chain polypeptide comprising a second binding domain, a hinge, an Fc region portion, and a CL region.
In embodiment (42), a polypeptide heterodimer comprises the following two single chain polypeptides: from amino to carboxyl terminus, a first single chain polypeptide comprising a first binding domain, a hinge, an Fc region portion, a CH1 region, and a second hinge; and a second single chain polypeptide comprising a second binding domain, a hinge, an Fc region portion, a CL region and a second hinge.
In embodiment (43), a polypeptide heterodimer comprises the following two single chain polypeptides: from amino to carboxyl terminus, a first single chain polypeptide comprising a first binding domain, a CH1 region, a hinge an Fc region portion, a CL region, a second hinge and a second binding domain; and the second single chain polypeptide comprising a CL region, a hinge, an Fc region portion and a CH1 region.
In embodiment (44), a polypeptide heterodimer comprises the following two single chain polypeptides: from amino to carboxyl terminus, a first single chain polypeptide comprising a CH1 region, a hinge, an Fc region portion, and a CL region; and a second single chain polypeptide comprising a first binding domain, a CL region, a hinge, an Fc region, a CH1 region, a second hinge and a second binding domain.
In embodiment (45), a polypeptide heterodimer comprises the following two single chain polypeptides: from amino to carboxyl terminus, a first single chain polypeptide comprising a first binding domain, a CH1 region, a hinge, an Fc region portion, and a CL region; and a second single chain polypeptide comprising a CL region, a hinge, an Fc region portion, a CH1 region, a second hinge, and a second binding domain.
In embodiment (46), a polypeptide heterodimer comprises the following two single chain polypeptides: from amino to carboxyl terminus, a first single chain polypeptide comprising a CH1 region, a hinge, an Fc region portion, a CL, a second hinge and a first binding domain; and a second single chain polypeptide comprising a second binding domain, a CL region, a hinge, an Fc region portion, and a CH1 region.
In embodiment (47), a polypeptide heterodimer comprises the following two single chain polypeptides: from amino to carboxyl terminus, a first single chain polypeptide comprising a first binding domain, a CH1 region, a hinge, an Fc region portion, a second CH1 region, a second hinge, and a second binding domain; and a second single chain polypeptide comprising a CL region, a hinge, an Fc region portion, and a second CL region.
In embodiment (48), a polypeptide heterodimer comprises the following two single chain polypeptides: from amino to carboxyl terminus, a first single chain polypeptide comprising a CH1 region, a hinge, an Fc region portion, and a second CH1 region; and a second single chain polypeptide comprising a first binding domain, a CL region, a hinge, an Fc region portion, a second CL region, a second hinge, and a second binding domain.
In embodiment (49), a polypeptide heterodimer comprises the following two single chain polypeptides: from amino to carboxyl terminus, a first single chain polypeptide comprising a first binding domain, a CH1 region, a hinge, an Fc region portion, and a second CH1 region; and a second single chain polypeptide comprising a CL region, a hinge, an Fc region portion, a second CL region, a second hinge and a second binding domain.
In embodiment (50), a polypeptide heterodimer comprises the following two single chain polypeptides: from amino to carboxyl terminus, a first single chain polypeptide comprising a CH1 region, a hinge, an Fc region portion, a CH1 region, a second hinge, and a first binding domain; and a second single chain polypeptide comprising a second binding domain, a CL region, a hinge, an Fc region portion, and a second CL region.
Exemplary embodiments (51) to (60) are also provided below in which three or four binding domains are included in each heterodimer. Additional binding domains may be included to make polypeptide heterodimers that comprise five to eight binding domains according to the present disclosure in view of the general description provided herein.
In embodiment (51), a polypeptide heterodimer comprises the following two single chain polypeptides: from amino to carboxyl terminus, a first single chain polypeptide comprising a first binding domain, a CH1 region, a hinge, an Fc region portion, a second hinge and a second binding domain; and a second single chain polypeptide comprising a third binding domain, a CL region, a hinge and an Fc region portion.
In embodiment (52), a polypeptide heterodimer comprises the following two single chain polypeptides: from amino to carboxyl terminus, a first single chain polypeptide comprising a first binding domain, a CH1 region, a hinge, an Fc region portion, a second hinge, and a second binding domain; and a second single chain polypeptide comprising a CL region, a hinge, an Fc region portion, a second hinge and a third binding domain.
In embodiment (53), a polypeptide heterodimer comprises the following two single chain polypeptides: from amino to carboxyl terminus, a first single chain polypeptide comprising a first binding domain, a CL region, a hinge, an Fc region portion, a second hinge and a second binding domain; and the second single chain polypeptide comprising a third binding domain, a CH1 region, a hinge and an Fc region portion.
In embodiment (54), a polypeptide heterodimer comprises the following two single chain polypeptides: from amino to carboxyl terminus, a first single chain polypeptide comprising a first binding domain, a CL region, a hinge, an Fc region portion, a second hinge and a second binding domain; and a second single chain polypeptide comprising a CH1 region, a hinge, an Fc region portion, a second hinge and a third binding domain.
In embodiment (55), a polypeptide heterodimer comprises the following two single chain polypeptides: from amino to carboxyl terminus, a first single chain polypeptide comprising a first binding domain, a hinge, an Fc region portion, a CH1 region, a second hinge and a second binding domain; and the second single chain polypeptide comprising a third binding domain, a hinge, an Fc region portion and a CL region.
In embodiment (56), a polypeptide heterodimer comprises the following two single chain polypeptides: from amino to carboxyl terminus, a first single chain polypeptide comprising a first binding domain, a hinge, an Fc region portion, a CH1 region, a second hinge, and a second binding domain; and a second single chain polypeptide comprising a hinge, an Fc region portion, a CL region, a second hinge, and a third binding domain.
In embodiment (57), a polypeptide heterodimer comprises the following two single chain polypeptides: from amino to carboxyl terminus, a first single chain polypeptide comprising a first binding domain, a hinge, an Fc region portion, a CL region, a second hinge, and a second binding domain; and a second single chain polypeptide comprising a third binding domain, a hinge, an Fc region portion and a CH1 region.
In embodiment (58), a polypeptide heterodimer comprises the following two single chain polypeptides: from amino to carboxyl terminus, a first single chain polypeptide comprising a first binding domain, a hinge, an Fc region portion, a CL region, a second hinge, and a second binding domain; and a second single chain polypeptide comprising a hinge, an Fc region portion, a CH1 region, a second hinge, and a third binding domain.
In embodiment (59), a polypeptide heterodimer comprises the following two single chain polypeptides: from amino to carboxyl terminus: a first binding domain, a CH1 region, a hinge, an Fc region portion, a second hinge, and a second binding domain; and a second single chain polypeptide comprising a third binding domain, a CL region, a hinge, an Fc region portion, a second hinge and a fourth binding domain.
In embodiment (60), a polypeptide heterodimer comprises the following two single chain polypeptides: from amino to carboxyl terminus, a first single chain polypeptide comprising a first binding domain, a hinge, an Fc region portion, a CH1 region, a second hinge and a second binding domain; and a second single chain polypeptide comprising a third binding domain, a hinge, an Fc region portion, a CL region, a second hinge and a fourth binding domain.

In embodiments (1) to (32), a polypeptide heterodimer of the present disclosure comprises the following two single chain polypeptides: a first single chain polypeptide comprising a first binding domain (BD1) that specifically binds to a T cell target (*e.g.,* a TCR complex or a component thereof, including TCRα, TCRβ, CD3γ, CD3δ, and CD3ε), a hinge that is an SCC-P IgG1 hinge, an Fc region portion that is a wild type or altered human IgG1 CH2CH3, and a CL region that is wild type or an altered human Ck region with N30Y V55A T70E (YAE) substitutions; and a second single chain polypeptide comprising binding domain (BD2) that specifically binds to a B-cell target (*e.g.,* CD19, CD79b, HLA-DR, CD37, CD20) or a tumor or cancer antigen (*e.g.,* RON, c-Met, EpCAM, CEACAM-6, PSMA), a hinge that is also an SCC-P IgG1 hinge, an Fc region portion that is a wild type or altered human IgG1 CH2CH3, and a CH1 region that is human CH1 region.

In further embodiments, a polypeptide heterodimer of embodiments (1) to (32) may further comprise a third binding domain (BD3) that is identical to BD1 or BD2, and is linked to a single chain polypeptide via a second hinge *(e.g.,* linker H75 or H68 as set forth in SEQ ID NOS:742 and 78, respectively). In still further embodiments, a polypeptide heterodimer of embodiment of embodiments (1) to (32) may further comprise third and fourth binding domains (BD3) and (BD4) that each are identical or different to BD1 or BD2 and linked to the single chain polypeptide(s) via a second hinge *(e.g.,* linker H75 or H68 as set forth in SEQ ID NOS:742 and 78).

In certain embodiments, the polypeptide heterodimers of the present disclosure can be engineered to have binding domains with different affinities to target specific cell types. For example, it may be desirable for a polypeptide heterodimer with a binding domain for a TCR complex or a component thereof and another binding domain for a tumor antigen (or a B-cell target) to preferentially bind tumor cells having the tumor antigen (or B-cells carrying the B-cell target) with a higher affinity so that the polypeptide heterodimer will bind the tumor cells (or the B-cells) first and then recruit T cells via its TCR/CD3 binding domain to the tumor site or cells. Differential binding affinities may be achieved by, for example, choosing a binding domain for one target with a higher binding affinity than the other binding domain has for its target or by including multiple binding domains for one target on a polypeptide heterodimer and a single binding domain or fewer binding domains for the second or other targets. In addition, using different hinges (*e.g.,* using hinges of different lengths) may affect binding of one domain more than another or using different hinges for different binding domains in order to alter the binding activity of the binding domains.

In certain embodiments, multiple binding domains may need to be located at an appropriate distance from each other so that their interactions with their targets will produce a desirable effect. For example, in certain embodiments, a polypeptide heterodimer comprising a binding domain for a TCR complex or a component thereof in a single chain polypeptide and another binding domain for a tumor antigen or a B-cell target in a second single chain polypeptide may have both binding domains at the amino or carboxyl termini of their corresponding chains so that they are in physical proximity to each other in the polypeptide heterodimer.

Exemplary heterodimers may be formed from single chain polypeptide pairs described herein. If sequence identification numbers noted herein contain signal peptide sequences (*e.g.,* the first 20 amino acids), such signal peptide sequences are not part of the mature single chain polypeptides that form the exemplary polypeptide heterodimers and thus should be considered excluded.

Exemplary single chain polypeptides are set forth in SEQ ID NOS:2, 4, 6, 8, 10, 12, 14, 16, 18, 20, 22, 24, 29-32, 53-72, 74, 810-826, 859-864, and 874-882.

Exemplary heterodimers may be formed from the following single chain polypeptide pairs: SEQ ID NOS:2 and 4, SEQ ID NOS:6 and 8, SEQ ID NOS:10 and 12, SEQ ID NOS:14 and 16, SEQ ID NOS:18 and 20, SEQ ID NOS:20 and 22, SEQ ID NOS:20 and 24, SEQ ID NOS:30 and 32, SEQ ID NOS:29 and 31, SEQ ID NOS:29 and 32, SEQ ID NOS:30 and 72, SEQ ID NOS:53 and 72, SEQ ID NOS:54 and 72, SEQ ID NOS:55 and 72, SEQ ID NOS:70 and 72, SEQ ID NOS:71 and 72, SEQ ID NOS:63 and 56, SEQ ID NOS:64 and 57, SEQ ID NOS:65 and 60, SEQ ID NOS:66 and 58, SEQ ID NOS:67 and 59, SEQ ID NOS:68 and 61, SEQ ID NOS:69 and 62, SEQ ID NOS:54 and 811, SEQ ID NOS:54 and 812, SEQ ID NOS:54 and 813, SEQ ID NOS:814 and 818, SEQ ID NOS:815 and 818, SEQ ID NOS:816 and 818, SEQ ID NOS:817 and 818, SEQ ID NOS:814 and 820, SEQ ID NOS:814 and 821, SEQ ID NOS:54 and 819, SEQ ID NOS:814 and 826, SEQ ID NOS:814 and 822, SEQ ID NOS:814 and 823, SEQ ID NOS:814 and 824, SEQ ID NOS:859 and 862, SEQ ID NOS:860 and 863, SEQ ID NOS:861 and 864, SEQ ID NOS:874 and 825, SEQ ID NOS:875 and 879, SEQ ID NOS:876 and 880, SEQ ID NOS:877 and 881, or SEQ ID NOS:878 and 882.

### Nucleic Acids, Vectors, Host Cells, and Methods for Making Heterodimers

In a related aspect, the present disclosure also provides isolated nucleic acid (used interchangeably with "polynucleotide") molecules that encode single chain polypeptides provided herein. Exemplary nucleic acid molecules (either with or without a nucleotide sequence encoding a signal peptide sequence) are set forth in SEQ ID NOS : 1, 3, 5, 7, 9, 11, 13, 15,17, 19, 21, 23, 25-28, 33-52 and 792-808.

The present disclosure also provides vectors that comprise nucleic acid sequence encoding single chain polypeptides provided herein. As used herein, "vector" refers to a nucleic acid molecule capable of transporting another nucleic acid to which it has been linked. Exemplary vectors include plasmids, yeast artificial chromosomes, and viral genomes. Certain vectors can autonomously replicate in a host cell, while other vectors can be integrated into the genome of a host cell and thereby are replicated with the host genome.

In certain embodiments, the vectors may be recombinant expression vectors. "Recombinant expression vectors" or "expression vectors" refer to vectors that contain nucleic acid sequences that are operatively linked to an expression control sequence (*e.g.,* a promoter) and are thus capable of directing the expression of those sequences.

Promoter sequences useful in expression vectors provided herein can be selected from any desired gene using CAT (chloramphenicol transferase) vectors or other vectors with selectable markers. Eukaryotic promoters include CMV immediate early, HSV thymidine kinase, early and late SV40, LTRs from retrovirus, and mouse metallothionein-I. In certain embodiments, the promoters are inducible promoters.

In certain embodiments, a vector is an expression vector that comprises a nucleic acid sequence encoding a first single chain polypeptide of a polypeptide heterodimer provided herein. In certain other embodiments, a vector is an expression vector that comprises a nucleic acid sequence encoding a second single chain polypeptide of a polypeptide heterodimer provided herein.

In certain embodiments, a vector is an expression vector that comprises nucleic acid sequences encoding both first and second single chain polypeptides of a polypeptide heterodimer. The promoter for the nucleic acid sequence encoding the first single chain polypeptide may be the same as the promoter for the nucleic acid encoding the second single chain polypeptide. Alternatively, the promoter for the nucleic acid sequence encoding the first single chain polypeptide may be different from the promoter for the nucleic acid encoding the second single chain polypeptide so that the expression level of the first and second single chain polypeptides may be differentially modulated to maximum heterodimerization of the first and second single chain polypeptides. In certain embodiments, one or both the promoters for the nucleic acid encoding the first and second single chain polypeptides are inducible promoters.

The present disclosure also provides a host cell transformed or transfected with, or otherwise containing, any of the nucleic acids or vectors provided herein. Exemplary host cells include VERO cells, HeLa cells, Chinese hamster ovary (CHO) cell lines (including modified CHO cells capable of modifying the glycosylation pattern of expressed multivalent binding molecules, *see* US Patent Application Publication No. 2003/0115614), COS cells (such as COS-7), W138, BHK, HepG2, 3T3, RIN, MDCK, A549, PC 12, K562, HEK293 cells, HepG2 cells, N cells, 3T3 cells, *Spodoptera frugiperda* cells (*e.g.,* Sf9 cells), *Saccharomyces cerevisiae* cells, *Escherichia coli, Bacillus subtilis, Salmonella typhimurium,* or members of the *Streptomycete* family.

In certain embodiments, a host cell comprises a first expression vector containing a nucleic acid encoding a first single chain polypeptide and a second expression vector containing a nucleic acid encoding a second single chain polypeptide.

In certain other embodiments, a host cell comprises an expression vector containing a nucleic acid encoding both first and second single chain polypeptides.

The disclosure also includes a method of producing polypeptide heterodimers described herein. In certain embodiments, the method comprises culturing a host cell that comprises nucleic acids encoding both the first and second single chain polypeptides under conditions suitable to express the polypeptides, and optionally isolating or purifying the heterodimers formed from the first and second single chain polypeptides from the culture. The nucleic acid encoding the first single chain polypeptide and the nucleic acid encoding the second single chain polypeptide may be present in a single expression vector in the host cell or in two different expression vectors in the host cells. In the latter case, the ratio between the two expression vectors may be controlled to maximize heterodimerization of the first and second single chain polypeptides.

The present disclosure provides purified polypeptide heterodimers as described herein. The term "purified," as used herein, refers to a composition, isolatable from other components, wherein the polypeptide heterodimer is enriched to any degree relative to its naturally obtainable state. In certain embodiments, the present disclosure provides substantially purified polypeptide heterodimers as described herein. "Substantially purified" refers to a polypeptide heterodimer composition in which the polypeptide heterodimer forms the major component of the composition, such as constituting at least about 50%, such as at least about 60%, about 70%, about 80%, about 90%, about 95%, about 99%, of the polypeptides, by weight, in the composition.

Protein purification techniques are well known to those of skill in the art. These techniques involve, at one level, the crude fractionation of the polypeptide and non-polypeptide fractions. Further purification using chromatographic and electrophoretic techniques to achieve partial or complete purification (or purification to homogeneity) is frequently desired. Analytical methods particularly suited to the preparation of a pure fusion protein are ion-exchange chromatography, size exclusion chromatography; polyacrylamide gel electrophoresis; and isoelectric focusing. Particularly efficient methods of purifying peptides are fast protein liquid chromatography and HPLC.

Various methods for quantifying the degree of purification are known to those of skill in the art in light of the present disclosure. These include, for example, assessing the amount of polypeptide heterodimers in a fraction by SDS/PAGE analysis and HPLC as illustrated in the examples provided herein.

The method for making polypeptide heterodimers provided herein is advantageous over a method for first expressing and purifying separately individual single chain polypeptides and then incubating purified individual single chain polypeptides together to form polypeptide heterodimers. For example, certain single chain polypeptides (e.g., certain polypeptides containing only CH1 regions as their immunoglobulin heterodimerization domains) are unstable when expressed alone. In addition, separate expression and purification of individual single chain polypeptides followed by combining the purified individual single chain polypeptides involve more steps than coexpressing both single chain polypeptides followed by purifying resulting polypeptide heterodimers and generally less efficient.

### Compositions and Methods for Using Heterodimers

In addition to polypeptide heterodimers, the present disclosure also provides pharmaceutical compositions and unit dose forms that comprise the polypeptide heterodimers as well as methods for using the polypeptide heterodimers, the pharmaceutical compositions and unit dose forms.

Compositions of polypeptide heterodimers of this disclosure generally comprise a polypeptide heterodimer provided herein in combination with a pharmaceutically acceptable excipient, including pharmaceutically acceptable carriers and diluents. Pharmaceutical acceptable excipients will be nontoxic to recipients at the dosages and concentrations employed. They are well known in the pharmaceutical art and described, for example, in Rowe et al., Handbook of Pharmaceutical Excipients: A Comprehensive Guide to Uses, Properties, and Safety, 5th Ed., 2006.

Pharmaceutically acceptable carriers for therapeutic use are also well known in the pharmaceutical art, and are described, for example, in Remington's Pharmaceutical Sciences, Mack Publishing Co. (A.R. Gennaro (Ed.) 1985). Exemplary pharmaceutically acceptable carriers include sterile saline and phosphate buffered saline at physiological pH. Preservatives, stabilizers, dyes and the like may be provided in the pharmaceutical composition. In addition, antioxidants and suspending agents may also be used.

Pharmaceutical compositions may also contain diluents such as buffers, antioxidants such as ascorbic acid, low molecular weight (less than about 10 residues) polypeptides, proteins, amino acids, carbohydrates (*e.g.,* glucose, sucrose, dextrins), chelating agents (*e.g.,* EDTA), glutathione and other stabilizers and excipients. Neutral buffered saline or saline mixed with nonspecific serum albumin are exemplary diluents. For instance, the product may be formulated as a lyophilizate using appropriate excipient solutions (*e.g.,* sucrose) as diluents.

The present disclosure also provides a method for treating a disease or disorder associated with, for example, excessive receptor-mediated signal transduction, comprising administering to a patient in need thereof an effective amount of a polypeptide heterodimer comprising a binding domain that specifically binds a receptor, such as a receptor tyrosine kinase, including EGFR, EGFRvIII, ErbB2, ErbB3, ErbB4, IGF1R, c-Met, RON, and EphA2.

Exemplary diseases or disorders associated with excess receptor-mediated signal transduction include cancer *(e.g.,* solid malignancy and hematologic malignancy), autoimmune or inflammatory diseases or conditions, sepsis resulting from bacterial infection, and viral infection.

In one aspect, the present disclosure provides a method for directing T cell activation, comprising administering to a patient in need thereof an effective amount of a polypeptide heterodimer that comprises a binding domain that specifically binds TCRα, TCRβ, CD3γ, CD3δ, CD3ε or a combination thereof, and a second binding domain that specifically binds a different target, for instance, a tumor-specific antigen or other antigen of choice at a site or cell where T cell activation is desired.

In another aspect, the present disclosure provides a method for inhibiting growth, metastasis or metastatic growth of a malignancy (*e.g.,* a solid malignancy or a hematologic malignancy), comprising administering to a patient in need thereof an effective amount of a polypeptide heterodimer provided herein or a composition thereof.

A wide variety of cancers, including solid malignancy and hematologic malignancy, are amenable to the compositions and methods disclosed herein. Types of cancer that may be treated include, but are not limited to: adenocarcinoma of the breast, prostate, pancreas, colon and rectum; all forms of bronchogenic carcinoma of the lung (including squamous cell carcinoma, adenocarcinoma, small cell lung cancer and non-small cell lung cancer); myeloid; melanoma; hepatoma; neuroblastoma; papilloma; apudoma; choristoma; branchioma; malignant carcinoid syndrome; carcinoid heart disease; and carcinoma (*e.g.,* Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell). Additional types of cancers that may be treated include: histiocytic disorders; leukemia; histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin; and glioblastoma multiforme. The types of cancers that may be treated also include, but are not limited to, angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

Additional exemplary cancers that are also amenable to the compositions and methods disclosed herein are B-cell cancers, including B-cell lymphomas [such as various forms of Hodgkin's disease, non-Hodgkins lymphoma (NHL) or central nervous system lymphomas], leukemias [such as acute lymphoblastic leukemia (ALL), chronic lymphocytic leukemia (CLL), Hairy cell leukemia and chronic myoblastic leukemia] and myelomas (such as multiple myeloma). Additional B cell cancers include small lymphocytic lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma/leukemia, B-cell proliferations of uncertain malignant potential, lymphomatoid granulomatosis, and post-transplant lymphoproliferative disorder.

In certain embodiments, polypeptide heterodimers useful for inhibiting growth of a solid malignancy or metastasis or metastatic growth of a solid malignancy or a hematologic malignancy include those that specifically bind to a tumor or cancer antigen and a T cell target. Such heterodimers are typically designed to have a higher binding affinity to the tumor or cancer antigen (*e.g.,* at least about 5, 10, 15, 20, 25, 50, 75 or 100 folds higher) than to the T cell target so that they preferentially bind to the tumor or cancer antigen first and subsequently bind to the T cell target, which in turn recruit and activate T cells to damage or destroy the tumor or cancer cells carrying the tumor or cancer antigen on their surface. Exemplary tumor or cancer antigens and T cell targets include those provided above in the section describing binding domains of polypeptide heterodimers. For instance, a T cell target can be CD3 or PSMA.

In certain embodiments, polypeptide heterodimers useful for inhibiting growth, metastasis, or metastatic growth of a malignancy, or by directed T cell activation, comprise at least one binding domain that specifically binds an oncology target (including a tumor or cell antigen, such as RON, c-Met, CEACAM-6, or PSMA) and another binding domain that specifically binds a TCR complex or a component thereof (*e.g.,* TCRα, TCRβ, CD3γ, CD3δ, and CD3ε).

In certain other embodiments, polypeptide heterodimers useful for inhibiting growth, metastasis, or metastatic growth of a malignancy comprise at least one binding domain that specifically binds CD28 and another binding domain that specifically binds CD79b. Such polypeptide heterodimers may further comprise a binding domain that comprises a PDL2 ectodomain, a binding domain that specifically binds hyperIL-6, or both binding domains.

In certain other embodiments, polypeptide heterodimers useful for inhibiting growth, metastasis, or metastatic growth of a malignancy comprise at least one binding domain that specifically binds RON and another binding domain that specifically binds c-Met.

In certain other embodiments, polypeptide heterodimers useful for inhibiting growth, metastasis, or metastatic growth of a malignancy comprise binding domains that specifically bind one or more of the following receptor tyrosine kinases: c-Met, RON, EGFR, EGFRvIII, Her2, ErbB3, ErbB4, and IGF1R, EphA2. In certain embodiments, the heterodimers may further comprise one or more binding domains that specifically bind to one or more of the following antiangiogenic agents: PDGFR, VEGFR1-4, and angiopoietin 2. In certain embodiments, the above heterodimers may further comprise one or more binding domains that specifically bind to one or more the following Fc receptors to increase targeting of cytotoxic effector function: CD64, CD32A and CD16. In certain embodiments, the above heterodimers may further comprise a binding domain that specifically binds the transferrin receptor (CD71) to enable degradation of the receptors.

In certain other embodiments, polypeptide heterodimers useful for inhibiting growth, metastasis, or metastatic growth of a malignancy comprise binding domains that are agonists of two or more of the following TNFSFRs: TNFR1, TNFR2, DR4, DR5, TWEAKR, and FAS.

In certain other embodiments, polypeptide heterodimers useful for inhibiting growth, metastasis, or metastatic growth of a malignancy comprise binding domains that specifically bind two or more of the following pro-oncogenic cytokines or growth factors: HGF, MSP, EGF (including epiregulin, herregulin, β-regulin, neuregulin), HIF-1α, VEGFA, VEGFB, VEGFC, VEGFD, TNFα, IL-6, hyperIL-6, IL-8, Wnt, sHH, TGFβ, or PDGF.

In certain embodiments, polypeptide heterodimers useful for inhibiting growth of a solid malignancy or metastasis or metastatic growth of a solid malignancy include those that specifically bind to, for example, EGFR, ErbB3, ErbB4, c-Met, RON, EphA2, IGF1R, VEGFR1, VEGFR2, VEGFR3, CD44v6, CD151, EpCAM, CEACAM6, TGFBR2, , GHRHR, GHR, IL-6R, gp130, TNFR2, PD1, TWEAK-R, OSMRβ, Patched-1, Frizzled, or Robo1.

Polypeptide heterodimers useful for inhibiting metastasis or metastatic growth of a hematologic malignancy include those that specifically bind to, for example, EGFR, ErbB3, c-Met, RON, EphA2, IGF1R, TGFBR2, IL-6R, gp130, TNFR2, PD1, OSMRβ, LTβR, CD 19, CD80, CD81, or CD86.

In another aspect, the present disclosure provides a method for treating an autoimmune or inflammatory disease, disorder or condition, comprising administering to a patient in need thereof an effective amount of a polypeptide heterodimer provided herein or a composition thereof.

Exemplary autoimmune or inflammatory diseases, disorders or conditions that may be treated by the fusion proteins and compositions and unit dose forms thereof include, and are not limited to, inflammatory bowel disease (e.g., Crohn's disease or ulcerative colitis), diabetes mellitus (e.g., type I diabetes), dermatomyositis, polymyositis, pernicious anaemia, primary biliary cirrhosis, acute disseminated encephalomyelitis (ADEM), Addison's disease, ankylosing spondylitis, antiphospholipid antibody syndrome (APS), autoimmune hepatitis, Goodpasture's syndrome, Graves' disease, Guillain-Barré syndrome (GBS), Hashimoto's disease, idiopathic thrombocytopenic purpura, systemic lupus erythematosus, lupus nephritis, neuropsychiatric lupus, multiple sclerosis (MS), myasthenia gravis, pemphigus vulgaris, asthma, psoriatic arthritis, rheumatoid arthritis, Sjögren's syndrome, temporal arteritis (also known as "giant cell arteritis"), autoimmune hemolytic anemia, Bullous pemphigoid, vasculitis, coeliac disease, chronic obstructive pulmonary disease, endometriosis, Hidradenitis suppurativa, interstitial cystitis, morphea, scleroderma, narcolepsy, neuromyotonia, vitiligo, and autoimmune inner ear disease.

Exemplary polypeptide heterodimers useful for treating an autoimmune or inflammatory disease, disorder or condition may comprises one or more binding domain that specifically binds CD28, and one, two, or three of the following additional binding domains: a binding domain that comprises PDL2 ectodomain, a binding domain that comprises monoIL-10, and a binding domain that specifically binds CD86. For example, a polypeptide heterodimer may comprise a binding domain that specifically binds CD28 and one to three binding domains that specifically bind CD86.

Additional exemplary polypeptide heterodimers useful for treating an autoimmune or inflammatory disease, disorder or condition may comprise one or more binding domains that specifically bind CD28 and one or more binding domains that are an IL-10 agonist (*e.g.,* monoIL-10), an HLA-G agonist, an HGF agonist, an IL-35 agonist, a PD-1 agonist, a BTLA agonist, a LIGHT antagonist, a GITRL antagonist or a CD40 antagonist. Alternatively, polypeptide heterodimers may comprise two or more binding domains selected from an IL-10 agonist (*e.g.,* monoIL-10), an HLA-G agonist, an HGF agonist, an IL-35 agonist, a PD-1 agonist, a BTLA agonist, a LIGHT antagonist, a GITRL antagonist or a CD40 antagonist.

Additional exemplary polypeptide heterodimers useful for treating an autoimmune or inflammatory disease, disorder or condition may comprise one or more binding domains that specifically bind CD32B and one or more binding domains that are an IL-10 agonist (*e.g.,* monoIL-10), an HLA-G agonist, an HGF agonist, an IL-35 agonist, a PD-1 agonist, a BTLA agonist, a LIGHT antagonist, a GITRL antagonist or a CD40 antagonist.

Additional exemplary polypeptide heterodimers useful for treating an autoimmune or inflammatory disease, disorder or condition may comprise binding domains that specifically bind one or more of the following TNFSFRs: TNFR1, TNFR2, HVEW, LTβR, TWEAKR, TACI, BAFF-R, BCMA, or FAS.

Additional exemplary polypeptide heterodimers useful for treating an autoimmune or inflammatory disease, disorder or condition may comprise binding domains that specifically bind two or more of the following pro-inflammatory cytokines/chemokines, such as TNFα, IL-6, IL-2, IL-1, IL-8, IP-10, IFNγ, IFNα, RANKL, FASL, TGFβ, IL7, IL10, IL17A/F, TWEAK, CSF2, IGF1, IGF2 or BLyS/APRIL.

Polypeptide heterodimers useful for treating an autoimmune or inflammatory disease, disorder or condition include those that specifically bind to, for example, TGFBR2, IL-6R, gp130, TNFR1, TNFR2, PD1, HVEM, OX40, CD40, CD137, TWEAK-R, LTβR, LIFRβ, OSMRβ, CD3, TCRα, TCRβ, CD19, CD28, CD80, CD81, CD86, TLR7, TLR9, or any combination thereof.

In another aspect, the present disclosure provides a method for treating a B-cell associated disorder or disease that comprises administering to a patient in need thereof *(e.g.,* a patient having or suspected of having a B-cell associated disorder or disease) an effective amount of a polypeptide heterodimer provided herein, such as those that specifically bind a B cell target.

Polypeptide heterodimers useful in treating a B-cell associated disorder or disease may comprise binding domains that specifically bind one or more B-cell targets, such as CD79b, CD19, HLA-DR, CD20, CD21, CD22, CD30, CD33, CD37, CD38, or CD70. The polypeptide heterodimers may further comprise a binding domain that specifically binds a T-cell target, such as a TCR complex or a component thereof, including TCRα, TCRβ, CD3γ, CD3δ, or CD3ε.

Polypeptide heterodimers useful in treating a B-cell associated disorder or disease may comprise a binding domain that specifically bind a B-cell target, such as CD79b, CD19, HLA-DR, CD20, CD21, CD22, CD30, CD33, CD37, CD38, and CD70, and a binding domain that specifically binds CD64, CD32A, or CD16 to increase targeting of cytotoxic effector function.

The polypeptide heterodimers or compositions thereof of the present disclosure may be administered orally, topically, transdermally, parenterally, by inhalation spray, vaginally, rectally, or by intracranial injection, or any combination thereof. In one embodiment, the polypeptide heterodimers or compositions thereof are administered parenterally. The term "parenteral," as used herein, includes subcutaneous injections, intravenous, intramuscular, intracisternal injection, or infusion techniques. Administration by intravenous, intradermal, intramusclar, intramammary, intraperitoneal, intrathecal, retrobulbar, intrapulmonary injection and/or surgical implantation at a particular site is contemplated as well. For instance, the invention includes administering polypeptide heterodimers or compositions thereof by intravenous injection.

The pharmaceutically effective dose depends on the type of disease, the composition used, the route of administration, the type of subject being treated, the physical characteristics of the specific subject under consideration for treatment, concurrent medication, and other factors that those skilled in the medical arts will recognize. For example, an amount between 0.01 mg/kg and 1000 mg/kg (*e.g.,* about 0.1 to 1 mg/kg, about 1 to 10 mg/kg, about 10-50 mg/kg, about 50-100 mg/kg, about 100-500 mg/kg, or about 500-1000 mg/kg) body weight (which can be administered as a single dose, daily, weekly, monthly, or at any appropriate interval) of active ingredient may be administered depending on the potency of a polypeptide heterodimer of this disclosure.

Also contemplated is the administration of polypeptide heterodimers or compositions thereof in combination with a second agent. A second agent may be one accepted in the art as a standard treatment for a particular disease state or disorder, such as in cancer, inflammation, autoimmunity, and infection. Exemplary second agents contemplated include polypeptide heterodimers that bind to targets different from those that primary polypeptide heterodimers bind, polyclonal antibodies, monoclonal antibodies, immunoglobulin-derived fusion proteins, chemotherapeutics, ionizing radiation, steroids, NSAIDs, anti-infective agents, or other active and ancillary agents, or any combination thereof.

In certain embodiments, a polypeptide heterodimer and a second agent act synergistically. In other words, these two compounds interact such that the combined effect of the compounds is greater than the sum of the individual effects of each compound when administered alone (*see, e.g.,* Berenbaum, Pharmacol. Rev. 41:93, 1989).

In certain other embodiments, a polypeptide heterodimer and a second agent act additively. In other words, these two compounds interact such that the combined effect of the compounds is the same as the sum of the individual effects of each compound when administered alone.

Second agents useful in combination with polypeptide heterodimers or compositions thereof provided herein may be steroids, NSAIDs, mTOR inhibitors (*e.g.,* rapamycin (sirolimus), temsirolimus, deforolimus, everolimus, zotarolimus, curcumin, farnesylthiosalicylic acid), calcineurin inhibitors (*e.g.,* cyclosporine, tacrolimus), antimetabolites (*e.g.,* mycophenolic acid, mycophenolate mofetil), polyclonal antibodies *(e.g.,* anti-thymocyte globulin), monoclonal antibodies *(e.g.,* daclizumab, basiliximab), and CTLA4-Ig fusion proteins (e.g., abatacept or belatacept).

Second agents useful for inhibiting growth of a solid malignancy, inhibiting metastasis or metastatic growth of a solid malignancy, or treating or ameliorating a hematologic malignancy include chemotherapeutic agents, ionizing radiation, and other anti-cancer drugs. Examples of chemotherapeutic agents contemplated as further therapeutic agents include alkylating agents, such as nitrogen mustards (e.g., mechlorethamine, cyclophosphamide, ifosfamide, melphalan, and chlorambucil); bifunctional chemotherapeutics (*e.g*., bendamustine); nitrosoureas (e.g., carmustine (BCNU), lomustine (CCNU), and semustine (methyl-CCNU)); ethyleneimines and methyl-melamines (e.g., triethylenemelamine (TEM), triethylene thiophosphoramide (thiotepa), and hexamethylmelamine (HMM, altretamine)); alkyl sulfonates (e.g., buslfan); and triazines (e.g., dacabazine (DTIC)); antimetabolites, such as folic acid analogues (e.g., methotrexate, trimetrexate, and pemetrexed (multi-targeted antifolate)); pyrimidine analogues (such as 5-fluorouracil (5-FU), fluorodeoxyuridine, gemcitabine, cytosine arabinoside (AraC, cytarabine), 5-azacytidine, and 2,2'-difluorodeoxycytidine); and purine analogues (*e.g*., 6-mercaptopurine, 6-thioguanine, azathioprine, 2'-deoxycoformycin (pentostatin), erythrohydroxynonyladenine (EHNA), fludarabine phosphate, 2-chlorodeoxyadenosine (cladribine, 2-CdA)); Type I topoisomerase inhibitors such as camptothecin (CPT), topotecan, and irinotecan; natural products, such as epipodophylotoxins (*e.g.,* etoposide and teniposide); and vinca alkaloids (e.g., vinblastine, vincristine, and vinorelbine); anti-tumor antibiotics such as actinomycin D, doxorubicin, and bleomycin; radiosensitizers such as 5-bromodeozyuridine, 5-iododeoxyuridine, and bromodeoxycytidine; platinum coordination complexes such as cisplatin, carboplatin, and oxaliplatin; substituted ureas, such as hydroxyurea; and methylhydrazine derivatives such as N-methylhydrazine (MIH) and procarbazine.

In certain embodiments, second agents useful for inhibiting growth metastasis or metastatic growth of a malignancy include polypeptide heterodimers according to the present disclosure that bind to cancer cell targets other than the target that the first polypeptide heterodimer binds. In certain other embodiments, second agents useful for such treatments include polyclonal antibodies, monoclonal antibodies, and immunoglobulin-derived fusion proteins that bind to cancer cell targets. Exemplary cancer cell targets are provided above in the context of describing targets of polypeptide heterodimers useful for the above-noted treatment.

Further therapeutic agents contemplated by this disclosure for treatment of autoimmune diseases are referred to as immunosuppressive agents, which act to suppress or mask the immune system of the individual being treated. Immunosuppressive agents include, for example, non-steroidal anti-inflammatory drugs (NSAIDs), analgesics, glucocorticoids, disease-modifying antirheumatic drugs (DMARDs) for the treatment of arthritis, or biologic response modifiers. Compositions in the DMARD description are also useful in the treatment of many other autoimmune diseases aside from rheumatoid arthritis.

Exemplary NSAIDs are chosen from the group consisting of ibuprofen, naproxen, naproxen sodium, Cox-2 inhibitors such as Vioxx and Celebrex, and sialylates. Exemplary analgesics are chosen from the group consisting of acetaminophen, oxycodone, tramadol of proporxyphene hydrochloride. Exemplary glucocorticoids are chosen from the group consisting of cortisone, dexamethasone, hydrocortisone, methylprednisolone, prednisolone, or prednisone. Exemplary biological response modifiers include molecules directed against cell surface markers (e.g., CD4, CD5, etc.), cytokine inhibitors, such as the TNF antagonists (e.g. etanercept (Enbrel), adalimumab (Humira) and infliximab (Remicade)), chemokine inhibitors and adhesion molecule inhibitors. The biological response modifiers include monoclonal antibodies as well as recombinant forms of molecules. Exemplary DMARDs include azathioprine, cyclophosphamide, cyclosporine, methotrexate, penicillamine, leflunomide, sulfasalazine, hydroxychloroquine, Gold (oral (auranofin) and intramuscular) and minocycline.

Additional second agents useful for treating an autoimmune or inflammatory disease, disorder or condition may be a polyclonal or monoclonal antibody, an immunoglobulin-derived fusion protein (*e.g.,* scFv, SMIP™, PIMS, SCORPION™ fusion proteins), or a polypeptide heterodimer according to the present disclosure that specifically bind a target associated with such a disease, disorder or condition. Examples of such targets are provided above in the context of targets of polypeptide heterodimers of the present disclosure useful in the above-noted treatment.

It is contemplated the binding molecule composition and the second active agent may be given simultaneously in the same formulation. Alternatively, the second agents may be administered in a separate formulation but concurrently (*i.e.,* given within less than one hour of each other).

In certain embodiments, the second active agent may be administered prior to administration of a polypeptide heterodimer or a composition thereof. Prior administration refers to administration of the second active agent at least one hour prior to treatment with the polypeptide heterodimer or the composition thereof. It is further contemplated that the active agent may be administered subsequent to administration of the binding molecule composition. Subsequent administration is meant to describe administration at least one hour after the administration of the polypeptide heterodimer or the composition thereof.

This disclosure contemplates a dosage unit comprising a pharmaceutical composition of this disclosure. Such dosage units include, for example, a single-dose or a multi-dose vial or syringe, including a two-compartment vial or syringe, one comprising the pharmaceutical composition of this disclosure in lyophilized form and the other a diluent for reconstitution. A multi-dose dosage unit can also be, *e.g.,* a bag or tube for connection to an intravenous infusion device.

This disclosure also contemplates a kit comprising a pharmaceutical composition of this disclosure in unit dose, or multi-dose, container, *e.g.,* a vial, and a set of instructions for administering the composition to patients suffering a disorder such as a disorder described above.

### EXAMPLES

### EXAMPLE 1

### BIVALENT POLYPEPTIDE HETERODIMER WITH TWO PAIRS OF HETERODIMERIZATION DOMAINS

Polypeptide heterodimer X0172 was made by co-expressing single chain polypeptides X0130 (2E12 CH1 CH2 CH3 Ck) and X0168 (Ck CH2 CH3 CH1 H68 2E12). Single chain polypeptide X0130 comprises, from its amino to carboxyl terminus: 2E12 (anti-CD28) scFv, human IgG1 CH1, human IgG1 SCC-P hinge, human IgG1 CH2, human IgG1 CH3, and altered human Ck (without the first Arg or the last Cys). The nucleic acid and amino acid sequences of X130 are set forth in SEQ ID NOS:1 and 2, respectively. Single chain polypeptide X0168 comprises, from its amino to carboxyl terminus: altered human Ck (without the first Arg or the last Cys), human IgG1 SCC-P hinge, human IgG1 CH2, human IgG1 CH3, human IgG1 CH1, H68 linker, and 2E12 (anti-CD28) scFv. The nucleic acid and amino acid sequences of X0168 are set forth in SEQ ID NOS:3 and 4, respectively. The amino acid sequence of H68 linker is set forth in SEQ ID NO:78.

For comparison, polypeptide heterodimer X0124 was made by co-expressing single chain polypeptide X0112 (2E12 CH1 CH2 CH3) and X0113 (Ck CH2 CH3). Single chain polypeptide X0112 comprises, from its amino to carboxyl terminus: 2E12 (anti-CD28) scFv, human IgG1 CH1, human IgG1 SCC-P hinge, human IgG1 CH2, and human IgG1 CH3. The nucleic acid and amino acid sequences of X0112 are set forth in SEQ ID NOS:5 and 6, respectively. Single chain polypeptide X0113 comprises, from its amino to carboxyl terminus: altered human Ck (without the first Arg or the last Cys), human IgG1 SCC-P hinge, human IgG1 CH2, and human IgG1 CH3. The nucleic acid and amino acid sequence of X0113 are set forth in SEQ ID NOS:7 and 8, respectively.

### Expression

The day before transfection, HEK293 cells were suspended at a cell concentration of 0.5x10⁶ cells/ml in Freestyle 293 expression medium (Gibco). For a large transfection, 250 ml of cells were used, but for a small transfection, 60 ml of cells were used. On the transfection day, 320 ul of 293fectin reagent (Invitrogen) was mixed with 8 ml of media. At the same time, 250 ug of DNA for each of the two chains were also mixed with 8 ml of media and incubated for 5 minutes. After 15 minutes of incubation, the DNA-293fectin mixture was added to the 250ml of 293 cells and returned to the shaker at 37°C and shaken at a speed of 120 RPM. For the smaller transfection using 60 ml of cells, a fourth of the DNA, 293fectin and media were used.

### Purification

Protein A affinity chromatography was used to purify the proteins. 2 mL of packed protein A agarose (Repligen) was added to a Biorad column (Econo-column chromatography column, size 2.5 x 10 cm), washed extensively with PBS (10x column volume) and the supernatants were loaded, washed with PBS again and eluted with 3 column volumes of Pierce IgG elution buffer. Proteins were then dialyzed extensively against PBS. Proteins were then concentrated using Amicon centrifugal filter devices to a final volume of around 0.5 mL.

For second step purification, Protein L affinity chromatography or cation exchange chromatography were used. For Protein L purification, protein A purified Polypeptide heterodimer was passed over a Protein L agarose column that had been pre-equilibrated with PBS, washed with PBS (10x column volume) and then eluted with Pierce IgG elution buffer. Proteins were then dialyzed against PBS extensively and concentrated using Amicon centrifugal filter devices to a final volume of around 0.5 mL.

Samples (200-300 ug) of previously affinity purified (Protein A or Protein L) Polypeptide heterodimer constructs were dialyzed into 20 mM MES, pH 6.0 (Buffer A) and loaded onto a MonoS 5/50 GL cation exchange column (GE Healthcare) at a flow rate of 2 mL/min, using an AKTA Explorer FPLC. The column was allowed to equilibrate for 5 column volumes (CV) and then run in a gradient format to a mixture of 50%:50% buffer A:buffer B (buffer B being 20 mM MES, 1 M NaCl, pH 6.0) over 20 CV. A following mixture of 100% buffer B was run for 5 CV to clean the column, and the system was run for another 5 CV at 100% buffer A to re-equilibrate prior to the next injection. Peaks were collected and analyzed by SDS-PAGE and electrospray mass spectrometry.

### SDS-PAGE Analysis

Proteins purified were analyzed on a 10 % SDS-PAGE gel using Invitrogen's X-cell Surelock gel box.

### Synergy with Suboptimal Concentration of PMA

Peripheral blood mononuclear cells (PBMC) from in-house donors were isolated from heparinized whole blood via centrifugation over Lymphocyte Separation Media (MP Biomedicals, Aurora, OH) and washed two times with RPMI media (Gibco-Invitrogen, Carlsbad, CA). CD4+ T-cells were then enriched from the PBMC using negative selection with a MACS CD4+ T-cell Isolation Kit (Miltenyi Biotec, Auburn, CA). The enriched (>95%) CD4+ T-cells were then resuspended at a concentration of 1x10⁶ cell/ml in complete RPMI/10% FCS. Test reagents were prepared at 40 ug/ml (yielding a final concentration of 10 µg/ml) in complete RPMI/10% FCS and added in 50 µl/well to flat-bottom 96-well plates (BD Falcon, San Jose, CA). PMA (Phorbol 12 myristate 13-acetate; A.G. Scientific, Inc., San Diego, CA) in complete RPMI/10% FCS was added in 50 ul/well at 4 ng/ml (final concentration of 1 ng/ml). Then T-cells in complete RPMI/10% FCS were added at a concentration of 5 x 10⁴ cells/well in a 50 µl volume, and finally an appropriate amount of complete RPMI/10% FCS was added to each well (typically 50 ul) to bring the final volume to 200 ul/well. The cells were treated with the test samples +/- PMA and incubated for 72 hours at 37°C in 5% CO₂. One microliter of tritiated thymidine (Amersham Biosciences, Pisctaway, NJ) in a 1:50 dilution of complete RPMI/10% FCS (50 ul/well) was added to the wells for the last 6 hours of culture. Plates were harvested onto a Unifilter-96, GF/C microplate (Perkin Elmer, Boston, MA) with a Packard Filtermate Harvester (Perkin Elmer, Boston, MA). Numbers are expressed as cpm and are the mean of replicate samples.

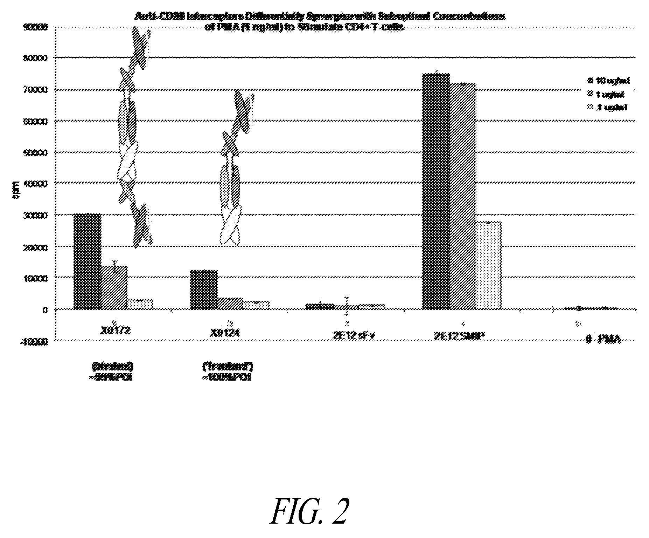

Figure 2 shows that Polypeptide heterodimer X0172 has a differential property compared to 2E12 SMIP M0039 (SEQ ID NO:77). It did not synergize with PMA as well as the SMIP. This bivalent polypeptide heterodimer has a property closer to the monovalent polypeptide heterodimer, X0124, rather than the bivalent SMIP.

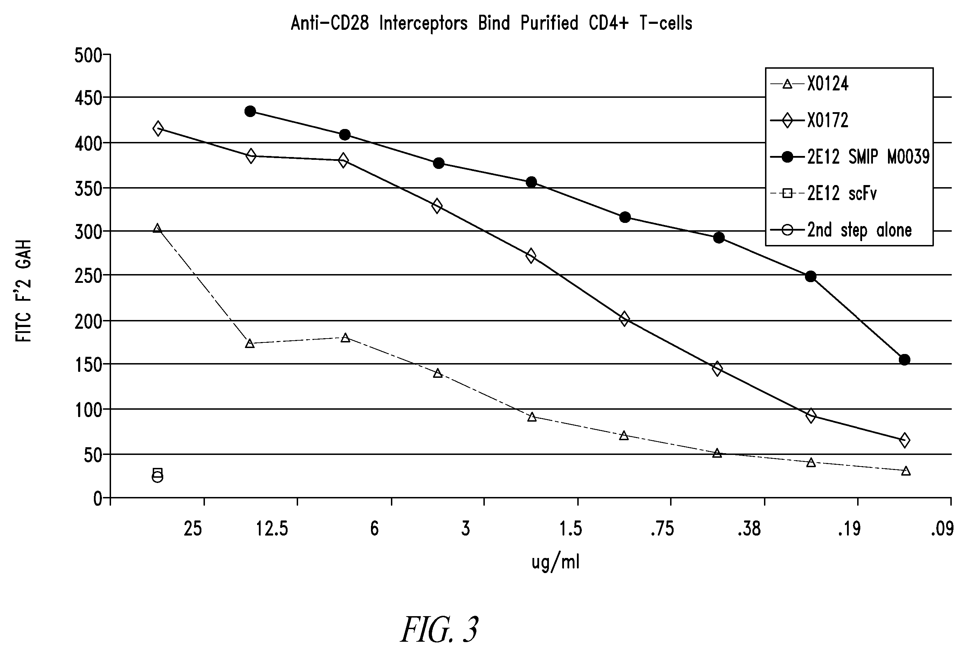

Figure 3 shows that bivalent polypeptide heterodimer X0172 bound to CD4⁺ cells better than 2E12 scFv (SEQ ID NO:109) and monovalent polypeptide heterodimer X0124.

### EXAMPLE 2

### BIVALENT, TRIVALENT, TETRAVALENT POLYPEPTIDE HETERODIMER USING SINGLE HETERODIMERIZATION DOMAIN PAIR

Bivalent polypeptide heterodimer X0251 was made by co-expressing single chain polypeptides X0244 (Ck(YAE) CH2(N297A) CH3 H68 2E12) and X0245 (2E12 CH1 CH2(N297A) CH3). Single chain polypeptide X0244 comprises, from its amino to carboxyl terminus: human Ck(YAE) (*i.e.,* human Ck without the first Arg or last Cys but with N30Y, V55A, and T70E substitutions), human IgG1 SCC-P hinge, human IgG1 CH2 (N297A) (*i.e.,* human IgG1 CH2 with a N297A substitution), human IgG1 CH3, H68 linker, and 2E12 (anti-CD28) scFv. The nucleotide and amino acid sequences of X0244 are set forth in SEQ ID NOS:9 and 10, respectively. Single chain polypeptide X0245 comprises, from its amino to carboxyl terminus: 2E12 (anti-CD28) scFv, human IgG1 CH1, human IgG1 SCC-P hinge, human IgG1 CH2 (N297A), and human IgG1 CH3. The nucleotide and amino acid sequences of X0245 are set forth in SEQ ID NOS:11 and 12, respectively.

Trivalent, bispecific polypeptide heterodimer X0252 was made by co-expressing single chain polypeptides X0246 (P2C2 Ck(YAE) CH2(N297A) CH3) and X0247 (2E12 CH1 CH2(N297A) CH3 H68 2E12). Single chain polypeptide X0246 comprises, from its amino to carboxyl terminus: P2C2 (anti-CD79b) scFv, human Ck (YAE), human IgG1 SCC-P hinge, human IgG1 CH2 (N297A), and human IgG1 CH3. The nucleotide and amino acid sequences of X0246 are set forth in SEQ ID NOS:13 and 14, respectively. Single chain polypeptide X0247 comprises, from its amino to carboxyl terminus: 2E12 (anti-CD28) scFv, human IgG1 CH1, human IgG1 SCC-P hinge, human IgG1 CH2 (N297A), human IgG1 CH3, H68 linker, and 2E12 (anti-CD28) scFv. The nucleotide and amino acid sequences of X0247 are set forth in SEQ ID NOS:15 and 16, respectively.

Tetravalent, trispecific polypeptide heterodimer X0253 was made by co-expressing single chain polypeptides X0248 (P2C2 CK(YAE) CH2(N297A) CH3 H68 A2) and X0249 (PDL2 ECD CH1 CH2(N297A) CH3 H68 2E12). Single chain polypeptide X0248 comprises, from its amino to carboxyl terminus: P2C2 (anti-CD79b) scFv, human Ck (YAE), human IgG1 SCC-P hinge, human IgG1 CH2 (N297A), human IgG1 CH3, H68 linker, and A2 (anti-hyperIL-6) scFv. The nucleotide and amino acid sequences of X0248 are set forth in SEQ ID NOS:17 and 18, respectively. Single chain polypeptide X0249 comprises, from its amino to carboxyl terminus: PDL2 ECD (*i.e.,* PDL2 ectodomain), human IgG1 CH1, human IgG1 SCC-P hinge, human IgG1 CH2 (N297A), human IgG1 CH3, H68 linker, and 2E12 (anti-CD28) scFv. The nucleotide and amino acid sequences of X0249 are set forth in SEQ ID NOS:19 and 20, respectively.

Another tetravalent, tetraspecific polypeptide heterodimer X0283, was made by co-expressing single chain polypeptides X0249 (PDL2 ECD CH1 CH2(N297A) CH3 H68 2E12) and X0281 (monoIL-10 Ck(YAE) CH2(N297A) CH3 H68 3D1). Single chain polypeptide X0281 comprises, from its amino to carboxyl terminus: monoIL-10, human Ck(YAE), human IgG1 SCC-P hinge, human IgG1 CH2 (N297A), human IgG1 CH3, H68 linker, and 3D1(anti-CD86) scFv. The nucleotide and amino acid sequences of X0281 are set forth in SEQ ID NOS:21 and 22.

As a control for polypeptide heterodimer X0283, tetravalent heterodimer, polypeptide heterodimer X0284, was made by co-expressing single chain polypeptides X0249 (PDL2 ECD CH1 CH2(N297A) CH3 H68 2E12) and X0282 (monoIL-10 Ck CH2(N297A) CH3 H68 3D1). Single chain polypeptide X0282 is identical to X0281 except that it does not contain N30Y V55A T70E substitutions in the human Ck sequence. Thus, single chain polypeptide X0282 comprises, from its amino to carboxyl terminus: monoIL-10, altered human Ck (without the first Arg or the last Cys), human IgG1 SCC-P hinge, human IgG1 CH2 (N297A), human IgG1 CH3, H68 linker, and 3D1(anti-CD86) scFv. The nucleotide and amino acid sequences of X0282 are set forth in SEQ ID NOS:23 and 24, respectively.

The polypeptide heterodimer molecules were expressed and purified according to Example 1. Purified proteins were analyzed using SDS-PAGE electrophoresis under reduced and non-reduced conditions. Size exclusion chromatography was performed on an AKTA Explorer FPLC (Pharmacia Biotech) using a Superdex200 10/300 GL column. Some proteins were analyzed by electrospray mass spectrometry using an Agilent 6120 TOF ES/MS.

Surface plasmon resonance (SPR) measurements were performed on a Biacore T100 SPR using HBS-P+ (GE Healthcare) as a running buffer. Target was directly immobilized onto a CM5 chip using standard amine coupling chemistry (BIACORE® Amine Coupling Kit, GE Healthcare), with final immobilization levels between 800 and 1900 Ru (resonance units). Polypeptide heterodimer X0283 was injected at 25°C or 37°C for 150 seconds at a flow rate of 30 µl/min in a series of concentrations from 10 nM to 1 µM. Dissociation was monitored for 1200 seconds, and the surface was regenerated by injecting 50 mM NaOH for 60 seconds. Binding interactions with the surface were stable through at least 60 regeneration cycles. Data were analyzed using BiaEvaluation for the T100 software (version 2.0, GE Healthcare).

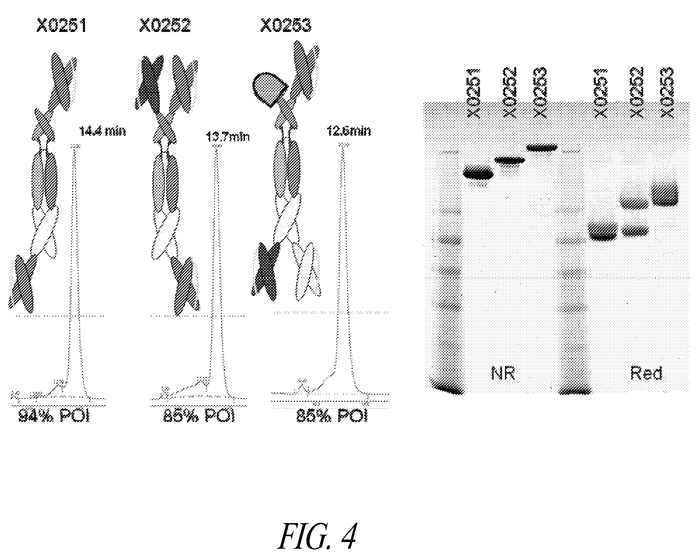

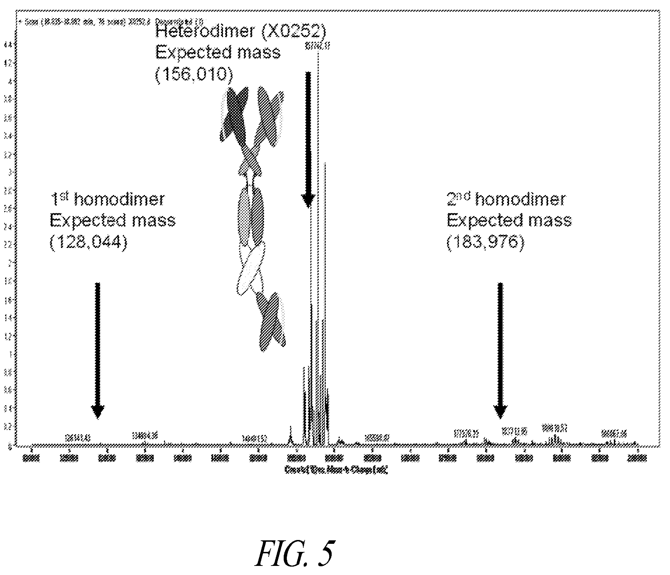

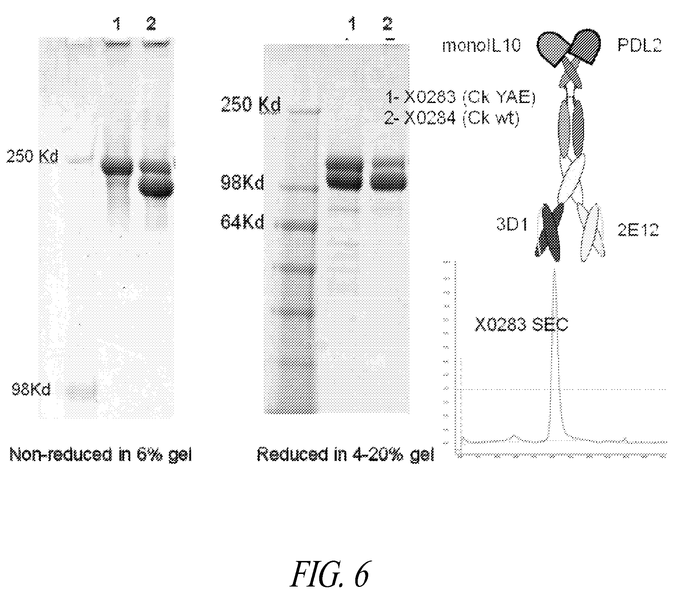

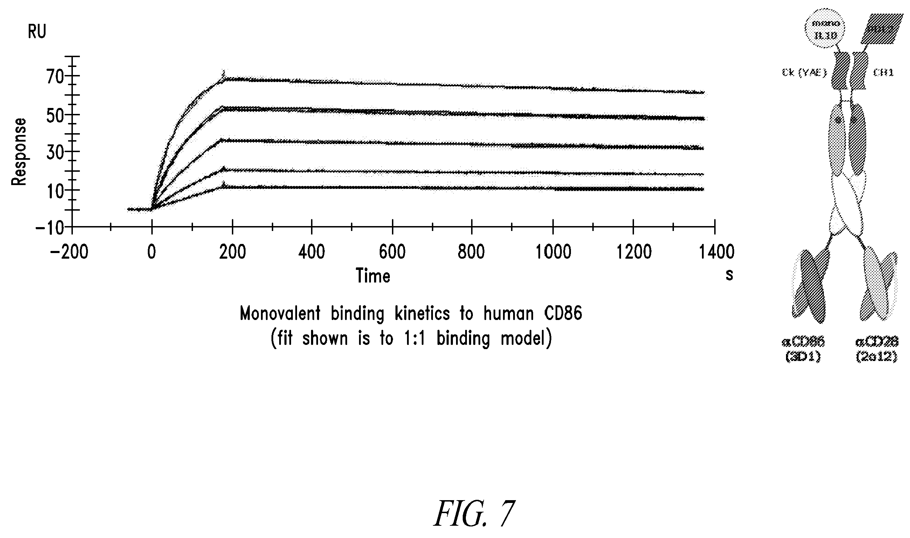

The SDS-PAGE electrophoresis and cation exchange chromatography analyses show that polypeptide heterodimers X0251, X0252 and X0253 were reasonably well expressed and purified (Figure 4). Mass spectrometry analysis of X0252 shows that the protein is mostly homogeneous with an undetectable amount of the homodimers (Figure 5). In addition, the SDS-PAGE electrophoresis and cation exchange chromatography analyses of polypeptide heterodimers X0283 and X0284 show that polypeptide heterodimer X0283 having the CH1/Ck(YAE) heterodimerization domains efficiently assembled into a heterodimer compared to control polypeptide heterodimer X0284 having the CH1/Ck heterodimerization domains, which formed homodimers as well (Figure 6). The Biocore analysis further shows that the 3D1 (anti-CD86) binding domain at the carboxyl terminus of single chain polypeptide X0281 monovalently bound to CD86 (Figure 7).

### EXAMPLE 3

### POLYPEPTIDE HETERODIMERS WITH ANTI-RON AND ANTI-C-MET BINDING DOMAINS

A bivalent polypeptide heterodimer with anti-RON binding domains (ORN151) and two bispecific polypeptide heterodimers comprising anti-RON and anti-cMet binding domains (ORN152 and ORN153) were made.

Bivalent polypeptide heterodimer ORN151 comprises single chain polypeptides ORN145 (4C04 CH2 CH3 CH1) and ORN148 (11H09 CH2 CH3 Ck(YAE)). Single chain polypeptide ORN145 comprises from its amino to carboxyl terminus: 4C04 (anti-RON) scFv, human IgG1 SCC-P hinge, human IgG1 CH2, human IgG1 CH3 and human IgG1 CH1. The nucleotide and amino acid sequences of ORN145 are set forth in SEQ ID NOS:26 and 30, respectively. Single chain polypeptide ORN148 comprises from its amino to carboxyl terminus: 11H09 (anti-RON) scFv, human IgG1 SCC-P hinge, human CH2, human CH3, and human Ck(YAE). The nucleotide and amino acid sequences of ORN148 are set forth in SEQ ID NOS:28 and 32, respectively.

Bispecific (c-Met, RON) polypeptide heterodimer ORN152 comprises single chain polypeptides ORN116 (MET021 CH2 CH3 CH1) and ORN146 (4C04 CH2 CH3 Ck(YAE)). Single chain polypeptide ORN116 comprises from its amino to carboxyl terminus: MET021 (anti-c-Met) scFv, human IgG1 SCC-P hinge, human IgG1 CH2, human IgG1 CH3 and human IgG1 CH1. The nucleotide and amino acid sequences of ORN116 are set forth in SEQ ID NOS:25 and 29, respectively. Single chain polypeptide ORN146 comprises from its amino to carboxyl terminus: 4C04 (anti-RON) scFv, human IgG1 SCC-P hinge, human CH2, human CH3, and human Ck(YAE). The nucleotide and amino acid sequences of ORN146 are set forth in SEQ ID NOS:27 and 31, respectively.

Bispecific (c-Met, RON) polypeptide heterodimer ORN153 comprises single chain polypeptides ORN116 (MET021 CH2 CH3 CH1) and ORN148 (11H09 CH2 CH3 Ck(YAE)).

Polypeptide heterodimers ORN151, ORN152 and ORN153 were expressed according to Example 1. The following expression levels were obtained: 1.9 µg protein / mL of culture for ORN151, 3.1 µg / mL for ORN152, and 4.9 µg / mL for ORN153.

### EXAMPLE 4

### POLYPEPTIDE HETERODIMERS WITH ANTI-CD3 BINDING DOMAINS

Several bispecific polypeptide heterodimers with an anti-CD3 binding domain were made: polypeptide heterodimers S0268, S0269, and TSC020 to TSC030. Polypeptide heterodimer S0268 comprises single chain polypeptides ORN145 (4C04 CH2 CH3 CH1) and TSC019 (G19-4 CH2 CH3 Ck(YAE)). Single chain polypeptide TSC019 comprises from its amino to carboxyl terminus: G19-4 (anti-CD3) scFv, human IgG1 SCC-P hinge, human CH2, human CH3, and human Ck(YAE). The nucleotide and amino acid sequences of TSC019 are set forth in SEQ ID NOS:52 and 72, respectively.

Bispecific (CD3, c-Met) Polypeptide heterodimer S0269 comprises single chain polypeptides ORN160 (5D5 CH2 CH3 CH1) and TSC019 (G19-4 CH2 CH3 Ck(YAE)). Single chain polypeptide ORN160 comprises from its amino to carboxyl terminus: 5D5 (anti-c-Met) scFv, human IgG1 SCC-P hinge, human IgG1 CH2, human IgG1 CH3, and human IgG1 CH1. The nucleotide and amino acid sequences of ORN160 are set forth in SEQ ID NOS:33 and 53, respectively.

Bivalent (CD19) polypeptide heterodimer TSC020 comprises single chain polypeptides TSC001 (HD37 CH2 CH3 CH1) and TSC019 (G19-4 CH2 CH3 Ck(YAE)). Single chain polypeptide TSC001 comprises from its amino to carboxyl terminus: HD37 (anti-CD19) scFv, human IgG1 SCC-P hinge, human IgG1 CH2, human IgG1 CH3 and human IgG1 CH1. The nucleotide and amino acid sequences of TSC001 are set forth in SEQ ID NOS:34 and 54, respectively.

Bispecific (CD20, CD3) polypeptide heterodimer TSC021 comprises single chain polypeptides TSC002 (2H7 CH2 CH3 CH1) and TSC019 (G19-4 CH2 CH3 Ck(YAE)). Single chain polypeptide TSC002 comprises from its amino to carboxyl terminus: 2H7 (anti-CD20) scFv, human IgG1 SCC-P hinge, human IgG1 CH2, human IgG1 CH3 and human IgG1 CH1. The nucleotide and amino acid sequences of TSC002 are set forth in SEQ ID NOS:35 and 55, respectively.

Bispecific (CD79b, CD3) polypeptide heterodimer TSC022 comprises single chain polypeptides TSC017 (P2C2 H2 CH3 CH1) and TSC019 (G19-4 CH2 CH3 Ck(YAE)). Single chain polypeptide TSC017 comprises from its amino to carboxyl terminus: P2C2 (anti-CD79b) scFv, human IgG1 SCC-P hinge, human IgG1 CH2, human IgG1 CH3 and human IgG1 CH1. The nucleotide and amino acid sequences of TSC017 are set forth in SEQ ID NOS:50 and 70, respectively.

Bispecific (HLA-DR, CD3) polypeptide heterodimer TSC023 comprises single chain polypeptides TSC018 (M0042 CH2 CH3 CH1) and TSC019 (G19-4 CH2 CH3 Ck(YAE)). Single chain polypeptide TSC018 comprises from its amino to carboxyl terminus: M0042 (anti-HLA-DR) scFv, human IgG1 SCC-P hinge, human IgG1 CH2, human IgG1 CH3, and human IgG1 CH1. The nucleotide and amino acid sequences of TSC018 are set forth in SEQ ID NOS:51 and 71, respectively.

Bivalent polypeptide heterodimer TSC024 comprises single chain polypeptides TSC010 (HD37-"IgA1 hinge"-CH2 CH3 CH1) and TSC003 (G19-4-"IgA1 hinge"-CH2 CH3 Ck(YAE)). Single chain polypeptide TSC010 comprises from its amino to carboxyl terminus: HD37 (anti-CD19) scFv, altered human IgA1 hinge (PSTPPTPSPSTPPTPSPSCPPCP, SEQ ID NO:752), human IgG1 CH2, human IgG1 CH3, and human IgG1 CH1. The nucleotide and amino acid sequences of TSC010 are set forth in SEQ ID NOS:43 and 63, respectively. Single chain polypeptide TSC003 comprises from its amino to carboxyl terminus: G19-4 (anti-CD3) scFv, altered human IgA1 hinge (SEQ ID NO:752), human IgG1 CH2, human IgG1 CH3, and human Ck(YAE). The nucleotide and amino acid sequences of TSC003 are set forth in SEQ ID NOS:36 and 56, respectively.

Bivalent polypeptide heterodimer TSC025 comprises single chain polypeptides TSC011 (HD37-"IgA2 hinge"-CH2 CH3 CH1) and TSC004 (G19-4-"IgA2 hinge"-CH2 CH3 Ck(YAE)). Single chain polypeptide TSC011 comprises from its amino to carboxyl terminus: HD37 (anti-CD19) scFv, altered human IgA2 hinge (PPPPPCPPCP, SEQ ID NO:748), human IgG1 CH2, human IgG1 CH3, and human IgG1 CH1. The nucleotide and amino acid sequences of TSC011 are set forth in SEQ ID NOS:44 and 64, respectively. Single chain polypeptide TSC004 comprises from its amino to carboxyl terminus: G19-4 (anti-CD3) scFv, altered human IgA2 hinge (SEQ ID NO:748), human IgG1 CH2, human IgG1 CH3, and human Ck(YAE). The nucleotide and amino acid sequences of TSC004 are set forth in SEQ ID NOS:37 and 57, respectively.

Bivalent polypeptide heterodimer TSC026 comprises single chain polypeptides TSC012 (HD37-"IgG1 hinge"-CH2 CH3 CH1) and TSC007 (G19-4-"IgG1 hinge"-CH2 CH3 Ck(YAE)). Single chain polypeptide TSC012 comprises from its amino to carboxyl terminus: HD37 (anti-CD19) scFv, altered human IgG1 hinge (EPKSSDKTHTSPPSPCPPCP, SEQ ID NO:750), human IgG1 CH2, human IgG1 CH3, and human IgG1 CH1. The nucleotide and amino acid sequences of TSC012 are set forth in SEQ ID NOS:45 and 65, respectively. Single chain polypeptide TSC007 comprises from its amino to carboxyl terminus: G19-4 (anti-CD3) scFv, altered human IgG1 hinge (SEQ ID NO:750), human IgG1 CH2, human IgG1 CH3, and human Ck(YAE). The nucleotide and amino acid sequences of TSC007 are set forth in SEQ ID NOS:40 and 60, respectively.

Bivalent polypeptide heterodimer TSC027 comprises single chain polypeptides TSC013 (HD37-"IgG3 hinge"-CH2 CH3 CH1) and TSC005 (G19-4-"IgG3 hinge"-CH2 CH3 Ck(YAE)). Single chain polypeptide TSC013 comprises from its amino to carboxyl terminus: HD37 (anti-CD19) scFv, altered human IgG3 hinge (EPKSSDTPPPSPRSPCPPCP, SEQ ID NO:751), human IgG1 CH2, human IgG1 CH3, and human IgG1 CH1. The nucleotide and amino acid sequences of TSC013 are set forth in SEQ ID NOS:46 and 66, respectively. Single chain polypeptide TSC005 comprises from its amino to carboxyl terminus: G19-4 (anti-CD3) scFv, altered human IgG3 hinge (SEQ ID NO:751), human IgG1 CH2, human IgG1 CH3, and human Ck(YAE). The nucleotide and amino acid sequences of TSC005 are set forth in SEQ ID NOS:38 and 58, respectively.

Bivalent polypeptide heterodimer TSC028 comprises single chain polypeptides TSC014 (HD37-"IgD hinge"-CH2 CH3 CH1) and TSC006 (G19-4-"IgD hinge"-CH2 CH3 Ck(YAE)). Single chain polypeptide TSC014 comprises from its amino to carboxyl terminus: HD37 (anti-CD19) scFv, altered human IgD hinge (ESPKAQASSVPTAQPQAEGSLAKATTAPATTRNTCPPCP, SEQ ID NO:754), human IgG1 CH2, human IgG1 CH3, and human IgG1 CH1. The nucleotide and amino acid sequences of TSC014 are set forth in SEQ ID NOS:47 and 67, respectively. Single chain polypeptide TSC006 comprises from its amino to carboxyl terminus: G19-4 (anti-CD3) scFv, altered human IgD hinge (SEQ ID NO:754), human IgG1 CH2, human IgG1 CH3, and human Ck(YAE). The nucleotide and amino acid sequences of TSC006 are set forth in SEQ ID NOS:39 and 59, respectively.

Bivalent polypeptide heterodimer TSC029 comprises single chain polypeptides TSC015 (HD37-"IgE CH2 hinge"-CH2 CH3 CH1) and TSC008 (G19-4-"IgE CH2 hinge"-CH2 CH3 Ck(YAE)). Single chain polypeptide TSC015 comprises from its amino to carboxyl terminus: HD37 (anti-CD19) scFv, altered human IgE CH2 hinge (SEQ ID NO:757), human IgG1 CH2, human IgG1 CH3, and human IgG1 CH1. The nucleotide and amino acid sequences of TSC014 are set forth in SEQ ID NOS:48 and 68, respectively. Single chain polypeptide TSC008 comprises from its amino to carboxyl terminus: G19-4 (anti-CD3) scFv, altered human IgE CH2 hinge (SEQ ID NO:757), human IgG1 CH2, human IgG1 CH3, and human Ck(YAE). The nucleotide and amino acid sequences of TSC008 are set forth in SEQ ID NOS:41 and 61, respectively.

Bivalent polypeptide heterodimer TSC030 comprises single chain polypeptides TSC016 (HD37-"IgM CH2 hinge"-CH2 CH3 CH1) and TSC009 (G19-4-"IgM CH2 hinge"-CH2 CH3 Ck(YAE)). Single chain polypeptide TSC016 comprises from its amino to carboxyl terminus: HD37 (anti-CD19) scFv, altered human IgM CH2 hinge (SEQ ID NO:759), human IgG1 CH2, human IgG1 CH3, and human IgG1 CH1. The nucleotide and amino acid sequences of TSC015 are set forth in SEQ ID NOS:49 and 69, respectively. Single chain polypeptide TSC009 comprises from its amino to carboxyl terminus: G19-4 (anti-CD3) scFv, altered human IgM CH2 hinge (SEQ ID NO:759), human IgG1 CH2, human IgG1 CH3, and human Ck(YAE). The nucleotide and amino acid sequences of TSC009 are set forth in SEQ ID NOS:42 and 62, respectively.

Bispecific polypeptide heterodimers S0268 (RON, CD3) and S0269 (CD3, c-Met) were expressed according to Example 1. The following expression levels were obtained: 2.2 µg protein / mL of culture for S0268 and 2.7 µg / mL for S0269.

### EXAMPLE 5

### CELL BINDING OF BISPECIFIC POLYPEPTIDE HETERODIMERS

To compare the effectiveness of bispecific polypeptide heterodimer molecules at targeting a tumor cell antigen and T-cells, the on-cell binding characteristics of an anti-RON (4C04 binding domain) x anti-CD3 (G19-4 binding domain) polypeptide heterodimer, S0268 (as described in Example 4), with a different bispecific scaffold (SCORPION™ protein) containing the same binding domains, S0266, were compared. Additionally, the on-cell binding characteristics of two bispecific polypeptide heterodimers, TSC020 and TSC021 (as described in Example 4), targeting different B-cell antigens (CD19 for TSC020 and CD20 for TSC021) as well as a T-cell antigen (CD3) were compared. The nucleotide and amino acid sequences of SCORPION protein S0266 are set forth in SEQ ID NOS:73 and 74, respectively. Nucleotide and amino acid sequences of the single chain polypeptides making up the bispecific polypeptide heterodimers S0268, TSC020, and TSC021 are set forth in SEQ ID NOS:26 and 52 (nucleotide), 30 and 72 (amino acid); 34 and 52 (nucleotide), 54 and 72 (amino acid); and 35 and 52 (nucleotide), 55 and 72 (amino acid), respectively (see also Example 4). Transient transfection in human 293 cells produced 6.9 µg protein / mL of culture for S0266; 2.3 µg / mL of culture for S0268; 3.0 µg / mL of culture for TSC020; and 3.2 µg / mL of culture for TSC021.

MDA-MB-453 (RON+) breast carcinoma cells, Rec1 (CD19⁺, CD20⁺) mantle cell lymphoma cells, and Jurkat (CD3⁺) T cell leukemia cells were obtained from ATCC (Manassas, VA), and cultured according to the provided protocol. T-cells were isolated from donor PBMCs using a Pan T-cell Isolation Kit II from Miltenyi Biotec (Bergisch Gladbach, Germany). Non T-cells were separated from PBMCs by being indirectly magnetically labeled with biotin-conjugated monoclonal antibodies and anti-biotin magnetic microbeads. These cells were then depleted by retaining them in a column surrounded by a magnetic field. The T-cells were not retained in the column and were collected in the flow through.

Binding was assessed by incubating 5 × 10⁵ T cells or target (MDA-MB-453, Rec1, Jurkat) cells for 30 minutes at 4°C with serially diluted bispecific molecules S0266 (αRON × αCD3 SCORPION™ protein) or S0268 (αRON × αCD3 polypeptide heterodimer) (for MDA-MB-453 cells and isolated T cells); or TSC020 (αCD19 x αCD3) or TSC021 (αCD20 x αCD3) for Rec1 and Jurkat cells, in concentrations from 100 nM to 0.1 nM. The cells were washed three times and then incubated with goat anti-human IgG-FITC (1:200 dilution) for another 30 minutes at 4°C. The cells were then washed again three times, fixed in 1% paraformaldehyde and read on a FACS-Calibur instrument.

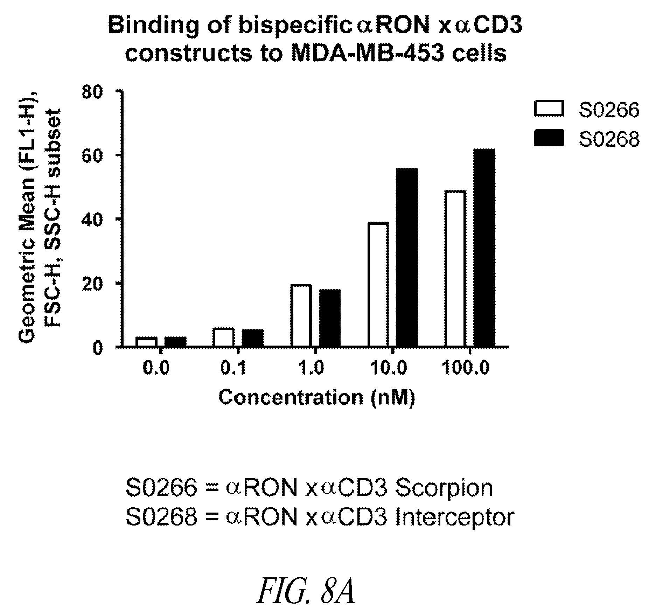

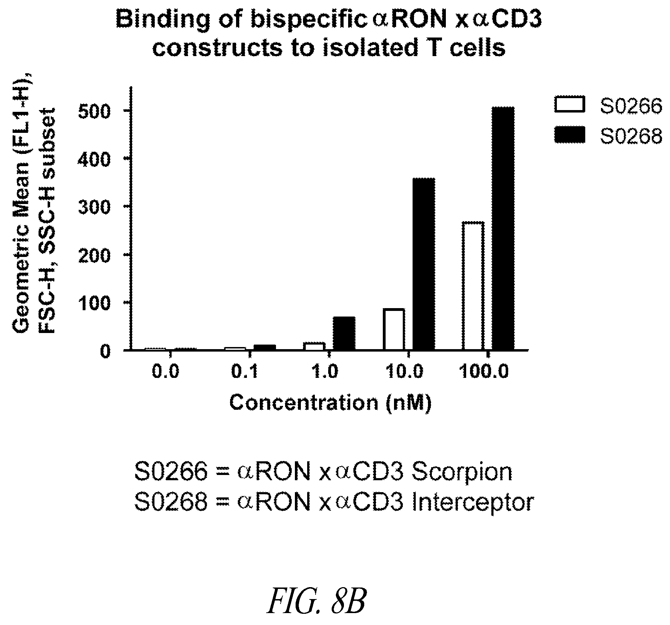

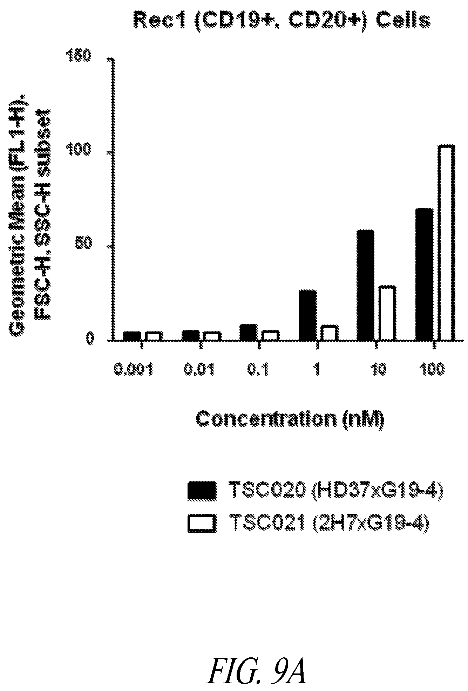

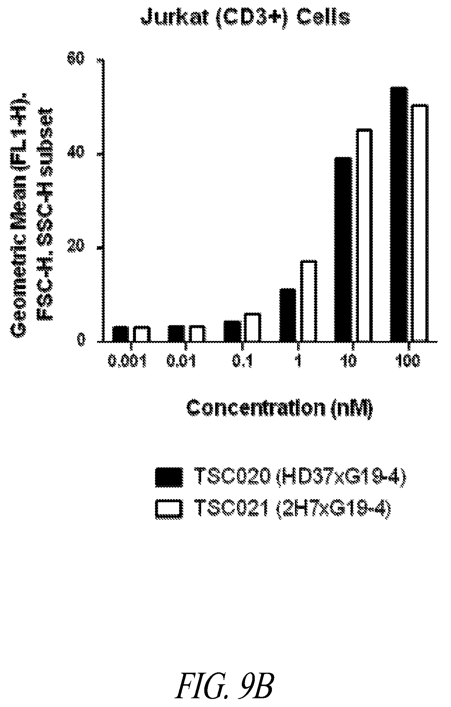

Analysis of the FSC high, SSC high subset in FlowJo v7.5 (Tree Star, Inc, Ashland, OR) showed dose-dependent binding of bispecific molecules S0266 and S0268 to both MDA-MB-453 and isolated T-cells (Figures 8A and 8B). Unexpectedly, the S0268 polypeptide heterodimer bound with similar affinity to the comparable SCORPION™ molecule (S0266) on both MDA-MB-453 cells and T-cells, although it lacked the potential for any avidity. Higher saturation on both target cell types was also observed with the polypeptide heterodimer, which would be the case if the polypeptide heterodimer was binding at a higher stoichometry (1:1 binding of polypeptide heterodimer to surface antigen) than the equivalent SCORPION™ (potential 1:2 binding of the bivalent Scorpion to surface antigens). Comparison of the CD 19-targeting bispecific heterodimer (TSC020; HD37 and G19-4 binding domains) and the CD20-targeting bispecific heterodimer (TSC021; 2H7 and G19-4 binding domains) on Rec1 and Jurkat cells revealed that the TSC020 heterodimer had higher affinity for the Rec1 cells than TSC021 heterodimer (Figure 9A). However, both TSC020 and TSC021 heterodimers showed similar binding to CD3⁺ Jurkat cells, which is expected since both heterodimers possess the same anti-CD3 binding domain (G19-4) (Figure 9B).

### EXAMPLE 6

### ADDITIONAL BIVALENT & TRIVALENT POLYPEPTIDE HETERODIMERS USING SINGLE HETERODIMERIZATION DOMAIN PAIR

Additional multispecific heterodimers were made: TSC046, TSC047, TSC048, TSC054, TSC055, TSC056, TSC057, TSC078, TSC079, TSC080, TSC099, TSC100, TSC101, and TSC102.

Bivalent polypeptide heterodimer TSC046 comprises single chain polypeptides TSC001 (HD37 CH2 CH3 CH1) and TSC039 (BMA031 CH2 CH3 Ck(YAE)). Single chain polypeptide TSC001 comprises from its amino to carboxyl terminus: HD37 (anti-CD19) scFv, human IgG1 SCC-P hinge, human IgG1 CH2, human IgG1 CH3, and human IgG1 CH1. The nucleotide and amino acid sequences of TSC001 are set forth in SEQ ID NOS:34 and 54, respectively. Single chain polypeptide TSC039 comprises from its amino to carboxyl terminus: BMA031 (anti-TCR) scFv, human IgG1 SCC-P hinge, human IgG1 CH2, human IgG1 CH3, and human Ck(YAE). The nucleotide and amino acid sequences of TSC039 are set forth in SEQ ID NOS:793 and 811, respectively.

Bivalent polypeptide heterodimer TSC047 comprises single chain polypeptides TSC001 (HD37 CH2 CH3 CH1) and TSC041 (CRIS7 CH2 CH3 Ck(YAE)). Single chain polypeptide TSC041 comprises from its amino to carboxyl terminus: CRIS7 (anti-CD3) scFv, human IgG1 SCC-P hinge, human IgG1 CH2, human IgG1 CH3, and human Ck(YAE). The nucleotide and amino acid sequences of TSC041 are set forth in SEQ ID NOS:794 and 812, respectively.

Bivalent polypeptide heterodimer TSC048 comprises single chain polypeptides TSC001 (HD37 CH2 CH3 CH1) and TSC043 (OKT3-M CH2 CH3 Ck(YAE)). Single chain polypeptide TSC043 comprises from its amino to carboxyl terminus: OKT3-M (Micromet variant anti-CD3, see also US 7,635,472) scFv, human IgG1 SCC-P hinge, human IgG1 CH2, human IgG1 CH3, and human Ck(YAE). The nucleotide and amino acid sequences of TSC043 are set forth in SEQ ID NOs:795 and 813, respectively.

Bivalent polypeptide heterodimer TSC054 comprises single chian polypeptides TSC049 (HD37 CH2(ADCC/CDC null) CH3 CH1) and TSC053 (G19-4 CH2(ADCC/CDC null) CH3 Ck(YAE)). Single chain polypeptide TSC049 comprises from its amino to carboxyl terminus: HD37 (anti-CD19) scFv, human IgG1 SCC-P hinge, human IgG1 CH2(ADCC/CDC null) (*i.e.,* human IgG1 CH2 with L234A, L235A, G237A, E318A, K320A, and K322A substitutions), human IgG1 CH3, and human IgG1 CH1. The nucleotide and amino acid sequences of TSC049 are set forth in SEQ ID NOS:796 and 814, respectively. Single chain polypeptide TSC053 comprises from its amino to carboxyl terminus: G19-4 (anti-CD3) scFv, human IgG1 SCC-P hinge, human IgG1 CH2(ADCC/CDC null) (*i.e.,* human IgG1 CH2 with L234A, L235A, G237A, E318A, K320A, and K322A substitutions), human IgG1 CH3, and human Ck(YAE). The nucleotide and amino acid sequences of TSC053 are set forth in SEQ ID NOS:800 and 818, respectively.

Bivalent polypeptide heterodimer TSC055 comprises single chain polypeptides TSC050 (2H7 CH2(ADCC/CDC null) CH3 CH1) and TSC053 (G19-4 CH2(ADCC/CDC null) CH3 Ck(YAE)). Single chain polypeptide TSC050 comprises from its amino to carboxyl terminus: 2H7 (anti-CD20) scFv, human IgG1 SCC-P hinge, human IgG1 CH2(ADCC/CDC null), human IgG1 CH3, and human IgG1 CH1. The nucleotide and amino acid sequences of TSC050 are set forth in SEQ ID NOS:797 and 815, respectively.

Bivalent polypeptide heterodimer TSC056 comprises single chain polypeptides TSC051 (P2C2 CH2(ADCC/CDC null) CH3 CH1) and TSC053 (G19-4 CH2(ADCC/CDC null) CH3 Ck(YAE)). Single chain polypeptide TSC051 comprises from its amino to carboxyl terminus: P2C2 (anti-CD79) scFv, human IgG1 SCC-P hinge, human IgG1 CH2(ADCC/CDC null), human IgG1 CH3, and human IgG1 CH1. The nucleotide and amino acid sequences of TSC051 are set forth in SEQ ID NOS:798 and 816, respectively.

Bivalent polypeptide heterodimer TSC057 comprises single chain polypeptides TSC052 (5D5 CH2(ADCC/CDC null) CH3 CH1) and TSC053 (G19-4 CH2(ADCC/CDC null) CH3 Ck(YAE)). Single chain polypeptide TSC052 comprises from its amino to carboxyl terminus: 5D5 (anti-cMet) scFv, human IgG1 SCC-P hinge, human IgG1 CH2(ADCC/CDC null), human IgG1 CH3, and human IgG1 CH1. The nucleotide and amino acid sequences of TSC052 are set forth in SEQ ID NOS:799 and 817, respectively.

Bivalent polypeptide heterodimer TSC078 comprises single chain polypeptides TSC049 (HD37 CH2(ADCC/CDC null) CH3 CH1) and TSC076 (OKT3 CH2(ADCC/CDC null) CH3 Ck(YAE)). Single chain polypeptide TSC076 comprises from its amino to carboxyl terminus: OKT3 (anti-CD3) scFv, human IgG1 SCC-P hinge, human IgG1 CH2(ADCC/CDC null), human IgG1 CH3, and human Ck(YAE). The nucleotide and amino acid sequences of TSC076 are set forth in SEQ ID NOS:802 and 820, respectively.

Bivalent polypeptide heterodimer TSC079 comprises single chain polypeptides TSC049 (HD37 CH2(ADCC/CDC null) CH3 CH1) and TSC077 (Nuvion CH2(ADCC/CDC null) CH3 Ck(YAE)). Single chain polypeptide TSC077 comprises from its amino to carboxyl terminus: Nuvion (anti-CD3) scFv, human IgG1 SCC-P hinge, human IgG1 CH2(ADCC/CDC null), human IgG1 CH3, and human Ck(YAE). The nucleotide and amino acid sequences of TSC077 are set forth in SEQ ID NOS:803 and 821, respectively.

Trivalent polypeptide heterodimer TSC080 comprises single chain polypeptides TSC001 (HD37 CH2 CH3 CH1) and TSC064 (G19-4 CH2 CH3 Ck(YAE) H75 Met021). Single chain polypeptide TSC064 comprises from its amino to carboxyl terminus: G19-4 (anti-CD3) scFv, human IgG1 SCC-P hinge, human IgG1 CH2, human IgG1 CH3, human Ck(YAE), H75 linker, and Met021 (anti-c-Met) scFv (with the three serine residues at the C-temrinus deleted). The nucleotide and amino acid sequences of TSC064 are set forth in SEQ ID NOS:801 and 819, respectively.

Bivalent polypeptide heterodimer TSC099 comprises single chain polypeptides TSC049 (HD37 CH2(ADCC/CDC null) CH3 CH1) and TSC097 (4C04 CH2(ADCC/CDC null) CH3 Ck(YAE)). Single chain polypeptide TSC097 comprises from its amino to carboxyl terminus: 4C04 (anti-RON) scFv, human IgG1 SCC-P hinge, human IgG1 CH2(ADCC/CDC null), human IgG1 CH3, and human Ck(YAE). The nucleotide and amino acid sequences of TSC097 are set forth in SEQ ID NOS:808 and 826, respectively.

Bivalent polypeptide heterodimer TSC100 comprises single chain polypeptides TSC049 (HD37 CH2(ADCC/CDC null) CH3 CH1) and TSC093 (CRIS7 CH2(ADCC/CDC null) CH3 Ck(YAE)). Single chain polypeptide TSC093 comprises from its amino to carboxyl terminus: CRIS7 (anti-CD3) scFv, human IgG1 SCC-P hinge, human IgG1 CH2(ADCC/CDC null), human IgG1 CH3, and human Ck(YAE). The nucleotide and amino acid sequences of TSC093 are set forth in SEQ ID NOS:804 and 822, respectively.

Bivalent polypeptide heterodimer TSC101 comprises single chain polypeptides TSC049 (HD37 CH2(ADCC/CDC null) CH3 CH1) and TSC094 (OKT3-M CH2(ADCC/CDC null) CH3 Ck(YAE)). Single chain polypeptide TSC094 comprises from its amino to carboxyl terminus: OKT3-M (Micromet variant anti-CD3) scFv, human IgG1 SCC-P hinge, human IgG1 CH2(ADCC/CDC null), human IgG1 CH3, human Ck(YAE). The nucleotide and amino acid sequences of TSC094 are set forth in SEQ ID NOS:805 and 823, respectively.

Bivalent polypeptide heterodimer TSC102 comprises single chain polypeptides TSC049 (HD37 CH2(ADCC/CDC null) CH3 CH1) and TSC095 (BMA031 CH2(ADCC/CDC null) CH3 Ck(YAE)). Single chain polypeptide TSC095 comprises from its amino to carboxyl terminus: BMA031 (anti-TCR) scFv, human IgG1 SCC-P hinge, human IgG1 CH2(ADCC/CDC null), human IgG1 CH3, human Ck(YAE). The nucleotide and amino acid sequences of TSC095 are set forth in SEQ ID NOS:806 and 824, respectively.

The polypeptide heterodimer molecules were expressed and purified according to Example 1.

### EXAMPLE 7

### TARGET-DEPENDENT T-CELL PROLIFERATION BY POLYPEPTIDE HETERODIMERS

To compare the effectiveness of different bispecific polypeptide heterodimer molecules at inducing target-dependent T-cell activation and proliferation, three different bispecific molecules (TSC054, TSC078, and TSC079 as described in Example 6) with a common anti-CD 19 binding domain (HD37) and three different anti-CD3 binding domains (G19-4 for TSC054, OKT3 for TSC078, HuM291 for TSC079) were compared. As a positive control, a Bispecific T-Cell Engaging molecule (BiTE) known as bsc19x3 was also prepared (see U.S. Patent 7,635,472). The nucleotide and amino acid sequences for bsc19x3 are set forth in SEQ ID NOS:809 and 827, respectively. Transient transfection in human 293 cells produced 2.33 ug/mL protein for TSC054, 0.67 ug/mL protein for TSC078, and 3.5 ug/mL for TSC079.

Daudi Burkitt's lymphoma cells (CD19⁺) and MDA-MB-453 (CD19⁻) breast carcinoma cells were obtained from ATCC (Manassas, VA) and cultured according to the provided protocol. Peripheral blood mononuclear cells (PBMC) were isolated from human blood using standard ficoll gradients. The isolated cells were washed in saline buffer. T cells were additionally isolated using a Pan T-cell Isolation Kit II from Miltenyi Biotec (Bergisch Gladbach, Germany) using the manufacturer's protocol (see also Example 5 for more information).

Proliferation was assessed by labeling isolated PBMC or T cell populations with carboxyfluorescein diacetate succinimidyl ester (CFSE). CFSE-labeled PBMC or T cells were plated in U-bottom 96-well plates at 150,000 or 100,000 cells/well, respectively, with various numbers of tumor cells, to achieve T cell to tumor cell ratios of 10:1 to 3:1. Concentrations of test molecules ranging from 8 nM to 0.08 pM were added to the cell mixtures in a total of 200ul/well in RPMI 1640 media supplemented with 10% human or bovine serum, sodium pyruvate and non-essential amino acids. Plates were incubated at 37°C, 5% CO₂ in humidified incubators. After 3 days, cells were labeled with antibodies for flow cytometric analysis. Cells were labeled and washed in their original plates to minimize cell losses during transfers, and all labeling was done in saline buffer with 0.2% bovine serum albumin. First, cells were pre-incubated with 100ug/ml human IgG at room temperature for 15 min. Subsequently, cells were incubated with a mixture (total volume 50 ul) of the following dye-labeled antibodies: CD5-PE, CD4-APC, CD8-Pacific Blue, CD25-PE-Cy7, as well as 7-Amino Actinomycin D (7AAD hereafter) for 40 min. Plates were washed twice, resuspended in 80 to 120ul volumes and ran immediately in a BD LSRII flow cytometer to acquire 80% of the contents of each well. The sample files were analyzed using FlowJo software to calculate the percentages and numbers of cells that had undergone at least one cell division, according to their CFSE profile, by gating sequentially on activated, live CD4+ or CD8+ T cells (7AAD-, CD5+ CD25+ CD4+ or 7AAD- CD5+ CD25+ CD8+, respectively). Mean values and standard deviations were calculated using Microsoft Excel software. Graphs were plotted using Microsoft Excel or Graphpad Prism.

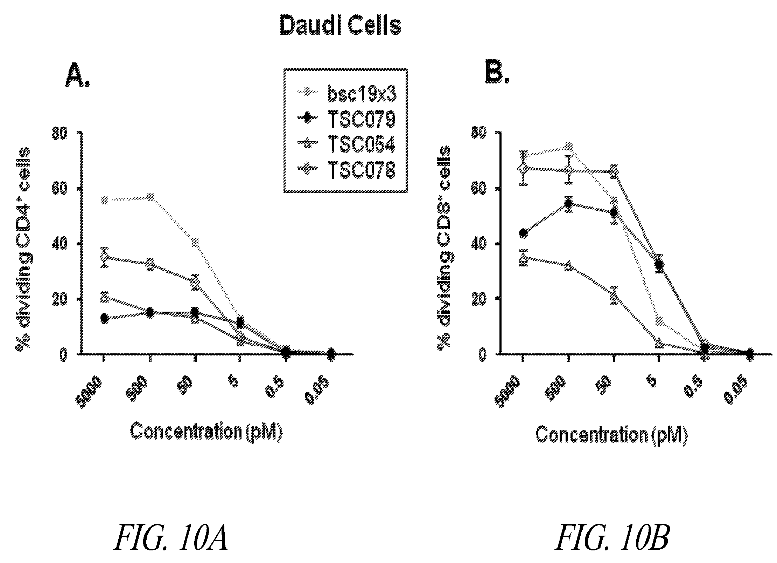

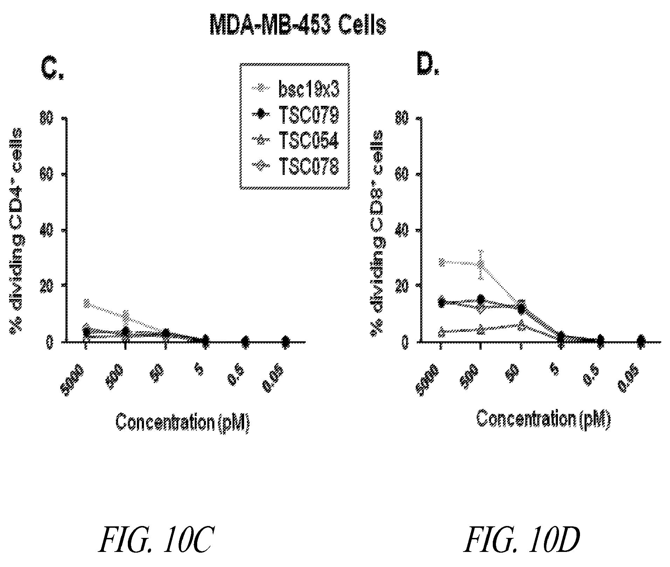

Analysis of live CD4+ and CD8+ populations from Daudi cells or MDA-MB-453 cells treated with whole PBMCs (Figures 10A-10D) revealed a significant increase in both the total number of cells and percent proliferating cells in the presence of Daudi cells displaying the target CD 19 antigen (Figures 10A, 10B), but a lack of significant proliferation in the presence of MDA-MB-453 cells that lacked the CD19 antigen (Figures 10C, 10D). Some proliferation was observed at a lower level with the MDA-MB-453 cells and whole PBMCs, since B-cells (CD19+) were present in the PBMCs, but overall proliferation was greatly reduced in comparison. This selectivity was observed with isolated T-cells as well. Proliferation was higher for CD8+ T-cells than CD4+ T-cells in the presence of Daudi cells or MDA-MB-453 cells treated with PBMCs (Figure 10A -10D), and the proliferation induced by TSC078 (HD37xOKT3) was generally higher at all concentrations than the response induced by TSC054 (HD37xG19-4) or TSC079 (HD37xHuM291)(Figures 10A-10D). Induced proliferation of CD4+ T-cells was lower in all cases for TSC054, TSC078, and TSC079 than the BiTE bsc19x3 (Figure 10A), although TSC078 and TSC079 showed superior induction of proliferation of CD8+ cells at lower concentrations (e.g. 5 pM) than the BiTE molecule (Figure 10B).

### EXAMPLE 8

### REDIRECTED T-CELL CYTOTOXICITY BY POLYPEPTIDE HETERODIMERS

To compare the effectiveness of different bispecific polypeptide heterodimer molecules at inducing target-dependent T-cell cytotoxicity, four different bispecific molecules were compared in a chromium (⁵¹Cr) release assay. Three different bispecific molecules (TSC054, TSC078, TSC079, as described in Example 6) with a common anti-CD19 binding domain (HD37) and three different anti-CD3 binding domains (G19-4 for TSC054, OKT3 for TSC078, HuM291 for TSC079) were tested alongside a fourth bispecific molecule (S0268, described in Example 4) with an anti-RON binding domain (4C04) and an anti-CD3 binding domain (G19-4). Transient transfection in human 293 cells produced about 2.33 µg/mL protein for TSC054, about 0.67 µg/mL protein for TSC078, and about 3.5 µg/mL protein for TSC079.

Daudi Burkitt's lymphoma cells (CD19+, RON-) and BxPC-3 cells (CD 19-, RON+) were obtained from ATCC (Manassas, VA) and cultured according to the provided protocol. Peripheral blood mononuclear cells (PBMC) were isolated from human blood using standard ficoll gradients. The isolated cells were washed in saline buffer. T cells were additionally isolated using a Pan T-cell Isolation Kit II from Miltenyi Biotec (Bergisch Gladbach, Germany) using the manufacturer's protocol (see also Example 5 for more information).

Cytotoxicity was assessed by a ⁵¹Cr release assay. Approximately 5×10⁶ Daudi or BxPC-3 cells were treated with 0.3 mCi of ⁵¹Cr and incubated for 75 minutes at 37°C. After 75 minutes, cells were washed 3 times with media (RPMI + 10% FBS) and resuspended in 11.5 mL of media. From this suspension, 50 µL was dispensed per well into 96 well U-bottom plates (approximately 20,000 cells/well). Concentrations of bispecific molecules ranging from 10 nM to 0.1 pM were added to the target (Daudi, BxPC-3) cells, bringing the total volume to 100 µL/well. Target cells were incubated at room temperature for 15 minutes. Then 100 µL of isolated T-cells (approximately 200,000) were added to bring the T-cell to target cell ratio to 10:1. 50 µL of 0.8% NP-40 was added to a control well containing target cells, left for 15 minutes, then 100 µL of media was added to provide a total lysis control.

Plates were incubated for 4 hours, spun at 1500 rpm for 3 minutes, and 25 µL of supernatant was transferred from each well to the corresponding well of a 96-well Luma sample plate. Sample plates were allowed to air dry in a chemical safety hood for 18 hours, and then radioactivity was read on a Topcount scintillation counter using a standard protocol.

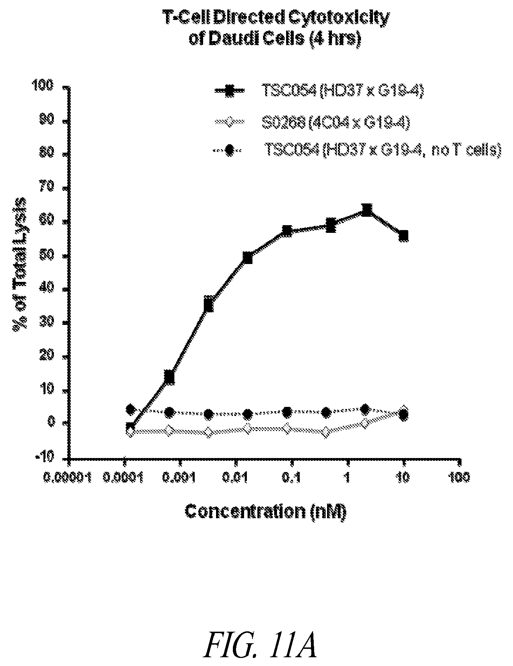

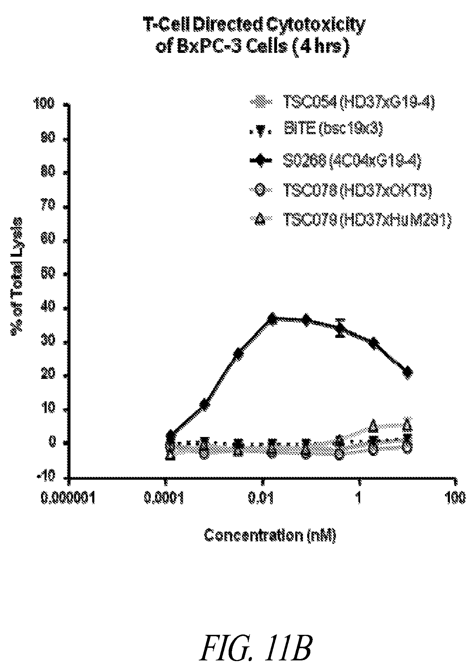

Analysis of cytotoxicity data showed a lack of off-target cytotoxicity on the Daudi (RON-) cells from the anti-RON directed bispecific molecule S0268 (Figure 11A). Similarly, there was a lack of direct cytotoxicity observed from treating Daudi cells with TSC054 in the absence of T-cells (Figure 11A). However, strong T-cell directed cytotoxicity was observed with the Daudi cells in the presence of T-cells and an anti-CD19 directed bispecific molecule (TSC054), reaching maximal lysis at a concentration between 10 and 100 pM (Figure 11A). Similarly, using a second T-cell donor (Figure 11B), no off-target cytotoxicity of the BxPC-3 (CD19-) cells was observed from the CD19-directed bispecifics TSC054, TSC078, or TSC079, or the CD19-directed BiTE bsc19x3. The anti-RON directed S0268 bispecific molecule induced cytotoxicity in BxPC-3 (RON+) cells, reaching a maximum between 10 and 100 pM (Figure 11B).

### EXAMPLE 9

### MODULATION OF TARGET-DEPENDENT T-CELL PROLIFERATION BY POLYPEPTIDE HETERODIMERS WITH ALTERED HINGE SEQUENCES

To compare the effectiveness of different bispecific polypeptide heterodimer molecules with altered hinge sequences at inducing target-dependent T-cell activation and proliferation, six different bispecific heterodimers (TSC100, TSC127, TSC165, TSC166, TSC167 and TSC168 with a common anti-CD19 binding domain (HD37), a common anti-CD3 binding domain (Cris7) and five different hinge constructs (IgG1 SCC-P hinge for TSC100 and TSC127, IgA2 hinge without the first Val residue linked to a human IgG1 core hinge for TSC165, IgG1 SSS-P hinge linked to a human IgG1 core hinge for TSC166, a portion of mutated IgG3 hinge linked to a IgG1 core hinge for TSC167, and the IgM CH2 domain without the first Val linked to a IgG1 core hinge for TSC168) were compared.

More specifically, bispecific heterodimer TSC100 is as described in Example 6.

Bispecific heterodimer TSC127 comprises single chain polypeptides TSC125 (Cris7 CH2(ADCC/CDC null) CH3 CH1) and TSC096 (HD37 CH2(ADCC/CDC null) CH3 Ck(YAE). Single chain polypeptide TSC125 comprises from its amino to carboxyl terminus: humanized Cris7 (anti-CD3) scFv, human IgG1 SCC-P hinge, human IgG1 CH2 (ADCC/CDC null), human IgG1 CH3, and human IgG1 CH1. The nucleotide and amino acid sequences of TSC125 are set forth in SEQ ID NOS:865 and 874, respectively. Single chain polypeptide TSC096 comprises from its amino to carboxyl terminus: HD37 (anti-CD19) scFv, human IgG1 SCC-P hinge, human IgG1 CH2 (ADCC/CDC null), human IgG1 CH3, and human Ck (YAE). The nucleotide and amino acid sequences of TSC096 are set forth in SEQ ID NOS:807 and 825, respectively.

Bispecific heterodimer TSC165 comprises single chain polypeptides TSC157 (Cris7 IgA2UH CH2 (ADCC/CDC null) CH3 CH1) and TSC161 (HD37 IgA2UH CH2 (ADCC/CDC null) CH3 Ck(YAE)). Single chain polypeptide TSC157 comprises from its amino to carboxyl terminus: humanized Cris7(anti-CD3) scFv, human IgA2 hinge without the first Val linked with a human IgG1 core hinge, human IgG1 CH2 (ADCC/CDC null), human IgG1 CH3, and human IgG1 CH1. The nucleotide and amino acid sequences of TSC157 are set forth in SEQ ID NOS:866 and 875, respectively. Single chain polypeptide TSC161 comprises from its amino to carboxyl terminus: HD37 (anti-CD19) scFv, human IgA2 hinge without the first Val linked with a human IgG1 core hinge, human IgG1 CH2 (ADCC/CDC null), human IgG1 CH3, and human Ck (YAE). The nucleotide and amino acid sequences of TSC161 are set forth in SEQ ID NOS:870 and 879, respectively. The amino acid sequence of the hinge used in TSC157 and TSC161 is set forth in SEQ ID NO:748.

Bispecific heterodimer TSC166 comprises single chain polypeptide TSC158 (Cris7 IgG1miniUH CH2 (ADCC/CDC null) CH3 CH1) and TSC162 (HD37 IgG1miniUH CH2 (ADCC/CDC null) CH3 Ck(YAE)). Single chain polypeptide TSC158 comprises from its amino to carboxyl terminus: humanized Cris7 (anti-CD3) scFv, human IgG1 SSC-P hinge linked with a human IgG1 core hinge, human IgG1 CH2 (ADCC/CDC null), human IgG1 CH3, and human IgG1 CH1. The nucleotide and amino acid sequences of TSC158 are set forth in SEQ ID NOS:867 and 876, respectively. Single chain polypeptide TSC162 comprises from its amino to carboxyl terminus: HD37 (anti-CD19) scFv, human IgG1 SSC-P hinge linked with a human IgG1 core hinge, human IgG1 CH2 (ADCC/CDC null), human IgG1 CH3, and human Ck (YAE). The nucleotide and amino acid sequences of TSC162 are set forth in SEQ ID NOS:871 and 880. The amino acid sequence of the hinge used in TSC158 and TSC162 is set forth in SEQ ID NO:750.

Bispecific heterodimer TSC167 comprises single chain polypeptide TSC159 (Cris7 IgG3UH CH2 (ADCC/CDC null) CH3 CH1) and TSC163 (HD37 IgG3UH CH2 (ADCC/CDC null) CH3 Ck(YAE)). Single chain polypeptide TSC159 comprises from its amino to carboxyl terminus: humanized Cris7 (anti-CD3) scFv, a portion of mutated human IgG3 hinge linked with a human IgG1 core hinge, human IgG1 CH2 (ADCC/CDC null), human IgG1 CH3, and human IgG1 CH1. The nucleotide and amino acid sequences of TSC159 are set forth in SEQ ID NOS:868 and 877, respectively. Single chain polypeptide TSC163 comprises from its amino to carboxyl terminus: HD37 (anti-CD19) scFv, a portion of mutated human IgG3 hinge linked with a human IgG1 core hinge, human IgG1 CH2 (ADCC/CDC null), human IgG1 CH3, and human Ck (YAE). The nucleotide and amino acid sequences of TSC163 are set forth in SEQ ID NOS:872 and 881. The amino acid sequence of the hinge used in TSC159 and TSC163 is set forth in SEQ ID NO:751.

Bispecific heterodimer TSC168 comprises single chain polypeptide TSC160 (Cris7 IgMCH2UH CH2 (ADCC/CDC null) CH3 CH1) and TSC164 (HD37 IgMCH2UH CH2 (ADCC/CDC null) CH3 Ck(YAE)). Single chain polypeptide TSC160 comprises from its amino to carboxyl terminus: humanized Cris7(anti-CD3) scFv, human IgM CH2 without the first Val linked with a human IgG1 core hinge, human IgG1 CH2 (ADCC/CDC null), human IgG1 CH3, and human IgG1 CH1. The nucleotide and amino acid sequences of TSC160 are set forth in SEQ ID NOS:869 and 878, respectively. Single chain polypeptide TSC163 comprises from its amino to carboxyl terminus: HD37 (anti-CD19) scFv, human IgM CH2 without the first Val linked with a human IgG1core hinge, human IgG1 CH2 (ADCC/CDC null), human IgG1 CH3, and human Ck (YAE). The nucleotide and amino acid sequences of TSC164 are set forth in SEQ ID NOS:873 and 882. The amino acid sequence of the hinge used in TSC160 and TSC164 is set forth in SEQ ID NO:759.

As a positive control, a Bispecific T-Cell Engaging molecule (BiTE) known as bsc19x3 was also prepared (see U.S. Patent 7,635,472). The nucleotide and amino acid sequences for bsc19x3 are set forth in SEQ ID NOS:809 and 827, respectively.

Transient transfection in human 293 cells produced 3.2 ug protein / mL of culture for TSC100, 6.1 ug protein / mL of culture for TSC127, 4.8 ug protein / mL of culture for TSC165, 6.2 ug protein / mL of culture for TSC166, 6.4 ug protein / mL of culture for TSC167, and 6.4 ug/mL protein for TSC168.

Daudi Burkitt's lymphoma cells (CD19⁺) and C4-2 (CD19⁻) prostate carcinoma cells were obtained from ATCC (Manassas, VA) and MD Anderson Cancer Center (Houston, TX) and cultured according to the provided protocol. T cells were isolated using a Pan T-cell Isolation Kit II from Miltenyi Biotec (Bergisch Gladbach, Germany) using the manufacturer's protocol (see also Example 5 for more information).

Proliferation was assessed by labeling isolated T cell populations with carboxyfluorescein diacetate succinimidyl ester (CFSE). CFSE-labeled T cells were plated in U-bottom 96-well plates at 100,000 cells/well, with 10,000 tumor cells/well, to achieve a T cell to tumor cell ratio of 10:1. Concentrations of test molecules ranging from 5 nM to 0.005 pM were added to the cell mixtures in a total of 200 ul/well in RPMI 1640 media supplemented with 10% human or bovine serum, sodium pyruvate and non-essential amino acids. Plates were incubated at 37°C, 5% CO₂ in humidified incubators. After 3 days, cells were labeled with antibodies for flow cytometric analysis. Cells were labeled and washed in their original plates to minimize cell losses during transfers, and all labeling was done in saline buffer with 0.2% bovine serum albumin. First, cells were pre-incubated with 100 ug/ml human IgG at room temperature for 15 min. Subsequently, cells were incubated with a mixture (total volume 50 ul) of the following dye-labeled antibodies: CD5-PE, CD4-APC, CD8-Pacific Blue, CD25-PE-Cy7, as well as 7-Amino Actinomycin D (7AAD hereafter) for 40 minutes. Plates were washed twice, resuspended in 80 to 120 ul volumes and ran immediately in a BD LSRII flow cytometer to acquire 80% of the contents of each well. The sample files were analyzed using FlowJo software to calculate the percentages and numbers of cells that had undergone at least one cell division, according to their CFSE profile, by gating sequentially on activated, live CD4+ or CD8+ T cells (7AAD-, CD5+ CD25+ CD4+ or 7AAD- CD5+ CD25+ CD8+, respectively). Mean values and standard deviations were calculated using Microsoft Excel software. Graphs were plotted using Microsoft Excel or Graphpad Prism.

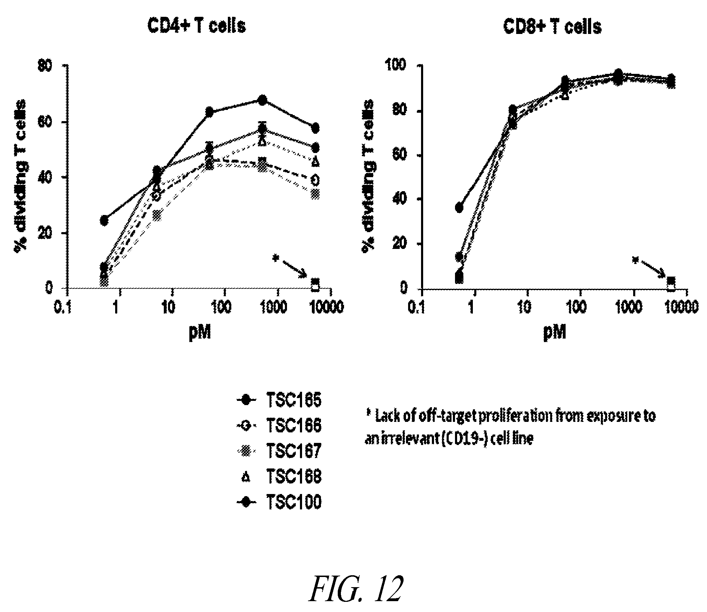

Analysis of live CD4+ and CD8+ populations from Daudi cells or C4-2 cells treated with isolated T-cells revealed a significant increase in both the total number of cells and percent proliferating cells in the presence of Daudi cells displaying the target CD 19 antigen, but a lack of significant proliferation in the presence of C4-2 cells that lacked the CD19 antigen (Figure 12). Proliferation of CD8+ cells was very similar for bispecific molecules with the default IgG1 hinge (TSC100) as well as those with longer hinges (TSC166, TSC167, TSC168). Slightly higher CD8+ proliferation at low concentrations was observed with the bispecific with the shorter IgA2 upper hinge (TSC165). Similar, but more marked differences were observed with proliferation of CD4+ cells, wherein the molecule containing the shorter IgA2 hinge (TSC165) showed higher proliferation at most concentrations than the standard IgG1 hinge (TSC100), and molecules with longer hinges (TSC166, TSC167, TSC168) showed lower proliferation.

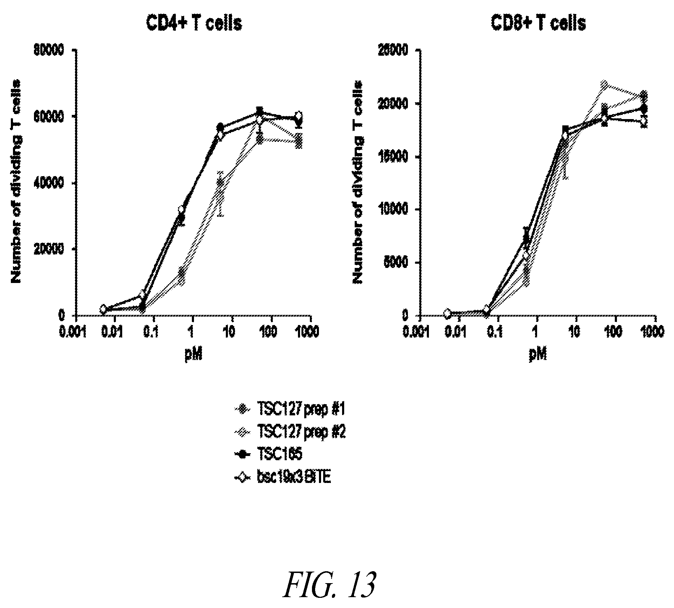

To confirm the differential activity of the IgA2 hinge, a second proliferation experiment was carried out with a titration to lower protein concentrations (Figure 13), comparing the bispecific featuring the IgA2 hinge (TSC165) to two different production lots of a bispecific featuring the default IgG1 hinge (TSC127) and the bsc19x3 BiTE molecule. Similar to the previous experiment, less variation was observed with proliferation of CD8+ cells, with TSC165 showing comparable or greater induction of proliferation to bsc19x3, which in turn showed slightly higher or comparable proliferation to TSC127. Again, similar to the previous experiment, these trends repeated in a magnified fashion with the CD4+ cell proliferation, with TSC165 and bsc19x3 showing comparable proliferation, which in turn was noticeably greater than that of TSC127.

### EXAMPLE 10

### MODULATION OF REDIRECTED T-CELL CYTOTOXICITY BY POLYPEPTIDE HETERODIMERS WITH ALTERED HINGE SEQUENCES

To compare the effectiveness of changing hinge composition in bispecific polypeptide heterodimer molecules on inducing target-dependent T-cell cytotoxicity, five different bispecific molecules were compared in a chromium (⁵¹Cr) release assay. Five different bispecific molecules (TSC100, TSC165, TSC166, TSC167 and TSC168, as described in Example 9) with a common anti-CD 19 binding domain (HD37), a common anti-CD3 binding domain (Cris7) and five different hinge constructs (IgG1 SCC-P hinge for TSC100 and TSC127, IgA2 hinge without the first Val linked to a human IgG1 core hinge for TSC165, IgG1 SSS-P hinge linked to a human IgG1 core hinge for TSC166, a portion of mutated IgG3 hinge linked to a human IgG1 core hinge for TSC167, and the IgM CH2 domain without the first Val linked to a human IgG1 core hinge for TSC168) were compared.

Daudi Burkitt's lymphoma cells (CD19+, RON-) were obtained from ATCC (Manassas, VA) and cultured according to the provided protocol. T cells were isolated using a Pan T-cell Isolation Kit II from Miltenyi Biotec (Bergisch Gladbach, Germany) using the manufacturer's protocol (see also Example 5 for more information).

Cytotoxicity was assessed by a ⁵¹Cr release assay. Approximately 5×10⁶ Daudi cells were treated with 0.3 mCi of ⁵¹Cr and incubated for 75 minutes at 37°C. After 75 minutes, cells were washed 3 times with media (RPMI + 10% FBS) and resuspended in 11.5 mL of media. From this suspension, 50 µL was dispensed per well into 96 well U-bottom plates (approximately 20,000 cells/well). Concentrations of bispecific molecules ranging from 10 nM to 0.1 pM were added to the target (Daudi) cells, bringing the total volume to 100 µL/well. Target cells were incubated at room temperature for 15 minutes. Then 100 µL of isolated T-cells (approximately 200,000) were added to bring the T-cell to target cell ratio to 10:1. 50 µL of 0.8% NP-40 was added to a control well containing target cells, left for 15 minutes, then 100 µL of media was added to provide a total lysis control.

Plates were incubated for 4 hours, spun at 1500 rpm for 3 minutes, and 25 µL of supernatant was transferred from each well to the corresponding well of a 96-well Luma sample plate. Sample plates were allowed to air dry in a chemical safety hood for 18 hours, and then radioactivity was read on a Topcount scintillation counter using a standard protocol.

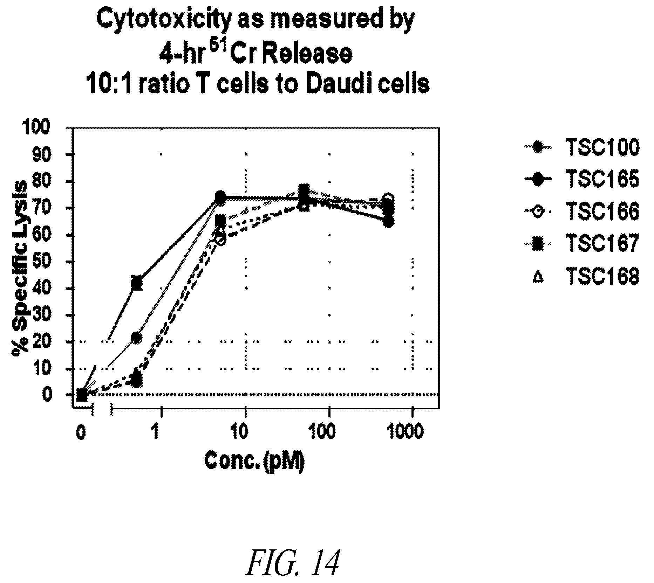

Analysis of cytotoxicity data showed strong T-cell directed cytotoxicity with the Daudi cells in the presence of T-cells and the anti-CD 19 directed bispecific molecules (TSC100 - TSC168), reaching maximal lysis at a concentration between 5 and 50 pM (Figure 14). Similar to the trends observed in Example 9, the bispecific molecule with a shorter IgA2 upper hinge region (TSC165) showed comparable or greater cytotoxicity to the molecule with the standard IgG1 upper hinge region (TSC100), whereas the molecules with longer upper hinge regions (TSC166, TSC167, TSC168) were less potent at inducing cytotoxicity.

### EXAMPLE 11

### BISPECIFIC HETERODIMERS WITH CH3-CH1 AND CH3-CK LINKER VARIATIONS

The following bispecific heterodimers with CH3-CH1 and CH3-Ck linker variations were made:
Bispecific heterodimer TSC151 comprises single chain polypeptides TSC145 and TSC148. Single chain polypeptide TSC145 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence GGGSS (SEQ ID NO:847). The nucleotide and amino acid sequences of TSC145 are set forth in SEQ ID NOS:853 and 859, respectively. Single chain polypeptide TSC148 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence GGGSR (SEQ ID NO:850) in which R may alternatively be regarded as the first arginine of human Ck (YAE). The nucleotide and amino acid sequences of TSC148 are set forth in SEQ ID NOS:856 and 862, respectively.
Bispecific heterodimer TSC152 comprises single chain polypeptides TSC146 and TSC149. Single chain polypeptide TSC146 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence SYSPNS (SEQ ID NO:848). The nucleotide and amino acid sequences of TSC146 are set forth in SEQ ID NOS:854 and 860, respectively. Single chain polypeptide TSC149 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence SYSPNSR (SEQ ID NO:851) in which R may alternatively be regarded as the first arginine of human Ck (YAE). The nucleotide and amino acid sequences of TSC149 are set forth in SEQ ID NOS:857 and 863, respectively.
Bispecific heterodimer TSC153 comprises single chain polypeptides TSC147 and TSC150. Single chain polypeptide TSC147 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence SSLNTPNS (SEQ ID NO:849). The nucleotide and amino acid sequences of TSC147 are set forth in SEQ ID NOS:855 and 861, respectively. Single chain polypeptide TSC150 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence SSLNTPNSR (SEQ ID NO:852) in which R may alternatively be regarded as the first arginine of human Ck (YAE). The nucleotide and amino acid sequences of TSC150 are set forth in SEQ ID NOS:858 and 864, respectively.

The above bispecific heterodimers were expressed according to Example 1. The following expression levels were obtained: 9.2 µg protein / ml of culture for TSC151, 11.2 µg protein / ml of culture for TSC152, and 14.7 µg protein / ml of culture for TSC153. In comparison, about 6 µg protein / ml of culture was obtained for heterodimers with a CH3-CH1 and CH3-Ck linker having the amino acid sequence KSR (SEQ ID NO:846).

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet, are incorporated herein by reference, in their entirety. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.
The present invention will now be described by way of reference to the following clauses:
1. A polypeptide heterodimer, comprising:
   (a) a first single chain polypeptide (SCP-I) comprising from one to four binding domains that specifically bind from one to four targets, a hinge (H-I), an immunoglobulin heterodimerization domain (HD-I), and an Fc region portion (FRP-I); and
   (b) a second single chain polypeptide (SCP-II) comprising from zero to four binding domains that specifically bind from zero to four targets, a hinge (H-II), an immunoglobulin heterodimerization domain (HD-II), and an Fc region portion (FRP- II); wherein
      (i) the immunoglobulin heterodimerization domain of the first single chain polypeptide (HD-I) and the immunoglobulin heterodimerization domain of the second single chain polypeptide (HD-II) preferentially associate with each other to form a polypeptide heterodimer comprised of the first single chain polypeptide (SCP-I) and the second single chain polypeptide (SCP-II), and
         (1) the immunoglobulin heterodimerization domain of the first single chain polypeptide (HD-I) comprises a first immunoglobulin CH1 region, and the immunoglobulin heterodimerization domain of the second single chain polypeptide (HD-II) comprises a first immunoglobulin CL region, or
         (2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region; and
      (ii) the Fc region portion of the first single chain polypeptide (FCP-I) and the Fc region portion of the second single chain polypeptide (FCP-II) each comprise an immunoglobulin CH2 and CH3 domain of IgG1, IgG2, IgG3, IgG4, IgA1, IgA2, IgD, or any combination thereof; an immunoglobulin CH3 domain of IgG1, IgG2, IgG3, IgG4, IgA1, IgA2, IgD, IgE, IgM, or any combination thereof; or an immunoglobulin CH3 and CH4 domain of IgE, IgM, or a combination thereof,
   provided that the polypeptide heterodimer comprises at least two binding domains that specifically bind at least two different targets.
2. The polypeptide heterodimer of clause 1, wherein the binding domains are single chain Fv (scFv) polypeptides.
3. The polypeptide heterodimer of clause 1, wherein the polypeptide heterodimer comprises two binding domains (BD1I and BD2).
4. The polypeptide heterodimer of clause 3, wherein the two binding domains (BDI and BD2) are both on the first single chain polypeptide (SCP-I) and wherein the HD-I and FRP-I are disposed between BD1 and BD2.
5. The polypeptide heterodimer of clause 3, wherein the first binding domain (BD1) is on the first single chain polypeptide (SCP-I) and the second binding domain (BD2) is on the second single chain polypeptide (SCP-II).
6. The polypeptide heterodimer of clause 5, wherein the first binding domain (BD1) is amino terminal to the Fc region portion of the first single chain polypeptide (FRP-I), and the second binding domain (BD2) is amino terminal to the Fc region portion of the second single chain polypeptide (FRP-II).
7. The polypeptide heterodimer of clause 5, wherein the first binding domain (BD1) is amino terminal to the Fc region portion of the first single chain polypeptide (FRP-I), and the second binding domain (BD2) is carboxyl terminal to the Fc region portion of the second single chain polypeptide (FRP-II).
8. The polypeptide heterodimer of clause 5, wherein the first binding domain (BD1) is carboxyl terminal to the Fc region portion of the first single chain polypeptide (FRP-I), and the second binding domain (BD2) is carboxyl terminal to the Fc region portion of the second single chain polypeptide (FRP-II).
9. The polypeptide heterodimer of clause 1, wherein the polypeptide heterodimer comprises three binding domains (BD1, BD2 and BD3).
10. The polypeptide heterodimer of clause 9, wherein the HD-I and FRP-I are disposed between BD1 and BD2, and the third binding domain (BD3) is amino terminal to the Fc region portion of the second single chain polypeptide (FRP-II).
11. The polypeptide heterodimer of clause 9, wherein the HD-I and FRP-I are disposed between BD1 and BD2, and the third binding domain (BD3) is carboxyl terminal to the Fc region portion of the second single chain polypeptide (FRP-II).
12. The polypeptide heterodimer of clause 1, wherein the polypeptide heterodimer comprises four binding domains (BD1, BD2, BD3, and BD4).
13. The polypeptide heterodimer of clause 12, wherein the HD-I and FRP-I are disposed between BD1 and BD2, and the HD-II and FRP-II are disposed between BD3 and BD4.
14. The polypeptide heterodimer of clause 1, wherein the polypeptide heterodimer comprises five to eight binding domains.
15. The polypeptide heterodimer of any one of clauses 1 to 14, wherein at least one of the binding domains specifically binds to, or is an antagonist of, TCRα, TCRβ, CD3γ, CD3δ, CD3ε, CD28, CD79b, hyperIL-6, monoIL-10, CD86, CD20, PSMA, CD19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACl, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL,, HGF, MSP, EGF (including epiregulin, herregulin, β-regulin, neuregulin), HIF-1, VEGFA, VEGFB, VEGFC, VEGFD, TNFα, Wnt, sHH, TGFβ, PDGF, TWEAK, EpCAM, CEA, PCTA-1, STEAP-1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, ganglioside GD3, 9-0-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD 137, LIFRβ, TLR7 or TLR9.
16. The polypeptide heterodimer of any one of clauses 1 to 6, wherein at least one of the binding domains is an agonist of IL-10, HLA-G, HGF, IL-35, PD-1, BTLA, TNFR1, TNFR2, DR4, DR5, TWEAKR, or FAS.
17. The polypeptide heterodimer of any one of clauses 1 to 6, wherein at least one binding domain specifically binds a TCR complex or a component thereof, and at least another binding domain specifically binds to PSMA, CD79b, CD19, HLA-DR, CD20, RON, c-Met, or CEACAM-6.
18. The polypeptide heterodimer of any one of clauses 1 to 6, wherein at least one binding domain specifically binds to CD28, and at least another binding domain specifically binds to, or is an antagonist of, CD79b, hyperIL-6, PDL2, monoIL-10, CD86, LIGHT, GITRL, CD40, PDL1, HVEM, or LTBR.
19. The polypeptide heterodimer of any one of clause 1 to 6, wherein at least one binding domain specifically binds to CD28, and at least another binding domain is an agonist of IL-10, HLA-G, HGF, IL-35, PD-1, or BTLA.
20. The polypeptide heterodimer according to any one of clauses 1 to 19 wherein the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region.
21. The polypeptide heterodimer of clause 20 wherein the first CH1 region is amino terminal to the Fc region portion of the first single chain polypeptide, and the first CL region is amino terminal to the Fc region portion of the second single chain polypeptide.
22. The polypeptide heterodimer of clause 20 wherein the first CH1 region is carboxyl terminal to the Fc region portion of the first single chain polypeptide, and the first CL region is carboxyl terminal to the Fc region portion of the second single chain polypeptide.
23. The polypeptide heterodimer of clause 20 wherein the first single chain polypeptide further comprises a second CH1 region and the second single chain polypeptide further comprises a second CL region, and wherein the second CH1 region of the first single chain polypeptide and the second CL region of the second single chain polypeptide associate with each other in the polypeptide heterodimer.
24. The polypeptide heterodimer of clause 23 wherein the Fc region portion of the first single chain polypeptide is disposed between the first and second CH1 regions, and wherein the Fc region portion of the second single chain polypeptide is disposed between the first and second CL regions.
25. The polypeptide heterodimer of clause 23 wherein both the first and second CH1 regions are amino terminal to the Fc region portion of the first single chain polypeptide, and both the first and second CL regions are amino terminal to the Fc region portion of the second single chain polypeptide.
26. The polypeptide heterodimer of clause 23 wherein both the first and second CH1 regions are carboxyl terminal to the Fc region portion of the first single chain polypeptide, and both the first and second CL regions are carboxyl terminal to the Fc region portion of the second single chain polypeptide.
27. The polypeptide heterodimer according to any one of clauses 1 to 19 wherein the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region.
28. The polypeptide heterodimer of clause 27 wherein the first CL region is amino terminal to the Fc region portion of the first single chain polypeptide, and the first CH1 region is amino terminal to the Fc region portion of the second single chain polypeptide.
29. The polypeptide heterodimer of clause 27 wherein the first CL region is carboxyl terminal to the Fc region portion of the first single chain polypeptide, and the first CH1 region is carboxyl terminal to the Fc region portion of the second single chain polypeptide.
30. The polypeptide heterodimer of clause 27 wherein the first single chain polypeptide further comprises a second CL region and the second single chain polypeptide further comprises a second CH1 region, and wherein the second CL region of the first single chain polypeptide and the second CH1 region of the second single chain polypeptide associate with each other in the polypeptide heterodimer.
31. The polypeptide heterodimer of clause 30 wherein the Fc region portion of the first single chain polypeptide is disposed between the first and second CL regions, and wherein the Fc region portion of the second single chain polypeptide is disposed between the first and second CH1 regions.
32. The polypeptide heterodimer of clause 30, wherein both the first and second CL regions are amino terminal to the Fc region portion of the first single chain polypeptide, and both the first and second CH1 regions are amino terminal to the Fc region portion of the second single chain polypeptide.
33. The polypeptide heterodimer of clause 30, wherein both the first and second CL regions are carboxyl terminal to the Fc region portion of the first single chain polypeptide, and both the first and second CH1 regions are carboxyl terminal to the Fc region portion of the second single chain polypeptide.
34. The polypeptide heterodimer of clause 20 wherein the first single chain polypeptide further comprises a second CL region and the second single chain polypeptide further comprises a second CHI region, and wherein the second CL region of the first single chain polypeptide and the second CHI region of the second single chain polypeptide associate with each other in the polypeptide heterodimer.
35. The polypeptide heterodimer of clause 34 wherein
   (a) in the first single chain polypeptide, the first CH1 region is amino terminal to the Fc region portion, and the second CL region is carboxyl terminal to the Fc region portion, and
   (b) in the second single chain polypeptide, the first CL region is amino terminal to the Fc region portion, and the second CH1 region is carboxyl terminal to the Fc region portion.
36. The polypeptide heterodimer of clause 34 wherein
   (a) in the first single chain polypeptide, the first CH1 region is carboxyl terminal to the Fc region portion, and the second CL region is amino terminal to the Fc region portion, and
   (b) in the second single chain polypeptide, the first CL region is carboxyl terminal to the Fc region portion, and the second CH1 region is amino terminal to the Fc region portion.
37. The polypeptide heterodimer of clause 34 wherein
   (a) in the first single chain polypeptide, both the first CH1 region and the second CL regions are amino terminal to the Fc region portion, and the first CH1 region is amino terminal to the second CL region, and
   (b) in the second single chain polypeptide, both the first CL region and the second CH1 region are amino terminal to the Fc region portion, and the first CL region is amino terminal to the second CH1 region.
38. The polypeptide heterodimer of clause 34 wherein
   (a) in the first single chain polypeptide, both the first CH1 region and the second CL regions are amino terminal to the Fc region portion, and the second CL region is amino terminal to the first CH1 region, and
   (b) in the second single chain polypeptide, both the first CL region and the second CH1 region are amino terminal to the Fc region portion, and the second CH1 region is amino terminal to the first CL region.
39. The polypeptide heterodimer of clause 34 wherein
   (a) in the first single chain polypeptide, both the first CH1 region and the second CL regions are carboxyl terminal to the Fc region portion, and the first CH1 region is amino terminal to the second CL region, and
   (b) in the second single chain polypeptide, both the first CL region and the second CH1 region are carboxyl terminal to the Fc region portion, and the first CL region is amino terminal to the second CH1 region.
40. The polypeptide heterodimer of clause 34 wherein
   (a) in the first single chain polypeptide, both the first CH1 region and the second CL regions are carboxyl terminal to the Fc region portion, and the second CL region is amino terminal to the first CH1 region, and
   (b) in the second single chain polypeptide, both the first CL region and the second CH1 region are carboxyl terminal to the Fc region portion, and the second CH1 region is amino terminal to the first CL region.
41. The polypeptide heterodimer of any one of clauses 1 to 40 wherein the first CL region is a Cκ region.
42. The polypeptide heterodimer of any one of clauses 1 to 40 wherein the first CL region is a Cλ region.
43. The polypeptide heterodimer of any one of clauses 24 to 27 and 31 to 41, wherein the second CL region is a Cκ region.
44. The polypeptide heterodimer of any one of clauses 23 to 26 and 30 to 40, wherein the second CL region is a Cλ region.
45. The polypeptide heterodimer of clause 41 or clause 43, wherein the Cκ region is a wild type human immunoglobulin Cκ region.
46. The polypeptide heterodimer of clause 41 or clause 43, wherein the Cκ region is an altered human immunoglobulin Cκ region with one or more amino acids of a wild type human Cκ region substituted at N29, N30, Q52, V55, T56, T56, S68, or T70.
47. he polypeptide heterodimer of clause 46, wherein the one or more amino acid substitutions are selected from Ala (A), Arg (R), Trp (W), Tyr (Y), Glu (E), Gin (Q), Lys (K), Asp (D), Met (M), Ser (S), and Phe (F).
48. The polypeptide heterodimer of clause 41 or clause 43, wherein the CH1 region is an altered human immunoglobulin CH1 region comprising an amino acid substitution by which Val (V) at position 68 is substituted by Lys (K), Arg (R) or His (H), and wherein the Ck region is an altered human immunoglobulin Ck region comprising an amino acid substitution by which Leu (L) at position 29 is substituted by Asp (D) or Glu (E).
49. The polypeptide heterodimer of clause 41 or clause 43, wherein the CH1 region is an altered human immunoglobulin CH1 region comprising an amino acid substitution by which Val (V) at position 68 is changed to Asp (D) or Glu (E), and wherein the Ck region is an altered human immunoglobulin Ck region comprising an amino acid substitution by which Leu (L) at position 29 is changed to Lys (K), Arg (R) or His (H).
50. The polypeptide heterodimer of clause 42 or clause 44, wherein the Cλ region is a wild type human immunoglobulin Cλ region.
51. The polypeptide heterodimer of any one of clauses 1 to 50, wherein the first CH1 region or the second CH1 region when present is a wild type human immunoglobulin CH1 region.
52. The polypeptide heterodimer of any one of clauses 1 to 50, wherein the first CH1 region or the second CH1 region when present is a wild type human IgG1 CH1 region.
53. The polypeptide heterodimer of any one of clauses 1 to 50, wherein the first CH1 region or the second CH1 region when present is an altered human immunoglobulin CH1 region with the cysteine of a wild type human immunoglobulin CH1 region that is involved in forming a disulfide bond with a wild type human immunoglobulin CL region deleted or substituted.
54. The polypeptide heterodimer of any one of clauses 1 to 50, wherein the first CH1 region or the second CH1 region when present is an altered human IgG1 CH1 region with the cysteine of a wild type human immunoglobulin CH1 region that is involved in forming a disulfide bond with a wild type human immunoglobulin CL region deleted or substituted.
55. The polypeptide heterodimer of clause 41 or 43, wherein the CK region is an altered human immunoglobulin Cκ region with the cysteine residue of a wild type human Cκ region that is involved in forming a disulfide bond with a wild type human immunoglobulin CHI region deleted or substituted.
56. The polypeptide heterodimer of clause 42 or clause 44, wherein the Cλ region is an altered human immunoglobulin Cλ region with the cysteine residue of a wild type human Cλ region that is involved in forming a disulfide bond with a wild type human immunoglobulin CH1 region deleted or substituted.
57. The polypeptide heterodimer of clause 53, wherein the first CH1 region and the second CH1 region when present is a polypeptide comprising SEQ ID NO: 114, 844 or 845.
58. The polypeptide heterodimer of clause 46 or 55, wherein the Ck region is selected from any one of the polypeptides comprising SEQ ID NOS: 141-178, 202, and 838-843.
59. The polypeptide heterodimer of clause 50 or 56, wherein the Cλ region is a polypeptide comprising SEQ ID NO: 140.
60. The polypeptide heterodimer of any one of clauses 1 to 59, wherein the Fc region portion of the first single chain polypeptide (FRP-I) and the Fc region portion of the second single chain polypeptide (FRP-II) each comprise an immunoglobulin CH2 domain.
61. The polypeptide heterodimer of clause 60, wherein the immunoglobulin CH2 domain is an IgG1 CH2 domain.
62. The polypeptide heterodimer of clause 60, wherein the immunoglobulin CH2 domain is an IgG2, IgG3, IgG4, IgA1, IgA2, or IgD CH2 domain.
63. The polypeptide heterodimer of any one of clauses 1 to 59, wherein the Fc region portion of the first single chain polypeptide (FRP-I) and the Fc region portion of the second single chain polypeptide (FRP-II) each comprise an immunoglobulin CH3 domain.
64. The polypeptide heterodimer of clause 63, wherein the immunoglobulin CH3 domain is an IgG1 CH3 domain.
65. The polypeptide heterodimer of clause 63, wherein the immunoglobulin CH3 domain is an IgG2, IgG3, IgG4, IgA1, IgA2, IgD, IgE or IgM CH3 domain.
66. The polypeptide heterodimer of any one of clauses 1 to 65, wherein the Fc region portion of the first single chain polypeptide (FRP-I) and the Fc region portion of the second single chain polypeptide (FRP-II) each comprise an immunoglobulin CH2 domain and an immunoglobulin CH3 domain.
67. The polypeptide heterodimer of clause 66, wherein
   (i) the immunoglobulin CH3 domain is linked to a CH1 domain immediately carboxyl terminal to the immunoglobulin CH3 domain in one single chain polypeptide via a peptide comprising SEQ ID NOS:846, 847, 848, or 849, and
   (ii) the immunoglobulin CH3 domain is linked to a Ck domain immediately carboxyl terminal to the immunoglobulin CH3 domain in the other single chain polypeptide via a peptide comprising SEQ ID NOs:846, 850, 951, or 852.
68. The polypeptide heterodimer of clause 66, wherein the immunoglobulin CH2 and CH3 domains are IgG1, IgG2, IgG3, IgG4, IgA1, IgA2, or IgD CH2 and CH3 domains.
69. The polypeptide heterodimer of any one of clauses 1 to 59, wherein the Fc region portion of the first single chain polypeptide (FRP-I) and the Fc region portion of the second chain polypeptide (FRP-II) comprise IgM or IgE CH3 and CH4 domains.
70. The polypeptide heterodimer of any one of clauses 60 to 62 and 66 to 68, wherein the CH2 domain is an altered human IgG1, IgG2, IgG3, or IgG4 CH2 domain that comprises an amino acid substitution at position 297 and at least one additional substitution or deletion at positions 234 to 238.
71. The polypeptide heterodimer of any one of clauses 60 to 62 and 66 to 68, wherein the CH2 domain is an altered human IgG1, IgG2, IgG3, or IgG4 CH2 domain that comprises one or more amino acid mutations at positions 234-238 and at least one substitution at position 253, 310, 318, 320, 322, or 331.
72. The polypeptide heterodimer of any one of clauses 60 to 62 and 66 to 68, wherein the CH2 domain is an altered human IgG1, IgG2, IgG3, or IgG4 CH2 domain that comprises an amino acid substitution at the asparagine of position 297, one or more substitutions or deletions at positions 234 to 238, and at least one substitution at position 253, 310, 318, 320, 322, or 331.
73. The polypeptide heterodimer of any one of clauses 60 to 62 and 66 and 68, wherein the CH2 domain is an altered human IgG1 CH2 domain that comprises amino acid substitutions at positions L234, L235, G237, E318, K320 and K322.
74. The polypeptide heterodimer of any one of clauses 63 to 67, wherein
   (a) the CH3 domain of the first single chain polypeptide is an altered human IgG1, IgG2, IgG3, or IgG4 CH3 domain that comprises a T366W, and the CH3 domain of the second single chain polypeptide is an altered human IgG1, IgG2, IgG3 or IgG4 CH3 domain that comprises a Y407A substitution,
   (b) the CH3 domain of the first single chain polypeptide is an altered human IgG1, IgG2, IgG3, or IgG4 CH3 domain that comprises a T366Y substitution, and the CH3 domain of the second single chain polypeptide is an altered human IgG1, IgG2, IgG3 or IgG4 CH3 domain that comprises a Y407T substitution,
   (c) the CH3 domain of the first single chain polypeptide is an altered human IgG1, IgG2, IgG3, or IgG4 CH3 domain that comprises a T366W substitution, and the CH3 domain of the second single chain polypeptide is an altered human IgG1, IgG2, IgG3 or IgG4 CH3 domain that comprises T366S, L368A and Y407V substitutions,
   (d) the CH3 domain of the first single chain polypeptide is an altered human IgG1, IgG2, IgG3, or IgG4 CH3 domain that comprises a Y407A substitution, and the CH3 domain of the second single chain polypeptide is an altered human IgG1, IgG2, IgG3 or IgG4 CH3 domain that comprises a T366W substitution,
   (e) the CH3 domain of the first single chain polypeptide is an altered human IgG1, IgG2, IgG3, or IgG4 CH3 domain that comprises a Y407T substitution, and the CH3 domain of the second single chain polypeptide is an altered human IgG1, IgG2, IgG3 or IgG4 CH3 domain that comprises a T366Y substitution, or
   (f) the CH3 domain of the first single chain polypeptide is an altered human IgG1, IgG2, IgG3, or IgG4 CH3 domain that comprises T366S, L368A and Y407W substitutions, and the CH3 domain of the second single chain polypeptide is an altered human IgG1, IgG2, IgG3 or IgG4 CH3 domain that comprises a T366W substitution.
75. The polypeptide heterodimer of any one of clauses 1 to 74 wherein the hinge of both the first and second single chain polypeptides is an immunoglobulin hinge region.
76. The polypeptide heterodimer of clause 75 wherein the immunoglobulin hinge is an IgG1, IgG2, IgG3, IgG4, IgA1, IgA2, IgD, or IgE hinge.
77. The polypeptide heterodimer of clause 75 wherein the immunoglobulin hinge is a wild type immunoglobulin hinge.
78. The polypeptide heterodimer of clause 75 wherein the immunoglobulin hinge is an altered immunoglobulin hinge selected from SEQ ID NOS:232, 234, 240, 664-673, 675 and 676.
79. The polypeptide heterodimer of any one of clauses 75 to 78 wherein the hinge region is
   (a) at the amino terminal to the Fc region portion,
   (b) disposed between the binding domain and the immunoglobulin heterodimerization domain,
   (c) disposed between the immunoglobulin heterodimerization domain and the Fc region portion, or
   (d) at the amino terminus of the first or second single chain polypeptide.
80. The polypeptide heterodimer of any one of clauses 1 to 74 wherein at least one of the first and second single chain polypeptide hinges is a C-type lectin hinge region.
81. The polypeptide heterodimer of clause 80 wherein the C-type lectin hinge region is an NKg2A or NKg2D peptide, or a derivative thereof.
82. The polypeptide heterodimer of clause 80 or clause 81 wherein the hinge region is disposed between the Fc region portion and a binding domain, or at the carboxyl terminus of the first or second single chain polypeptide.
83. The polypeptide heterodimer of any one of clauses 1 to 82 wherein the hinges of the first and second single chain polypeptides are identical.
84. The polypeptide heterodimer of any one of clauses 1 to 82 wherein the hinges of the first and second single chain polypeptides are different.
85. The heterodimer of any one of clauses 1-14 and 20-84, wherein the first single chain polypeptide comprises a binding domain that specifically binds a TCR complex or a component thereof, and the second single chain polypeptide comprises a binding domain that specifically binds CD19, CD79b, HLA-DR or CD20.
86. The heterodimer of any one of clauses 1-14 and 20-84, wherein the first single chain polypeptide comprises a binding domain that specifically binds CD28, and the second single chain polypeptide comprises a binding domain (a) that specifically binds CD79b, hyperIL-6, or CD86 or (b) that comprises a PDL ectodomain or a monoIL-10.
87. The heterodimer of any one of clauses 1-14 and 20-84, wherein the first single chain polypeptide comprises a binding domain that specifically binds c-Met, and the second single chain polypeptide comprises a binding domain that specifically binds RON.
88. The heterodimer of clause 1, wherein the first and second single chain polypeptides comprise SEQ ID NOS: SEQ ID NOS:2 and 4, SEQ ID NOS:6 and 8, SEQ ID NOS: 10 and 12, SEQ ID NOS: 14 and 16, SEQ ID NOS: 18 and 20, SEQ ID NOS:20 and 22, SEQ ID NOS:20 and 24, SEQ ID NOS:30 and 32, SEQ ID NOS:29 and 31, SEQ ID NOS:29 and 32, SEQ ID NOS:30 and 72, SEQ ID NOS:53 and 72, SEQ ID NOS:54 and 72, SEQ ID NOS:55 and 72, SEQ ID NOS:70 and 72, SEQ ID NOS:71 and 72, SEQ ID NOS:63 and 56, SEQ ID NOS:64 and 57, SEQ ID NOS:65 and 60, SEQ ID NOS:66 and 58, SEQ ID NOS:67 and 59, SEQ ID NOS:68 and 61, SEQ ID NOS:69 and 62, SEQ ID NOS:54 and 811, SEQ ID NOS:54 and 812, SEQ ID NOS:54 and 813, SEQ ID NOS:814 and 818, SEQ ID NOS:815 and 818, SEQ ID NOS:816 and 818, SEQ ID NOS:817 and 818, SEQ ID NOS:814 and 820, SEQ ID NOS:814 and 821, SEQ ID NOS:54 and 819, SEQ ID NOS:814 and 826, SEQ ID NOS:814 and 822, SEQ ID NOS: 814 and 823, SEQ ID NOS:814 and 824, SEQ ID NOS:859 and 862, SEQ ID NOS:860 and 863, SEQ ID NOS:861 and 864, SEQ ID NOS:874 and 825, SEQ ID NOS:875 and 879, SEQ ID NOS:876 and 880, SEQ ID NOS:877 and 881, or SEQ ID NOS:878 and 882.
89. A composition comprising a polypeptide heterodimer of any one of clauses 1 to 88 and a pharmaceutically acceptable excipient.
90. An expression vector capable of expressing the polypeptide heterodimer of any one of clauses 1 to 88, comprising a first polynucleotide encoding the first single chain polypeptide and a second polynucleotide encoding the second single chain polypeptide.
91. A host cell comprising the expression vector of clause 90.
92. A host cell comprising first and second expression vectors capable of expressing the first and second single chain polypeptides, respectively, of the polypeptide heterodimer of any one of clauses 1 to 88.
93. A method for making a polypeptide heterodimer, comprising
   (a) culturing a host cell of clause 91 or clause 92 under conditions suitable to express first and second single chain polypeptides, and
   (b) optionally isolating or purifying the heterodimers formed from the first and second single chain polypeptides from the culture.
94. A method for directing T cell activation, comprising administering to a patient in need thereof an effective amount of a polypeptide heterodimer according to any one of clauses 1 to 88, wherein the polypeptide heterodimer comprises a binding domain that specifically binds TCRα, TCRβ, CD3γ, CD3δ, CD3ε or a combination thereof, and a second binding domain that specifically binds a different target.
95. A method for inhibiting growth, metastasis or metastatic growth of a malignancy, comprising administering to a patient in need thereof an effective amount of a polypeptide heterodimer according to any one of clauses 1 to 88, wherein the polypeptide heterodimer comprises a binding domain that specifically binds TCRα, TCRβ, CD3γ, CD3δ, CD3ε, c-Met, or Ron.
96. A method for treating an autoimmune or inflammatory condition, comprising administering to a patient in need thereof an effective amount of a polypeptide heterodimer according to any one of clauses 1 to 88, wherein the polypeptide heterodimer comprises a binding domain that specifically binds TCRα, TCRβ, CD3γ, CD3δ, CD3ε, or CD28.
97. A method for treating a B-cell associated disorder or disease comprising administering to a patient in need thereof an effective amount of a polypeptide heterodimer according to any one of clauses 1 to 88, wherein the polypeptide heterodimer comprises a binding domain that specifically binds TCRα, TCRβ, CD3γ, CD3δ, CD3ε, and a second binding domain that specifically binds to CD 19, CD20, CD79b or HLA-DR.
98. The method of clause 95, further comprising administering to a patient in need thereof a chemotherapeutic agent or ionizing radiation.
99. The method of any one of clauses 94 to 97, further comprising administering to a patient in need thereof a second active agent.
100. The method of clause 99, wherein the second active agent is a second polypeptide heterodimer according to any one of clauses 1 to 88.
101. The method of clause 99, wherein the second active agent is a monoclonal antibody or an immunoglobulin-derived fusion protein.

## Claims

1. A polypeptide heterodimer, comprising:
(a) a first single chain polypeptide (SCP-I) comprising from one to four binding domains that specifically bind from one to four targets, a hinge (H-I), an immunoglobulin heterodimerization domain (HD-I), and an Fc region portion (FRP-I); and
(b) a second single chain polypeptide (SCP-II) comprising from zero to four binding domains that specifically bind from zero to four targets, a hinge (H-II), an immunoglobulin heterodimerization domain (HD-II), and an Fc region portion (FRP- II); wherein
(i) the immunoglobulin heterodimerization domain of the first single chain polypeptide (HD-I) and the immunoglobulin heterodimerization domain of the second single chain polypeptide (HD-II) preferentially associate with each other to form a polypeptide heterodimer comprised of the first single chain polypeptide (SCP-I) and the second single chain polypeptide (SCP-II), and
(1) the immunoglobulin heterodimerization domain of the first single chain polypeptide (HD-I) comprises a first immunoglobulin CH1 region, and the immunoglobulin heterodimerization domain of the second single chain polypeptide (HD-II) comprises a first immunoglobulin CL region, or
(2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region; and
(ii) the Fc region portion of the first single chain polypeptide (FCP-I) and the Fc region portion of the second single chain polypeptide (FCP-II) each comprise an immunoglobulin CH2 and CH3 domain of IgG1, IgG2, IgG3, IgG4, IgA1, IgA2, IgD, or any combination thereof; an immunoglobulin CH3 domain of IgG1, IgG2, IgG3, IgG4, IgA1, IgA2, IgD, IgE, IgM, or any combination thereof; or an immunoglobulin CH3 and CH4 domain of IgE, IgM, or a combination thereof,
wherein the polypeptide heterodimer comprises two binding domains (BDI and BD2) and wherein the two binding domains (BDI and BD2) are both on the first single chain polypeptide (SCP-I) and wherein the HD-I and FRP-I are disposed between BD1 and BD2.

2. The polypeptide heterodimer of claim 1, wherein the binding domains are single chain Fv (scFv) polypeptides.

3. The polypeptide heterodimer of any one of claims 1 or 2, wherein at least one of the binding domains specifically binds to, or is an antagonist of, TCRα, TCRβ, CD3γ, CD3δ, CD3ε, CD28, CD79b, hyperIL-6, monoIL-10, CD86, CD20, PSMA, CD19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACl, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL,, HGF, MSP, EGF (including epiregulin, herregulin, β-regulin, neuregulin), HIF-1, VEGFA, VEGFB, VEGFC, VEGFD, TNFα, Wnt, sHH, EGFβ, PDGF, TWEAK, EpCAM, CEA, PCTA-1, STEAP- 1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, ganglioside GD3, 9-0-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD 137, LIFRβ, TLR7 or TLR9; or wherein at least one of the binding domains is an agonist of IL-10, HLA-G, HGF, IL-35, PD-1, BTLA, TNFR1, TNFR2, DR4, DR5, TWEAKR, or FAS; or wherein at least one binding domain specifically binds a TCR complex or a component thereof, and at least another binding domain specifically binds to PSMA, CD79b, CD19, HLA-DR, CD20, RON, c-Met, or CEACAM-6; or wherein at least one binding domain specifically binds to CD28, and at least another binding domain specifically binds to, or is an antagonist of, CD79b, hyperIL-6, PDL2, monoIL-10, CD86, LIGHT, GITRL, CD40, PDL1, HVEM, or LTBR; or wherein at least one binding domain specifically binds to CD28, and at least another binding domain is an agonist of IL-10, HLA-G, HGF, IL-35, PD-1, or BTLA.

4. The polypeptide heterodimer according to any one of claims 1 to 3 wherein the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region.

5. The polypeptide heterodimer of claim 4 wherein the first CH1 region is amino terminal to the Fc region portion of the first single chain polypeptide, and the first CL region is amino terminal to the Fc region portion of the second single chain polypeptide; or wherein the first CH1 region is carboxyl terminal to the Fc region portion of the first single chain polypeptide, and the first CL region is carboxyl terminal to the Fc region portion of the second single chain polypeptide.

6. The polypeptide heterodimer of claim 4 wherein the first single chain polypeptide further comprises a second CH1 region and the second single chain polypeptide further comprises a second CL region, and wherein the second CH1 region of the first single chain polypeptide and the second CL region of the second single chain polypeptide associate with each other in the polypeptide heterodimer and wherein
a) the Fc region portion of the first single chain polypeptide is disposed between the first and second CH1 regions, and wherein the Fc region portion of the second single chain polypeptide is disposed between the first and second CL regions; or
b) wherein both the first and second CH1 regions are amino terminal to the Fc region portion of the first single chain polypeptide, and both the first and second CL regions are amino terminal to the Fc region portion of the second single chain polypeptide; or
c) wherein both the first and second CH1 regions are carboxyl terminal to the Fc region portion of the first single chain polypeptide, and both the first and second CL regions are carboxyl terminal to the Fc region portion of the second single chain polypeptide.

7. The polypeptide heterodimer according to any one of claims 1 to 6 wherein the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region.

8. The polypeptide heterodimer of any one of the preceding claims wherein the first CL region is a Cκ region or is a Cλ region.

9. The polypeptide heterodimer of claim 8, wherein the Cκ region is a wild type human immunoglobulin Cκ region.

10. The polypeptide heterodimer of claim 9, wherein the Cκ region is an altered human immunoglobulin Cκ region with one or more amino acids of a wild type human Cκ region substituted at N29, N30, Q52, V55, T56, T56, S68, or T70.

11. The polypeptide heterodimer of claim10, wherein the one or more amino acid substitutions are selected from Ala (A), Arg (R), Trp (W), Tyr (Y), Glu (E), Gin (Q), Lys (K), Asp (D), Met (M), Ser (S), and Phe (F).

12. The polypeptide heterodimer of claim 8, wherein the CH1 region is an altered human immunoglobulin CH1 region comprising an amino acid substitution by which Val (V) at position 68 is substituted by Lys (K), Arg (R) or His (H), and wherein the Ck region is an altered human immunoglobulin Ck region comprising an amino acid substitution by which Leu (L) at position 29 is substituted by Asp (D) or Glu (E); or wherein the CH1 region is an altered human immunoglobulin CH1 region comprising an amino acid substitution by which Val (V) at position 68 is changed to Asp (D) or Glu (E), and wherein the Ck region is an altered human immunoglobulin Ck region comprising an amino acid substitution by which Leu (L) at position 29 is changed to Lys (K), Arg (R) or His (H).

13. The polypeptide heterodimer of any one of the preceding claims wherein the hinge of both the first and second single chain polypeptides is an immunoglobulin hinge region, preferably an IgG1, IgG2, IgG3, IgG4, IgA1, IgA2, IgD, or IgE hinge.

14. A composition comprising a polypeptide heterodimer of any one of the preceding claims and a pharmaceutically acceptable excipient.

15. The polypeptide heterodimer of any one of the preceding claims for the use in:
a) a method for directing T cell activation, comprising administering to a patient in need thereof an effective amount of the polypeptide heterodimer, wherein the polypeptide heterodimer comprises a binding domain that specifically binds TCRα, TCRβ, CD3γ, CD3δ, CD3ε or a combination thereof, and a second binding domain that specifically binds a different target; or
b) a method for inhibiting growth, metastasis or metastatic growth of a malignancy, comprising administering to a patient in need thereof an effective amount of the polypeptide heterodimer, wherein the polypeptide heterodimer comprises a binding domain that specifically binds TCRα, TCRβ, CD3γ, CD3δ, CD3ε, c-Met, or Ron; or
c) a method for treating an autoimmune or inflammatory condition, comprising administering to a patient in need thereof an effective amount of the polypeptide heterodimer, wherein the polypeptide heterodimer comprises a binding domain that specifically binds TCRα, TCRβ, CD3γ, CD3δ, CD3ε, or CD28; or
d) a method for treating a B-cell associated disorder or disease comprising administering to a patient in need thereof an effective amount of the polypeptide heterodimer, wherein the polypeptide heterodimer comprises a binding domain that specifically binds TCRα, TCRβ, CD3γ, CD3δ, CD3ε, and a second binding domain that specifically binds to CD 19, CD20, CD79b or HLA-DR.
